# EUROPEAN PATENT SPECIFICATION

(11) **EP 4 259 822 B1**
(45) Date of publication and mention of the grant of the patent: **16.09.2026**
(21) Application number: 21907673.4
(22) Date of filing: 14.12.2021
(51) Int. Cl.: A01H 3/00, B01L 3/00, C12N 15/11, C12N 15/74, C12Q 1/6809, B01L 7/00, C12Q 1/70, C12Q 1/6806, C12Q 1/682

(54) **SINGLE-BUFFER COMPOSITIONS FOR NUCLEIC ACID DETECTION**
EINZELPUFFERZUSAMMENSETZUNGEN ZUM NACHWEIS VON NUKLEINSÄUREN
COMPOSITIONS À TAMPON UNIQUE POUR LA DÉTECTION D'ACIDES NUCLÉIQUES

(30) Priority: 14.12.2020 US 202063125384 P; 14.12.2020 US 202063125387 P; 19.02.2021 US 202163151592 P; 26.03.2021 US 202163166923 P; 15.07.2021 US 202163222377 P; 01.09.2021 US 202163239884 P; 01.09.2021 US 202163239917 P
(43) Date of publication of application: 18.10.2023
(73) Proprietor: Mammoth Biosciences, Inc., Brisbane CA 94005 (US)
(72) Inventor: CHEN, Janice Sha, Brisbane, California 94005 (US); HENDRIKS, Carley Gelenter, Brisbane, California 94005 (US); FASCHING, Clare Louise, Brisbane, California 94005 (US); MIAO, Xin, Brisbane, California 94005 (US); ALPAY, Nazmiye Emel, Brisbane, California 94005 (US); BROUGHTON, James Paul, Brisbane, California 94005 (US); HAWKINS, Elizabeth M., Brisbane, California 94005 (US); VEROSLOFF, Matthew S., Brisbane, California 94005 (US); HUBBELL, Sophia, Brisbane, California 94005 (US); SHAPIRO, Sarah Jane, Brisbane, CA 94005 (US); KREINDLER, Lior, Brisbane, CA 94005 (US); CHING, Jesus, Brisbane, CA 94005 (US)
(74) Representative: Carpmaels & Ransford LLP
(86) International application number: PCT/US2021/063405
(87) International publication number: WO 2022/132833

(56) References cited:
- WO-A1-2018/107129
- WO-A1-2020/028729
- WO-A1-2020/142739
- WO-A1-2021/252836
- WO-A2-2020/142754
- WO-A2-2021/243308

## Description

### CROSS-REFERENCE

This application claims the benefit of U.S. Provisional Application No. 63/125,384, filed on December 14, 2020; U.S. Provisional Application No. 63/166,923, filed on March 26, 2021; U.S. Provisional Application No. 63/239,884 filed on September 1, 2021; U.S. Provisional Application No. 63/125,387 filed on December 14, 2020; U.S. Provisional Application No. 63/222,377 filed on July 15, 2021; U.S. Provisional Application No. 63/239,917 filed on September 1, 2021; and U.S. Provisional Application No. 63/151,592 filed on February 19, 2021.

### STATEMENT AS TO FEDERALLY SPONSORED RESEARCH

This invention was made with government support under Contract No. N66001-21-C-4048 awarded by the Department of Defense, Defense Advanced Research Projects Agency (DARPA). The US government has certain rights in the invention.

### BACKGROUND

Detection of ailments, especially at the early stages of disease or infection, can provide guidance on treatment or intervention to reduce the progression or transmission of said ailments. Such ailments can be detected at the point of need by devices capable of running diagnostic assays. Various biological species associated with an organism, disease state, phenotype, or genotype can be detected by these devices. In particular, there is a need for rapid and low cost point-of-care testing for detection of viral infections, for example, including human immunodeficiency virus (HIV), SARS and MERS coronaviruses, influenza H1N1 virus, Ebola virus (EBOV), Zika virus (ZIKV), and SARS-CoV-2.

Despite decades of continuous and rapid development in nucleic acid diagnostics, nucleic acid detection is typically time intensive, error prone, and susceptible to contamination. Many of these problems stem from the multi-condition requirements requisite for multi-reaction detection schemes. Nucleic acid targets are often present in low copy numbers and as minor constituents in complex samples. Therefore, detection of these targets often requires amplification prior to detection.

However, amplification and detection reagents often comprise stringent condition requirements that are not cross-compatible, necessitating buffer exchange and sample transfer steps that can increase time and user input requirements and result in contamination and sample volume loss.

WO 2020/028729 A1 describes programmable nuclease compositions and methods of use thereof.

WO 2018/107129 A1 describes CRISPR effector system-based diagnostics.

WO 2021/252836 A1 describes CRISPR-based SARS-CoV-2 detection.

WO 2021/243308 A1 describes a programmable nuclease diagnostic device.

### SUMMARY

The invention is defined by appended claims 1, 14, 15, and 16. Various embodiments are defined in the appended dependent claims.

Described herein, in certain embodiments, is a system for detecting a target nucleic acid, comprising a buffer comprising: (i) reagents for an amplification reaction targeting the target nucleic acid; and (ii) reagents for a DETECTR reaction targeting the target nucleic acid, comprising a programmable nuclease, a non-naturally occurring guide nucleic acid, and a reporter, wherein the non-naturally occurring guide nucleic acid comprises a sequence that hybridizes to a segment of the target nucleic acid, wherein at least 1 nM of the reporter undergoes transcollateral cleavage within one hour of addition of at least 5000 copies of the target nucleic acid to the system. In some examples, at least 5 nM of the reporter undergoes transcollateral cleavage within one hour of addition of at least 5000 copies of the target nucleic acid to the system. In some examples, at least 10 nM of the reporter undergoes transcollateral cleavage within one hour of addition of at least 5000 copies of the target nucleic acid to the system. In some embodiments, at least 1 nM of the reporter undergoes transcollateral cleavage within one hour of addition of at least 1000 copies of the target nucleic acid to the system. In some embodiments, at least 5 nM of the reporter undergoes transcollateral cleavage within one hour of addition of at least 100 copies of the target nucleic acid to the system. In some embodiments, the time to completion for the amplification and DETECTR reactions is each less than 45 minutes when performed in the buffer. In some embodiments, the amplification and DETECTR reagents have half-lives of greater than 1 week at room temperature. In some embodiments, the activities of the amplification reagents and the DETECTR reagents diminishes by less than 10% following a freeze-thaw cycle. In some embodiments, the reagents for the amplification reaction comprise reagents for thermal cycling amplification. In some embodiments, the reagents for the amplification reaction comprise reagents for isothermal amplification. In some embodiments, the reagents for the amplification reaction comprise reagents for transcription mediated amplification (TMA), helicase dependent amplification (HDA), circular helicase dependent amplification (cHDA), strand displacement amplification (SDA), loop mediated amplification (LAMP), exponential amplification reaction (EXPAR), rolling circle amplification (RCA), ligase chain reaction (LCR), simple method amplifying RNA targets (SMART), single primer isothermal amplification (SPIA), multiple displacement amplification (MDA), nucleic acid sequence based amplification (NASBA), hinge-initiated primer-dependent amplification of nucleic acids (HIP), nicking enzyme amplification reaction (NEAR), or improved multiple displacement amplification (IMDA). In some embodiments, the reagents for the amplification reaction comprise reagents for loop mediated amplification (LAMP). In some embodiments, the system further comprises an activator for the amplification reaction. In some embodiments, the activator for the amplification reaction comprises a magnesium or calcium salt. In some embodiments, the programmable nuclease comprises at least 60% sequence identity to SEQ ID NO: 18-170 or 221-268. In some embodiments, the programmable nuclease comprises a RuvC catalytic domain. In some embodiments, the programmable nuclease is a type V CRISPR/Cas effector protein. In some embodiments, the type V CRISPR/Cas effector protein is a Cas12 protein. In some embodiments, the Cas12 protein comprises a Cas12a polypeptide, a Cas12b polypeptide, a Cas12c polypeptide, a Cas12d polypeptide, a Cas12e polypeptide, a C2c4 polypeptide, a C2c8 polypeptide, a C2c5 polypeptide, a C2c10 polypeptide, and a C2c9 polypeptide. In some embodiments, the Cas12 protein has at least 80%, at least 85%, at least 90%, at least 92%, at least 95%, at least 97%, or at least 99% sequence identity to any one of SEQ ID NO: 18 - SEQ ID NO: 60. In some embodiments, the Cas12 protein is selected from SEQ ID NO: 18 - SEQ ID NO: 60. In some embodiments, the type V CRISPR/Cas effector protein is a Cas14 protein. In some embodiments, the Cas14 protein comprises a Cas14a polypeptide, a Cas14b polypeptide, a Cas14c polypeptide, a Cas14d polypeptide, a Cas14e polypeptide, a Cas14f polypeptide, a Cas14g polypeptide, a Cas14h polypeptide, a Cas14i polypeptide, a Cas14j polypeptide, or a Cas14k polypeptide. In some embodiments, the Cas14 protein has at least 80%, at least 85%, at least 90%, at least 92%, at least 95%, at least 97%, or at least 99% sequence identity to any one of SEQ ID NO: 61 - SEQ ID NO: 152. In some embodiments, the Cas14 protein is selected from SEQ ID NO: 61 - SEQ ID NO: 152. In some embodiments, the type V CRISPR/Cas effector protein is a CasΦ protein. In some embodiments, the CasΦ protein has at least 80%, at least 85%, at least 90%, at least 92%, at least 95%, at least 97%, or at least 99% sequence identity to any one of SEQ ID NO: 221 - SEQ ID NO: 268. In some embodiments, the CasΦ protein is selected from SEQ ID NO: 221 - SEQ ID NO: 268. In some embodiments, the system further comprises a reverse transcriptase, an oligonucleotide primer, and dNTPs for reverse transcribing the target nucleic acid. In some embodiments, the buffer is a lysis buffer. In some embodiments, the viscosity of the buffer is at least 5 centipoise (cP). In some embodiments, the buffer comprises a pH of 7.5 to 8.5, at least 10 mM of a buffering agent, at least 1 mM ammonium acetate, at least 10 mM potassium acetate, at least 2.5 mM magnesium acetate, and at least 0.5% glycerol. In some embodiments, the buffer comprises a pH of 7.7 to 8.3. In some embodiments, the buffer comprises a pH of 7.85 to 8.15. In some embodiments, the buffering agent comprises HEPES, imidazole, TRIS-HCl, or phosphate. In some embodiments, the buffer further comprises at least 0.05% by volume of a detergent. In some embodiments, the detergent comprises Tween 20. In some embodiments, the buffer comprises a pH of 7.5 to 8.5, at least 5 mM of a buffering agent, at least 20 mM potassium acetate, at least 2.5 mM magnesium acetate, and at least 0.5% glycerol. In some embodiments, the buffer comprises a pH of 7.7 to 8.3. In some embodiments, the buffer comprises a pH of 7.85 to 8.15. In some embodiments, the buffering agent comprises phosphate or TRIS-HCl. In some embodiments, the buffer comprises at least 1 mM ammonium sulfate. In some embodiments, the buffer comprises at least 0.05% by volume of a detergent. In some embodiments, the detergent comprises Tween 20. In some embodiments, the buffer comprises a pH of 7.25 to 8.75, at least 5 mM of a buffering agent, at least 7.5 mM potassium acetate, at least 1 mM magnesium acetate, and at least 0.5% glycerol. In some embodiments, the buffering agent comprises phosphate. In some embodiments, the buffer comprises a pH of 7.5 to 8.5. In some embodiments, the buffer comprises a pH of 7.75 to 8.25. In some embodiments, the buffer further comprises at least 1 mM ammonium sulfate. In some embodiments, the buffer further comprises at least 0.05% by volume of a detergent. In some embodiments, the detergent comprises Tween 20.

The invention provides a system for detecting a target nucleic acid, comprising a buffer comprising: (i) amplification reagents for an amplification reaction targeting the target nucleic acid; and (ii) detection reagents for a detection reaction targeting the target nucleic acid; wherein the amplification reagents comprise one or more oligonucleotide primers, and a DNA polymerase; wherein the detection reagents comprise a programmable nuclease, a non-naturally occurring guide nucleic acid, and reporters; wherein the non-naturally occurring guide nucleic acid comprises a sequence that hybridizes to a segment of the target nucleic acid or DNA amplicons thereof; wherein the amplification reagents are present in amounts effective to amplify the target nucleic acid in a test sample to produce DNA amplicons of the target nucleic acid; wherein the programmable nuclease and non-naturally occurring guide nucleic acid form a complex in the buffer that is activated upon binding one of the DNA amplicons to induce detectable transcollateral cleavage of the reporters; and wherein the buffer is a lysis buffer. In some embodiments, (a) at least 1 nM of the reporters undergo transcollateral cleavage within one hour of addition of at least 5000 copies of the target nucleic acid to the system; (b) at least 5 nM of the reporters undergo transcollateral cleavage within one hour of addition of at least 5000 copies of the target nucleic acid to the system; (c) at least 10 nM of the reporters undergo transcollateral cleavage within one hour of addition of at least 5000 copies of the target nucleic acid to the system; (d) at least 1 nM of the reporters undergo transcollateral cleavage within one hour of addition of at least 1000 copies of the target nucleic acid to the system; or (e) at least 1 nM of the reporters undergo transcollateral cleavage within one hour of addition of at least 1000 copies of the target nucleic acid to the system. In some embodiments, the amplification reagents and detection reagents are present in amounts effective to produce a detectable signal in less than 45 minutes in the presence of the target nucleic acid. In some embodiments, the amplification and detection reagents have half-lives of greater than 1 week at room temperature. In some embodiments, the activities of the amplification reagents and the detection reagents diminishes by less than 10% following a freeze-thaw cycle. In some embodiments, the amplification reagents comprise reagents for isothermal amplification. In some embodiments, the amplification reagents comprise reagents for transcription mediated amplification (TMA), helicase dependent amplification (HDA), circular helicase dependent amplification (cHDA), strand displacement amplification (SDA), loop mediated amplification (LAMP), exponential amplification reaction (EXPAR), rolling circle amplification (RCA), ligase chain reaction (LCR), simple method amplifying RNA targets (SMART), single primer isothermal amplification (SPIA), multiple displacement amplification (MDA), nucleic acid sequence based amplification (NASBA), hinge-initiated primer-dependent amplification of nucleic acids (HIP), nicking enzyme amplification reaction (NEAR), or improved multiple displacement amplification (IMDA). In some embodiments, the amplification reagents comprise reagents for loop mediated amplification (LAMP), and further wherein (a) the one or more primers comprise a first primer and a second primer targeted to the target nucleic acid; (b) the first primer comprises a 5' region that is complementary to a sequence generated by extension of the first primer; (c) the second primer comprises a 5' region that is complementary to a sequence generated by extension of the second primer; and (d) the DNA polymerase is a strand-displacing DNA polymerase. In some embodiments, the system further comprises an activator for the amplification reaction, such as a magnesium or calcium salt. In some embodiments, the programmable nuclease comprises at least 60% sequence identity to SEQ ID NO: 18-170 or 221-268. In some embodiments, the programmable nuclease comprises an RuvC catalytic domain. In some embodiments, the programmable nuclease is a type V CRISPR/Cas effector protein. In some embodiments, the type V CRISPR/Cas effector protein is a Cas12 protein, such as (a) a Cas12a polypeptide, a Cas12b polypeptide, a Cas12c polypeptide, a Cas12d polypeptide, a Cas12e polypeptide, a C2c4 polypeptide, a C2c8 polypeptide, a C2c5 polypeptide, a C2c10 polypeptide, and a C2c9 polypeptide; (b) a protein that has at least 80%, at least 85%, at least 90%, at least 92%, at least 95%, at least 97%, or at least 99% sequence identity to any one of SEQ ID NO: 18 - SEQ ID NO: 60; or (c) a protein having a sequence selected from SEQ ID NO: 18 - SEQ ID NO: 60. In some embodiments, the type V CRISPR/Cas effector protein is a Cas14 protein, such as (a) a Cas14a polypeptide, a Cas14b polypeptide, a Cas14c polypeptide, a Cas14d polypeptide, a Cas14e polypeptide, a Cas 14f polypeptide, a Cas14g polypeptide, a Cas14h polypeptide, a Cas14i polypeptide, a Cas14j polypeptide, or a Cas14k polypeptide; (b) a protein that has at least 80%, at least 85%, at least 90%, at least 92%, at least 95%, at least 97%, or at least 99% sequence identity to any one of SEQ ID NO: 61 - SEQ ID NO: 152; or (c) a protein having a sequence selected from SEQ ID NO: 61 - SEQ ID NO: 152. In some embodiments, the type V CRISPR/Cas effector protein is a CasΦ protein, such as (a) a protein that has at least 80%, at least 85%, at least 90%, at least 92%, at least 95%, at least 97%, or at least 99% sequence identity to any one of SEQ ID NO: 221 - SEQ ID NO: 268; or (b) a protein having a sequence selected from SEQ ID NO: 221 - SEQ ID NO: 268. In some embodiments, the system further comprises a reverse transcriptase, an oligonucleotide primer, and dNTPs for reverse transcribing the target nucleic acid. The buffer is a lysis buffer. In some embodiments, the viscosity of the buffer is at least 5 centipoise (cP). In some embodiments, the buffer comprises a pH of 7.5 to 8.5, at least 10 mM of a buffering agent, at least 1 mM ammonium acetate, at least 10 mM potassium acetate, at least 2.5 mM magnesium acetate, and at least 0.5% glycerol; and optionally: (a) the buffer comprises a pH of 7.7 to 8.3, or a pH of 7.85 to 8.15; (b) the buffering agent comprises HEPES, imidazole, TRIS-HCl, or phosphate; and/or (c) the buffer further comprises at least 0.05% by volume of a detergent, optionally wherein the detergent comprises Tween 20. In some embodiments, the buffer comprises a pH of 7.5 to 8.5, at least 5 mM of a buffering agent, at least 20 mM potassium acetate, at least 2.5 mM magnesium acetate, and at least 0.5% glycerol; and optionally: (a) the buffer comprises a pH of 7.7 to 8.3, or a pH of 7.85 to 8.15; (b) the buffering agent comprises phosphate or TRIS-HCl; (c) the buffer comprises at least 1 mM ammonium sulfate; and/or (d) the buffer comprises at least 0.05% by volume of a detergent, optionally wherein the detergent comprises Tween 20. In some embodiments, the buffer comprises a pH of 7.25 to 8.75, at least 5 mM of a buffering agent, at least 7.5 mM potassium acetate, at least 1 mM magnesium acetate, and at least 0.5% glycerol; and optionally: (a) the buffering agent comprises phosphate; (b) the buffer comprises a pH of 7.5 to 8.5, or a pH of 7.75 to 8.25; (c) the buffer further comprises at least 1 mM ammonium sulfate; and/or (d) the buffer further comprises at least 0.05% by volume of a detergent, optionally wherein the detergent comprises Tween 20.

In some embodiments, the system further comprises a circular template with internal complementarity formed from a single polynucleotide strand, wherein: (a) the circular template comprises a first portion with complementarity to one of the one or more oligonucleotide primers and a second portion with complementarity to a portion of the target nucleic acid; (b) the internal complementarity comprises part of the first portion and part of the second portion; (c) the second portion has a total length that is longer than a combined length of the first portion and second portion that are within the internal complementarity; and (d) the circular template undergoes a conformational change upon hybridization to the target nucleic acid to expose the first portion to hybridization to the oligonucleotide primer. In some embodiments, the system further comprises a circular template, wherein: (a) the circular template comprises a first portion with complementarity to one of the one or more oligonucleotide primers and a second portion with complementarity to the target nucleic acid; (b) the oligonucleotide primer complementary to the first portion comprises a blocking motif at its 3' end; and (c) the oligonucleotide primer complementary to the first portion undergoes cleavage to remove the blocking motif by the programmable nuclease in the presence of the target nucleic acid.

In some embodiments, the system further comprises a polymer matrix, wherein the polymer matrix is complexed with the reporters. In some embodiments, the polymer matrix is formed from copolymerization of at least a first plurality of monomers with the reporters. In some embodiments, the polymer matrix comprises a hydrogel.

In some embodiments, the buffer of system further comprises: (a) one or more of betaine monohydrate, acetamide, GABA, L-proline, beta-alanine, 6-aminohexanoic acid, urea, methylurea, ethylurea, hypotaurine, NDSB-256, and ammonium acetate; (b) one or more of trehalose, xylitol, D-sorbitol, sucrose, and trimethylamine N-oxide dihydrate; and/or (c) trimethylamine N-oxide dihydrate.

The invention provides a system for detecting a target nucleic acid, comprising reagents in a buffer, wherein (a) the reagents comprise hairpin polynucleotides, programmable nucleases, non-naturally occurring guide nucleic acids, and reporters; (b) each hairpin polynucleotide comprises one or more RNA loops, a first portion comprising DNA, and a second portion joined to the first portion by one of the one or more RNA loops; (c) each non-naturally occurring guide nucleic acid comprises a sequence that hybridizes to a segment of the target nucleic acid; (d) in each hairpin polynucleotide, the second portion of the hairpin polynucleotide hybridizes to a segment of the first portion; (e) the programmable nucleases and non-naturally occurring guide nucleic acids form complexes in the buffer that are activated upon binding the target nucleic acid; (f) an activated programmable nuclease is effective to induce (i) transcollateral cleavage of the one or more RNA loops, and (ii) detectable transcollateral cleavage of the reporters; and (g) cleavage of the one or more RNA loops of one of the hairpin polynucleotides is effective to release the first portion of the hairpin polynucleotide to hybridize with one of the non-naturally occurring guide nucleic acids and form a further activated programmable nuclease. In some embodiments, the one or more RNA loops comprise a first RNA loop joining the first portion and the second portion, and a second RNA loop joining the first portion and a third portion; wherein the third portion hybridizes to a different segment of the first portion. In some embodiments, the second portion comprises RNA, DNA, or both.

In some embodiments described herein is a method of assaying for a target nucleic acid in a sample, the method comprising: (a) amplifying a portion of the target nucleic acid; (b) performing a DETECTR reaction targeting the target nucleic acid, comprising contacting the target nucleic acid with a programmable nuclease, a non-naturally occurring guide nucleic acid that hybridizes to a segment of the target nucleic acid, and a reporter, wherein the contacting the sample to reagents for amplifying and the contacting the sample to reagents for the DETECTR reaction are performed in the same reaction volume; and (c) assaying for a change in a signal, wherein the change in the signal is produced by cleavage of the reporter. In some embodiments, the amplifying and the DETECTR reaction occur simultaneously. In some embodiments, the assaying comprises measuring the rate of the change in the signal. In some embodiments, the change in the signal identifies a concentration of the target nucleic acid in the sample. In some embodiments, the amplifying comprises thermal cycling amplification. In some embodiments, the amplifying comprises isothermal amplification. In some embodiments, the amplifying comprises transcription mediated amplification (TMA), helicase dependent amplification (HDA), circular helicase dependent amplification (cHDA), strand displacement amplification (SDA), loop mediated amplification (LAMP), exponential amplification reaction (EXPAR), rolling circle amplification (RCA), ligase chain reaction (LCR), simple method amplifying RNA targets (SMART), single primer isothermal amplification (SPIA), multiple displacement amplification (MDA), nucleic acid sequence based amplification (NASBA), hinge-initiated primer-dependent amplification of nucleic acids (HIP), nicking enzyme amplification reaction (NEAR), or improved multiple displacement amplification (IMDA). In some embodiments, the amplifying comprises loop mediated amplification (LAMP). In some embodiments, the amplifying comprises providing a plurality of primers that target different portions of the target nucleic acid. In some embodiments, the reagents for amplification comprise a forward inner primer (FIP) primer, a backward inner primer (BIP) primer, a forward loop primer (LF) primer, and a backward loop primer (LB primer). In some embodiments, the method comprises reverse transcribing the target nucleic acid. In some embodiments, the reverse transcribing comprises contacting the sample to reagents for reverse transcription, and wherein the reagents for reverse transcription comprise a reverse transcriptase, an oligonucleotide primer, and dNTPs. In some embodiments, the programmable nuclease comprises a RuvC catalytic domain. In some embodiments, the programmable nuclease has at least 80%, at least 85%, at least 90%, at least 92%, at least 95%, at least 97%, or at least 99% sequence identity to any one of SEQ ID NO: 397 - SEQ ID NO: 423; or is selected from SEQ ID NO: 397- SEQ ID NO: 423.

In some embodiments, the programmable nuclease is a type V CRISPR/Cas effector protein. In some embodiments, the type V CRISPR/Cas effector protein is a Cas12 protein. In some embodiments, the Cas12 protein comprises a Cas12a polypeptide, a Cas12b polypeptide, a Cas12c polypeptide, a Cas12d polypeptide, a Cas12e polypeptide, a C2c4 polypeptide, a C2c8 polypeptide, a C2c5 polypeptide, a C2c10 polypeptide, and a C2c9 polypeptide. In some embodiments, the Cas12 protein has at least 80%, at least 85%, at least 90%, at least 92%, at least 95%, at least 97%, or at least 99% sequence identity to any one of SEQ ID NO: 18 - SEQ ID NO: 60. In some embodiments, the Cas12 protein is selected from SEQ ID NO: 18 - SEQ ID NO: 60. In some embodiments, the type V CRISPR/Cas effector protein is a Cas14 protein. In some embodiments, the Cas14 protein comprises a Cas14a polypeptide, a Cas14b polypeptide, a Cas14c polypeptide, a Cas14d polypeptide, a Cas14e polypeptide, a Cas14f polypeptide, a Cas14g polypeptide, a Cas14h polypeptide, a Cas14i polypeptide, a Cas14j polypeptide, or a Cas14k polypeptide. In some embodiments, the Cas14 protein has at least 80%, at least 85%, at least 90%, at least 92%, at least 95%, at least 97%, or at least 99% sequence identity to any one of SEQ ID NO: 61 - SEQ ID NO: 152. In some embodiments, the Cas14 protein is selected from SEQ ID NO: 61 - SEQ ID NO: 152. In some embodiments, the type V CRISPR/Cas effector protein is a CasΦ protein. In some embodiments, the CasΦ protein has at least 80%, at least 85%, at least 90%, at least 92%, at least 95%, at least 97%, or at least 99% sequence identity to any one of SEQ ID NO: 221 - SEQ ID NO: 268. In some embodiments, the CasΦ protein is selected from SEQ ID NO: 221 - SEQ ID NO: 268. In some embodiments, the method further comprises lysing a cell or virus comprising the target nucleic acid. In some embodiments, the lysing is performed in the same reaction volume as the amplifying and the DETECTR reaction. In some embodiments, the change in the signal comprises a change in a calorimetric, potentiometric, amperometric, or piezo-electric signal. In some embodiments, the change in the signal comprises a change in a colorimetric signal (e.g., an increase in intensity, a decrease in intensity, a change in color, etc.). In some embodiments, the change in the signal comprises a fluorescence signal (e.g., an increase in intensity, a decrease in intensity, a change in phase or wavelength, etc.). In some embodiments, the assaying comprises detecting the change in a signal with a smartphone. In some embodiments, the reaction volume comprises a viscosity of at least 5 cP. In some embodiments, the reaction volume comprises a total dissolved solids concentration of at least 200 mM.

The invention also provides a method of assaying for a target nucleic acid in a sample, the method comprising: (a) amplifying a portion of the target nucleic acid with a DNA polymerase to produce DNA amplicons of the target nucleic acid; (b) forming a complex comprising one of the DNA amplicons, a programmable nuclease, and a non-naturally occurring guide nucleic acid that hybridizes to a segment of the DNA amplicon, thereby activating the programmable nuclease; (c) cleaving reporters with the activated programmable nuclease; and (d) detecting a change in a signal, wherein the change in the signal is produced by cleavage of the reporters; wherein the target nucleic acid and reagents for the amplifying and cleaving are present in the same reaction volume; and wherein the method further comprises lysing a cell or virus comprising the target nucleic acid and wherein the lysing is performed in the same reaction volume as the amplifying and the cleaving. In some embodiments, the amplifying and the cleaving occur simultaneously. In some embodiments, the method further comprises measuring the rate of the change in the signal. In some embodiments, the method further comprises measuring a concentration of the target nucleic acid in the sample based on the change in the signal. In some embodiments, the amplifying comprises isothermal amplification. In some embodiments, the amplifying comprises transcription mediated amplification (TMA), helicase dependent amplification (HDA), circular helicase dependent amplification (cHDA), strand displacement amplification (SDA), loop mediated amplification (LAMP), exponential amplification reaction (EXPAR), rolling circle amplification (RCA), ligase chain reaction (LCR), simple method amplifying RNA targets (SMART), single primer isothermal amplification (SPIA), multiple displacement amplification (MDA), nucleic acid sequence based amplification (NASBA), hinge-initiated primer-dependent amplification of nucleic acids (HIP), nicking enzyme amplification reaction (NEAR), or improved multiple displacement amplification (IMDA). In some embodiments, the amplifying comprises loop mediated amplification (LAMP), wherein the LAMP comprises amplification with a first primer and a second primer targeted to the target nucleic acid and a strand-displacing polymerase, wherein the first primer comprises a 5' region that is complementary to a sequence generated by extension of the first primer, and wherein the second primer comprises a 5' region that is complementary to a sequence generated by extension of the second primer. In some embodiments, the amplifying comprises providing a plurality of primers that target different portions of the target nucleic acid. In some embodiments, reagents for the amplification comprise a FIP primer, a BIP primer, a LF primer, and a LB primer. In some embodiments, the amplifying comprises reverse transcribing the target nucleic acid. In some embodiments, the programmable nuclease comprises a RuvC catalytic domain. In some embodiments, the programmable nuclease has at least 80%, at least 85%, at least 90%, at least 92%, at least 95%, at least 97%, or at least 99% sequence identity to any one of SEQ ID NO: 397 - SEQ ID NO: 423; or is selected from SEQ ID NO: 397- SEQ ID NO: 423.

In some embodiments, the programmable nuclease is a type V CRISPR/Cas effector protein. In some embodiments, the type V CRISPR/Cas effector protein is a Cas12 protein, such as (a) a Cas12a polypeptide, a Cas12b polypeptide, a Cas12c polypeptide, a Cas12d polypeptide, a Cas12e polypeptide, a C2c4 polypeptide, a C2c8 polypeptide, a C2c5 polypeptide, a C2c10 polypeptide, and a C2c9 polypeptide; (b) a protein that has at least 80%, at least 85%, at least 90%, at least 92%, at least 95%, at least 97%, or at least 99% sequence identity to any one of SEQ ID NO: 18 - SEQ ID NO: 60; or (c) a protein having a sequence selected from SEQ ID NO: 18 - SEQ ID NO: 60. In some embodiments, the type V CRISPR/Cas effector protein is a Cas14 protein, such as (a) a Cas14a polypeptide, a Cas14b polypeptide, a Cas14c polypeptide, a Cas14d polypeptide, a Cas14e polypeptide, a Cas14f polypeptide, a Cas14g polypeptide, a Cas14h polypeptide, a Cas14i polypeptide, a Cas14j polypeptide, or a Cas14k polypeptide; (b) a protein that has at least 80%, at least 85%, at least 90%, at least 92%, at least 95%, at least 97%, or at least 99% sequence identity to any one of SEQ ID NO: 61 - SEQ ID NO: 152; or (c) a protein having a sequence selected from SEQ ID NO: 61 - SEQ ID NO: 152. In some embodiments, the type V CRISPR/Cas effector protein is a CasΦ protein, such as (a) a protein that has at least 80%, at least 85%, at least 90%, at least 92%, at least 95%, at least 97%, or at least 99% sequence identity to any one of SEQ ID NO: 221 - SEQ ID NO: 268; or (b) a protein having a sequence selected from SEQ ID NO: 221 - SEQ ID NO: 268. The method further comprises lysing a cell or virus comprising the target nucleic acid. The lysing is performed in the same reaction volume as the amplifying and the cleaving. In some embodiments, the change in the signal comprises a change in a calorimetric, potentiometric, amperometric, or piezo-electric signal. In some embodiments, the change in the signal comprises a change in a colorimetric signal. In some embodiments, the change in the signal comprises a fluorescence signal. In some embodiments, the assaying comprises detecting the change in a signal with a smartphone. In some embodiments, the reaction volume comprises a viscosity of at least 5 cP. In some embodiments, the reaction volume comprises a total dissolved solids concentration of at least 200 mM. In some embodiments, the system further comprises Thermostable inorganic pyrophosphatase (TIPP). In some embodiments, the method further comprises signal enhancement via hydrolysis of inorganic pyrophosphates, such as by enzymatic hydrolysis. In some embodiments, the amplification and the cleaving are carried out in the presence of TIPP, and wherein the signal is enhanced by the hydrolysis of inorganic pyrophosphates by TIPP, as compared to the same reaction carried out without TIPP. In some embodiments, the reaction volume further comprises Thermostable inorganic pyrophosphatase (TIPP).

In some embodiments, the amplifying comprises amplification of a circular template with an oligonucleotide primer, and further wherein: (a) the circular template comprises a single polynucleotide strand having internal complementarity; (b) the circular template comprises a first portion with complementarity to the primer and a second portion with complementarity to a portion of the target nucleic acid; (c) the internal complementarity comprises part of the first portion and part of the second portion; (d) the second portion has a total length that is longer than a combined length of the first portion and second portion that are within the internal complementarity; (e) the circular template undergoes a conformational change upon hybridization to the target nucleic acid to expose the first portion to hybridization to the oligonucleotide primer; and (f) extension of the oligonucleotide primer along the circular template produces the DNA amplicons. In some embodiments, the amplifying comprises amplification of a circular template with an oligonucleotide primer, and further wherein: (a) the circular template comprises a first portion with complementarity to the oligonucleotide primer and a second portion with complementarity to the target nucleic acid; (b) the oligonucleotide primer complementary to the first portion comprises a blocking motif at its 3' end; (c) the oligonucleotide primer undergoes cleavage to remove the blocking motif by the programmable nuclease in the presence of the target nucleic acid; and (d) extension of the oligonucleotide primer along the circular template produces the DNA amplicons. In some embodiments, the amplification is isothermal. In some embodiments, the DNA polymerase is a strand-displacing polymerase.

In some embodiments of the methods, the reporters are complexed with a polymer matrix. In some embodiments, the polymer matrix is formed from copolymerization of a plurality of monomers with the reporters. In some embodiments, the polymer matrix comprises a hydrogel. In some embodiments, (i) the step of cleaving the reporters releases detectable moieties from the polymer matrix, and (ii) the step of detecting comprises capturing and detecting the released detectable moieties at a capture region of a support medium.

In some embodiments, said same reaction volume comprises one or more additives comprising: (a) one or more of betaine monohydrate, acetamide, GABA, L-proline, beta-alanine, 6-aminohexanoic acid, urea, methylurea, ethylurea, hypotaurine, NDSB-256, and ammonium acetate; (b) one or more of trehalose, xylitol, D-sorbitol, sucrose, and trimethylamine N-oxide dihydrate; and/or (c) trimethylamine N-oxide dihydrate.

The invention also provides a method of assaying for a target nucleic acid in a sample, the method comprising the following steps in a single reaction volume: (a) forming a complex comprising the target nucleic acid, a first programmable nuclease, and a first non-naturally occurring guide nucleic acid that hybridizes to a segment of the target nucleic acid, thereby activating the programmable nuclease; (b) cleaving a hairpin polynucleotide of a plurality of hairpin polynucleotides with the activated programmable nuclease, wherein each hairpin polynucleotide comprises (i) one or more RNA loops that are cleaved, (i) a first portion comprising DNA, and (iii) a second portion joined to the first portion by one of the one or more RNA loops, wherein the second portion is hybridized to a segment of the first portion; (c) forming a second complex comprising the first portion of the cleaved hairpin polynucleotide, a second programmable nuclease, and a second non-naturally occurring guide nucleic acid that hybridizes to the first portion of the cleaved hairpin, thereby activating the second programmable nuclease; (d) cleaving reporters with the activated first or second programmable nuclease; and (e) detecting a change in a signal, wherein the change in the signal is produced by cleavage of the reporters. In some embodiments, the first programmable nuclease and the second programmable nuclease are the same, such as any of the programmable nucleases disclosed herein. In some embodiments, the one or more RNA loops comprise a first RNA loop joining the first portion and the second portion, and a second RNA loop joining the first portion and a third portion; wherein the third portion hybridizes to a different segment of the first portion. In some embodiments, the second portion comprises RNA, DNA, or both.

### BRIEF DESCRIPTION OF THE DRAWINGS

The patent or application file contains at least one drawing executed in color. Copies of this patent or patent application publication with color drawing(s) will be provided by the Office upon request and payment of the necessary fee.

The novel features of the disclosure are set forth with particularity in the appended claims. A better understanding of the features and advantages of the present invention will be obtained by reference to the following detailed description that sets forth illustrative embodiments, in which the principles of the invention are utilized, and the accompanying drawings (also "figure" and **"FIG."** herein), of which:
**FIG. 1** shows the mean time to result for RT-LAMP reactions performed in a variety of buffers.
**FIG. 2** provides summary effect sizes for different buffer constituents on the mean time to result for RT-LAMP reactions. The values on the right of the table provides the statistical significance for each variable or set of variables on the time to result relative to a commercial buffer control.
**FIG. 3** provides a graphical representation of measured effect sizes for various buffer components on RT-LAMP reaction times. The final column shows the weighting function used to generate the bottom ('Desirability') row of the chart, which shows whether a variable correlates positively or negatively with RT-LAMP reaction time.
**FIG. 4** shows the mean reaction times for DETECTR reactions performed with the buffers listed in TABLE 2, SEQ ID NO: 28 targeting SARS-CoV-2 N-gene, and two separate dilutions of SARS-CoV N-gene LAMP amplicons ('Dil 1' & 'Dil 2').
**FIG. 5** provides effect summary sizes for the buffer constituents in the DETECTR assay summarized in **FIG. 4****.**
**FIG. 6** provides a graphical representations of measured effect sizes for various buffer components on the DETECTR reaction times shown in **FIG. 4****.**
**FIG. 7A - 7C** graphically depicts RT-LAMP, DETECTR reaction times, and total reaction times (RT-LAMP + DETECTR reaction times) performed in a variety of buffers.
**FIG. 8** shows the mean reaction times for a variety of RT-LAMP assays performed in different buffers over seven (Dil-1 through Dil-7) replicates. NTC denotes controls lacking the target nucleic acid.
**FIG. 9** displays combined RT-LAMP and DETECTR assay times for 4 different buffer formulations.
**FIG. 10** provides the rates of RT-LAMP reactions performed in 10 different buffers.
**FIG. 11** provides combined RT-LAMP and DETECTR assay times for 4 different buffer systems and 3 different initial target nucleic acid copy numbers.
**FIG. 12A - 12B** panel A displays reaction times for 4 different buffer systems and 3 different initial copy numbers for the target nucleic acid. Panel A provides reaction times for RT-LAMP reactions, while panel B provides reaction times for DETECTR assays.
**FIG. 13A - 13B** shows the effect size and statistical significance of different buffer parameters on the rates of RT-LAMP reactions. The far right column shows the weighting function used to generate the bottom ('Desirability') row of the chart, which depicts whether a variable correlates positively or negatively with RT-LAMP reaction time.
**FIG. 14A - 14B** shows the effect size and statistical significance of buffer parameters on the rates of DETECTR reactions. The far right column shows the weighting function used to generate the bottom ('Desirability') row of the chart, which depicts whether a variable correlates positively or negatively with RT-LAMP reaction time.
**FIG. 15A** presents results for RT-LAMP amplification with Cas14a DETECTR in single reaction volume (one-pot).
**FIG. 15B** shows a DETECTR assay screening buffers and alternative polymerases that enable RT-LAMP to work at lower temperatures (normally at 65C).
**FIG. 16** shows the layout of the LAMP plate used in an RT-LAMP assay screening buffers and alternative polymerases that enable RT-LAMP to work at lower temperatures (normally at 65C).
**FIGs. 17A-17B** show assays evaluating the performance of Cas variants on a putative target site in SARS-CoV-2.
**FIG. 18** shows a strategy to simply DETECTR workflow for CRISPR diagnostics into a one-pot assay.
**FIGs. 19A-19B** present results for HotPot involving LAMP amplification with Cas14a DETECTR in single reaction volume (one-pot).
**FIG. 20** presents results for identifying buffers that are compatible with Cas14a and low temperature RT-LAMP (LowLAMP).
**FIG. 21** presents results involving the impact of individual components on the performance of Cas14 at low temperature RT-LAMP conditions.
**FIG. 22** presents results for LAMP amplification with Cas 14a DETECTR in single reaction volume (one-pot).
**FIGs. 23A-23B** presents results for one-pot Cas14 with LowLAMP at 50°C.
**FIG. 24** presents results for one-pot Cas14 with Bsm DNA polymerase at 55°C.
**FIG. 25** presents results for a limit of detection study involving one-pot DETECTR (HotPot).
**FIG. 26** presents results for a limit of detection study involving one-pot DETECTR (HotPot), where two different DNA polymerases at 55C were tested.
**FIG. 27** shows the results of using thermostable tracrRNA in the HotPot assays.
**FIG. 28** shows the results of using sgRNA in the HotPot assays.
**FIG. 29** presents results for a study involving replacing Bst polymerase in the NEAR assay, showing enablement for SARS-CoV-2 detection at lower temperatures. **FIG. 30** presents results for NEAR assay amplification functions in Cas14a optimal buffers.
**FIG. 31** presents results for Cas14a functions in a range of KOAc salt concentrations.
**FIG. 32** presents results for a study involving increasing concentrations of KOAc to improve NEAR performance in Cas14a optimal buffers.
**FIG. 33** presents results for a study involving increasing concentrations of KOAc to improve NEAR performance in Cas14a optimal buffers.
**FIG. 34A - 34B** present sequences and results for performance of Cas 14a.1 crRNAs on SARS-CoV-2 E-gene amplicon, respectively.
**FIG. 35** presents results for the evaluation of the performance of Klenow(exo-) NEAR assay in IB13 buffer at decreasing salt concentrations.
**FIG. 36** presents an overview of rolling circle amplification (sRCA)
**FIG.** 37 presents results from screening dumbbell DNA templates for sRCA.
**FIG. 38** presents results from a study involving the ability of Cas14a to detect product of RCA reaction across increasing temperatures.
**FIG. 39** presents results from a study involving the effects of trigger oligos.
**FIG. 40** presents results from a study involving a titration of trigger oligos for Cas14 one-pot sRCA.
**FIG. 41** presents results from evaluating of a Cas12 variant (SEQ ID NO: 28) in one-pot sRCA.
**FIG. 42** presents an overview of RCA positive feedback for Cas13.
**FIG. 43** presents results from evaluating Cas13-compatible DNA templates for RCA.
**FIG. 44** presents results from a study evaluating whether a Cas13-compatible DNA template is functional in RCA.
**FIG. 45** presents results from a study involving Cas13 functionality in a one-pot sRCA reaction across increasing temperatures.
**FIG. 46** presents an overview of CasPin.
**FIG. 47** presents potential hairpin structures for CasPin.
**FIG. 48** presents results for an initial design using two hairpins.
**FIG. 49** presents a schematic of combined gRNA and reporter immobilization on the left and results for immobilization of DETECTR components using NHS-Amine chemistries on the right.
**FIG. 50** presents results from optimizing the conjugation buffer to reduce non-specific binding.
**FIG. 51** presents results from a study involving immobilizing different combinations of reporter + guide + a Cas12 variant (SEQ ID NO: 28).
**FIG. 52** presents results from a study optimizing gRNA and target concentrations to improve signal-to-noise ratio for immobilized DETECTR.
**FIGs. 53A-53B** present modifications and results from evaluating various amino modifications for DETECTR immobilization, respectively.
**FIG. 54** presents results for the FASTR assay, involving detection of SARS-CoV-2 with rapid thermocycling + CRISPR Dx.
**FIG. 55** presents results from a study to determine top performing polymerases and buffers for the FASTR assay.
**FIG. 56** presents results for single copy detection of SARS-CoV-2 with FASTR.
**FIG. 57** presents results for variations on rapid cycling times for denaturation and annealing/extension in FASTR.
**FIG. 58** presents results for minimizing RT time for FASTR.
**FIG. 59** presents results for higher pH buffers that improve FASTR performance.
**FIG. 60** presents results for FASTR compatibility with crude lysis buffers.
**FIG. 61** presents results for non-optimized multiplexing of FASTR.
**FIG. 62** presents results for multiplex FASTR.
**FIG. 63** presents results for the limit of detection of multiplex FASTR.
**FIG. 64** presents key primers and gRNAs.
**FIG. 65** presents results depicting the enhancement of the signal generated in a Hotpot reaction with the addition of Thermostable Inorganic Pyrophosphatase (TIPP) in comparison to control conditions lacking TIPP, target RNA or both.
**FIG. 66A** and **FIG. 66B** present results from DETECTR lateral flow Hotpot reaction assay strips, depicting the enhancement of the signal generated in a Hotpot reaction with the addition of Thermostable Inorganic Pyrophosphatase (TIPP) in comparison to control conditions lacking TIPP, target RNA or both.
**FIG. 67** shows an exemplary workflow for DETECTR-based HotPot reactions.
**FIG. 68** shows fluorescence results of HotPot reactions with reporters immobilized on glass beads.
**FIG. 69** shows lateral flow strip results using samples from the same experiments conducted to yield results illustrated in **FIG. 68****.**
**FIG. 70** shows fluorescence results of HotPot reactions with reporters immobilized on magnetic beads.
**FIG. 71** shows fluorescence results of DETECTR-based HotPot assays for a variety of respiratory disease nucleic acid sequence targets.
**FIGS. 72A-72C** shows results of limit of detection experiments for initial HotPot assay testing.
**FIG. 73** shows the fluorescence detected from HotPot assays in the presence of various additives.
**FIGS. 74A-74B** shows the influence of select additives that increase the speed and/or the signal strength of some HotPot assays.
**FIG. 75** shows HotPot results from experiments conducted with various amounts of a few additives.
**FIG. 76** shows HotPot results from experiments conducted with various amounts of a few additives with BSM DNA Polymerases.
**FIGS. 77A-77B** show lateral flow assay results of DETECTR-based OnePot and HotPot assays conducted with hydrogels comprising immobilized reporters.
**FIG. 78** shows an exemplary hydrogel comprising immobilized reporters copolymerized therein.
**FIGS. 79A** and **79B** show exemplary multiplexing strategies for hydrogel immobilized DETECTR systems.
**FIG.** 80 shows a schematic of the NEAR reaction. A forward and reverse primer consisting of a nicking enzyme stabilization site and recognition region able amplify a target region of interest into a single-stranded DNA molecule. A guide RNA will bind a region complementary to the amplified ssDNA, allowing further detection by a DETECTR system.
**FIGS. 81A-81B** show an exemplary NEAR-DETECTR reaction. **FIG. 81A** show a forward and reverse primer flanking the target region. In addition, a panel of 19 guide RNAs are shown in comparison to the amplicon. **FIG. 81B** shows the detection of the amplicon using NEAR-DETECTR using the above mentioned guide RNAs.
**FIGS. 82A-82B** shows an example NEAR reaction with an exemplary guide RNA showing detection of the E-gene of SARS-CoV2. **FIG. 82A** shows an example NEAR reaction with forward and reverse NEAR primers with a guide RNA. **FIG. 82B** shows the NEAR-DETECTR reaction following amplification, showing the resulting signal of 20,000 copies or 0 copies of the amplicon in solution.
**FIG. 83** shows the resulting signal of a NEAR-DETECTR reaction in which the pre-amplification time is varied prior to DETECTR.
**FIG. 84** shows the comparison of the NEAR-DETECTR reaction using orthogonal Cas systems (Cas12 Variant **(SEQ ID NO: 28),** Cas13 Variant **(SEQ ID NO: 154),** and Cas14 Variant **(SEQ ID NO. 63)).** The experiments were performed in the presence or absence of the target NEAR amplicon, showing different cleavage preferences for the reporter molecule.
**FIG. 85** shows the optimization of the NEAR reaction using different magnesium (Mg2+) concentrations. This shows the time to result (in minutes) comparing different buffer compositions and different concentrations of added magnesium. The top panel shows the following conditions using Bst2.0, and the bottom panel shows the following experimental conditions using Bst.3.0. These results informed the following experimental conditions for the NEAR reaction: Bst2.0, 12 mM Mg2+, at 60C, resulting in a less than 5 minute amplification time and approximately 20,000 copies.
**FIGS. 86A-86B** shows the experimental design of the primer and guide RNA design and the results of these designs. In **FIG. 86A****,** eight primer pairs (R1763 F(1-8)/R(1-8)) and the guide RNA R1763. In **FIG. 86B****,** the aforementioned primer pairs and guides R1763 and R1765 are used in a NEAR-DETECTR reaction to determine the efficacy of the reaction using different primer pairs. The raw fluorescence (AU) of the NEAR-DETECTR reaction is reported.
**FIGS. 87A-87B** describes the optimization of the hinge stabilization region in the NEAR primers to determine if the nicking enzyme activity can be modulated. In **FIG. 87A****,** the hinge stabilization loop region is modified to alter the melting temperature of the stem loop. In **FIG. 87B****,** the modified stem loops as shown in **FIG. 87A** were used for detection of the SARS-CoV-2 E-gene using different inputs of amplicons resulting from a NEAR amplification (1,000, 500, and 200 input amplicon copies).
**FIG. 88** shows a comparison of a reverse transcription-NEAR-DETECTR (RT-NECTR) reaction using different reverse transcriptases. Wartmstart RTx (NEB), Bst 3.0, and Omniscript RT (Qiagen) were used on different amounts of input RNA in order to compare the limit of detection (LOD) of these reaction conditions.
**FIG. 89** shows the experimental results of an RT-NECTR reaction using differing concentrations of NEAR primers.
**FIG. 90** shows the LOD of the E-gene of SARS-COV-2 using RT-NECTR using different amplification times and input copies for the RT-NECTR reaction.
**FIG. 91** shows detection of the SARS-COV-2 E gene using different Cas systems (Cas12 Variant **(SEQ ID NO: 28),** Cas13 Variant **(SEQ ID NO: 154),** and Cas14 Variant **(SEQ ID NO. 63))** and a panel of different guides.

### DETAILED DESCRIPTION

Unless otherwise defined, all technical terms used herein have the same meaning as commonly understood by one of ordinary skill in the art to which this invention belongs. As used in this specification and the appended claims, the singular forms "a," "an," and "the" include plural references unless the context clearly dictates otherwise. Any reference to "or" herein is intended to encompass "and/or" unless otherwise stated.

Whenever the term "at least," "greater than," or "greater than or equal to" precedes the first numerical value in a series of two or more numerical values, the term "at least," "greater than" or "greater than or equal to" applies to each of the numerical values in that series of numerical values. For example, greater than or equal to 1, 2, or 3 is equivalent to greater than or equal to 1, greater than or equal to 2, or greater than or equal to 3.

Whenever the term "no more than," "less than," "less than or equal to," or "at most" precedes the first numerical value in a series of two or more numerical values, the term "no more than," "less than," "less than or equal to," or "at most" applies to each of the numerical values in that series of numerical values. For example, less than or equal to 3, 2, or 1 is equivalent to less than or equal to 3, less than or equal to 2, or less than or equal to 1.

Where values are described as ranges, it will be understood that such disclosure includes the disclosure of all possible sub-ranges within such ranges, as well as specific numerical values that fall within such ranges irrespective of whether a specific numerical value or specific sub-range is expressly stated.

The present disclosure provides various compositions, methods, and devices of use thereof for assaying and detecting a nucleic acid. In many cases, nucleic acid detection comprises multiple steps (e.g., multiple reactions). For example, nucleic acid detection often comprises amplification of a target nucleic acid followed by detection. Augmenting the challenge of nucleic acid detection, multiple steps often require different conditions, which can necessitate buffer exchanges or sample transfers that increase the potential for contamination and sample loss. Furthermore, many single-buffer systems only support a single type of reaction, and drastically retard the rates of other reactions performed within them.

To address these challenges, aspects of the present disclosure provide buffers, systems, and compositions that can support multiple nucleic acid detection steps (e.g., multiple types of reactions) with optimal or close to optimal rates. Such buffers, systems, and compositions can enable nucleic acid detection without buffer exchanges or sample transfers, thereby diminishing total assay times, decreasing sample loss and contamination, and minimizing user input.

For example, particular aspects of the present disclosure provide containers comprising all of the reactants necessary for detection of a target nucleic acid from a sample. In such cases, no further user steps may be required once the sample is inserted into the container (e.g., through a pierceable film, membrane, or septum) and the container is placed within an instrument (e.g., a fluorimeter comprising a sample heater). In some aspects, a plurality of containers may be provided in a kit or on a single support (e.g., each container is a well on a multi-well plate). Two containers among the plurality of containers may comprise reagents targeting different nucleic acid sequences for detection to enable multiplexing.

The target nucleic acid can be a nucleic acid or a portion of a nucleic acid from a pathogen, virus, bacterium, fungi, protozoa, worm, or other agent(s) or organism(s) responsible for and/or related to a disease or condition in living organisms (e.g., humans, animals, plants, crops, and the like). The target nucleic acid can be a nucleic acid, or a portion thereof. The target nucleic acid can be a portion of a nucleic acid from a gene expressed in a cancer or genetic disorder in the sample The target nucleic acid can be a portion of an RNA or DNA from any organism in the sample. In some embodiments, one or more programmable nucleases as disclosed herein can be activated to initiate trans cleavage activity of a reporter (also referred to herein as a detector nucleic acid). In general, the term "reporter" as used in this context refers to a reagent comprising a polynucleotide, wherein cleavage of the polynucleotide results in a change in a signal. For example, the reporter may comprise a fluorescent label joined to a quencher by a short polynucleotide sequence. Little to no fluorescence is detectable from the fluorescent label when joined to the quencher. However, upon cleavage of the polynucleotide, the fluorescent label is separated from the quencher, resulting in a significant and detectable increase in fluorescent signal upon excitation of the label. Alternative labels and arrangements for producing a change in signal upon cleavage of the polynucleotide portion of the reporter are possible, and illustrative examples are described herein. The polynucleotide of the reporter can comprise DNA, RNA, modified nucleotides, or a combination of two or more of these. A programmable nuclease as disclosed herein can, in some cases, bind to a target sequence or target nucleic acid to initiate trans cleavage of a reporter. The programmable nuclease can be referred to as an RNA-activated programmable RNA nuclease. In some instances, the programmable nuclease as disclosed herein can bind to a target DNA to initiate trans cleavage of an RNA reporter. Such a programmable nuclease can be referred to herein as a DNA-activated programmable RNA nuclease. In some cases, a programmable nuclease as described herein can be activated by a target RNA or a target DNA. For example, a programmable nuclease, e.g., a Cas enzyme, can be activated by a target RNA nucleic acid or a target DNA nucleic acid to cleave RNA reporters. In some embodiments, the Cas enzyme can bind to a target ssDNA which initiates trans cleavage of RNA reporters. In some instances, a programmable nuclease as disclosed herein can bind to a target DNA to initiate trans cleavage of a DNA reporter, and this programmable nuclease can be referred to as a DNA-activated programmable DNA nuclease.

The systems and methods of the present disclosure can be implemented using a device that is compatible with any type of programmable nuclease that is human-engineered or naturally occurring. The programmable nuclease can comprise a nuclease that is capable of being activated when complexed with a guide nucleic acid and a target nucleic acid segment or a portion thereof. A programmable nuclease can become activated when complexed with a guide nucleic acid and a target sequence of a target gene of interest. The programmable nuclease can be activated upon binding of a guide nucleic acid to a target nucleic acid and can exhibit or enable trans cleavage activity once activated. In any instances or embodiments where a CRISPR-based programmable nuclease is described or used, it is recognized herein that any other type of programmable nuclease can be used in addition to or in substitution of such a CRISPR-based programmable nuclease.

Various methods, reagents, compositions, systems, and devices disclosed herein use a programmable nuclease complexed with guide nucleic acid sequence to detect the presence or absence of, and/or quantify the amount of, a target nucleic acid sequence. Binding of a guide nucleic acid with a target nucleic acid may activate a programmable nuclease to cleave single stranded nucleotides in a sequence non-specific manner, hereinafter referred to as "transcollateral" cleavage or "trans cleavage". Some assays of the present disclosure detect single-stranded, non-target nucleic acid cleavage to determine the presence and/or quantity of a target nucleic acid. Assays which leverage the transcollateral cleavage properties of programmable nuclease enzymes (e.g., CRISPR-Cas enzymes) are often referred to herein as DNA endonuclease targeted CRISPR trans reporter (DETECTR) reactions. Herein, detection of reporter cleavage (directly or indirectly) to determine the presence of a target nucleic acid sequence may be referred to as 'DETECTR'. In some embodiments, described herein is a method of assaying for a target nucleic acid in a sample comprising contacting the target nucleic acid with a programmable nuclease, a non-naturally occurring guide nucleic acid that hybridizes to a segment of the target nucleic acid, and a reporter, and assaying for a change in a signal, wherein the change in the signal is produced by or indicative of cleavage of the reporter.

Assays disclosed herein may comprise amplification (e.g., loop-mediated amplification (LAMP) or recombinase polymerase amplification (RPA), rolling circle amplification (RCA), nicking enzyme amplification reaction (NEAR), etc.) of a target nucleic acid sequence (e.g., a viral nucleic acid extracted from a patient). A target nucleic acid may also be reverse transcribed. An assay targeting an RNA sequence may utilize reverse transcription (RT) and amplification to generate amplicons for a programmable nuclease-based detection (e.g., DETECTR) reaction. A target nucleic acid may be amplified by thermal amplification (e.g., PCR) or isothermal amplification (e.g., LAMP, RCA, NEAR). An assay may utilize an amplification reaction and a programmable nuclease-based detection (e.g., DETECTR) reaction.

Among the various aspects of the present disclosure are compositions and methods for rapid and accurate detection of a target nucleic acid. In many cases, target nucleic acids are present in low copy numbers or as small proportions of the total nucleic acid content from a sample. This is a particular challenge in pathogen (e.g., viral) diagnostics, as nucleic acid biomarkers are often present in copy numbers below 1000 in samples derived from patients. Owing to this challenge, nucleic acid detection typically requires multi-step and multi-reaction assays. However, the assays themselves often have strict and non-overlapping requirements for the physical and chemical conditions in which they can be performed. For example, conditions that support rapid amplification reactions are often unsuitable for programmable nuclease-mediated reactions (e.g., CRISPR-Cas enzyme-mediated reactions), such as DETECTR reactions. Thus, nucleic acid detection methods frequently require sample transfers and buffer exchanges, resulting in long assay times leading to low yields.

In various aspects, the present disclosure provides a range of compositions that facilitate rapid, single buffer nucleic acid detection assays. Among such compositions, the present disclosure provides buffers capable of supporting rapid amplification and programmable nuclease (e.g., CRISPR-Cas) enzyme mediated reactions (e.g., DETECTR reactions). In such cases, the buffer may not only enable fast reaction rates, but also high reaction yields. Furthermore, multi-reaction buffer compatibility can drastically diminish an assay's required user input. Further aspects of the disclosure build on these favorable characteristics, and provide methods for performing single buffer nucleic acid detection assays, as well as kits for performing such assays.

### Assaying Methods

In some embodiments, a programmable nuclease can be used for detection of a target nucleic acid in a sample from a subject. The programmable nuclease may be provided in a buffer which enables fast (e.g., within 25%, 50%, or 75% of the fastest reported reaction rate for the programmable nuclease at a particular temperature) kinetics for the programmable nuclease and for reagents for other reactions (e.g., amplification, viral or cellular lysis, nucleic acid digestion, etc.). This can be particularly advantageous for low stability targets, such as RNA, which can be prone to rapid degradation, precipitation, denaturation, or side reactions. Whereas detecting a nucleic acid from a low titer sample (e.g., a sample comprising 1000, 500, 100, 50, 25, or 10 target nucleic acid molecules) may be unfeasible if a sample needs to be transferred between multiple reaction volumes, a single-buffer reaction system can enable detection of sparse nucleic acid targets by minimizing sample loss between steps.

The target nucleic acid may comprise a sequence associated with a pathogen (e.g., a virus), a human gene of interest (e.g., an oncogene such as BRCA1), a nucleic acid sequence for uniquely identifying an individual, or a fungal or bacterial nucleic acid sequence (e.g., for assessing skin health or a gut microbiome). For example, a programmable nuclease can be complexed with a guide nucleic acid that hybridizes to a target sequence of a target nucleic acid from coronavirus. The complex can be contacted to a sample from a subject. The target nucleic acid may or may not be present in the sample. If present, the target nucleic acid in the sample can optionally be reverse transcribed (RT). The target nucleic acid can be amplified by thermal amplification (e.g., PCR, FASTR) or isothermal amplification (e.g., LAMP, RPA, RT-RPA, or RT-LAMP). In some embodiments, reverse transcription and isothermal amplification may be performed simultaneously.

Upon activation, the programmable nuclease can cleave a reporter, which may comprise a detectable label attached to a polynucleotide (e.g., polydeoxyribonucleotide or polyribonucleotide). In some embodiments of the assay, upon cleavage of the polynucleotide, the detectable label emits a detectable signal, which is then detected and quantified (e.g., the detectable label may be a fluorophore and the detectable signal may be fluorescence). Upon detection of the detectable signal, it can be determined that the sample from the subject contained a target nucleic acid. In some embodiments, a programmable nuclease-based detection assay may detect multiple target nucleic acids or amplicons. For example, a programmable nuclease-based detection assay may target multiple non-overlapping or partially overlapping portions of a sequence of interest, while an amplification reaction may tile primers over a region of a target nucleic acid. Additionally, a programmable nuclease-based detection assay and/or amplification reaction may target multiple distinct sequences, such as sequences from separate portions of a genome or from separate genomes.

The compositions and methods of use thereof disclosed herein include using a programmable nuclease such as a Cas12 protein, a CasΦ protein, Cas14 protein, or a Cas13 protein to assay for, detect, and/or quantify a target nucleic acid. In some embodiments, a Cas12 protein, Cas13 protein, Cas14 protein, or a CasΦ protein is used for detection of a target nucleic acid in a sample from a subject. For use in an assay with a Cas12 protein, CasΦ protein, Cas14 protein, or a Cas13 protein, a target nucleic acid in a sample can be reverse transcribed and amplified by thermal (e.g., PCR, FASTR) or isothermal amplification (e.g., LAMP, RCA, NEAR). For use in an assay with a Cas13 protein, the amplified target nucleic acids can be transcribed back into RNA. If the subject is infected with coronavirus, the guide nucleic acid hybridizes to the target nucleic acid or amplicon thereof leading to activation of the Cas12 protein, Cas14 protein, CasΦ protein, or Cas13 protein. Upon activation, the Cas12 protein, the CasΦ protein, the Cas14 protein, or the Cas13 protein can cleave a reporter, wherein the reporter comprises a detectable label attached to the nucleic acid for cleavage by a Cas12 protein, the Cas14 protein a Cas13 protein, or a CasΦ protein. In some embodiments of the assay, upon cleavage of the reporter, the detectable label emits a detectable signal, which can then be captured and quantified (e.g., the detectable label may be a fluorophore and the detectable signal may be fluorescence). Upon detection of a detectable label, it can be determined that the sample comprised the target nucleic acid or target nucleic acids.

In some embodiments, a programmable nuclease having at least 60% sequence identity to SEQ ID NO: 18-170, 221-268, or 397-423 can be used for detection of a target nucleic acid (e.g., from a coronavirus such as SARS-CoV-2) in a sample from a subject. For example, a programmable nuclease having at least 60% sequence identity to SEQ ID NO: 18 can be complexed with a guide nucleic acid that hybridizes to a target sequence of a target nucleic acid from coronavirus. For example, a programmable nuclease having at least 60% sequence identity to any one of SEQ ID NOs: 397 , 398, 399, 400, 401, 402, 403, 404, 405, 406, 407, 408, 409, 410, 411, 412, 413, 414, 415, 416, 417, 418, 419, 420, 421, 422, or 423 can be complexed with a guide nucleic acid that hybridizes to a target sequence of a target nucleic acid from coronavirus. For example, a programmable nuclease having at least 60% sequence identity to SEQ ID NO: 406 can be complexed with a guide nucleic acid that hybridizes to a target sequence of a target nucleic acid from coronavirus.

Also described herein are methods, reagents, and devices for detecting the presence of a target nucleic acid in a sample. The methods, reagents, and devices for detecting the presence of a target nucleic acid in a sample can be used in rapid lab tests for detection of a target nucleic acid of interest (e.g., target nucleic acids from a target population). In particular, provided herein are methods, reagents, and devices wherein the rapid lab tests can be performed in a single system. The target nucleic acid may be a portion of a nucleic acid from a virus (e.g., coronavirus) or other agents responsible for a disease in the sample.

In some embodiments, programmable nucleases disclosed herein are activated by RNA or DNA to initiate trans cleavage activity of a reporter. A programmable nuclease as disclosed herein, in some cases, binds to a target RNA to initiate trans cleavage of a reporter, and this programmable nuclease can be referred to as an RNA-activated programmable RNA nuclease. In some instances, a programmable nuclease as disclosed herein binds to a target DNA to initiate trans cleavage of a reporter, and this programmable nuclease can be referred to as a DNA-activated programmable RNA nuclease. In some cases, a programmable nuclease as described herein is capable of being activated by a target RNA or a target DNA. For example, a Cas13 protein, such as a Cas13a, disclosed herein is activated by a target RNA nucleic acid or a target DNA nucleic acid to transcollaterally cleave an RNA reporter. In some embodiments, the Cas13 binds to a target ssDNA which initiates trans cleavage of an RNA reporter.

The detection of the target nucleic acid in the sample may indicate the presence of the disease in the sample and may provide information for taking action to reduce the transmission of the disease to individuals in the disease-affected environment or near the disease-carrying individual. The detection of the target nucleic acid in the sample may indicate the presence of a disease mutation, such as a single nucleotide polymorphism (SNP) that provide antibiotic resistance to a disease-causing bacteria. The detection of the target nucleic acid is facilitated by a programmable nuclease. The programmable nuclease can become activated after binding of a guide nucleic acid with a target nucleic, in which the activated programmable nuclease can cleave the target nucleic acid and can have trans cleavage activity, which can also be referred to as "collateral" or "transcollateral" cleavage.

Trans cleavage activity can be non-specific cleavage of nearby single-stranded nucleic acids by the activated programmable nuclease, such as trans cleavage of reporters with a detection moiety. Once the reporter is cleaved by the activated programmable nuclease, the detection moiety is released from the reporter and generates a detectable signal. Often the detection moiety is at least one of a fluorophore, a dye, a polypeptide, or a nucleic acid. Sometimes the detection moiety binds to a capture molecule on the support medium to be immobilized. The detectable signal can be visualized on the support medium to assess the presence or level of the target nucleic acid associated with an ailment, such as a disease. The programmable nuclease can be a CRISPR-Cas (clustered regularly interspaced short palindromic repeats - CRISPR associated) nucleoprotein complex with trans cleavage activity, which can be activated by binding of a guide nucleic acid with a target nucleic acid. These assays, which leverage the transcollateral cleavage properties of programmable nucleases (e.g., CRISPR-Cas enzymes) are often referred to herein as DNA endonuclease targeted CRISPR trans reporter (DETECTR) reactions. A programmable nuclease-based detection (e.g., DETECTR) reaction can be performed in a fluidic device.

In some embodiments, the present disclosure provides for Cas12 detection of a target nucleic acid from a coronavirus. In this case, nucleic acids (e.g., RNA) from a sample may be optionally reverse transcribed and/or amplified into DNA. Any Cas12 protein disclosed herein may be complexed with a guide nucleic acid designed to hybridize to a nucleic acid sequence of the (optionally reverse transcribed and/or amplified) DNA. DETECTR reactions can then be carried out. In the presence of reverse transcribed and amplified DNA indicative of coronavirus, Cas12 is activated to transcollaterally cleave a reporter, emitting a detectable signal (e.g., fluorescence). In some embodiments, the present disclosure provides for Cas13 detection of a target nucleic acid from a coronavirus. In this case, RNA in a sample may be either directly detected by complexing a Cas13 enzyme with a guide nucleic acid designed to hybridize to a target RNA sequence from a coronavirus or, RNA may be reverse transcribed, amplified, and in vitro transcribed prior to contacting it with a Cas13 enzyme complexed with a guide nucleic acid designed to hybridize this amplified target RNA sequence from a coronavirus. In the presence of the RNA (unamplified or amplified), Cas13 may be activated to transcollaterally cleave a reporter, thereby emitting a detectable signal (e.g., fluorescence). In some embodiments, the present disclosure provides for Cas14 detection of a target nucleic acid from a coronavirus. In this case, nucleic acids (e.g., RNA) from a sample may be optionally reverse transcribed and/or amplified into DNA. Any Cas14 protein disclosed herein may be complexed with a guide nucleic acid designed to hybridize to a nucleic acid sequence of the (optionally reverse transcribed and/or amplified) DNA. DETECTR reactions can then be carried out. In the presence of reverse transcribed and amplified DNA indicative of coronavirus, Cas14 is activated to transcollaterally cleave a reporter, emitting a detectable signal (e.g., fluorescence). In some embodiments, the present disclosure provides for CasPhi detection of a target nucleic acid from a coronavirus. In this case, nucleic acids (e.g., RNA) from a sample may be optionally reverse transcribed and/or amplified into DNA. Any CasPhi protein disclosed herein may be complexed with a guide nucleic acid designed to hybridize to a nucleic acid sequence of the (optionally reverse transcribed and/or amplified) DNA. DETECTR reactions can then be carried out. In the presence of reverse transcribed and amplified DNA indicative of coronavirus, CasPhi is activated to transcollaterally cleave a reporter, emitting a detectable signal (e.g., fluorescence).

Also described herein is a kit for detecting a target nucleic acid (e.g., from a coronavirus such as SARS-CoV-2). The kit may comprise a support medium; a guide nucleic acid sequences targeted to a target nucleic acid sequence; a programmable nuclease capable of being activated when complexed with a guide nucleic acid and a target nucleic acid; and a single-stranded reporter comprising a detection moiety, wherein the reporter is capable of being cleaved by the activated nuclease, thereby generating a first detectable signal. In some cases, the target nucleic acid for detecting a coronavirus may comprise a sequence with at least 60% sequence similarity to that of SEQ ID NOs: 179-184. In some cases, the guide nucleic acid for detection of a coronavirus may comprise a sequence with at least 60% sequence similarity to the sequences in SEQ ID NOs: 318-327.

A biological sample from an individual or an environmental sample can be tested for the presence of a particular nucleic acid sequence (e.g., whether a human sample comprises coronavirus). The detection of the target nucleic acid detected can also indicate that one or more of the target populations is wild-type or comprises a mutation, such as a mutation that confers resistance to treatment, such as antibiotic treatment. A sample from an individual or from an environment is applied to the reagents described herein. If the target nucleic acid is present in the sample, the target nucleic acid binds to the guide nucleic acid to activate the programmable nuclease. The activated programmable nuclease cleaves the reporter and generates a detectable signal that can be visualized, for example on a support medium. If the target nucleic acid is absent in the sample or below the threshold of detection, the guide nucleic acid remains unbound, the programmable nuclease remains inactivated, and the reporter remains uncleaved. Such methods, reagents, and devices described herein may allow for detection of target nucleic acid, and in turn the disease associated with the target nucleic acids (e.g., coronavirus such as SARS-CoV-2), in remote regions or low resource settings without specialized equipment. Also, such methods, reagents, and devices described herein may allow for detection of target nucleic acid, and in turn the disease associated with the target nucleic acids, in healthcare clinics or doctor offices without specialized equipment. In some cases, this provides a point of care testing for users to quickly and easily test for a disease or infection with high sensitivity at home or in an office of a healthcare provider. Assays that deliver results in under an hour, for example, in 15 to 60 minutes, are particularly desirable for at home testing for many reasons. For example, antivirals can be most effective when administered within the first 48 hours after disease exposure. Thus, the methods disclosed herein, which are capable of delivering results in under an hour, may allow for the delivery of anti-viral therapy during the first 48 hours after infection. Additionally, the systems and assays provided herein, which are capable of delivering quick diagnoses and results, can help keep or send a patient at home, improve comprehensive disease surveillance, and prevent the spread of an infection. In other cases, this provides a test, which can be used in a lab to detect one or more nucleic acid populations or varieties of interest in a sample from a subject. In particular, provided herein are methods, reagents, and devices, wherein the high sensitivity lab tests can be performed in a single assay. In some cases, this may be valuable in detecting diseases in a developing country and as a global healthcare tool to detect the spread of a disease or efficacy of a treatment or provide early detection of a disease.

Some methods as described herein use an editing technique, such as a technique using an editing enzyme or a programmable nuclease and guide nucleic acid, to detect a target nucleic acid. An editing enzyme or a programmable nuclease in the editing technique can be activated by one or more target nucleic acids, after which the activated editing enzyme or activated programmable nuclease can cleave nearby single-stranded nucleic acids, such reporters with a detection moiety. A target nucleic acid can be amplified by isothermal amplification or thermocycling amplification and then an editing technique can be used to detect the marker. In some instances, the editing technique can comprise an editing enzyme or programmable nuclease that, when activated, cleaves nearby RNA or DNA as the readout of the detection. The methods as described herein in some instances comprise obtaining a cell-free DNA sample, amplifying DNA from the sample, using an editing technique to cleave reporters, and reading the output of the editing technique. In other instances, the method comprises obtaining a fluid sample from a patient, and without amplifying a nucleic acid of the fluid sample, using an editing technique to cleave reporters, and detecting the nucleic acid. The method can also comprise using single-stranded detector DNA, cleaving the single-stranded detector DNA using an activated editing enzyme, wherein the editing enzyme cleaves at least 50% of a population of single-stranded detector DNA as measured by a change in color. A number of samples, guide nucleic acids, programmable nucleases or editing enzymes, support mediums, target nucleic acids, single-stranded reporters, and reagents are consistent with the devices, systems, fluidic devices, kits, and methods disclosed herein.

Also disclosed herein are reporters and methods detecting a target nucleic using the reporters. Often, the reporter is a protein-nucleic acid. For example, a method of assaying for a target nucleic acid in a sample comprises contacting the sample to a plurality of complexes comprising a guide nucleic acid, each guide nucleic acid sequence comprising a segment that is reverse complementary to a segment of a target nucleic acid sequence within a target nucleic acid population and programmable nucleases that exhibits sequence independent cleavage upon forming complexes comprising the segment of the guide nucleic acid binding to the segment of the target nucleic acid; and assaying for a signal indicating cleavage of at least some protein-nucleic acids of a population of protein-nucleic acids, wherein the signal indicates a presence of one or more of the target nucleic acid populations in the sample and wherein absence of the signal indicates an absence of the target nucleic acid population in the sample. Often, the protein-nucleic acid is an enzyme-nucleic acid or an enzyme substrate-nucleic acid. The nucleic acid can be DNA, RNA, or a DNA/RNA hybrid. The methods described herein use a programmable nuclease, such as the CRISPR/Cas system, to detect a target nucleic acid (e.g. from a coronavirus such as SARS-CoV-2). A method of assaying for a target nucleic acid (e.g. from a coronavirus such as SARS-CoV-2) in a sample, for example, comprises: a) contacting the sample to a plurality of complexes comprising a guide nucleic acid, each guide nucleic acid sequence comprising a segment that is reverse complementary to a segment of a nucleic acid target sequence within a target nucleic acid population, and programmable nucleases that exhibits sequence independent cleavage upon forming complexes comprising the segment of the guide nucleic acid binding to the segment of the target nucleic acid; b) contacting the complexes to a substrate; c) contacting the substrate to a reagent that differentially reacts with a cleaved substrate; and d) assaying for a signal indicating cleavage of the substrate, wherein the signal indicates a presence of one or more of the target nucleic acid populations in the sample and wherein absence of the signal indicates an absence of the target nucleic acid population in the sample. Often, the substrate is an enzyme-nucleic acid. Sometimes, the substrate is an enzyme substrate-nucleic acid.

Cleavage of the protein-nucleic acid produces a signal. For example, cleavage of the protein-nucleic acid produces a calorimetric signal, a potentiometric signal, an amperometric signal, an optical signal, or a piezo-electric signal. Various devices can be used to detect these different types of signals, which indicate whether a target nucleic acid is present in the sample.

The present disclosure provides buffers capable of supporting rapid amplification and programmable nuclease-based reactions (e.g., DETECTR).

In some assays, such a buffer comprising amplification and programmable nuclease-based detection reagents is contacted to a sample, thereby enabling simultaneous amplification and programmable nuclease-based detection reactions. For example, a buccal swab sample may be added to a buffer comprising amplification and programmable nuclease-based detection reagents targeting a particular oral bacterium gene, thereby enabling an assay for that particular oral bacterium. In some assays, a target nucleic acid is first contacted with amplification reagents, and later (e.g., after the amplification reaction has come to completion) contacted with reagents for a programmable nuclease-based detection reaction. In some assays, a target nucleic acid is first contacted with amplification reagents, and later sequentially contacted with different sets of programmable nuclease-based detection reagents targeting different nucleic acid sequences, wherein programmable nuclease-based detection reagent additions that lead to an increase in signal (e.g., fluorescence signal) may indicate the presence of a particular nucleic acid sequence.

### Sample

A number of samples are consistent with the methods, reagents, and devices disclosed herein.

These samples can comprise a target nucleic acid for detection of an ailment, such as a disease, pathogen, or virus, such as influenza. Generally, a sample from an individual or an animal or an environmental sample can be obtained to test for presence of a disease, or any mutation of interest. A biological sample from the individual may be blood, serum, plasma, saliva, urine, mucosal sample, peritoneal sample, cerebrospinal fluid, gastric secretions, nasal secretions, sputum, pharyngeal exudates, urethral or vaginal secretions, an exudate, an effusion, or tissue. A tissue sample may be dissociated or liquified prior to application to the detection system of the present disclosure. A sample from an environment may be from soil, air, or water. In some instances, the environmental sample is taken as a swab from a surface of interest or taken directly from the surface of interest. In some instances, the raw sample is applied to the detection system. In some instances, the sample is diluted with a buffer or a fluid or concentrated prior to application to the detection system or be applied neat to the detection system. Sometimes, the sample is contained in no more 20 µL. The sample, in some cases, is contained in no more than 1, 5, 10, 15, 20, 25, 30, 35 40, 45, 50, 55, 60, 65, 70, 75, 80, 90, 100, 200, 300, 400, 500 µL, or any of value from 1 µL to 500 µL. Sometimes, the sample is contained in more than 500 µL. The sample may be contained within a solid, membranous, or mesh material, such as a swab (e.g., a buccal or nasal swab).

In some instances, the sample is taken from single-cell eukaryotic organisms; a plant or a plant cell; an algal cell; a fungal cell; an animal cell, tissue, or organ; a cell, tissue, or organ from an invertebrate animal; a cell, tissue, fluid, or organ from a vertebrate animal such as fish, amphibian, reptile, bird, and mammal; a cell, tissue, fluid, or organ from a mammal such as a human, a non-human primate, an ungulate, a feline, a bovine, an ovine, and a caprine. In some instances, the sample is taken from nematodes, protozoans, helminths, or malarial parasites. In some cases, the sample comprises nucleic acids from a cell lysate from a eukaryotic cell, a mammalian cell, a human cell, a prokaryotic cell, or a plant cell. In some cases, the sample comprises nucleic acids expressed from a cell.

The sample used for disease testing may comprise at least one target sequence that can bind to a guide nucleic acid of the reagents described herein. A portion of a nucleic acid can be from a genomic locus, a transcribed mRNA, or a reverse transcribed cDNA. A portion of a nucleic acid can be from 5 to 100, 5 to 90, 5 to 80, 5 to 70, 5 to 60, 5 to 50, 5 to 40, 5 to 30, 5 to 25, 5 to 20, 5 to 15, or 5 to 10 nucleotides in length. A portion of a nucleic acid can be 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 45, 50, 60, 70, 80, 90, or 100 nucleotides in length. The target sequence can be reverse complementary to a guide nucleic acid. Each target sequence of the multiple target sequences can be reverse complementary to a distinct guide nucleic acid.

The systems and methods of the present disclosure can be used to detect one or more target sequences or nucleic acids in one or more samples. The one or more samples can comprise one or more target sequences or nucleic acids for detection of an ailment, such as a disease, cancer, or genetic disorder, or genetic information, such as for phenotyping, genotyping, or determining ancestry and are compatible with the reagents and support mediums as described herein. Generally, a sample can be taken from any place where a nucleic acid can be found. Samples can be taken from an individual/human, a non-human animal, or a crop, or an environmental sample can be obtained to test for presence of a disease, virus, pathogen, cancer, genetic disorder, or any mutation or pathogen of interest. A biological sample can be blood, serum, plasma, lung fluid, exhaled breath condensate, saliva, spit, urine, stool, feces, mucus, lymph fluid, peritoneal , cerebrospinal fluid, amniotic fluid, breast milk, gastric secretions, bodily discharges, secretions from ulcers, pus, nasal secretions, sputum, pharyngeal exudates, urethral secretions/mucus, vaginal secretions/mucus, anal secretion/mucus, semen, tears, an exudate, an effusion, tissue fluid, interstitial fluid (e.g., tumor interstitial fluid), cyst fluid, tissue, or, in some instances, any combination thereof. A sample can be an aspirate of a bodily fluid from an animal (e.g., human, animals, livestock, pet, etc.) or plant. A tissue sample can be from any tissue that can be infected or affected by a pathogen (e.g., a wart, lung tissue, skin tissue, and the like). A tissue sample (e.g., from animals, plants, or humans) can be dissociated or liquified prior to application to detection system of the present disclosure. A sample can be from a plant (e.g., a crop, a hydroponically grown crop or plant, and/or house plant). Plant samples can include extracellular fluid, from tissue (e.g., root, leaves, stem, trunk etc.). A sample can be taken from the environment immediately surrounding a plant, such as hydroponic fluid/ water, or soil. A sample from an environment can be from soil, air, or water. In some instances, the environmental sample is taken as a swab from a surface of interest or taken directly from the surface of interest. In some instances, the raw sample is applied to the detection system. In some instances, the sample is diluted with a buffer or a fluid or concentrated prior to application to the detection system. In some cases, the sample is contained in no more than about 200 nanoliters (nL). In some cases, the sample is contained in about 200 nL. In some cases, the sample is contained in a volume that is greater than about 200 nL and less than about 20 microliters (µL). In some cases, the sample is contained in no more than 20 µl. In some cases, the sample is contained in no more than 1, 5, 10, 15, 20, 25, 30, 35 40, 45, 50, 55, 60, 65, 70, 75, 80, 90, 100, 200, 300, 400, 500 µl, or any of value from 1 µl to 500 µl. In some cases, the sample is contained in from 1 µL to 500 µL, from 10 µL to 500 µL, from 50 µL to 500 µL, from 100 µL to 500 µL, from 200 µL to 500 µL, from 300 µL to 500 µL, from 400 µL to 500 µL, from 1 µL to 200 µL, from 10 µL to 200 µL, from 50 µL to 200 µL, from 100 µL to 200 µL, from 1 µL to 100 µL, from 10 µL to 100 µL, from 50 µL to 100 µL, from 1 µL to 50 µL, from 10 µL to 50 µL, from 1 µL to 20 µL, from 10 µL to 20 µL, or from 1 µL to 10 µL. Sometimes, the sample is contained in more than 500 µl.

In some instances, the sample is taken from a single-cell eukaryotic organism; a plant or a plant cell; an algal cell; a fungal cell; an animal or an animal cell, tissue, or organ; a cell, tissue, or organ from an invertebrate animal; a cell, tissue, fluid, or organ from a vertebrate animal such as fish, amphibian, reptile, bird, and mammal; a cell, tissue, fluid, or organ from a mammal such as a human, a non-human primate, an ungulate, a feline, a bovine, an ovine, and a caprine. In some instances, the sample is taken from nematodes, protozoans, helminths, or malarial parasites. In some cases, the sample may comprise nucleic acids from a cell lysate from a eukaryotic cell, a mammalian cell, a human cell, a prokaryotic cell, or a plant cell. In some cases, the sample may comprise nucleic acids expressed from a cell.

The sample used for disease testing can comprise at least one target sequence that can bind to a guide nucleic acid of the reagents described herein. In some cases, the target sequence is a portion of a nucleic acid. A nucleic acid can be from a genomic locus, a transcribed mRNA, or a reverse transcribed cDNA. A nucleic acid can be from 5 to 100, 5 to 90, 5 to 80, 5 to 70, 5 to 60, 5 to 50, 5 to 40, 5 to 30, 5 to 25, 5 to 20, 5 to 15, or 5 to 10 nucleotides in length. A nucleic acid can be from 10 to 90, from 20 to 80, from 30 to 70, or from 40 to 60 nucleotides in length. A nucleic acid sequence can be from 10 to 95, from 20 to 95, from 30 to 95, from 40 to 95, from 50 to 95, from 60 to 95, from 10 to 75, from 20 to 75, from 30 to 75, from 40 to 75, from 50 to 75, from 5 to 50, from 15 to 50, from 25 to 50, from 35 to 50, or from 45 to 50 nucleotides in length. A nucleic acid can be 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 45, 50, 60, 70, 80, 90, or 100 nucleotides in length. The target nucleic acid can be reverse complementary to a guide nucleic acid. In some cases, at least 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 45, 50, 60, 70, 80, 90, or 100 nucleotides of a guide nucleic acid can be reverse complementary to a target nucleic acid.

In some cases, the target sequence is a portion of a nucleic acid from a virus or a bacterium or other agents responsible for a disease in the sample. The target sequence, in some cases, is a portion of a nucleic acid from a sexually transmitted infection or a contagious disease, in the sample. The target sequence, in some cases, is a portion of a nucleic acid from an upper respiratory tract infection, a lower respiratory tract infection, or a contagious disease, in the sample. The target sequence, in some cases, is a portion of a nucleic acid from a hospital acquired infection or a contagious disease, in the sample. The target sequence, in some cases, is a portion of a nucleic acid from sepsis, in the sample. These diseases can include but are not limited to respiratory viruses (e.g., SARS-CoV-2 (i.e., a virus that causes COVID-19), SARS, MERS, influenza, Adenovirus, Coronavirus HKU1, Coronavirus NL63, Coronavirus 229E, Coronavirus OC43, Severe Acute Respiratory Syndrome Coronavirus 2 (SARS-CoV-2), Human Metapneumovirus (hMPV), Human Rhinovirus/Enterovirus, Influenza A, Influenza A/H1, Influenza A/H3, Influenza A/H1-2009, Influenza B, Influenza C, Parainfluenza Virus 1, Parainfluenza Virus 2, Parainfluenza Virus 3, Parainfluenza Virus 4, Respiratory Syncytial Virus) and respiratory bacteria (e.g. Bordetella parapertussis, Bordetella pertussis, Chlamydia pneumoniae, Mycoplasma pneumoniae). Other viruses include human immunodeficiency virus (HIV), human papillomavirus (HPV), chlamydia, gonorrhea, syphilis, trichomoniasis, sexually transmitted infection, malaria, Dengue fever, Ebola, chikungunya, and leishmaniasis. Pathogens include viruses, fungi, helminths, protozoa, malarial parasites, *Plasmodium* parasites, *Toxoplasma* parasites, and *Schistosoma* parasites. Helminths include roundworms, heartworms, and phytophagous nematodes, flukes, Acanthocephala, and tapeworms. Protozoan infections include infections from *Giardia spp., Trichomonas spp.,* African trypanosomiasis, amoebic dysentery, babesiosis, balantidial dysentery, Chaga's disease, coccidiosis, malaria and toxoplasmosis. Examples of pathogens such as parasitic/protozoan pathogens include, but are not limited to: *Plasmodium falciparum, P. vivax, Trypanosoma cruzi* and *Toxoplasma gondii.* Fungal pathogens include, but are not limited to *Cryptococcus neoformans, Histoplasma capsulatum, Coccidioides immitis, Blastomyces dermatitidis, Chlamydia trachomatis, Chlamydia pneumoniae, Chlamydia psittaci, and Candida albicans.* Pathogenic viruses include but are not limited to: respiratory viruses (e.g., adenoviruses, parainfluenza viruses, severe acute respiratory syndrome (SARS), coronavirus, MERS), gastrointestinal viruses (e.g., noroviruses, rotaviruses, some adenoviruses, astroviruses), exanthematous viruses (e.g., the virus that causes measles, the virus that causes rubella, the virus that causes chickenpox/shingles, the virus that causes roseola, the virus that causes smallpox, the virus that causes fifth disease, chikungunya virus infection); hepatic viral diseases (e.g., hepatitis A, B, C, D, E); cutaneous viral diseases (e.g., warts (including genital, anal), herpes (including oral, genital, anal), molluscum contagiosum); hemmorhagic viral diseases (e.g. Ebola, Lassa fever, dengue fever, yellow fever, Marburg hemorrhagic fever, Crimean-Congo hemorrhagic fever); neurologic viruses (e.g., polio, viral meningitis, viral encephalitis, rabies), sexually transmitted viruses (e.g., HIV, HPV, and the like), immunodeficiency virus (e.g., HIV); influenza virus; dengue; West Nile virus; herpes virus; yellow fever virus; Hepatitis Virus C; Hepatitis Virus A; Hepatitis Virus B; papillomavirus; and the like. Pathogens include, e.g., HIV virus, *Mycobacterium tuberculosis, Klebsiella pneumoniae, Acinetobacter baumannii, Bacillus anthracis, Bortadella pertussis, Burkholderia cepacia, Corynebacterium diphtheriae, Coxiella burnetii, Streptococcus agalactiae, methicillin-resistant Staphylococcus aureus, Legionella longbeachae, Legionella pneumophila, Leptospira interrogans, Moraxella catarrhalis, Streptococcus pyogenes, Escherichia coli, Neisseria gonorrhoeae, Neisseria meningitidis, Neisseria elongate, Neisseria gonorrhoeae,* Parechovirus, *Pneumococcus, Pneumocystis jirovecii, Cryptococcus neoformans, Histoplasma capsulatum, Haemophilus influenzae B, Treponema pallidum,* Lyme disease spirochetes, *Pseudomonas aeruginosa, Mycobacterium leprae, Brucella abortus,* rabies virus, influenza virus, cytomegalovirus, herpes simplex virus I, herpes simplex virus II, human serum parvo-like virus, respiratory syncytial virus (RSV), *M. genitalium, T. Vaginalis,* varicella-zoster virus, hepatitis B virus, hepatitis C virus, measles virus, adenovirus, human T-cell leukemia viruses, Epstein-Barr virus, murine leukemia virus, mumps virus, vesicular stomatitis virus, Sindbis virus, lymphocytic choriomeningitis virus, wart virus, blue tongue virus, Sendai virus, feline leukemia virus, Reovirus, polio virus, simian virus 40, mouse mammary tumor virus, dengue virus, rubella virus, West Nile virus, *Plasmodium falciparum, Plasmodium vivax, Toxoplasma gondii, Trypanosoma rangeli, Trypanosoma cruzi, Trypanosoma rhodesiense, Trypanosoma brucei, Schistosoma mansoni, Schistosoma japonicum, Babesia bovis, Eimeria tenella, Onchocerca volvulus, Leishmania tropica, Mycobacterium tuberculosis, Trichinella spiralis, Theileria parva, Taenia hydatigena, Taenia ovis, Taenia saginata, Echinococcus granulosus, Mesocestoides corti, Mycoplasma arthritidis, M. hyorhinis, M. orale, M. arginini, Acholeplasma laidlawii, M. salivarium, M. pneumoniae, Enterobacter cloacae, Kiebsiella aerogenes, Proteus vulgaris, Serratia macesens, Enterococcus faecalis, Enterococcus faecium, Streptococcus intermdius, Streptococcus pneumoniae, and Streptococcus pyogenes.* Often the target nucleic acid may comprise a sequence from a virus or a bacterium or other agents responsible for a disease that can be found in the sample. In some cases, the target nucleic acid is a portion of a nucleic acid from a genomic locus, a transcribed mRNA, or a reverse transcribed cDNA from a gene locus in at least one of: human immunodeficiency virus (HIV), human papillomavirus (HPV), chlamydia, gonorrhea, syphilis, trichomoniasis, sexually transmitted infection, malaria, Dengue fever, Ebola, chikungunya, and leishmaniasis. Pathogens include viruses, fungi, helminths, protozoa, malarial parasites, *Plasmodium* parasites, *Toxoplasma* parasites, and *Schistosoma* parasites. Helminths include roundworms, heartworms, and phytophagous nematodes, flukes, Acanthocephala, and tapeworms. Protozoan infections include infections from *Giardia spp., Trichomonas spp.,* African trypanosomiasis, amoebic dysentery, babesiosis, balantidial dysentery, Chaga's disease, coccidiosis, malaria and toxoplasmosis. Examples of pathogens such as parasitic/protozoan pathogens include, but are not limited to: *Plasmodium falciparum, P. vivax, Trypanosoma cruzi* and *Toxoplasma gondii.* Fungal pathogens include, but are not limited to *Cryptococcus neoformans, Histoplasma capsulatum, Coccidioides immitis, Blastomyces dermatitidis, Chlamydia trachomatis, and Candida albicans.* Pathogenic viruses include but are not limited to immunodeficiency virus (e.g., HIV); influenza virus; dengue; West Nile virus; herpes virus; yellow fever virus; Hepatitis Virus C; Hepatitis Virus A; Hepatitis Virus B; papillomavirus; and the like. Pathogens include, e.g., HIV virus, *Mycobacterium tuberculosis, Streptococcus agalactiae, methicillin-resistant Staphylococcus aureus, Staphylococcus epidermidis, Legionella pneumophila, Streptococcus pyogenes, Streptococcus salivarius, Escherichia coli, Neisseria gonorrhoeae, Neisseria meningitidis, Pneumococcus, Cryptococcus neoformans, Histoplasma capsulatum, Hemophilus influenzae B, Treponema pallidum,* Lyme disease spirochetes, *Pseudomonas aeruginosa, Mycobacterium leprae, Brucella abortus,* rabies virus, influenza virus, cytomegalovirus, herpes simplex virus I, herpes simplex virus II, human serum parvo-like virus, respiratory syncytial virus (RSV), *M. genitalium, T. vaginalis,* varicella-zoster virus, hepatitis B virus, hepatitis C virus, measles virus, adenovirus, human T-cell leukemia viruses, Epstein-Barr virus, murine leukemia virus, mumps virus, vesicular stomatitis virus, Sindbis virus, lymphocytic choriomeningitis virus, wart virus, blue tongue virus, Sendai virus, feline leukemia virus, Reovirus, polio virus, simian virus 40, mouse mammary tumor virus, dengue virus, rubella virus, West Nile virus, *Plasmodium falciparum, Plasmodium vivax, Toxoplasma gondii, Trypanosoma rangeli, Trypanosoma cruzi, Trypanosoma rhodesiense, Trypanosoma brucei, Schistosoma mansoni, Schistosoma japonicum, Babesia bovis, Eimeria tenella, Onchocerca volvulus, Leishmania tropica, Mycobacterium tuberculosis, Trichinella spiralis, Theileria parva, Taenia hydatigena, Taenia ovis, Taenia saginata, Echinococcus granulosus, Mesocestoides corti, Mycoplasma arthritidis, M. hyorhinis, M. orale, M. arginini, Acholeplasma laidlawii, M. salivarium* and *M. pneumoniae.* In some cases, the target sequence is a portion of a nucleic acid from a genomic locus, a transcribed mRNA, or a reverse transcribed cDNA from a gene locus of bacterium or other agents responsible for a disease in the sample comprising a mutation that confers resistance to a treatment, such as a single nucleotide mutation that confers resistance to antibiotic treatment.

The sample used for cancer testing or cancer risk testing can comprise at least one target sequence or target nucleic acid segment that can bind to a guide nucleic acid of the reagents described herein. The target nucleic acid segment, in some cases, is a portion of a nucleic acid from a gene with a mutation associated with cancer, from a gene whose overexpression is associated with cancer, a tumor suppressor gene, an oncogene, a checkpoint inhibitor gene, a gene associated with cellular growth, a gene associated with cellular metabolism, or a gene associated with cell cycle. Sometimes, the target nucleic acid encodes for a cancer biomarker, such as a prostate cancer biomarker or non-small cell lung cancer. In some cases, the assay can be used to detect "hotspots" in target nucleic acids that can be predictive of cancer, such as lung cancer, cervical cancer, in some cases, the cancer can be a cancer that is caused by a virus. Some non-limiting examples of viruses that cause cancers in humans include Epstein-Barr virus (e.g., Burkitt's lymphoma, Hodgkin's Disease, and nasopharyngeal carcinoma); papillomavirus (e.g., cervical carcinoma, anal carcinoma, oropharyngeal carcinoma, penile carcinoma); hepatitis B and C viruses (e.g., hepatocellular carcinoma); human adult T-cell leukemia virus type 1 (HTLV-1) (e.g., T-cell leukemia); and Merkel cell polyomavirus (e.g., Merkel cell carcinoma). One skilled in the art will recognize that viruses can cause or contribute to other types of cancers. In some cases, the target nucleic acid is a portion of a nucleic acid that is associated with a blood fever. In some cases, the target nucleic acid segment is a portion of a nucleic acid from a genomic locus, a transcribed mRNA, or a reverse transcribed cDNA from a locus of at least one of: ALK, APC, ATM, AXIN2, BAP1, BARD1, BLM, BMPR1A, BRCA1, BRCA2, BRIP1, CASR, CDC73, CDH1, CDK4, CDKN1B, CDKN1C, CDKN2A, CEBPA, CHEK2, CTNNA1, DICER1, DIS3L2, EGFR, EPCAM, FH, FLCN, GATA2, GPC3, GREM1, HOXB13, HRAS, KIT, MAX, MEN1, MET, MITF, MLH1, MSH2, MSH3, MSH6, MUTYH, NBN, NF1, NF2, NTHL1, PALB2, PDGFRA, PHOX2B, PMS2, POLD1, POLE, POT1, PRKAR1A, PTCH1, PTEN, RAD50, RAD51C, RAD51D, RB1, RECQL4, RET, RUNX1, SDHA, SDHAF2, SDHB, SDHC, SDHD, SMAD4, SMARCA4, SMARCB1, SMARCE1, STK11, SUFU, TERC, TERT, TMEM127, TP53, TSC1, TSC2, VHL, WRN, and WT1.

The sample used for genetic disorder testing can comprise at least one target sequence or target nucleic acid segment that can bind to a guide nucleic acid of the reagents described herein. In some embodiments, the genetic disorder is hemophilia, sickle cell anemia, β-thalassemia, Duchene muscular dystrophy, severe combined immunodeficiency, or cystic fibrosis. The target nucleic acid segment, in some cases, is a portion of a nucleic acid from a gene with a mutation associated with a genetic disorder, from a gene whose overexpression is associated with a genetic disorder, from a gene associated with abnormal cellular growth resulting in a genetic disorder, or from a gene associated with abnormal cellular metabolism resulting in a genetic disorder. In some cases, the target nucleic acid segment is a portion of a nucleic acid from a genomic locus, a transcribed mRNA, or a reverse transcribed cDNA from a locus of at least one of: CFTR, FMR1, SMN1, ABCB11, ABCC8, ABCD1, ACAD9, ACADM, ACADVL, ACAT1, ACOX1, ACSF3, ADA, ADAMTS2, ADGRG1, AGA, AGL, AGPS, AGXT, AIRE, ALDH3A2, ALDOB, ALG6, ALMS1, ALPL, AMT, AQP2, ARG1, ARSA, ARSB, ASL, ASNS, ASPA, ASS1, ATM, ATP6V1B1, ATP7A, ATP7B, ATRX, BBS1, BBS10, BBS12, BBS2, BCKDHA, BCKDHB, BCS1L, BLM, BSND, CAPN3, CBS, CDH23, CEP290, CERKL, CHM, CHRNE, CIITA, CLN3, CLN5, CLN6, CLN8, CLRN1, CNGB3, COL27A1, COL4A3, COL4A4, COL4A5, COL7A1, CPS1, CPT1A, CPT2, CRB1, CTNS, CTSK, CYBA, CYBB, CYP11B1, CYP11B2, CYP17A1, CYP19A1, CYP27A1, DBT, DCLRE1C, DHCR7, DHDDS, DLD, DMD, DNAH5, DNAI1, DNAI2, DYSF, EDA, EIF2B5, EMD, ERCC6, ERCC8, ESCO2, ETFA, ETFDH, ETHE1, EVC, EVC2, EYS, F9, FAH, FAM161A, FANCA, FANCC, FANCG, FH, FKRP, FKTN, G6PC, GAA, GALC, GALK1, GALT, GAMT, GBA, GBE1, GCDH, GFM1, GJB1, GJB2, GLA, GLB1, GLDC, GLE1, GNE, GNPTAB, GNPTG, GNS, GRHPR, HADHA, HAX1, HBA1,, HBA2, HBB, HEXA, HEXB, HGSNAT, HLCS, HMGCL, HOGA1, HPS1, HPS3, HSD17B4, HSD3B2, HYAL1, HYLS1, IDS, IDUA, IKBKAP, IL2RG, IVD, KCNJ11, LAMA2, LAMA3, LAMB3, LAMC2, LCA5, LDLR, LDLRAP1, LHX3, LIFR, LIPA, LOXHD1, LPL, LRPPRC, MAN2B1, MCOLN1, MED17, MESP2, MFSD8, MKS1, MLC1, MMAA, MMAB, MMACHC, MMADHC, MPI, MPL, MPV17, MTHFR, MTM1, MTRR, MTTP, MUT, MYO7A, NAGLU, NAGS, NBN, NDRG1, NDUFAF5, NDUFS6, NEB, NPC1, NPC2, NPHS1, NPHS2, NR2E3, NTRK1, OAT, OPA3, OTC, PAH, PC, PCCA, PCCB, PCDH15, PDHA1, PDHB, PEX1, PEX10, PEX12, PEX2, PEX6, PEX7, PFKM, PHGDH, PKHD1, PMM2, POMGNT1, PPT1, PROP1, PRPS1, PSAP, PTS, PUS1, PYGM, RAB23, RAG2, RAPSN, RARS2, RDH12, RMRP, RPE65, RPGRIP1L, RS1, RTEL1, SACS, SAMHD1, SEPSECS, SGCA, SGCB, SGCG, SGSH, SLC12A3, SLC12A6, SLC17A5, SLC22A5, SLC25A13, SLC25A15, SLC26A2, SLC26A4, SLC35A3, SLC37A4, SLC39A4, SLC4A11, SLC6A8, SLC7A7, SMARCAL1, SMPD1, STAR, SUMF1, TAT, TCIRG1, TECPR2, TFR2, TGM1, TH, TMEM216, TPP1, TRMU, TSFM, TTPA, TYMP, USH1C, USH2A, VPS13A, VPS13B, VPS45, VRK1, VSX2, WNT10A, XPA, XPC, and ZFYVE26.

The method can comprise generating one or more droplets, aliquots, or subsamples from the sample. The one or more droplets, aliquots, or subsamples can correspond to a volumetric portion of the sample. The sample can be divided into 1, 2, 3, 4, 5, 6, 7, 8, 9, 10, 20, 30, 40, 50, 60, 70, 80, 90, 100, 200, 300, 400, 500, 600, 700, 800, 900, 1000, or more droplets, aliquots, or subsamples. In some embodiments, the sample is not divided into subsamples. As used herein, a droplet can refer to a volumetric portion of the sample, a partitioned sub-sample of the sample, and/or an aliquot of the sample. In some cases, the detection chamber is configured to receive and contact the plurality of droplets with at least one programmable nuclease disposed on a surface of said detection chamber. The at least one programmable nuclease can comprise a guide nucleic acid complexed with a programmable nuclease. In some cases, the programmable nuclease may comprise a CRISPR/Cas enzyme. In some cases, the guide nucleic acid may comprise a guide RNA. In some embodiments, the device may comprise a plurality of programmable nuclease complexes comprising different guide RNAs.

The method can comprise amplifying one or more targets within each droplet, aliquot, or subsample. Amplification of the one or more targets within each droplet can be performed in parallel and/or simultaneously for each droplet. Dividing the sample into a plurality of droplets can enhance a speed and/or an efficiency of the amplification process (e.g., a thermocycling process) since the droplets comprise a smaller volume of material than the bulk sample introduced. Amplifying the one or more targets within each individual droplet can also permit effective amplification of various target nucleic acids that cannot be amplified as efficiently in a bulk sample containing the various target nucleic acids if the bulk sample were to undergo a singular amplification process. In some embodiments, amplification is performed on the bulk sample without first dividing the sample into subsamples.The method can further comprise using a CRISPR-based or programmable nuclease-based detection module to detect one or more targets (e.g., target sequences or target nucleic acids) in the sample. In some cases, the sample can be divided into a plurality of droplets, aliquots, or subsamples to facilitate sample preparation.

Described herein are various methods of sample preparation and reagent storage. Any of the devices described herein may comprise one or more sample preparation reagents. Any of the devices described herein may comprise sample preparation reagents as dried reagents. Dried reagents may comprise solids and/or semi-solids. In certain instances, dried reagents may comprise lyophilized reagents. Any of the devices described herein may comprise one or more lyophilized reagents (e.g., amplification reagents, programmable nucleases, buffers, excipients, etc.). In certain instances, methods include sample lysis, concentration, and/or filtration, as claimed. In certain instances, methods include reconstitution of one or more lyophilized reagents. In some embodiments, lyophilized reagents may be in the form of lyophilized beads, spheres, and/or particulates. In some embodiments, the lyophilized bead, sphere, and/or particulate may comprise either single or multiple compounds. In some embodiments, the lyophilized bead, sphere, and/or particulate may be adjusted to various moisture levels or hygroscopy. In some embodiments, the lyophilized bead, sphere, and/or particulate may comprise assay internal standards. In some embodiments, the lyophilized bead, sphere, and/or particulate may have diameters between about 0.5 millimeters to about 5 millimeters in diameter.

The sample can be prepared before one or more targets are detected within the sample. The sample preparation steps described herein can process a crude sample to generate a pure or purer sample. Sample preparation can one or more physical or chemical processes, including, for example, nucleic acid purification, lysis, binding, washing, and/or eluting. In certain instances, sample preparation can comprise the following steps, in any order, including sample collection, nucleic acid purification, heat inactivation, and/or base/acid lysis.

In some embodiments, *nucleic acid purification* can be performed on the sample. Purification can comprise disrupting a biological matrix of a cell to release nucleic acids, denaturing structural proteins associated with the nucleic acids (nucleoproteins), inactivating nucleases that can degrade the isolated product (RNase and/or DNase), and/or removing contaminants (e.g., proteins, carbohydrates, lipids, biological or environmental elements, unwanted nucleic acids, and/or other cellular debris).

In some embodiments, *lysis* of a collected sample can be performed, as claimed. Lysis can be performed using a protease (e.g., a Proteinase K or PK enzyme). In some cases, a solution of reagents can be used to lyse the cells in the sample and release the nucleic acids so that they are accessible to the programmable nuclease. Active ingredients of the solution can be chaotropic agents, detergents, salts, and can be of high osmolality, ionic strength, and pH. Chaotropic agents or chaotropes are substances that disrupt the three-dimensional structure in macromolecules such as proteins, DNA, or RNA. One example protocol may comprise a 4 M guanidinium isothiocyanate, 25 mM sodium citrate.2H20, 0.5% (w/v) sodium lauryl sarcosinate, and 0.1 M β-mercaptoethanol), but numerous commercial buffers for different cellular targets can also be used. Alkaline buffers can also be used for cells with hard shells, particularly for environmental samples. Detergents such as sodium dodecyl sulphate (SDS) and cetyl trimethylammonium bromide (CTAB) can also be implemented to chemical lysis buffers. Cell lysis can also be performed by physical, mechanical, thermal or enzymatic means, in addition to chemically-induced cell lysis mentioned previously. In some cases, depending on the type of sample, nanoscale barbs, nanowires, acoustic generators, integrated lasers, integrated heaters, and/or microcapillary probes can be used to perform lysis.

In certain instances, *heat inactivation* can be performed on the sample. In some embodiments, a processed/lysed sample can undergo heat inactivation to inactivate, in the lysed sample, the proteins used during lysing (e.g., a PK enzyme or a lysing reagent). In some cases, a heating element integrated into the detection device can be used for heat-inactivation. The heating element can be powered by a battery or another source of thermal or electric energy that is integrated with the detection device.

In some cases, a target nucleic acid within the sample can undergo *amplification* before binding to a guide nucleic acid, for example a crRNA or sgRNA of a CRISPR enzyme, as claimed. The target nucleic acid within a purified sample can be amplified. In some instances, amplification can be accomplished using loop mediated amplification (LAMP), isothermal recombinase polymerase amplification (RPA), rolling circle amplification (RCA), nicking enzyme amplification reaction (NEAR), FASTR, and/or polymerase chain reaction (PCR). In some instances, digital droplet amplification can used. Such nucleic acid amplification of the sample can improve at least one of a sensitivity, specificity, or accuracy of the detection of the target nucleic acid. The reagents for nucleic acid amplification can comprise a recombinase, an oligonucleotide primer, a single-stranded DNA binding (SSB) protein, and a polymerase. The nucleic acid amplification can be transcription mediated amplification (TMA). Nucleic acid amplification can be helicase dependent amplification (HDA) or circular helicase dependent amplification (cHDA). In additional cases, nucleic acid amplification is strand displacement amplification (SDA). The nucleic acid amplification can be recombinase polymerase amplification (RPA). The nucleic acid amplification can be at least one of loop mediated amplification (LAMP) or the exponential amplification reaction (EXPAR). Nucleic acid amplification is, in some cases, by rolling circle amplification (RCA), ligase chain reaction (LCR), simple method amplifying RNA targets (SMART), single primer isothermal amplification (SPIA), multiple displacement amplification (MDA), nucleic acid sequence-based amplification (NASBA), hinge-initiated primer-dependent amplification of nucleic acids (HIP), nicking enzyme amplification reaction (NEAR), or improved multiple displacement amplification (IMDA). The nucleic acid amplification can be performed for no greater than 1, 2, 3, 4, 5, 6, 7, 8, 9, 10, 11, 12, 13, 14, 15, 16, 17, 18, 19, 20, 25, 30, 40, 50, or 60 minutes. Sometimes, the nucleic acid amplification is performed for from 1 to 60, from 5 to 55, from 10 to 50, from 15 to 45, from 20 to 40, or from 25 to 35 minutes. Sometimes, the nucleic acid amplification is performed for from 5 to 60, from 10 to 60, from 15 to 60, from 30 to 60, from 45 to 60, from 1 to 45, from 5 to 45, from 10 to 45, from 30 to 45, from 1 to 30, from 5 to 30, from 10 to 30, from 15 to 30, from 1 to 15, from 5 to 15, or from 10 to 15 minutes.

In some embodiments, amplification can comprise *thermocycling* of the sample. Thermocycling can be carried out for one or more droplets of the sample in parallel and/or independently in separate locations. This can be accomplished by methods such as (1) by holding droplets stationary in locations where a heating element is in close proximity to the droplet on one of the droplet sides and a heat sink element is in close proximity to the other side of the droplet, or (2) flowing the droplet through zones in a fluid channel where heat flows across it from a heating source to a heat sink. In some cases, one or more resistive heating elements can be used to perform thermocycling. In some cases, the thermocyling of the sample may comprise one or more reactions at different temperatures. In some cases, the reactions can include one or more reactions selected from an annealing reaction, a denaturation reaction, an extension reaction, reverse transcription reaction, and a detection reaction. In some cases, the thermocycling of the sample may comprise amplification of one or more nucleic acids in the sample.

In some cases, the annealing temperature of the reaction is performed at a temperature around 45°C to 75°C. In some embodiments, the annealing temperature may be at a temperature of about 45 °C, about 47 °C, about 48 °C, about 49 °C, about 50 °C, about 52 °C, about 54 °C, about 56 °C, about 58 °C, about 60 °C, about 62 °C, about 64 °C, about 66 °C, about 68 °C, about 70 °C, about 72 °C, about 74 °C, or about 76 °C.

In some cases, the denaturation temperature of the reaction is performed at a temperature around 90°C to about 110°C. In some embodiments, the denaturation temperature may be at a temperature of about 90°C, about 91°C, about 92°C, about 93°C, about 94°C, about 95°C, about 96°C, about 97°C, about 98°C, about 99°C, about 100°C, about 101°C, about 102°C, about 103°C, about 104°C, about 105°C, about 106°C, about 107°C, about 108°C, about 109°C, or about 110°C.

In some cases, the extension temperature of the reaction is performed at a temperature from around 55°C to about 85°C. In some embodiments, the extension temperature may be at a temperature of about 55°C, about 57°C, about 59°C, about 60°C, about 61°C, about 62°C, about 63°C, about 64°C, about 65°C, about 66°C, about 68°C, about 70°C, about 71°C, about 72°C, about 73°C, about 75°C, about 76°C, about 78°C, about 80°C, about 81°C, about 82°C, about 83°C, about 84°C, or about 85°C.

In some cases, the reverse transcription step of the reaction is performed at a temperature of around 45°C to about 75°C. In some embodiments, the reverse transcription may be at a temperature of about 45°C, about 47°C, about 48°C, about 49°C, about 50°C, about 52°C, about 54°C, about 55°C, about 57°C, about 59°C, about 60°C, about 61°C, about 63°C, about 65°C, about 66°C, about 68°C, about 70°C, about 72°C, about 73°C, or about 75°C.

In some cases, the detection step of the reaction is performed at a temperature of about 30°C to about 50°C. In some embodiments, the detection step may be at a temperature of about 30°C, about 32°C, about 33°C, about 34°C, about 35°C, about 36°C, about 37°C, about 38°C, about 39°C, about 40°C, about 42°C, about 43°C, about 44°C, about 45°C, about 46°C, or about 50°C.

Sometimes, the nucleic acid amplification reaction is performed at a temperature of around 20-45°C. The nucleic acid amplification reaction can be performed at a temperature no greater than 20°C, 25°C, 30°C, 35°C, 37°C, 40°C, 45°C, 50°C, 55°C, 60°C, or 65°C. The nucleic acid amplification reaction can be performed at a temperature of at least 20°C, 25°C, 30°C, 35°C, 37°C, 40°C, 45°C, 50°C, 55°C, 60°C, or 65°C. In some cases, the nucleic acid amplification reaction is performed at a temperature of from 20°C to 45°C, from 25°C to 40°C, from 30°C to 40°C, or from 35°C to 40°C. In some cases, the nucleic acid amplification reaction is performed at a temperature of from 45°C to 65°C, from 50°C to 65°C, from 55°C to 65°C, or from 60°C to 65°C. In some cases, the nucleic acid amplification reaction can be performed at a temperature that ranges from about 20 °C to 45 °C, from 25 °C to 45 °C, from 30 °C to 45 °C, from 35 °C to 45 °C, from 40 °C to 45 °C, from 20 °C to 37 °C, from 25 °C to 37 °C, from 30 °C to 37 °C, from 35 °C to 37 °C, from 20 °C to 30 °C, from 25 °C to 30 °C, from 20 °C to 25 °C, or from about 22 °C to 25 °C. In some cases, the nucleic acid amplification reaction can be performed at a temperature that ranges from about 40 °C to 65 °C, from 45 °C to 65 °C, from 50 °C to 65 °C, from 55 °C to 65 °C, from 60 °C to 65 °C, from 40 °C to 60 °C, from 45 °C to 60 °C, from 50 °C to 60 °C, from 55 °C to 60 °C, from 40 °C to 55 °C, from 45 °C to 55 °C, from 50 °C to 55 °C, from 40 °C to 50 °C, or from about 45 °C to 50 °C.

Additionally, target nucleic acid can optionally be amplified before binding to the guide nucleic acid (e.g., crRNA or sgRNA) of the programmable nuclease (e.g., CRISPR enzyme). This amplification can be PCR amplification or isothermal amplification. This nucleic acid amplification of the sample can improve at least one of sensitivity, specificity, or accuracy of the detection the target nucleic acid. The reagents for nucleic acid amplification can comprise a recombinase, a oligonucleotide primer, a single-stranded DNA binding (SSB) protein, and a polymerase. The nucleic acid amplification can be transcription mediated amplification (TMA). Nucleic acid amplification can be helicase dependent amplification (HDA) or circular helicase dependent amplification (cHDA). In additional cases, nucleic acid amplification is strand displacement amplification (SDA). The nucleic acid amplification can be recombinase polymerase amplification (RPA). The nucleic acid amplification can be at least one of loop mediated amplification (LAMP) or the exponential amplification reaction (EXPAR). Nucleic acid amplification is, in some cases, by rolling circle amplification (RCA), ligase chain reaction (LCR), simple method amplifying RNA targets (SMART), single primer isothermal amplification (SPIA), multiple displacement amplification (MDA), nucleic acid sequence based amplification (NASBA), hinge-initiated primer-dependent amplification of nucleic acids (HIP), nicking enzyme amplification reaction (NEAR), or improved multiple displacement amplification (IMDA). The nucleic acid amplification can be performed for no greater than 1, 2, 3, 4, 5, 6, 7, 8, 9, 10, 11, 12, 13, 14, 15, 16, 17, 18, 19, 20, 25, 30, 40, 50, or 60 minutes. Sometimes, the nucleic acid amplification reaction is performed at a temperature of around 20-45°C. Sometimes, the nucleic acid amplification reaction is performed at a temperature of around 45-65 °C. The nucleic acid amplification reaction can be performed at a temperature no greater than 20°C, 25°C, 30°C, 35°C, 37°C, 40°C, 45°C, 50°C, 55°C, 60°C, or 65°C. The nucleic acid amplification reaction can be performed at a temperature of at least 20°C, 25°C, 30°C, 35°C, 37°C, 40°C, 45°C, 50°C, 55°C, 60°C, or 65°C.

Devices of the present disclosure can be configured to perform *droplet digitization* or droplet generation. Droplet digitization or generation can comprise splitting a volume of the sample into multiple droplets, aliquots, or subsamples. The sample can have a volume that ranges from about 10 microliters to about 500 microliters. The plurality of droplets, aliquots, or subsamples can have a volume that ranges from about 0.01 microliters to about 100 microliters. The plurality of droplets, aliquots, or subsamples can have a same or substantially similar volume. In some cases, the plurality of droplets, aliquots, or subsamples can have different volumes. In some cases, the droplets, aliquots, or subsamples can be generated using a physical filter or one or more movable mechanisms (e.g., valves, etc.). In some cases, each droplet of the sample can undergo one or more sample preparation steps (e.g., nucleic acid purification, lysis, heat inactivation, amplification, etc.) independently and/or in parallel while the droplets are physically constrained or thermally isolated between two movable mechanisms.

The sample used for phenotyping testing can comprise at least one target nucleic acid segment that can bind to a guide nucleic acid of the reagents described herein. The target nucleic acid segment, in some cases, is a portion of a nucleic acid from a gene associated with a phenotypic trait.

The sample used for genotyping testing can comprise at least one target nucleic acid segment that can bind to a guide nucleic acid of the reagents described herein. The target nucleic acid segment, in some cases, is a portion of a nucleic acid from a gene associated with a genotype.

The sample used for ancestral testing can comprise at least one target nucleic acid segment that can bind to a guide nucleic acid of the reagents described herein. The target nucleic acid segment, in some cases, is a portion of a nucleic acid from a gene associated with a geographic region of origin or ethnic group.

The sample can be used for identifying a disease status. For example, a sample is any sample described herein, and is obtained from a subject for use in identifying a disease status of a subject. The disease can be a cancer or genetic disorder. Sometimes, a method may comprise obtaining a serum sample from a subject; and identifying a disease status of the subject. Often, the disease status is prostate disease status. In any of the embodiments described herein, the device can be configured for asymptomatic, pre-symptomatic, and/or symptomatic diagnostic applications, irrespective of immunity. In any of the embodiments described herein, the device can be configured to perform one or more serological assays on a sample (e.g., a sample comprising blood).

In some embodiments, the sample can be used to identify a mutation in a target nucleic acid of a plant or of a bacteria, virus, or microbe associated with a plant or soil. The devices and methods of the present disclosure can be used to identify a mutation of a target nucleic acid that affects the expression of a gene. A mutation that affects the expression of gene can be a mutation of a target nucleic acid within the gene, a mutation of a target nucleic acid comprising RNA associated with the expression of a gene, or a target nucleic acid comprising a mutation of a nucleic acid associated with regulation of expression of a gene, such as an RNA or a promoter, enhancer, or repressor of the gene. Often, the mutation is a single nucleotide mutation.

In some instances, the target nucleic acid is a single stranded nucleic acid. Alternatively, or in combination, the target nucleic acid is a double stranded nucleic acid and is prepared into single stranded nucleic acids before or upon contacting the reagents. The target nucleic acid can be a RNA, DNA, synthetic nucleic acids, or nucleic acids found in biological or environmental samples. The target nucleic acids include but are not limited to mRNA, rRNA, tRNA, non-coding RNA, long non-coding RNA, and microRNA (miRNA). In some cases, the target nucleic acid is mRNA. In some cases, the target nucleic acid is from a virus, a parasite, or a bacterium described herein. In some cases, the target nucleic acid is transcribed from a gene as described herein.

A number of target nucleic acids are consistent with the systems and methods disclosed herein. Some methods described herein can detect a target nucleic acid present in the sample in various concentrations or amounts as a target nucleic acid population. In some cases, the sample has at least 2 target nucleic acids. In some cases, the sample has at least 3, 5, 10, 20, 30, 40, 50, 100, 200, 300, 400, 500, 600, 700, 800, 900, 1000, 2000, 3000, 4000, 5000, 6000, 7000, 8000, 9000, or 10000 target nucleic acids. In some cases, the sample has from 1 to 10,000, from 100 to 8000, from 400 to 6000, from 500 to 5000, from 1000 to 4000, or from 2000 to 3000 target nucleic acids. In some cases, the sample has from 100 to 9500, from 100 to 9000, from 100 to 8500, from 100 to 8000, from 100 to 7500, from 100 to 7000, from 100 to 6500, from 100 to 6000, from 100 to 5500, from 100 to 5000, from 250 to 9500, from 250 to 9000, from 250 to 8500, from 250 to 8000, from 250 to 7500, from 250 to 7000, from 250 to 6500, from 250 to 6000, from 250 to 5500, from 250 to 5000, from 2500 to 9500, from 2500 to 9000, from 2500 to 8500, from 2500 to 8000, from 2500 to 7500, from 2500 to 7000, from 2500 to 6500, from 2500 to 6000, from 2500 to 5500, or from 2500 to 5000 target nucleic acids. In some cases, the method detects target nucleic acid present at least at one copy per 10¹ non-target nucleic acids, 10² non-target nucleic acids, 10³ non-target nucleic acids, 10⁴ non-target nucleic acids, f, 10⁶ non-target nucleic acids, 10⁷ non-target nucleic acids, 10⁸ non-target nucleic acids, 10⁹ non-target nucleic acids, or 10¹⁰ non-target nucleic acids.

In some cases, the target nucleic acid is present at less than 1 µg/ml in a sample. In some cases, the target nucleic acid is present at less than 100 ng/ml in a sample. In some cases, the target nucleic acid is present at less than 10 ng/ml in a sample. In some cases, the target nucleic acid is present at less than 1 ng/ml in a sample. In some cases, the target nucleic acid is present at less than 100 pg/ml in a sample. In some cases, the target nucleic acid is present at less than 10 pg/ml in a sample. In some cases, the target nucleic acid is present at less than 1 pg/ml in a sample. In some cases, the target nucleic acid is present at less than 100 fg/ml in a sample. In some cases, the target nucleic acid is present at less than 10 fg/ml in a sample. In some cases, the target nucleic acid is present at less than 1 fg/ml in a sample.

A number of target nucleic acid populations are consistent with the systems and methods disclosed herein. Some methods described herein can be implemented to detect two or more target nucleic acid populations present in the sample in various concentrations or amounts. In some cases, the sample has at least 2 target nucleic acid populations. In some cases, the sample has at least 3, 4, 5, 6, 7, 8, 9, 10, 20, 30, 40, or 50 target nucleic acid populations. In some cases, the sample has from 3 to 50, from 5 to 40, or from 10 to 25 target nucleic acid populations. In some cases, the sample has from 2 to 50, from 5 to 50, from 10 to 50, from 2 to 25, from 3 to 25, from 4 to 25, from 5 to 25, from 10 to 25, from 2 to 20, from 3 to 20, from 4 to 20, from 5 to 20, from 10 to 20, from 2 to 10, from 3 to 10, from 4 to 10, from 5 to 10, from 6 to 10, from 7 to 10, from 8 to 10, or from 9 to 10 target nucleic acid populations. In some cases, the methods of the present disclosure can be implemented to detect target nucleic acid populations that are present at least at one copy per 10¹ non-target nucleic acids, 10² non-target nucleic acids, 10³ non-target nucleic acids, 10⁴ non-target nucleic acids, 10⁵ non-target nucleic acids, 10⁶ non-target nucleic acids, 10⁷ non-target nucleic acids, 10⁸ non-target nucleic acids, 10⁹ non-target nucleic acids, or 10¹⁰ non-target nucleic acids. The target nucleic acid populations can be present at different concentrations or amounts in the sample.

The compositions and methods of the present disclosure are compatible with intact and damaged nucleic acids. The target nucleic acid may be fragmented prior to amplification and/or detection (e.g., by a DETECTR assay). The target nucleic acid may comprise ordered or random fragmentation. The target nucleic acid may also comprise damage, such as oxidation or cross-linking. The target nucleic acid may comprise chemical, light-induced, and/or enzymatic cleavage. Conversely, the target nucleic acid may be unfragmented. A target nucleic acid sequence may be embedded within a longer nucleic acid. For example, a target nucleic acid may be a gene within an intact chromosome. A target nucleic acid may comprise a chemical modification, such as acetylation or methylation. A target nucleic acid may comprise a defined secondary, tertiary, or quaternary structure. A target nucleic acid may be complexed with another species, such as a histone.

Any of the above disclosed samples are consistent with the systems, assays, and programmable nucleases disclosed herein and can be used as a companion diagnostic with any of the diseases disclosed herein (e.g., a coronavirus infection), or can be used in reagent kits, point-of-care diagnostics, or over-the-counter diagnostics.

### Reagents

A number of reagents are consistent with the methods, reagents, and devices disclosed herein. Many aspects of the present disclosure provide buffers, systems, and compositions that enable fast kinetics (e.g., within 25%, 50%, 75%, or 90% of the fastest reported rate for a reaction at a given temperature, or coming to completion within one hour) for multiple types of reactions or processes. Such buffers, systems, and compositions enable multiple reactions to be performed on a sample without intervening sample transfers, buffer exchanges, or reagent removal. In some cases, reagents (e.g., enzymes) for multiple reactions may be provided in a single solution. Such cases may require no more than sample addition to determine whether a nucleic acid (e.g., a particular nucleic acid sequence) is present in the sample.

These reagents are compatible with the samples, methods, and devices as described herein for detection of an ailment, such as a disease. The reagents described herein for detecting a disease, such as coronavirus, comprise multiple guide nucleic acids, each guide nucleic acid targeting a target nucleic acid segment indicative of the disease. Each guide nucleic acid binds to the target nucleic acid comprising a segment of a nucleic acid sequence (e.g., a nucleic acid from coronavirus) as described herein. Each guide nucleic acid can bind to the target nucleic acid comprising a portion of a nucleic acid (e.g., a target nucleic acid from coronavirus) as described herein and further comprising a mutation, such as a single nucleotide polymorphism (SNP), that can confer resistance to a treatment, such as antibiotic treatment. Each guide nucleic acid binds to the target nucleic acid comprising a portion of a nucleic acid. Each guide nucleic acid is complementary to a target nucleic acid. Often the guide nucleic acid binds specifically to the target nucleic acid. The target nucleic acid may be a RNA, DNA, or synthetic nucleic acids.

Disclosed herein are methods of assaying for a plurality of target nucleic acids (e.g., a plurality of nucleic acids from coronavirus) as described herein. For example, a method of assaying for a plurality of target nucleic acids in a sample comprises contacting the sample to a complex comprising a plurality guide nucleic acid sequences, each guide nucleic acid sequence comprising a segment that is reverse complementary to a segment of the target nucleic acid, and programmable nucleases that exhibits sequence independent cleavage upon forming a complex comprising the segment of the guide nucleic acid binding to the segment of the target nucleic acid; and assaying for a signal indicating cleavage of at least some protein-nucleic acids of a population of protein-nucleic acids, wherein the signal indicates a presence of one or more target nucleic acid of the plurality of target nucleic acids in the sample and wherein absence of the signal indicates an absence of the target nucleic acids in the sample. As another example, a method of assaying for a target nucleic acid in a sample, for example, comprises: a) contacting the sample to a plurality of complexes, each complex comprising a guide nucleic acid comprising a segment that is reverse complementary to a segment of the target nucleic acid and a programmable nuclease that exhibits sequence independent cleavage upon forming a complex comprising the segment of the guide nucleic acid binding to the segment of the target nucleic acid; b) contacting the plurality of complexes to a substrate; c) contacting the substrate to a reagent that differentially reacts with a cleaved substrate; and d) assaying for a signal indicating cleavage of the substrate, wherein the signal indicates a presence of the target nucleic acid in the sample and wherein absence of the signal indicates an absence of the target nucleic acid in the sample. Often, the substrate is an enzyme-nucleic acid. Sometimes, the substrate is an enzyme substrate-nucleic acid.

A programmable nuclease can comprise a programmable nuclease capable of being activated when complexed with a guide nucleic acid and target nucleic acid. The programmable nuclease can become activated after binding of a guide nucleic acid with a target nucleic acid, in which the activated programmable nuclease can cleave the target nucleic acid and can have trans cleavage activity. Trans cleavage activity can be non-specific cleavage of nearby single-stranded nucleic acids by the activated programmable nuclease, such as trans cleavage of reporters with a detection moiety. Once the reporter is cleaved by the activated programmable nuclease, the detection moiety can be released from the reporter and can generate a signal. A signal can be a calorimetric, potentiometric, amperometric, optical (e.g., fluorescent, colorimetric, etc.), or piezo-electric signal. Often, the signal is present prior to reporter cleavage and changes upon reporter cleavage. Sometimes, the signal is absent prior to reporter cleavage and is present upon reporter cleavage. The detectable moiety generating the detectable signal can be immobilized on a support medium for detection. The programmable nuclease can be a CRISPR-Cas (clustered regularly interspaced short palindromic repeats - CRISPR associated) nucleoprotein complex with trans cleavage activity, which can be activated by binding of a guide nucleic acid with a target nucleic acid. The CRISPR-Cas nucleoprotein complex can comprise a Cas protein (also referred to as a Cas nuclease) complexed with a guide nucleic acid, which can also be referred to as CRISPR enzyme. A guide nucleic acid can be a CRISPR RNA (crRNA). Sometimes, a guide nucleic acid comprises a crRNA and a trans-activating crRNA (tracrRNA).

The term, "guide nucleic acid," as used herein refers to a nucleic acid comprising: a first nucleotide sequence that hybridizes to a target nucleic acid; and a second nucleotide sequence that capable of being non-covalently bound by an effector protein. The first sequence may be referred to herein as a spacer sequence. The second sequence may be referred to herein as a repeat sequence. In some embodiments, the first sequence is located 5' of the second nucleotide sequence. In some embodiments, the first sequence is located 3' of the second nucleotide sequence. Guide nucleic acids, when complexed with an effector protein, may bring the effector protein into proximity of a target nucleic acid. Sufficient conditions for hybridization of a guide nucleic acid to a target nucleic acid and/or for binding of a guide nucleic acid to an effector protein include *in vivo* physiological conditions of a desired cell type or *in vitro* conditions sufficient for assaying catalytic activity of a protein, polypeptide or peptide described herein, such as the nuclease activity of an effector protein. Guide nucleic acids may comprise DNA, RNA, or a combination thereof *(e.g.,* RNA with a thymine base). Guide nucleic acids may include a chemically modified nucleobase or phosphate backbone. Guide nucleic acids may be referred to herein as a guide RNA (gRNA). However, a guide RNA is not limited to ribonucleotides, but may comprise deoxyribonucleotides and other chemically modified nucleotides.

A guide nucleic acid may comprise a CRISPR RNA (crRNA), a short-complementarity untranslated RNA (scoutRNA), an associated trans-activating RNA (tracrRNA) or a combination thereof. The combination of a crRNA with a tracrRNA may be referred to herein as a single guide RNA (sgRNA), wherein the crRNA and the tracrRNA are covalently linked. In some embodiments, the crRNA and tracrRNA are linked by a phosphodiester bond. In some instances, the crRNA and tracrRNA are linked by one or more linked nucleotides. A guide nucleic acid may comprise a naturally occurring guide nucleic acid. A guide nucleic acid may comprise a non-naturally occurring guide nucleic acid, including a guide nucleic acid that is designed to contain a chemical or biochemical modification.

Guide nucleic acids and portions thereof may be found in or identified from a CRISPR array present in the genome of a host organism. A crRNA may be the product of processing of a longer precursor CRISPR RNA (pre-crRNA) transcribed from the CRISPR array by cleavage of the pre-crRNA within each direct repeat sequence to afford shorter, mature crRNAs. A crRNA may be generated by a variety of mechanisms, including the use of dedicated endonucleases (e.g., Cas6 or Cas5d in Type I and III systems), coupling of a host endonuclease *(e.g.,* RNase III) with tracrRNA (Type II systems), or a ribonuclease activity endogenous to the effector protein itself *(e.g.,* Cpfl, from Type V systems). A crRNA may also be specifically generated outside of processing of a pre-crRNA and individually contacted to an effector protein *in vivo* or *in vitro.*

The CRISPR/Cas system used to detect modified target nucleic acids can comprise CRISPR RNAs (crRNAs), trans-activating crRNAs (tracrRNAs), Cas proteins, and reporters. The term, "CRISPR RNA (crRNA)," as used herein refers to a nucleic acid comprising a first sequence, often referred to as a "spacer sequence," that hybridizes to a target sequence of a target nucleic acid, and a second sequence that either a) hybridizes to a portion of a tracrRNA or b) is capable of being non-covalently bound by an effector protein. In some embodiments, the crRNA is covalently linked to an additional nucleic acid (e.g., a tracrRNA), wherein the additional nucleic acid interacts with the effector protein.

A guide nucleic acid can comprise a sequence that is reverse complementary to the sequence of a target nucleic acid. A guide nucleic acid can be a crRNA. Sometimes, a guide nucleic acid comprises a crRNA and tracrRNA. The guide nucleic acid can bind specifically to the target nucleic acid. In some cases, the guide nucleic acid is not naturally occurring and made by artificial combination of otherwise separate segments of sequence. Often, the artificial combination is performed by chemical synthesis, by genetic engineering techniques, or by the artificial manipulation of isolated segments of nucleic acids. The target nucleic acid can be designed and made to provide desired functions. In some cases, the targeting region of a guide nucleic acid is 20 nucleotides in length. The targeting region of the guide nucleic acid may have a length of at least 10, 11, 12, 13, 14, 15, 16, 17, 18, 19, 20, 21, 22, 23, 24, 25, 26, 27, 28, 29, or 30 nucleotides in length. In some instances, the targeting region of the guide nucleic acid is 10, 11, 12, 13, 14, 15, 16, 17, 18, 19, 20, 21, 22, 23, 24, 25, 26, 27, 28, 29, or 30 nucleotides in length. In some cases, the targeting region of a guide nucleic acid has a length from exactly or about 12 nucleotides (nt) to about 80 nt, from about 12 nt to about 50 nt, from about 12 nt to about 45 nt, from about 12 nt to about 40 nt, from about 12 nt to about 35 nt, from about 12 nt to about 30 nt, from about 12 nt to about 25 nt, from about 12 nt to about 20 nt, from about 12 nt to about 19 nt, from about 19 nt to about 20 nt, from about 19 nt to about 25 nt, from about 19 nt to about 30 nt, from about 19 nt to about 35 nt, from about 19 nt to about 40 nt, from about 19 nt to about 45 nt, from about 19 nt to about 50 nt, from about 19 nt to about 60 nt, from about 20 nt to about 25 nt, from about 20 nt to about 30 nt, from about 20 nt to about 35 nt, from about 20 nt to about 40 nt, from about 20 nt to about 45 nt, from about 20 nt to about 50 nt, or from about 20 nt to about 60 nt. It is understood that the sequence of a polynucleotide need not be 100% complementary to that of its target nucleic acid to be specifically hybridizable or bind specifically. The guide nucleic acid can have a sequence comprising at least one uracil in a region from nucleic acid residue 5 to 20 that is reverse complementary to a modification variable region in the target nucleic acid. The guide nucleic acid, in some cases, has a sequence comprising at least one uracil in a region from nucleic acid residue 5 to 9, 10 to 14, or 15 to 20 that is reverse complementary to a modification variable region in the target nucleic acid. The guide nucleic acid can have a sequence comprising at least one uracil in a region from nucleic acid residue 5 to 20 that is reverse complementary to a methylation variable region in the target nucleic acid. The guide nucleic acid, in some cases, has a sequence comprising at least one uracil in a region from nucleic acid residue 5 to 9, 10 to 14, or 15 to 20 that is reverse complementary to a methylation variable region in the target nucleic acid.

The guide nucleic acid can be selected from a group of guide nucleic acids that have been tiled against the nucleic acid sequence of a strain of an infection or genomic locus of interest. The guide nucleic acid can be selected from a group of guide nucleic acids that have been tiled against the nucleic acid sequence of a strain of coronavirus. Often, guide nucleic acids that are tiled against the nucleic acid of a strain of an infection or genomic locus of interest can be pooled for use in a method described herein. Often, these guide nucleic acids are pooled for detecting a target nucleic acid in a single assay. The pooling of guide nucleic acids that are tiled against a single target nucleic acid can enhance the detection of the target nucleic using the methods described herein. The pooling of guide nucleic acids that are tiled against a single target nucleic acid can ensure broad coverage of the target nucleic acid within a single reaction using the methods described herein. The tiling, for example, is sequential along the target nucleic acid. Sometimes, the tiling is overlapping along the target nucleic acid. In some instances, the tiling comprises gaps between the tiled guide nucleic acids along the target nucleic acid. In some instances the tiling of the guide nucleic acids is non-sequential. Often, a method for detecting a target nucleic acid comprises contacting a target nucleic acid to a pool of guide nucleic acids and a programmable nuclease, wherein a guide nucleic acid of the pool of guide nucleic acids has a sequence selected from a group of tiled guide nucleic acid that correspond to nucleic acids of a target nucleic acid; and assaying for a signal produce by cleavage of at least some reporters of a population of reporters. Pooling of guide nucleic acids can ensure broad spectrum identification, or broad coverage, of a target species within a single reaction. This can be particularly helpful in diseases or indications, like sepsis, that may be caused by multiple organisms.

Described herein are reagents comprising a programmable nuclease capable of being activated when complexed with the guide nucleic acid and the target nucleic acid segment. A programmable nuclease can be capable of being activated when complexed with a guide nucleic acid and the target sequence. The programmable nuclease can be activated upon binding of the guide nucleic acid to its target nucleic acid and degrades non-specifically nucleic acid in its environment. The programmable nuclease has trans cleavage activity once activated. A programmable nuclease can be a Cas protein (also referred to, interchangeably, as a Cas nuclease). A crRNA and Cas protein can form a CRISPR enzyme.

Trans cleavage activity can be non-specific cleavage of nearby single-stranded nucleic acids by the activated programmable nuclease, such as trans cleavage of reporters with a detection moiety. Once the reporter is cleaved by the activated programmable nuclease, the detection moiety can be released from the reporter and can generate a signal. A signal can be a calorimetric, potentiometric, amperometric, optical (e.g., fluorescent, colorimetric, etc.), or piezo-electric signal. Often, the signal is present prior to reporter cleavage and changes upon reporter cleavage. Sometimes, the signal is absent prior to reporter cleavage and is present upon reporter cleavage. The detection moiety capable of generating the detectable signal can be immobilized on a support medium for detection. The programmable nuclease can be a CRISPR-Cas (clustered regularly interspaced short palindromic repeats - CRISPR associated) nucleoprotein complex with trans cleavage activity, which can be activated by binding of a guide nucleic acid with a target nucleic acid. The CRISPR-Cas nucleoprotein complex can comprise a Cas protein (also referred to as a Cas nuclease) complexed with a guide nucleic acid, which can also be referred to as CRISPR enzyme. A guide nucleic acid can be a CRISPR RNA (crRNA). Sometimes, a guide nucleic acid may comprise a crRNA and a trans-activating crRNA (tracrRNA).

The devices, systems, fluidic devices, kits, and methods for detecting the presence of a target nucleic acid in a sample described herein may comprise a generation of a signal indicative of the presence or absence of the target nucleic acid in the sample. The generation of a signal indicative of the presence or absence of the target nucleic acid in the sample as described herein is compatible with the methods and devices described herein (e.g., pneumatic valve devices, sliding valve devices, rotating valve devices, and lateral flow devices) and may result from the use of compositions disclosed herein (e.g. programmable nucleases, guide nucleic acids, reagents for in vitro transcription, reagents for amplification, reagents for reverse transcription, reporters, or any combination thereof) to carry out highly efficient, rapid, and accurate reactions for detecting whether a target nucleic acid is present in a sample (e.g., DETECTR reactions). As disclosed herein, in some embodiments, detecting the presence or absence of a target nucleic acid of interest involves measuring a signal emitted from a detection moiety present in a reporter, after cleavage of the reporter by an activated programmable nuclease. Alternatively, or in combination, in some embodiments, detecting the presence or absence of a target nucleic acid of interest involves measuring a signal emitted from a conjugate bound to a detection moiety present in a reporter, after cleavage of the reporter by an activated programmable nuclease. The conjugates may comprise a nanoparticle, a gold nanoparticle, a latex nanoparticle, a quantum dot, a chemiluminescent nanoparticle, a carbon nanoparticle, a selenium nanoparticle, a fluorescent nanoparticle, a liposome, or a dendrimer. The surface of the conjugate may be coated by a conjugate binding molecule that binds to the detection moiety or another affinity molecule of the cleaved detector molecule as described herein. Thus, the detecting steps disclosed herein involve indirectly (e.g., via a reporter) measuring the presence of a target nucleic acid, quantifying how much of the target nucleic acid is present, or, measuring a signal indicating that the target nucleic acid is absent in a sample. In some embodiments, a signal is generated upon cleavage of the reporter by the programmable nuclease. In other embodiments, the signal changes upon cleavage of the reporter by the programmable nuclease. In other embodiments, a signal may be present in the absence of reporter cleavage and disappear upon cleavage of the target nucleic acid by the programmable nuclease. For example, a signal may be produced in a microfluidic device or lateral flow device after contacting a sample with a composition comprising a programmable nuclease.

"Percent identity" and "% identity" can refer to the extent to which two sequences (nucleotide or amino acid) have the same residue at the same positions in an alignment. For example, "an amino acid sequence is X% identical to SEQ ID NO: Y" can refer to % identity of the amino acid sequence to SEQ ID NO: Y and is elaborated as X% of residues in the amino acid sequence are identical to the residues of sequence disclosed in SEQ ID NO: Y. Generally, computer programs can be employed for such calculations. Illustrative programs that compare and align pairs of sequences, include ALIGN (Myers and Miller, Comput Appl Biosci. 1988 Mar;4(1):11-7), FASTA (Pearson and Lipman, Proc Natl Acad Sci U S A. 1988 Apr;85(8):2444-8; Pearson, Methods Enzymol. 1990;183:63-98) and gapped BLAST (Altschul et al., Nucleic Acids Res. 1997 Sep 1;25(17):3389-40), BLASTP, BLASTN, or GCG (Devereux et al., Nucleic Acids Res. 1984 Jan 11;12(1 Pt 1):387-95).

An effector protein may be brought into proximity of a target nucleic acid in the presence of a guide nucleic acid when the guide nucleic acid includes a nucleotide sequence that is complementary with a target sequence in the target nucleic acid. The ability of an effector protein to detect a target nucleic acid and cleave a reporter may be dependent upon the effector protein being bound to a guide nucleic acid and the guide nucleic acid being hybridized to a target nucleic acid. An effector protein may also recognize a protospacer adjacent motif (PAM) sequence present in the target nucleic acid, which may direct the modification activity of the effector protein. An effector protein may modify a nucleic acid by *cis* cleavage or *trans* cleavage. An effector protein may be a CRISPR-associated ("Cas") protein. An effector protein may function as a single protein, including a single protein that is capable of binding to a guide nucleic acid and modifying a target nucleic acid. Alternatively, an effector protein may function as part of a multiprotein complex, including, for example, a complex having two or more effector proteins, including two or more of the same effector proteins (e.g., dimer or multimer). An effector protein, when functioning in a multiprotein complex, may have only one functional activity (e.g., binding to a guide nucleic acid), while other effector proteins present in the multiprotein complex are capable of the other functional activity (e.g., modifying a target nucleic acid). An effector protein may be a modified effector protein having reduced modification activity *(e.g.,* a catalytically defective effector protein) or no modification activity *(e.g., a* catalytically inactive effector protein). Accordingly, an effector protein as used herein encompasses a modified or programmable nuclease that does not have nuclease activity.

Several programmable nucleases are consistent with the methods and devices of the present disclosure. For example, CRISPR/Cas enzymes are programmable nucleases used in the methods and systems disclosed herein. CRISPR/Cas enzymes can include any of the known Classes and Types of CRISPR/Cas enzymes. Programmable nucleases disclosed herein include Class 1 CRISPR/Cas enzymes, such as the Type I, Type IV, or Type III CRISPR/Cas enzymes. Programmable nucleases disclosed herein also include the Class 2 CRISPR/Cas enzymes, such as the Type II, Type V, and Type VI CRISPR/Cas enzymes. Preferable programmable nucleases included in the several assays disclosed herein (e.g., for assaying for coronavirus in a device, such as a microfluidic device or a lateral flow assay) and methods of use thereof include a Type V or Type VI CRISPR/Cas enzyme.

The programmable nuclease system used to detect modified target nucleic acids can comprise CRISPR RNAs (crRNAs), trans-activating crRNAs (tracrRNAs), Cas proteins, and reporters.

Described herein are reagents comprising a programmable nuclease capable of being activated when complexed with the guide nucleic acid and the target nucleic acid segment or portion. A programmable nuclease can be capable of being activated when complexed with a guide nucleic acid and the target sequence. The programmable nuclease can be activated upon binding of the guide nucleic acid to its target nucleic acid and degrades non-specifically nucleic acid in its environment. The programmable nuclease has trans cleavage activity once activated. A programmable nuclease can be a Cas protein (also referred to, interchangeably, as a Cas nuclease). A crRNA and Cas protein can form a CRISPR enzyme.

Several programmable nucleases are consistent with the methods and devices of the present disclosure. For example, CRISPR/Cas enzymes are programmable nucleases used in the methods and systems disclosed herein.

The systems and methods of the present disclosure can be implemented using a device that is compatible with a plurality of programmable nucleases. The device can comprise a plurality of programmable nucleases and one or more corresponding guide nucleic acids. In some cases, the plurality of programmable nucleases are complexed with one or more corresponding guide nucleic acids. The plurality of programmable nucleases (and guide nucleic acids) can be the same. Alternatively, the plurality of programmable nuclease complexes can be different. For example, the plurality of programmable nuclease complexes can comprise different programmable nucleases and/or different guide nucleic acids associated with the programmable nucleases.

As used herein, a programmable nuclease generally refers to any enzyme that can cleave nucleic acid. The programmable nuclease can be any enzyme that can be or has been designed, modified, or engineered by human contribution so that the enzyme targets or cleaves the nucleic acid in a sequence-specific manner. Programmable nucleases can include, for example, zinc-finger nucleases (ZFNs), transcription activator-like effector nucleases (TALENs), and/or RNA-guided nucleases such as the bacterial clustered regularly interspaced short palindromic repeat (CRISPR)-Cas (CRISPR-associated) nucleases or Cpfl. Programmable nucleases can also include, for example, PfAgo and/or NgAgo.

ZFNs can cut genetic material in a sequence- specific matter and can be designed, or programmed, to target specific viral targets. A ZFN is composed of two domains: a DNA-binding zinc-finger protein linked to the Fokl nuclease domain. The DNA-binding zinc-finger protein is fused with the non-specific Fokl cleave domain to create ZFNs. The protein will typically dimerize for activity. Two ZFN monomers form an active nuclease; each monomer binds to adjacent half- sites on the target. The sequence specificity of ZFNs is determined by ZFPs. Each zinc-finger recognizes a 3-bp DNA sequence, and 3-6 zinc-fingers are used to generate a single ZFN subunit that binds to DNA sequences of 9-18 bp. The DNA-binding specificities of zinc-fingers is altered by mutagenesis. New ZFPs are programmed by modular assembly of pre-characterized zinc fingers.

Transcription activator-like effector nucleases (TALENs) can cut genetic material in a sequence-specific matter and can be designed, or programmed, to target specific viral targets. TALENs contain the Fokl nuclease domain at their carboxyl termini and a class of DNA binding domains known as transcription activator- like effectors (TALEs). TALENs are composed of tandem arrays of 33-35 amino acid repeats, each of which recognizes a single base-pair in the major groove of target viral DNA. The nucleotide specificity of a domain comes from the two amino acids at positions 12 and 13 where Asn-Asn, Asn-Ile, His-Asp and Asn-Gly recognize guanine, adenine, cytosine and thymine, respectively. That pattern allows one to program TALENs to target various nucleic acids.

The programmable nuclease can comprise any type of human engineered enzymes. Alternatively, the programmable nuclease can comprise CRISPR enzymes derived from naturally occurring bacteria or phage. A programmable nuclease can be a Cas protein (also referred to, interchangeably, as a Cas nuclease). A crRNA and Cas protein can form a CRISPR enzyme. The programmable nuclease can be a CRISPR-Cas (clustered regularly interspaced short palindromic repeats - CRISPR associated) nucleoprotein complex with trans cleavage activity, which can be activated by binding of a guide nucleic acid with a target nucleic acid. The programmable nuclease can comprise one or more amino acid modifications. The programmable nuclease be a nuclease derived from a CRISPR-Cas system. The programmable nuclease can be a nuclease derived from recombineering.

CRISPR/Cas enzymes can include any of the known Classes and Types of CRISPR/Cas enzymes. Programmable nucleases disclosed herein include Class 1 CRISPR/Cas enzymes, such as the Type I, Type IV, or Type III CRISPR/Cas enzymes. Programmable nucleases disclosed herein also include the Class 2 CRISPR/Cas enzymes, such as the Type II, Type V, and Type VI CRISPR/Cas enzymes. Preferable programmable nucleases included in the several devices disclosed herein (e.g., a microfluidic device such as a pneumatic valve device or a sliding valve device or a lateral flow assay) and methods of use thereof include a Type V or Type VI CRISPR/Cas enzyme.

In some embodiments, the Type V CRISPR/Cas enzyme is a programmable Cas12 nuclease. Type V CRISPR/Cas enzymes (e.g., Cas12 or Cas14) lack an HNH domain. A Cas12 nuclease of the present disclosure cleaves a nucleic acid via a single catalytic RuvC domain. The RuvC domain is within a nuclease, or "NUC" lobe of the protein, and the Cas12 nucleases further comprise a recognition, or "REC" lobe. The REC and NUC lobes are connected by a bridge helix and the Cas12 proteins additionally include two domains for PAM recognition termed the PAM interacting (PI) domain and the wedge (WED) domain. In some instances, a programmable Cas12 nuclease can be a Cas12a (also referred to as Cpf1) protein, a Cas12b protein, Cas12c protein, Cas12d protein, or a Cas12e protein.

In some embodiments, the programmable nuclease can be Cas13. Sometimes the Cas13 can be Cas13a, Cas13b, Cas13c, Cas13d, or Cas13e. In some cases, the programmable nuclease can be Mad7 or Mad2. In some cases, the programmable nuclease can be Cas12. Sometimes the Cas12 can be Cas12a, Cas12b, Cas12c, Cas12d, or Cas12e. In some cases, the Cas12 can be a Cas12 variant (SEQ ID NO: 28), which is a specific protein variant within the Cas12 protein family/classification). In some cases, the programmable nuclease can be Csm1, Cas9, C2c4, C2c8, C2c5, C2c10, C2c9, or CasZ. Sometimes, the Csm1 can also be also called smCms1, miCms1, obCms1, or suCms1. Sometimes Cas13a can also be also called C2c2. Sometimes CasZ can also be called Cas14a, Cas14b, Cas14c, Cas14d, Cas14e, Cas14f, Cas14g, or Cas14h. Sometimes, the programmable nuclease can be a type V CRISPR-Cas system. In some cases, the programmable nuclease can be a type VI CRISPR-Cas system. Sometimes the programmable nuclease can be a type III CRISPR-Cas system. Sometimes the programmable nuclease can be an engineered nuclease that is not from a naturally occurring CRISPR-Cas system. In some cases, the programmable nuclease can be from at least one of Leptotrichia shahii (Lsh), Listeria seeligeri (Lse), Leptotrichia buccalis (Lbu), Leptotrichia wadeu (Lwa), Rhodobacter capsulatus (Rca), Herbinix hemicellulosilytica (Hhe), Paludibacter propionicigenes (Ppr), Lachnospiraceae bacterium (Lba), [Eubacterium] rectale (Ere), Listeria newyorkensis (Lny), Clostridium aminophilum (Cam), Prevotella sp. (Psm), Capnocytophaga canimorsus (Cca, Lachnospiraceae bacterium (Lba), Bergeyella zoohelcum (Bzo), Prevotella intermedia (Pin), Prevotella buccae (Pbu), Alistipes sp. (Asp), Riemerella anatipestifer (Ran), Prevotella aurantiaca (Pau), Prevotella saccharolytica (Psa), Prevotella intermedia (Pin2), Capnocytophaga canimorsus (Cca), Porphyromonas gulae (Pgu), Prevotella sp. (Psp), Porphyromonas gingivalis (Pig), Prevotella intermedia (Pin3), Enterococcus italicus (Ei), Lactobacillus salivarius (Ls), or Thermus thermophilus (Tt). Sometimes the Cas13 is at least one of LbuCas13a, LwaCas13a, LbaCas13a, HheCas13a, PprCas13a, EreCas13a, CamCas13a, or LshCas13a. The trans cleavage activity of the CRISPR enzyme can be activated when the crRNA is complexed with the target nucleic acid. The trans cleavage activity of the CRISPR enzyme can be activated when the guide nucleic acid comprising a tracrRNA and crRNA are complexed with the target nucleic acid. The target nucleic acid can be RNA or DNA.

In some embodiments, a programmable nuclease as disclosed herein is an RNA-activated programmable RNA nuclease. In some embodiments, a programmable nuclease as disclosed herein is a DNA-activated programmable RNA nuclease. In some embodiments, a programmable nuclease is capable of being activated by a target RNA to initiate trans cleavage of an RNA reporter and is capable of being activated by a target DNA to initiate trans cleavage of an RNA reporter, such as a Type VI CRISPR/Cas enzyme (e.g., a Cas13 nuclease). For example, Cas13a of the present disclosure can be activated by a target RNA to initiate trans cleavage activity of the Cas13a for the cleavage of an RNA reporter and can be activated by a target DNA to initiate trans cleavage activity of the Cas 13a for trans cleavage of an RNA reporter. An RNA reporter can be an RNA-based reporter. In some embodiments, the Cas13a recognizes and detects ssDNA to initiate transcleavage of RNA reporters. Multiple Cas13a isolates can recognize, be activated by, and detect target DNA, including ssDNA, upon hybridization of a guide nucleic acid with the target DNA. For example, Lbu-Cas13a and Lwa-Cas13a can both be activated to transcollaterally cleave RNA reporters by target DNA. Thus, Type VI CRISPR/Cas enzyme (e.g., a Cas13 nuclease, such as Cas13a) can be DNA-activated programmable RNA nucleases, and therefore can be used to detect a target DNA using the methods as described herein. DNA-activated programmable RNA nuclease detection of ssDNA can be robust at multiple pH values. For example, target ssDNA detection by Cas13 can exhibit consistent cleavage across a wide range of pH conditions, such as from a pH of 6.8 to a pH of 8.2. In contrast, target RNA detection by Cas13 can exhibit high cleavage activity of pH values from 7.9 to 8.2. In some embodiments, a DNA-activated programmable RNA nuclease that also is capable of being an RNA-activated programmable RNA nuclease, can have DNA targeting preferences that are distinct from its RNA targeting preferences. For example, the optimal ssDNA targets for Cas13a have different properties than optimal RNA targets for Cas13a. As one example, gRNA performance on ssDNA can not necessarily correlate with the performance of the same gRNAs on RNA. As another example, gRNAs can perform at a high level regardless of target nucleotide identity at a 3' position on a target RNA sequence. In some embodiments, gRNAs can perform at a high level in the absence of a G at a 3' position on a target ssDNA sequence. Furthermore, target DNA detected by Cas13 disclosed herein can be directly taken from organisms or can be indirectly generated by nucleic acid amplification methods, such as PCR and LAMP or any amplification method described herein. Key steps for the sensitive detection of a target DNA, such as a target ssDNA, by a DNA-activated programmable RNA nuclease, such as Cas13a, can include: (1) production or isolation of DNA to concentrations above about 0.1 nM per reaction for in vitro diagnostics, (2) selection of a target sequence with the appropriate sequence features to enable DNA detection as these features are distinct from those required for RNA detection, and (3) buffer composition that enhances DNA detection.

The detection of a target DNA by a DNA-activated programmable RNA nuclease can be connected to a variety of readouts including fluorescence, lateral flow, electrochemistry, or any other readouts described herein. Multiplexing of programmable DNA nuclease, such as a Type V CRISPR-Cas protein, with a DNA-activated programmable RNA nuclease, such as a Type VI protein, with a DNA reporter and an RNA reporter, can enable multiplexed detection of target ssDNAs or a combination of a target dsDNA and a target ssDNA, respectively. Multiplexing of different RNA-activated programmable RNA nucleases that have distinct RNA reporter cleavage preferences can enable additional multiplexing. Methods for the generation of ssDNA for DNA-activated programmable RNA nuclease-based diagnostics can include (1) asymmetric PCR, (2) asymmetric isothermal amplification, such as RPA, LAMP, SDA, etc. (3) NEAR for the production of short ssDNA molecules, and (4) conversion of RNA targets into ssDNA by a reverse transcriptase followed by RNase H digestion. Thus, DNA-activated programmable RNA nuclease detection of target DNA is compatible with the various systems, kits, compositions, reagents, and methods disclosed herein.

The programmable nuclease can comprise a programmable nuclease capable of being activated when complexed with a guide nucleic acid and target nucleic acid. The programmable nuclease can become activated after binding of a guide nucleic acid with a target nucleic acid, in which the activated programmable nuclease can cleave the target nucleic acid, which can initiate trans cleavage activity. In some cases, the trans cut or trans cleavage can cut and/or release a reporter. In other cases, the trans cut or trans cleavage can produce an analog of a target, which can be directly detected. Trans cleavage activity can be non-specific cleavage of nearby nucleic acids by the activated programmable nuclease, such as trans cleavage of reporters with a detection moiety. Once the reporter is cleaved by the activated programmable nuclease, the detection moiety can be released from the reporter and can generate a signal.

In some embodiments, the Type V CRISPR/Cas enzyme is a programmable Cas12 nuclease. Type V CRISPR/Cas enzymes (e.g., Cas12 or Cas14) lack an HNH domain. A Cas12 nuclease of the present disclosure cleaves a nucleic acids via a single catalytic RuvC domain. The RuvC domain is within a nuclease, or "NUC" lobe of the protein, and the Cas12 nucleases further comprise a recognition, or "REC" lobe. The REC and NUC lobes are connected by a bridge helix and the Cas12 proteins additionally include two domains for PAM recognition termed the PAM interacting (PI) domain and the wedge (WED) domain. (Murugan et al., Mol Cell. 2017 Oct 5; 68(1): 15-25). A programmable Cas12 nuclease can be a Cas12a (also referred to as Cpf1) protein, a Cas12b protein, Cas12c protein, Cas12d protein, or a Cas12e protein. In some cases, a suitable Cas12 protein comprises an amino acid sequence having at least 80%, at least 85%, at least 90%, at least 95%, at least 98%, at least 99%, or 100%, amino acid sequence identity to any one of SEQ ID NO: 18 - SEQ ID NO: 60.

**TABLE 1 - Cas12 Protein Sequences**

| **SEQ ID NO** | **Description** | **Sequence** |
|---|---|---|
| SEQ ID NO: 18 | Lachnospira ceae bacterium ND2006 (LbCas12a) | MSKLEKFTNCYSLSKTLRFKAIPVGKTQENIDNKRLLVEDEKRAEDY KGVKKLLDRYYLSFINDVLHSIKLKNLNNYISLFRKKTRTEKENKEL ENLEINLRKEIAKAFKGNEGYKSLFKKDIIETILPEFLDDKDEIALVNS FNGFTTAFTGFFDNRENMFSEEAKSTSIAFRCINENLTRYISNMDIFEK VDAIFDKHEVQEIKEKILNSDYDVEDFFEGEFFNFVLTQEGIDVYNAII GGFVTESGEKIKGLNEYINLYNQKTKQKLPKFKPLYKQVLSDRESLS FYGEGYTSDEEVLEVFRNTLNKNSEIFSSIKKLEKLFKNFDEYSSAGIF VKNGPAISTISKDIFGEWNVIRDKWNAEYDDIHLKKKAVVTEKYED DRRKSFKKIGSFSLEQLQEYADADLSVVEKLKEIIIQKVDEIYKVYGS SEKLFDADFVLEKSLKKNDAVVAIMKDLLDSVKSFENYIKAFFGEGK ETNRDESFYGDFVLAYDILLKVDHIYDAIRNYVTQKPYSKDKFKLYF QNPQFMGGWDKDKETDYRATILRYGSKYYLAIMDKKYAKCLQKID KDDVNGNYEKINYKLLPGPNKMLPKVFFSKKWMAYYNPSEDIQKIY KNGTFKKGDMFNLNDCHKLIDFFKDSISRYPKWSNAYDFNFSETEK YKDIAGFYREVEEQGYKVSFESASKKEVDKLVEEGKLYMFQIYNKD FSDKSHGTPNLHTMYFKLLFDENNHGQIRLSGGAELFMRRASLKKEE LVVHPANSPIANKNPDNPKKTTTLSYDVYKDKRFSEDQYELHIPIAIN KCPKNIFKINTEVRVLLKHDDNPYVIGIDRGERNLLYIVVVDGKGNIV EQYSLNEIINNFNGIRIKTDYHSLLDKKEKERFEARQNWTSIENIKELK AGYISQVVHKICELVEKYDAVIALEDLNSGFKNSRVKVEKQVYQKF EKMLIDKLNYMVDKKSNPCATGGALKGYQITNKFESFKSMSTQNGF IFYIPAWLTSKIDPSTGFVNLLKTKYTSIADSKKFISSFDRIMYVPEEDL FEFALDYKNFSRTDADYIKKWKLYSYGNRIRIFRNPKKNNVFDWEE VCLTSAYKELFNKYGINYQQGDIRALLCEQSDKAFYSSFMALMSLM LQMRNSITGRTDVDFLISPVKNSDGIFYDSRNYEAQENAILPKNADA NGAYNIARKVLWAIGQFKKAEDEKLDKVKIAISNKEWLEYAQTSVK H |
| SEQ ID NO: 19 | Acidaminoc occus sp. BV316 (AsCas12a) | MTQFEGFTNLYQVSKTLRFELIPQGKTLKHIQEQGFIEEDKARNDHY KELKPIIDRIYKTYADQCLQLVQLDWENLSAAIDSYRKEKTEETRNA LIEEQATYRNAIHDYFIGRTDNLTDAINKRHAEIYKGLFKAELFNGKV LKQLGTVTTTEHENALLRSFDKFTTYFSGFYENRKNVFSAEDISTAIP HRIVQDNFPKFKENCHIFTRLITAVPSLREHFENVKKAIGIFVSTSIEEV FSFPFYNQLLTQTQIDLYNQLLGGISREAGTEKIKGLNEVLNLAIQKN DETAHIIASLPHRFIPLFKQILSDRNTLSFILEEFKSDEEVIQSFCKYKTL LRNENVLETAEALFNELNSIDLTHIFISHKKLETISSALCDHWDTLRN ALYERRISELTGKITKSAKEKVQRSLKHEDINLQEIISAAGKELSEAFK QKTSEILSHAHAALDQPLPTTLKKQEEKEILKSQLDSLLGLYHLLDW FAVDESNEVDPEFSARLTGIKLEMEPSLSFYNKARNYATKKPYSVEK FKLNFQMPTLASGWDVNKEKNNGAILFVKNGLYYLGIMPKQKGRY KALSFEPTEKTSEGFDKMYYDYFPDAAKMIPKCSTQLKAVTAHFQT HTTPILLSNNFIEPLEITKEIYDLNNPEKEPKKFQTAYAKKTGDQKGY REALCKWIDFTRDFLSKYTKTTSIDLSSLRPSSQYKDLGEYYAELNPL LYHISFQRIAEKEIMDAVETGKLYLFQIYNKDFAKGHHGKPNLHTLY WTGLFSPENLAKTSIKLNGQAELFYRPKSRMKRMAHRLGEKMLNK KLKDQKTPIPDTLYQELYDYVNHRLSHDLSDEARALLPNVITKEVSH EIIKDRRFTSDKFFFHVPITLNYQAANSPSKFNQRVNAYLKEHPETPII GIDRGERNLIYITVIDSTGKILEQRSLNTIQQFDYQKKLDNREKERVA ARQAWSVVGTIKDLKQGYLSQVIHEIVDLMIHYQAVVVLENLNFGF KSKRTGIAEKAVYQQFEKMLIDKLNCLVLKDYPAEKVGGVLNPYQL TDQFTSFAKMGTQSGFLFYVPAPYTSKIDPLTGFVDPFVWKTIKNHE SRKHFLEGFDFLHYDVKTGDFILHFKMNRNLSFQRGLPGFMPAWDI VFEKNETQFDAKGTPFIAGKRIVPVIENHRFTGRYRDLYPANELIALL EEKGIVFRDGSNILPKLLENDDSHAIDTMVALIRSVLQMRNSNAATG EDYINSPVRDLNGVCFDSRFQNPEWPMDADANGAYHIALKGQLLLN HLKESKDLKLQNGISNQDWLAYIQELRN |
| SEQ ID NO: 20 | Francisella novicida U112 (FnCas12a) | MSIYQEFVNKYSLSKTLRFELIPQGKTLENIKARGLILDDEKRAKDYK KAKQIIDKYHQFFIEEILSSVCISEDLLQNYSDVYFKLKKSDDDNLQK DFKSAKDTIKKQISEYIKDSEKFKNLFNQNLIDAKKGQESDLILWLKQ SKDNGIELFKANSDITDIDEALEIIKSFKGWTTYFKGFHENRKNVYSS NDIPTSIIYRIVDDNLPKFLENKAKYESLKDKAPEAINYEQIKKDLAEE LTFDIDYKTSEVNQRVFSLDEVFEIANFNNYLNQSGITKFNTIIGGKFV NGENTKRKGINEYINLYSQQINDKTLKKYKMSVLFKQILSDTESKSF VIDKLEDDSDVVTTMQSFYEQIAAFKTVEEKSIKETLSLLFDDLKAQ KLDLSKIYFKNDKSLTDLSQQVFDDYSVIGTAVLEYITQQIAPKNLDN PSKKEQELIAKKTEKAKYLSLETIKLALEEFNKHRDIDKQCRFEEILA NFAAIPMIFDEIAQNKDNLAQISIKYQNQGKKDLLQASAEDDVKAIK DLLDQTNNLLHKLKIFHISQSEDKANILDKDEHFYLVFEECYFELANI VPLYNKIRNYITQKPYSDEKFKLNFENSTLANGWDKNKEPDNTAILFI KDDKYYLGVMNKKNNKIFDDKAIKENKGEGYKKIVYKLLPGANKM LPKVFFSAKSIKFYNPSEDILRIRNHSTHTKNGSPQKGYEKFEFNIEDC RKFIDFYKQSISKHPEWKDFGFRFSDTQRYNSIDEFYREVENQGYKLT FENISESYIDSVVNQGKLYLFQIYNKDFSAYSKGRPNLHTLYWKALF DERNLQDVVYKLNGEAELFYRKQSIPKKITHPAKEAIANKNKDNPK KESVFEYDLIKDKRFTEDKFFFHCPITINFKSSGANKFNDEINLLLKEK ANDVHILSIDRGERHLAYYTLVDGKGNIIKQDTFNIIGNDRMKTNYH DKLAAIEKDRDSARKDWKKINNIKEMKEGYLSQVVHEIAKLVIEYN AIVVFEDLNFGFKRGRFKVEKQVYQKLEKMLIEKLNYLVFKDNEFD KTGGVLRAYQLTAPFETFKKMGKQTGIIYYVPAGFTSKICPVTGFVN QLYPKYESVSKSQEFFSKFDKICYNLDKGYFEFSFDYKNFGDKAAKG KWTIASFGSRLINFRNSDKNHNWDTREVYPTKELEKLLKDYSIEYGH GECIKAAICGESDKKFFAKLTSVLNTILQMRNSKTGTELDYLISPVAD VNGNFFDSRQAPKNMPQDADANGAYHIGLKGLMLLGRIKNNQEGK KLNLVIKNEEYFEFVQNRNN |
| SEQ ID NO: 21 | Porphyromo nas macacae (PmCas12a) | MKTQHFFEDFTSLYSLSKTIRFELKPIGKTLENIKKNGLIRRDEQRLDD YEKLKKVIDEYHEDFIANILSSFSFSEEILQSYIQNLSESEARAKIEKTM RDTLAKAFSEDERYKSIFKKELVKKDIPVWCPAYKSLCKKFDNFTTS LVPFHENRKNLYTSNEITASIPYRIVHVNLPKFIQNIEALCELQKKMG ADLYLEMMENLRNVWPSFVKTPDDLCNLKTYNHLMVQSSISEYNRF VGGYSTEDGTKHQGINEWINIYRQRNKEMRLPGLVFLHKQILAKVD SSSFISDTLENDDQVFCVLRQFRKLFWNTVSSKEDDAASLKDLFCGL SGYDPEAIYVSDAHLATISKNIFDRWNYISDAIRRKTEVLMPRKKESV ERYAEKISKQIKKRQSYSLAELDDLLAHYSEESLPAGFSLLSYFTSLG GQKYLVSDGEVILYEEGSNIWDEVLIAFRDLQVILDKDFTEKKLGKD EEAVSVIKKALDSALRLRKFFDLLSGTGAEIRRDSSFYALYTDRMDK LKGLLKMYDKVRNYLTKKPYSIEKFKLHFDNPSLLSGWDKNKELNN LSVIFRQNGYYYLGIMTPKGKNLFKTLPKLGAEEMFYEKMEYKQIAE PMLMLPKVFFPKKTKPAFAPDQSVVDIYNKKTFKTGQKGFNKKDLY RLIDFYKEALTVHEWKLFNFSFSPTEQYRNIGEFFDEVREQAYKVSM VNVPASYIDEAVENGKLYLFQIYNKDFSPYSKGIPNLHTLYWKALFS EQNQSRVYKLCGGGELFYRKASLHMQDTTVHPKGISIHKKNLNKKG ETSLFNYDLVKDKRFTEDKFFFHVPISINYKNKKITNVNQMVRDYIA QNDDLQIIGIDRGERNLLYISRIDTRGNLLEQFSLNVIESDKGDLRTDY QKILGDREQERLRRRQEWKSIESIKDLKDGYMSQVVHKICNMVVEH KAIVVLENLNLSFMKGRKKVEKSVYEKFERMLVDKLNYLVVDKKN LSNEPGGLYAAYQLTNPLFSFEELHRYPQSGILFFVDPWNTSLTDPST GFVNLLGRINYTNVGDARKFFDRFNAIRYDGKGNILFDLDLSRFDVR VETQRKLWTLTTFGSRIAKSKKSGKWMVERIENLSLCFLELFEQFNIG YRVEKDLKKAILSQDRKEFYVRLIYLFNLMMQIRNSDGEEDYILSPA LNEKNLQFDSRLIEAKDLPVDADANGAYNVARKGLMVVQRIKRGD HESIHRIGRAQWLRYVQEGIVE |
| SEQ ID NO: 22 | Moraxella bovoculi 237 (MbCas12a) | MLFQDFTHLYPLSKTVRFELKPIDRTLEHIHAKNFLSQDETMADMHQ KVKVILDDYHRDFIADMMGEVKLTKLAEFYDVYLKFRKNPKDDEL QKQLKDLQAVLRKEIVKPIGNGGKYKAGYDRLFGAKLFKDGKELG DLAKFVIAQEGESSPKLAHLAHFEKFSTYFTGFHDNRKNMYSDEDK HTAIAYRLIHENLPRFIDNLQILTTIKQKHSALYDQIINELTASGLDVSL ASHLDGYHKLLTQEGITAYNTLLGGISGEAGSPKIQGINELINSHHNQ HCHKSERIAKLRPLHKQILSDGMSVSFLPSKFADDSEMCQAVNEFYR HYADVFAKVQSLFDGFDDHQKDGIYVEHKNLNELSKQAFGDFALLG RVLDGYYVDVVNPEFNERFAKAKTDNAKAKLTKEKDKFIKGVHSL ASLEQAIEHYTARHDDESVQAGKLGQYFKHGLAGVDNPIQKIHNNH STIKGFLERERPAGERALPKIKSGKNPEMTQLRQLKELLDNALNVAH FAKLLTTKTTLDNQDGNFYGEFGVLYDELAKIPTLYNKVRDYLSQK PFSTEKYKLNFGNPTLLNGWDLNKEKDNFGVILQKDGCYYLALLDK AHKKVFDNAPNTGKSIYQKMIYKYLEVRKQFPKVFFSKEAIAINYHP SKELVEIKDKGRQRSDDERLKLYRFILECLKIHPKYDKKFEGAIGDIQ LFKKDKKGREVPISEKDLFDKINGIFSSKPKLEMEDFFIGEFKRYNPSQ DLVDQYNIYKKIDSNDNRKKENFYNNHPKFKKDLVRYYYESMCKH EEWEESFEFSKKLQDIGCYVDVNELFTEIETRRLNYKISFCNINADYID ELVEQGQLYLFQIYNKDFSPKAHGKPNLHTLYFKALFSEDNLADPIY KLNGEAQIFYRKASLDMNETTIHRAGEVLENKNPDNPKKRQFVYDII KDKRYTQDKFMLHVPITMNFGVQGMTIKEFNKKVNQSIQQYDEVN VIGIDRGERHLLYLTVINSKGEILEQCSLNDITTASANGTQMTTPYHKI LDKREIERLNARVGWGEIETIKELKSGYLSHVVHQISQLMLKYNAIV VLEDLNFGFKRGRFKVEKQIYQNFENALIKKLNHLVLKDKADDEIGS YKNALQLTNNFTDLKSIGKQTGFLFYVPAWNTSKIDPETGFVDLLKP RYENIAQSQAFFGKFDKICYNADKDYFEFHIDYAKFTDKAKNSRQIW TICSHGDKRYVYDKTANQNKGAAKGINVNDELKSLFARHHINEKQP NLVMDICQNNDKEFHKSLMYLLKTLLALRYSNASSDEDFILSPVAND EGVFFNSALADDTQPQNADANGAYHIALKGLWLLNELKNSDDLNK VKLAIDNQTWLNFAQNR |
| SEQ ID NO: 23 | Moraxella bovoculi AAX08_00 205 (Mb2Cas12 a) | MGIHGVPAALFQDFTHLYPLSKTVRFELKPIGRTLEHIHAKNFLSQDE TMADMYQKVKVILDDYHRDFIADMMGEVKLTKLAEFYDVYLKFRK NPKDDGLQKQLKDLQAVLRKESVKPIGSGGKYKTGYDRLFGAKLFK DGKELGDLAKFVIAQEGESSPKLAHLAHFEKFSTYFTGFHDNRKNM YSDEDKHTAIAYRLIHENLPRFIDNLQILTTIKQKHSALYDQIINELTA SGLDVSLASHLDGYHKLLTQEGITAYNRIIGEVNGYTNKHNQICHKS ERIAKLRPLHKQILSDGMGVSFLPSKFADDSEMCQAVNEFYRHYTDV FAKVQSLFDGFDDHQKDGIYVEHKNLNELSKQAFGDFALLGRVLDG YYVDVVNPEFNERFAKAKTDNAKAKLTKEKDKFIKGVHSLASLEQA IEHHTARHDDESVQAGKLGQYFKHGLAGVDNPIQKIHNNHSTIKGFL ERERPAGERALPKIKSGKNPEMTQLRQLKELLDNALNVAHFAKLLTT KTTLDNQDGNFYGEFGVLYDELAKIPTLYNKVRDYLSQKPFSTEKY KLNFGNPTLLNGWDLNKEKDNFGVILQKDGCYYLALLDKAHKKVF DNAPNTGKNVYQKMVYKLLPGPNKMLPKVFFAKSNLDYYNPSAEL LDKYAKGTHKKGDNFNLKDCHALIDFFKAGINKHPEWQHFGFKFSP TSSYRDLSDFYREVEPQGYQVKFVDINADYIDELVEQGKLYLFQIYN KDFSPKAHGKPNLHTLYFKALFSEDNLADPIYKLNGEAQIFYRKASL DMNETTIHRAGEVLENKNPDNPKKRQFVYDIIKDKRYTQDKFMLHV PITMNFGVQGMTIKEFNKKVNQSIQQYDEVNVIGIDRGERHLLYLTVI NSKGEILEQRSLNDITTASANGTQVTTPYHKILDKREIERLNARVGW GEIETIKELKSGYLSHVVHQINQLMLKYNAIVVLEDLNFGFKRGRFK VEKQIYQNFENALIKKLNHLVLKDKADDEIGSYKNALQLTNNFTDL KSIGKQTGFLFYVPAWNTSKIDPETGFVDLLKPRYENIAQSQAFFGKF DKICYNTDKGYFEFHIDYAKFTDKAKNSRQKWAICSHGDKRYVYDK TANQNKGAAKGINVNDELKSLFARYHINDKQPNLVMDICQNNDKEF HKSLMCLLKTLLALRYSNASSDEDFILSPVANDEGVFFNSALADDTQ PQNADANGAYHIALKGLWLLNELKNSDDLNKVKLAIDNQTWLNFA QNR |
| SEQ ID NO: 24 | Moraxella bovoculi AAX11_00 205 (Mb3Cas12 a) | MGIHGVPAALFQDFTHLYPLSKTVRFELKPIGKTLEHIHAKNFLNQD ETMADMYQKVKAILDDYHRDFIADMMGEVKLTKLAEFYDVYLKFR KNPKDDGLQKQLKDLQAVLRKEIVKPIGNGGKYKAGYDRLFGAKLF KDGKELGDLAKFVIAQEGESSPKLAHLAHFEKFSTYFTGFHDNRKN MYSDEDKHTAIAYRLIHENLPRFIDNLQILATIKQKHSALYDQIINELT ASGLDVSLASHLDGYHKLLTQEGITAYNTLLGGISGEAGSRKIQGINE LINSHHNQHCHKSERIAKLRPLHKQILSDGMGVSFLPSKFADDSEVC QAVNEFYRHYADVFAKVQSLFDGFDDYQKDGIYVEYKNLNELSKQ AFGDFALLGRVLDGYYVDVVNPEFNERFAKAKTDNAKAKLTKEKD KFIKGVHSLASLEQAIEHYTARHDDESVQAGKLGQYFKHGLAGVDN PIQKIHNNHSTIKGFLERERPAGERALPKIKSDKSPEIRQLKELLDNAL NVAHFAKLLTTKTTLHNQDGNFYGEFGALYDELAKIATLYNKVRDY LSQKPFSTEKYKLNFGNPTLLNGWDLNKEKDNFGVILQKDGCYYLA LLDKAHKKVFDNAPNTGKSVYQKMIYKLLPGPNKMLPKVFFAKSNL DYYNPSAELLDKYAQGTHKKGDNFNLKDCHALIDFFKAGINKHPEW QHFGFKFSPTSSYQDLSDFYREVEPQGYQVKFVDINADYINELVEQG QLYLFQIYNKDFSPKAHGKPNLHTLYFKALFSEDNLVNPIYKLNGEA EIFYRKASLDMNETTIHRAGEVLENKNPDNPKKRQFVYDIIKDKRYT QDKFMLHVPITMNFGVQGMTIKEFNKKVNQSIQQYDEVNVIGIDRG ERHLLYLTVINSKGEILEQRSLNDITTASANGTQMTTPYHKILDKREIE RLNARVGWGEIETIKELKSGYLSHVVHQISQLMLKYNAIVVLEDLNF GFKRGRFKVEKQIYQNFENALIKKLNHLVLKDKADDEIGSYKNALQ LTNNFTDLKSIGKQTGFLFYVPAWNTSKIDPETGFVDLLKPRYENIAQ SQAFFGKFDKICYNADRGYFEFHIDYAKFNDKAKNSRQIWKICSHGD KRYVYDKTANQNKGATIGVNVNDELKSLFTRYHINDKQPNLVMDIC QNNDKEFHKSLMYLLKTLLALRYSNASSDEDFILSPVANDEGVFFNS ALADDTQPQNADANGAYHIALKGLWLLNELKNSDDLNKVKLAIDN QTWLNFAQNR |
| SEQ ID NO: 25 | Thiomicrosp ira sp. XS5 (TsCas12a) | MGIHGVPAATKTFDSEFFNLYSLQKTVRFELKPVGETASFVEDFKNE GLKRVVSEDERRAVDYQKVKEIIDDYHRDFIEESLNYFPEQVSKDAL EQAFHLYQKLKAAKVEEREKALKEWEALQKKLREKVVKCFSDSNK ARFSRIDKKELIKEDLINWLVAQNREDDIPTVETFNNFTTYFTGFHEN RKNIYSKDDHATAISFRLIHENLPKFFDNVISFNKLKEGFPELKFDKV KEDLEVDYDLKHAFEIEYFVNFVTQAGIDQYNYLLGGKTLEDGTKK QGMNEQINLFKQQQTRDKARQIPKLIPLFKQILSERTESQSFIPKQFES DQELFDSLQKLHNNCQDKFTVLQQAILGLAEADLKKVFIKTSDLNAL SNTIFGNYSVFSDALNLYKESLKTKKAQEAFEKLPAHSIHDLIQYLEQ FNSSLDAEKQQSTDTVLNYFIKTDELYSRFIKSTSEAFTQVQPLFELEA LSSKRRPPESEDEGAKGQEGFEQIKRIKAYLDTLMEAVHFAKPLYLV KGRKMIEGLDKDQSFYEAFEMAYQELESLIIPIYNKARSYLSRKPFKA DKFKINFDNNTLLSGWDANKETANASILFKKDGLYYLGIMPKGKTFL FDYFVSSEDSEKLKQRRQKTAEEALAQDGESYFEKIRYKLLPGASKM LPKVFFSNKNIGFYNPSDDILRIRNTASHTKNGTPQKGHSKVEFNLND CHKMIDFFKSSIQKHPEWGSFGFTFSDTSDFEDMSAFYREVENQGYV ISFDKIKETYIQSQVEQGNLYLFQIYNKDFSPYSKGKPNLHTLYWKAL FEEANLNNVVAKLNGEAEIFFRRHSIKASDKVVHPANQAIDNKNPHT EKTQSTFEYDLVKDKRYTQDKFFFHVPISLNFKAQGVSKFNDKVNGF LKGNPDVNIIGIDRGERHLLYFTVVNQKGEILVQESLNTLMSDKGHV NDYQQKLDKKEQERDAARKSWTTVENIKELKEGYLSHVVHKLAHLI IKYNAIVCLEDLNFGFKRGRFKVEKQVYQKFEKALIDKLNYLVFKEK ELGEVGHYLTAYQLTAPFESFKKLGKQSGILFYVPADYTSKIDPTTGF VNFLDLRYQSVEKAKQLLSDFNAIRFNSVQNYFEFEIDYKKLTPKRK VGTQSKWVICTYGDVRYQNRRNQKGHWETEEVNVTEKLKALFASD SKTTTVIDYANDDNLIDVILEQDKASFFKELLWLLKLTMTLRHSKIKS EDDFILSPVKNEQGEFYDSRKAGEVWPKDADANGAYHIALKGLWN LQQINQWEKGKTLNLAIKNQDWFSFIQEKPYQE |
| SEQ ID NO: 26 | Butyrivibrio sp. NC3005 (BsCas12a) | MGIHGVPAAYYQNLTKKYPVSKTIRNELIPIGKTLENIRKNNILESDV KRKQDYEHVKGIMDEYHKQLINEALDNYMLPSLNQAAEIYLKKHV DVEDREEFKKTQDLLRREVTGRLKEHENYTKIGKKDILDLLEKLPSIS EEDYNALESFRNFYTYFTSYNKVRENLYSDEEKSSTVAYRLINENLP KFLDNIKSYAFVKAAGVLADCIEEEEQDALFMVETFNMTLTQEGID MYNYQIGKVNSAINLYNQKNHKVEEFKKIPKMKVLYKQILSDREEV FIGEFKDDETLLSSIGAYGNVLMTYLKSEKINIFFDALRESEGKNVYV KNDLSKTTMSNIVFGSWSAFDELLNQEYDLANENKKKDDKYFEKRQ KELKKNKSYTLEQMSNLSKEDISPIENYIERISEDIEKICIYNGEFEKIV VNEHDSSRKLSKNIKAVKVIKDYLDSIKELEHDIKLINGSGQELEKNL VVYVGQEEALEQLRPVDSLYNLTRNYLTKKPFSTEKVKLNFNKSTLL NGWDKNKETDNLGILFFKDGKYYLGIMNTTANKAFVNPPAAKTEN VFKKVDYKLLPGSNKMLPKVFFAKSNIGYYNPSTELYSNYKKGTHK KGPSFSIDDCHNLIDFFKESIKKHEDWSKFGFEFSDTADYRDISEFYRE VEKQGYKLTFTDIDESYINDLIEKNELYLFQIYNKDFSEYSKGKLNLH TLYFMMLFDQRNLDNVVYKLNGEAEVFYRPASIAENELVIHKAGEG IKNKNPNRAKVKETSTFSYDIVKDKRYSKYKFTLHIPITMNFGVDEV RRFNDVINNALRTDDNVNVIGIDRGERNLLYVVVINSEGKILEQISLN SIINKEYDIETNYHALLDEREDDRNKARKDWNTIENIKELKTGYLSQ VVNVVAKLVLKYNAIICLEDLNFGFKRGRQKVEKQVYQKFEKMLIE KLNYLVIDKSREQVSPEKMGGALNALQLTSKFKSFAELGKQSGIIYY VPAYLTSKIDPTTGFVNLFYIKYENIEKAKQFFDGFDFIRFNKKDDMF EFSFDYKSFTQKACGIRSKWIVYTNGERIIKYPNPEKNNLFDEKVINV TDEIKGLFKQYRIPYENGEDIKEIIISKAEADFYKRLFRLLHQTLQMRN STSDGTRDYIISPVKNDRGEFFCSEFSEGTMPKDADANGAYNIARKG LWVLEQIRQKDEGEKVNLSMTNAEWLKYAQLHLL |
| SEQ ID NO: 27 | AacCas12b | MAVKSIKVKLRLDDMPEIRAGLWKLHKEVNAGVRYYTEWLSLLRQ ENLYRRSPNGDGEQECDKTAEECKAELLERLRARQVENGHRGPAGS DDELLQLARQLYELLVPQAIGAKGDAQQIARKFLSPLADKDAVGGL GIAKAGNKPRWVRMREAGEPGWEEEKEKAETRKSADRTADVLRAL ADFGLKPLMRVYTDSEMSSVEWKPLRKGQAVRTWDRDMFQQAIER MMSWESWNQRVGQEYAKLVEQKNRFEQKNFVGQEHLVHLVNQLQ QDMKEASPGLESKEQTAHYVTGRALRGSDKVFEKWGKLAPDAPFD LYDAEIKNVQRRNTRRFGSHDLFAKLAEPEYQALWREDASFLTRYA VYNSILRKLNHAKMFATFTLPDATAHPIWTRFDKLGGNLHQYTFLFN EFGERRHAIRFHKLLKVENGVAREVDDVTVPISMSEQLDNLLPRDPN EPIALYFRDYGAEQHFTGEFGGAKIQCRRDQLAHMHRRRGARDVYL NVSVRVQSQSEARGERRPPYAAVFRLVGDNHRAFVHFDKLSDYLAE HPDDGKLGSEGLLSGLRVMSVDLGLRTSASISVFRVARKDELKPNSK GRVPFFFPIKGNDNLVAVHERSQLLKLPGETESKDLRAIREERQRTLR QLRTQLAYLRLLVRCGSEDVGRRERSWAKLIEQPVDAANHMTPDW REAFENELQKLKSLHGICSDKEWMDAVYESVRRVWRHMGKQVRD WRKDVRSGERPKIRGYAKDVVGGNSIEQIEYLERQYKFLKSWSFFG KVSGQVIRAEKGSRFAITLREHIDHAKEDRLKKLADRIIMEALGYVY ALDERGKGKWVAKYPPCQLILLEELSEYQFNNDRPPSENNQLMQWS HRGVFQELINQAQVHDLLVGTMYAAFSSRFDARTGAPGIRCRRVPA RCTQEHNPEPFPWWLNKFVVEHTLDACPLRADDLIPTGEGEIFVSPFS AEEGDFHQIHADLNAAQNLQQRLWSDFDISQIRLRCDWGEVDGELV LIPRLTGKRTADSYSNKVFYTNTGVTYYERERGKKRRKVFAQEKLSE EEAELLVEADEAREKSVVLMRDPSGIINRGNWTRQKEFWSMVNQRI EGYLVKQIRSRVPLQDSACENTGDI |
| SEQ ID NO: 28 | Cas 12 Variant | MKKIDNFVGCYPVSKTLRFKAIPIGKTQENIEKKRLVEEDEVRAKDY KAVKKLIDRYHREFIEGVLDNVKLDGLEEYYMLFNKSDREESDNKKI EIMEERFRRVISKSFKNNEEYKKIFSKKIIEEILPNYIKDEEEKELVKGF KGFYTAFVGYAQNRENMYSDEKKSTAISYRIVNENMPRFITNIKVFE KAKSILDVDKINEINEYILNNDYYVDDFFNIDFFNYVLNQKGIDIYNAI IGGIVTGDGRKIQGLNECINLYNQENKKIRLPQFKPLYKQILSESESMS FYIDEIESDDMLIDMLKESLQIDSTINNAIDDLKVLFNNIFDYDLSGIFI NNGLPITTISNDVYGQWSTISDGWNERYDVLSNAKDKESEKYFEKRR KEYKKVKSFSISDLQELGGKDLSICKKINEIISEMIDDYKSKIEEIQYLF DIKELEKPLVTDLNKIELIKNSLDGLKRIERYVIPFLGTGKEQNRDEVF YGYFIKCIDAIKEIDGVYNKTRNYLTKKPYSKDKFKLYFENPQLMGG WDRNKESDYRSTLLRKNGKYYVAIIDKSSSNCMMNIEEDENDNYEK INYKLLPGPNKMLPKVFFSKKNREYFAPSKEIERIYSTGTFKKDTNFV KKDCENLITFYKDSLDRHEDWSKSFDFSFKESSAYRDISEFYRDVEK QGYRVSFDLLSSNAVNTLVEEGKLYLFQLYNKDFSEKSHGIPNLHTM YFRSLFDDNNKGNIRLNGGAEMFMRRASLNKQDVTVHKANQPIKN KNLLNPKKTTTLPYDVYKDKRFTEDQYEVHIPITMNKVPNNPYKINH MVREQLVKDDNPYVIGIDRGERNLIYVVVVDGQGHIVEQLSLNEIIN ENNGISIRTDYHTLLDAKERERDESRKQWKQIENIKELKEGYISQVVH KICELVEKYDAVIALEDLNSGFKNSRVKVEKQVYQKFEKMLITKLN YMVDKKKDYNKPGGVLNGYQLTTQFESFSKMGTQNGIMFYIPAWL TSKMDPTTGFVDLLKPKYKNKADAQKFFSQFDSIRYDNQEDAFVFK VNYTKFPRTDADYNKEWEIYTNGERIRVFRNPKKNNEYDYETVNVS ERMKELFDSYDLLYDKGELKETICEMEESKFFEELIKLFRLTLQMRNS ISGRTDVDYLISPVKNSNGYFYNSNDYKKEGAKYPKDADANGAYNI ARKVLWAIEQFKMADEDKLDKTKISIKNQEWLEYAQTHCE |
| SEQ ID NO: 29 | Cas 12 Variant | MATLVSFTKQYQVQKTLRFELIPQGKTQANIDAKGFINDDLKRDENY MKVKGVIDELHKNFIEQTLVNVDYDWRSLATAIKNYRKDRSDTNKK NLEKTQEAARKEIIAWFEGKRGNSAFKNNQKSFYGKLFKKELFSEIL RSDDLEYDEETQDAIACFDKFTTYFVGFHENRKNMYSTEAKSTSVA YRVVNENFSKFLSNCEAFSVLEAVCPNVLVEAEQELHLHKAFSDLKL SDVFKVEAYNKYLSQTGIDYYNQIIGGISSAEGVRKIRGVNEVVNNAI QQNDELKVALRNKQFTMVQLFKQILSDRSTLSFVSEQFTSDQEVITV VKQFNDDIVNNKVLAVVKTLFENFNSYDLEKIYINSKELASVSNALL KDWSKIRNAVLENKIIELGANPPKTKISAVEKEVKNKDFSIAELASYN DKYLDKEGNDKEICSIANVVLEAVGALEIMLAESLPADLKTLENKNK VKGILDAYENLLHLLNYFKVSAVNDVDLAFYGAFEKVYVDISGVMP LYNKVRNYATKKPYSVEKFKLNFAMPTLADGWDKNKERDNGSIILL KDGQYYLGVMNPQNKPVIDNAVCNDAKGYQKMVYKMFPEISKMV TKCSTQLNAVKAHFEDNTNDFVLDDTDKFISDLTITKEIYDLNNVLY DGKKKFQIDYLRNTGDFAGYHKALETWIDFVKEFLSKYRSTAIYDLT TLLPTNYYEKLDVFYSDVNNLCYKIDYENISVEQVNEWVEEGNLYL FKIYNKDFATGSTGKPNLHTMYWNAVFAEENLHDVVVKLNGGAEL FYRPKSNMPKVEHRVGEKLVNRKNVNGEPIADSVHKEIYAYANGKI SKSELSENAQEELPLAIIKDVKHNITKDKRYLSDKYFFHVPITLNYKA NGNPSAFNTKVQAFLKNNPDVNIIGIDRGERNLLYVVVIDQQGNIIDK KQVSYNKVNGYDYYEKLNQREKERIEARQSWGAVGKIKELKEGYL SLVVREIADMMVKYNAIVVMENLNAGFKRVRGGIAEKAVYQKFEK MLIDKLNYLVFKDVEAKEAGGVLNAYQLTDKFDSFEKMGNQSGFLF YVPAAYTSKIDPVTGFANVFSTKHITNTEAKKEFICSFNSLRYDEAKD KFVLECDLNKFKIVANSHIKNWKFIIGGKRIVYNSKNKTYMEKYPCE DLKATLNASGIDFSSSEIINLLKNVPANREYGKLFDETYWAIMNTLQ MRNSNALTGEDYIISAVADDNEKVFDSRTCGAELPKDADANGAYHI ALKGLYLLQRIDISEEGEKVDLSIKNEEWFKFVQQKEYAR |
| SEQ ID NO: 30 | Cas 12 Variant | MKEQFINRYPLSKTLRFSLIPVGETENNFNKNLLLKKDKQRAENYEK VKCYIDRFHKEYIESVLSKARIEKVNEYANLYWKSNKDDSDIKAMES LENDMRKQISKQLTSTEIYKKRLFGKELICEDLPSFLTDKDERETVEC FRSFTTYFKGFNTNRENMYSSDGKSTAIAYRCINDNLPRFLDNVKSF QKVFDNLSDETITKLNTDLYNIFGRNIEDIFSVDYFEFVLTQSGIEIYNS MIGGYTCSDKTKIQGLNECINLYNQQVAKNEKSKKLPLMKPLYKQIL SEKDSVSFIPEKFNSDNEVLHAIDDYYTGHIGDFDLLTELLQSLNTYN ANGIFVKSGVAITDISNGAFNSWNVLRSAWNEKYEALHPVTSKTKID KYIEKQDKIYKAIKSFSLFELQSLGNENGNEITDWYISSINESNSKIKE AYLQAQKLLNSDYEKSYNKRLYKNEKATELVKNLLDAIKEFQKLIK PLNGTGKEENKDELFYGKFTSYYDSIADIDRLYDKVRNYITQKPYSK DKIKLNFDNPQLLGGWDKNKESDYRTVLLHKDGLYYLAVMDKSHS KAFVDAPEITSDDKDYYEKMEYKLLPGPNKMLPKVFFASKNIDTFQP SDRILDIRKRESFKKGATFNKAECHEFIDYFKDSIKKHDDWSQFGFKF SPTESYNDISEFYREISDQGYSVRFNKISKNYIDGLVNNGYIYLFQIYN KDFSKYSKGTPNLHTLYFKMLFDERNLSNVVYKLNGEAEMFYREAS IGDKEKITHYANQPIKNKNPDNEKKESVFEYDIVKDKRFTKRQFSLH LPITINFKAHGQEFLNYDVRKAVKYKDDNYVIGIDRGERNLIYISVIN SNGEIVEQMSLNEIISDNGHKVDYQKLLDTKEKERDKARKNWTSVE NIKELKEGYISQVVHKICELVIKYDAVIAMEDLNFGFKRGRFPVEKQ VYQKFENMLISKLNLLIDKKAEPTEDGGLLRAYQLTNKFDGVNKAK QNGIIFYVPAWDTSKIDPATGFVNLLKPKCNTSVPEAKKLFETIDDIK YNANTDMFEFYIDYSKFPRCNSDFKKSWTVCTNSSRILTFRNKEKNN KWDNKQIVLTDEFKSLFNEFGIDYKGNLKDSILSISNADFYRRLIKLL SLTLQMRNSITGSTLPEDDYLISPVANKSGEFYDSRNYKGTNAALPC DADANGAYNIARKALWAINVLKDTPDDMLNKAKLSITNAEWLEYT QK |
| SEQ ID NO: 31 | Cas 12 Variant | MNNPRGAFGGFTNLYSLSKTLRFELKPYLEIPEGEKGKLFGDDKEYY KNCKTYTEYYLKKANKEYYDNEKVKNTDLQLVNFLHDERIEDAYQ VLKPVFDTLHEEFITDSLESAEAKKIDFGNYYGLYEKQKSEQNKDEK KKIDKPLETERGKLRKAFTPIYEAEGKNLKNKAGKEKKDKDILKESG FKVLIEAGILKYIKNNIDEFADKKLKNNEGKEITKKDIETALGAENIEG IFDGFFTYFSGFNQNRENYYSTEEKATAVASRIVDENLSKFCDNILLY RKNENDYLKIFNFLKNKGKDLKLKNSKFGKENEPEFIPAYDMKNDE KSFSVADFVNCLSQGEIEKYNAKIANANYLINLYNQNKDGNSSKLSM FKILYKQIGCGEKKDFIKTIKDNAELKQILEKACEAGKKYFIRGKSED GGVSNIFDFTDYIQSHENYKGVYWSDKAINTISGKYFANWDTLKNK LGDAKVFNKNTGEDKADVKYKVPQAVMLSELFAVLDDNAGEDWR EKGIFFKASLFEGDQNKSEIIKNANRPSQALLKMICDDMESLAKNFID SGDKILKISDRDYQKDENKQKIKNWLDNALWINQILKYFKVKANKI KGDSIDARIDSGLDMLVFSSDNPAEDYDMIRNYLTQKPQDEINKLKL NFENSSLAGGWDENKEKDNSCIILKDEQDKQYLAVMKYENTKVFEQ KNSQLYIADNAAWKKMIYKLVPGASKTLPKVFFSKKWTANRPTPSD IVEIYQKGSFKKENVDFNDKKEKDESRKEKNREKIIAELQKTCWMDI RYNIDGKIESAKYVNKEKLAKLIDFYKENLKKYPSEEESWDRLFAFG FSDTKSYKSIDQFYIEVDKQGYKLEFVTINKARLDEYVRDGKIYLFEI RSRDNNLVNGEEKTSAKNLQTIYWNAAFGGDDNKPKLNGEAEIFYR PAIAENKLNKKKDKNGKEIIDGYRFSKEKFIFHCPITLNFCLKETKIND KLNAALAKPENGQGVYFLGIDRGEKHLAYYSLVNQKGEILEQGTLN LPFLDKNGKSRSIKVEKKSFEKDSNGGIIKDKDGNDKIKIEFVECWNY NDLLDARAGDRDYARKNWTTIGTIKELKDGYISQVVRKIVDLSIYKN TETKEFREMPAFIVLEDLNIGFKRGRQKIEKQVYQKLELALAKKLNF LVDKKADIGEIGSVTKAIQLTPPVNNFGDMENRKQFGNMLYIRADYT SQTDPATGWRKSIYLKSGSESNVKEQIEKSFFDIRYESGDYCFEYRDR HGKMWQLYSSKNGVSLDRFHGERNNSKNVWESEKQPLNEMLDILF DEKRFDKSKSLYEQMFKGGVALTRLPKEINKKDKPAWESLRFVIILIQ QIRNTGKNGDDRNGDFIQSPVRDEKTGEHFDSRIYLDKEQKGEKADL PTSGDANGAYNIARKGIVVAEHIKRGFDKLYISDEEWDTWLAGDEI WDKWLKENRESLTKTRK |
| SEQ ID NO: 32 | Cas 12 Variant | MNGNRIIVYREFVGVTPVAKTLRNELRPIGHTQEHIIHNGLIQEDELR QEKSTELKNIMDDYYREYIDKSLSGVTDLDFTLLFELMNLVQSSPSK DNKKALEKEQSKMREQICTHMQSDSNYKNIFNAKFLKEILPDFIKNY NQYDAKDKAGKLETLALFNGFSTYFTDFFEKRKNVFTKEAVSTSIAY RIVHENSLTFLANMTSYKKISEKALDEIEVIEKNNQDKMGDWELNQI FNPDFYNMVLIQSGIDFYNEICGVVNAHMNLYCQQTKNNYNLFKMR KLHKQILAYTSTSFEVPKMFEDDMSVYNAVNAFIDETEKGNIIGKLK DIVNKYDELDEKRIYISKDFYETLSCFMSGNWNLITGCVENFYDENIH AKGKSKEEKVKKAVKEDKYKSINDVNDLVEKYIDEKERNEFKNSNA KQYIREISNIITDTETAHLEYDEHISLIESEEKADEMKKRLDMYMNMY HWAKAFIVDEVLDRDEMFYSDIDDIYNILENIVPLYNRVRNYVTQKP YNSKKIKLNFQSPTLANGWSQSKEFDNNAIILIRDNKYYLAIFNAKNK PDKKIIQGNSDKKNDNDYKKMVYNLLPGANKMLPKVFLSKKGIETF KPSDYIISGYNAHKHIKTSENFDISFCRDLIDYFKNSIEKHAEWRKYEF KFSATDSYNDISEFYREVEMQGYRIDWTYISEADINKLDEEGKIYLFQ IYNKDFAENSTGKENLHTMYFKNIFSEENLKDIIIKLNGQAELFYRRA SVKNPVKHKKDSVLVNKTYKNQLDNGDVVRIPIPDDIYNEIYKMYN GYIKENDLSEAAKEYLDKVEVRTAQKDIVKDYRYTVDKYFIHTPITI NYKVTARNNVNDMAVKYIAQNDDIHVIGIDRGERNLIYISVIDSHGNI VKQKSYNILNNYDYKKKLVEKEKTREYARKNWKSIGNIKELKEGYI SGVVHEIAMLMVEYNAIIAMEDLNYGFKRGRFKVERQVYQKFESML INKLNYFASKGKSVDEPGGLLKGYQLTYVPDNIKNLGKQCGVIFYVP AAFTSKIDPSTGFISAFNFKSISTNASRKQFFMQFDEIRYCAEKDMFSF GFDYNNFDTYNITMSKTQWTVYTNGERLQSEFNNARRTGKTKSINL TETIKLLLEDNEINYADGHDVRIDMEKMDEDKNSEFFAQLLSLYKLT VQMRNSYTEAEEQEKGISYDKIISPVINDEGEFFDSDNYKESDDKECK MPKDADANGAYCIALKGLYEVLKIKSEWTEDGFDRNCLKLPHAEW LDFIQNKRYE |
| SEQ ID NO: 33 | Cas 12 Variant | MKKIDSFVNYYPLSKTLRFSLIPVGKTEDNFNAKLLLEEDEKRAIEYE KVKRYIDRYHKHFIETVLANFHLDDVNEYAELYYKAGKDDKDLKY MEKLEGKMRKSISAAFTKDKKYKEIFGQEIIKNILPEFLENEDEKESV KMFQGFFTYFTGFNDNRKNMYTHEAQTTAISYRCINENLPKFLDNV QSFAKIKESISSDIMNKLDEVCMDLYGVYAQDMFCTDYFSFVLSQSG IDRYNNIIGGYVDDKGVKIQGINEYINLYNQQVDEKNKRLPLMKKLY KQILIEKESISFIPEKFESDNIVINAISDYYHNNVENLFDDFNKLFNEFS EYDDNGIFVTSGLAVTDISNAVFGSWNIISDSWNEEYKDSHPMKKTT NAEKYYEDMKKEYKKNLSFTIAELQRLGEAGCNDECKGDIKEYYKT TVAEKIENIKNAYEISKDLLASDYEKSNDKKLCKNDSAISLLKNLLDS IKDLEKTIKPLLGTGKEENKDDVFYGKFTNLYEMISEIDRLYDKVRN YVTQKPYSKDKIKLNFENPQHLGGWDKNKERDYRSVLLKKEDKYY LAIMDKSNNKAFIDFPDDGECYEKIEYKLLPGPNKMLPKVFFASSNIE YFAPSKKILEIRSRESFKKGDMFNLKDCHEFIDFFKESIKKHEDWSQF GFEFSPTEKYNDISEFYNEVKIQGYSLKYKNVSKKYIDELIECGQLYL FQIYNKDFSVYAKGNPNLHTMYFKMLFDERNLANVVYQLNGGAEM FYRKASIKDSEKIVHHANQPIKNKNADNVKKESVFEYDIIKDKRFTK RQFSIHIPITLNFKAKGQNFINNDVRMALKKADENYVIGIDRGERNLL YICVINSKGEIVEQKSLNEIIGDNGYRVDYHKLLDKKEAERDEARKS WGTIENIKELKEGYLSQIVHEISKLVIKYDAVIAIEDLNSGFKKGRFKV EKQVYQKFENMLCTKLNYLVDKNADANECGGLLKAYQLTNKEDG ANRGRQNGIIFSVPAWLTSKIDPVTGFADLLRPKYKSVSESVEFISKID NIRYNSKEDYFEFDIDYSKFPNSTASYKKKWTVCTYGERIINVRNKE KNNMWDNKTIVLTDEFKKLFADFGVDVSKNIKESVLAIDSKDFYYR FINLLANTLQLRNSEVGNVDVDYLISPVKGVDGSFYDSRLVKEKTLP ENADANGAYNIARKALWAIDVLKQTKDEELKNANLSIKNAEWLEY VQK |
| SEQ ID NO: 34 | Cas 12 Variant | MRTMVTFEDFTKQYQVSKTLRFELIPQGKTLENMKRDGIISVDRQRN EDYQKAKGILDKLYKYILDFTMETVVIDWEALATATEEFRKSKDKK TYEKVQSKIRTALLEHVKKQKVGTEDLFKGMFSSKIITGEVLAAFPEI RLSDEENLILEKFKDFTTYFTGFFENRKNVFTDEALSTSFTYRLVNDN FIKFFDNCIVFKNVVNISPHMAKSLETCASDLGIFPGVSLEEVFSISFY NRLLTQTGIDQFNQLLGGISGKEGEHKQQGLNEIINLAMQQNLEVKE VLKNKAHRFTPLFKQILSDRSTMSFIPDAFADDDEVLSAVDAYRKYL SEKNIGDRAFQLISDMEAYSPELMRIGGKYVSVLSQLLFYSWSEIRDG VKAYKESLITGKKTKKELENIDKEIKYGVTLQEIKEALPKKDIYEEVK KYAMSVVKDYHAGLAEPLPEKIETDDERASIKHIMDSMLGLYRFLE YFSHDSIEDTDPVFGECLDTILDDMNETVPLYNKVRNFSTRKVYSTE KFKLNFNNSSLANGWDKNKEQANGAILLRKEGEYFLGIFNSKNKPK LVSDGGAGIGYEKMIYKQFPDFKKMLPKCTISLKDTKAHFQKSDEDF TLQTDKFEKSIVITKQIYDLGTQTVNGKKKFQVDYPRLTGDMEGYR AALKEWIDFGKEFIQAYTSTAIYDTSLFRDSSDYPDLPSFYKDVDNIC YKLTFEWIPDAVIDDCIDDGSLYLFKLHNKDFSSGSIGKPNLHTLYW KALFEEENLSDVVVKLNGQAELFYRPKSLTRPVVHEEGEVIINKTTST GLPVPDDVYVELSKFVRNGKKGNLTDKAKNWLDKVTVRKMPHAIT KDRRFTVDKFFFHVPITLNYKADSSPYRFNDFVRQYIKDCSDVKIIGI DRGERNLIYAVVIDGKGNIIEQRSFNTVGTYNYQEKLEQKEKERQTA RQDWATVTKIKDLKKGYLSAVVHELSKMIVKYKAIVALENLNVGFK RMRGGIAERSVYQQFEKALIDKLNYLVFKDEEQSGYGGVLNAYQLT DKFESFSKMGQQTGFLFYVPAAYTSKIDPLTGFINPFSWKHVKNRED RRNFLNLFSKLYYDVNTHDFVLAYHHSNKDSKYTIKGNWEIADWDI LIQENKEVFGKTGTPYCVGKRIVYMDDSTTGHNRMCAYYPHTELKK LLSEYGIEYTSGQDLLKIIQEFDDDKLVKGLFYIIKAALQMRNSNSET GEDYISSPIEGRPGICFDSRAEADTLPYDADANGAFHIAMKGLLLTER IRNDDKLAISNEEWLNYIQEMRG |
| SEQ ID NO: 35 | Cas 12 Variant | MNKDIRKNFTDFVGISEIQKTLRFILIPIGKTAQNIDKYNMFEDDEIRH EYYPILKEACDDFYRNHIDQQFENLELDWSKLDEALASEDRDLINET RATYRQVLFNRLKNSVDIKGDSKKNKTLSLESSDKNLGKKKTKNTF QYNFNDLFKAKLIKAILPLYIEYIYEGEKLENAKKALKMYNRFTSRLS NFWQARANIFTDDEISTGSPYRLVNDNFTIFRINNSIYTKNKPFIEEDIL EFEKKLKSKKIIKDFESVDDYFTVNAFNKLCTQNGIDKYNSILGGFTT KEREKVKGLNELFNLAQQSINKGKKGEYRKNIRLGKLTKLKKQILAI SDSTSFLIEQIEDDQDLYNKIKDFFELLLKEEIENENIFTQYANLQKLIE QADLSKIYINAKHLNKISHQVTGKWDSLNKGIALLLENININEESKEK SEVISNGQTKDISSEAYKRYLQIQSEEKDIERLRTQIYFSLEDLEKALD LVLIDENMDRSDKSILSYVQSPDLNVNFERDLTDLYSRIMKLEENNE KLLANHSAIDLIKEFLDLIMLRYSRWQILFCDSNYELDQTFYPIYDAV MEILSNIIRLYNLARNYLSRKPDRMKKKKINFNNPTLADGWSESKIPD NSSMLFIKDGMYYLGIIKNRAAYSELLEAESLQSSEKKKSENSSYER MNYHFLPDAFRSIPKSSIAMKAVKEHFEINQKTADLLLDTDKFSKPLR ITKEIFDMQYVDLHKNKKKYQVDYLRDTGDKKGYRKALNTWLNFC KDFISKYKGRNLFDYSKIKDADHYETVNEFYNDVDKYSYHIFFTSVA ETTVEKFISEGKLYLFQLYNKDFSPHSTGKPNLHTIYWRALFSEENLT SKNIKLNGQAEIFFRPKQIETPFTHKKGSILVNRFDVNGNPIPINVYQEI KGFKNNVIKWDDLNKTTQEGLENDQYLYFESEFEIIKDRRYTEDQLF FHVPISFNWDIGSNPKINDLATQYIVNSNDIHIIGIDRGENHLIYYSVID LQGAIVEQGSLNTITEYTENKFLNNKTNNLRKIPYKDILQQREDERAD ARIKWHAIDKIKDLKDGYLGQIVHFLAKLIIKYNAIVILEDLNYGFKR GRFKVERQVYQKFEMALMKKLNVLVFKDYDIDEIGGPLKPWQLTRP IDSYERMGRQNGILFYVPAAYTSAVDPVTGFANLFYLNNVKNSEKF HFFSKFESIKYHSDQDMFSFAFDYNNFGTTTRINDLSKSKWQVFTNH ERSVWNNKEKNYVTQNLTDLIKKLLRTYNIEFKNNQNVLDSILKIEN NTDKENFARELFRLFRLTIQLRNTTVNENNTEITENELDYIISPVKDKN GNFFDSRDELKNLPDNGDANGAYNIARKGLLYIEQLQESIKTGKLPT LSISTLDWFNYIMK |
| SEQ ID NO: 36 | Cas 12 Variant | MTPIFCNFVVYQIMLFNNNININVKTMNKKHLSDFTNLFPVSKTLRFR LEPQGKTMENIVKAQTIETDEERSHDYEKTKEYIDDYHRQFIDDTLD KFAFKVESTGNNDSLQDYLDAYLSANDNRTKQTEEIQTNLRKAIVSA FKMQPQFNLLFKKEMVKHLLPQFVDTDDKKRIVAKFNDFTTYFTGF FTNRENMYSDEAKSTSIAYRIVNQNLIKFVENMLTFKSHILPILPQEQL ATLYDDFKEYLNVASIAEMFELDHFSIVLTQRQIEVYNSVIGGRKDE NNKQIKPGLNQYINQHNQAVKDKSARLPLLKPLFNQILSEKAGVSFL PKQFKSASEVVKSLNEAYAELSPVLAAIQDVVTNITDYDCNGIFIKND LGLTDIAQRFYGNYDAVKRGLRNQYELETPMHNGQKAEKYEEQVA KHLKSIESVSLAQINQVVTDGGDICDYFKAFGATDDGDIQRENLLASI NNAHTAISPVLNKENANDNELRKNTMLIKDLLDAIKRLQWFAKPLL GAGDETNKDQVFYGKFEPLYNQLDETISPLYDKVRSYLTKKPYSLD KFKINFEKSNLLGGWDPGADRKYQYNAVILRKDNDFYLGIMRDEAT SKRKCIQVLDCNDEGLDENFEKVEYKQIKPSQNMPRCAFAKKECEE NADIMELKRKKNAKSYNTNKDDKNALIRHYQRYLDRTYPEFGFVY KDADEYDTVKAFTDSMDSQDYKLSFLQVSETGLNKLVDEGDLYLFK ITNKDFSSYAKGRPNLHTIYWRMLFDPKNLANVVYKLEGKAEVFFR RKSLASTTTHKAKQAIKNKSRYNEAVKPQSTFDYDIIKDRRFTADKF EFHVPIKMNFKAAGWNSTRLTNEVREFIKSQGVRHIIGIDRGERHLLY LTMIDMDGNIVKQCSLNAPAQDNARASEVDYHQLLDSKEADRLAA RRNWGTIENIKELKQGYLSQVVHLLATMMVDNDAILVLENLNAGF MRGRQKVEKSVYQKFEKMLIDKLNYIVDKGQSPDKPTGALHAVQL TGLYSDFNKSNMKRANVRQCGFVFYIPAWNTSKIDPVTGFVNLFDT HLSSMGEIKAFFSKFDSIRYNQDKGWFEFKFDYSRFTTRAEGCRTQW TVCTYGERIWTHRSKNQNNQFVNDTVNVTQQMLQLLQDCGIDPNG NLKEAIANIDSKKSLETLLHLFKLTVQMRNSVTGSEVDYMISPVADE RGHFFDSRESDEHLPANADANGAFNIARKGLMVVRQIMATDDVSKI KFAVSNKDWLRFAQHIDD |
| SEQ ID NO: 37 | Cas 12 Variant | MNKGGYVIMEKMTEKNRWENQFRITKTIKEEIIPTGYTKVNLQRVN MLKREMERNEDLKKMKEICDEYYRNMIDVSLRLEQVRTLGWESLIH KYRMLNKDEKEIKALEKEQEDLRKKISKGFGEKKAWTGEQFIKKILP QYLMDHYTGEELEEKLRIVKKFKGCTMFLSTFFKNRENIFTDKPIHT AVGHRITSENAMLFAANINTYEKMESNVTLEIERLQREFWRRGINISE IFTDAYYVNVLTQKQIEAYNKICGDINQHMNEYCQKQLKFSEFRM RELKKQILAVVGEHFEIPEKIESTKEVYRELNEYYESLKELHGQFEEL KSVQLKYSQIYVQKKGYDRISRYIGGQWDLIQECMKKDCASGMKGT KKNHDAKIEEEVAKVKYQSIEHIQKLVCTYEEDRGHKVTDYVDEFIV SVCDLLGADHIITRDGERIELPLQYEPGTDLLKNDTINQRRLSDIKTIL DWHMDMLEWLKTFLVNDLVIKDEEFYMAIEELNERMQCVISVYNRI RNYVTQKGYEPEKIRICFDKGTILTGWTTGDNYQYSGFLLMRNDKY YLGIINTNEKSVRKILDGNEECKDENDYIRVGYHLINDASKQLPRIFV MPKAGKKSEILMKDEQCDYIWDGYCHNKHNESKEFMRELIDYYKR SIMNYDKWEGYCFKFSSTESYDNMQDFYKEVREQSYNISFSYINENV LEQLDKDGKIYLFQVYNKDFAAGSTGTPNLHTMYLQNLFSSQNLEL KRLRLGGNAELFYRPGTEKDVTHRKGSILVDRTYVREEKDGIEVRDT VPEKEYLEIYRYLNGKQKGDLSESAKQWLDKVHYREAPCDIIKDKR YAQEKYFLHFSVEINPNAEGQTALNDNVRRWLSEEEDIHVIGIDRGE RNLIYVSLMDGKGRIKDQKSYNIVNSGNKEPVDYLAKLKVREKERD EARRNWKAIGKIKDIKTGYLSYVVHEIVEMAVREKAIIVMEDLNYGF KRGRFKVERQVYQKFEEMLINKLNYVVDKQLSVDEPGGLLRGYQL AFIPKDKKSSMRQNGIVFYVPAGYTSKIDPTTGFVNIFKFPQFGKGDD DGNGKDYDKIRAFFGKFDEIRYECDEKVTADNTREVKERYRFDFDY SKFETHLVHMKKTKWTVYAEGERIKRKKVGNYWTSEVISDIALRMS NTLNIAGIEYKDGHNLVNEICALRGKQAGIILNELLEIVRLTVQLRNS TTEGDVDERDEIISPVLNEKYGCFYHSTEYKQQNGDVLPKDADANG AYCIGLKGIYEIRQIKNKWKEDMTKGEGKALNEGMRISHDQWFEFIQ NMNKGE |
| SEQ ID NO: 38 | Cas 12 Variant | MNELVKNRCKQTKTICQKLIPIGKTRETIEKYNLMEIDRKIAANKEL MNKLFSLIAGKHINDTLSKCTDLDFEPLLTSLSSLNNAKENDRDNLRE YYDSVFEEKKTLAEEISSRLTAVKFAGKDFFTKNIPDFLETYEGDDKN EMSELVSLVIENTVTAGYVKKLEKIDRSMEYRLVSGTVVKRVLTDN ADIYEKNIEKAKDFDYGVLNIDEASQFTTLVAKDYANYLTADGIAIY NVGIGKINLALNEYCQKNKEYSYNKLALLPLQKMLYGEKLSLFEKL EDFTSDEELINSYNKFAKTVNESGLAEIIKKAVPSYDEIVIKPNKISNY SNSITGHWSLVNRIMKDYLENNGIKNADKYMEKGLTLSEIGDALEN KNIKHSDFISNLINDLGHTYTEIKENKESLKKDESVNALIIKKELDMLL SILQNLKVFDIDNEMFDTGFGIEVSKAIEILGYGVPLYNKIRNYITKKP DPKKKFMTKFGSATIGTGITTSVEGSKKATFLKDGDAVFLLLYNTAG CKANNVSVSNLADLINSSLEIENSGKCYQKMIYQTPGDIKKQIPRVFV YKSEDDDLIKDFKAGLHKTDLSFLNGRLIPYLKEAFATHETYKNYTF SYRNSYESYDEFCEHMSEQAYILEWKWIDKKLIDDLVEDGSLLMFR VWNRFMKKKEGKISKHAKIVNELFSDENASNAAIKLLSVFDIFYRDK QIDNPIVHKAGTTLYNKRTKDGEVIVDYTTMVKNKEKRPNVYTTTK KYDIIKDRRYTEEQFEIHLHVNIGKEENKEKLETSKVINEKKNTLVVT RSNEHLLYVVIFDENDNILLKKSLNTVKGMNFKSKLEVVEIQKKENM QSWKTVGSNQALMEGYLSFAIKEIADLVKEYDAILVLEQNSVGKNIL NERVYTRFKEMLITNLSLDVDYENKDFYSYTELGGKVASWRDCVTN GICIQVPSAYKYKDPTTSFSTISMYAKTTAEKSKKLKQIKSFKYNRER GLFELVIAKGVGLENNIVCDSFGSRSIIENDISKEVSCTLKIEKYLIDAG IEYNDEKEVLKDLDTAAKTDAVHKAVTLLLKCFNESPDGRYYISPCG EHFTLCDAPEVLSAINYYIRSRYIREQIVEGVKKMEYKKTILLAK |
| SEQ ID NO: 39 | Cas 12 Variant | MNYKTGLEDFIGKESLSKTLRNALIPTESTKIHMEEMGVIRDDELRAE KQQELKEIMDDYYRTFIEEKLGQIQGIQWNSLFQKMEETMEDISVRK DLDKIQNEKRKEICCYFTSDKRFKDLFNAKLITDILPNFIKDNKEYTEE EKAEKEQTRVLFQRFATAFTNYFNQRRNNFSEDNISTAISFRIVNENS EIHLQNMRAFQRIEQQYPEEVCGMEEEYKDMLQEWQMKHIYSVDF YDRELTQPGIEYYNGICGKINEHMNQFCQKNRINKNDFRMKKLHKQI LCKKSSYYEIPFRFESDQEVYDALNEFIKTMKKKEIIRRCVHLGQECD DYDLGKIYISSNKYEQISNALYGSWDTIRKCIKEEYMDALPGKGEKK EEKAEAAAKKEEYRSIADIDKIISLYGSEMDRTISAKKCITEICDMAG QISIDPLVCNSDIKLLQNKEKTTEIKTILDSFLHVYQWGQTFIVSDIIEK DSYFYSELEDVLEDFEGITTLYNHVRSYVTQKPYSTVKFKLHFGSPTL ANGWSQSKEYDNNAILLMRDQKFYLGIFNVRNKPDKQIIKGHEKEE KGDYKKMIYNLLPGPSKMLPKVFITSRSGQETYKPSKHILDGYNEKR HIKSSPKFDLGYCWDLIDYYKECIHKHPDWKNYDFHFSDTKDYEDIS GFYREVEMQGYQIKWTYISADEIQKLDEKGQIFLFQIYNKDFSVHST GKDNLHTMYLKNLFSEENLKDIVLKLNGEAELFFRKASIKTPIVHKK GSVLVNRSYTQTVGNKEIRVSIPEEYYTEIYNYLNHIGKGKLSSEAQR YLDEGKIKSFTATKDIVKNYRYCCDHYFLHLPITINFKAKSDVAVNE RTLAYIAKKEDIHIIGIDRGERNLLYISVVDVHGNIREQRSFNIVNGYD YQQKLKDREKSRDAARKNWEEIEKIKELKEGYLSMVIHYIAQLVVK YNAVVAMEDLNYGFKTGRFKVERQVYQKFETMLIEKLHYLVFKDR EVCEEGGVLRGYQLTYIPESLKKVGKQCGFIFYVPAGYTSKIDPTTGF VNLFSFKNLTNRESRQDFVGKFDEIRYDRDKKMFEFSFDYNNYIKKG TILASTKWKVYTNGTRLKKIVVNGKYTSQSMEVELTDAMEKMLQR AGIEYHDGKDLKGQIVEKGIEAEIIDIFRLTVQMRNSRSESEDREYDR LISPVLNDKGEFFDTATADKTLPQDADANGAYCIALKGLYEVKQIKE NWKENEQFPRNKLVQDNKTWFDFMQKKRYL |
| SEQ ID NO: 40 | Cas 12 Variant | MEDKQFLERYKEFIGLNSLSKTLRNSLIPVGSTLKHIQEYGILEEDSLR AQKREELKGIMDDYYRNYIEMHLRDVHDIDWNELFEALTEVKKNQ TDDAKKRLEKIQEKKRKEIYQYLSDDAVFSEMFKEKMISGILPDFIRC NEGYSEEEKEEKLKTVALFHRFTSSFNDFFLNRKNVFTKEAIVTAIGY RVVHENAEIFLENMVAFQNIQKSAESQISIIERKNEHYFMEWKLSHIF TADYYMMLMTQKAIEHYNEMCGVVNQQMREYCQKEKKNWNLYR MKRLHKQILSNASTSFKIPEKYENDAEVYESVNSFLQNVMEKTVME RIAVLKNSTDNFDLSKIYITAPYYEKISNYLCGSWNTITDCLTHYYEQ QIAGKGARKDQKVKAAVKADKWKSLSEIEQLLKEYARAEEVKRKP EEYIAEIENIVSLKEAHLLEYHPEVNLIENEKYATEIKDVLDNYMELF HWMKWFYIEEAVEKEVNFYGELDDLYEEIKDIVPLYNKVRNYVTQK PYSDTKIKLNFGTPTLANGWSKSKEYDYNAILLQKDGKYYMGIFNPI QKPEKEIIEGHSQPLEGNEYKKMVYYYLPSANKMLPKVLLSKKGMEI YQPSEYIINGYKERRHIKSEEKFDLQFCHDLIDYFKSGIERNSDWKVF GFDFSDTDTYQDISGFYREVEDQGYKIDWTYIKEADIDRLNEEGKLY LFQIYNKDFSEKSTGRENLHTMYLKNLFSEENVREQVLKLNGEAEIF FRKSSVKKPIIHKKGTMLVNRTYMEEVNGNSVRRNIPEKEYQEIYNY KNHRLKGELSTEAKKYLEKAVCHETKKDIVKDYRYSVDKFFIHLPITI NYRASGKETLNSVAQRYIAHQNDMHVIGIDRGERNLIYVSVINMQG EIKEQKSFNIINEFNYKEKLKEREQSRGAARRNWKEIGQIKDLKEGYL SGVIHEIAKMMIKYHAIIAMEDLNYGFKRGRFKVERQVYQKFENMLI QKLNYLVFKDRPADEDGGVLRGYQLAYIPDSVKKMGRQCGMIFYV PAAFTSKIDPTTGFVDIFKHKVYTTEQAKREFILSFDEICYDVERQLFR FTFDYANFVTQNVTLARNNWTIYTNGTRAQKEFGNGRMRDKEDYN PKDKMVELLESEGIEFKSGKNLLPALKKVSNAKVFEELQKIVRFTVQ LRNSKSEENDVDYDHVISPVLNEEGNFFDSSKYKNKEEKKESLLPVD ADANGAYCIALKGLYIMQAIQKNWSEEKALSPDVLRLNNNDWFDYI QNKRYR |
| SEQ ID NO: 41 | Cas 12 Variant | MEKSLNDFIGLYSVSKTLRFELKPVSETLENIKKFHFLEEDKKKAND YKDVKKIIDNYHKYFIDDVLKNASFNWKKLEEAIREYNKNKSDDSA LVAEQKKLGDAILKLFTSDKRYKALTAATPKELFESILPDWFGEQCN QDLNKAALKTFQKFTSYFTGFQENRKNVYSAEAIPTAVPYRIVNDNF PKFLQNVLIFKTIQEKCPQIIDEVEKELSSYLGKEKLAGIFTLESFNKY LGQGGKENQRGIDFYNQIIGGVVEKEGGINLRGVNQFLNLYWQQHP DFTKEDRRIKMVPLYKQILSDRSSLSFKIESIENDEELKNALLECADKL ELKNDEKKSIFEEVCDLFSSVKNLDLSGIYINRKDINSVSRILTGDWS WLQSRMNVYAEEKFTTKAEKARWQKSLDDEGENKSKGFYSLTDLN EVLEYSSENVAETDIRITDYFEHRCRYYVDKETEMFVQGSELVALSL QEMCDDILKKRKAMNTVLENLSSENKLREKTDDVAVIKEYLDAVQE LLHRIKPLKVNGVGDSTFYSVYDSIYSALSEVISVYNKTRNYITKKAA SPEKYKLNFDNPTLADGWDLNKEQANTSVILRKDGMFYLGIMNPKN KPKFAEKYDCGNESCYEKMIYKQFDATKQIPKCSTQKKEVQKYFLS GATEPYILNDKKSFKSELIITKDIWFMNNHVWDGEKFVPKRDNETRP KKFQIGYFKQTGDFDGYKNALSNWISFCKNFLQSYLSATVYDYNFK NSEEYEGLDEFYNYLNATCYKLNFINIPETEINKMVSEGKLYLFQIYN KDFASGSTGMPNMHTLYWKNLFSDENLKNVCLKLNGEAELFYRPA GIKEPVIHKEGSYLVNRTTEDGESIPEKIYFEIYKNANGKLEKLSDEA QNYISNHEVVIKKAGHEIIKDRHYTEPKFLFHVPLTINFKASGNSYSIN ENVRKFLKNNPDVNIIGLDRGERHLIYLSLINQKGEIIKQFTFNEVERN KNGRTIKVNYHEKLDQREKERDAARKSWQAIGKIAELKEGYLSAVI HQLTKLMVEYNAVVVMEDLNFGFKRGRFHVEKQVYQKFEHILIDKS NYLVFKDRGLNEPGGVLNGYQIAGQFESFQKLGKQSGMLFYVPAGY TSKIDPKTGFVSMMNFKDLTNVHKKRDFFSKFDNIHYDEANGSFVFT FDYKKFDGKAKEEMKLTKWSVYSRDKRIVYFAKTKSYEDVLPTEKL QKIFESNGIDYKSGNNIQDSVMAIGADLKEGAKPSKEISDFWDGLLS NFKLILQMRNSNARTGEDYIISPVMADDGTFFDSREEFKKGEDAKLP LDADANGAYHIALKGLSLINKINLSKDEELKKFDMKISNADWFKFAQ EKNYAK |
| SEQ ID NO: 42 | Cas 12 Variant | MEEKKMSKIEKFIGKYKISKTLRFRAVPVGKTQDNIEKKGILEKDKK RSEDYEKVKAYLDSLHRDFIENTLKKVKLNELNEYACLFFSGTKDD GDKKKMEKLEEKMRKTISNEFCNDEMYKKIFSEKILSENNEEDVSDI VSSYKGFFTSLNGYVNNRKNLYVSDAKPTSIAYRCINENLPKFLRNV ECYKKVVQVIPKEQIEYMSNNLNLSPYRIEDCFNIDFFEFCLSQGGIDL YNTFIGGYSKKDGTKVQGINEIVNLYNQKNKKDKEKYKLPQFTPLFK QILSDRDTKSFSIEKLENIYEVVELVKKSYSDEMFDDIETVFSNLNYY DASGIYVKNGPAITHISMNLTKDWATIRNNWNYEYDEKHSTKKNKN IEKYEDTRNTMYKKIDSFTLEYISRLVGKDIDELVKYFENEVANFVM DIKKTYSKLTPLFDRCQKENFDISEDEVNDIKGYLDNVKLLESFMKSF TINGKENNIDYVFYGKFTDDYDKLHEFDHIYNKVRNYITTSRKPYKL DKYKLYFDNPQLLGGWDINKEKDYRTVMLTKDGKYYFAIIDKGEHP FDNIPKDYFDNNGYYKKIIYRQIPNAAKYLSSKQIVPQNPPEEVKRIL DKKKADSKSLTEEEKNIFIDYIKSDFLKNYKLLFDKNNNPYFNFAFRE SSTYESLNEFFEDVERQAYSVRYENLPADYIDNLVNEGKIYLFEIYSK DFSEYSKGTNNLHTMYFKALFDNDNLKNTVFKLSGNAELFIRPASIK KDELVIHPKNQLLQNKNPLNPKKQSIFDYDLVKDKRFFENQYMLHIS IEINKNERDAKKIKNINEMVRKELKDSDDNYIIGIDRGERNLLYVCVI NSAGKIVEQMSLNEIINEYNGIKHTVDYQGLLDKCEKERNAQRQSW KSIENIKELKDGYISQVVHKLCQLVEKYDAIIAMENLNGGFKRGRTK FEKQVYQKFENKLINKMEYMADKKRKTTENGGILRGYQLTNGCINN SYQNGFIFYVPAWLTSKIDPTTGFVDLLKPKYTNVEEAHLWINKFNSI TYDKKLDMFAFNINYSQFPRADIDYRKIWTFYTNGYRIETFRNSEKN NEFDWKEVHLTSVIKKLLEEYQINYISGKNIIDDLIQIKDKPFWNSFIK YIRLTLQMRNSITGRTDVDYIISPVINNEGTFYDSRKDLDEITLPQDAD ANGAYNIARKALWIIEKLKESPDEELNKVKLAITQREWLEYAQINI |
| SEQ ID NO: 43 | Cas 12 Variant | MIIHNCYIGGSFMKKIDSFTNCYSLSKTLRFKLIPIGATQSNFDLNKML DEDKKRAENYSKAKSIIDKYHRFFIDKVLSSVTENKAFDSFLEDVRA YAELYYRSNKDDSDKASMKTLESKMRKFIALALQSDEGFKDLFGQN LIKKTLPEFLESDTDKEIIAEFDGFSTYFTGFFNNRKNMYSADDQPTAI SYRCINDNLPKFLDNVRTFKNSDVASILNDNLKILNEDFDGIYGTSAE DVFNVDYFPFVLSQKGIEAYNSILGGYTNSDGSKIKGLNEYINLYNQ KNENIHRIPKMKQLFKQILSERESVSFIPEKFDSDDVLSSINDYYLER DGGKVLSIEKTVEKIEKLFSAVTDYSTDGIFVKNAAELTAVCSGAFG YWGTVQNAWNNEYDALNGYKETEKYIDKRKKAYKSIESFSLADIQK YADVSESSETNAEVTEWLRNEIKEKCNLAVQGYESSKDLISKPYTES KKLFNNDNAVELIKNALDSVKELENVLRLLLGTGKEESKDENFYGEF LPCYERICEVDSLYDKVRNYMTQKLYKTDKIKLNFQNPQFLGGWDR NKEADYSAVLLRRNSLYYIAIMPSGYKRVFEKIPAPKADETVYEKVI YKLLPGPNKMLPKVFFSKKGIETFNPPKEILEKYELGTHKTGDGFNL DDCHALIDYFKSALDVHSDWSNFGFRFSDTSTYKNIADFYNEVKNQ GYKITFCDVPQSYINELVDEGKLYLFQLYNKDFSEHSKGTPNLHTLY FKMLFDERNLENVVFKLNGEAEMFYREASISKDDMIVHPKNQPIKN KNEQNSRKQSTFEYDIVKDRRYTVDQFMLHIPITLNFTANGGTNINN EVRKALKDCDKNYVIGIDRGERNLLYICVVDSEGRIIEQYSLNEIINEY NGNTYSTDYHALLDKKEKERLESRKAWKTVENIKELKEGYISQVVH KICELVEKYDAVIVMEDLNLGFKQGRSGKFEKSVYQKFEKMLIDKL NYFADKKKSPEEIGSVLNAYQLTNAFESFEKMGKQNGFIFYVPAYLT SKIDPTTGFADLLHPSSKQSKESMRDFVGRFDSITFNKTENYFEFELD YNKFPRCNTDYRKKWTVCTYGSRIKTFRNPEKNSEWDNKTVELTPA FMALFEKYSIDVNGDIKAQIMSVDKKDFFVELIGLLRLTLQMRNSET GKVDRDYLISPVKNSEGVFYNSDDYKGIENASLPKDADANGAYNIA RKGLWIIEQIKACENDAELNKIRLAISNAEWLEYAQKK |
| SEQ ID NO: 44 | Cas 12 Variant | MKEQFVNQYPISKTLRFSLIPIGKTEENFNKNLLLKEDEKKAEEYQKV KGYIDRYHKFFIETALCNINFEGFEEYSLLYYKCSKDDNDLKTMEDIE IKLRKQISKTMTSHKLYKDLFGENMIKTILPNFLDSDEEKNSLEMFRG FYTYFSGFNTNRKNMYTEEAKSTSIAYRCINDNLPKFLDNSKSFEKIK CALNKEELKAKNEEFYEIFQIYATDIFNIDFFNFVLTQPGIDKYNGIIG GYTCSDGTKVQGLNEIINLYNQQIAKDDKSKRLPLLKMLYKQILSDR ETVSFIPEKFSSDNEVLESINNYFSKNVSNAIKSLKELFQGFEAYNMN GIFISSGVAITDLSNAVFGDWNAISTAWEKAYFETNPPKKNKSQEKY EEELKANYKKIKSFSLDEIQRLGSIAKSPDSIGSVAEYYKITVTEKIDNI TELYDGSKELLNCNYSESYDKKLIKNDTVIEKVKTLLDAVKSLEKLI KPLVGTGKEDKDELFYGTFLPLYTSLSAVDRLYDKVRNYATQKPYS KDKIKLNFNCSSFLSGWATDYSSNGGLIFEKDGLYYLGIVNKKFTTE EIDYLQQNADENPAQRIVYDFQKPDNKNTPRLFIRSKGTNYSPSVKE YNLPVEEIVELYDKRYFTTEYRNKNPELYKASLVKLIDYFKLGFTRH ESYRHYDFKWKKSEEYNDISEFYKDVEISCYSLKQEKINYNTLLNFV AENRIYLFQIYNKDFSKYSKGTPNLHTRYFKALFDENNLSDVVFKLN GGSEMFFRKASIKDNEKVVHPANQPIDNKNPDNSKKQSTFDYELIKD KRFTKHQFSIHIPITMNFKARGRDFINNDIRKAIKSEYKPYVIGIDRGE RNLIYISVINNNGEIVEQMSLNDIISDNGYKVDYQRLLDRKEKERDNA RKSWGTIENIKELKEGYISQVIHKICELVIKYDAVIAMEDLNFGFKRG RFNVEKQVYQKFENMLISKLNYLCDKKSEANSEGGLLKAYQLTNKF DGVNKGKQNGIIFYVPAWLTSKIDPVTGFVDLLHPKYISVEETHSLFE KLDDIRYNFEKDMFEFDIDYSKLPKCNADFKQKWTVCTNADRIMTF RNSEKNNEWDNKRILLSDEFKRLFEEFGIDYCHNLKNKILSISNKDFC YRFIKLFALTMQMRNSITGSTNPEDDYLISPVRDENGVFYDSRNFIGS KAGLPIDADANGAYNIARKGLWAINAIKSTADDMLDKVDLSISNAK WLEYVQK |
| SEQ ID NO: 45 | Cas 12 Variant | MADLSQFTHKYQVPKTLRFELIPQGKTLENLSAYGMVADDKQRSEN YKKLKPVIDRIYKYFIEESLKNTNLDWNPLYEAIREYRKEKTTATITN LKEQQDICRRAIASRFEGKVPDKGDKSVKDFNKKQSKLFKELFGKEL FTDSVLEQLPGVSLSDEDKALLKSFDKFTTYFVGFYDNRKNVFSSDD ISTGIPHRLVQENFPKFIDNCDDYKRLVLVAPELKEKLEKAAEATKIF EDVSLDEIFSIKFYNRLLQQNQIDQFNQLLGGIAGAPGTPKIQGLNETL NLSMQQDKTLEQKLKSVPHRFSPLYKQILSDRSSLSFIPESFSCDAEVL LAVQEYLDNLKTEHVIEDLKEVFNRLTTLDLKHIYVNSTKVTAFSQA LFGDWNLCREQLRVYKMSNGNEKITKKALGELESWLKNSDIAFTEL QEALADEALPAKVNLKVQEAISGLNEQMAKSLPKELKIPEEKEELKA LLDAIQEVYHTLEWFIVSDDVETDTDFYVPLKETLQIIQPIIPLYNKVR NFATQKPYSVEKFKLNFANPTLADGWDENKEQQNCAVLFQKGNNY YLGILNPKNKPDFDNVDTEKQGNCYQKMVYKQFPDFSKMMPKCTT QLKEVKQHFEGKDSDYILNNKNFIKPLTITREVYDLNNVLYDGKKKF QIDYLRKTKDEDGYYTALHTWIDFAKKFVASYKSTSIYDTSTILPPEK YEKLNEFYGALDNLFYQIKFENIPEEIIDTYVEDGKLFLFQIYNKDFA AGATGAPNLHTIYWKAVFDPENVKDVVVKLNGQAELFYRPKSNMD VIRHKVGEKLVNRTLKDGSILTDELHKELYLYANGSLKKGLSEDAKI ILDKNLAVIYDVHHEIVKDRRFTTDKFFFHVPLTLNYKCDKNPVKFN AEVQEYLKENPDTYVIGIDRGERNLIYAVVIDPKGRIVEQKSFNVING FDYHGKLDQREKERVKARQAWTAVGKIKELKQGYLSLVVHEISKM MVRYQAVVVLENLNVGFKRVRSGIAEKAVYQQFEKMLINKLNYLM FKDAGGTEPGSVLNAYQLTDRFESFAKMGLQTGFLFYIPAAFTSKIDP ATGFVDPFRWGAIKTLADKREFLSGFESLKFDSTTGNFILHFDVSKNK NFQKKLEGFVPDWDIIIEANKMKTGKGATYIAGKRIEFVRDNNSQGH YEDYLPCNALAETLRQCDIPYEEGKDILPLILEKNDSKLLHSVFKVVR LTLQMRNSNAETGEDYISSPVEDVSGSCFDSRMENEKLPKDADANG AYHIALKGMLALERLRKDEKMAISNNDWLNYIQEKRA |
| SEQ ID NO: 46 | Cas 12 Variant | MTNFDNFTKKYVNSKTIRLEAIPVGKTLKNIEKMGFIAADRQRDEDY QKAKSVIDHIYKAFMDDCLKDLFLDWDPLYEAVVACWRERSPEGR QALQIMQADYRKKIADRFRNHELYGSLFTKKIFDGSVAQRLPDLEQS AEEKSLLSNFNKFTSYFRDFFDKRKRLFSDDEKHSAIAYRLINENFLK FVANCEAFRRMTERVPELREKLQNTGSLQVYNGLALDEVFSADFYN QLIVQKQIDLYNQLIGGIAGEPGTPNIQGLNATINLALQGDSSLHEKL AGIPHRFNPLYKQILSDVSTLSFVPSAFQSDGEMLAAVRGFKVQLESG RVLQNVRRLFNGLETEADLSRVYVNNSKLAAFSSMFFGRWNLCSDA LFAWKKGKQKKITNKKLTEIKKWLKNSDIAIAEIQEAFGEDFPRGKIN EKIQAQADALHSQLALPIPENLKALCAKDGLKSMLDTVLGLYRMLQ WFIVGDDNEKDSDFYFGLGKILGSLDPVLVLYNRVRNYITKKPYSLT KFRLNFDNSQLLNGWDENNLDTNCASIFIKDGKYYLGISNKNNRPQF DTVATSGKSGYQRMVYKQFANWGRDLPHSTTQMKKVKKHFSASD ADYVLDGDKFIRPLIITKEIFDLNNVKFNGKKKLQVDYLRNTGDREG YTHALHTWINFAKDFCACYKSTSIYDISCLRPTDQYDNLMDFYADLG NLSHRIVWQTIPEEAIDNYVEQGQLFLFQLYNKDFAPGADGKPNLHT LYWKAVFNPENLEDVVVKLNGKAELFYRPRSNMDVVRHKVGEKLV NRKLKNGLTLPSRLHEEIYRYVNGTLNKDLSADARSVLPLAVVRDV QHEIIKDRRFTADKFFFHASLTFNFKSSDKPVGFNEDVREYLREHPDT YVVGVDRGERNLIYIVVIDPQGNIVEQRSFNMINGIDYWSLLDQKEK ERVEAKQAWETVGKIKDLKCGYLSFLIHEITKIIIKYHAVVILENLSLG FKRVRTGIAEKAVYQQFERMLVTKLGYVVFKDRAGKAPGGVLNAY QLTDNTRTAENTGIQNGFLFYVPAAFTSRVDPATGFFDFYDWGKIKT ATDKKNFIAGFNSVRYERSTGDFIVHVGAKNLAVRRVAEDVRTEWD IVIEANVRKMGIDGNSYISGKRIRYRSGEQGHGQYENHLPCQELIRAL QQYGIQYETGKDILPAILQQDDAKLTDTVFDVFRLALQMRNTSAETG EDYFNSVVRDRSGRCFDTRRAEAAMPKEADANDAYHIALKGLFVLE KLRKGESIGIKNTEWLRYVQQRHS |
| SEQ ID NO: 47 | Cas 12 Variant | MENYGGFTGLYPLQKTLKFELRPQGRTMEHLVSSNFFEEDRDRAEK YKIVKKVIDNYHKDFINECLSKRSFDWTPLMKTSEKYYASKEKNGK KKQDLDQKIIPTIENLSEKDRKELELEQKRMRKEIVSVFKEDKRFKYL FSEKLFSILLKDEDYSKEKLTEKEILALKSFNKFSGYFIGLHKNRANFY SEGDESTAIAYRIVNENFPKFLSNLKKYREVCEKYPEIIQDAEQSLAG LNIKMDDIFPMENFNKVMTQDGIDLYNLAIGGKAQALGEKQKGLNE FLNEVNQSYKKGNDRIRMTPLFKQILSERTSYSYILDAFDDNSQLITSI NGFFTEVEKDKEGNTFDRAVGLIASYMKYDLSRVYIRKADLNKVSM EIFGSWERLGGLLRIFKSELYGDVNAEKTSKKVDKWLNSGEFSLSDV INAIAGSKSAETFDEYILKMRVARGEIDNALEKIKCINGNFSEDENSK MIIKAILDSVQRLFHLFSSFQVRADFSQDGDFYAEYNEIYEKLFAIVPL YNRVRNYLTKNNLSMKKIKLNFKNPALANGWDLNKEYDNTAVIFL REGKYYLGIMNPSKKKNIKFEEGSGTGPFYKKMAYKLLPDPNKMLP KVFFAKKNINYYNPSDEIVKGYKAGKYKKGENFDIDFCHKLIDFFKE SIQKNEDWRAFNYLFSATESYKDISDFYSEVEDQGYRMYFLNVPVA NIDEYVEKGDLFLFQIYNKDFASGAKGNKDMHTIYWNAAFSDENLR NVVVKLNGEAELFYRDKSIIEPICHKKGEMLVNRTCFDKTPVPDKIH KELFDYHNGRAKTLSIEAKGYLDRVGVFQASYEIIKDRRYSENKMYF HVPLKLNFKADGKKNLNKMVIEKFLSDKDVHIIGIDRGERNLLYYSV IDRRGNIIDQDSLNIIDGFDYQKKLGQREIERREARQSWNSIGKIKDLK EGYLSKAVHKVSKMVLEYNAIVVLEDLNFGFKRGRFKVEKQVYQK FEKMLIDKLNYLVFKEVLDSRDAGGVLNAYQLTTQLESFNKLGKQS GILFYVPAAYTSKIDPTTGFVSLFNTSRIESDSEKKDFLSGFDSIVYSA KDGGIFAFKFDYRNRNFQREKTDHKNIWTVYTNGDRIKYKGRMKG YEITSPTKRIKDVLSSSGIRYDDGQELRDSIIQSGNKVLINEVYNSFIDT LQMRNSDGEQDYIISPVKNRNGEFFRTDPDRRELPVDADANGAYHIA LRGELLMQKIAEDFDPKSDKFTMPKMEHKDWFEFMQTRGD |
| SEQ ID NO: 48 | Cas 12 Variant | MLHAFTNQYQLSKTLRFGATLKEDEKKCKSHEELKGFVDISYENMK SSATIAESLNENELVKKCERCYSEIVKFHNAWEKIYYRTDQIAVYKD FYRQLSRKARFDAGKQNSQLITLASLCGMYQGAKLSRYITNYWKDN ITRQKSFLKDFSQQLHQYTRALEKSDKAHTKPNLINFNKTFMVLANL VNEIVIPLSNGAISFPNISKLEDGEESHLIEFALNDYSQLSELIGELKDAI ATNGGYTPFAKVTLNHYTAEQKPHVFKNDIDAKIRELKLIGLVETLK GKSSEQIEEYFSNLDKFSTYNDRNQSVIVRTQCFKYKPIPFLVKHQLA KYISEPNGWDEDAVAKVLDAVGAIRSPAHDYANNQEGFDLNHYPIK VAFDYAWEQLANSLYTTVTFPQEMCEKYLNSIYGCEVSKEPVFKFY ADLLYIRKNLAVLEHKNNLPSNQEEFICKINNTFENIVLPYKISQFETY KKDILAWINDGHDHKKYTDAKQQLGFIRGGLKGRIKAEEVSQKDKY GKIKSYYENPYTKLTNEFKQISSTYGKTFAELRDKFKEKNEITKITHF GIIIEDKNRDRYLLASELKHEQINHVSTILNKLDKSSEFITYQVKSLTS KTLIKLIKNHTTKKGAISPYADFHTSKTGFNKNEIEKNWDNYKREQV LVEYVKDCLTDSTMAKNQNWAEFGWNFEKCNSYEDIEHEIDQKSYL LQSDTISKQSIASLVEGGCLLLPIINQDITSKERKDKNQFSKDWNHIFE GSKEFRLHPEFAVSYRTPIEGYPVQKRYGRLQFVCAFNAHIVPQNGE FINLKKQIENFNDEDVQKRNVTEFNKKVNHALSDKEYVVIGIDRGLK QLATLCVLDKRGKILGDFEIYKKEFVRAEKRSESHWEHTQAETRHIL DLSNLRVETTIEGKKVLVDQSLTLVKKNRDTPDEEATEENKQKIKLK QLSYIRKLQHKMQTNEQDVLDLINNEPSDEEFKKRIEGLISSFGEGQK YADLPINTMREMISDLQGVIARGNNQTEKNKIIELDAADNLKQGIVA NMIGIVNYIFAKYSYKAYISLEDLSRAYGGAKSGYDGRYLPSTSQDE DVDFKEQQNQMLAGLGTYQFFEMQLLKKLQKIQSDNTVLRFVPAFR SADNYRNILRLEETKYKSKPFGVVHFIDPKFTSKKCPVCSKTNVYRD KDDILVCKECGFRSDSQLKERENNIHYIHNGDDNGAYHIALKSVENL IQMK |
| SEQ ID NO: 49 | Cas 12 Variant | MKNGINLFKTKTTKTKGVDMEKYQITKTIRFKLLPDNAHEIVEKVKS LKTSNVDELMDEVKNVHLKGLELLFALKKYFYFDGNQCKSFKSTLE IKARWLRLYTPDQYYLKKSSKNSYQLKSLSYFKDVFNDWLFNWEES VSELAIIYEKYKICQHQRDSRADIALLIKKLSMKEYFPFISDLIDCVND KNSNKTFLMKLSEELSVLLEKCNSRALPYQSNGIVVGKASLNYYTVS KSEKMLQNEYEDVCQSLDKNYDITEMKVILYKEKLDNLNFKDVTIA NAYNLLKENKALQKRLFSEYVSQGKVLSLIKTELPLFSNINDNDFEK YKEWSNEIKKLADKKNTFCKKTQQDKIKDIQNKISELKKKRGALFQ YKFTSFQKHCDNYKKVAVQYGKLKARKKAIEKDEIEANLLRYWSVI LEQEDKHSLVLIPKNNAKDAKQYIETINTKGGKYIIHHLDSLTLRALN KLCFNAVDIEKGQMVRENTFYQGIKEEFERNKINCDNQGVLKIQGLY SFKTEGGQINEKEAVEFFKEVLKSNYAREVLNLPYDLESNIFQKEYT NLDQFRQDLEKCCYALHSKIGKDDLDEFTRRFEAQVFDITSIDLKSK KEKTKTTGEMKKHTQLWLEFWKGAIEQNFATRVNPELSIFWRAPKS SREKKYGKGSDLYDPNKNNRYLYEQYTLALTITENAGSHFKDIAFK DTSKIKEAIKEFNMSLSQSKYCFGIDRGNAELVSLCLIKNEKDFPFEKF PVYRLRDLTYQGDFKDKHDQMRYGVAIKNISYFIDQEDLFEKNNLS AIDMTTAKLIKNKIVLNGDVLTYLKLKEETAKHKLTQFFQGSSINKN SRVYFDEDENVFKITTNRNHNPEEIIYFYRGEYGAIKNKNDLEDILNE YLCKMETGESEIVLLNRVNHLRDAISANIVGILSYLIDLFPETIVALEN LAKGTIDRHVSQSYENITRRFEWALYRKLLNKQLAPPELKENILLRE GDDKIDQFGIIHFVEEKNTSKDCPNCRKTTQQTNDNKFKEKKFVCKS CGFDTSKDRKGMDSLNSPDTVAAYNVARKKFES |
| SEQ ID NO: 50 | Cas 12 Variant | MAKETKEFKTFDDFTNLYEVQKTLRFELEAVPETEIVLENRGIWYKR DKKRADEKPIVKFYMDILHREFTDEALEKIKESGVLNLSGYFKLFEEL RRLQNHGANTKEEKKLKLEEIRAKKREISNELSQIRRVFSVRGFDVV DSDWKKKYTIEGKKIKNDKSKTYLILSENILNFLENRFTSKEVERLRSI DKKHVEDYGNVVNSGGENIFATFKGFFGYFDSLIKNRENFYETDGK AGRVATRSVDENLNFFAENLHIFSTDLPKALKDDLSDTQKAIFERSY YKNCLLQKDIKSYNLIIGDINKEINKHRQQRDTKIKFLNTLFKQILSIE EKEQYKHIEINNDEDLIRAIRDFISLNESKISEGTKIFNQFIQRCLQKED LGQIYLPKDSVNTIAHRIFKPWDEIMALFDRKYFVSLEEIKDLTESSV WKERVLEESKTKSLIFKDTHIHTIISGQEIFSNFILILEKEYKNQFSGFIS ETRRGKAAFVGYDESLKNLRATIKWFEGKNLKLSETEKVEWIKAIK DYADAALRIFQMTKYLWLPVVGDEEDKDYLRIKAEIDQLTKDNDFY NKINAFIDGYKPEPFIYRSSFQEYLTRRPFSTDKFKINFENSRLLDGWD KDMIDDRMGILLQRDGDYFLGILNKEDRHCLDNLVDVKSEDKNSYA LMQFKQLTGLYRQLPRMAFPKKKQPVLEANAEIKKIKEDFDFLQKQ KKEREVNVNVVFDNKKLNLLINHYAEFLKENYKDEKCYDFSLLNKE KVYESLSDFYADVDKITYSLSFIQVSIDQLIKTGKILLFRLKNKDLLKG SLGQNKNLHTYYFHALFERENLSQGRIRLGAQAEIFFRPASIEKEKDK NRSNALKKSPKTRYVKEILKNKRYSEDKVFLHLPIQLNADAYDLPSI NQNVFEFIKNRQEKVKIIGIDRGEKNLAYYSVISQNSNGKIKIEEPPRD LNLGYLEPLDELENKRQDERKAWQSISEIKSKRDGYISYAVSKIVEL MLKYQAIIVLEDLSGKFKRSRMKFEKAPYQQLELALIKKLNYLVKK NSKSGKPGHYLSAYQLTEPVGSYKEMGKQTGIIFYTQAGYTSRTCPT CGWRKRVQGLYYKDRTSAQRRFDPKTGVKIFYDSVNDRFVFQYHP VYEQKELKEWDKEIYSDVTRIRWNNEEKKNNEYRKGDITLKIKRLFR DRGIDLSRNINEQLVNVGDASFWEELINLLRLITEIRNIDNENNRDFIE CPHCHFQSENGFHGVAWNGDANGAYNIARKGLLITKAVCDPEKNV GDITWSDLKVDMKDWDAATDEWAKKNPEK |
| SEQ ID NO: 51 | Cas 12 Variant | MENEKIFSDLTNRYQVVKTLPFELKPVPRTRVLLGLDNPNKGEIFSK DRERAENFTIIKKYIDRLHSLFINESLKKADIDFSNFYKQYGKNINTKN NKNIDDDNDINDDEKEDSENDNLKKYRQEIANLFNKSKYKSWVNVG KDGDKISGMLFEKGLIDLLRTHFSDNLNEDIEIPELFSNKKIKDTRKLK EIINSFGKDGKDGQNFTTYFSVSFHNNRKNYYKSDGKMGRVSTRIVD ENLERFCKNIYLYKEIIGKNEIKEIFSGNWDIYLQKKPNFSNDKTYKK LDEFKNDKYDWEMIFRDVNSYNKYFLQSDIEFYNYIRGKLNQDINEY NGKKRDSKEKINSQFENLRNQVHGEKKNYDDDFEIDEDNIIQFINEIF VRHNQNKMRFSEKLFSDFIDLLMVDNGDKLDKVYFSQKAVENAIAR YYFVEETTNEGREPLLISLLLQNAGKDRKKLSNKPIKLGDIKFVLDQA NNKPAEDIFKNRYVLSESNNDGIINANDKNHWANLLRLIKKDFYFHK DNLIKSQDKLALETKYNKGSDEGERQIETIKNFAESAKAILRMTKYF DLRKNGVIQNVIGGKDPIHEEVDKYFDGDVLSGEESCRISKYYDALR NFITKKAWSADKIILNFDCSEFLGGWDRSQEQKKRGIILRHRDGDEE RYYLAVLGKNGKQYFENRTLFKGCESSDWQKIEYNVIQKPHMSLPK NLITPFFKKDKITNERFIDRSKKGAKALIEIDINPSDEFLNNYNLGKHT KENLDKSFLCDYFKYLMDAIAKYYKGEFNFNFPDVSNFDNTQPFYSF IEKNAYSIKYFGISSKEIEKLIADCYYKEDVYLFQIYCKDFEIDPKIGK AKYGNEFRTKAEIRKSKGEEAGNENLNTKYFKLLFDEKNLKNQNGI VYKLNGGAKMFYRPSSIKKDEKIDGKWRYKEDKYSLNITITCNFSSK KDDLSIDKDINKKIAEVNANSDFRIISIDRGEKNLAYCCVMDENANIL DIKSLNRITRYDKNGKAIKEKNMFHEVKDGKLCYGEPVYDFYKDYQ NLLDEREIKRLVNRRSWNVIEDIKNLKKGYVALLINYICKAVVIAINE GKYPIIVLESLDKGMLHNRVKIEKQIYRGVEEGLVRKLNYFVDKKTD NVLNAWQLLAKFETVGSSLDRKKQLGIIFYVDPGYTSITCPCCGFRQ RKYIKAERAEENFKEIKIKFDGKRYSFAYDYRCIDDNGKEKSKEDIIY SNVKRLLRSGRNGRAVQIEDVTDELTNLFKKHNINIEQDINEQLAGK DNKFWKQLLWWFNAIEQIRNTQSLRRKFNTEENKLEILENNDCDFIL CPHCYFDSNKDKFQNKIWNGDANGAFNIGRKGIIDIFEIKKHQRMLS DFMEQWGIDKLPKANGGNQAVIEIVKNDKKYNLCILNNKKIPYYCL RIGKEKIDSIADDRKCNQLPDLMVNWKKWDMWLDKWGK |
| SEQ ID NO: 52 | Cas 12 Variant | MPEVKNVFQDFTNLYELSKTLRFELKPVPETEKILELNAAKTKKFPK DLYRAENFEIIKKYTDELHRTYIRETLNNVNIDYLKFLEIFRINGKKKN EMTDENEESDENNEKDDIQKIKKELRSKIGNLFNKWNNDKDNKFKD WVKIDVGKKEKEVSGDLFGKELITILKNYFKNKLDSKVNVPMLFFNE QEIKNGEAKKQRKLEAVFENFDKFTTYFTDSFYNNRKNYYKTEGRV GQVATRIIDENLPRFCSNLIAFNEVVSLYSTLLNNFDLGWKEYLNEK KINQTWVEKFELSNYDWKALFNDVNYYNQCLLQEGIDKYNYIIKKL NKDINEYTQNKYKSVEKGNNNNPDINFFQKLHKQIHGERDFKLIEIDI DENNIFTKILPEFILHSDMKLMTKIDEEVGVEEIVGAERIIKIFIKQELK DLEKIYLSRRAIETISAKWFHSWETLKDLILGYLNKDLLESKKRKKVP DFVDFNIIKIVLENNKDDYKDLFKRKYFEADKNEFVDWIDSSGGTKK LEFGGENWINFLNVFEYEFGTLLTEYKKNKNALLYLIDKKIDYDKNN EVGQTAAIKNFADSALGIFRMVSYFALRKKGVMVEPKNGKDEIFYA FVDRYLDGDDNDREEQNKIVQYYNTLRNFVTQKAWSIDKVRLCFDC GEFLKGWDKDKIHERLGIILRNNNKFYLGILNKNHKQIFIKIKSHDNN NFYYVIYDYKQLNNVYRQIPRLAFPSRSVKKGDAYMLRAIQERKKK FFLEDEEFIELQEIKNEYDKIGNDLSKEKLTKLIEYYKKVVISNYSSLY NVSNLNNKKFNSINEFNQYVENLMYSLIPTRISPDFIKEKISKGELYLF QIYNKDFELDESIGKEKFGEDFAPVIMDGKNNLHTEYFKLLFNDSNL KNPNGVVFKLSGGAKMFYRPATENLPIKKDRDGNIIKNKKGENVIVG QRYKEDKYFLHLPIILNFVNKGKNYSINDMVNKAITNASDDQDKFRII GLDRGEKHLVYYSVINERQEIIEIGSLNNISRKDNKGEIIEEKNWYHD KFGNIEKEPTKEYHKDYHNLLDQREIERLKSRQSWEKIENIKELKEG YISAVINKICNLVIKAIKENKIPIVALENLNSGMKRGRIKIDKQIYQKL ELKLAKKLNFLVDKKEKNYLSAWQFTPKIETFSGDIEKKNQVGIIFY VDPAFTSATCPNCGFRKRIKMDPQNAKKKIKDMEITYENGIYKFDYP IENGENDVVYSDVERLKWDNEKKKVIKTKNVSDDFGKLFEDIKDKN NLKKELLSIGEENKEFWKEFSRCFNLLLRIRNSKLIKRKLNDDTGKVE IIADDDLADRDRDFIYCPQCHFHSEGGDVFGEFVKKKYLGKDNFEFN GDANGAYNIARKTIIAVNKIKDYQLGLNHFIEKYRISELPNNGKDKK NIFYNNNSYILSFFEVQDEKFRKVKVYGLKKDGDRQIIQKKEMWYR RYPDIFVNNKEWDKFVQNKS |
| SEQ ID NO: 53 | Cas 12 Variant | MLFFMSTDITNKPREKGVFDNFTNLYEFSKTLTFGLIPLKWDDNKKM IVEDEDFSVLRKYGVIEEDKRIAESIKIAKFYLNILHRELIGKVLGSLKF EKKNLENYDRLLGEIEKNNKNENISEDKKKEIRKNFKKELSIAQDILL KKVGEVFESNGSGILSSKNCLDELTKRFTRQEVDKLRRENKDIGVEY PDVAYREKDGKEETKSFFAMDVGYLDDFHKNRKQLYSVKGKKNSL GRRILDNFEIFCKNKKLYEKYKNLDIDFSEIERNFNLTLEKVFDFDNY NERLTQEGLDEYAKILGGESNKQERTANIHGLNQIINLYIQKKQSEQK AEQKETGKKKIKFNKKDYPTFTCLQKQILSQVFRKEIIIESDRDLIREL KFFVEESKEKVDKARGIIEFLLNHEENDIDLAMVYLPKSKINSFVYKV FKEPQDFLSVFQDGASNLDFVSFDKIKTHLENNKLTYKIFFKTLIKEN HDFESFLILLQQEIDLLIDGGETVTLGGKKESITSLDEKKNRLKEKLG WFEGKVRENEKMKDEEEGEFCSTVLAYSQAVLNITKRAEIFWLNEK QDAKVGEDNKDMIFYKKFDEFADDGFAPFFYFDKFGNYLKRRSRNT TKEIKLHFGNDDLLEGWDMNKEPEYWSFILRDRNQYYLGIGKKDGE IFHKKLGNSVEAVKEAYELENEADFYEKIDYKQLNIDRFEGIAFPKKT KTEEAFRQVCKKRADEFLGGDTYEFKILLAIKKEYDDFKARRQKEK DWDSKFSKEKMSKLIEYYITCLGKRDDWKRFNLNFRQPKEYEDRSD FVRHIQRQAYWIDPRKVSKDYVDKKVAEGEMFLFKVHNKDFYDFE RKSEDKKNHTANLFTQYLLELFSCENIKNIKSKDLIESIFELDGKAEIR FRPKTDDVKLKIYQKKGKDVTYADKRDGNKEKEVIQHRRFAKDAL TLHLKIRLNFGKHVNLFDFNKLVNTELFAKVPVKILGMDRGENNLIY YCFLDEHGEIENGKCGSLNRVGEQIITLEDDKKVKEPVDYFQLLVDR EGQRDWEQKNWQKMTRIKDLKKAYLGNVVSWISKEMLSGIKEGVV TIGVLEDLNSNFKRTRFFRERQVYQGFEKALVNKLGYLVDKKYDNY RNVYQFAPIVDSVEEMEKNKQIGTLVYVPASYTSKICPHPKCGWRER LYMKNSASKEKIVGLLKSDGIKISYDQKNDRFYFEYQWEQEHKSDG KKKKYSGVDKVFSNVSRMRWDVEQKKSIDFVDGTDGSITNKLKSLL KGKGIELDNINQQIVNQQKELGVEFFQSIIFYFNLIMQIRNYDKEKSGS EADYIQCPSCLFDSRKPEMNGKLSAITNGDANGAYNIARKGFMQLC RIRENPQEPMKLITNREWDEAVREWDIYSAAQKIPVLSEEN |
| SEQ ID NO: 54 | Cas 12 Variant | MTIKKHKPFTNFECLTPVQKTLRFRLIPVGRTTEFVKCRNIIEADRKRS EMYPLLKELADRFYREFMTDQLSNLLFDWSPLVEALLLARNNTDPR ENQRIASLVRDEQKKYRTLLLKRLSGQVDRNGTPLPKNTASVNKKY YDDLFKARFVTETLPAYLEHLKNKPDGRISDELFDAYKDALDSYQKF TSRLTNFWQARKNIFTDEDIATGFAYRIVHEIVPDYLFNRRVYEQHK LDFPEPLDLLETELKKKNLIANDESLDALFTIPAINRLLTQKGVDLHN AVIGGFFTDDHTKVQGFNELANLKNQTLKNVSDNSEIKPVGKMTRL KKHILSISESTSFLFEQIESDDDLLARIIEFNNTLSEPDIDGLSIADINDQ LYNIMTGVDPSTILVHARNLNKLSHEASLSWNRLRDGLYQMATESP YREDERFKRYIDASEEERDLSKLKNDIYFSLQELQFALDQSIDLEEEA TPTEDIFLPFEFPGMDLKSELTVLFRSIEQLISSETKLIGNPDAIATIKKY LDAIMARYSIWNLLSCEAVELQDDLFYPEYDRVMGSLSNIILLYNLA RNYLSRKPSSKEKFRLNFDKPTLADGWSESKVPDNFSVLLRKDDLFY LGILKDRKAYRVLSYENCDETAKNIKGYYERMIYHFSPDAYRMIPKC STARKDVKKHFGEQGETTGYTLYPGASNFVKPFTIPYEIYRLQTELV NDKKRYQADYLKQTEDEEGYRQAVTAWIDFCKSYLESYEGTSTFDY SHLLKSEDYEDVNQFYADVDRASYSIYFEKVSVDLIHTMVDRGDLY LFQLYNKDFSPHSTGKPNLHTMYWRALFSNDNLQNNTIKLNGQAEL FYRPKQVEQPTVHLQGSYLLNRFDKHGDVIPAGLYCEIYNHINERHP EGYTLSEEATQGLLDGRFVYREAPFELVKDKRYTEDQLFLHVPLEFN WTASANVPFENLANEYIKKDSDLHIIGIDRGERNLLYYSVINLQGDIV KQGSLNTLIQQTTLKGETVERQIPYQSMLKQREDERAEARQNWQSID RIKDLKEGYLSHVIYKLSRLIIKYHAIVVMENLNVGFKRGRFKVERQ VYQKFEVALINKLNALSFKEYEPNELGGVMRPWQLARRVVSPEDTR SQNGIVFYVPASYTSIVDPVTGFANLFYLNRIRNKDLNSFYGHFQEIR YDHEFDRFIFRFNYADFGVFCRIKNVPSRTWNLVSGERKAFNPKRRM IEKRDTTDEIKKALEAHGIAYQNEQNLLPLLLENENLLARIHRSFRLV LQLRNSDSDRDDIVSPALDKENNTFDSGQQPYESSLPINADANGAYN IARKGLLLVDKVKNDKRAVLSNREWFEYLMAEE |
| SEQ ID NO: 55 | Cas 12 Variant | MENKDYSLSRFTKQYQNSKTVRFALTPIGRTEEYIIQNQYIEAARRKN QAYKIVKPIIDEKFRSMIDDVLTHCEKQDWVTLDKLILQYQNNKCRE NMDALAEQQEEIRKNISEEFTKSDEYKNFFGKEDSKKLFKIFLPEYLN QINASESDKEAVNEFQKFKTYFSNFLIVRADIFKADNKHNTIPYRIVN ENFMIFAGNKRTFSNIIRLIPNALEEIAKDGMKKEEWSFYNIQNVDSW FEPDSFQMCMSQKGIQKYNFIIGLVNSYINLYTQQNPQATEVKRSRL KLRMLHKQILSDRVNPSWLPEQFKEGEEGEKQIYEAILALENDLIKN CFDKKYDLWIQSIDIQNPRIYIAASEMARVSSALHMGWNGLNDVRK TILLKSDKKQAKVEKILKQDVSLKDLSDTLNRYADIYKEEQIPSLYQY IEYGSELLQDCAITRKEYHDLLNGNSNTLSLNQNEKLIEGLKAYLDS YQAIVHFLNVFIVGDELDKDTDFYAELDGLVESLSEIVPLYNKVRNYI TRKVYSLDKMRIMFERSDFLGGWGQSFDTKEALLFQKDNLYYIGIIE KKYTNMDVEYLHEGIKEGNRAIRFIYNFQKADNKNIPRTFIRSKGTN YAPAVRKYNLPIESIIDIYDVGKFKTNYKKINEKEYYESLEKLIDYFK DGILKNENYKKFHFNWKPSNEYENINEFYNDTNNACFLLEKEEINYD HLKEQANQGKIYLFQISSKDFNEGSKGTPNLQTMYWRELFSNQNCK DGVIKLCGGASIYMRDASIKQPVVHRKNAWLINKWYKVNGQNVVIP DNTYVKFTKIAQERMNEDELTPQERQLWNSGLIQKKKATHDIMKDR RFTKKQYMLHAPLTINYKQQDSPRYFNEKVRSFLKDNPDINIIGIDRG EKNLIYITIIDQKGNILKGMQKSFNQIEEKGKEGRTIDYYSKLESVEAR HDAARKNWKQIGTIRELKEGYLSQVVHEITQLMIQYNAVIVMENLN MGFKKGRMKVEKSVYQKFEKMLIDKMNYLAFKRDMQGNAIDPYE VGGVMNGYQLTDRFTSFADMGSQNGFIFYVPAAYTSVIDPVTGFVN VFQKTEFKTNDFLHRFDSISWNDKEQSFVFTFDYQNFKCNGTCYQN KWSLYADVDRIETIIKNNQVDRIEPCNPNQKLIDFFDKKGIIYRDGHNI VDDLEKYDSKTISEIIHNFKLILQLRNSMRNPDTGEIIDYIASPVMHNE ERFDSRKRNPELPQDADANGAYHIALKGLMFLQKINEYADSDGNMD NRKLKITNEEWFKYMQTRKEHTYF |
| SEQ ID NO: 56 | Cas 12 Variant | MSNKTSSITTTNKLSYTGFHNNGKQSKTLMFELKPIGRTTEHLDRKG YLADDIDRAESYKTFKEIADNFHKNLIEESLATFTFSDTLKDYFDLWL SPVRTNEDTPKLRKMEAKLRKELSSALKQHPSFAATSSGKRLIDEAL YPNASDKERQCLDRFKGRSSYLDSYTEVRSFIYTDLCKHNTIAYRVV NENLKIYLENILAYEKLMQTAVNGKLETVKEMFHDLYPTFSMDISIFF TSYGFDYCLSQNAITRYNILLGGWSDDNGIHHKGLNNYINEYNQTVP RNKRLPKLNKLQKMILSEENSMSFIIDKFENDVDLANAIRYWLKNCQ FDALNLLIWTLDVHYNLDEIHFKNDNQGKNISDLSQALFKNHHVIRD AWDYDYDIVNAKAKSRQKPERYAEKRDKAFKKINSFSLSYLANILS QYDNQYANFVAQFKTRISVHIQNVQQMIADKTLDMRLDPLMLLKSI SSDTKLVEDIKRVLDSLKDMQRMLTPLLGEGTEPNRDAMFYSDFEPL MNYVDTLTPLYNKVRNYITKKPYSTKKTSLYFGASNFGSGFDVTKLP VSHTIIMRDKGCYYLAVIDNNKLIDKLYDHNDNDGYEYMVYKQIPS PIKYFSLKNILPQDPPDDIRQLLEDRKNGAKWSHDDETRFIDYIVNEF LPTYPPIHDKNGNPYFSWKFKNPDEYESLNEFFDDVSKQAYQTSFRF VSRDFVDDAVENGDIFFFQIYNQDFSPASHGKPSPHTLWFRALFSDV NLETKDIRLKGNATAYFRPASIFYTDEKWRKGHHYEQLKNKFKFPII KDKRYALDKFFFHITLEINCNATVEKYFNNRVNEEIRKADRYNILAIN RGERNLLYAVVMDQDGTILEQKSFNIIKSELPNKTVKETDYWKKLH AREKERDTARKSWKSIECIKDLKKGYLSYVVKTITDMMFEYNAVLV MENLDIEMKRSRQKIEKNVYAQFQNAIIQKLSMYVNKDIDLHIARTA PGGTLNPYQLTYIPASRTKTPKQNGFVFFLNPWNITEIDPTTGFVDLF QTCFRTKNEYKDFFAKFKDIRYNEAQGWFEFDTDYTYFRDKEKAGK RTRWNICSYGTRLRRFRNPDKNYAEDAMTVYPTQMLKDLFDEYNIP YAPASAKSTSISIKDDIIQIDKLDFYKKLLYILKLIVQLRNTSPSSTEQE DDYIISPVINEDTNWFYDSRDYNEESLLPCNTDANGAYSLALKCNMV IDRIKNTIPGEPVDMYISNADWLDARQ |
| SEQ ID NO: 57 | Cas 12 Variant | MNSKTSIFDFSNIFGRDITLRFKLTPVTINSKGEVKDANGADPYRPYL SADEELQEQYELLKTAIDAYHQMYIDKKLKHILCLPLTEKGKDGVEH DTAKSKFVKSCLAYIKDYGEKDKKRQTADLRTFISRVFADDNISSLPP YKVKSDFITKTLRQWLEQPDTKVEKKEAILDLIEKNGSKLYANCQGL LEARQRLYEKDGKSTSVPYRCIDRNLPRFSKDYHLFEKILGDCSDVF DFEQLDKDFSEELKGIARLSGIRVESVREVFQPLLYLAYLNQEGIQYL NTIIGTKKEKGTSALGLNEYINQYNQKQGIKKKKDGIPMLNKLNNQI LFGDEVFIETLAEHKEAIPVIKKVVSSLGKLGAFDGECHENKLYQFLL SLSSYAGNIYVNTKVVAQISSSLWGDYSILYDAVKHDKNGRLIQKSV TLGELNEKIERLKLEDNRDAFEYFRRSQVKDVVHGSSNVGVFEQLK NCYNDFVEKKILKCSFFSEDQVLVIQRLFDSILSLQRIFKVFCPSLYEV DSDGLFVAKFSDYWNVLRGFDKDYDLLRNLFKRKPYSTDKIRVHFG LSNLMDGFVDSWTDKKDKGTQYNGYILRQAHSFVDENTSKELQEFQ RYNYYLVISGNVRLFREKGNALVCEKKKEKLVASDEFSGFERFDYY QSSINNFNREFKRLTGRDRKSFTDEILQNEGKKELKSTYIENLIKVAK SMKRLTALQNLVSDEKVRKYSENLDYETLSAEIGQILATGRERKYVP VSTNEMKNLLKSSKNNKGEEVRTFMFRISNKDLSYAETMQKGERKS HGAENMHTMYFRALLDTLQNTFDIGTGTVYFRKASDKRKMKYDEK NPTHRKGDELAFKNPYNKGKKKSVFGYDLIKDRRYTKDSYLFHLSIT QNYQKKGNAEDLNAMVRDYIRTQEDLRVIGIDRGERNLLYATMIDG EGHILAQKSFNVIGYQGTTASGESFQVETDYHQLLNEKAEKMRSLQR EWKEMDKIQDMKDGYLSVVVHELAKMVVENNAIIVMEDLNMGFM ESRQSQLANVYQKFEEKLRNKLQFYVDKRKRNDEPSGLYHALQLAG TETKDNQNGFIFYIPAWNTSKIDSVTGFVNLFNLKYTNIKDAKAFFST FEKIEKNVETGHYDFTFSYSSMARKKMAKRMDGTRDSWTISTHGSRI VREQKGNYWEYREIESLTSEFDALFEKYSIDTRCRLKEAIDKCGEAEF FKELIRLMKWTLQLRNYDDRGNDYIVSPVCYRGNEYYCSLDYDNEE GMCISKIPCQMPKDADANGAFNIARKGLMLCERLKKGEKIGVIKGTE WLQYVQNMSERYVGMV |
| SEQ ID NO: 58 | Cas 12 Variant | MINTMEQPKKSIWDEFTNLYSLQKTLRFELKPQGKTKELVRTLFINPE EHHHKLISDDLELSKNYKKVKKLIDCMHRNIINNVLSKHQFTGEELK KLDKNSNAEDNDTETDNADKKDPFAKIRERLTKALNEESKIMFDNK LLNPKKGKNKGECELKKWMDKAEDKYFELGNNEKIDKEAVKADM ERLEGFFTYFGGFNKNRENVYSSKKIATAIPFRIIHDNFPIFKKNIENY KKITEKHPELAKLLNEKGANEIFQLEHFNKCLTQDGIDVYNNEKLGII AKEQGKEQDKGINQLINEYAQKKNKEIKENAKGGEKPKKIKIAVFDK LKKQILSISKTKSFQFEVFEDTSDIINGINKRYTFLTEAKEGMSIVDEIK KIIGSVGDEKYSLDEIYLKEKFISTLSKKLFNYSRYIEVALEKWYDDR YDDKINKSGTDKRKFISAKQFSITSIQDAINYYLEKYEKDEELSKKYT GKNIIVDYFKNPTITIEHKQKEEVISEEKDLFKELEVRRNVIQHILNGD YKKDLKEEKQQDGDSEKVKAFLDALLEFNYILNPFIIKDKNLRKEQE KDEEFYNEIKKLQESIFEAEILDLYNQTRNYITKKPYKLDKFKLTFGS GYFLSGWSNDMEEREGSILIKYNEDRSKNYYLIIMAKPLTDDDKKQL FSDNGTHSKICIYEFQKMDMKNFPRMFINSKGSNPAPAIEKYNLPIKTI WADYQKYKNLNQKGKDKFLEENPDFRHNLIGYFKICAEKHESLAPF KHQFSSIWKPTKEYENLAQFYKDTLEACYNLKFENVNFDNISQLVSS GKLHLFKIHNKDFNPGSTGKKNLHTLYWEMLFDEKNLQDVIFKLSG GAELFYREASILKNKIIHKIGEKVLKKFFKLPDGKLEPVPAESIKNLSA YFRKELPEHELTEIDRKYIDNYSIIGKKDDKLGIMKDERFTVDKIQFH CPITINFKSKNKNFINDDVLEYLHKRDDVHIIGLDRGERHLIYLTMIN KDGKIVDNMQFSLNELQRRYKINGNEEIQKINYQKLLDTREVSRTEA RRNWQTIENIKNLKEGYLSLIVHQLAKLMIEKNAIVVMENLNYGFKD SRARVEKQIYQKFESILIKKLQYLVMDKNNLYDSGGVLSAYQLTNQE VPAYKYISKQNGFLFYVPPDYTSKIDPETGFINLLDTRYYSRKNAVAL LNKFDKIYYDRDNKYFRFDFDYNSTDSNGNKNFDKLRVDISELTRTK WSVCSHPAKRSITVQINNKWVRQPINDVTDKLIKLFEDKQIGYESGK CLKDEILKVEDAKFFEDLLRYLSVLLALRHTYTENGVEYDLIISSVEK APGSNEFFVSGKDNNLPANADANGAYNIARKGLWLLRKLDEIDNQE LAIKKFNELKHAKEIKKNGEESKEDKGDRKRKKKWVSQWCPNKEW LAFAQSMQDVSEK |
| SEQ ID NO: 59 | Cas 12 Variant | MNNGTNNFQNFIGISSLQKTLRNALIPTETTQQFIVKNGIIKEDELRGE NRQILKDIMDDYYRGFISETLSSIDDIDWTSLFEKMEIQLKNGDNKDT LIKEQTEYRKAIHKKFANDDRFKNMFSAKLISDILPEFVIHNNNYSAS EKEEKTQVIKLFSRFATSFKDYFKNRANCFSADDISSSSCHRIVNDNA EIFFSNALVYRRIVKSLSNDDINKISGDMKDSLKEMSLEEIYSYEKYG EFITQEGISFYNDICGKVNSFMNLYCQKNKENKNLYKLQKLHKQILCI ADTSYEVPYKFESDEEVYQSVNGFLDNISSKHIVERLRKIGDNYNGY NLDKIYIVSKFYESVSQKTYRDWETINTALEIHYNNILPGNGKSKADK VKKAVKNDLQKSITEINELVSNYKLCSDDNIKAETYIHEISHILNNFE AQELKYNPEIHLVESELKASELKNVLDVIMNAFHWCSVFMTEELVD KDNNFYAELEEIYDEIYPVISLYNLVRNYVTQKPYSTKKIKLNFGIPTL ADGWSKSKEYSNNAIILMRDNLYYLGIFNAKNKPDKKIIEGNTSENK GDYKKMIYNLLPGPNKMIPKVFLSSKTGVETYKPSAYILEGYKQNKH IKSSKDFDITFCHDLIDYFKNCIAIHPEWKNFGFDFSDTSTYEDISGFY REVELQGYKIDWTYISEKDIDLLQEKGQLYLFQIYNKDFSKKSTGND NLHTMYLKNLFSEENLKDIVLKLNGEAEIFFRKSSIKNPIIHKKGSILV NRTYEAEEKDQFGNIQIVRKNIPENIYQELYKYFNDKSDKELSDEAA KLKNVVGHHEAATNIVKDYRYTYDKYFLHMPITINFKANKTGFIND RILQYIAKEKDLHVIGIDRGERNLIYVSVIDTCGNIVEQKSFNIVNGYD YQIKLKQQEGARQIARKEWKEIGKIKEIKEGYLSLVIHEISKMVIKYN AIIAMEDLSYGFKKGRFKVERQVYQKFETMLINKLNYLVFKDISITEN GGLLKGYQLTYIPDKLKNVGHQCGCIFYVPAAYTSKIDPTTGFVNIF KFKDLTVDAKREFIKKFDSIRYDSEKNLFCFTFDYNNFITQNTVMSKS SWSVYTYGVRIKRRFVNGRFSNESDTIDITKDMEKTLEMTDINWRDG HDLRQDIIDYEIVQHIFEIFRLTVQMRNSLSELEDRDYDRLISPVLNEN NIFYDSAKAGDALPKDADANGAYCIALKGLYEIKQITENWKEDGKF SRDKLKISNKDWFDFIQNKRYL |
| SEQ ID NO: 60 | Cas 12 Variant | MSNLNTFISPEFTGKIKMTKSLKVSMIPIGETEHWIAKHKVFEKDREL FDKNLKARPILDEFIKYTVSRALPNLLFDFEAYYLVKKDRTKARAFE KELAKTVTDLILKEMDELKSASLIDSADFVKTTLKKFAGTHDIPGLSR IEAIESLEAASKLTALNGKFNTSRIAIINTLIPKRIIENFDIYLSNMEKIR NVYESGEFGFLFERYPDTLLFMEPANYRTVCSPEAIEDYNRFISGYGD STESWIKGFNQELSEASNSSKSSNGGVRRYSLIKPLHKQHLFETKKFF TFASISSDDDVRELINSVKGSTEDACLNALAFFSSSDPKTLFVKGSYL HTLSAFLYGSANSYILPERIKEGEKARLTAEYDSVAKKTKAVTTRYN VAMNNISKKINEKIFSLADIDAYCCDISKRRSVREILLGIMQEMYAAV YGENGKWSNIEAEAVLDSKTKIWKAKNGAVAKAVNDYLTAILEIRK FIRPFALRMEELEELGLDTSSALDAGEITNTLFEAVRAQKLVHAYLTR NDADIALSTQVYFGGTQKAAASWWNYETGDIQNRQIALAKKDGMY YFIGTFDERGSYSIEPASPGEDYYEMLDVKKGQDANKQIKKVLFSNK AIREHFADSSNDYVITTKVNSPITVRREIFDKYQAGEFKLTSQKIRKG DLVGEKEMTYYREYMDLLFQMAKGYTEYSRFNMDTLLPIEEYDTE NDLLDDVNTNTIDYRWVRISAACIDDGVRNGDIFVFRAQTSSMYGK RENKKGYTGLFLELVSDENLLVTRGMSLNSAMSIYYRAKVHDAITV HKKGDVLVNKFTNARERIPENSYKAICAFYNSGKSIEELTIEDRDWL AKATTRICSGEIIKDRRYTKNQYSISISYNINRSVNNRKRVDLATIVDD TASAGRIISVTRGTKDLVYYTVIDDGGSVIEARSLNVINGINYAKMLA QISEERHDSNANFDIPKRVETIKEAYCAFAVHEIISAALKHNALIVVEL ISDAIKDKYSLLDNQVFLKFENVLKNCLMSVKVKGARGMEPGSISNP LQLCNADDKSFRNGILYQIPSSYINICPVTGYADIIDYYNIVSAGDIRN FFVRFENIVYNKEKARFEFSFDLKNIPIKLEKCPDRTKWTVLGRGEIT TYDPLTKSNHYVFDAAQMLAETVSKEGLDPCANIVEHIDELSAATLK KMFNTFRNIAKGIVSECDEVPVSYYKSPVIDEADIKNKSLDNKSISEIK CYNLDEKARYYLALAKSSSDGENKNRYVSSTAIEWLNYIQEKRTHE |

Alternatively, the Type V CRISPR/Cas enzyme is a programmable Cas14 nuclease. A Cas14 protein of the present disclosure includes 3 partial RuvC domains (RuvC-I, RuvC-II, and RuvC-III, also referred to herein as subdomains) that are not contiguous with respect to the primary amino acid sequence of the Cas14 protein, but form a RuvC domain once the protein is produced and folds. A naturally occurring Cas14 protein functions as an endonuclease that catalyzes cleavage at a specific sequence in a target nucleic acid.

In some instances, the TypeV CRISPR/Cas protein comprises a Cas14 protein. Cas14 proteins may comprise a bilobed structure with distinct amino-terminal and carboxy-terminal domains. The amino- and carboxy-terminal domains may be connected by a flexible linker. The flexible linker may affect the relative conformations of the amino- and carboxyl-terminal domains. The flexible linker may be short, for example less than 10 amino acids, less than 8 amino acids, less than 6 amino acids, less than 5 amino acids, or less than 4 amino acids in length. The flexible linker may be sufficiently long to enable different conformations of the amino- and carboxy-terminal domains among two Cas14 proteins of a Cas14 dimer complex (e.g., the relative orientations of the amino- and carboxy-terminal domains differ between two Cas14 proteins of a Cas14 homodimer complex). The linker domain may comprise a mutation which affects the relative conformations of the amino- and carboxyl-terminal domains. The linker may comprise a mutation which affects Cas14 dimerization. For example, a linker mutation may enhance the stability of a Cas14 dimer.

In some instances, the amino-terminal domain of a Cas14 protein comprises a wedge domain, a recognition domain, a zinc finger domain, or any combination thereof. The wedge domain may comprise a multi-strand β-barrel structure. A multi-strand β-barrel structure may comprise an oligonucleotide/oligosaccharide-binding fold that is structurally comparable to those of some Cas12 proteins. The recognition domain and the zinc finger domain may each (individually or collectively) be inserted between β-barrel strands of the wedge domain. The recognition domain may comprise a 4-α-helix structure, structurally comparable but shorter than those found in some Cas12 proteins. The recognition domain may comprise a binding affinity for a guide nucleic acid or for a guide nucleic acid-target nucleic acid heteroduplex. In some cases, a REC lobe may comprise a binding affinity for a PAM sequence in the target nucleic acid. The amino-terminal may comprise a wedge domain, a recognition domain, and a zinc finger domain. The carboxy-terminal may comprise a RuvC domain, a zinc finger domain, or any combination thereof. The carboxy-terminal may comprise one RuvC and one zinc finger domain.

Cas14 proteins may comprise a RuvC domain or a partial RuvC domain. The RuvC domain may be defined by a single, contiguous sequence, or a set of partial RuvC domains that are not contiguous with respect to the primary amino acid sequence of the Cas14 protein. In some instances, a partial RuvC domain does not have any substrate binding activity or catalytic activity on its own. A Cas14 protein of the present disclosure may include multiple partial RuvC domains, which may combine to generate a RuvC domain with substrate binding or catalytic activity. For example, a Cas14 may include 3 partial RuvC domains (RuvC-I, RuvC-II, and RuvC-III, also referred to herein as subdomains) that are not contiguous with respect to the primary amino acid sequence of the Cas14 protein, but form a RuvC domain once the protein is produced and folds. A Cas14 protein may comprise a linker loop connecting a carboxy terminal domain of the Cas14 protein with the amino terminal domain of the Cas 14 protein, and wherein the carboxy terminal domain comprises one or more RuvC domains and the amino terminal domain comprises a recognition domain.

Cas14 proteins may comprise a zinc finger domain. In some instances, a carboxy terminal domain of a Cas14 protein comprises a zinc finger domain. In some instances, an amino terminal domain of a Cas14 protein comprises a zinc finger domain. In some instances, the amino terminal domain comprises a wedge domain (e.g., a multi-β-barrel wedge structure), a zinc finger domain, or any combination thereof. In some cases, the carboxy terminal domain comprises the RuvC domains and a zinc finger domain, and the amino terminal domain comprises a recognition domain, a wedge domain, and a zinc finger domain.

Cas14 proteins may be relatively small compared to many other Cas proteins, making them suitable for nucleic acid detection or gene editing. For instance, a Cas14 protein may be less likely to adsorb to a surface or another biological species due to its small size. The smaller nature of these proteins also allows for them to be more easily packaged as a reagent in a system or assay, and delivered with higher efficiency as compared to other larger Cas proteins. In some cases, a Cas14 protein is 400 to 800 amino acid residues long, 400 to 600 amino acid residues long, 440 to 580 amino acid residues long, 460 to 560 amino acid residues long, 460 to 540 amino acid residues long, 460 to 500 amino acid residues long, 400 to 500 amino acid residues long, or 500 to 600 amino acid residues long. In some cases, a Cas14 protein is less than about 550 amino acid residues long. In some cases, a Cas14 protein is less than about 500 amino acid residues long.

In some instances, a Cas14 protein may function as an endonuclease that catalyzes cleavage at a specific position within a target nucleic acid. In some instances, a Cas14 protein is capable of catalyzing non-sequence-specific cleavage of a single stranded nucleic acid. In some cases, a Cas14 protein is activated to perform trans cleavage activity after binding of a guide nucleic acid with a target nucleic acid. This trans cleavage activity is also referred to as "collateral" or "transcollateral" cleavage. Trans cleavage activity may be non-specific cleavage of nearby single-stranded nucleic acid by the activated programmable nuclease, such as trans cleavage of reporters with a detection moiety.

A programmable Cas14 nuclease can be a Cas14a protein, a Cas14b protein, a Cas14c protein, a Cas14d protein, a Cas14e protein, a Cas 14f protein, a Cas14g protein, a Cas14h protein, or a Cas14u protein. In some cases, a suitable Cas14 protein comprises an amino acid sequence having at least 80%, at least 85%, at least 90%, at least 95%, at least 98%, at least 99%, or 100%, amino acid sequence identity to any one of SEQ ID NO: 61 - SEQ ID NO: 152.

**TABLE 2 - Cas14 Protein Sequences**

| **SEQ ID NO** | **Sequence** |
|---|---|
| SEQ ID NO: 61 | MEVQKTVMKTLSLRILRPLYSQEIEKEIKEEKERRKQAGGTGELDGGFYKKLEKKHSE MFSFDRLNLLLNQLQREIAKVYNHAISELYIATIAQGNKSNKHYISSIVYNRAYGYFYN AYIALGICSKVEANFRSNELLTQQSALPTAKSDNFPIVLHKQKGAEGEDGGFRISTEGS DLIFEIPIPFYEYNGENRKEPYKWVKKGGQKPVLKLILSTFRRQRNKGWAKDEGTDAEI RKVTEGKYQVSQIEINRGKKLGEHQKWFANFSIEQPIYERKPNRSIVGGLDVGIRSPLV CAINNSFSRYSVDSNDVFKFSKQVFAFRRRLLSKNSLKRKGHGAAHKLEPITEMTEKN DKFRKKIIERWAKEVTNFFVKNQVGIVQIEDLSTMKDREDHFFNQYLRGFWPYYQMQ TLIENKLKEYGIEVKRVQAKYTSQLCSNPNCRYWNNYFNFEYRKVNKFPKFKCEKCN LEISADYNAARNLSTPDIEKFVAKATKGINLPEK |
| SEQ ID NO: 62 | MEEAKTVSKTLSLRILRPLYSAEIEKEIKEEKERRKQGGKSGELDSGFYKKLEKKHTQM FGWDKLNLMLSQLQRQIARVFNQSISELYIETVIQGKKSNKHYTSKIVYNRAYSVFYN AYLALGITSKVEANFRSTELLMQKSSLPTAKSDNFPILLHKQKGVEGEEGGFKISADGN DLIFEIPIPFYEYDSANKKEPFKWIKKGGQKPTIKLILSTFRRQRNKGWAKDEGTDAEIR KVIEGKYQVSHIEINRGKKLGDHQKWFVNFTIEQPIYERKLDKNIIGGIDVGIKSPLVCA VNNSFARYSVDSNDVLKFSKQAFAFRRRLLSKNSLKRSGHGSKNKLDPITRMTEKNDR FRKKIIERWAKEVTNFFIKNQVGTVQIEDLSTMKDRQDNFFNQYLRGFWPYYQMQNLI ENKLKEYGIETKRIKARYTSQLCSNPSCRHWNSYFSFDHRKTNNFPKFKCEKCALEISA DYNAARNISTPDIEKFVAKATKGINLPDKNENVILE |
| SEQ ID NO: 63 | MAKNTITKTLKLRIVRPYNSAEVEKIVADEKNNREKIALEKNKDKVKEACSKHLKVA AYCTTQVERNACLFCKARKLDDKFYQKLRGQFPDAVFWQEISEIFRQLQKQAAEIYNQ SLIELYYEIFIKGKGIANASSVEHYLSDVCYTRAAELFKNAAIASGLRSKIKSNFRLKEL KNMKSGLPTTKSDNFPIPLVKQKGGQYTGFEISNHNSDFIIKIPFGRWQVKKEIDKYRP WEKFDFEQVQKSPKPISLLLSTQRRKRNKGWSKDEGTEAEIKKVMNGDYQTSYIEVKR GSKIGEKSAWMLNLSIDVPKIDKGVDPSIIGGIDVGVKSPLVCAINNAFSRYSISDNDLF HFNKKMFARRRILLKKNRHKRAGHGAKNKLKPITILTEKSERFRKKLIERWACEIADFF IKNKVGTVQMENLESMKRKEDSYFNIRLRGFWPYAEMQNKIEFKLKQYGIEIRKVAPN NTSKTCSKCGHLNNYFNFEYRKKNKFPHFKCEKCNFKENADYNAALNISNPKLKSTKE EP |
| SEQ ID NO: 64 | MERQKVPQIRKIVRVVPLRILRPKYSDVIENALKKFKEKGDDTNTNDFWRAIRDRDTE FFRKELNFSEDEINQLERDTLFRVGLDNRVLFSYFDFLQEKLMKDYNKIISKLFINRQSK SSFENDLTDEEVEELIEKDVTPFYGAYIGKGIKSVIKSNLGGKFIKSVKIDRETKKVTKL TAINIGLMGLPVAKSDTFPIKIIKTNPDYITFQKSTKENLQKIEDYETGIEYGDLLVQITIP WFKNENKDFSLIKTKEAIEYYKLNGVGKKDLLNINLVLTTYHIRKKKSWQIDGSSQSL VREMANGELEEKWKSFFDTFIKKYGDEGKSALVKRRVNKKSRAKGEKGRELNLDERI KRLYDSIKAKSFPSEINLIPENYKWKLHFSIEIPPMVNDIDSNLYGGIDFGEQNIATLCVK NIEKDDYDFLTIYGNDLLKHAQASYARRRIMRVQDEYKARGHGKSRKTKAQEDYSER MQKLRQKITERLVKQISDFFLWRNKFHMAVCSLRYEDLNTLYKGESVKAKRMRQFIN KQQLFNGIERKLKDYNSEIYVNSRYPHYTSRLCSKCGKLNLYFDFLKFRTKNIIIRKNPD GSEIKYMPFFICEFCGWKQAGDKNASANIADKDYQDKLNKEKEFCNIRKPSKKEDIG EENEEERDYSRRFNRNSFIYNSLKKDNKLNQEKLFDEWKNQLKRKIDGRNKFEPKEYK DRFSYLFAYYQEIIKNESES |
| SEQ ID NO: 65 | MVPTELITKTLQLRVIRPLYFEEIEKELAELKEQKEKEFEETNSLLLESKKIDAKSLKKL KRKARSSAAVEFWKIAKEKYPDILTKPEMEFIFSEMQKMMARFYNKSMTNIFIEMNND EKVNPLSLISKASTEANQVIKCSSISSGLNRKIAGSINKTKFKQVRDGLISLPTARTETFPI SFYKSTANKDEIPISKINLPSEEEADLTITLPFPFFEIKKEKKGQKAYSYFNIIEKSGRSNN KIDLLLSTHRRQRRKGWKEEGGTSAEIRRLMEGEFDKEWEIYLGEAEKSEKAKNDLIK NMTRGKLSKDIKEQLEDIQVKYFSDNNVESWNDLSKEQKQELSKLRKKKVEELKDW KHVKEILKTRAKIGWVELKRGKRQRDRNKWFVNITITRPPFINKELDDTKFGGIDLGV KVPFVCAVHGSPARLIIKENEILQFNKMVSARNRQITKDSEQRKGRGKKNKFIKKEIFN ERNELFRKKIIERWANQIVKFFEDQKCATVQIENLESFDRTSYK |
| SEQ ID NO: 66 | MKSDTKDKKIIIHQTKTLSLRIVKPQSIPMEEFTDLVRYHQMIIFPVYNNGAIDLYKKLF KAKIQKGNEARAIKYFMNKIVYAPIANTVKNSYIALGYSTKMQSSFSGKRLWDLRFGE ATPPTIKADFPLPFYNQSGFKVSSENGEFIIGIPFGQYTKKTVSDIEKKTSFAWDKFTLED TTKKTLIELLLSTKTRKMNEGWKNNEGTEAEIKRVMDGTYQVTSLEILQRDDSWFVNF NIAYDSLKKQPDRDKIAGIHMGITRPLTAVIYNNKYRALSIYPNTVMHLTQKQLARIKE QRTNSKYATGGHGRNAKVTGTDTLSEAYRQRRKKIIEDWIASIVKFAINNEIGTIYLEDI SNTNSFFAAREQKLIYLEDISNTNSFLSTYKYPISAISDTLQHKLEEKAIQVIRKKAYYV NQICSLCGHYNKGFTYQFRRKNKFPKMKCQGCLEATSTEFNAAANVANPDYEKLLIK HGLLQLKK |
| SEQ ID NO: 67 | MSTITRQVRLSPTPEQSRLLMAHCQQYISTVNVLVAAFDSEVLTGKVSTKDFRAALPS AVKNQALRDAQSVFKRSVELGCLPVLKKPHCQWNNQNWRVEGDQLILPICKDGKTQ QERFRCAAVALEGKAGILRIKKKRGKWIADLTVTQEDAPESSGSAIMGVDLGIKVPAV AHIGGKGTRFFGNGRSQRSMRRRFYARRKTLQKAKKLRAVRKSKGKEARWMKTINH QLSRQIVNHAHALGVGTIKIEALQGIRKGTTRKSRGAAARKNNRMTNTWSFSQLTLFIT YKAQRQGITVEQVDPAYTSQDCPACRARNGAQDRTYVCSECGWRGHRDTVGAINISR RAGLSGHRRGATGA |
| SEQ ID NO: 68 | MIAQKTIKIKLNPTKEQIIKLNSIIEEYIKVSNFTAKKIAEIQESFTDSGLTQGTCSECGKE KTYRKYHLLKKDNKLFCITCYKRKYSQFTLQKVEFQNKTGLRNVAKLPKTYYTNAIR FASDTFSGFDEIIKKKQNRLNSIQNRLNFWKELLYNPSNRNEIKIKVVKYAPKTDTREH PHYYSEAEIKGRIKRLEKQLKKFKMPKYPEFTSETISLQRELYSWKNPDELKISSITDKN ESMNYYGKEYLKRYIDLINSQTPQILLEKENNSFYLCFPITKNIEMPKIDDTFEPVGIDW GITRNIAVVSILDSKTKKPKFVKFYSAGYILGKRKHYKSLRKHFGQKKRQDKINKLGT KEDRFIDSNIHKLAFLIVKEIRNHSNKPIILMENITDNREEAEKSMRQNILLHSVKSRLQN YIAYKALWNNIPTNLVKPEHTSQICNRCGHQDRENRPKGSKLFKCVKCNYMSNADFN ASINIARKFYIGEYEPFYKDNEKMKSGVNSISM |
| SEQ ID NO: 69 | LKLSEQENITTGVKFKLKLDKETSEGLNDYFDEYGKAINFAIKVIQKELAEDRFAGKVR LDENKKPLLNEDGKKIWDFPNEFCSCGKQVNRYVNGKSLCQECYKNKFTEYGIRKRM YSAKGRKAEQDINIKNSTNKISKTHFNYAIREAFILDKSIKKQRKERFRRLREMKKKLQ EFIEIRDGNKILCPKIEKQRVERYIHPSWINKEKKLEDFRGYSMSNVLGKIKILDRNIKRE EKSLKEKGQINFKARRLMLDKSVKFLNDNKISFTISKNLPKEYELDLPEKEKRLNWLKE KIKIIKNQKPKYAYLLRKDDNFYLQYTLETEFNLKEDYSGIVGIDRGVSHIAVYTFVHN NGKNERPLFLNSSEILRLKNLQKERDRFLRRKHNKKRKKSNMRNIEKKIQLILHNYSKQ IVDFAKNKNAFIVFEKLEKPKKNRSKMSKKSQYKLSQFTFKKLSDLVDYKAKREGIKV LYISPEYTSKECSHCGEKVNTQRPFNGNSSLFKCNKCGVELNADYNASINIAKKGLNIL NSTN |
| SEQ ID NO: 70 | MEESIITGVKFKLRIDKETTKKLNEYFDEYGKAINFAVKIIQKELADDRFAGKAKLDQN KNPILDENGKKIYEFPDEFCSCGKQVNKYVNNKPFCQECYKIRFTENGIRKRMYSAKG RKAEHKINILNSTNKISKTHFNYAIREAFILDKSIKKQRKKRNERLRESKKRLQQFIDMR DGKREICPTIKGQKVDRFIHPSWITKDKKLEDFRGYTLSIINSKIKILDRNIKREEKSLKE KGQIIFKAKRLMLDKSIRFVGDRKVLFTISKTLPKEYELDLPSKEKRLNWLKEKIEIIKN QKPKYAYLLRKNIESEKKPNYEYYLQYTLEIKPELKDFYDGAIGIDRGINHIAVCTFISN DGKVTPPKFFSSGEILRLKNLQKERDRFLLRKHNKNRKKGNMRVIENKINLILHRYSKQ IVDMAKKLNASIVFEELGRIGKSRTKMKKSQRYKLSLFIFKKLSDLVDYKSRREGIRVT YVPPEYTSKECSHCGEKVNTQRPFNGNYSLFKCNKCGIQLNSDYNASINIAKKGLKIPN ST |
| SEQ ID NO: 71 | LWTIVIGDFIEMPKQDLVTTGIKFKLDVDKETRKKLDDYFDEYGKAINFAVKIIQKNLK EDRFAGKIALGEDKKPLLDKDGKKIYNYPNESCSCGNQVRRYVNAKPFCVDCYKLKF TENGIRKRMYSARGRKADSDINIKNSTNKISKTHFNYAIREGFILDKSLKKQRSKRIKKL LELKRKLQEFIDIRQGQMVLCPKIKNQRVDKFIHPSWLKRDKKLEEFRGYSLSVVEGKI KIFNRNILREEDSLRQRGHVNFKANRIMLDKSVRFLDGGKVNFNLNKGLPKEYLLDLP KKENKLSWLNEKISLIKLQKPKYAYLLRREGSFFIQYTIENVPKTFSDYLGAIGIDRGISH IAVCTFVSKNGVNKAPVFFSSGEILKLKSLQKQRDLFLRGKHNKIRKKSNMRNIDNKIN LILHKYSRNIVNLAKSEKAFIVFEKLEKIKKSRFKMSKSLQYKLSQFTFKKLSDLVEYK AKIEGIKVDYVPPEYTSKECSHCGEKVDTQRPFNGNSSLFKCNKCRVQLNADYNASINI AKKSLNISN |
| SEQ ID NO: 72 | MSKTTISVKLKIIDLSSEKKEFLDNYFNEYAKATTFCQLRIRRLLRNTHWLGKKEKSSK KWIFESGICDLCGENKELVNEDRNSGEPAKICKRCYNGRYGNQMIRKLFVSTKKREVQ ENMDIRRVAKLNNTHYHRIPEEAFDMIKAADTAEKRRKKNVEYDKKRQMEFIEMFND EKKRAARPKKPNERETRYVHISKLESPSKGYTLNGIKRKIDGMGKKIERAEKGLSRKKI FGYQGNRIKLDSNWVRFDLAESEITIPSLFKEMKLRITGPTNVHSKSGQIYFAEWFERIN KQPNNYCYLIRKTSSNGKYEYYLQYTYEAEVEANKEYAGCLGVDIGCSKLAAAVYYD SKNKKAQKPIEIFTNPIKKIKMRREKLIKLLSRVKVRHRRRKLMQLSKTEPIIDYTCHKT ARKIVEMANTAKAFISMENLETGIKQKQQARETKKQKFYRNMFLFRKLSKLIEYKALL KGIKIVYVKPDYTSQTCSSCGADKEKTERPSQAIFRCLNPTCRYYQRDINADFNAAVNI AKKALNNTEVVTTLL |
| SEQ ID NO: 73 | MARAKNQPYQKLTTTTGIKFKLDLSEEEGKRFDEYFSEYAKAVNFCAKVIYQLRKNL KFAGKKELAAKEWKFEISNCDFCNKQKEIYYKNIANGQKVCKGCHRTNFSDNAIRKK MIPVKGRKVESKFNIHNTTKKISGTHRHWAFEDAADIIESMDKQRKEKQKRLRREKRK LSYFFELFGDPAKRYELPKVGKQRVPRYLHKIIDKDSLTKKRGYSLSYIKNKIKISERNI ERDEKSLRKASPIAFGARKIKMSKLDPKRAFDLENNVFKIPGKVIKGQYKFFGTNVANE HGKKFYKDRISKILAGKPKYFYLLRKKVAESDGNPIFEYYVQWSIDTETPAITSYDNIL GIDAGITNLATTVLIPKNLSAEHCSHCGNNHVKPIFTKFFSGKELKAIKIKSRKQKYFLR GKHNKLVKIKRIRPIEQKVDGYCHVVSKQIVEMAKERNSCIALEKLEKPKKSKFRQRR REKYAVSMFVFKKLATFIKYKAAREGIEIIPVEPEGTSYTCSHCKNAQNNQRPYFKPNS KKSWTSMFKCGKCGIELNSDYNAAFNIAQKALNMTSA |
| SEQ ID NO: 74 | MDEKHFFCSYCNKELKISKNLINKISKGSIREDEAVSKAISIHNKKEHSLILGIKFKLFIEN KLDKKKLNEYFDNYSKAVTFAARIFDKIRSPYKFIGLKDKNTKKWTFPKAKCVFCLEE KEVAYANEKDNSKICTECYLKEFGENGIRKKIYSTRGRKVEPKYNIFNSTKELSSTHYN YAIRDAFQLLDALKKQRQKKLKSIFNQKLRLKEFEDIFSDPQKRIELSLKPHQREKRYIH LSKSGQESINRGYTLRFVRGKIKSLTRNIEREEKSLRKKTPIHFKGNRLMIFPAGIKFDFA SNKVKISISKNLPNEFNFSGTNVKNEHGKSFFKSRIELIKTQKPKYAYVLRKIKREYSKL RNYEIEKIRLENPNADLCDFYLQYTIETESRNNEEINGIIGIDRGITNLACLVLLKKGDKK PSGVKFYKGNKILGMKIAYRKHLYLLKGKRNKLRKQRQIRAIEPKINLILHQISKDIVKI AKEKNFAIALEQLEKPKKARFAQRKKEKYKLALFTFKNLSTLIEYKSKREGIPVIYVPPE KTSQMCSHCAINGDEHVDTQRPYKKPNAQKPSYSLFKCNKCGIELNADYNAAFNIAQ KGLKTLMLNHSH |
| SEQ ID NO: 75 | MLQTLLVKLDPSKEQYKMLYETMERFNEACNQIAETVFAIHSANKIEVQKTVYYPIRE KFGLSAQLTILAIRKVCEAYKRDKSIKPEFRLDGALVYDQRVLSWKGLDKVSLVTLQG RQIIPIKFGDYQKARMDRIRGQADLILVKGVFYLCVVVEVSEESPYDPKGVLGVDLGIK NLAVDSDGEVHSGEQTTNTRERLDSLKARLQSKGTKSAKRHLKKLSGRMAKFSKDVN HCISKKLVAKAKGTLMSIALEDLQGIRDRVTVRKAQRRNLHTWNFGLLRMFVDYKAK IAGVPLVFVDPRNTSRTCPSCGHVAKANRPTRDEFRCVSCGFAGAADHIAAMNIAFRA EVSQPIVTRFFVQSQAPSFRVG |
| SEQ ID NO: 76 | MDEEPDSAEPNLAPISVKLKLVKLDGEKLAALNDYFNEYAKAVNFCELKMQKIRKNL VNIRGTYLKEKKAWINQTGECCICKKIDELRCEDKNPDINGKICKKCYNGRYGNQMIR KLFVSTNKRAVPKSLDIRKVARLHNTHYHRIPPEAADIIKAIETAERKRRNRILFDERRY NELKDALENEEKRVARPKKPKEREVRYVPISKKDTPSKGYTMNALVRKVSGMAKKIE RAKRNLNKRKKIEYLGRRILLDKNWVRFDFDKSEISIPTMKEFFGEMRFEITGPSNVMS PNGREYFTKWFDRIKAQPDNYCYLLRKESEDETDFYLQYTWRPDAHPKKDYTGCLGI DIGGSKLASAVYFDADKNRAKQPIQIFSNPIGKWKTKRQKVIKVLSKAAVRHKTKKLE SLRNIEPRIDVHCHRIARKIVGMALAANAFISMENLEGGIREKQKAKETKKQKFSRNMF VFRKLSKLIEYKALMEGVKVVYIVPDYTSQLCSSCGTNNTKRPKQAIFMCQNTECRYF GKNINADFNAAINIAKKALNRKDIVRELS |
| SEQ ID NO: 77 | MEKNNSEQTSITTGIKFKLKLDKETKEKLNNYFDEYGKAINFAVRIIQMQLNDDRLAG KYKRDEKGKPILGEDGKKILEIPNDFCSCGNQVNHYVNGVSFCQECYKKRFSENGIRK RMYSAKGRKAEQDINIKNSTNKISKTHFNYAIREAFNLDKSIKKQREKRFKKLKDMKR KLQEFLEIRDGKRVICPKIEKQKVERYIHPSWINKEKKLEEFRGYSLSIVNSKIKSFDRNI QREEKSLKEKGQINFKAQRLMLDKSVKFLKDNKVSFTISKELPKTFELDLPKKEKKLN WLNEKLEIIKNQKPKYAYLLRKENNIFLQYTLDSIPEIHSEYSGAVGIDRGVSHIAVYTF LDKDGKNERPFFLSSSGILRLKNLQKERDKFLRKKHNKIRKKGNMRNIEQKINLILHEY SKQIVNFAKDKNAFIVFELLEKPKKSRERMSKKIQYKLSQFTFKKLSDLVDYKAKREGI KVIYVEPAYTSKDCSHCGERVNTQRPFNGNFSLFKCNKCGIVLNSDYNASLNIARKGL NISAN |
| SEQ ID NO: 78 | MAEEKFFFCEKCNKDIKIPKNYINKQGAEEKARAKHEHRVHALILGIKFKIYPKKEDIS KLNDYFDEYAKAVTFTAKIVDKLKAPFLFAGKRDKDTSKKKWVFPVDKCSFCKEKTE INYRTKQGKNICNSCYLTEFGEQGLLEKIYATKGRKVSSSFNLFNSTKKLTGTHNNYV VKESLQLLDALKKQRSKRLKKLSNTRRKLKQFEEMFEKEDKRFQLPLKEKQRELRFIH VSQKDRATEFKGYTMNKIKSKIKVLRRNIEREQRSLNRKSPVFFRGTRIRLSPSVQFDD KDNKIKLTLSKELPKEYSFSGLNVANEHGRKFFAEKLKLIKENKSKYAYLLRRQVNKN NKKPIYDYYLQYTVEFLPNIITNYNGILGIDRGINTLACIVLLENKKEKPSFVKFFSGKGI LNLKNKRRKQLYFLKGVHNKYRKQQKIRPIEPRIDQILHDISKQIIDLAKEKRVAISLEQ LEKPQKPKFRQSRKAKYKLSQFNFKTLSNYIDYKAKKEGIRVIYIAPEMTSQNCSRCA MKNDLHVNTQRPYKNTSSLFKCNKCGVELNADYNAAFNIAQKGLKILNS |
| SEQ ID NO: 79 | MISLKLKLLPDEEQKKLLDEMFWKWASICTRVGFGRADKEDLKPPKDAEGVWFSLTQ LNQANTDINDLREAMKHQKHRLEYEKNRLEAQRDDTQDALKNPDRREISTKRKDLFR PKASVEKGFLKLKYHQERYWVRRLKEINKLIERKTKTLIKIEKGRIKFKATRITLHQGSF KIRFGDKPAFLIKALSGKNQIDAPFVVVPEQPICGSVVNSKKYLDEITTNFLAYSVNAM LFGLSRSEEMLLKAKRPEKIKKKEEKLAKKQSAFENKKKELQKLLGRELTQQEEAIIEE TRNQFFQDFEVKITKQYSELLSKIANELKQKNDFLKVNKYPILLRKPLKKAKSKKINNL SPSEWKYYLQFGVKPLLKQKSRRKSRNVLGIDRGLKHLLAVTVLEPDKKTFVWNKLY PNPITGWKWRRRKLLRSLKRLKRRIKSQKHETIHENQTRKKLKSLQGRIDDLLHNISRK IVETAKEYDAVIVVEDLQSMRQHGRSKGNRLKTLNYALSLFDYANVMQLIKYKAGIE GIQIYDVKPAGTSQNCAYCLLAQRDSHEYKRSQENSKIGVCLNPNCQNHKKQIDADLN AARVIASCYALKINDSQPFGTRKRFKKRTTN |
| SEQ ID NO: 80 | METLSLKLKLNPSKEQLLVLDKMFWKWASICTRLGLKKAEMSDLEPPKDAEGVWFSK TQLNQANTDVNDLRKAMQHQGKRIEYELDKVENRRNEIQEMLEKPDRRDISPNRKDL FRPKAAVEKGYLKLKYHKLGYWSKELKTANKLIERKRKTLAKIDAGKMKFKPTRISL HTNSFRIKFGEEPKIALSTTSKHEKIELPLITSLQRPLKTSCAKKSKTYLDAAILNFLAYS TNAALFGLSRSEEMLLKAKKPEKIEKRDRKLATKRESFDKKLKTLEKLLERKLSEKEK SVFKRKQTEFFDKFCITLDETYVEALHRIAEELVSKNKYLEIKKYPVLLRKPESRLRSKK LKNLKPEDWTYYIQFGFQPLLDTPKPIKTKTVLGIDRGVRHLLAVSIFDPRTKTFTFNRL YSNPIVDWKWRRRKLLRSIKRLKRRLKSEKHVHLHENQFKAKLRSLEGRIEDHFHNLS KEIVDLAKENNSVIVVENLGGMRQHGRGRGKWLKALNYALSHFDYAKVMQLIKYKA ELAGVFVYDVAPAGTSINCAYCLLNDKDASNYTRGKVINGKKNTKIGECKTCKKEFD ADLNAARVIALCYEKRLNDPQPFGTRKQFKPKKP |
| SEQ ID NO: 81 | MKALKLQLIPTRKQYKILDEMFWKWASLANRVSQKGESKETLAPKKDIQKIQFNATQ LNQIEKDIKDLRGAMKEQQKQKERLLLQIQERRSTISEMLNDDNNKERDPHRPLNFRP KGWRKFHTSKHWVGELSKILRQEDRVKKTIERIVAGKISFKPKRIGIWSSNYKINFFKR KISINPLNSKGFELTLMTEPTQDLIGKNGGKSVLNNKRYLDDSIKSLLMFALHSRFFGL NNTDTYLLGGKINPSLVKYYKKNQDMGEFGREIVEKFERKLKQEINEQQKKIIMSQIKE QYSNRDSAFNKDYLGLINEFSEVFNQRKSERAEYLLDSFEDKIKQIKQEIGESLNISDWD FLIDEAKKAYGYEEGFTEYVYSKRYLEILNKIVKAVLITDIYFDLRKYPILLRKPLDKIK KISNLKPDEWSYYIQFGYDSINPVQLMSTDKFLGIDRGLTHLLAYSVFDKEKKEFIINQL EPNPIMGWKWKLRKVKRSLQHLERRIRAQKMVKLPENQMKKKLKSIEPKIEVHYHNI SRKIVNLAKDYNASIVVESLEGGGLKQHGRKKNARNRSLNYALSLFDYGKIASLIKYK ADLEGVPMYEVLPAYTSQQCAKCVLEKGSFVDPEIIGYVEDIGIKGSLLDSLFEGTELSS IQVLKKIKNKIELSARDNHNKEINLILKYNFKGLVIVRGQDKEEIAEHPIKEINGKFAILD FVYKRGKEKVGKKGNQKVRYTGNKKVGYCSKHGQVDADLNASRVIALCKYLDINDP ILFGEQRKSFK |
| SEQ ID NO: 82 | MVTRAIKLKLDPTKNQYKLLNEMFWKWASLANRFSQKGASKETLAPKDGTQKIQFN ATQLNQIKKDVDDLRGAMEKQGKQKERLLIQIQERLLTISEILRDDSKKEKDPHRPQNF RPFGWRRFHTSAYWSSEASKLTRQVDRVRRTIERIKAGKINFKPKRIGLWSSTYKINFL KKKINISPLKSKSFELDLITEPQQKIIGKEGGKSVANSKKYLDDSIKSLLIFAIKSRLFGLN NKDKPLFENIITPNLVRYHKKGQEQENFKKEVIKKFENKLKKEISQKQKEIIFSQIERQY ENRDATFSEDYLRAISEFSEIFNQRKKERAKELLNSFNEKIRQLKKEVNGNISEEDLKIL EVEAEKAYNYENGFIEWEYSEQFLGVLEKIARAVLISDNYFDLKKYPILIRKPTNKSKKI TNLKPEEWDYYIQFGYGLINSPMKIETKNFMGIDRGLTHLLAYSIFDRDSEKFTINQLEL NPIKGWKWKLRKVKRSLQHLERRMRAQKGVKLPENQMKKRLKSIEPKIESYYHNLSR KIVNLAKANNASIVVESLEGGGLKQHGRKKNSRHRALNYALSLFDYGKIASLIKYKSD LEGVPMYEVLPAYTSQQCAKCVLKKGSFVEPEIIGYIEEIGFKENLLTLLFEDTGLSSVQ VLKKSKNKMTLSARDKEGKMVDLVLKYNFKGLVISQEKKKEEIVEFPIKEIDGKFAVL DSAYKRGKERISKKGNQKLVYTGNKKVGYCSVHGQVDADLNASRVIALCKYLGINEP IVFGEQRKSFK |
| SEQ ID NO: 83 | LDLITEPIQPHKSSSLRSKEFLEYQISDFLNFSLHSLFFGLASNEGPLVDFKIYDKIVIPKPE ERFPKKESEEGKKLDSFDKRVEEYYSDKLEKKIERKLNTEEKNVIDREKTRIWGEVNK LEEIRSIIDEINEIKKQKHISEKSKLLGEKWKKVNNIQETLLSQEYVSLISNLSDELTNKK KELLAKKYSKFDDKIKKIKEDYGLEFDENTIKKEGEKAFLNPDKFSKYQFSSSYLKLIG EIARSLITYKGFLDLNKYPIIFRKPINKVKKIHNLEPDEWKYYIQFGYEQINNPKLETENI LGIDRGLTHILAYSVFEPRSSKFILNKLEPNPIEGWKWKLRKLRRSIQNLERRWRAQDN VKLPENQMKKNLRSIEDKVENLYHNLSRKIVDLAKEKNACIVFEKLEGQGMKQHGRK KSDRLRGLNYKLSLFDYGKIAKLIKYKAEIEGIPIYRIDSAYTSQNCAKCVLESRRFAQP EEISCLDDFKEGDNLDKRILEGTGLVEAKIYKKLLKEKKEDFEIEEDIAMFDTKKVIKEN KEKTVILDYVYTRRKEIIGTNHKKNIKGIAKYTGNTKIGYCMKHGQVDADLNASRTIA LCKNFDINNPEIWK |
| SEQ ID NO: 84 | MSDESLVSSEDKLAIKIKIVPNAEQAKMLDEMFKKWSSICNRISRGKEDIETLRPDEGK ELQFNSTQLNSATMDVSDLKKAMARQGERLEAEVSKLRGRYETIDASLRDPSRRHTNP QKPSSFYPSDWDISGRLTPRFHTARHYSTELRKLKAKEDKMLKTINKIKNGKIVFKPKR ITLWPSSVNMAFKGSRLLLKPFANGFEMELPIVISPQKTADGKSQKASAEYMRNALLG LAGYSINQLLFGMNRSQKMLANAKKPEKVEKFLEQMKNKDANFDKKIKALEGKWLL DRKLKESEKSSIAVVRTKFFKSGKVELNEDYLKLLKHMANEILERDGFVNLNKYPILSR KPMKRYKQKNIDNLKPNMWKYYIQFGYEPIFERKASGKPKNIMGIDRGLTHLLAVAV FSPDQQKFLFNHLESNPIMHWKWKLRKIRRSIQHMERRIRAEKNKHIHEAQLKKRLGSI EEKTEQHYHIVSSKIINWAIEYEAAIVLESLSHMKQRGGKKSVRTRALNYALSLFDYEK VARLITYKARIRGIPVYDVLPGMTSKTCATCLLNGSQGAYVRGLETTKAAGKATKRK NMKIGKCMVCNSSENSMIDADLNAARVIAICKYKNLNDPQPAGSRKVFKRF |
| SEQ ID NO: 85 | MLALKLKIMPTEKQAEILDAMFWKWASICSRIAKMKKKVSVKENKKELSKKIPSNSDI WFSKTQLCQAEVDVGDHKKALKNFEKRQESLLDELKYKVKAINEVINDESKREIDPN NPSKFRIKDSTKKGNLNSPKFFTLKKWQKILQENEKRIKKKESTIEKLKRGNIFFNPTKIS LHEEEYSINFGSSKLLLNCFYKYNKKSGINSDQLENKFNEFQNGLNIICSPLQPIRGSSKR SFEFIRNSIINFLMYSLYAKLFGIPRSVKALMKSNKDENKLKLEEKLKKKKSSFNKTVK EFEKMIGRKLSDNESKILNDESKKFFEIIKSNNKYIPSEEYLKLLKDISEEIYNSNIDFKPY KYSILIRKPLSKFKSKKLYNLKPTDYKYYLQLSYEPFSKQLIATKTILGIDRGLKHLLAV SVFDPSQNKFVYNKLIKNPVFKWKKRYHDLKRSIRNRERRIRALTGVHIHENQLIKKLK SMKNKINVLYHNVSKNIVDLAKKYESTIVLERLENLKQHGRSKGKRYKKLNYVLSNF DYKKIESLISYKAKKEGVPVSNINPKYTSKTCAKCLLEVNQLSELKNEYNRDSKNSKIG ICNIHGQIDADLNAARVIALCYSKNLNEPHFK |
| SEQ ID NO: 86 | VINLFGYKFALYPNKTQEELLNKHLGECGWLYNKAIEQNEYYKADSNIEEAQKKFELL PDKNSDEAKVLRGNISKDNYVYRTLVKKKSEINVQIRKAVVLRPAETIRNLAKVKKK GLSVGRLKFIPIREWDVLPFKQSDQIRLEENYLILEPYGRLKFKMHRPLLGKPKTFCIKR TATDRWTISFSTEYDDSNMRKNDGGQVGIDVGLKTHLRLSNENPDEDPRYPNPKIWK RYDRRLTILQRRISKSKKLGKNRTRLRLRLSRLWEKIRNSRADLIQNETYEILSENKLIAI EDLNVKGMQEKKDKKGRKGRTRAQEKGLHRSISDAAFSEFRRVLEYKAKRFGSEVKP VSAIDSSKECHNCGNKKGMPLESRIYECPKCGLKIDRDLNSAKVILARATGVRPGSNA RADTKISATAGASVQTEGTVSEDFRQQMETSDQKPMQGEGSKEPPMNPEHKSSGRGS KHVNIGCKNKVGLYNEDENSRSTEKQIMDENRSTTEDMVEIGALHSPVLTT |
| SEQ ID NO: 87 | MIASIDYEAVSQALIVFEFKAKGKDSQYQAIDEAIRSYRFIRNSCLRYWMDNKKVGKY DLNKYCKVLAKQYPFANKLNSQARQSAAECSWSAISRFYDNCKRKVSGKKGFPKFKK HARSVEYKTSGWKLSENRKAITFTDKNGIGKLKLKGTYDLHFSQLEDMKRVRLVRRA DGYYVQFCISVDVKVETEPTGKAIGLDVGIKYFLADSSGNTIENPQFYRKAEKKLNRA NRRKSKKYIRGVKPQSKNYHKARCRYARKHLRVSRQRKEYCKRVAYCVIHSNDVVA YEDLNVKGMVKNRHLAKSISDVAWSTFRHWLEYFAIKYGKLTIPVAPHNTSQNCSNC DKKVPKSLSTRTHICHHCGYSEDRDVNAAKNILKKALSTVGQTGSLKLGEIEPLLVLEQ SCTRKFDL |
| SEQ ID NO: 88 | LAEENTLHLTLAMSLPLNDLPENRTRSELWRRQWLPQKKLSLLLGVNQSVRKAAADC LRWFEPYQELLWWEPTDPDGKKLLDKEGRPIKRTAGHMRVLRKLEEIAPFRGYQLGS AVKNGLRHKVADLLLSYAKRKLDPQFTDKTSYPSIGDQFPIVWTGAFVCYEQSITGQL YLYLPLFPRGSHQEDITNNYDPDRGPALQVFGEKEIARLSRSTSGLLLPLQFDKWGEAT FIRGENNPPTWKATHRRSDKKWLSEVLLREKDFQPKRVELLVRNGRIFVNVACEIPTK PLLEVENFMGVSFGLEHLVTVVVINRDGNVVHQRQEPARRYEKTYFARLERLRRRGG PFSQELETFHYRQVAQIVEEALRFKSVPAVEQVGNIPKGRYNPRLNLRLSYWPFGKLA DLTSYKAVKEGLPKPYSVYSATAKMLCSTCGAANKEGDQPISLKGPTVYCGNCGTRH NTGFNTALNLARRAQELFVKGVVAR |
| SEQ ID NO: 89 | MSQSLLKWHDMAGRDKDASRSLQKSAVEGVLLHLTASHRVALEMLEKSVSQTVAVT MEAAQQRLVIVLEDDPTKATSRKRVISADLQFTREEFGSLPNWAQKLASTCPEIATKY ADKHINSIRIAWGVAKESTNGDAVEQKLQWQIRLLDVTMFLQQLVLQLADKALLEQIP SSIRGGIGQEVAQQVTSHIQLLDSGTVLKAELPTISDRNSELARKQWEDAIQTVCTYAL PFSRERARILDPGKYAAEDPRGDRLINIDPMWARVLKGPTVKSLPLLFVSGSSIRIVKLT LPRKHAAGHKHTFTATYLVLPVSREWINSLPGTVQEKVQWWKKPDVLATQELLVGK GALKKSANTLVIPISAGKKRFFNHILPALQRGFPLQWQRIVGRSYRRPATHRKWFAQLT IGYTNPSSLPEMALGIHFGMKDILWWALADKQGNILKDGSIPGNSILDFSLQEKGKIER QQKAGKNVAGKKYGKSLLNATYRVVNGVLEFSKGISAEHASQPIGLGLETIRFVDKAS GSSPVNARHSNWNYGQLSGIFANKAGPAGFSVTEITLKKAQRDLSDAEQARVLAIEAT KRFASRIKRLATKRKDDTLFV |
| SEQ ID NO: 90 | VEPVEKERFYYRTYTFRLDGQPRTQNLTTQSGWGLLTKAVLDNTKHYWEIVHHARIA NQPIVFENPVIDEQGNPKLNKLGQPRFWKRPISDIVNQLRALFENQNPYQLGSSLIQGT YWDVAENLASWYALNKEYLAGTATWGEPSFPEPHPLTEINQWMPLTFSSGKVVRLLK NASGRYFIGLPILGENNPCYRMRTIEKLIPCDGKGRVTSGSLILFPLVGIYAQQHRRMTD ICESIRTEKGKLAWAQVSIDYVREVDKRRRMRRTRKSQGWIQGPWQEVFILRLVLAHK APKLYKPRCFAGISLGPKTLASCVILDQDERVVEKQQWSGSELLSLIHQGEERLRSLRE QSKPTWNAAYRKQLKSLINTQVFTIVTFLRERGAAVRLESIARVRKSTPAPPVNFLLSH WAYRQITERLKDLAIRNGMPLTHSNGSYGVRFTCSQCGATNQGIKDPTKYKVDIESET FLCSICSHREIAAVNTATNLAKQLLDE |
| SEQ ID NO: 91 | MNDTETSETLTSHRTVCAHLHVVGETGSLPRLVEAALAELITLNGRATQALLSLAKNG LVLRRDKEENLIAAELTLPCRKNKYADVAAKAGEPILATRINNKGKLVTKKWYGEGN SYHIVRFTPETGMFTVRVFDRYAFDEELLHLHSEVVFGSDLPKGIKAKTDSLPANFLQA VFTSFLELPFQGFPDIVVKPAMKQAAEQLLSYVQLEAGENQQAEYPDTNERDPELRLV EWQKSLHELSVRTEPFEFVRARDIDYYAETDRRGNRFVNITPEWTKFAESPFARRLPLK IPPEFCILLRRKTEGHAKIPNRIYLGLQIFDGVTPDSTLGVLATAEDGKLFWWHDHLDE FSNLEGKPEPKLKNKPQLLMVSLEYDREQRFEESVGGDRKICLVTLKETRNFRRGWNG RILGIHFQHNPVITWALMDHDAEVLEKGFIEGNAFLGKALDKQALNEYLQKGGKWVG DRSFGNKLKGITHTLASLIVRLAREKDAWIALEEISWVQKQSADSVANHEIVEQPHHSL TR |
| SEQ ID NO: 92 | MNDTETSETLTSHRTVCAHLHVVGETGSLPRLVEAALAELITLNGRATQALLSLAKNG LVLRRDKEENLIAAELTLPCRKNKYADVAAKAGEPILATRINNKGKLVTKKWYGEGN SYHIVRFTPETGMFTVRVFDRYAFDEELLHLHSEVVFGSDLPKGIKAKTDSLPANFLQA VFTSFLELPFQGFPDIVVKPAMKQAAEQLLSYVQLEAGENQQAEYPDTNERDPELRLV EWQKSLHELSVRTEPFEFVRARDIDYYAETDRRGNRFVNITPEWTKFAESPFARRLPLK IPPEFCILLRRKTEGHAKIPNRIYLGLQIFDGVTPDSTLGVLATAEDGKLFWWHDHLDE FSNLEGKPEPKLKNKPQLLMVSLEYDREQRFEESVGGDRKICLVTLKETRNFRRGRHG HTRTDRLPAGNTLWRADFATSAEVAAPKWNGRILGIHFQHNPVITWALMDHDAEVLE KGFIEGNAFLGKALDKQALNEYLQKGGKWVGDRSFGNKLKGITHTLASLIVRLAREK DAWIALEEISWVQKQSADSVANRRFSMWNYSRLATLIEWLGTDIATRDCGTAAPLAH KVSDYLTHFTCPECGACRKAGQKKEIADTVRAGDILTCRKCGFSGPIPDNFIAEFVAKK ALERMLKKKPV |
| SEQ ID NO: 93 | MAKRNFGEKSEALYRAVRFEVRPSKEELSILLAVSEVLRMLFNSALAERQQVFTEFIAS LYAELKSASVPEEISEIRKKLREAYKEHSISLFDQINALTARRVEDEAFASVTRNWQEET LDALDGAYKSFLSLRRKGDYDAHSPRSRDSGFFQKIPGRSGFKIGEGRIALSCGAGRKL SFPIPDYQQGRLAETTKLKKFELYRDQPNLAKSGRFWISVVYELPKPEATTCQSEQVAF VALGASSIGVVSQRGEEVIALWRSDKHWVPKIEAVEERMKRRVKGSRGWLRLLNSGK RRMHMISSRQHVQDEREIVDYLVRNHGSHFVVTELVVRSKEGKLADSSKPERGGSLG LNWAAQNTGSLSRLVRQLEEKVKEHGGSVRKHKLTLTEAPPARGAENKLWMARKLR ESFLKEV |
| SEQ ID NO: 94 | LAKNDEKELLYQSVKFEIYPDESKIRVLTRVSNILVLVWNSALGERRARFELYIAPLYE ELKKFPRKSAESNALRQKIREGYKEHIPTFFDQLKKLLTPMRKEDPALLGSVPRAYQEE TLNTLNGSFVSFMTLRRNNDMDAKPPKGRAEDRFHEISGRSGFKIDGSEFVLSTKEQKL RFPIPNYQLEKLKEAKQIKKFTLYQSRDRRFWISIAYEIELPDQRPFNPEEVIYIAFGASSI GVISPEGEKVIDFWRPDKHWKPKIKEVENRMRSCKKGSRAWKKRAAARRKMYAMTQ RQQKLNHREIVASLLRLGFHFVVTEYTVRSKPGKLADGSNPKRGGAPQGFNWSAQNT GSFGEFILWLKQKVKEQGGTVQTFRLVLGQSERPEKRGRDNKIEMVRLLREKYLESQT IVV |
| SEQ ID | MAKGKKKEGKPLYRAVRFEIFPTSDQITLFLRVSKNLQQVWNEAWQERQSCYEQFFG SIYERIGQAKKRAQEAGFSEVWENEAKKGLNKKLRQQEISMQLVSEKESLLQELSIAFQ |
| NO: 95 | EHGVTLYDQINGLTARRIIGEFALIPRNWQEETLDSLDGSFKSFLALRKNGDPDAKPPR QRVSENSFYKIPGRSGFKVSNGQIYLSFGKIGQTLTSVIPEFQLKRLETAIKLKKFELCRD ERDMAKPGRFWISVAYEIPKPEKVPVVSKQITYLAIGASRLGVVSPKGEFCLNLPRSDY HWKPQINALQERLEGVVKGSRKWKKRMAACTRMFAKLGHQQKQHGQYEVVKKLLR HGVHFVVTELKVRSKPGALADASKSDRKGSPTGPNWSAQNTGNIARLIQKLTDKASE HGGTVIKRNPPLLSLEERQLPDAQRKIFIAKKLREEFLADQK |
| SEQ ID NO: 96 | MAKREKKDDVVLRGTKMRIYPTDRQVTLMDMWRRRCISLWNLLLNLETAAYGAKN TRSKLGWRSIWARVVEENHAKALIVYQHGKCKKDGSFVLKRDGTVKHPPRERFPGDR KILLGLFDALRHTLDKGAKCKCNVNQPYALTRAWLDETGHGARTADIIAWLKDFKGE CDCTAISTAAKYCPAPPTAELLTKIKRAAPADDLPVDQAILLDLFGALRGGLKQKECD HTHARTVAYFEKHELAGRAEDILAWLIAHGGTCDCKIVEEAANHCPGPRLFIWEHELA MIMARLKAEPRTEWIGDLPSHAAQTVVKDLVKALQTMLKERAKAAAGDESARKTGF PKFKKQAYAAGSVYFPNTTMFFDVAAGRVQLPNGCGSMRCEIPRQLVAELLERNLKP GLVIGAQLGLLGGRIWRQGDRWYLSCQWERPQPTLLPKTGRTAGVKIAASIVFTTYDN RGQTKEYPMPPADKKLTAVHLVAGKQNSRALEAQKEKEKKLKARKERLRLGKLEKG HDPNALKPLKRPRVRRSKLFYKSAARLAACEAIERDRRDGFLHRVTNEIVHKFDAVSV QKMSVAPMMRRQKQKEKQIESKKNEAKKEDNGAAKKPRNLKPVRKLLRHVAMARG RQFLEYKYNDLRGPGSVLIADRLEPEVQECSRCGTKNPQMKDGRRLLRCIGVLPDGTD CDAVLPRNRNAARNAEKRLRKHREAHNA |
| SEQ ID NO: 97 | MNEVLPIPAVGEDAADTIMRGSKMRIYPSVRQAATMDLWRRRCIQLWNLLLELEQAA YSGENRRTQIGWRSIWATVVEDSHAEAVRVAREGKKRKDGTFRKAPSGKEIPPLDPA MLAKIQRQMNGAVDVDPKTGEVTPAQPRLFMWEHELQKIMARLKQAPRTHWIDDLP SHAAQSVVKDLIKALQAMLRERKKRASGIGGRDTGFPKFKKNRYAAGSVYFANTQLR FEAKRGKAGDPDAVRGEFARVKLPNGVGWMECRMPRHINAAHAYAQATLMGGRIW RQGENWYLSCQWKMPKPAPLPRAGRTAAIKIAAAIPITTVDNRGQTREYAMPPIDRERI AAHAAAGRAQSRALEARKRRAKKREAYAKKRHAKKLERGIAAKPPGRARIKLSPGFY AAAAKLAKLEAEDANAREAWLHEITTQIVRNFDVIAVPRMEVAKLMKKPEPPEEKEE QVKAPWQGKRRSLKAARVMMRRTAMALIQTTLKYKAVDLRGPQAYEEIAPLDVTAA ACSGCGVLKPEWKMARAKGREIMRCQEPLPGGKTCNTVLTYTRNSARVIGRELAVRL AERQKA |
| SEQ ID NO: 98 | MTTQKTYNFCFYDQRFFELSKEAGEVYSRSLEEFWKIYDETGVWLSKFDLQKHMRNK LERKLLHSDSFLGAMQQVHANLASWKQAKKVVPDACPPRKPKFLQAILFKKSQIKYK NGFLRLTLGTEKEFLYLKWDINIPLPIYGSVTYSKTRGWKINLCLETEVEQKNLSENKY LSIDLGVKRVATIFDGENTITLSGKKFMGLMHYRNKLNGKTQSRLSHKKKGSNNYKKI QRAKRKTTDRLLNIQKEMLHKYSSFIVNYAIRNDIGNIIIGDNSSTHDSPNMRGKTNQKI SQNPEQKLKNYIKYKFESISGRVDIVPEPYTSRKCPHCKNIKKSSPKGRTYKCKKCGFIF DRDGVGAINIYNENVSFGQIISPGRIRSLTEPIGMKFHNEIYFKSYVAA |
| SEQ ID NO: 99 | MSVRSFQARVECDKQTMEHLWRTHKVFNERLPEIIKILFKMKRGECGQNDKQKSLYK SISQSILEANAQNADYLLNSVSIKGWKPGTAKKYRNASFTWADDAAKLSSQGIHVYDK KQVLGDLPGMMSQMVCRQSVEAISGHIELTKKWEKEHNEWLKEKEKWESEDEHKKY LDLREKFEQFEQSIGGKITKRRGRWHLYLKWLSDNPDFAAWRGNKAVINPLSEKAQIR INKAKPNKKNSVERDEFFKANPEMKALDNLHGYYERNFVRRRKTKKNPDGFDHKPTF TLPHPTIHPRWFVFNKPKTNPEGYRKLILPKKAGDLGSLEMRLLTGEKNKGNYPDDWI SVKFKADPRLSLIRPVKGRRVVRKGKEQGQTKETDSYEFFDKHLKKWRPAKLSGVKLI FPDKTPKAAYLYFTCDIPDEPLTETAKKIQWLETGDVTKKGKKRKKKVLPHGLVSCAV DLSMRRGTTGFATLCRYENGKIHILRSRNLWVGYKEGKGCHPYRWTEGPDLGHIAKH KREIRILRSKRGKPVKGEESHIDLQKHIDYMGEDRFKKAARTIVNFALNTENAASKNGF YPRADVLLLENLEGLIPDAEKERGINRALAGWNRRHLVERVIEMAKDAGFKRRVFEIP PYGTSQVCSKCGALGRRYSIIRENNRREIRFGYVEKLFACPNCGYCANADHNASVNLN RRFLIEDSFKSYYDWKRLSEKKQKEEIETIESKLMDKLCAMHKISRGSISK |
| SEQ ID NO: 100 | MHLWRTHCVFNQRLPALLKRLFAMRRGEVGGNEAQRQVYQRVAQFVLARDAKDSV DLLNAVSLRKRSANSAFKKKATISCNGQAREVTGEEVFAEAVALASKGVFAYDKDDM RAGLPDSLFQPLTRDAVACMRSHEELVATWKKEYREWRDRKSEWEAEPEHALYLNL RPKFEEGEAARGGRFRKRAERDHAYLDWLEANPQLAAWRRKAPPAVVPIDEAGKRRI ARAKAWKQASVRAEEFWKRNPELHALHKIHVQYLREFVRPRRTRRNKRREGFKQRPT FTMPDPVRHPRWCLFNAPQTSPQGYRLLRLPQSRRTVGSVELRLLTGPSDGAGFPDAW VNVRFKADPRLAQLRPVKVPRTVTRGKNKGAKVEADGFRYYDDQLLIERDAQVSGV KLLFRDIRMAPFADKPIEDRLLSATPYLVFAVEIKDEARTERAKAIRFDETSELTKSGKK RKTLPAGLVSVAVDLDTRGVGFLTRAVIGVPEIQQTHHGVRLLQSRYVAVGQVEARA SGEAEWSPGPDLAHIARHKREIRRLRQLRGKPVKGERSHVRLQAHIDRMGEDRFKKA ARKIVNEALRGSNPAAGDPYTRADVLLYESLETLLPDAERERGINRALLRWNRAKLIE HLKRMCDDAGIRHFPVSPFGTSQVCSKCGALGRRYSLARENGRAVIRFGWVERLFACP NPECPGRRPDRPDRPFTCNSDHNASVNLHRVFALGDQAVAAFRALAPRDSPARTLAV KRVEDTLRPQLMRVHKLADAGVDSPF |
| SEQ ID NO: 101 | MATLVYRYGVRAHGSARQQDAVVSDPAMLEQLRLGHELRNALVGVQHRYEDGKRA VWSGFASVAAADHRVTTGETAVAELEKQARAEHSADRTAATRQGTAESLKAARAAV KQARADRKAAMAAVAEQAKPKIQALGDDRDAEIKDLYRRFCQDGVLLPRCGRCAGD LRSDGDCTDCGAAHEPRKLYWATYNAIREDHQTAVKLVEAKRKAGQPARLRFRRWT GDGTLTVQLQRMHGPACRCVTCAEKLTRRARKTDPQAPAVAADPAYPPTDPPRDPAL LASGQGKWRNVLQLGTWIPPGEWSAMSRAERRRVGRSHIGWQLGGGRQLTLPVQLH RQMPADADVAMAQLTRVRVGGRHRMSVALTAKLPDPPQVQGLPPVALHLGWRQRP DGSLRVATWACPQPLDLPPAVADVVVSHGGRWGEVIMPARWLADAEVPPRLLGRRD KAMEPVLEALADWLEAHTEACTARMTPALVRRWRSQGRLAGLTNRWRGQPPTGSAE ILTYLEAWRIQDKLLWERESHLRRRLAARRDDAWRRVASWLARHAGVLVVDDADIA ELRRRDDPADTDPTMPASAAQAARARAALAAPGRLRHLATITATRDGLGVHTVASAG LTRLHRKCGHQAQPDPRYAASAVVTCPGCGNGYDQDYNAAMLMLDRQQQP |
| SEQ ID NO: 102 | MSRVELHRAYKFRLYPTPAQVAELAEWERQLRRLYNLAHSQRLAAMQRHVRPKSPG VLKSECLSCGAVAVAEIGTDGKAKKTVKHAVGCSVLECRSCGGSPDAEGRTAHTAAC SFVDYYRQGREMTQLLEEDDQLARVVCSARQETLRDLEKAWQRWHKMPGFGKPHF KKRIDSCRIYFSTPKSWAVDLGYLSFTGVASSVGRIKIRQDRVWPGDAKFSSCHVVRD VDEWYAVFPLTFTKEIEKPKGGAVGINRGAVHAIADSTGRVVDSPKFYARSLGVIRHR ARLLDRKVPFGRAVKPSPTKYHGLPKADIDAAAARVNASPGRLVYEARARGSIAAAE AHLAALVLPAPRQTSQLPSEGRNRERARRFLALAHQRVRRQREWFLHNESAHYAQSY TKIAIEDWSTKEMTSSEPRDAEEMKRVTRARNRSILDVGWYELGRQIAYKSEATGAEF AKVDPGLRETETHVPEAIVRERDVDVSGMLRGEAGISGTCSRCGGLLRASASGHADAE CEVCLHVEVGDVNAAVNVLKRAMFPGAAPPSKEKAKVTIGIKGRKKKRAA |
| SEQ ID NO: 103 | MSRVELHRAYKFRLYPTPVQVAELSEWERQLRRLYNLGHEQRLLTLTRHLRPKSPGV LKGECLSCDSTQVQEVGADGRPKTTVRHAEQCPTLACRSCGALRDAEGRTAHTVACA FVDYYRQGREMTELLAADDQLARVVCSARQEVLRDLDKAWQRWRKMPGFGKPRFK RRTDSCRIYFSTPKAWKLEGGHLSFTGAATTVGAIKMRQDRNWPASVQFSSCHVVRD VDEWYAVFPLTFVAEVARPKGGAVGINRGAVHAIADSTGRVVDSPRYYARALGVIRH RARLFDRKVPSGHAVKPSPTKYRGLSAIEVDRVARATGFTPGRVVTEALNRGGVAYA ECALAAIAVLGHGPERPLTSDGRNREKARKFLALAHQRVRRQREWFLHNESAHYART YSKIAIEDWSTKEMTASEPQGEETRRVTRSRNRSILDVGWYELGRQLAYKTEATGAEF AQVDPGLKETETNVPKAIADARDVDVSGMLRGEAGISGTCSKCGGLLRAPASGHADA ECEICLNVEVGDVNAAVNVLKRAMFPGDAPPASGEKPKVSIGIKGRQKKKKAA |
| SEQ ID NO: 104 | MEAIATGMSPERRVELGILPGSVELKRAYKFRLYPMKVQQAELSEWERQLRRLYNLA HEQRLAALLRYRDWDFQKGACPSCRVAVPGVHTAACDHVDYFRQAREMTQLLEVD AQLSRVICCARQEVLRDLDKAWQRWRKKLGGRPRFKRRTDSCRIYLSTPKHWEIAGR YLRLSGLASSVGEIRIEQDRAFPEGALLSSCSIVRDVDEWYACLPLTFTQPIERAPHRSV GLNRGVVHALADSDGRVVDSPKFFERALATVQKRSRDLARKVSGSRNAHKARIKLAK AHQRVRRQRAAFLHQESAYYSKGFDLVALEDMSVRKMTATAGEAPEMGRGAQRDL NRGILDVGWYELARQIDYKRLAHGGELLRVDPGQTTPLACVTEEQPARGISSACAVCG IPLARPASGNARMRCTACGSSQVGDVNAAENVLTRALSSAPSGPKSPKASIKIKGRQK RLGTPANRAGEASGGDPPVRGPVEGGTLAYVVEPVSESQSDT |
| SEQ ID NO: 105 | MTVRTYKYRAYPTPEQAEALTSWLRFASQLYNAALEHRKNAWGRHDAHGRGFRFW DGDAAPRKKSDPPGRWVYRGGGAHISKNDQGKLLTEFRREHAELLPPGMPALVQHE VLARLERSMAAFFQRATKGQKAGYPRWRSEHRYDSLTFGLTSPSKERFDPETGESLGR GKTVGAGTYHNGDLRLTGLGELRILEHRRIPMGAIPKSVIVRRSGKRWFVSIAMEMPS VEPAASGRPAVGLDMGVVTWGTAFTADTSAAAALVADLRRMATDPSDCRRLEELER EAAQLSEVLAHCRARGLDPARPRRCPKELTKLYRRSLHRLGELDRACARIRRRLQAAH DIAEPVPDEAGSAVLIEGSNAGMRHARRVARTQRRVARRTRAGHAHSNRRKKAVQA YARAKERERSARGDHRHKVSRALVRQFEEISVEALDIKQLTVAPEHNPDPQPDLPAHV QRRRNRGELDAAWGAFFAALDYKAADAGGRVARKPAPHTTQECARCGTLVPKPISLR VHRCPACGYTAPRTVNSARNVLQRPLEEPGRAGPSGANGRGVPHAVA |
| SEQ ID NO: 106 | MNCRYRYRIYPTPGQRQSLARLFGCVRVVWNDALFLCRQSEKLPKNSELQKLCITQA KKTEARGWLGQVSAIPLQQSVADLGVAFKNFFQSRSGKRKGKKVNPPRVKRRNNRQ GARFTRGGFKVKTSKVYLARIGDIKIKWSRPLPSEPSSVTVIKDCAGQYFLSFVVEVKP EIKPPKNPSIGIDLGLKTFASCSNGEKIDSPDYSRLYRKLKRCQRRLAKRQRGSKRRER MRVKVAKLNAQIRDKRKDFLHKLSTKVVNENQVIALEDLNVGGMLKNRKLSRAISQA GWYEFRSLCEGKAEKHNRDFRVISRWEPTSQVCSECGYRWGKIDLSVRSIVCINCGVE HDRDDNASVNIEQAGLKVGVGHTHDSKRTGSACKTSNGAVCVEPSTHREYVQLTLFD W |
| SEQ ID NO: 107 | MKSRWTFRCYPTPEQEQHLARTFGCVRFVWNWALRARTDAFRAGERIGYPATDKAL TLLKQQPETVWLNEVSSVCLQQALRDLQVAFSNFFDKRAAHPSFKRKEARQSANYTE RGFSFDHERRILKLAKIGAIKVKWSRKAIPHPSSIRLIRTASGKYFVSLVVETQPAPMPE TGESVGVDFGVARLATLSNGERISNPKHGAKWQRRLAFYQKRLARATKGSKRRMRIK RHVARIHEKIGNSRSDTLHKLSTDLVTRFDLICVEDLNLRGMVKNHSLARSLHDASIGS AIRMIEEKAERYGKNVVKIDRWFPSSKTCSDCGHIVEQLPLNVREWTCPECGTTHDRD ANAAANILAVGQTVSAHGGTVRRSRAKASERKSQRSANRQGVNRA |
| SEQ ID NO: 108 | KEPLNIGKTAKAVFKEIDPTSLNRAANYDASIELNCKECKFKPFKNVKRYEFNFYNNW YRCNPNSCLQSTYKAQVRKVEIGYEKLKNEILTQMQYYPWFGRLYQNFFHDERDKMT SLDEIQVIGVQNKVFFNTVEKAWREIIKKRFKDNKETMETIPELKHAAGHGKRKLSNK SLLRRRFAFVQKSFKFVDNSDVSYRSFSNNIACVLPSRIGVDLGGVISRNPKREYIPQEIS FNAFWKQHEGLKKGRNIEIQSVQYKGETVKRIEADTGEDKAWGKNRQRRFTSLILKL VPKQGGKKVWKYPEKRNEGNYEYFPIPIEFILDSGETSIRFGGDEGEAGKQKHLVIPFN DSKATPLASQQTLLENSRFNAEVKSCIGLAIYANYFYGYARNYVISSIYHKNSKNGQAI TAIYLESIAHNYVKAIERQLQNLLLNLRDFSFMESHKKELKKYFGGDLEGTGGAQKRR EKEEKIEKEIEQSYLPRLIRLSLTKMVTKQVEM |
| SEQ ID NO: 109 | ELIVNENKDPLNIGKTAKAVFKEIDPTSINRAANYDASIELACKECKFKPFNNTKRHDFS FYSNWHRCSPNSCLQSTYRAKIRKTEIGYEKLKNEILNQMQYYPWFGRLYQNFFNDQR DKMTSLDEIQVTGVQNKIFFNTVEKAWREIIKKRFRDNKETMRTIPDLKNKSGHGSRK LSNKSLLRRRFAFAQKSFKLVDNSDVSYRAFSNNVACVLPSKIGVDIGGIINKDLKREYI PQEITFNVFWKQHDGLKKGRNIEIHSVQYKGEIVKRIEADTGEDKAWGKNRQRRFTSL ILKITPKQGGKKIWKFPEKKNASDYEYFPIPIEFILDNGDASIKFGGEEGEVGKQKHLLIP FNDSKATPLSSKQMLLETSRFNAEVKSTIGLALYANYFVSYARNYVIKSTYHKNSKKG QIVTEIYLESISQNFVRAIQRQLQSLMLNLKDWGFMQTHKKELKKYFGSDLEGSKGGQ KRREKEEKIEKEIEASYLPRLIRLSLTKSVTKAEEM |
| SEQ ID NO: 110 | PEEKTSKLKPNSINLAANYDANEKFNCKECKFHPFKNKKRYEFNFYNNLHGCKSCTKS TNNPAVKRIEIGYQKLKFEIKNQMEAYPWFGRLRINFYSDEKRKMSELNEMQVTGVK NKIFFDAIECAWREILKKRFRESKETLITIPKLKNKAGHGARKHRNKKLLIRRRAFMKK NFHFLDNDSISYRSFANNIACVLPSKVGVDIGGIISPDVGKDIKPVDISLNLMWASKEGI KSGRKVEIYSTQYDGNMVKKIEAETGEDKSWGKNRKRRQTSLLLSIPKPSKQVQEFDF KEWPRYKDIEKKVQWRGFPIKIIFDSNHNSIEFGTYQGGKQKVLPIPFNDSKTTPLGSK MNKLEKLRFNSKIKSRLGSAIAANKFLEAARTYCVDSLYHEVSSANAIGKGKIFIEYYL EILSQNYIEAAQKQLQRFIESIEQWFVADPFQGRLKQYFKDDLKRAKCFLCANREVQTT CYAAVKLHKSCAEKVKDKNKELAIKERNNKEDAVIKEVEASNYPRVIRLKLTKTITNK AM |
| SEQ ID NO: 111 | SESENKIIEQYYAFLYSFRDKYEKPEFKNRGDIKRKLQNKWEDFLKEQNLKNDKKLSN YIFSNRNFRRSYDREEENEEGIDEKKSKPKRINCFEKEKNLKDQYDKDAINASANKDG AQKWGCFECIFFPMYKIESGDPNKRIIINKTRFKLFDFYLNLKGCKSCLRSTYHPYRSNV YIESNYDKLKREIGNFLQQKNIFQRMRKAKVSEGKYLTNLDEYRLSCVAMHFKNRWL FFDSIQKVLRETIKQRLKQMRESYDEQAKTKRSKGHGRAKYEDQVRMIRRRAYSAQA HKLLDNGYITLFDYDDKEINKVCLTAINQEGFDIGGYLNSDIDNVMPPIEISFHLKWKY NEPILNIESPFSKAKISDYLRKIREDLNLERGKEGKARSKKNVRRKVLASKGEDGYKKI FTDFFSKWKEELEGNAMERVLSQSSGDIQWSKKKRIHYTTLVLNINLLDKKGVGNLK YYEIAEKTKILSFDKNENKFWPITIQVLLDGYEIGTEYDEIKQLNEKTSKQFTIYDPNTKI IKIPFTDSKAVPLGMLGINIATLKTVKKTERDIKVSKIFKGGLNSKIVSKIGKGIYAGYFP TVDKEILEEVEEDTLDNEFSSKSQRNIFLKSIIKNYDKMLKEQLFDFYSFLVRNDLGVRF LTDRELQNIEDESFNLEKRFFETDRDRIARWFDNTNTDDGKEKFKKLANEIVDSYKPRL IRLPVVRVIKRIQPVKQREM |
| SEQ ID NO: 112 | KYSTRDFSELNEIQVTACKQDEFFKVIQNAWREIIKKRFLENRENFIEKKIFKNKKGRG KRQESDKTIQRNRASVMKNFQLIENEKIILRAPSGHVACVFPVKVGLDIGGFKTDDLEK NIFPPRTITINVFWKNRDRQRKGRKLEVWGIKARTKLIEKVHKWDKLEEVKKKRLKSL EQKQEKSLDNWSEVNNDSFYKVQIDELQEKIDKSLKGRTMNKILDNKAKESKEAEGL YIEWEKDFEGEMLRRIEASTGGEEKWGKRRQRRHTSLLLDIKNNSRGSKEIINFYSYAK QGKKEKKIEFFPFPLTITLDAEEESPLNIKSIPIEDKNATSKYFSIPFTETRATPLSILGDRV QKFKTKNISGAIKRNLGSSISSCKIVQNAETSAKSILSLPNVKEDNNMEIFINTMSKNYF RAMMKQMESFIFEMEPKTLIDPYKEKAIKWFEVAASSRAKRKLKKLSKADIKKSELLL SNTEEFEKEKQEKLEALEKEIEEFYLPRIVRLQLTKTILETPVM |
| SEQ ID NO: 113 | KKLQLLGHKILLKEYDPNAVNAAANFETSTAELCGQCKMKPFKNKRRFQYTFGKNYH GCLSCIQNVYYAKKRIVQIAKEELKHQLTDSIASIPYKYTSLFSNTNSIDELYILKQERA AFFSNTNSIDELYITGIENNIAFKVISAIWDEIIKKRRQRYAESLTDTGTVKANRGHGGT AYKSNTRQEKIRALQKQTLHMVTNPYISLARYKNNYIVATLPRTIGMHIGAIKDRDPQ KKLSDYAINFNVFWSDDRQLIELSTVQYTGDMVRKIEAETGENNKWGENMKRTKTSL LLEILTKKTTDELTFKDWAFSTKKEIDSVTKKTYQGFPIGIIFEGNESSVKFGSQNYFPLP FDAKITPPTAEGFRLDWLRKGSFSSQMKTSYGLAIYSNKVTNAIPAYVIKNMFYKIARA ENGKQIKAKFLKKYLDIAGNNYVPFIIMQHYRVLDTFEEMPISQPKVIRLSLTKTQHIIIK KDKTDSKM |
| SEQ ID NO: 114 | NTSNLINLGKKAINISANYDANLEVGCKNCKFLSSNGNFPRQTNVKEGCHSCEKSTYEP SIYLVKIGERKAKYDVLDSLKKFTFQSLKYQSKKSMKSRNKKPKELKEFVIFANKNKA FDVIQKSYNHLILQIKKEINRMNSKKRKKNHKRRLFRDREKQLNKLRLIESSNLFLPRE NKGNNHVFTYVAIHSVGRDIGVIGSYDEKLNFETELTYQLYFNDDKRLLYAYKPKQN KIIKIKEKLWNLRKEKEPLDLEYEKPLNKSITFSIKNDNLFKVSKDLMLRRAKFNIQGKE KLSKEERKINRDLIKIKGLVNSMSYGRFDELKKEKNIWSPHIYREVRQKEIKPCLIKNGD RIEIFEQLKKKMERLRRFREKRQKKISKDLIFAERIAYNFHTKSIKNTSNKINIDQEAKRG KASYMRKRIGYETFKNKYCEQCLSKGNVYRNVQKGCSCFENPFDWIKKGDENLLPKK NEDLRVKGAFRDEALEKQIVKIAFNIAKGYEDFYDNLGESTEKDLKLKFKVGTTINEQ ESLKL |
| SEQ ID NO: 115 | TSNPIKLGKKAINISANYDSNLQIGCKNCKFLSYNGNFPRQTNVKEGCHSCEKSTYEPP VYTVRIGERRSKYDVLDSLKKFIFLSLKYRQSKKMKTRSKGIRGLEEFVISANLKKAM DVIQKSYRHLILNIKNEIVRMNGKKRNKNHKRLLFRDREKQLNKLRLIEGSSFFKPPTV KGDNSIFTCVAIHNIGRDIGIAGDYFDKLEPKIELTYQLYYEYNPKKESEINKRLLYAYK PKQNKIIEIKEKLWNLRKEKSPLDLEYEKPLTKSITFLVKRDGVFRISKDLMLRKAKFII QGKEKLSKEERKINRDLIKIKSNIISLTYGRFDELKKDKTIWSPHIFRDVKQGKITPCIER KGDRMDIFQQLRKKSERLRENRKKRQKKISKDLIFAERIAYNFHTKSIKNTSNLINIKHE AKRGKASYMRKRIGNETFRIKYCEQCFPKNNVYKNVQKGCSCFEDPFEYIKKGNEDLI PNKNQDLKAKGAFRDDALEKQIIKVAFNIAKGYEDFYENLKKTTEKDIRLKFKVGTIIS EEM |
| SEQ ID NO: 116 | NNSINLSKKAINISANYDANLQVRCKNCKFLSSNGNFPRQTDVKEGCHSCEKSTYEPPV YDVKIGEIKAKYEVLDSLKKFTFQSLKYQLSKSMKFRSKKIKELKEFVIFAKESKALNV INRSYKHLILNIKNDINRMNSKKRIKNHKGRLFLDRQKQLSKLKLIEGSSFFVPAKNVG NKSVFTCVAIHSIGRDIGIAGLYDSFTKPVNEITYQIFFSGERRLLYAYKPKQLKILSIKE NLWSLKNEKKPLDLLYEKPLGKNLNFNVKGGDLFRVSKDLMIRNAKFNVHGRQRLSD EERLINRNFIKIKGEVVSLSYGRFEELKKDRKLWSPHIFKDVRQNKIKPCLVMQGQRIDI FEQLKRKLELLKKIRKSRQKKLSKDLIFGERIAYNFHTKSIKNTSNKINIDSDAKRGRAS YMRKRIGNETFKLKYCDVCFPKANVYRRVQNGCSCSENPYNYIKKGDKDLLPKKDEG LAIKGAFRDEKLNKQIIKVAFNIAKGYEDFYDDLKKRTEKDVDLKFKIGTTVLDQKPM EIFDGIVITWL |
| SEQ ID NO: 117 | LLTTVVETNNLAKKAINVAANFDANIDRQYYRCTPNLCRFIAQSPRETKEKDAGCSSC TQSTYDPKVYVIKIGKLLAKYEILKSLKRFLFMNRYFKQKKTERAQQKQKIGTELNEM SIFAKATNAMEVIKRATKHCTYDIIPETKSLQMLKRRRHRVKVRSLLKILKERRMKIKK IPNTFIEIPKQAKKNKSDYYVAAALKSCGIDVGLCGAYEKNAEVEAEYTYQLYYEYKG NSSTKRILYCYNNPQKNIREFWEAFYIQGSKSHVNTPGTIRLKMEKFLSPITIESEALDFR VWNSDLKIRNGQYGFIKKRSLGKEAREIKKGMGDIKRKIGNLTYGKSPSELKSIHVYRT ERENPKKPRAARKKEDNFMEIFEMQRKKDYEVNKKRRKEATDAAKIMDFAEEPIRHY HTNNLKAVRRIDMNEQVERKKTSVFLKRIMQNGYRGNYCRKCIKAPEGSNRDENVLE KNEGCLDCIGSEFIWKKSSKEKKGLWHTNRLLRRIRLQCFTTAKAYENFYNDLFEKKE SSLDIIKLKVSITTKSM |
| SEQ ID NO: 118 | ASTMNLAKQAINFAANYDSNLEIGCKGCKFMSTWSKKSNPKFYPRQNNQANKCHSCT YSTGEPEVPIIEIGERAAKYKIFTALKKFVFMSVAYKERRRQRFKSKKPKELKELAICSN REKAMEVIQKSVVHCYGDVKQEIPRIRKIKVLKNHKGRLFYKQKRSKIKIAKLEKGSFF KTFIPKVHNNGCHSCHEASLNKPILVTTALNTIGADIGLINDYSTIAPTETDISWQVYYE FIPNGDSEAVKKRLLYFYKPKGALIKSIRDKYFKKGHENAVNTGFFKYQGKIVKGPIKF VNNELDFARKPDLKSMKIKRAGFAIPSAKRLSKEDREINRESIKIKNKIYSLSYGRKKTL SDKDIIKHLYRPVRQKGVKPLEYRKAPDGFLEFFYSLKRKERRLRKQEKRQKDMSEII DAADEFAWHRHTGSIKKTTNHINFKSEVKRGKVPIMKKRIANDSFNTRHCGKCVKQG NAINKYYIEKQKNCFDCNSIEFKWEKAALEKKGAFKLNKRLQYIVKACFNVAKAYESF YEDFRKGEEESLDLKFKIGTTTTLKQYPQNKARAM |
| SEQ ID NO: 119 | HSHNLMLTKLGKQAINFAANYDANLEIGCKNCKFLSYSPKQANPKKYPRQTDVHEDG NIACHSCMQSTKEPPVYIVPIGERKSKYEILTSLNKFTFLALKYKEKKRQAFRAKKPKE LQELAIAFNKEKAIKVIDKSIQHLILNIKPEIARIQRQKRLKNRKGKLLYLHKRYAIKMG LIKNGKYFKVGSPKKDGKKLLVLCALNTIGRDIGIIGNIEENNRSETEITYQLYFDCLDA NPNELRIKEIEYNRLKSYERKIKRLVYAYKPKQTKILEIRSKFFSKGHENKVNTGSFNFE NPLNKSISIKVKNSAFDFKIGAPFIMLRNGKFHIPTKKRLSKEEREINRTLSKIKGRVFRL TYGRNISEQGSKSLHIYRKERQHPKLSLEIRKQPDSFIDEFEKLRLKQNFISKLKKQRQK KLADLLQFADRIAYNYHTSSLEKTSNFINYKPEVKRGRTSYIKKRIGNEGFEKLYCETCI KSNDKENAYAVEKEELCFVCKAKPFTWKKTNKDKLGIFKYPSRIKDFIRAAFTVAKSY NDFYENLKKKDLKNEIFLKFKIGLILSHEKKNHISIAKSVAEDERISGKSIKNILNKSIKLE KNCYSCFFHKEDM |
| SEQ ID NO: 120 | SLERVIDKRNLAKKAINIAANFDANINKGFYRCETNQCMFIAQKPRKTNNTGCSSCLQS TYDPVIYVVKVGEMLAKYEILKSLKRFVFMNRSFKQKKTEKAKQKERIGGELNEMSIF ANAALAMGVIKRAIRHCHVDIRPEINRLSELKKTKHRVAAKSLVKIVKQRKTKWKGIP NSFIQIPQKARNKDADFYVASALKSGGIDIGLCGTYDKKPHADPRWTYQLYFDTEDES EKRLLYCYNDPQAKIRDFWKTFYERGNPSMVNSPGTIEFRMEGFFEKMTPISIESKDFD FRVWNKDLLIRRGLYEIKKRKNLNRKAREIKKAMGSVKRVLANMTYGKSPTDKKSIP VYRVEREKPKKPRAVRKEENELADKLENYRREDFLIRNRRKREATEIAKIIDAAEPPIR HYHTNHLRAVKRIDLSKPVARKNTSVFLKRIMQNGYRGNYCKKCIKGNIDPNKDECR LEDIKKCICCEGTQNIWAKKEKLYTGRINVLNKRIKQMKLECFNVAKAYENFYDNLA ALKEGDLKVLKLKVSIPALNPEASDPEEDM |
| SEQ ID NO: 121 | NASINLGKRAINLSANYDSNLVIGCKNCKFLSFNGNFPRQTNVREGCHSCDKSTYAPE VYIVKIGERKAKYDVLDSLKKFTFQSLKYQIKKSMRERSKKPKELLEFVIFANKDKAFN VIQKSYEHLILNIKQEINRMNGKKRIKNHKKRLFKDREKQLNKLRLIGSSSLFFPRENKG DKDLFTYVAIHSVGRDIGVAGSYESHIEPISDLTYQLFINNEKRLLYAYKPKQNKIIELK ENLWNLKKEKKPLDLEFTKPLEKSITFSVKNDKLFKVSKDLMLRQAKFNIQGKEKLSK EERQINRDFSKIKSNVISLSYGRFEELKKEKNIWSPHIYREVKQKEIKPCIVRKGDRIELF EQLKRKMDKLKKFRKERQKKISKDLNFAERIAYNFHTKSIKNTSNKINIDQEAKRGKA SYMRKRIGNESFRKKYCEQCFSVGNVYHNVQNGCSCFDNPIELIKKGDEGLIPKGKED RKYKGALRDDNLQMQIIRVAFNIAKGYEDFYNNLKEKTEKDLKLKFKIGTTISTQESN NKEM |
| SEQ ID NO: 122 | SNLIKLGKQAINFAANYDANLEVGCKNCKFLSSTNKYPRQTNVHLDNKMACRSCNQS TMEPAIYIVRIGEKKAKYDIYNSLTKFNFQSLKYKAKRSQRFKPKQPKELQELSIAVRK EKALDIIQKSIDHLIQDIRPEIPRIKQQKRYKNHVGKLFYLQKRRKNKLNLIGKGSFFKV FSPKEKKNELLVICALTNIGRDIGLIGNYNTIINPLFEVTYQLYYDYIPKKNNKNVQRRL LYAYKSKNEKILKLKEAFFKRGHENAVNLGSFSYEKPLEKSLTLKIKNDKDDFQVSPSL RIRTGRFFVPSKRNLSRQEREINRRLVKIKSKIKNMTYGKFETARDKQSVHIFRLERQKE KLPLQFRKDEKEFMEEFQKLKRRTNSLKKLRKSRQKKLADLLQLSEKVVYNNHTGTL KKTSNFLNFSSSVKRGKTAYIKELLGQEGFETLYCSNCINKGQKTRYNIETKEKCFSCK DVPFVWKKKSTDKDRKGAFLFPAKLKDVIKATFTVAKAYEDFYDNLKSIDEKKPYIKF KIGLILAHVRHEHKARAKEEAGQKNIYNKPIKIDKNCKECFFFKEEAM |
| SEQ ID NO: 123 | NTTRKKFRKRTGFPQSDNIKLAYCSAIVRAANLDADIQKKHNQCNPNLCVGIKSNEQS RKYEHSDRQALLCYACNQSTGAPKVDYIQIGEIGAKYKILQMVNAYDFLSLAYNLTKL RNGKSRGHQRMSQLDEVVIVADYEKATEVIKRSINHLLDDIRGQLSKLKKRTQNEHIT EHKQSKIRRKLRKLSRLLKRRRWKWGTIPNPYLKNWVFTKKDPELVTVALLHKLGRD IGLVNRSKRRSKQKLLPKVGFQLYYKWESPSLNNIKKSKAKKLPKRLLIPYKNVKLFD NKQKLENAIKSLLESYQKTIKVEFDQFFQNRTEEIIAEEQQTLERGLLKQLEKKKNEFAS QKKALKEEKKKIKEPRKAKLLMEESRSLGFLMANVSYALFNTTIEDLYKKSNVVSGCI PQEPVVVFPADIQNKGSLAKILFAPKDGFRIKFSGQHLTIRTAKFKIRGKEIKILTKTKRE ILKNIEKLRRVWYREQHYKLKLFGKEVSAKPRFLDKRKTSIERRDPNKLADQTDDRQA ELRNKEYELRHKQHKMAERLDNIDTNAQNLQTLSFWVGEADKPPKLDEKDARGFGV RTCISAWKWFMEDLLKKQEEDPLLKLKLSIM |
| SEQ ID NO: 124 | PKKPKFQKRTGFPQPDNLRKEYCLAIVRAANLDADFEKKCTKCEGIKTNKKGNIVKGR TYNSADKDNLLCYACNISTGAPAVDYVFVGALEAKYKILQMVKAYDFHSLAYNLAK LWKGRGRGHQRMGGLNEVVIVSNNEKALDVIEKSLNHFHDEIRGELSRLKAKFQNEH LHVHKESKLRRKLRKISRLLKRRRWKWDVIPNSYLRNFTFTKTRPDFISVALLHRVGR DIGLVTKTKIPKPTDLLPQFGFQIYYTWDEPKLNKLKKSRLRSEPKRLLVPYKKIELYK NKSVLEEAIRHLAEVYTEDLTICFKDFFETQKRKFVSKEKESLKRELLKELTKLKKDFS ERKTALKRDRKEIKEPKKAKLLMEESRSLGFLAANTSYALFNLIAADLYTKSKKACST KLPRQLSTILPLEIKEHKSTTSLAIKPEEGFKIRFSNTHLSIRTPKFKMKGADIKALTKRK REILKNATKLEKSWYGLKHYKLKLYGKEVAAKPRFLDKRNPSIDRRDPKELMEQIENR RNEVKDLEYEIRKGQHQMAKRLDNVDTNAQNLQTKSFWVGEADKPPELDSMEAKKL GLRTCISAWKWFMKDLVLLQEKSPNLKLKLSLTEM |
| SEQ ID NO: 125 | KFSKRQEGFLIPDNIDLYKCLAIVRSANLDADVQGHKSCYGVKKNGTYRVKQNGKKG VKEKGRKYVFDLIAFKGNIEKIPHEAIEEKDQGRVIVLGKFNYKLILNIEKNHNDRASLE IKNKIKKLVQISSLETGEFLSDLLSGKIGIDEVYGIIEPDVFSGKELVCKACQQSTYAPLV EYMPVGELDAKYKILSAIKGYDFLSLAYNLSRNRANKKRGHQKLGGGELSEVVISAN YDKALNVIKRSINHYHVEIKPEISKLKKKMQNEPLKVMKQARIRRELHQLSRKVKRLK WKWGMIPNPELQNIIFEKKEKDFVSYALLHTLGRDIGLFKDTSMLQVPNISDYGFQIYY SWEDPKLNSIKKIKDLPKRLLIPYKRLDFYIDTILVAKVIKNLIELYRKSYVYETFGEEY GYAKKAEDILFDWDSINLSEGIEQKIQKIKDEFSDLLYEARESKRQNFVESFENILGLYD KNFASDRNSYQEKIQSMIIKKQQENIEQKLKREFKEVIERGFEGMDQNKKYYKVLSPNI KGGLLYTDTNNLGFFRSHLAFMLLSKISDDLYRKNNLVSKGGNKGILDQTPETMLTLE FGKSNLPNISIKRKFFNIKYNSSWIGIRKPKFSIKGAVIREITKKVRDEQRLIKSLEGVWH KSTHFKRWGKPRFNLPRHPDREKNNDDNLMESITSRREQIQLLLREKQKQQEKMAGR LDKIDKEIQNLQTANFQIKQIDKKPALTEKSEGKQSVRNALSAWKWFMEDLIKYQKRT PILQLKLAKM |
| SEQ ID NO: 126 | KFSKRQEGFVIPENIGLYKCLAIVRSANLDADVQGHVSCYGVKKNGTYVLKQNGKKSI REKGRKYASDLVAFKGDIEKIPFEVIEEKKKEQSIVLGKFNYKLVLDVMKGEKDRASL TMKNKSKKLVQVSSLGTDEFLLTLLNEKFGIEEIYGIIEPEVFSGKKLVCKACQQSTYAP LVEYMPVGELDSKYKILSAIKGYDFLSLAYNLARHRSNKKRGHQKLGGGELSEVVISA NNAKALNVIKRSLNHYYSEIKPEISKLRKKMQNEPLKVGKQARMRRELHQLSRKVKR LKWKWGKIPNLELQNITFKESDRDFISYALLHTLGRDIGMFNKTEIKMPSNILGYGFQI YYDWEEPKLNTIKKSKNTPKRILIPYKKLDFYNDSILVARAIKELVGLFQESYEWEIFGN EYNYAKEAEVELIKLDEESINGNVEKKLQRIKENFSNLLEKAREKKRQNFIESFESIARL YDESFTADRNEYQREIQSFIIEKQKQSIEKKLKNEFKKIVEKKFNEQEQGKKHYRVLNP TIINEFLPKDKNNLGFLRSKIAFILLSKISDDLYKKSNAVSKGGEKGIIKQQPETILDLEFS KSKLPSINIKKKLFNIKYTSSWLGIRKPKFNIKGAKIREITRRVRDVQRTLKSAESSWYA STHFRRWGFPRFNQPRHPDKEKKSDDRLIESITLLREQIQILLREKQKGQKEMAGRLDD VDKKIQNLQTANFQIKQTGDKPALTEKSAGKQSFRNALSAWKWFMENLLKYQNKTP DLKLKIARTVM |
| SEQ ID NO: 127 | KWIEPNNIDFNKCLAITRSANLDADVQGHKMCYGIKTNGTYKAIGKINKKHNTGIIEKR RTYVYDLIVTKEKNEKIVKKTDFMAIDEEIEFDEKKEKLLKKYIKAEVLGTGELIRKDL NDGEKFDDLCSIEEPQAFRRSELVCKACNQSTYASDIRYIPIGEIEAKYKILKAIKGYDFL SLKYNLGRLRDSKKRGHQKMGQGELKEFVICANKEKALDVIKRSLNHYLNEVKDEIS RLNKKMQNEPLKVNDQARWRRELNQISRRLKRLKWKWGEIPNPELKNLIFKSSRPEFV SYALIHTLGRDIGLINETELKPNNIQEYGFQIYYKWEDPELNHIKKVKNIPKRFIIPYKNL DLFGKYTILSRAIEGILKLYSSSFQYKSFKDPNLFAKEGEKKITNEDFELGYDEKIKKIKD DFKSYKKALLEKKKNTLEDSLNSILSVYEQSLLTEQINNVKKWKEGLLKSKESIHKQK KIENIEDIISRIEELKNVEGWIRTKERDIVNKEETNLKREIKKELKDSYYEEVRKDFSDLK KGEESEKKPFREEPKPIVIKDYIKFDVLPGENSALGFFLSHLSFNLFDSIQYELFEKSRLSS SKHPQIPETILDL |
| SEQ ID NO: 128 | FRKFVKRSGAPQPDNLNKYKCIAIVRAANLDADIMSNESSNCVMCKGIKMNKRKTAK GAAKTTELGRVYAGQSGNLLCTACTKSTMGPLVDYVPIGRIRAKYTILRAVKEYDFLS LAYNLARTRVSKKGGRQKMHSLSELVIAAEYEIAWNIIKSSVIHYHQETKEEISGLRKK LQAEHIHKNKEARIRREMHQISRRIKRLKWKWHMIPNSELHNFLFKQQDPSFVAVALL HTLGRDIGMINKPKGSAKREFIPEYGFQIYYKWMNPKLNDINKQKYRKMPKRSLIPYK NLNVFGDRELIENAMHKLLKLYDENLEVKGSKFFKTRVVAISSKESEKLKRDLLWKGE LAKIKKDFNADKNKMQELFKEVKEPKKANALMKQSRNMGFLLQNISYGALGLLANR MYEASAKQSKGDATKQPSIVIPLEMEFGNAFPKLLLRSGKFAMNVSSPWLTIRKPKFVI KGNKIKNITKLMKDEKAKLKRLETSYHRATHFRPTLRGSIDWDSPYFSSPKQPNTHRRS PDRLSADITEYRGRLKSVEAELREGQRAMAKKLDSVDMTASNLQTSNFQLEKGEDPR LTEIDEKGRSIRNCISSWKKFMEDLMKAQEANPVIKIKIALKDESSVLSEDSM |
| SEQ ID NO: 129 | KFHPENLNKSYCLAIVRAANLDADIQGHINCIGIKSNKSDRNYENKLESLQNVELLCKA CTKSTYKPNINSVPVGEKKAKYSILSEIKKYDFNSLVYNLKKYRKGKSRGHQKLNELR ELVITSEYKKALDVINKSVNHYLVNIKNKMSKLKKILQNEHIHVGTLARIRRERNRISR KLDHYRKKWKFVPNKILKNYVFKNQSPDFVSVALLHKLGRDIGLITKTAILQKSFPEYS LQLYYKYDTPKLNYLKKSKFKSLPKRILISYKYPKFDINSNYIEESIDKLLKLYEESPIYK NNSKIIEFFKKSEDNLIKSENDSLKRGIMKEFEKVTKNFSSKKKKLKEELKLKNEDKNS KMLAKVSRPIGFLKAYLSYMLFNIISNRIFEFSRKSSGRIPQLPSCIINLGNQFENFKNEL QDSNIGSKKNYKYFCNLLLKSSGFNISYEEEHLSIKTPNFFINGRKLKEITSEKKKIRKEN EQLIKQWKKLTFFKPSNLNGKKTSDKIRFKSPNNPDIERKSEDNIVENIAKVKYKLEDL LSEQRKEFNKLAKKHDGVDVEAQCLQTKSFWIDSNSPIKKSLEKKNEKVSVKKKMKA IRSCISAWKWFMADLIEAQKETPMIKLKLALM |
| SEQ ID NO: 130 | TTLVPSHLAGIEVMDETTSRNEDMIQKETSRSNEDENYLGVKNKCGINVHKSGRGSSK HEPNMPPEKSGEGQMPKQDSTEMQQRFDESVTGETQVSAGATASIKTDARANSGPRV GTARALIVKASNLDRDIKLGCKPCEYIRSELPMGKKNGCNHCEKSSDIASVPKVESGFR KAKYELVRRFESFAADSISRHLGKEQARTRGKRGKKDKKEQMGKVNLDEIAILKNESL IEYTENQILDARSNRIKEWLRSLRLRLRTRNKGLKKSKSIRRQLITLRRDYRKWIKPNPY RPDEDPNENSLRLHTKLGVDIGVQGGDNKRMNSDDYETSFSITWRDTATRKICFTKPK GLLPRHMKFKLRGYPELILYNEELRIQDSQKFPLVDWERIPIFKLRGVSLGKKKVKALN RITEAPRLVVAKRIQVNIESKKKKVLTRYVYNDKSINGRLVKAEDSNKDPLLEFKKQA EEINSDAKYYENQEIAKNYLWGCEGLHKNLLEEQTKNPYLAFKYGFLNIV |
| SEQ ID NO: 131 | LDFKRTCSQELVLLPEIEGLKLSGTQGVTSLAKKLINKAANVDRDESYGCHHCIHTRTS LSKPVKKDCNSCNQSTNHPAVPITLKGYKIAFYELWHRFTSWAVDSISKALHRNKVM GKVNLDEYAVVDNSHIVCYAVRKCYEKRQRSVRLHKRAYRCRAKHYNKSQPKVGRI YKKSKRRNARNLKKEAKRYFQPNEITNGSSDALFYKIGVDLGIAKGTPETEVKVDVSI CFQVYYGDARRVLRVRKMDELQSFHLDYTGKLKLKGIGNKDTFTIAKRNESLKWGST KYEVSRAHKKFKPFGKKGSVKRKCNDYFRSIASWSCEAASQRAQSNLKNAFPYQKAL VKCYKNLDYKGVKKNDMWYRLCSNRIFRYSRIAEDIAQYQSDKGKAKFEFVILAQSV AEYDISAIM |
| SEQ ID NO: 132 | VFLTDDKRKTALRKIRSAFRKTAEIALVRAQEADSLDRQAKKLTIETVSFGAPGAKNA FIGSLQGYNWNSHRANVPSSGSAKDVFRITELGLGIPQSAHEASIGKSFELVGNVVRYT ANLLSKGYKKGAVNKGAKQQREIKGKEQLSFDLISNGPISGDKLINGQKDALAWWLI DKMGFHIGLAMEPLSSPNTYGITLQAFWKRHTAPRRYSRGVIRQWQLPFGRQLAPLIH NFFRKKGASIPIVLTNASKKLAGKGVLLEQTALVDPKKWWQVKEQVTGPLSNIWERS VPLVLYTATFTHKHGAAHKRPLTLKVIRISSGSVFLLPLSKVTPGKLVRAWMPDINILR DGRPDEAAYKGPDLIRARERSFPLAYTCVTQIADEWQKRALESNRDSITPLEAKLVTGS DLLQIHSTVQQAVEQGIGGRISSPIQELLAKDALQLVLQQLFMTVDLLRIQWQLKQEV ADGNTSEKAVGWAIRISNIHKDAYKTAIEPCTSALKQAWNPLSGFEERTFQLDASIVRK RSTAKTPDDELVIVLRQQAAEMTVAVTQSVSKELMELAVRHSATLHLLVGEVASKQL SRSADKDRGAMDHWKLLSQSM |
| SEQ ID NO: 133 | EDLLQKALNTATNVAAIERHSCISCLFTESEIDVKYKTPDKIGQNTAGCQSCTFRVGYS GNSHTLPMGNRIALDKLRETIQRYAWHSLLFNVPPAPTSKRVRAISELRVAAGRERLFT VITFVQTNILSKLQKRYAANWTPKSQERLSRLREEGQHILSLLESGSWQQKEVVREDQ DLIVCSALTKPGLSIGAFCRPKYLKPAKHALVLRLIFVEQWPGQIWGQSKRTRRMRRR KDVERVYDISVQAWALKGKETRISECIDTMRRHQQAYIGVLPFLILSGSTVRGKGDCPI LKEITRMRYCPNNEGLIPLGIFYRGSANKLLRVVKGSSFTLPMWQNIETLPHPEPFSPEG WTATGALYEKNLAYWSALNEAVDWYTGQILSSGLQYPNQNEFLARLQNVIDSIPRKW FRPQGLKNLKPNGQEDIVPNEFVIPQNAIRAHHVIEWYHKTNDLVAKTLLGWGSQTTL NQTRPQGDLRFTYTRYYFREKEVPEV |
| SEQ ID NO: 134 | VPKKKLMRELAKKAVFEAIFNDPIPGSFGCKRCTLIDGARVTDAIEKKQGAKRCAGCE PCTFHTLYDSVKHALPAATGCDRTAIDTGLWEILTALRSYNWMSFRRNAVSDASQKQ VWSIEELAIWADKERALRVILSALTHTIGKLKNGFSRDGVWKGGKQLYENLAQKDLA KGLFANGEIFGKELVEADHDMLAWTIVPNHQFHIGLIRGNWKPAAVEASTAFDARWL TNGAPLRDTRTHGHRGRRFNRTEKLTVLCIKRDGGVSEEFRQERDYELSVMLLQPKN KLKPEPKGELNSFEDLHDHWWFLKGDEATALVGLTSDPTVGDFIQLGLYIRNPIKAHG ETKRRLLICFEPPIKLPLRRAFPSEAFKTWEPTINVFRNGRRDTEAYYDIDRARVFEFPET RVSLEHLSKQWEVLRLEPDRENTDPYEAQQNEGAELQVYSLLQEAAQKMAPKVVIDP FGQFPLELFSTFVAQLFNAPLSDTKAKIGKPLDSGFVVESHLHLLEEDFAYRDFVRVTF MGTEPTFRVIHYSNGEGYWKKTVLKGKNNIRTALIPEGAKAAVDAYKNKRCPLTLEA AILNEEKDRRLVLGNKALSLLAQTARGNLTILEALAAEVLRPLSGTEGVVHLHACVTR HSTLTESTETDNM |
| SEQ ID NO: 135 | VEKLFSERLKRAMWLKNEAGRAPPAETLTLKHKRVSGGHEKVKEELQRVLRSLSGTN QAAWNLGLSGGREPKSSDALKGEKSRVVLETVVFHSGHNRVLYDVIEREDQVHQRSS IMHMRRKGSNLLRLWGRSGKVRRKMREEVAEIKPVWHKDSRWLAIVEEGRQSVVGIS SAGLAVFAVQESQCTTAEPKPLEYVVSIWFRGSKALNPQDRYLEFKKLKTTEALRGQQ YDPIPFSLKRGAGCSLAIRGEGIKFGSRGPIKQFFGSDRSRPSHADYDGKRRLSLFSKYA GDLADLTEEQWNRTVSAFAEDEVRRATLANIQDFLSISHEKYAERLKKRIESIEEPVSA SKLEAYLSAIFETFVQQREALASNFLMRLVESVALLISLEEKSPRVEFRVARYLAESKE GFNRKAM |
| SEQ ID NO: 136 | VVITQSELYKERLLRVMEIKNDRGRKEPRESQGLVLRFTQVTGGQEKVKQKLWLIFEG FSGTNQASWNFGQPAGGRKPNSGDALKGPKSRVTYETVVFHFGLRLLSAVIERHNLK QQRQTMAYMKRRAAARKKWARSGKKCSRMRNEVEKIKPKWHKDPRWFDIVKEGEP SIVGISSAGFAIYIVEEPNFPRQDPLEIEYAISIWFRRDRSQYLTFKKIQKAEKLKELQYNP IPFRLKQEKTSLVFESGDIKFGSRGSIEHFRDEARGKPPKADMDNNRRLTMFSVFSGNL TNLTEEQYARPVSGLLAPDEKRMPTLLKKLQDFFTPIHEKYGERIKQRLANSEASKRPF KKLEEYLPAIYLEFRARREGLASNWVLVLINSVRTLVRIKSEDPYIEFKVSQYLLEKED NKAL |
| SEQ ID NO: 137 | KQDALFEERLKKAIFIKRQADPLQREELSLLPPNRKIVTGGHESAKDTLKQILRAINGTN QASWNPGTPSGKRDSKSADALAGPKSRVKLETVVFHVGHRLLKKVVEYQGHQKQQH GLKAFMRTCAAMRKKWKRSGKVVGELREQLANIQPKWHYDSRPLNLCFEGKPSVVG LRSAGIALYTIQKSVVPVKEPKPIEYAVSIWFRGPKAMDREDRCLEFKKLKIATELRKL QFEPIVSTLTQGIKGFSLYIQGNSVKFGSRGPIKYFSNESVRQRPPKADPDGNKRLALFS KFSGDLSDLTEEQWNRPILAFEGIIRRATLGNIQDYLTVGHEQFAISLEQLLSEKESVLQ MSIEQQRLKKNLGKKAENEWVESFGAEQARKKAQGIREYISGFFQEYCSQREQWAEN WVQQLNKSVRLFLTIQDSTPFIEFRVARYLPKGEKKKGKAM |
| SEQ ID NO: 138 | ANHAERHKRLRKEANRAANRNRPLVADCDTGDPLVGICRLLRRGDKMQPNKTGCRS CEQVEPELRDAILVSGPGRLDNYKYELFQRGRAMAVHRLLKRVPKLNRPKKAAGNDE KKAENKKSEIQKEKQKQRRMMPAVSMKQVSVADFKHVIENTVRHLFGDRRDREIAEC AALRAASKYFLKSRRVRPRKLPKLANPDHGKELKGLRLREKRAKLKKEKEKQAELAR SNQKGAVLHVATLKKDAPPMPYEKTQGRNDYTTFVISAAIKVGATRGTKPLLTPQPRE WQCSLYWRDGQRWIRGGLLGLQAGIVLGPKLNRELLEAVLQRPIECRMSGCGNPLQV RGAAVDFFMTTNPFYVSGAAYAQKKFKPFGTKRASEDGAAAKAREKLMTQLAKVLD KVVTQAAHSPLDGIWETRPEAKLRAMIMALEHEWIFLRPGPCHNAAEEVIKCDCTGG HAILWALIDEARGALEHKEFYAVTRAHTHDCEKQKLGGRLAGFLDLLIAQDVPLDDA PAARKIKTLLEATPPAPCYKAATSIATCDCEGKFDKLWAIIDATRAGHGTEDLWARTL AYPQNVNCKCKAGKDLTHRLADFLGLLIKRDGPFRERPPHKVTGDRKLVFSGDKKCK GHQYVILAKAHNEEVVRAWISRWGLKSRTNKAGYAATELNLLLNWLSICRRRWMDM LTVQRDTPYIRMKTGRLVVDDKKERKAM |
| SEQ ID NO: 139 | AKQREALRVALERGIVRASNRTYTLVTNCTKGGPLPEQCRMIERGKARAMKWEPKLV GCGSCAAATVDLPAIEEYAQPGRLDVAKYKLTTQILAMATRRMMVRAAKLSRRKGQ WPAKVQEEKEEPPEPKKMLKAVEMRPVAIVDFNRVIQTTIEHLWAERANADEAELKA LKAAAAYFGPSLKIRARGPPKAAIGRELKKAHRKKAYAERKKARRKRAELARSQARG AAAHAAIRERDIPPMAYERTQGRNDVTTIPIAAAIKIAATRGARPLPAPKPMKWQCSLY WNEGQRWIRGGMLTAQAYAHAANIHRPMRCEMWGVGNPLKVRAFEGRVADPDGA KGRKAEFRLQTNAFYVSGAAYRNKKFKPFGTDRGGIGSARKKRERLMAQLAKILDKV VSQAAHSPLDDIWHTRPAQKLRAMIKQLEHEWMFLRPQAPTVEGTKPDVDVAGNMQ RQIKALMAPDLPPIEKGSPAKRFTGDKRKKGERAVRVAEAHSDEVVTAWISRWGIQTR RNEGSYAAQELELLLNWLQICRRRWLDMTAAQRVSPYIRMKSGRMITDAADEGVAPI PLVENM |
| SEQ ID NO: 140 | KSISGRSIKHMACLKDMLKSEITEIEEKQKKESLRKWDYYSKFSDEILFRRNLNVSANH DANACYGCNPCAFLKEVYGFRIERRNNERIISYRRGLAGCKSCVQSTGYPPIEFVRRKF GADKAMEIVREVLHRRNWGALARNIGREKEADPILGELNELLLVDARPYFGNKSAAN ETNLAFNVITRAAKKFRDEGMYDIHKQLDIHSEEGKVPKGRKSRLIRIERKHKAIHGLD PGETWRYPHCGKGEKYGVWLNRSRLIHIKGNEYRCLTAFGTTGRRMSLDVACSVLGH PLVKKKRKKGKKTVDGTELWQIKKATETLPEDPIDCTFYLYAAKPTKDPFILKVGSLK APRWKKLHKDFFEYSDTEKTQGQEKGKRVVRRGKVPRILSLRPDAKFKVSIWDDPYN GKNKEGTLLRMELSGLDGAKKPLILKRYGEPNTKPKNFVFWRPHITPHPLTFTPKHDF GDPNKKTKRRRVFNREYYGHLNDLAKMEPNAKFFEDREVSNKKNPKAKNIRIQAKES LPNIVAKNGRWAAFDPNDSLWKLYLHWRGRRKTIKGGISQEFQEFKERLDLYKKHED ESEWKEKEKLWENHEKEWKKTLEIHGSIAEVSQRCVMQSMMGPLDGLVQKKDYVHI GQSSLKAADDAWTFSANRYKKATGPKWGKISVSNLLYDANQANAELISQSISKYLSK QKDNQGCEGRKMKFLIKIIEPLRENFVKHTRWLHEMTQKDCEVRAQFSRVSM |
| SEQ ID NO: 141 | FPSDVGADALKHVRMLQPRLTDEVRKVALTRAPSDRPALARFAAVAQDGLAFVRHL NVSANHDSNCTFPRDPRDPRRGPCEPNPCAFLREVWGFRIVARGNERALSYRRGLAGC KSCVQSTGFPSVPFHRIGADDCMRKLHEILKARNWRLLARNIGREREADPLLTELSEYL LVDARTYPDGAAPNSGRLAENVIKRAAKKFRDEGMRDIHAQLRVHSREGKVPKGRLQ RLRRIERKHRAIHALDPGPSWEAEGSARAEVQGVAVYRSQLLRVGHHTQQIEPVGIVA RTLFGVGRTDLDVAVSVLGAPLTKRKKGSKTLESTEDFRIAKARETRAEDKIEVAFVL YPTASLLRDEIPKDAFPAMRIDRFLLKVGSVQADREILLQDDYYRFGDAEVKAGKNKG RTVTRPVKVPRLQALRPDAKFRVNVWADPFGAGDSPGTLLRLEVSGVTRRSQPLRLLR YGQPSTQPANFLCWRPHRVPDPMTFTPRQKFGERRKNRRTRRPRVFERLYQVHIKHLA HLEPNRKWFEEARVSAQKWAKARAIRRKGAEDIPVVAPPAKRRWAALQPNAELWDL YAHDREARKRFRGGRAAEGEEFKPRLNLYLAHEPEAEWESKRDRWERYEKKWTAVL EEHSRMCAVADRTLPQFLSDPLGARMDDKDYAFVGKSALAVAEAFVEEGTVERAQG NCSITAKKKFASNASRKRLSVANLLDVSDKADRALVFQAVRQYVQRQAENGGVEGR RMAFLRKLLAPLRQNFVCHTRWLHM |
| SEQ ID NO: 142 | AARKKKRGKIGITVKAKEKSPPAAGPFMARKLVNVAANVDGVEVHLCVECEADAHG SASARLLGGCRSCTGSIGAEGRLMGSVDVDRERVIAEPVHTETERLGPDVKAFEAGTA ESKYAIQRGLEYWGVDLISRNRARTVRKMEEADRPESSTMEKTSWDEIAIKTYSQAYH ASENHLFWERQRRVRQHALALFRRARERNRGESPLQSTQRPAPLVLAALHAEAAAISG RARAEYVLRGPSANVRAAAADIDAKPLGHYKTPSPKVARGFPVKRDLLRARHRIVGL SRAYFKPSDVVRGTSDAIAHVAGRNIGVAGGKPKEIEKTFTLPFVAYWEDVDRVVHCS SFKADGPWVRDQRIKIRGVSSAVGTFSLYGLDVAWSKPTSFYIRCSDIRKKFHPKGFGP MKHWRQWAKELDRLTEQRASCVVRALQDDEELLQTMERGQRYYDVFSCAATHATR GEADPSGGCSRCELVSCGVAHKVTKKAKGDTGIEAVAVAGCSLCESKLVGPSKPRVH RQMAALRQSHALNYLRRLQREWEALEAVQAPTPYLRFKYARHLEVRSM |
| SEQ ID NO: 143 | AAKKKKQRGKIGISVKPKEGSAPPADGPFMARKLVNVAANVDGVEVNLCIECEADAH GSAPARLLGGCKSCTGSIGAEGRLMGSVDVDRADAIAKPVNTETEKLGPDVQAFEAG TAETKYALQRGLEYWGVDLISRNRSRTVRRTEEGQPESATMEKTSWDEIAIKSYTRAY HASENHLFWERQRRVRQHALALFKRAKERNRGDSTLPREPGHGLVAIAALACEAYAV GGRNLAETVVRGPTFGTARAVRDVEIASLGRYKTPSPKVAHGSPVKRDFLRARHRIVG LARAYYRPSDVVRGTSDAIAHVAGRNIGVAGGKPRAVEAVFTLPFVAYWEDVDRVV HCSSFQVSAPWNRDQRMKIAGVTTAAGTFSLHGGELKWAKPTSFYIRCSDTRRKFRPK GFGPMKRWRQWAKDLDRLVEQRASCVVRALQDDAALLETMERGQRYYDVFACAVT HATRGEADRLAGCSRCALTPCQEAHRVTTKPRGDAGVEQVQTSDCSLCEGKLVGPSK PRLHRTLTLLRQEHGLNYLRRLQREWESLEAVQVPTPYLRFKYARHLEVRSM |
| SEQ ID NO: 144 | TDSQSESVPEVVYALTGGEVPGRVPPDGGSAEGARNAPTGLRKQRGKIKISAKPSKPGS PASSLARTLVNEAANVDGVQSSGCATCRMRANGSAPRALPIGCVACASSIGRAPQEET VCALPTTQGPDVRLLEGGHALRKYDIQRALEYWGVDLIGRNLDRQAGRGMEPAEGA TATMKRVSMDELAVLDFGKSYYASEQHLFAARQRRVRQHAKALKIRAKHANRSGSV KRALDRSRKQVTALAREFFKPSDVVRGDSDALAHVVGRNLGVSRHPAREIPQTFTLPL CAYWEDVDRVISCSSLLAGEPFARDQEIRIEGVSSALGSLRLYRGAIEWHKPTSLYIRCS DTRRKFRPRGGLKKRWRQWAKDLDRLVEQRACCIVRSLQADVELLQTMERAQRFYD VHDCAATHVGPVAVRCSPCAGKQFDWDRYRLLAALRQEHALNYLRRLQREWESLEA QQVKMPYLRFKYARKLEVSGPLIGLEVRREPSMGTAIAEM |
| SEQ ID NO: 145 | AGTAGRRHGSLGARRSINIAGVTDRHGRWGCESCVYTRDQAGNRARCAPCDQSTYAP DVQEVTIGQRQAKYTIFLTLQSFSWTNTMRNNKRAAAGRSKRTTGKRIGQLAEIKITG VGLAHAHNVIQRSLQHNITKMWRAEKGKSKRVARLKKAKQLTKRRAYFRRRMSRQS RGNGFFRTGKGGIHAVAPVKIGLDVGMIASGSSEPADEQTVTLDAIWKGRKKKIRLIG AKGELAVAACRFREQQTKGDKCIPLILQDGEVRWNQNNWQCHPKKLVPLCGLEVSR KFVSQADRLAQNKVASPLAARFDKTSVKGTLVESDFAAVLVNVTSIYQQCHAMLLRS QEPTPSLRVQRTITSM |
| SEQ ID NO: 146 | GVRFSPAQSQVFFRTVIPQSVEARFAINMAAIHDAAGAFGCSVCRFEDRTPRNAKAVH GCSPCTRSTNRPDVFVLPVGAIKAKYDVFMRLLGFNWTHLNRRQAKRVTVRDRIGQL DELAISMLTGKAKAVLKKSICHNVDKSFKAMRGSLKKLHRKASKTGKSQLRAKLSDL RERTNTTQEGSHVEGDSDVALNKIGLDVGLVGKPDYPSEESVEVVVCLYFVGKVLILD AQGRIRDMRAKQYDGFKIPIIQRGQLTVLSVKDLGKWSLVRQDYVLAGDLRFEPKISK DRKYAECVKRIALITLQASLGFKERIPYYVTKQVEIKNASHIAFVTEAIQNCAENFREM TEYLMKYQEKSPDLKVLLTQLM |
| SEQ ID NO: 147 | RAVVGKVFLEQARRALNLATNFGTNHRTGCNGCYVTPGKLSIPQDGEKNAAGCTSCL MKATASYVSYPKPLGEKVAKYSTLDALKGFPWYSLRLNLRPNYRGKPINGVQEVAPV SKFRLAEEVIQAVQRYHFTELEQSFPGGRRRLRELRAFYTKEYRRAPEQRQHVVNGDR NIVVVTVLHELGFSVGMFNEVELLPKTPIECAVNVFIRGNRVLLEVRKPQFDKERLLVE SLWKKDSRRHTAKWTPPNNEGRIFTAEGWKDFQLPLLLGSTSRSLRAIEKEGFVQLAP GRDPDYNNTIDEQHSGRPFLPLYLYLQGTISQEYCVFAGTWVIPFQDGISPYSTKDTFQP DLKRKAYSLLLDAVKHRLGNKVASGLQYGRFPAIEELKRLVRMHGATRKIPRGEKDL LKKGDPDTPEWWLLEQYPEFWRLCDAAAKRVSQNVGLLLSLKKQPLWQRRWLESRT RNEPLDNLPLSMALTLHLTNEEAL |
| SEQ ID NO: 148 | AAVYSKFYIENHFKMGIPETLSRIRGPSIIQGFSVNENYINIAGVGDRDFIFGCKKCKYTR GKPSSKKINKCHPCKRSTYPEPVIDVRGSISEFKYKIYNKLKQEPNQSIKQNTKGRMNPS DHTSSNDGIIINGIDNRIAYNVIFSSYKHLMEKQINLLRDTTKRKARQIKKYNNSGKKK HSLRSQTKGNLKNRYHMLGMFKKGSLTITNEGDFITAVRKVGLDISLYKNESLNKQEV ETELCLNIKWGRTKSYTVSGYIPLPINIDWKLYLFEKETGLTLRLFGNKYKIQSKKFLIA QLFKPKRPPCADPVVKKAQKWSALNAHVQQMAGLFSDSHLLKRELKNRMHKQLDFK SLWVGTEDYIKWFEELSRSYVEGAEKSLEFFRQDYFCFNYTKQTTM |
| SEQ ID NO: 149 | PQQQRDLMLMAANYDQDYGNGCGPCTVVASAAYRPDPQAQHGCKRHLRTLGASAV THVGLGDRTATITALHRLRGPAALAARARAAQAASAPMTPDTDAPDDRRRLEAIDAD DVVLVGAHRALWSAVRRWADDRRAALRRRLHSEREWLLKDQIRWAELYTLIEASGT PPQGRWRNTLGALRGQSRWRRVLAPTMRATCAETHAELWDALAELVPEMAKDRRG LLRPPVEADALWRAPMIVEGWRGGHSVVVDAVAPPLDLPQPCAWTAVRLSGDPRQR WGLHLAVPPLGQVQPPDPLKATLAVSMRHRGGVRVRTLQAMAVDADAPMQRHLQV PLTLQRGGGLQWGIHSRGVRRREARSMASWEGPPIWTGLQLVNRWKGQGSALLAPD RPPDTPPYAPDAAVAPAQPDTKRARRTLKEACTVCRCAPGHMRQLQVTLTGDGTWR RFRLRAPQGAKRKAEVLKVATQHDERIANYTAWYLKRPEHAAGCDTCDGDSRLDGA CRGCRPLLVGDQCFRRYLDKIEADRDDGLAQIKPKAQEAVAAMAAKRDARAQKVAA RAAKLSEATGQRTAATRDASHEARAQKELEAVATEGTTVRHDAAAVSAFGSWVARK GDEYRHQVGVLANRLEHGLRLQELMAPDSVVADQQRASGHARVGYRYVLTAM |
| SEQ ID NO: 150 | AVAHPVGRGNAGSPGARGPEELPRQLVNRASNVTRPATYGCAPCRHVRLSIPKPVLTG CRACEQTTHPAPKRAVRGGADAAKYDLAAFFAGWAADLEGRNRRRQVHAPLDPQPD PNHEPAVTLQKIDLAEVSIEEFQRVLARSVKHRHDGRASREREKARAYAQVAKKRRN SHAHGARTRRAVRRQTRAVRRAHRMGANSGEILVASGAEDPVPEAIDHAAQLRRRIR ACARDLEGLRHLSRRYLKTLEKPCRRPRAPDLGRARCHALVESLQAAERELEELRRCD SPDTAMRRLDAVLAAAASTDATFATGWTVVGMDLGVAPRGSAAPEVSPMEMAISVF WRKGSRRVIVSKPIAGMPIRRHELIRLEGLGTLRLDGNHYTGAGVTKGRGLSEGTEPDF REKSPSTLGFTLSDYRHESRWRPYGAKQGKTARQFFAAMSRELRALVEHQVLAPMGP PLLEAHERRFETLLKGQDNKSIHAGGGGRYVWRGPPDSKKRPAADGDWFRFGRGHA DHRGWANKRHELAANYLQSAFRLWSTLAEAQEPTPYARYKYTRVTM |
| SEQ ID NO: 151 | WDFLTLQVYERHTSPEVCVAGNSTKCASGTRKSDHTHGVGVKLGAQEINVSANDDR DHEVGCNICVISRVSLDIKGWRYGCESCVQSTPEWRSIVRFDRNHKEAKGECLSRFEY WGAQSIARSLKRNKLMGGVNLDELAIVQNENVVKTSLKHLFDKRKDRIQANLKAVK VRMRERRKSGRQRKALRRQCRKLKRYLRSYDPSDIKEGNSCSAFTKLGLDIGISPNKPP KIEPKVEVVFSLFYQGACDKIVTVSSPESPLPRSWKIKIDGIRALYVKSTKVKFGGRTFR AGQRNNRRKVRPPNVKKGKRKGSRSQFFNKFAVGLDAVSQQLPIASVQGLWGRAET KKAQTICLKQLESNKPLKESQRCLFLADNWVVRVCGFLRALSQRQGPTPYIRYRYRCN M |
| SEQ ID NO: 152 | ARNVGQRNASRQSKRESAKARSRRVTGGHASVTQGVALINAAANADRDHTTGCEPC TWERVNLPLQEVIHGCDSCTKSSPFWRDIKVVNKGYREAKEEIMRIASGISADHLSRAL SHNKVMGRLNLDEVCILDFRTVLDTSLKHLTDSRSNGIKEHIRAVHRKIRMRRKSGKT ARALRKQYFALRRQWKAGHKPNSIREGNSLTALRAVGFDVGVSEGTEPMPAPQTEVV LSVFYKGSATRILRISSPHPIAKRSWKVKIAGIKALKLIRREHDFSFGRETYNASQRAEK RKFSPHAARKDFFNSFAVQLDRLAQQLCVSSVENLWVTEPQQKLLTLAKDTAPYGIRE GARFADTRARLAWNWVFRVCGFTRALHQEQEPTPYCRFTWRSKM |

In some embodiments, the Type V CRISPR/Cas enzyme is a CasΦ nuclease. A CasΦ polypeptide can function as an endonuclease that catalyzes cleavage at a specific sequence in a target nucleic acid. A programmable CasΦ nuclease of the present disclosure may have a single active site in a RuvC domain that is capable of catalyzing pre-crRNA processing and nicking or cleaving of nucleic acids. This compact catalytic site may render the programmable CasΦ nuclease especially advantageous for genome engineering and new functionalities for genome manipulation.

**TABLE** 3 provides amino acid sequences of illustrative CasΦ polypeptides that can be used in compositions and methods of the disclosure.

**TABLE 3 - CasΦ Amino Acid Sequences**

| **Name** | **SEQ ID NO** | **Amino Acid Sequence** |
|---|---|---|
| CasΦ.1 | SEQ ID NO: 221 | MADTPTLFTQFLRHHLPGQRFRKDILKQAGRILANKGEDATIA FLRGKSEESPPDFQPPVKCPIIACSRPLTEWPIYQASVAIQGYV YGQSLAEFEASDPGCSKDGLLGWFDKTGVCTDYFSVQGLNLI FQNARKRYIGVQTKVTNRNEKRHKKLKRINAKRIAEGLPELT SDEPESALDETGHLIDPPGLNTNIYCYQQVSPKPLALSEVNQLP TAYAGYSTSGDDPIQPMVTKDRLSISKGQPGYIPEHQRALLSQ KKHRRMRGYGLKARALLVIVRIQDDWAVIDLRSLLRNAYWR RIVQTKEPSTITKLLKLVTGDPVLDATRMVATFTYKPGIVQVR SAKCLKNKQGSKLFSERYLNETVSVTSIDLGSNNLVAVATYR LVNGNTPELLQRFTLPSHLVKDFERYKQAHDTLEDSIQKTAV ASLPQGQQTEIRMWSMYGFREAQERVCQELGLADGSIPWNV MTATSTILTDLFLARGGDPKKCMFTSEPKKKKNSKQVLYKIR DRAWAKMYRTLLSKETREAWNKALWGLKRGSPDYARLSKR KEELARRCVNYTISTAEKRAQCGRTIVALEDLNIGFFHGRGKQ EPGWVGLFTRKKENRWLMQALHKAFLELAHHRGYHVIEVNP AYTSQTCPVCRHCDPDNRDQHNREAFHCIGCGFRGNADLDV ATHNIAMVAITGESLKRARGSVASKTPQPLAAE |
| CasΦ.2 | SEQ ID NO: 222 | MPKPAVESEFSKVLKKHFPGERFRSSYMKRGGKILAAQGEEA VVAYLQGKSEEEPPNFQPPAKCHVVTKSRDFAEWPIMKASEA IQRYIYALSTTERAACKPGKSSESHAAWFAATGVSNHGYSHV QGLNLIFDHTLGRYDGVLKKVQLRNEKARARLESINASRADE GLPEIKAEEEEVATNETGHLLQPPGINPSFYVYQTISPQAYRPR DEIVLPPEYAGYVRDPNAPIPLGVVRNRCDIQKGCPGYIPEWQ REAGTAISPKTGKAVTVPGLSPKKNKRMRRYWRSEKEKAQD ALLVTVRIGTDWVVIDVRGLLRNARWRTIAPKDISLNALLDLF TGDPVIDVRRNIVTFTYTLDACGTYARKWTLKGKQTKATLD KLTATQTVALVAIDLGQTNPISAGISRVTQENGALQCEPLDRF TLPDDLLKDISAYRIAWDRNEEELRARSVEALPEAQQAEVRA LDGVSKETARTQLCADFGLDPKRLPWDKMSSNTTFISEALLS NSVSRDQVFFTPAPKKGAKKKAPVEVMRKDRTWARAYKPRL SVEAQKLKNEALWALKRTSPEYLKLSRRKEELCRRSINYVIEK TRRRTQCQIVIPVIEDLNVRFFHGSGKRLPGWDNFFTAKKENR WFIQGLHKAFSDLRTHRSFYVFEVRPERTSITCPKCGHCEVGN RDGEAFQCLSCGKTCNADLDVATHNLTQVALTGKTMPKREE PRDAQGTAPARKTKKASKSKAPPAEREDQTPAQEPSQTS |
| CasΦ.3 | SEQ ID NO: 223 | MYILEMADLKSEPSLLAKLLRDRFPGKYWLPKYWKLAEKKR LTGGEEAACEYMADKQLDSPPPNFRPPARCVILAKSRPFEDW PVHRVASKAQSFVIGLSEQGFAALRAAPPSTADARRDWLRSH GASEDDLMALEAQLLETIMGNAISLHGGVLKKIDNANVKAA KRLSGRNEARLNKGLQELPPEQEGSAYGADGLLVNPPGLNLN IYCRKSCCPKPVKNTARFVGHYPGYLRDSDSILISGTMDRLTII EGMPGHIPAWQREQGLVKPGGRRRRLSGSESNMRQKVDPST GPRRSTRSGTVNRSNQRTGRNGDPLLVEIRMKEDWVLLDAR GLLRNLRWRESKRGLSCDHEDLSLSGLLALFSGDPVIDPVRNE VVFLYGEGIIPVRSTKPVGTRQSKKLLERQASMGPLTLISCDL GQTNLIAGRASAISLTHGSLGVRSSVRIELDPEIIKSFERLRKDA DRLETEILTAAKETLSDEQRGEVNSHEKDSPQTAKASLCRELG LHPPSLPWGQMGPSTTFIADMLISHGRDDDAFLSHGEFPTLEK RKKFDKRFCLESRPLLSSETRKALNESLWEVKRTSSEYARLSQ RKKEMARRAVNFVVEISRRKTGLSNVIVNIEDLNVRIFHGGG KQAPGWDGFFRPKSENRWFIQAIHKAFSDLAAHHGIPVIESDP QRTSMTCPECGHCDSKNRNGVRFLCKGCGASMDADFDAACR NLERVALTGKPMPKPSTSCERLLSATTGKVCSDHSLSHDAIEK AS |
| CasΦ.4 | SEQ ID NO: 224 | MEKEITELTKIRREFPNKKFSSTDMKKAGKLLKAEGPDAVRD FLNSCQEIIGDFKPPVKTNIVSISRPFEEWPVSMVGRAIQEYYFS LTKEELESVHPGTSSEDHKSFFNITGLSNYNYTSVQGLNLIFKN AKAIYDGTLVKANNKNKKLEKKFNEINHKRSLEGLPIITPDFE EPFDENGHLNNPPGINRNIYGYQGCAAKVFVPSKHKMVSLPK EYEGYNRDPNLSLAGFRNRLEIPEGEPGHVPWFQRMDIPEGQI GHVNKIQRFNFVHGKNSGKVKFSDKTGRVKRYHHSKYKDAT KPYKFLEESKKVSALDSILAIITIGDDWVVFDIRGLYRNVFYRE LAQKGLTAVQLLDLFTGDPVIDPKKGVVTFSYKEGVVPVFSQ KIVPRFKSRDTLEKLTSQGPVALLSVDLGQNEPVAARVCSLK NINDKITLDNSCRISFLDDYKKQIKDYRDSLDELEIKIRLEAINS LETNQQVEIRDLDVFSADRAKANTVDMFDIDPNLISWDSMSD ARVSTQISDLYLKNGGDESRVYFEINNKRIKRSDYNISQLVRP KLSDSTRKNLNDSIWKLKRTSEEYLKLSKRKLELSRAVVNYTI RQSKLLSGINDIVIILEDLDVKKKFNGRGIRDIGWDNFFSSRKE NRWFIPAFHKAFSELSSNRGLCVIEVNPAWTSATCPDCGFCSK ENRDGINFTCRKCGVSYHADIDVATLNIARVAVLGKPMSGPA DRERLGDTKKPRVARSRKTMKRKDISNSTVEAMVTA |
| CasΦ.5 | SEQ ID NO: 225 | MDMLDTETNYATETPAQQQDYSPKPPKKAQRAPKGFSKKAR PEKKPPKPITLFTQKHFSGVRFLKRVIRDASKILKLSESRTITFL EQAIERDGSAPPDVTPPVHNTIMAVTRPFEEWPEVILSKALQK HCYALTKKIKIKTWPKKGPGKKCLAAWSARTKIPLIPGQVQA TNGLFDRIGSIYDGVEKKVTNRNANKKLEYDEAIKEGRNPAV PEYETAYNIDGTLINKPGYNPNLYITQSRTPRLITEADRPLVEKI LWQMVEKKTQSRNQARRARLEKAAHLQGLPVPKFVPEKVD RSQKIEIRIIDPLDKIEPYMPQDRMAIKASQDGHVPYWQRPFLS KRRNRRVRAGWGKQVSSIQAWLTGALLVIVRLGNEAFLADIR GALRNAQWRKLLKPDATYQSLFNLFTGDPVVNTRTNHLTMA YREGVVNIVKSRSFKGRQTREHLLTLLGQGKTVAGVSFDLGQ KHAAGLLAAHFGLGEDGNPVFTPIQACFLPQRYLDSLTNYRN RYDALTLDMRRQSLLALTPAQQEFADAQRDPGGQAKRACC LKLNLNPDEIRWDLVSGISTMISDLYIERGGDPRDVHQQVETK PKGKRKSEIRILKIRDGKWAYDFRPKIADETRKAQREQLWKL QKASSEFERLSRYKINIARAIANWALQWGRELSGCDIVIPVLE DLNVGSKFFDGKGKWLLGWDNRFTPKKENRWFIKVLHKAV AELAPHRGVPVYEVMPHRTSMTCPACHYCHPTNREGDRFEC QSCHVVKNTDRDVAPYNILRVAVEGKTLDRWQAEKKPQAEP DRPMILIDNQES |
| CasΦ.6 | SEQ ID NO: 226 | MDMLDTETNYATETPAQQQDYSPKPPKKAQRAPKGFSKKAR PEKKPPKPITLFTQKHFSGVRFLKRVIRDASKILKLSESRTITFL EQAIERDGSAPPDVTPPVHNTIMAVTRPFEEWPEVILSKALQK HCYALTKKIKIKTWPKKGPGKKCLAAWSARTKIPLIPGQVQA TNGLFDRIGSIYDGVEKKVTNRNANKKLEYDEAIKEGRNPAV PEYETAYNIDGTLINKPGYNPNLYITQSRTPRLITEADRPLVEKI LWQMVEKKTQSRNQARRARLEKAAHLQGLPVPKFVPEKVD RSQKIEIRIIDPLDKIEPYMPQDRMAIKASQDGHVPYWQRPFLS KRRNRRVRAGWGKQVSSIQAWLTGALLVIVRLGNEAFLADIR GALRNAQWRKLLKPDATYQSLFNLFTGDPVVNTRTNHLTMA YREGVVDIVKSRSFKGRQTREHLLTLLGQGKTVAGVSFDLGQ KHAAGLLAAHFGLGEDGNPVFTPIQACFLPQRYLDSLTNYRN RYDALTLDMRRQSLLALTPAQQQEFADAQRDPGGQAKRACC LKLNLNPDEIRWDLVSGISTMISDLYIERGGDPRDVHQQVETK PKGKRKSEIRILKIRDGKWAYDFRPKIADETRKAQREQLWKL QKASSEFERLSRYKINIARAIANWALQWGRELSGCDIVIPVLE DLNVGSKFFDGKGKWLLGWDNRFTPKKENRWFIKVLHKAV AELAPHKGVPVYEVMPHRTSMTCPACHYCHPTNREGDRFEC QSCHVVKNTDRDVAPYNILRVAVEGKTLDRWQAEKKPQAEP DRPMILIDNQES |
| CasΦ.7 | SEQ ID NO: 227 | MSSLPTPLELLKQKHADLFKGLQFSSKDNKMAGKVLKKDGE EAALAFLSERGVSRGELPNFRPPAKTLVVAQSRPFEEFPIYRVS EAIQLYVYSLSVKELETVPSGSSTKKEHQRFFQDSSVPDFGYT SVQGLNKIFGLARGIYLGVITRGENQLQKAKSKHEALNKKRR ASGEAETEFDPTPYEYMTPERKLAKPPGVNHSIMCYVDISVDE FDFRNPDGIVLPSEYAGYCREINTAIEKGTVDRLGHLKGGPGY IPGHQRKESTTEGPKINFRKGRIRRSYTALYAKRDSRRVRQGK LALPSYRHHMMRLNSNAESAILAVIFFGKDWVVFDLRGLLRN VRWRNLFVDGSTPSTLLGMFGDPVIDPKRGVVAFCYKEQIVP VVSKSITKMVKAPELLNKLYLKSEDPLVLVAIDLGQTNPVGV GVYRVMNASLDYEVVTRFALESELLREIESYRQRTNAFEAQIR AETFDAMTSEEQEEITRVRAFSASKAKENVCHRFGMPVDAVD WATMGSNTIHIAKWVMRHGDPSLVEVLEYRKDNEIKLDKNG VPKKVKLTDKRIANLTSIRLRFSQETSKHYNDTMWELRRKHP VYQKLSKSKADFSRRVVNSIIRRVNHLVPRARIVFIIEDLKNLG KVFHGSGKRELGWDSYFEPKSENRWFIQVLHKAFSETGKHK GYYIIECWPNWTSCTCPKCSCCDSENRHGEVFRCLACGYTCN TDFGTAPDNLVKIATTGKGLPGPKKRCKGSSKGKNPKIARSSE TGVSVTESGAPKVKKSSPTQTSQSSSQSAP |
| CasΦ.8 | SEQ ID NO: 228 | MNKIEKEKTPLAKLMNENFAGLRFPFAIIKQAGKKLLKEGEL KTIEYMTGKGSIEPLPNFKPPVKCLIVAKRRDLKYFPICKASCE IQSYVYSLNYKDFMDYFSTPMTSQKQHEEFFKKSGLNIEYQN VAGLNLIFNNVKNTYNGVILKVKNRNEKLKKKAIKNNYEFEE IKTFNDDGCLINKPGINNVIYCFQSISPKILKNITHLPKEYNDYD CSVDRNIIQKYVSRLDIPESQPGHVPEWQRKLPEFNNTNNPRR RRKWYSNGRNISKGYSVDQVNQAKIEDSLLAQIKIGEDWIILD IRGLLRDLNRRELISYKNKLTIKDVLGFFSDYPIIDIKKNLVTFC YKEGVIQVVSQKSIGNKKSKQLLEKLIENKPIALVSIDLGQTNP VSVKISKLNKINNKISIESFTYRFLNEEILKEIEKYRKDYDKLEL KLINEA |
| CasΦ.9 | SEQ ID NO: 229 | MDMLDTETNYATETPSQQQDYSPKPPKKDRRAPKGFSKKAR PEKKPPKPITLFTQKHFSGVRFLKRVIRDASKILKLSESRTITFL EQAIERDGSAPPDVTPPVHNTIMAVTRPFEEWPEVILSKALQK HCYALTKKIKIKTWPKKGPGKKCLAAWSARTKIPLIPGQVQA TNGLFDRIGSIYDGVEKKVTNRNANKKLEYDEAIKEGRNPAV PEYETAYNIDGTLINKPGYNPNLYITQSRTPRLITEADRPLVEKI LWQMVEKKTQSRNQARRARLEKAAHLQGLPVPKFVPEKVD RSQKIEIRIIDPLDKIEPYMPQDRMAIKASQDGHVPYWQRPFLS KRRNRRVRAGWGKQVSSIQAWLTGALLVIVRLGNEAFLADIR GALRNAQWRKLLKPDATYQSLFNLFTGDPVVNTRTNHLTMA YREGVVDIVKSRSFKGRQTREHLLTLLGQGKTVAGVSFDLGQ KHAAGLLAAHFGLGEDGNPVFTPIQACFLPQRYLDSLTNYRN RYDALTLDMRRQSLLALTPAQQQEFADAQRDPGGQAKRACC LKLNLNPDEIRWDLVSGISTMISDLYIERGGDPRDVHQQVETK PKGKRKSEIRILKIRDGKWAYDFRPKIADETRKAQREQLWKL QKASSEFERLSRYKINIARAIANWALQWGRELSGCDIVIPVLE DLNVGSKFFDGKGKWLLGWDNRFTPKKENRWFIKVLHKAV AELAPHRGVPVYEVMPHRTSMTCPACHYCHPTNREGDRFEC QSCHVVKNTDRDVAPYNILRVAVEGKTLDRWQAEKKPQAEP DRPMILIDNQES |
| CasΦ.10 | SEQ ID NO: 230 | MDMLDTETNYATETPSQQQDYSPKPPKKDRRAPKGFSKKAR PEKKPPKPITLFTQKHFSGVRFLKRVIRDASKILKLSESRTITFL EQAIERDGSAPPDVTPPVHNTIMAVTRPFEEWPEVILSKALQK HCYALTKKIKIKTWPKKGPGKKCLAAWSARTKIPLIPGQVQA TNGLFDRIGSIYDGVEKKVTNRNANKKLEYDEAIKEGRNPAV PEYETAYNIDGTLINKPGYNPNLYITQSRTPRLITEADRPLVEKI LWQMVEKKTQSRNQARRARLEKAAHLQGLPVPKFVPEKVD RSQKIEIRIIDPLDKIEPYMPQDRMAIKASQDGHVPYWQRPFLS KRRNRRVRAGWGKQVSSIQAWLTGALLVIVRLGNEAFLADIR GALRNAQWRKLLKPDATYQSLFNLFTGDPVVNTRTNHLTMA YREGVVNIVKSRSFKGRQTREHLLTLLGQGKTVAGVSFDLGQ KHAAGLLAAHFGLGEDGNPVFTPIQACFLPQRYLDSLTNYRN RYDALTLDMRRQSLLALTPAQQQEFADAQRDPGGQAKRACC LKLNLNPDEIRWDLVSGISTMISDLYIERGGDPRDVHQQVETK PKGKRKSEIRILKIRDGKWAYDFRPKIADETRKAQREQLWKL QKASSEFERLSRYKINIARAIANWALQWGRELSGCDIVIPVLE DLNVGSKFFDGKGKWLLGWDNRFTPKKENRWFIKVLHKAV AELAPHRGVPVYEVMPHRTSMTCPACHYCHPTNREGDRFEC QSCHVVKNTDRDVAPYNILRVAVEGKTLDRWQAEKKPQAEP DRPMILIDNQES |
| CasΦ.11 | SEQ ID NO: 231 | MSNKTTPPSPLSLLLRAHFPGLKFESQDYKIAGKKLRDGGPEA VISYLTGKGQAKLKDVKPPAKAFVIAQSRPFIEWDLVRVSRQI QEKIFGIPATKGRPKQDGLSETAFNEAVASLEVDGKSKLNEET RAAFYEVLGLDAPSLHAQAQNALIKSAISIREGVLKKVENRNE KNLSKTKRRKEAGEEATFVEEKAHDERGYLIHPPGVNQTIPG YQAVVIKSCPSDFIGLPSGCLAKESAEALTDYLPHDRMTIPKG QPGYVPEWQHPLLNRRKNRRRRDWYSASLNKPKATCSKRSG TPNRKNSRTDQIQSGRFKGAIPVLMRFQDEWVIIDIRGLLRNA RYRKLLKEKSTIPDLLSLFTGDPSIDMRQGVCTFIYKAGQACS AKMVKTKNAPEILSELTKSGPVVLVSIDLGQTNPIAAKVSRVT QLSDGQLSHETLLRELLSNDSSDGKEIARYRVASDRLRDKLA NLAVERLSPEHKSEILRAKNDTPALCKARVCAALGLNPEMIA WDKMTPYTEFLATAYLEKGGDRKVATLKPKNRPEMLRRDIK FKGTEGVRIEVSPEAAEAYREAQWDLQRTSPEYLRLSTWKQE LTKRILNQLRHKAAKSSQCEVVVMAFEDLNIKMMHGNGKW ADGGWDAFFIKKRENRWFMQAFHKSLTELGAHKGVPTIEVT PHRTSITCTKCGHCDKANRDGERFACQKCGFVAHADLEIATD NIERVALTGKPMPKPESERSGDAKKSVGARKAAFKPEEDAEA AE |
| CasΦ.12 | SEQ ID NO: 232 | MIKPTVSQFLTPGFKLIRNHSRTAGLKLKNEGEEACKKFVREN EIPKDECPNFQGGPAIANIIAKSREFTEWEIYQSSLAIQEVIFTLP KDKLPEPILKEEWRAQWLSEHGLDTVPYKEAAGLNLIIKNAV NTYKGVQVKVDNKNKNNLAKINRKNEIAKLNGEQEISFEEIK AFDDKGYLLQKPSPNKSIYCYQSVSPKPFITSKYHNVNLPEEYI GYYRKSNEPIVSPYQFDRLRIPIGEPGYVPKWQYTFLSKKENK RRKLSKRIKNVSPILGIICIKKDWCVFDMRGLLRTNHWKKYH KPTDSINDLFDYFTGDPVIDTKANVVRFRYKMENGIVNYKPV REKKGKELLENICDQNGSCKLATVDVGQNNPVAIGLFELKKV NGELTKTLISRHPTPIDFCNKITAYRERYDKLESSIKLDAIKQLT SEQKIEVDNYNNNFTPQNTKQIVCSKLNINPNDLPWDKMISGT HFISEKAQVSNKSEIYFTSTDKGKTKDVMKSDYKWFQDYKPK LSKEVRDALSDIEWRLRRESLEFNKLSKSREQDARQLANWISS MCDVIGIENLVKKNNFFGGSGKREPGWDNFYKPKKENRWWI NAIHKALTELSQNKGKRVILLPAMRTSITCPKCKYCDSKNRN GEKFNCLKCGIELNADIDVATENLATVAITAQSMPKPTCERSG DAKKPVRARKAKAPEFHDKLAPSYTVVLREAV |
| CasΦ.13 | SEQ ID NO: 233 | MRQPAEKTAFQVFRQEVIGTQKLSGGDAKTAGRLYKQGKME AAREWLLKGARDDVPPNFQPPAKCLVVAVSHPFEEWDISKTN HDVQAYIYAQPLQAEGHLNGLSEKWEDTSADQHKLWFEKTG VPDRGLPVQAINKIAKAAVNRAFGVVRKVENRNEKRRSRDN RIAEHNRENGLTEVVREAPEVATNADGFLLHPPGIDPSILSYAS VSPVPYNSSKHSFVRLPEEYQAYNVEPDAPIPQFVVEDRFAIPP GQPGYVPEWQRLKCSTNKHRRMRQWSNQDYKPKAGRRAKP LEFQAHLTRERAKGALLVVMRIKEDWVVFDVRGLLRNVEWR KVLSEEAREKLTLKGLLDLFTGDPVIDTKRGIVTFLYKAEITKI LSKRTVKTKNARDLLLRLTEPGEDGLRREVGLVAVDLGQTHP IAAAIYRIGRTSAGALESTVLHRQGLREDQKEKLKEYRKRHT ALDSRLRKEAFETLSVEQQKEIVTVSGSGAQITKDKVCNYLG VDPSTLPWEKMGSYTHFISDDFLRRGGDPNIVHFDRQPKKGK VSKKSQRIKRSDSQWVGRMRPRLSQETAKARMEADWAAQN ENEEYKRLARSKQELARWCVNTLLQNTRCITQCDEIVVVIED LNVKSLHGKGAREPGWDNFFTPKTENRWFIQILHKTFSELPK HRGEHVIEGCPLRTSITCPACSYCDKNSRNGEKFVCVACGATF HADFEVATYNLVRLATTGMPMPKSLERQGGGEKAGGARKA RKKAKQVEKIVVQANANVTMNGASLHSP |
| CasΦ.14 | SEQ ID NO: 234 | MSSLPTPLELLKQKHADLFKGLQFSSKDNKMAGKVLKKDGE EAALAFLSERGVSRGELPNFRPPAKTLVVAQSRPFEEFPIYRVS EAIQLYVVYSLSVKELETVPSGSSTKKEHQRFFQDSSVPDFGYT SVQGLNKIFGLARGIYLGVITRGENQLQKAKSKHEALNKKRR ASGEAETEFDPTPYEYMTPERKLAKPPGVNHSIMCYVDISVDE FDFRNPDGIVLPSEYAGYCREINTAIEKGTVDRLGHLKGGPGY IPGHQRKESTTEGPKINFRKGRIRRSYTALYAKRDSRRVRQGK LALPSYRHHMMRLNSNAESAILAVIFFGKDWVVFDLRGLLRN VRWRNLFVDGSTPSTLLGMFGDPVIDPKRGVVAFCYKEQIVP VVSKSITKMVKAPELLNKLYLKSEDPLVLVAIDLGQTNPVGV GVYRVMNASLDYEVVTRFALESELLREIESYRQRTNAFEAQIR AETFDAMTSEEQEEITRVRAFSASKAKENVCHRFGMPVDAVD WATMGSNTIHIAKWVMRHGDPSLVEVLEYRKDNEIKLDKNG VPKKVKLTDKRIANLTSIRLRFSQETSKHYNDTMWELRRKHP VYQKLSKSKADFSRRVVNSIIRRVNHLVPRARIVFIIEDLKNLG KVFHGSGKRELGWDSYFEPKSENRWFIQVLHKAFSETGKHK GYYIIECWPNWTSCTCPKCSCCDSENRHGEVFRCLACGYTCN TDFGTAPDNLVKIATTGKGLPGPKKRCKGSSKGKNPKIARSSE TGVSVTESGAPKVKKSSPTQTSQSSSQSAP |
| CasΦ.15 | SEQ ID NO: 235 | MIKPTVSQFLTPGFKLIRNHSRTAGLKLKNEGEEACKKFVREN EIPKDECPNFQGGPAIANIIAKSREFTEWEIYQSSLAIQEVIFTLP KDKLPEPILKEEWRAQWLSEHGLDTVPYKEAAGLNLIIKNAV NTYKGVQVKVDNKNKNNLAKINRKNEIAKLNGEQEISFEEIK AFDDKGYLLQKPSPNKSIYCYQSVSPKPFITSKYHNVNLPEEYI GYYRKSNEPIVSPYQFDRLRIPIGEPGYVPKWQYTFLSKKENK RRKLSKRIKNVSPILGIICIKKDWCVFDMRGLLRTNHWKKYH KPTDSINDLFDYFTGDPVIDTKANVVRFRYKMENGIVNYKPV REKKGKELLENICDQNGSCKLATVDVGQNNPVAIGLFELKKV NGELTKTLISRHPTPIDFCNKITAYRERYDKLESSIKLDAIKQLT SEQKIEVDNYNNNFTPQNTKQIVCSKLNINPNDLPWDKMISGT HFISEKAQVSNKSEIYFTSTDKGKTKDVMKSDYKWFQDYKPK LSKEVRDALSDIEWRLRRESLEFNKLSKSREQDARQLANWISS MCDVIGIENLVKKNNFFGGSGKREPGWDNFYKPKKENRWWI NAIHKALTELSQNKGKRVILLPAMRTSITCPKCKYCDSKNRN GEKFNCLKCGIELNADIDVATENLATVAITAQSMPKPTCERSG DAKKPVRARKAKAPEFHDKLAPSYTVVLREAV |
| CasΦ.16 | SEQ ID NO: 236 | MSNKTTPPSPLSLLLRAHFPGLKFESQDYKIAGKKLRDGGPEA VISYLTGKGQAKLKDVKPPAKAFVIAQSRPFIEWDLVRVSRQI QEKIFGIPATKGRPKQDGLSETAFNEAVASLEVDGKSKLNEET RAAFYEVLGLDAPSLHAQAQNALIKSAISIREGVLKKVENRNE KNLSKTKRRKEAGEEATFVEEKAHDERGYLIHPPGVNQTIPG YQAVVIKSCPSDFIGLPSGCLAKESAEALTDYLPHDRMTIPKG QPGYVPEWQHPLLNRRKNRRRRDWYSASLNKPKATCSKRSG TPNRKNSRTDQIQSGRFKGAIPVLMRFQDEWVIIDIRGLLRNA RYRKLLKEKSTIPDLLSLFTGDPSIDMRQGVCTFIYKAGQACS AKMVKTKNAPEILSELTKSGPVVLVSIDLGQTNPIAAKVSRVT QLSDGQLSHETLLRELLSNDSSDGKEIARYRVASDRLRDKLA NLAVERLSPEHKSEILRAKNDTPALCKARVCAALGLNPEMIA WDKMTPYTEFLATAYLEKGGDRKVATLKPKNRPEMLRRDIK FKGTEGVRIEVSPEAAEAYREAQWDLQRTSPEYLRLSTWKQE LTKRILNQLRHKAAKSSQCEVVVMAFEDLNIKMMHGNGKW ADGGWDAFFIKKRENRWFMQAFHKSLTELGAHKGVPTIEVT PHRTSITCTKCGHCDKANRDGERFACQKCGFVAHADLEIATD NIERVALTGKPMPKPESERSGDAKKSVGARKAAFKPEEDAEA AE |
| CasΦ.17 | SEQ ID NO: 237 | MYSLEMADLKSEPSLLAKLLRDRFPGKYWLPKYWKLAEKKR LTGGEEAACEYMADKQLDSPPPNFRPPARCVILAKSRPFEDW PVHRVASKAQSFVIGLSEQGFAALRAAPPSTADARRDWLRSH GASEDDLMALEAQLLETIMGNAISLHGGVLKKIDNANVKAA KRLSGRNEARLNKGLQELPPEQEGSAYGADGLLVNPPGLNLN IYCRKSCCPKPVKNTARFVGHYPGYLRDSDSILISGTMDRLTII EGMPGHIPAWQREQGLVKPGGRRRRLSGSESNMRQKVDPST GPRRSTRSGTVNRSNQRTGRNGDPLLVEIRMKEDWVLLDAR GLLRNLRWRESKRGLSCDHEDLSLSGLLALFSGDPVIDPVRNE VVFLYGEGIIPVRSTKPVGTRQSKKLLERQASMGPLTLISCDL GQTNLIAGRASAISLTHGSLGVRSSVRIELDPEIIKSFERLRKDA DRLETEILTAAKETLSDEQRGEVNSHEKDSPQTAKASLCRELG LHPPSLPWGQMGPSTTFIADMLISHGRDDDAFLSHGEFPTLEK RKKFDKRFCLESRPLLSSETRKALNESLWEVKRTSSEYARLSQ RKKEMARRAVNFVVEISRRKTGLSNVIVNIEDLNVRIFHGGG KQAPGWDGFFRPKSENRWFIQAIHKAFSDLAAHHGIPVIESDP QRTSMTCPECGHCDSKNRNGVRFLCKGCGASMDADFDAACR NLERVALTGKPMPKPSTSCERLLSATTGKVCSDHSLSHDAIEK AS |
| CasΦ.18 | SEQ ID NO: 238 | MEKEITELTKIRREFPNKKFSSTDMKKAGKLLKAEGPDAVRD FLNSCQEIIGDFKPPVKTNIVSISRPFEEWPVSMVGRAIQEYYFS LTKEELESVHPGTSSEDHKSFFNITGLSNYNYTSVQGLNLIFKN AKAIYDGTLVKANNKNKKLEKKFNEINHKRSLEGLPIITPDFE EPFDENGHLNNPPGINRNIYGYQGCAAKVFVPSKHKMVSLPK EYEGYNRDPNLSLAGFRNRLEIPEGEPGHVPWFQRMDIPEGQI GHVNKIQRFNFVHGKNSGKVKFSDKTGRVKRYHHSKYKDAT KPYKFLEESKKVSALDSILAIITIGDDWVVFDIRGLYRNVFYRE LAQKGLTAVQLLDLFTGDPVIDPKKGVVTFSYKEGVVPVFSQ KIVPRFKSRDTLEKLTSQGPVALLSVDLGQNEPVAARVCSLK NINDKITLDNSCRISFLDDYKKQIKDYRDSLDELEIKIRLEAINS LETNQQVEIRDLDVFSADRAKANTVDMFDIDPNLISWDSMSD ARVSTQISDLYLKNGGDESRVYFEINNKRIKRSDYNISQLVRP KLSDSTRKNLNDSIWKLKRTSEEYLKLSKRKLELSRAVVNYTI RQSKLLSGINDIVIILEDLDVKKKFNGRGIRDIGWDNFFSSRKE NRWFIPAFHKTFSELSSNRGLCVIEVNPAWTSATCPDCGFCSK ENRDGINFTCRKCGVSYHADIDVATLNIARVAVLGKPMSGPA DRERLGDTKKPRVARSRKTMKRKDISNSTVEAMVTA |
| CasΦ.19 | SEQ ID NO: 239 | MLVRTSTLVQDNKNSRSASRAFLKKPKMPKNKHIKEPTELAK LIRELFPGQRFTRAINTQAGKILKHKGRDEVVEFLKNKGIDKE QFMDFRPPTKARIVATSGAIEEFSYLRVSMAIQECCFGKYKFP KEKVNGKLVLETVGLTKEELDDFLPKKYYENKKSRDRFFLKT GICDYGYTYAQGLNEIFRNTRAIYEGVFTKVNNRNEKRREKK DKYNEERRSKGLSEEPYDEDESATDESGHLINPPGVNLNIWTC EGFCKGPYVTKLSGTPGYEVILPKVFDGYNRDPNEIISCGITDR FAIPEGEPGHIPWHQRLEIPEGQPGYVPGHQRFADTGQNNSGK ANPNKKGRMRKYYGHGTKYTQPGEYQEVFRKGHREGNKRR YWEEDFRSEAHDCILYVIHIGDDWVVCDLRGPLRDAYRRGLV PKEGITTQELCNLFSGDPVIDPKHGVVTFCYKNGLVRAQKTIS AGKKSRELLGALTSQGPIALIGVDLGQTEPVGARAFIVNQARG SLSLPTLKGSFLLTAENSSSWNVFKGEIKAYREAIDDLAIRLKK EAVATLSVEQQTEIESYEAFSAEDAKQLACEKFGVDSSFILWE DMTPYHTGPATYYFAKQFLKKNGGNKSLIEYIPYQKKKSKKT PKAVLRSDYNIACCVRPKLLPETRKALNEAIRIVQKNSDEYQR LSKRKLEFCRRVVNYLVRKAKKLTGLERVIIAIEDLKSLEKFF TGSGKRDNGWSNFFRPKKENRWFIPAFHKAFSELAPNRGFYV IECNPARTSITDPDCGYCDGDNRDGIKFECKKCGAKHHTDLD VAPLNIAIVAVTGRPMPKTVSNKSKRERSGGEKSVGASRKRN HRKSKANQEMLDATSSAAE |
| CasΦ.20 | SEQ ID NO: 240 | MPKIKKPTEISLLRKEVFPDLHFAKDRMRAASLVLKNEGREA AIEYLRVNHEDKPPNFMPPAKTPYVALSRPLEQWPIAQASIAI QKYIFGLTKDEFSATKKLLYGDKSTPNTESRKRWFEVTGVPN FGYMSAQGLNAIFSGALARYEGVVQKVENRNKKRFEKLSEK NQLLIEEGQPVKDYVPDTAYHTPETLQKLAENNHVRVEDLGD MIDRLVHPPGIHRSIYGYQQVPPFAYDPDNPKGIILPKAYAGY TRKPHDIIEAMPNRLNIPEGQAGYIPEHQRDKLKKGGRVKRLR TTRVRVDATETVRAKAEALNAEKARLRGKEAILAVFQIEEDW ALIDMRGLLRNVYMRKLIAAGELTPTTLLGYFTETLTLDPRRT EATFCYHLRSEGALHAEYVRHGKNTRELLLDLTKDNEKIALV TIDLGQRNPLAAAIFRVGRDASGDLTENSLEPVSRMLLPQAYL DQIKAYRDAYDSFRQNIWDTALASLTPEQQRQILAYEAYTPD DSKENVLRLLLGGNVMPDDLPWEDMTKNTHYISDRYLADGG DPSKVWFVPGPRKRKKNAPPLKKPPKPRELVKRSDHNISHLSE FRPQLLKETRDAFEKAKIDTERGHVGYQKLSTRKDQLCKEIL NWLEAEAVRLTRCKTMVLGLEDLNGPFFNQGKGKVRGWVS FFRQKQENRWIVNGFRKNALARAHDKGKYILELWPSWTSQT CPKCKHVHADNRHGDDFVCLQCGARLHADAEVATWNLAVV AIQGHSLPGPVREKSNDRKKSGSARKSKKANESGKVVGAWA AQATPKRATSKKETGTARNPVYNPLETQASCPAP |
| CasΦ.21 | SEQ ID NO: 241 | MTPSPQIARLVETPLAAALKAHHPGKKFRSDYLKKAGKILKD QGVEAAMAHLDGKDQAEPPNFKPPAKCRIVARSREFSEWPIV KASVEIQKYIYGLTLEERKACDPGKSSASHKAWFAKTGVNTF GYSSVQGFNLIFGHTLGRYDGVLVKTENLNKKRAEKNERFRA KALAEGRAEPVCPPLVTATNDTGQDVTLEDGRVVRPGQLLQP PGINPNIYAYQQVSPKAYVPGIIELPEEFQGYSRDPNAVILPLV PRDRLSIPKGQPGYVPEPHREGLTGRKDRRMRRYYETERGTK LKRPPLTAKGRADKANEALLVVVRIDSDWVVMDVRGLLRNA RWRRLVSKEGITLNGLLDLFTGDPVLNPKDCSVSRDTGDPVN DPRHGVVTFCYKLGVVDVCSKDRPIKGFRTKEVLERLTSSGT VGMVSIDLGQTNPVAAAVSRVTKGLQAETLETFTLPDDLLGK VRAYRAKTDRMEEGFRRNALRKLTAEQQAEITRYNDATEQQ AKALVCSTYGIGPEEVPWERMTSNTTYISDHILDHGGDPDTVF FMATKRGQNKPTLHKRKDKAWGQKFRPAISVETRLARQAAE WELRRASLEFQKLSVWKTELCRQAVNYVMERTKKRTQCDVI IPVIEDLPVPLFHGSGKRDPGWANFFVHKRENRWFIDGLHKAF SELGKHRGIYVFEVCPQRTSITCPKCGHCDPDNRDGEKFVCLS CQATLNADLDVATTNLVRVALTGKVMPRSERSGDAQTPGPA RKARTGKIKGSKPTSAPQGATQTDAKAHLSQTGV |
| CasΦ.22 | SEQ ID NO: 242 | MTPSPQIARLVETPLAAALKAHHPGKKFRSDYLKKAGKILKD QGVEAAMAHLDGKDQAEPPNFKPPAKCRIVARSREFSEWPIV KASVEIQKYIYGLTLEERKACDPGKSSASHKAWFAKTGVNTF GYSSVQGFNLIFGHTLGRYDGVLVKTENLNKKRAEKNERFRA KALAEGRAEPVCPPLVTATNDTGQDVTLEDGRVVRPGQLLQP PGINPNIYAYQQVSPKAYVPGIIELPEEFQGYSRDPNAVILPLV PRDRLSIPKGQPGYVPEPHREGLTGRKDRRMRRYYETERGTK LKRPPLTAKGRADKANEALLVVVRIDSDWVVMDVRGLLRNA RWRRLVSKEGITLNGLLDLFTGDPVLNPKDCSVSRDTGDPVN DPRHGVVTFCYKLGVVDVCSKDRPIKGFRTKEVLERLTSSGT VGMVSIDLGQTNPVAAAVSRVTKGLQAETLETFTLPDDLLGK VRAYRAKTDRMEEGFRRNALRKLTAEQQAEITRYNDATEQQ AKALVCSTYGIGPEEVPWERMTSNTTYISDHILDHGGDPDTVF FMATKRGQNKPTLHKRKDKAWGQKFRPAISVETRLARQAAE WELRRASLEFQKLSVWKTELCRQAVNYVMERTKKRTQCDVI IPVIEDLPVPLFHGSGKRDPGWANFFVHKRENRWFIDGLHKAF SELGKHRGIYVFEVCPQRTSITCPKCGHCDPDNRDGEKFVCLS CQATLHADLDVATTNLVRVALTGKVMPRSERSGDAQTPGPA RKARTGKIKGSKPTSAPQGATQTDAKAHLSQTGV |
| CasΦ.23 | SEQ ID NO: 243 | MKTEKPKTALTLLREEVFPGKKYRLDVLKEAGKKLSTKGRE ATIEFLTGKDEERPQNFQPPAKTSIVAQSRPFDQWPIVQVSLA VQKYIYGLTQSEFEANKKALYGETGKAISTESRRAWFEATGV DNFGFTAAQGINPIFSQAVARYEGVIKKVENRNEKKLKKLTK KNLLRLESGEEIEDFEPEATFNEEGRLLQPPGANPNIYCYQQIS PRIYDPSDPKGVILPQIYAGYDRKPEDIISAGVPNRLAIPEGQPG YIPEHQRAGLKTQGRIRCRASVEAKARAAILAVVHLGEDWVV LDLRGLLRNVYWRKLASPGTLTLKGLLDFFTGGPVLDARRGI ATFSYTLKSAAAVHAENTYKGKGTREVLLKLTENNSVALVT VDLGQRNPLAAMIARVSRTSQGDLTYPESVEPLTRLFLPDPFL EEVRKYRSSYDALRLSIREAAIASLTPEQQAEIRYIEKFSAGDA KKNVAEVFGIDPTQLPWDAMTPRTTYISDLFLRMGGDRSRVF FEVPPKKAKKAPKKPPKKPAGPRIVKRTDGMIARLREIRPRLS AETNKAFQEARWEGERSNVAFQKLSVRRKQFARTVVNHLVQ TAQKMSRCDTVVLGIEDLNVPFFHGRGKYQPGWEGFFRQKK ENRWLINDMHKALSERGPHRGGYVLELTPFWTSLRCPKCGH TDSANRDGDDFVCVKCGAKLHSDLEVATANLALVAITGQSIP RPPREQSSGKKSTGTARMKKTSGETQGKGSKACVSEALNKIE QGTARDPVYNPLNSQVSCPAP |
| CasΦ.24 | SEQ ID NO: 244 | VYNPDMKKPNNIRRIREEHFEGLCFGKDVLTKAGKIYEKDGE EAAIDFLMGKDEEDPPNFKPPAKTTIVAQSRPFDQWPIYQVSQ AVQERVFAYTEEEFNASKEALFSGDISSKSRDFWFKTNNISDQ GIGAQGLNTILSHAFSRYSGVIKKVENRNKKRLKKLSKKNQL KIEEGLEILEFKPDSAFNENGLLAQPPGINPNIYGYQAVTPFVF DPDNPGDVILPKQYEGYSRKPDDIIEKGPSRLDIPKGQPGYVPE HQRKNLKKKGRVRLYRRTPPKTKALASILAVLQIGKDWVLFD MRGLLRSVYMREAATPGQISAKDLLDTFTGCPVLNTRTGEFT FCYKLRSEGALHARKIYTKGETRTLLTSLTSENNTIALVTVDL GQRNPAAIMISRLSRKEELSEKDIQPVSRRLLPDRYLNELKRY RDAYDAFRQEVRDEAFTSLCPEHQEQVQQYEALTPEKAKNL VLKHFFGTHDPDLPWDDMTSNTHYIANLYLERGGDPSKVFFT RPLKKDSKSKKPRKPTKRTDASISRLPEIRPKMPEDARKAFEK AKWEIYTGHEKFPKLAKRVNQLCREIANWIEKEAKRLTLCDT VVVGIEDLSLPPKRGKGKFQETWQGFFRQKFENRWVIDTLKK AIQNRAHDKGKYVLGLAPYWTSQRCPACGFIHKSNRNGDHF KCLKCEALFHADSEVATWNLALVAVLGKGITNPDSKKPSGQ KKTGTTRKKQIKGKNKGKETVNVPPTTQEVEDIIAFFEKDDET VRNPVYKPTGT |
| CasΦ.25 | SEQ ID NO: 245 | MKKPNNIRRIREEHFEGLCFGKDVLTKAGKIYEKDGEEAAIDF LMGKDEEDPPNFKPPAKTTIVAQSRPFDQWPIYQVSQAVQER VFAYTEEEFNASKEALFSGDISSKSRDFWFKTNNISDQGIGAQ GLNTILSHAFSRYSGVIKKVENRNKKRLKKLSKKNQLKIEEGL EILEFKPDSAFNENGLLAQPPGINPNIYGYQAVTPFVFDPDNPG DVILPKQYEGYSRKPDDIIEKGPSRLDIPKGQPGYVPEHQRKN LKKKGRVRLYRRTPPKTKALASILAVLQIGKDWVLFDMRGLL RSVYMREAATPGQISAKDLLDTFTGCPVLNTRTGEFTFCYKL RSEGALHARKIYTKGETRTLLTSLTSENNTIALVTVDLGQRNP AAIMISRLSRKEELSEKDIQPVSRRLLPDRYLNELKRYRDAYD AFRQEVRDEAFTSLCPEHQEQVQQYEALTPEKAKNLVLKHFF GTHDPDLPWDDMTSNTHYIANLYLERGGDPSKVFFTRPLKKD SKSKKPRKPTKRTDASISRLPEIRPKMPEDARKAFEKAKWEIY TGHEKFPKLAKRVNQLCREIANWIEKEAKRLTLCDTVVVGIE DLSLPPKRGKGKFQETWQGFFRQKFENRWVIDTLKKAIQNRA HDKGKYVLGLAPYWTSQRCPACGFIHKSNRNGDHFKCLKCE ALFHADSEVATWNLALVAVLGKGITNPDSKKPSGQKKTGTT RKKQIKGKNKGKETVNVPPTTQEVEDIIAFFEKDDETVRNPVY KPTGT |
| CasΦ.26 | SEQ ID NO: 246 | VIKTHFPAGRFRKDHQKTAGKKLKHEGEEACVEYLRNKVSD YPPNFKPPAKGTIVAQSRPFSEWPIVRASEAIQKYVYGLTVAE LDVFSPGTSKPSHAEWFAKTGVENYGYRQVQGLNTIFQNTVN RFKGVLKKVENRNKKSLKRQEGANRRRVEEGLPEVPVTVES ATDDEGRLLQPPGVNPSIYGYQGVAPRVCTDLQGFSGMSVDF AGYRRDPDAVLVESLPEGRLSIPKGERGYVPEWQRDPERNKF PLREGSRRQRKWYSNACHKPKPGRTSKYDPEALKKASAKDA LLVSISIGEDWAIIDVRGLLRDARRRGFTPEEGLSLNSLLGLFT EYPVFDVQRGLITFTYKLGQVDVHSRKTVPTFRSRALLESLVA KEEIALVSVDLGQTNPASMKVSRVRAQEGALVAEPVHRMFLS DVLLGELSSYRKRMDAFEDAIRAQAFETMTPEQQAEITRVCD VSVEVARRRVCEKYSISPQDVPWGEMTGHSTFIVDAVLRKGG DESLVYFKNKEGETLKFRDLRISRMEGVRPRLTKDTRDALNK AVLDLKRAHPTFAKLAKQKLELARRCVNFIEREAKRYTQCER VVFVIEDLNVGFFHGKGKRDRGWDAFFTAKKENRWVIQALH KAFSDLGLHRGSYVIEVTPQRTSMTCPRCGHCDKGNRNGEKF VCLQCGATLHADLEVATDNIERVALTGKAMPKPPVRERSGD VQKAGTARKARKPLKPKQKTEPSVQEGSSDDGVDKSPGDAS RNPVYNPSDTLSI |
| CasΦ.27 | SEQ ID NO: 247 | MAKAKTLAALLRELLPGQHLAPHHRWVANKLLMTSGDAAA FVIGKSVSDPVRGSFRKDVITKAGRIFKKDGPDAAAAFLDGK WEDRPPNFQPPAKAAIVAISRSFDEWPIVKVSCAIQQYLYALP VQEFESSVPEARAQAHAAWFQDTGVDDCNFKSTQGLNAIFN HGKRTYEGVLKKAQNRNDKKNLRLERINAKRAEAGQAPLVA GPDESPTDDAGCLLHPPGINANIYCYQQVSPRPYEQSCGIQLPP EYAGYNRLSNVAIPPMPNRLDIPQGQPGYVPEHHRHGIKKFG RVRKRYGVVPGRNRDADGKRTRQVLTEAGAAAKARDSVLA VIRIGDDWTVVDLRGLLRNAQWRKLVPDGGITVQGLLDLFTG DPVIDPRRGVVTFIYKADSVGIHSEKVCRGKQSKNLLERLCA MPEKSSTRLDCARQAVALVSVDLGQRNPVAARFSRVSLAEG QLQAQLVSAQFLDDAMVAMIRSYREEYDRFESLVREQAKAA LSPEQLSEIVRHEADSAESVKSCVCAKFGIDPAGLSWDKMTSG TWRIADHVQAAGGDVEWFFFKTCGKGKEIKTVRRSDFNVAK QFRLRLSPETRKDWNDAIWELKRGNPAYVSFSKRKSEFARRV VNDLVHRARRAVRCDEVVFAIEDLNISFFHGKGQRQMGWDA FFEVKQENRWFIQALHKAFVERATHKGGYVLEVAPARTSTTC PECRHCDPESRRGEQFCCIKCRHTCHADLEVATFNIEQVALTG VSLPKRLSSTLL |
| CasΦ.28 | SEQ ID NO: 248 | MSKEKTPPSAYAILKAKHFPDLDFEKKHKMMAGRMFKNGAS EQEVVQYLQGKGSESLMDVKPPAKSPILAQSRPFDEWEMVRT SRLIQETIFGIPKRGSIPKRDGLSETQFNELVASLEVGGKPMLN KQTRAIFYGLLGIKPPTFHAMAQNILIDLAINIRKGVLKKVDNL NEKNRKKVKRIRDAGEQDVMVPAEVTAHDDRGYLNHPPGV NPTIPGYQGVVIPFPEGFEGLPSGMTPVDWSHVLVDYLPHDRL SIPKGSPGYIPEWQRPLLNRHKGRRHRSWYANSLNKPRKSRT EEAKDRQNAGKRTALIEAERLKGVLPVLMRFKEDWLIIDARG LLRNARYRGVLPEGSTLGNLIDLFSDSPRVDTRRGICTFLYRK GRAYSTKPVKRKESKETLLKLTEKSTIALVSIDLGQTNPLTAK LSKVRQVDGCLVAEPVLRKLIDNASEDGKEIARYRVAHDLLR ARILEDAIDLLGIYKDEVVRARSDTPDLCKERVCRFLGLDSQA IDWDRMTPYTDFIAQAFVAKGGDPKVVTIKPNGKPKMFRKD RSIKNMKGIRLDISKEASSAYREAQWAIQRESPDFQRLAVWQS QLTKRIVNQLVAWAKKCTQCDTVVLAFEDLNIGMMHGSGK WANGGWNALFLHKQENRWFMQAFHKALTELSAHKGIPTIEV LPHRTSITCTQCGHCHPGNRDGERFKCLKCEFLANTDLEIATD NIERVALTGLPMPKGERSSAKRKPGGTRKTKKSKHSGNSPLA AE |
| CasΦ.29 | SEQ ID NO: 249 | MEKAGPTSPLSVLIHKNFEGCRFQIDHLKIAGRKLAREGEAAA IEYLLDKKCEGLPPNFQPPAKGNVIAQSRPFTEWAPYRASVAI QKYIYSLSVDERKVCDPGSSSDSHEKWFKQTGVQNYGYTHV QGLNLIFKHALARYDGVLKKVDNRNEKNRKKAERVNSFRRE EGLPEEVFEEEKATDETGHLLQPPGVNHSIYCYQSVRPKPFNP RKPGGISLPEAYSGYSLKPQDELPIGSLDRLSIPPGQPGYVPEW QRSQLTTQKHRRKRSWYSAQKWKPRTGRTSTFDPDRLNCAR AQGAILAVVRIHEDWVVFDVRGLLRNALWRELAGKGLTVRD LLDFFTGDPVVDTKRGVVTFTYKLGKVDVHSLRTVRGKRSK KVLEDLTLSSDVGLVTIDLGQTNVLAADYSKVTRSENGELLA VPLSKSFLPKHLLHEVTAYRTSYDQMEEGFRRKALLTLTEDQ QVEVTLVRDFSVESSKTKLLQLGVDVTSLPWEKMSSNTTYIS DQLLQQGADPASLFFDGERDGKPCRHKKKDRTWAYLVRPKV SPETRKALNEALWALKNTSPEFESLSKRKIQFSRRCMNYLLNE AKRISGCGQVVFVIEDLNVRVHHGRGKRAIGWDNFFKPKREN RWFMQALHKAASELAIHRGMHIIEACPARSSITCPKCGHCDPE NRCSSDREKFLCVKCGAAFHADLEVATFNLRKVALTGTALPK SIDHSRDGLIPKGARNRKLKEPQANDEKACA |
| CasΦ.30 | SEQ ID NO: 250 | MKEQSPLSSVLKSNFPGKKFLSADIRVAGRKLAQLGEAAAVE YLSPRQRDSVPNFRPPAFCTVVAKSRPFEEWPIYKASVLLQEQ IYGMTGQEFEERCGSIPTSLSGLRQWASSVGLGAAMEGLHVQ GMNLMVKNAINRYKGVLVKVENRNKKLVEANEAKNSSREE RGLPPLRPPELGSAFGPDGRLVNPPGIDKSIRLYQGVSPVPVVK TTGRPTVHRLDIPAGEKGHVPLWQREAGLVKEGPRRRRMWY SNSNLKRSRKDRSAEASEARKADSVVVRVSVKEDWVDIDVR GLLRNVAWRGIERAGESTEDLLSLFSGDPVVDPSRDSVVFLY KEGVVDVLSKKVVGAGKSRKQLEKMVSEGPVALVSCDLGQT NYVAARVSVLDESLSPVRSFRVDPREFPSADGSQGVVGSLDRI RADSDRLEAKLLSEAEASLPEPVRAEIEFLRSERPSAVAGRLCL KLGIDPRSIPWEKMGSTTSFISEALSAKGSPLALHDGAPIKDSR FAHAARGRLSPESRKALNEALWERKSSSREYGVISRRKSEASR RMANAVLSESRRLTGLAVVAVNLEDLNMVSKFFHGRGKRAP GWAGFFTPKMENRWFIRSIHKAMCDLSKHRGITVIESRPERTS ISCPECGHCDPENRSGERFSCKSCGVSLHADFEVATRNLERVA LTGKPMPRRENLHSPEGATASRKTRKKPREATASTFLDLRSVL SSAENEGSGPAARAG |
| CasΦ.31 | SEQ ID NO: 251 | MLPPSNKIGKSMSLKEFINKRNFKSSIIKQAGKILKKEGEEAVK KYLDDNYVEGYKKRDFPITAKCNIVASNRKIEDFDISKFSSFIQ NYVFNLNKDNFEEFSKIKYNRKSFDELYKKIANEIGLEKPNYE NIQGEIAVIRNAINIYNGVLKKVENRNKKIQEKNQSKDPPKLL SAFDDNGFLAERPGINETIYGYQSVRLRHLDVEKDKDIIVQLP DIYQKYNKKSTDKISVKKRLNKYNVDEYGKLISKRRKERINK DDAILCVSNFGDDWIIFDARGLLRQTYRYKLKKKGLCIKDLL NLFTGDPIINPTKTDLKEALSLSFKDGIINNRTLKVKNYKKCPE LISELIRDKGKVAMISIDLGQTNPISYRLSKFTANNVAYIENGVI SEDDIVKMKKWREKSDKLENLIKEEAIASLSDDEQREVRLYE NDIADNTKKKILEKFNIREEDLDFSKMSNNTYFIRDCLKNKNI DESEFTFEKNGKKLDPTDACFAREYKNKLSELTRKKINEKIWE IKKNSKEYHKISIYKKETIRYIVNKLIKQSKEKSECDDIIVNIEK LQIGGNFFGGRGKRDPGWNNFFLPKEENRWFINACHKAFSEL APHKGIIVIESDPAYTSQTCPKCENCDKENRNGEKFKCKKCNY EANADIDVATENLEKIAKNGRRLIKNFDQLGERLPGAEMPGG ARKRKPSKSLPKNGRGAGVGSEPELINQSPSQVIA |
| CasΦ.32 | SEQ ID NO: 252 | VPDKKETPLVALCKKSFPGLRFKKHDSRQAGRILKSKGEGAA VAFLEGKGGTTQPNFKPPVKCNIVAMSRPLEEWPIYKASVVIQ KYVYAQSYEEFKATDPGKSEAGLRAWLKATRVDTDGYFNV QGLNLIFQNARATYEGVLKKVENRNSKKVAKIEQRNEHRAER GLPLLTLDEPETALDETGHLRHRPGINCSVFGYQHMKLKPYV PGSIPGVTGYSRDPSTPIAACGVDRLEIPEGQPGYVPPWDREN LSVKKHRRKRASWARSRGGAIDDNMLLAVVRVADDWALLD LRGLLRNTQYRKLLDRSVPVTIESLLNLVTNDPTLSVVKKPGK PVRYTATLIYKQGVVPVVKAKVVKGSYVSKMLDDTTETFSL VGVDLGVNNLIAANALRIRPGKCVERLQAFTLPEQTVEDFFRF RKAYDKHQENLRLAAVRSLTAEQQAEVLALDTFGPEQAKMQ VCGHLGLSVDEVPWDKVNSRSSILSDLAKERGVDDTLYMFPF FKGKGKKRKTEIRKRWDVNWAQHFRPQLTSETRKALNEAK WEAERNSSKYHQLSIRKKELSRHCVNYVIRTAEKRAQCGKVI VAVEDLHHSFRRGGKGSRKSGWGGFFAAKQEGRWLMDALF GAFCDLAVHRGYRVIKVDPYNTSRTCPECGHCDKANRDRVN REAFICVCCGYRGNADIDVAAYNIAMVAITGVSLRKAARASV ASTPLESLAAE |
| CasΦ.33 | SEQ ID NO: 253 | MSKTKELNDYQEALARRLPGVRHQKSVRRAARLVYDRQGE DAMVAFLDGKEVDEPYTLQPPAKCHILAVSRPIEEWPIARVT MAVQEHVYALPVHEVEKSRPETTEGSRSAWFKNSGVSNHGV THAQTLNAILKNAYNVYNGVIKKVENRNAKKRDSLAAKNKS RERKGLPHFKADPPELATDEQGYLLQPPSPNSSVYLVQQHLR TPQIDLPSGYTGPVVDPRSPIPSLIPIDRLAIPPGQPGYVPLHDR EKLTSNKHRRMKLPKSLRAQGALPVCFRVFDDWAVVDGRGL LRHAQYRRLAPKNVSIAELLELYTGDPVIDIKRNLMTFRFAEA VVEVTARKIVEKYHNKYLLKLTEPKGKPVREIGLVSIDLNVQ RLIALAIYRVHQTGESQLALSPCLHREILPAKGLGDFDKYKSK FNQLTEEILTAAVQTLTSAQQEEYQRYVEESSHEAKADLCLK YSITPHELAWDKMTSSTQYISRWLRDHGWNASDFTQITKGRK KVERLWSDSRWAQELKPKLSNETRRKLEDAKHDLQRANPEW QRLAKRKQEYSRHLANTVLSMAREYTACETVVIAIENLPMKG GFVDGNGSRESGWDNFFTHKKENRWMIKDIHKALSDLAPNR GVHVLEVNPQYTSQTCPECGHRDKANRDPIQRERFCCTHCGA QRHADLEVATHNIAMVATTGKSLTGKSLAPQRLQEAAE |
| CasΦ.41 | SEQ ID NO: 254 | VLLSDRIQYTDPSAPIPAMTVVDRRKIKKGEPGYVPPFMRKNL STNKHRRMRLSRGQKEACALPVGLRLPDGKDGWDFIIFDGRA LLRACRRLRLEVTSMDDVLDKFTGDPRIQLSPAGETIVTCMLK PQHTGVIQQKLITGKMKDRLVQLTAEAPIAMLTVDLGEHNLV ACGAYTVGQRRGKLQSERLEAFLLPEKVLADFEGYRRDSDEH SETLRHEALKALSKRQQREVLDMLRTGADQARESLCYKYGL DLQALPWDKMSSNSTFIAQHLMSLGFGESATHVRYRPKRKAS ERTILKYDSRFAAEEKIKLTDETRRAWNEAIWECQRASQEFRC LSVRKLQLARAAVNWTLTQAKQRSRCPRVVVVVEDLNVRF MHGGGKRQEGWAGFFKARSEKRWFIQALHKAYTELPTNRGI HVMEVNPARTSITCTKCGYCDPENRYGEDFHCRNPKCKVRG GHVANADLDIATENLARVALSGPMPKAPKLK |
| CasΦ.34 | SEQ ID NO: 255 | MTPSFGYQMIIVTPIHHASGAWATLRLLFLNPKTSGVMLGMT KTKSAFALMREEVFPGLLFKSADLKMAGRKFAKEGREAAIEY LRGKDEERPANFKPPAKGDIIAQSRPFDQWPIVQVSQAIQKYIF GLTKAEFDATKTLLYGEGNHPTTESRRRWFEATGVPDFGFTS AQGLNAIFSSALARYEGVIQKVENRNEKRLKKLSEKNQRLVE EGHAVEAYVPETAFHTLESLKALSEKSLVPLDDLMDKIDRLA QPPGINPCLYGYQQVAPYIYDPENPRGVVLPDLYLGYCRKPD DPITACPNRLDIPKGQPGYIPEHQRGQLKKHGRVRRFRYTNPQ AKARAKAQTAILAVLRIDEDWVVMDLRGLLRNVYFREVAAP GELTARTLLDTFTGCPVLNLRSNVVTFCYDIESKGALHAEYV RKGWATRNKLLDLTKDGQSVALLSVDLGQRHPVAVMISRLK RDDKGDLSEKSIQVVSRTFADQYVDKLKRYRVQYDALRKEIY DAALVSLPPEQQAEIRAYEAFAPGDAKANVLSVMFQGEVSPD ELPWDKMNTNTHYISDLYLRRGGDPSRVFFVPQPSTPKKNAK KPPAPRKPVKRTDENVSHMPEFRPHLSNETREAFQKAKWTM ERGNVRYAQLSRFLNQIVREANNWLVSEAKKLTQCQTVVWA IEDLHVPFFHGKGKYHETWDGFFRQKKEDRWFVNVFHKAISE RAPNKGEYVMEVAPYRTSQRCPVCGFVDADNRHGDHFKCLR CGVELHADLEVATWNIALVAVQGHGIAGPPREQSCGGETAG TARKGKNIKKNKGLADAVTVEAQDSEGGSKKDAGTARNPVY IPSESQVNCPAP |
| CasΦ.35 | SEQ ID NO: 256 | MKPKTPKPPKTPVAALIDKHFPGKRFRASYLKSVGKKLKNQG EDVAVRFLTGKDEERPPNFQPPAKSNIVAQSRPIEEWPIHKVS VAVQEYVYGLTVAEKEACSDAGESSSSHAAWFAKTGVENFG YTSVQGLNKIFPPTFNRFDGVIKKVENRNEKKRQKATRINEAK RNKGQSEDPPEAEVKATDDAGYLLQPPGINHSVYGYQSITLCP YTAEKFPTIKLPEEYAGYHSNPDAPIPAGVPDRLAIPEGQPGH VPEEHRAGLSTKKHRRVRQWYAMANWKPKPKRTSKPDYDR LAKARAQGALLIVIRIDEDWVVVDARGLLRNVRWRSLGKREI TPNELLDLFTGDPVLDLKRGVVTFTYAEGVVNVCSRSTTKGK QTKVLLDAMTAPRDGKKRQIGMVAVDLGQTNPIAAEYSRVG KNAAGTLEATPLSRSTLPDELLREIALYRKAHDRLEAQLREEA VLKLTAEQQAENARYVETSEEGAKLALANLGVDTSTLPWDA MTGWSTCISDHLINHGGDTSAVFFQTIRKGTKKLETIKRKDSS WADIVRPRLTKETREALNDFLWELKRSHEGYEKLSKRLEELA RRAVNHVVQEVKWLTQCQDIVIVIEDLNVRNFHGGGKRGGG WSNFFTVKKENRWFMQALHKAFSDLAAHRGIPVLEVYPART SITCLGCGHCDPENRDGEAFVCQQCGATFHADLEVATRNIAR VALTGEAMPKAPAREQPGGAKKRGTSRRRKLTEVAVKSAEP TIHQAKNQQLNGTSRDPVYKGSELPAL |
| CasΦ.43 | SEQ ID NO: 257 | MSEITDLLKANFKGKTFKSADMRMAGRILKKSGAQAVIKYLS DKGAVDPPDFRPPAKCNIIAQSRPFDEWPICKASMAIQQHIYG LTKNEFDESSPGTSSASHEQWFAKTGVDTHGFTHVQGLNLIF QHAKKRYEGVIKKVENYNEKERKKFEGINERRSKEGMPLLEP RLRTAFGDDGKFAEKPGVNPSIYLYQQTSPRPYDKTKHPYVH APFELKEITTIPTQDDRLKIPFGAPGHVPEKHRSQLSMAKHKR RRAWYALSQNKPRPPKDGSKGRRSVRDLADLKAASLADAIPL VSRVGFDWVVIDGRGLLRNLRWRKLAHEGMTVEEMLGFFSG DPVIDPRRNVATFIYKAEHATVKSRKPIGGAKRAREELLKATA SSDGVIRQVGLISVDLGQTNPVAYEISRMHQANGELVAEHLE YGLLNDEQVNSIQRYRAAWDSMNESFRQKAIESLSMEAQDEI MQASTGAAKRTREAVLTMFGPNATLPWSRMSSNTTCISDALI EVGKEEETNFVTSNGPRKRTDAQWAAYLRPRVNPETRALLN QAVWDLMKRSDEYERLSKRKLEMARQCVNFVVARAEKLTQ CNNIGIVLENLVVRNFHGSGRRESGWEGFFEPKRENRWFMQV LHKAFSDLAQHRGVMVFEVHPAYSSQTCPACRYVDPKNRSS EDRERFKCLKCGRSFNADREVATFNIREIARTGVGLPKPDCER SRGVQTTGTARNPGRSLKSNKNPSEPKRVLQSKTRKKITSTET QNEPLATDLKT |
| CasΦ.44 | SEQ ID NO: 258 | MTPKTESPLSALCKKHFPGKRFRTNYLKDAGKILKKHGEDAV VAFLSDKQEDEPANFCPPAKVHILAQSRPFEDWPINLASKAIQ TYVYGLTADERKTCEPGTSKESHDRWFKETGVDHHGFTSVQ GLNLIFKHTLNRYDGVIKKVETRNEKRRSSVVRINEKKAAEG LPLIAAEAEETAFGEDGRLLQPPGVNHSIYCFQQVSPQPYSSK KHPQVVLPHAVQGVDPDAPIPVGRPNRLDIPKGQPGYVPEWQ RPHLSMKCKRVRMWYARANWRRKPGRRSVLNEARLKEASA KGALPIVLVIGDDWLVMDARGLLRSVFWRRVAKPGLSLSELL NVTPTGLFSGDPVIDPKRGLVTFTSKLGVVAVHSRKPTRGKKS KDLLLKMTKPTDDGMPRHVGMVAIDLGQTNPVAAEYSRVV QSDAGTLKQEPVSRGVLPDDLLKDVARYRRAYDLTEESIRQE AIALLSEGHRAEVTKLDQTTANETKRLLVDRGVSESLPWEKM SSNTTYISDCLVALGKTDDVFFVPKAKKGKKETGIAVKRKDH GWSKLLRPRTSPEARKALNENQWAVKRASPEYERLSRRKLEL GRRCVNHIIQETKRWTQCEDIVVVLEDLNVGFFHGSGKRPDG WDNFFVSKRENRWFIQVLHKAFGDLATHRGTHVIEVHPARTS ITCIKCGHCDAGNRDGESFVCLASACGDRRHADLEVATRNVA RVAITGERMPPSEQARDVQKAGGARKRKPSARNVKSSYPAV EPAPASP |
| CasΦ.36 | SEQ ID NO: 259 | MSDNKMKKLSKEEKPLTPLQILIRKYIDKSQYPSGFKTTIIKQA GVRIKSVKSEQDEINLANWIISKYDPTYIKRDFNPSAKCQIIATS RSVADFDIVKMSNKVQEIFFASSHLDKNVFDIGKSKSDHDSW FERNNVDRGIYTYSNVQGMNLIFSNTKNTYLGVAVKAQNKFS SKMKRIQDINNFRITNHQSPLPIPDEIKIYDDAGFLLNPPGVNP NIFGYQSCLLKPLENKEIISKTSFPEYSRLPADMIEVNYKISNRL KFSNDQKGFIQFKDKLNLFKINSQELFSKRRRLSGQPILLVASF GDDWVVLDGRGLLRQVYYRGIAKPGSITISELLGFFTGDPIVD PIRGVVSLGFKPGVLSQETLKTTSARIFAEKLPNLVLNNNVGL MSIDLGQTNPVSYRLSEITSNMSVEHICSDFLSQDQISSIEKAKT SLDNLEEEIAIKAVDHLSDEDKINFANFSKLNLPEDTRQSLFEK YPELIGSKLDFGSMGSGTSYIADELIKFENKDAFYPSGKKKFD LSFSRDLRKKLSDETRKSYNDALFLEKRTNDKYLKNAKRRKQ IVRTVANSLVSKIEELGLTPVINIENLAMSGGFFDGRGKREKG WDNFFKVKKENRWVMKDFHKAFSELSPHHGVIVIESPPYCTS VTCTKCNFCDKKNRNGHKFTCQRCGLDANADLDIATENLEK VAISGKRMPGSERSSDERKVAVARKAKSPKGKAIKGVKCTIT DEPALLSANSQDCSQSTS |
| CasΦ.37 | SEQ ID NO: 260 | MALSLAEVRERHFKGLRFRSSYLKRAGKILKKEGEAACVAYL TGKDEESPPNFKPPAKCDVVAQSRPFEEWPIVQASVAVQSYV YGLTKEAFEAFNPGTTKQSHEACLAATGIDTCGYSNVQGLNL IFRQAKNRYEGVITKVENRNKKAKKKLTRKNEWRQKNGHSE LPEAPEELTFNDEGRLLQPPGINPSLYTYQQISPTPWSPKDSSIL PPQYAGYERDPNAPIPFGVAKDRLTIASGCPGYIPEWMRTAGE KTNPRTQKKFMHPGLSTRKNKRMRLPRSVRSAPLGALLVTIH LGEDWLVLDVRGLLRNARWRGVAPKDISTQGLLNLFTGDPVI DTRRGVVTFTYKPETVGIHSRTWLYKGKQTKEVLEKLTQDQT VALVAIDLGQTNPVSAAASRVSRSGENLSIETVDRFFLPDELIK ELRLYRMAHDRLEERIREESTLALTEAQQAEVRALEHVVRDD AKNKVCAAFNLDAASLPWDQMTSNTTYLSEAILAQGVSRDQ VFFTPNPKKGSKEPVEVMRKDRAWVYAFKAKLSEETRKAKN EALWALKRASPDYARLSKRREELCRRSVNMVINRAKKRTQC QVVIPVLEDLNIGFFHGSGKRLPGWDNFFVAKKENRWLMNG LHKSFSDLAVHRGFYVFEVMPHRTSITCPACGHCDSENRDGE AFVCLSCKRTYHADLDVATHNLTQVAGTGLPMPEREHPGGT KKPGGSRKPESPQTHAPILHRTDYSESADRLGS |
| CasΦ.45 | SEQ ID NO: 261 | QAVIKYLSDKGAVDPPDFRPPAKCNIIAQSRPFDEWPICKASM AIQQHIYGLTKNEFDESSPGTSSASHEQWFAKTGVDTHGFTH VQGLNLIFQHAKKRYEGVIKKVENYNEKERKKFEGINERRSK EGMPLLEPRLRTAFGDDGKFAEKPGVNPSIYLYQQTSPRPYD KTKHPYVHAPFELKEITTIPTQDDRLKIPFGAPGHVPEKHRSQL SMAKHKRRRAWYALSQNKPRPPKDGSKGRRSVRDLADLKA ASLADAIPLVSRVGFDWVVIDGRGLLRNLRWRKLAHEGMTV EEMLGFFSGDPVIDPRRNVATFIYKAEHATVKSRKPIGGAKRA REELLKATASSDGVIRQVGLISVDLGQTNPVAYEISRMHQAN GELVAEHLEYGLLNDEQVNSIQRYRAAWDSMNESFRQKAIES LSMEAQDEIMQASTGAAKRTREAVLTMFGPNATLPWSRMSS NTTCISDALIEVGKEEETNFVTSNGPRKRTDAQWAAYLRPRV NPETRALLNQAVWDLMKRSDEYERLSKRKLEMARQCVNFV VARAEKLTQCNNIGIVLENLVVRNFHGSGRRESGWEGFFEPK RENRWFMQVLHKAFSDLAQHRGVMVFEVHPAYSSQTCPACR YVDPKNRSSEDRERFKCLKCGRSFNADREVATFNIREIARTGV GLPKPDCERSRDVQTPGTARKSGRSLKSQDNLSEPKRVLQSK TRKKITSTETQNEPLATDLKT |
| CasΦ.38 | SEQ ID NO: 262 | MIKEQSELSKLIEKYYPGKKFYSNDLKQAGKHLKKSEHLTAK ESEELTVEFLKSCKEKLYDFRPPAKALIISTSRPFEEWPIYKASE SIQKYIYSLTKEELEKYNISTDKTSQENFFKESLIDNYGFANVS GLNLIFQHTKAIYDGVLKKVNNRNNKILKKYKRKIEEGIEIDSP ELEKAIDESGHFINPPGINKNIYCYQQVSPTIFNSFKETKIICPFN YKRNPNDIIQKGVIDRLAIPFGEPGYIPDHQRDKVNKHKKRIR KYYKNNENKNKDAILAKINIGEDWVLFDLRGLLRNAYWRKL IPKQGITPQQLLDMFSGDPVIDPIKNNITFIYKESIIPIHSESIIKTK KSKELLEKLTKDEQIALVSIDLGQTNPVAARFSRLSSDLKPEH VSSSFLPDELKNEICRYREKSDLLEIEIKNKAIKMLSQEQQDEI KLVNDISSEELKNSVCKKYNIDNSKIPWDKMNGFTTFIADEFI NNGGDKSLVYFTAKDKKSKKEKLVKLSDKKIANSFKPKISKE TREILNKITWDEKISSNEYKKLSKRKLEFARRATNYLINQAKK ATRLNNVVLVVEDLNSKFFHGSGKREDGWDNFFIPKKENRW FIQALHKSLTDVSIHRGINVIEVRPERTSITCPKCGCCDKENRK GEDFKCIKCDSVYHADLEVATFNIEKVAITGESMPKPDCERLG GEESIG |
| CasΦ.39 | SEQ ID NO: 263 | VAFLDGKEVDEPYTLQPPAKCHILAVSRPIEEWPIARVTMAVQ EHVYALPVHEVEKSRPETTEGSRSAWFKNSGVSNHGVTHAQ TLNAILKNAYNVYNGVIKKVENRNAKKRDSLAAKNKSRERK GLPHFKADPPELATDEQGYLLQPPSPNSSVYLVQQHLRTPQID LPSGYTGPVVDPRSPIPSLIPIDRLAIPPGQPGYVPLHDREKLTS NKHRRMKLPKSLRAQGALPVCFRVFDDWAVVDGRGLLRHA QYRRLAPKNVSIAELLELYTGDPVIDIKRNLMTFRFAEAVVEV TARKIVEKYHNKYLLKLTEPKGKPVREIGLVSIDLNVQRLIAL AIYRVHQTGESQLALSPCLHREILPAKGLGDFDKYKSKFNQLT EEILTAAVQTLTSAQQEEYQRYVEESSHEAKADLCLKYSITPH ELAWDKMTSSTQYISRWLRDHGWNASDFTQITKGRKKVERL WSDSRWAQELKPKLSNETRRKLEDAKHDLQRANPEWQRLA KRKQEYSRHLANTVLSMAREYTACETVVIAIENLPMKGGFVD GNGSRESGWDNFFTHKKENRWMIKDIHKALSDLAPNRGVHV LEVNPQYTSQTCPECGHRDKANRDPIQRERFCCTHCGAQRHA DLEVATHNIAMVATTGKSLTGKSLAPQRLQ |
| CasΦ.42 | SEQ ID NO: 264 | LEIPEGEPGHVPWFQRMDIPEGQIGHVNKIQRFNFVHGKNSGK VKFSDKTGRVKRYHHSKYKDATKPYKFLEESKKVSALDSILA IITIGDDWVVFDIRGLYRNVFYRELAQKGLTAVQLLDLFTGDP VIDPKKGIITFSYKEGVVPVFSQKIVSRFKSRDTLEKLTSQGPV ALLSVDLGQNEPVAARVCSLKNINDKIALDNSCRIPFLDDYKK QIKDYRDSLDELEIKIRLEAINSLDVNQQVEIRDLDVFSADRAK ASTVDMFDIDPNLISWDSMSDARFSTQISDLYLKNGGDESRV YFEINNKRIKRSDYNISQLVRPKLSDSTRKNLNDSIWKLKRTSE EYLKLSKRKLELSRAVVNYTIRQSKLLSGINDIVIILEDLDVKK KFNGRGIRDIGWDNFFSSRKENRWFIPAFHKSFSELSSNRGLC VIEVNPAWTSATCPDCGFCSKENRDGINFTCRKCGVSYHADI DVATLNIARVAVLGKPMSGPADRERLGGTKKPRVARSRKDM KRKDISNGTVEVMVTA |
| CasΦ.46 | SEQ ID NO: 265 | IPSFGYLDRLKIAKGQPGYIPEWQRETINPSKKVRRYWATNHE KIRNAIPLVVFIGDDWVIIDGRGLLRDARRRKLADKNTTIEQL LEMVSNDPVIDSTRGIATLSYVEGVVPVRSFIPIGEKKGREYLE KSTQKESVTLLSVDIGQINPVSCGVYKVSNGCSKIDFLDKFFL DKKHLDAIQKYRTLQDSLEASIVNEALDEIDPSFKKEYQNINS QTSNDVKKSLCTEYNIDPEAISWQDITAHSTLISDYLIDNNITN DVYRTVNKAKYKTNDFGWYKKFSAKLSKEAREALNEKIWEL KIASSKYKKLSVRKKEIARTIANDCVKRAETYGDNVVVAMES LTKNNKVMSGRGKRDPGWHNLGQAKVENRWFIQAISSAFED KATHHGTPVLKVNPAYTSQTCPSCGHCSKDNRSSKDRTIFVC KSCGEKFNADLDVATYNIAHVAFSGKKLSPPSEKSSATKKPRS ARKSKKSRKS |
| CasΦ.47 | SEQ ID NO: 266 | SPIEKLLNGLLVKITFGNDWIICDARGLLDNVQKGIIHKSYFTN KSSLVDLIDLFTCNPIVNYKNNVVTFCYKEGVVDVKSFTPIKS GPKTQENLIKKLKYSRFQNEKDACVLGVGVDVGVTNPFAING FKMPVDESSEWVMLNEPLFTIETSQAFREEIMAYQQRTDEMN DQFNQQSIDLLPPEYKVEFDNLPEDINEVAKYNLLHTLNIPNN FLWDKMSNTTQFISDYLIQIGRGTETEKTITTKKGKEKILTIRD VNWFNTFKPKISEETGKARTEIKRDLQKNSDQFQKLAKSREQ SCRTWVNNVTEEAKIKSGCPLIIFVIEALVKDNRVFSGKGHRA IGWHNFGKQKNERRWWVQAIHKAFQEQGVNHGYPVILCPPQ YTSQTCPKCNHVDRDNRSGEKFKCLKYGWIGNADLDVGAYN IARVAITGKALSKPLEQKKIKKAKNKT |
| CasΦ.48 | SEQ ID NO: 267 | LLDNVQKGIIHKSYFTNKSSLVDLIDLFTCNPIVNYKNNVVTF CYKEGVVDVKSFTPIKSGPKTQENLIKKLKYSRFQNEKDACV LGVGVDVGVTNPFAINGFKMPVDESSEWVMLNEPLFTIETSQ AFREEIMAYQQRTDEMNDQFNQQSIDLLPPEYKVEFDNLPEDI NEVAKYNLLHTLNIPNNFLWDKMSNTTQFISDYLIQIGRGTET EKTITTKKGKEKILTIRDVNWFNTFKPKISEETGKARTEIKRDL QKNSDQFQKLAKSREQSCRTWVNNVTEEAKIKSGCPLIIFVIE ALVKDNRVFSGKGHRAIGWHNFGKQKNERRWWVQAIHKAF QEQGVNHGYPVILCPPQYTSQTCPKCNHVDRDNRSGEKFKCL KYGWIGNADLDVGAYNIARVAITGKALSKPLEQKKIKKAKN KT |
| CasΦ.49 | SEQ ID NO: 268 | MIKPTVSQFLTPGFKLIRNHSRTAGLKLKNEGEEACKKFVREN EIPKDECPNFQGGPAIANIIAKSREFTEWEIYQSSLAIQEVIFTLP KDKLPEPILKEEWRAQWLSEHGLDTVPYKEAAGLNLIIKNAV NTYKGVQVKVDNKNKNNLAKINRKNEIAKLNGEQEISFEEIK AFDDKGYLLQKPSPNKSIYCYQSVSPKPFITSKYHNVNLPEEYI GYYRKSNEPIVSPYQFDRLRIPIGEPGYVPKWQYTFLSKKENK RRKLSKRIKNVSPILGIICIKKDWCVFDMRGLLRTNHWKKYH KPTDSINDLFDYFTGDPVIDTKANVVRFRYKMENGIVNYKPV REKKGKELLENICDQNGSCKLATVDVGQNNPVAIGLFELKKV NGELTKTLISRHPTPIDFCNKITAYRERYDKLESSIKLDAIKQLT SEQKIEVDNYNNNFTPQNTKQIVCSKLNINPNDLPWDKMISGT HFISEKAQVSNKSEIYFTSTDKGKTKDVMKSDYKWFQDYKPK LSKEVRDALSDIEWRLRRESLEFNKLSKSREQDARQLANWISS MCDVIGIENLVKKNNFFGGSGKREPGWDNFYKPKKENRWWI NAIHKALTELSQNKGKRVILLPAMRTSITCPKCKYCDSKNRN GEKFNCLKCGIELNADIDVATENLATVAITAQSMPKPTCERSG DAKKPVRARKAKAPEFHDKLAPSYTVVLREAVKRPAATKK AGQAKKKKEF (Bold sequence is Nuclear Localization Signal) |

In some embodiments, any of the programmable CasΦ nuclease of the present disclosure (e.g., any one of SEQ ID NO: 221 - SEQ ID NO: 268 or fragments or variants thereof) may include a nuclear localization signal (NLS). In some cases, said NLS may have a sequence of KRPAATKKAGQAKKKKEF (SEQ ID NO: 269).

A CasΦ polypeptide or a variant thereof can comprise at least 70%, at least 75%, at least 80%, at least 85%, at least 90%, at least 92%, at least 95%, at least 97%, at least 99%, or 100% sequence identity with any one of SEQ ID NO: 221 - SEQ ID NO: 268.

In some embodiments, the Type VI CRISPR/Cas enzyme is a programmable Cas13 nuclease. The general architecture of a Cas13 protein includes an N-terminal domain and two HEPN (higher eukaryotes and prokaryotes nucleotide-binding) domains separated by two helical domains (Liu et al., Cell 2017 Jan 12;168(l-2):121-134.el2). The HEPN domains each comprise aR-X₄-H motif. Shared features across Cas13 proteins include that upon binding of the crRNA of the guide nucleic acid to a target nucleic acid, the protein undergoes a conformational change to bring together the HEPN domains and form a catalytically active RNase. (Tambe et al., Cell Rep. 2018 Jul 24; 24(4): 1025-1036.). Thus, two activatable HEPN domains are characteristic of a programmable Cas13 nuclease of the present disclosure. However, programmable Cas13 nucleases also consistent with the present disclosure include Cas13 nucleases comprising mutations in the HEPN domain that enhance the Cas13 proteins cleavage efficiency or mutations that catalytically inactivate the HEPN domains. Programmable Cas13 nucleases consistent with the present disclosure also Cas13 nucleases comprising catalytic

A programmable Cas13 nuclease can be a Cas13a protein (also referred to as "c2c2"), a Cas13b protein, a Cas13c protein, a Cas13d protein, or a Cas13e protein. Example C2c2 proteins are set forth as SEQ ID NO: 153 - SEQ ID NO: 160. In some cases, a subject C2c2 protein includes an amino acid sequence having 80% or more (e.g., 85% or more, 90% or more, 95% or more, 98% or more, 99% or more, 99.5% or more, or 100%) amino acid sequence identity with the amino acid sequence set forth in any one of SEQ ID NO: 153 - SEQ ID NO: 160. In some cases, a suitable C2c2 polypeptide comprises an amino acid sequence having at least 80%, at least 85%, at least 90%, at least 95%, at least 98%, at least 99%, or 100%, amino acid sequence identity to the Listeria seeligeri C2c2 amino acid sequence set forth in SEQ ID NO: 153. In some cases, a suitable C2c2 polypeptide comprises an amino acid sequence having at least 80%, at least 85%, at least 90%, at least 95%, at least 98%, at least 99%, or 100%, amino acid sequence identity to the Leptotrichia buccalis C2c2 amino acid sequence set forth in SEQ ID NO: 154. In some cases, a suitable C2c2 polypeptide comprises an amino acid sequence having at least 80%, at least 85%, at least 90%, at least 95%, at least 98%, at least 99%, or 100%, amino acid sequence identity to the Rhodobacter capsulatus C2c2 amino acid sequence set forth in SEQ ID NO: 156. In some cases, a suitable C2c2 polypeptide comprises an amino acid sequence having at least 80%, at least 85%, at least 90%, at least 95%, at least 98%, at least 99%, or 100%, amino acid sequence identity to the Carnobacterium gallinarum C2c2 amino acid sequence set forth in SEQ ID NO: 157. In some cases, a suitable C2c2 polypeptide comprises an amino acid sequence having at least 80%, at least 85%, at least 90%, at least 95%, at least 98%, at least 99%, or 100%, amino acid sequence identity to the Herbinix hemicellulosilytica C2c2 amino acid sequence set forth in SEQ ID NO: 158. In some cases, the C2c2 protein includes an amino acid sequence having 80% or more amino acid sequence identity with the Leptotrichia buccalis (Lbu) C2c2 amino acid sequence set forth in SEQ ID NO: 154. In some cases, the C2c2 protein is a Leptotrichia buccalis (Lbu) C2c2 protein (e.g., see SEQ ID NO: 154). In some cases, the C2c2 protein includes the amino acid sequence set forth in any one of SEQ ID NO: 153, SEQ ID NO: 154 and SEQ ID NO: 156 - SEQ ID NO: 160. In some cases, a C2c2 protein used in a method of the present disclosure is not a Leptotrichia shahii (Lsh) C2c2 protein. In some cases, a C2c2 protein used in a method of the present disclosure is not a C2c2 polypeptide having at least 80%, at least 85%, at least 90%, at least 95%, at least 98%, at least 99%, or 100%, amino acid sequence identity to the Lsh C2c2 polypeptide set forth in SEQ ID NO: 155. Other Cas13 protein sequences are set forth in SEQ ID NO: 153 - SEQ ID NO: 170.

**TABLE 4 - Cas13 Protein Sequences**

| **SEQ ID NO** | **Description** | **Sequence** |
|---|---|---|
| SEQ ID NO: 153 | Listeria seeligeri C2c2 amino acid sequence | MWISIKTLIHHLGVLFFCDYMYNRREKKIIEVKTMRITKVEVDRKKVLIS RDKNGGKLVYENEMQDNTEQIMHHKKSSFYKSVVNKTICRPEQKQMK KLVHGLLQENSQEKIKVSDVTKLNISNFLNHRFKKSLYYFPENSPDKSEE YRIEINLSQLLEDSLKKQQGTFICWESFSKDMELYINWAENYISSKTKLI KKSIRNNRIQSTESRSGQLMDRYMKDILNKNKPFDIQSVSEKYQLEKLT SALKATFKEAKKNDKEINYKLKSTLQNHERQIIEELKENSELNQFNIEIR KHLETYFPIKKTNRKVGDIRNLEIGEIQKIVNHRLKNKIVQRILQEGKLA SYEIESTVNSNSLQKIKIEEAFALKFINACLFASNNLRNMVYPVCKKDIL MIGEFKNSFKEIKHKKFIRQWSQFFSQEITVDDIELASWGLRGAIAPIRNE IIHLKKHSWKKFFNNPTFKVKKSKIINGKTKDVTSEFLYKETLFKDYFYS ELDSVPELIINKMESSKILDYYSSDQLNQVFTIPNFELSLLTSAVPFAPSFK RVYLKGFDYQNQDEAQPDYNLKLNIYNEKAFNSEAFQAQYSLFKMVY YQVFLPQFTTNNDLFKSSVDFILTLNKERKGYAKAFQDIRKMNKDEKPS EYMSYIQSQLMLYQKKQEEKEKINHFEKFINQVFIKGFNSFIEKNRLTYI CHPTKNTVPENDNIEIPFHTDMDDSNIAFWLMCKLLDAKQLSELRNEMI KFSCSLQSTEEISTFTKAREVIGLALLNGEKGCNDWKELFDDKEAWKK NMSLYVSEELLQSLPYTQEDGQTPVINRSIDLVKKYGTETILEKLFSSSD DYKVSAKDIAKLHEYDVTEKIAQQESLHKQWIEKPGLARDSAWTKKY QNVINDISNYQWAKTKVELTQVRHLHQLTIDLLSRLAGYMSIADRDFQF SSNYILERENSEYRVTSWILLSENKNKNKYNDYELYNLKNASIKVSSKN DPQLKVDLKQLRLTLEYLELFDNRLKEKRNNISHFNYLNGQLGNSILEL FDDARDVLSYDRKLKNAVSKSLKEILSSHGMEVTFKPLYQTNHHLKID KLQPKKIHHLGEKSTVSSNQVSNEYCQLVRTLLTMK |
| SEQ ID NO: 154 | Leptotrichia buccalis (Lbu) C2c2 amino acid sequence | MKVTKVGGISHKKYTSEGRLVKSESEENRTDERLSALLNMRLDMYIKN PSSTETKENQKRIGKLKKFFSNKMVYLKDNTLSLKNGKKENIDREYSET DILESDVRDKKNFAVLKKIYLNENVNSEELEVFRNDIKKKLNKINSLKY SFEKNKANYQKINENNIEKVEGKSKRNIIYDYYRESAKRDAYVSNVKE AFDKLYKEEDIAKLVLEIENLTKLEKYKIREFYHEIIGRKNDKENFAKIIY EEIQNVNNMKELIEKVPDMSELKKSQVFYKYYLDKEELNDKNIKYAFC HFVEIEMSQLLKNYVYKRLSNISNDKIKRIFEYQNLKKLIENKLLNKLDT YVRNCGKYNYYLQDGEIATSDFIARNRQNEAFLRNIIGVSSVAYFSLRNI LETENENDITGRMRGKTVKNNKGEEKYVSGEVDKIYNENKKNEVKEN LKMFYSYDFNMDNKNEIEDFFANIDEAISSIRHGIVHFNLELEGKDIFAF KNIAPSEISKKMFQNEINEKKLKLKIFRQLNSANVFRYLEKYKILNYLKR TRFEFVNKNIPFVPSFTKLYSRIDDLKNSLGIYWKTPKTNDDNKTKEIID AQIYLLKNIYYGEFLNYFMSNNGNFFEISKEIIELNKNDKRNLKTGFYKL QKFEDIQEKIPKEYLANIQSLYMINAGNQDEEEKDTYIDFIQKIFLKGFM TYLANNGRLSLIYIGSDEETNTSLAEKKQEFDKFLKKYEQNNNIKIPYEI NEFLREIKLGNILKYTERLNMFYLILKLLNHKELTNLKGSLEKYQSANK EEAFSDQLELINLLNLDNNRVTEDFELEADEIGKFLDFNGNKVKDNKEL KKFDTNKIYFDGENIIKHRAFYNIKKYGMLNLLEKIADKAGYKISIEELK KYSNKKNEIEKNHKMQENLHRKYARPRKDEKFTDEDYESYKQAIENIE EYTHLKNKVEFNELNLLQGLLLRILHRLVGYTSIWERDLRFRLKGEFPE NQYIEEIFNFENKKNVKYKGGQIVEKYIKFYKELHQNDEVKINKYSSAN IKVLKQEKKDLYIRNYIAHFNYIPHAEISLLEVLENLRKLLSYDRKLKNA VMKSVVDILKEYGFVATFKIGADKKIGIQTLESEKIVHLKNLKKKKLMT DRNSEELCKLVKIMFEYKMEEKKSEN |
| SEQ ID NO: 155 | Leptotrichia shahii (Lsh) C2c2 protein | MGNLFGHKRWYEVRDKKDFKIKRKVKVKRNYDGNKYILNINENNNKE KIDNNKFIRKYINYKKNDNILKEFTRKFHAGNILFKLKGKEGIIRIENNDD FLETEEVVLYIEAYGKSEKLKALGITKKKIIDEAIRQGITKDDKKIEIKRQ ENEEEIEIDIRDEYTNKTLNDCSIILRIIENDELETKKSIYEIFKNINMSLYK IIEKIIENETEKVFENRYYEEHLREKLLKDDKIDVILTNFMEIREKIKSNLE ILGFVKFYLNVGGDKKKSKNKKMLVEKILNINVDLTVEDIADFVIKELE FWNITKRIEKVKKVNNEFLEKRRNRTYIKSYVLLDKHEKFKIERENKKD KIVKFFVENIKNNSIKEKIEKILAEFKIDELIKKLEKELKKGNCDTEIFGIF KKHYKVNFDSKKFSKKSDEEKELYKIIYRYLKGRIEKILVNEQKVRLKK MEKIEIEKILNESILSEKILKRVKQYTLEHIMYLGKLRHNDIDMTTVNTD DFSRLHAKEELDLELITFFASTNMELNKIFSRENINNDENIDFFGGDREK NYVLDKKILNSKIKIIRDLDFIDNKNNITNNFIRKFTKIGTNERNRILHAIS KERDLQGTQDDYNKVINIIQNLKISDEEVSKALNLDVVFKDKKNIITKIN DIKISEENNNDIKYLPSFSKVLPEILNLYRNNPKNEPFDTIETEKIVLNALI YVNKELYKKLILEDDLEENESKNIFLQELKKTLGNIDEIDENIIENYYKN AQISASKGNNKAIKKYQKKVIECYIGYLRKNYEELFDFSDFKMNIQEIK KQIKDINDNKTYERITVKTSDKTIVINDDFEYIISIFALLNSNAVINKIRNR FFATSVWLNTSEYQNIIDILDEIMQLNTLRNECITENWNLNLEEFIQKMK EIEKDFDDFKIQTKKEIFNNYYEDIKNNILTEFKDDINGCDVLEKKLEKIV IFDDETKFEIDKKSNILQDEQRKLSNINKKDLKKKVDQYIKDKDQEIKSK ILCRIIFNSDFLKKYKKEIDNLIEDMESENENKFQEIYYPKERKNELYIYK KNLFLNIGNPNFDKIYGLISNDIKMADAKFLFNIDGKNIRKNKISEIDAIL KNLNDKLNGYSKEYKEKYIKKLKENDDFFAKNIQNKNYKSFEKDYNR VSEYKKIRDLVEFNYLNKIESYLIDINWKLAIQMARFERDMHYIVNGLR ELGIIKLSGYNTGISRAYPKRNGSDGFYTTTAYYKFFDEESYKKFEKICY GFGIDLSENSEINKPENESIRNYISHFYIVRNPFADYSIAEQIDRVSNLLSY STRYNNSTYASVFEVFKKDVNLDYDELKKKFKLIGNNDILERLMKPKK VSVLELESYNSDYIKNLIIELLTKIENTNDTL |
| SEQ ID NO: 156 | Rhodobacter capsulatus C2c2 amino acid sequence | MQIGKVQGRTISEFGDPAGGLKRKISTDGKNRKELPAHLSSDPKALIGQ WISGIDKIYRKPDSRKSDGKAIHSPTPSKMQFDARDDLGEAFWKLVSEA GLAQDSDYDQFKRRLHPYGDKFQPADSGAKLKFEADPPEPQAFHGRW YGAMSKRGNDAKELAAALYEHLHVDEKRIDGQPKRNPKTDKFAPGLV VARALGIESSVLPRGMARLARNWGEEEIQTYFVVDVAASVKEVAKAA VSAAQAFDPPRQVSGRSLSPKVGFALAEHLERVTGSKRCSFDPAAGPSV LALHDEVKKTYKRLCARGKNAARAFPADKTELLALMRHTHENRVRNQ MVRMGRVSEYRGQQAGDLAQSHYWTSAGQTEIKESEIFVRLWVGAFA LAGRSMKAWIDPMGKIVNTEKNDRDLTAAVNIRQVISNKEMVAEAMA RRGIYFGETPELDRLGAEGNEGFVFALLRYLRGCRNQTFHLGARAGFLK EIRKELEKTRWGKAKEAEHVVLTDKTVAAIRAIIDNDAKALGARLLAD LSGAFVAHYASKEHFSTLYSEIVKAVKDAPEVSSGLPRLKLLLKRADGV RGYVHGLRDTRKHAFATKLPPPPAPRELDDPATKARYIALLRLYDGPFR AYASGITGTALAGPAARAKEAATALAQSVNVTKAYSDVMEGRSSRLRP PNDGETLREYLSALTGETATEFRVQIGYESDSENARKQAEFIENYRRDM LAFMFEDYIRAKGFDWILKIEPGATAMTRAPVLPEPIDTRGQYEHWQA ALYLVMHFVPASDVSNLLHQLRKWEALQGKYELVQDGDATDQADAR REALDLVKRFRDVLVLFLKTGEARFEGRAAPFDLKPFRALFANPATFDR LFMATPTTARPAEDDPEGDGASEPELRVARTLRGLRQIARYNHMAVLS DLFAKHKVRDEEVARLAEIEDETQEKSQIVAAQELRTDLHDKVMKCHP KTISPEERQSYAAAIKTIEEHRFLVGRVYLGDHLRLHRLMMDVIGRLID YAGAYERDTGTFLINASKQLGAGADWAVTIAGAANTDARTQTRKDLA HFNVLDRADGTPDLTALVNRAREMMAYDRKRKNAVPRSILDMLARLG LTLKWQMKDHLLQDATITQAAIKHLDKVRLTVGGPAAVTEARFSQDY LQMVAAVFNGSVQNPKPRRRDDGDAWHKPPKPATAQSQPDQKPPNKA PSAGSRLPPPQVGEVYEGVVVKVIDTGSLGFLAVEGVAGNIGLHISRLR RIREDAIIVGRRYRFRVEIYVPPKSNTSKLNAADLVRID |
| SEQ ID NO: 157 | Camobacter ium gallinarum C2c2 amino acid sequence | MRITKVKIKLDNKLYQVTMQKEEKYGTLKLNEESRKSTAEILRLKKASF NKSFHSKTINSQKENKNATIKKNGDYISQIFEKLVGVDTNKNIRKPKMS LTDLKDLPKKDLALFIKRKFKNDDIVEIKNLDLISLFYNALQKVPGEHFT DESWADFCQEMMPYREYKNKFIERKIILLANSIEQNKGFSINPETFSKRK RVLHQWAIEVQERGDFSILDEKLSKLAEIYNFKKMCKRVQDELNDLEK SMKKGKNPEKEKEAYKKQKNFKIKTIWKDYPYKTHIGLIEKIKENEELN QFNIEIGKYFEHYFPIKKERCTEDEPYYLNSETIATTVNYQLKNALISYL MQIGKYKQFGLENQVLDSKKLQEIGIYEGFQTKFMDACVFATSSLKNII EPMRSGDILGKREFKEAIATSSFVNYHHFFPYFPFELKGMKDRESELIPF GEQTEAKQMQNIWALRGSVQQIRNEIFHSFDKNQKFNLPQLDKSNFEFD ASENSTGKSQSYIETDYKFLFEAEKNQLEQFFIERIKSSGALEYYPLKSLE KLFAKKEMKFSLGSQVVAFAPSYKKLVKKGHSYQTATEGTANYLGLS YYNRYELKEESFQAQYYLLKLIYQYVFLPNFSQGNSPAFRETVKAILRIN KDEARKKMKKNKKFLRKYAFEQVREMEFKETPDQYMSYLQSEMREE KVRKAEKNDKGFEKNITMNFEKLLMQIFVKGFDVFLTTFAGKELLLSSE EKVIKETEISLSKKINEREKTLKASIQVEHQLVATNSAISYWLFCKLLDSR HLNELRNEMIKFKQSRIKFNHTQHAELIQNLLPIVELTILSNDYDEKNDS QNVDVSAYFEDKSLYETAPYVQTDDRTRVSFRPILKLEKYHTKSLIEAL LKDNPQFRVAATDIQEWMHKREEIGELVEKRKNLHTEWAEGQQTLGA EKREEYRDYCKKIDRFNWKANKVTLTYLSQLHYLITDLLGRMVGFSAL FERDLVYFSRSFSELGGETYHISDYKNLSGVLRLNAEVKPIKIKNIKVIDN EENPYKGNEPEVKPFLDRLHAYLENVIGIKAVHGKIRNQTAHLSVLQLE LSMIESMNNLRDLMAYDRKLKNAVTKSMIKILDKHGMILKLKIDENHK NFEIESLIPKEIIHLKDKAIKTNQVSEEYCQLVLALLTTNPGNQLN |
| SEQ ID NO: 158 | Herbinix hemicellulos ilytica C2c2 amino acid sequence | MKLTRRRISGNSVDQKITAAFYRDMSQGLLYYDSEDNDCTDKVIESMD FERSWRGRILKNGEDDKNPFYMFVKGLVGSNDKIVCEPIDVDSDPDNL DILINKNLTGFGRNLKAPDSNDTLENLIRKIQAGIPEEEVLPELKKIKEMI QKDIVNRKEQLLKSIKNNRIPFSLEGSKLVPSTKKMKWLFKLIDVPNKTF NEKMLEKYWEIYDYDKLKANITNRLDKTDKKARSISRAVSEELREYHK NLRTNYNRFVSGDRPAAGLDNGGSAKYNPDKEEFLLFLKEVEQYFKKY FPVKSKHSNKSKDKSLVDKYKNYCSYKVVKKEVNRSIINQLVAGLIQQ GKLLYYFYYNDTWQEDFLNSYGLSYIQVEEAFKKSVMTSLSWGINRLT SFFIDDSNTVKFDDITTKKAKEAIESNYFNKLRTCSRMQDHFKEKLAFFY PVYVKDKKDRPDDDIENLIVLVKNAIESVSYLRNRTFHFKESSLLELLKE LDDKNSGQNKIDYSVAAEFIKRDIENLYDVFREQIRSLGIAEYYKADMIS DCFKTCGLEFALYSPKNSLMPAFKNVYKRGANLNKAYIRDKGPKETGD QGQNSYKALEEYRELTWYIEVKNNDQSYNAYKNLLQLIYYHAFLPEVR ENEALITDFINRTKEWNRKETEERLNTKNNKKHKNFDENDDITVNTYR YESIPDYQGESLDDYLKVLQRKQMARAKEVNEKEEGNNNYIQFIRDVV VWAFGAYLENKLKNYKNELQPPLSKENIGLNDTLKELFPEEKVKSPFNI KCRFSISTFIDNKGKSTDNTSAEAVKTDGKEDEKDKKNIKRKDLLCFYL FLRLLDENEICKLQHQFIKYRCSLKERRFPGNRTKLEKETELLAELEELM ELVRFTMPSIPEISAKAESGYDTMIKKYFKDFIEKKVFKNPKTSNLYYHS DSKTPVTRKYMALLMRSAPLHLYKDIFKGYYLITKKECLEYIKLSNIIKD YQNSLNELHEQLERIKLKSEKQNGKDSLYLDKKDFYKVKEYVENLEQV ARYKHLQHKINFESLYRIFRIHVDIAARMVGYTQDWERDMHFLFKALV YNGVLEERRFEAIFNNNDDNNDGRIVKKIQNNLNNKNRELVSMLCWN KKLNKNEFGAIIWKRNPIAHLNHFTQTEQNSKSSLESLINSLRILLAYDR KRQNAVTKTINDLLLNDYHIRIKWEGRVDEGQIYFNIKEKEDIENEPIIHL KHLHKKDCYIYKNSYMFDKQKEWICNGIKEEVYDKSILKCIGNLFKFD YEDKNKSSANPKHT |
| SEQ ID NO: 159 | Paludibacter propionicige nes C2c2 amino acid sequence | MRVSKVKVKDGGKDKMVLVHRKTTGAQLVYSGQPVSNETSNILPEKK RQSFDLSTLNKTIIKFDTAKKQKLNVDQYKIVEKIFKYPKQELPKQIKAE EILPFLNHKFQEPVKYWKNGKEESFNLTLLIVEAVQAQDKRKLQPYYD WKTWYIQTKSDLLKKSIENNRIDLTENLSKRKKALLAWETEFTASGSID LTHYHKVYMTDVLCKMLQDVKPLTDDKGKINTNAYHRGLKKALQNH QPAIFGTREVPNEANRADNQLSIYHLEVVKYLEHYFPIKTSKRRNTADDI AHYLKAQTLKTTIEKQLVNAIRANIIQQGKTNHHELKADTTSNDLIRIKT NEAFVLNLTGTCAFAANNIRNMVDNEQTNDILGKGDFIKSLLKDNTNS QLYSFFFGEGLSTNKAEKETQLWGIRGAVQQIRNNVNHYKKDALKTVF NISNFENPTITDPKQQTNYADTIYKARFINELEKIPEAFAQQLKTGGAVS YYTIENLKSLLTTFQFSLCRSTIPFAPGFKKVFNGGINYQNAKQDESFYE LMLEQYLRKENFAEESYNARYFMLKLIYNNLFLPGFTTDRKAFADSVG FVQMQNKKQAEKVNPRKKEAYAFEAVRPMTAADSIADYMAYVQSEL MQEQNKKEEKVAEETRINFEKFVLQVFIKGFDSFLRAKEFDFVQMPQPQ LTATASNQQKADKLNQLEASITADCKLTPQYAKADDATHIAFYVFCKL LDAAHLSNLRNELIKFRESVNEFKFHHLLEIIEICLLSADVVPTDYRDLYS SEADCLARLRPFIEQGADITNWSDLFVQSDKHSPVIHANIELSVKYGTTK LLEQIINKDTQFKTTEANFTAWNTAQKSIEQLIKQREDHHEQWVKAKN ADDKEKQERKREKSNFAQKFIEKHGDDYLDICDYINTYNWLDNKMHF VHLNRLHGLTIELLGRMAGFVALFDRDFQFFDEQQIADEFKLHGFVNLH SIDKKLNEVPTKKIKEIYDIRNKIIQINGNKINESVRANLIQFISSKRNYYN NAFLHVSNDEIKEKQMYDIRNHIAHFNYLTKDAADFSLIDLINELRELLH YDRKLKNAVSKAFIDLFDKHGMILKLKLNADHKLKVESLEPKKIYHLG SSAKDKPEYQYCTNQVMMAYCNMCRSLLEMKK |
| SEQ ID NO: 160 | Leptotrichia wadei (Lwa) C2c2 amino acid sequence | MYMKITKIDGVSHYKKQDKGILKKKWKDLDERKQREKIEARYNKQIES KIYKEFFRLKNKKRIEKEEDQNIKSLYFFIKELYLNEKNEEWELKNINLEI LDDKERVIKGYKFKEDVYFFKEGYKEYYLRILFNNLIEKVQNENREKVR KNKEFLDLKEIFKKYKNRKIDLLLKSINNNKINLEYKKENVNEEIYGINP TNDREMTFYELLKEIIEKKDEQKSILEEKLDNFDITNFLENIEKIFNEETEI NIIKGKVLNELREYIKEKEENNSDNKLKQIYNLELKKYIENNFSYKKQKS KSKNGKNDYLYLNFLKKIMFIEEVDEKKEINKEKFKNKINSNFKNLFVQ HILDYGKLLYYKENDEYIKNTGQLETKDLEYIKTKETLIRKMAVLVSFA ANSYYNLFGRVSGDILGTEVVKSSKTNVIKVGSHIFKEKMLNYFFDFEIF DANKIVEILESISYSIYNVRNGVGHFNKLILGKYKKKDINTNKRIEEDLN NNEEIKGYFIKKRGEIERKVKEKFLSNNLQYYYSKEKIENYFEVYEFEIL KRKIPFAPNFKRIIKKGEDLFNNKNNKKYEYFKNFDKNSAEEKKEFLKT RNFLLKELYYNNFYKEFLSKKEEFEKIVLEVKEEKKSRGNINNKKSGVS FQSIDDYDTKINISDYIASIHKKEMERVEKYNEEKQKDTAKYIRDFVEEI FLTGFINYLEKDKRLHFLKEEFSILCNNNNNVVDFNININEEKIKEFLKEN DSKTLNLYLFFNMIDSKRISEFRNELVKYKQFTKKRLDEEKEFLGIKIEL YETLIEFVILTREKLDTKKSEEIDAWLVDKLYVKDSNEYKEYEEILKLFV DEKILSSKEAPYYATDNKTPILLSNFEKTRKYGTQSFLSEIQSNYKYSKV EKENIEDYNKKEEIEQKKKSNIEKLQDLKVELHKKWEQNKITEKEIEKY NNTTRKINEYNYLKNKEELQNVYLLHEMLSDLLARNVAFFNKWERDF KFIVIAIKQFLRENDKEKVNEFLNPPDNSKGKKVYFSVSKYKNTVENID GIHKNFMNLIFLNNKFMNRKIDKMNCAIWVYFRNYIAHFLHLHTKNEKI SLISQMNLLIKLFSYDKKVQNHILKSTKTLLEKYNIQINFEISNDKNEVFK YKIKNRLYSKKGKMLGKNNKFEILENEFLENVKAMLEYSE |
| SEQ ID NO: 161 | Bergeyella zoohelcum Cas 13b | MENKTSLGNNIYYNPFKPQDKSYFAGYFNAAMENTDSVFRELGKRLKG KEYTSENFFDAIFKENISLVEYERYVKLLSDYFPMARLLDKKEVPIKERK ENFKKNFKGIIKAVRDLRNFYTHKEHGEVEITDEIFGVLDEMLKSTVLT VKKKKVKTDKTKEILKKSIEKQLDILCQKKLEYLRDTARKIEEKRRNQR ERGEKELVAPFKYSDKRDDLIAAIYNDAFDVYIDKKKDSLKESSKAKY NTKSDPQQEEGDLKIPISKNGVVFLLSLFLTKQEIHAFKSKIAGFKATVID EATVSEATVSHGKNSICFMATHEIFSHLAYKKLKRKVRTAEINYGEAEN AEQLSVYAKETLMMQMLDELSKVPDVVYQNLSEDVQKTFIEDWNEYL KENNGDVGTMEEEQVIHPVIRKRYEDKFNYFAIRFLDEFAQFPTLRFQV HLGNYLHDSRPKENLISDRRIKEKITVFGRLSELEHKKALFIKNTETNED REHYWEIFPNPNYDFPKENISVNDKDFPIAGSILDREKQPVAGKIGIKVK LLNQQYVSEVDKAVKAHQLKQRKASKPSIQNIIEEIVPINESNPKEAIVF GGQPTAYLSMNDIHSILYEFFDKWEKKKEKLEKKGEKELRKEIGKELEK KIVGKIQAQIQQIIDKDTNAKILKPYQDGNSTAIDKEKLIKDLKQEQNILQ KLKDEQTVREKEYNDFIAYQDKNREINKVRDRNHKQYLKDNLKRKYP EAPARKEVLYYREKGKVAVWLANDIKRFMPTDFKNEWKGEQHSLLQK SLAYYEQCKEELKNLLPEKVFQHLPFKLGGYFQQKYLYQFYTCYLDKR LEYISGLVQQAENFKSENKVFKKVENECFKFLKKQNYTHKELDARVQSI LGYPIFLERGFMDEKPTIIKGKTFKGNEALFADWFRYYKEYQNFQTFYD TENYPLVELEKKQADRKRKTKIYQQKKNDVFTLLMAKHIFKSVFKQDS IDQFSLEDLYQSREERLGNQERARQTGERNTNYIWNKTVDLKLCDGKIT VENVKLKNVGDFIKYEYDQRVQAFLKYEENIEWQAFLIKESKEEENYP YVVEREIEQYEKVRREELLKEVHLIEEYILEKVKDKEILKKGDNQNFKY YILNGLLKQLKNEDVESYKVFNLNTEPEDVNINQLKQEATDLEQKAFV LTYIRNKFAHNQLPKKEFWDYCQEKYGKIEKEKTYAEYFAEVFKKEKE ALIK |
| SEQ ID NO: 162 | Prevotella intermedia Cas 13b | MEDDKKTTDSIRYELKDKHFWAAFLNLARHNVYITVNHINKILEEGEIN RDGYETTLKNTWNEIKDINKKDRLSKLIIKHFPFLEAATYRLNPTDTTKQ KEEKQAEAQSLESLRKSFFVFIYKLRDLRNHYSHYKHSKSLERPKFEEG LLEKMYNIFNASIRLVKEDYQYNKDINPDEDFKHLDRTEEEFNYYFTKD NEGNITESGLLFFVSLFLEKKDAIWMQQKLRGFKDNRENKKKMTNEVF CRSRMLLPKLRLQSTQTQDWILLDMLNELIRCPKSLYERLREEDREKFR VPIEIADEDYDAEQEPFKNTLVRHQDRFPYFALRYFDYNEIFTNLRFQID LGTYHFSIYKKQIGDYKESHHLTHKLYGFERIQEFTKQNRPDEWRKFVK TFNSFETSKEPYIPETTPHYHLENQKIGIRFRNDNDKIWPSLKTNSEKNEK SKYKLDKSFQAEAFLSVHELLPMMFYYLLLKTENTDNDNEIETKKKEN KNDKQEKHKIEEIIENKITEIYALYDTFANGEIKSIDELEEYCKGKDIEIGH LPKQMIAILKDEHKVMATEAERKQEEMLVDVQKSLESLDNQINEEIENV ERKNSSLKSGKIASWLVNDMMRFQPVQKDNEGKPLNNSKANSTEYQL LQRTLAFFGSEHERLAPYFKQTKLIESSNPHPFLKDTEWEKCNNILSFYR SYLEAKKNFLESLKPEDWEKNQYFLKLKEPKTKPKTLVQGWKNGFNLP RGIFTEPIRKWFMKHRENITVAELKRVGLVAKVIPLFFSEEYKDSVQPFY NYHFNVGNINKPDEKNFLNCEERRELLRKKKDEFKKMTDKEKEENPSY LEFKSWNKFERELRLVRNQDIVTWLLCMELFNKKKIKELNVEKIYLKNI NTNTTKKEKNTEEKNGEEKNIKEKNNILNRIMPMRLPIKVYGRENFSKN KKKKIRRNTFFTVYIEEKGTKLLKQGNFKALERDRRLGGLFSFVKTPSK AESKSNTISKLRVEYELGEYQKARIEIIKDMLALEKTLIDKYNSLDTDNF NKMLTDWLELKGEPDKASFQNDVDLLIAVRNAFSHNQYPMRNRIAFA NINPFSLSSANTSEEKGLGIANQLKDKTHKTIEKIIEIEKPIETKE |
| SEQ ID NO: 163 | Prevotella buccae Cas 13b | MQKQDKLFVDRKKNAIFAFPKYITIMENKEKPEPIYYELTDKHFWAAFL NLARHNVYTTINHINRRLEIAELKDDGYMMGIKGSWNEQAKKLDKKV RLRDLIMKHFPFLEAAAYEMTNSKSPNNKEQREKEQSEALSLNNLKNV LFIFLEKLQVLRNYYSHYKYSEESPKPIFETSLLKNMYKVFDANVRLVK RDYMHHENIDMQRDFTHLNRKKQVGRTKNIIDSPNFHYHFADKEGNM TIAGLLFFVSLFLDKKDAIWMQKKLKGFKDGRNLREQMTNEVFCRSRIS LPKLKLENVQTKDWMQLDMLNELVRCPKSLYERLREKDRESFKVPFDI FSDDYNAEEEPFKNTLVRHQDRFPYFVLRYFDLNEIFEQLRFQIDLGTYH FSIYNKRIGDEDEVRHLTHHLYGFARIQDFAPQNQPEEWRKLVKDLDHF ETSQEPYISKTAPHYHLENEKIGIKFCSAHNNLFPSLQTDKTCNGRSKFN LGTQFTAEAFLSVHELLPMMFYYLLLTKDYSRKESADKVEGIIRKEISNI YAIYDAFANNEINSIADLTRRLQNTNILQGHLPKQMISILKGRQKDMGK EAERKIGEMIDDTQRRLDLLCKQTNQKIRIGKRNAGLLKSGKIADWLVN DMMRFQPVQKDQNNIPINNSKANSTEYRMLQRALALFGSENFRLKAYF NQMNLVGNDNPHPFLAETQWEHQTNILSFYRNYLEARKKYLKGLKPQ NWKQYQHFLILKVQKTNRNTLVTGWKNSFNLPRGIFTQPIREWFEKHN NSKRIYDQILSFDRVGFVAKAIPLYFAEEYKDNVQPFYDYPFNIGNRLKP KKRQFLDKKERVELWQKNKELFKNYPSEKKKTDLAYLDFLSWKKFER ELRLIKNQDIVTWLMFKELFNMATVEGLKIGEIHLRDIDTNTANEESNNI LNRIMPMKLPVKTYETDNKGNILKERPLATFYIEETETKVLKQGNFKAL VKDRRLNGLFSFAETTDLNLEEHPISKLSVDLELIKYQTTRISIFEMTLGL EKKLIDKYSTLPTDSFRNMLERWLQCKANRPELKNYVNSLIAVRNAFS HNQYPMYDATLFAEVKKFTLFPSVDTKKIELNIAPQLLEIVGKAIKEIEK SENKN |
| SEQ ID NO: 164 | Porphyromo nas gingivalis Cas 13b | MNTVPASENKGQSRTVEDDPQYFGLYLNLARENLIEVESHVRIKFGKK KLNEESLKQSLLCDHLLSVDRWTKVYGHSRRYLPFLHYFDPDSQIEKD HDSKTGVDPDSAQRLIRELYSLLDFLRNDFSHNRLDGTTFEHLEVSPDIS SFITGTYSLACGRAQSRFAVFFKPDDFVLAKNRKEQLISVADGKECLTV SGFAFFICLFLDREQASGMLSRIRGFKRTDENWARAVHETFCDLCIRHP HDRLESSNTKEALLLDMLNELNRCPRILYDMLPEEERAQFLPALDENSM NNLSENSLDEESRLLWDGSSDWAEALTKRIRHQDRFPYLMLRFIEEMDL LKGIRFRVDLGEIELDSYSKKVGRNGEYDRTITDHALAFGKLSDFQNEE EVSRMISGEASYPVRFSLFAPRYAIYDNKIGYCHTSDPVYPKSKTGEKR ALSNPQSMGFISVHDLRKLLLMELLCEGSFSRMQSDFLRKANRILDETA EGKLQFSALFPEMRHRFIPPQNPKSKDRREKAETTLEKYKQEIKGRKDK LNSQLLSAFDMDQRQLPSRLLDEWMNIRPASHSVKLRTYVKQLNEDCR LRLRKFRKDGDGKARAIPLVGEMATFLSQDIVRMIISEETKKLITSAYYN EMQRSLAQYAGEENRRQFRAIVAELRLLDPSSGHPFLSATMETAHRYTE GFYKCYLEKKREWLAKIFYRPEQDENTKRRISVFFVPDGEARKLLPLLIR RRMKEQNDLQDWIRNKQAHPIDLPSHLFDSKVMELLKVKDGKKKWNE AFKDWWSTKYPDGMQPFYGLRRELNIHGKSVSYIPSDGKKFADCYTHL MEKTVRDKKRELRTAGKPVPPDLAADIKRSFHRAVNEREFMLRLVQED DRLMLMAINKMMTDREEDILPGLKNIDSILDEENQFSLAVHAKVLEKE GEGGDNSLSLVPATIEIKSKRKDWSKYIRYRYDRRVPGLMSHFPEHKAT LDEVKTLLGEYDRCRIKIFDWAFALEGAIMSDRDLKPYLHESSSREGKS GEHSTLVKMLVEKKGCLTPDESQYLILIRNKAAHNQFPCAAEMPLIYRD VSAKVGSIEGSSAKDLPEGSSLVDSLWKKYEMIIRKILPILDPENRFFGKL LNNMSQPINDL |
| SEQ ID NO: 165 | Bacteroides pyogenes Cas13b | MESIKNSQKSTGKTLQKDPPYFGLYLNMALLNVRKVENHIRKWLGDV ALLPEKSGFHSLLTTDNLSSAKWTRFYYKSRKFLPFLEMFDSDKKSYEN RRETAECLDTIDRQKISSLLKEVYGKLQDIRNAFSHYHIDDQSVKHTALI ISSEMHRFIENAYSFALQKTRARFTGVFVETDFLQAEEKGDNKKFFAIG GNEGIKLKDNALIFLICLFLDREEAFKFLSRATGFKSTKEKGFLAVRETF CALCCRQPHERLLSVNPREALLMDMLNELNRCPDILFEMLDEKDQKSF LPLLGEEEQAHILENSLNDELCEAIDDPFEMIASLSKRVRYKNRFPYLML RYIEEKNLLPFIRFRIDLGCLELASYPKKMGEENNYERSVTDHAMAFGR LTDFHNEDAVLQQITKGITDEVRFSLYAPRYAIYNNKIGFVRTSGSDKIS FPTLKKKGGEGHCVAYTLQNTKSFGFISIYDLRKILLLSFLDKDKAKNIV SGLLEQCEKHWKDLSENLFDAIRTELQKEFPVPLIRYTLPRSKGGKLVSS KLADKQEKYESEFERRKEKLTEILSEKDFDLSQIPRRMIDEWLNVLPTSR EKKLKGYVETLKLDCRERLRVFEKREKGEHPLPPRIGEMATDLAKDIIR MVIDQGVKQRITSAYYSEIQRCLAQYAGDDNRRHLDSIIRELRLKDTKN GHPFLGKVLRPGLGHTEKLYQRYFEEKKEWLEATFYPAASPKRVPRFV NPPTGKQKELPLIIRNLMKERPEWRDWKQRKNSHPIDLPSQLFENEICRL LKDKIGKEPSGKLKWNEMFKLYWDKEFPNGMQRFYRCKRRVEVFDKV VEYEYSEEGGNYKKYYEALIDEVVRQKISSSKEKSKLQVEDLTLSVRRV FKRAINEKEYQLRLLCEDDRLLFMAVRDLYDWKEAQLDLDKIDNMLG EPVSVSQVIQLEGGQPDAVIKAECKLKDVSKLMRYCYDGRVKGLMPYF ANHEATQEQVEMELRHYEDHRRRVFNWVFALEKSVLKNEKLRRFYEE SQGGCEHRRCIDALRKASLVSEEEYEFLVHIRNKSAHNQFPDLEIGKLPP NVTSGFCECIWSKYKAIICRIIPFIDPERRFFGKLLEQK |
| SEQ ID NO: 166 | Cas13c | MTEKKSIIFKNKSSVEIVKKDIFSQTPDNMIRNYKITLKISEKNPRVVEAE IEDLMNSTILKDGRRSARREKSMTERKLIEEKVAENYSLLANCPMEEVD SIKIYKIKRFLTYRSNMLLYFASINSFLCEGIKGKDNETEEIWHLKDNDV RKEKVKENFKNKLIQSTENYNSSLKNQIEEKEKLLRKESKKGAFYRTIIK KLQQERIKELSEKSLTEDCEKIIKLYSELRHPLMHYDYQYFENLFENKEN SELTKNLNLDIFKSLPLVRKMKLNNKVNYLEDNDTLFVLQKTKKAKTL YQIYDALCEQKNGFNKFINDFFVSDGEENTVFKQIINEKFQSEMEFLEKR ISESEKKNEKLKKKFDSMKAHFHNINSEDTKEAYFWDIHSSSNYKTKYN ERKNLVNEYTELLGSSKEKKLLREEITQINRKLLKLKQEMEEITKKNSLF RLEYKMKIAFGFLFCEFDGNISKFKDEFDASNQEKIIQYHKNGEKYLTYF LKEEEKEKFNLEKMQKIIQKTEEEDWLLPETKNNLFKFYLLTYLLLPYE LKGDFLGFVKKHYYDIKNVDFMDENQNNIQVSQTVEKQEDYFYHKIRL FEKNTKKYEIVKYSIVPNEKLKQYFEDLGIDIKYLTGSVESGEKWLGEN LGIDIKYLTVEQKSEVSEEKIKKFL |
| SEQ ID NO: 167 | Cas13c | MEKDKKGEKIDISQEMIEEDLRKILILFSRLRHSMVHYDYEFYQALYSG KDFVISDKNNLENRMISQLLDLNIFKELSKVKLIKDKAISNYLDKNTTIH VLGQDIKAIRLLDIYRDICGSKNGFNKFINTMITISGEEDREYKEKVIEHF NKKMENLSTYLEKLEKQDNAKRNNKRVYNLLKQKLIEQQKLKEWFGG PYVYDIHSSKRYKELYIERKKLVDRHSKLFEEGLDEKNKKELTKINDEL SKLNSEMKEMTKLNSKYRLQYKLQLAFGFILEEFDLNIDTFINNFDKDK DLIISNFMKKRDIYLNRVLDRGDNRLKNIIKEYKFRDTEDIFCNDRDNNL VKLYILMYILLPVEIRGDFLGFVKKNYYDMKHVDFIDKKDKEDKDTFF HDLRLFEKNIRKLEITDYSLSSGFLSKEHKVDIEKKINDFINRNGAMKLP EDITIEEFNKSLILPIMKNYQINFKLLNDIEISALFKIAKDRSITFKQAIDEI KNEDIKKNSKKNDKNNHKDKNINFTQLMKRALHEKIPYKAGMYQIRN NISHIDMEQLYIDPLNSYMNSNKNNITISEQIEKIIDVCVTGGVTGKELNN NIINDYYMKKEKLVFNLKLRKQNDIVSIESQEKNKREEFVFKKYGLDYK DGEINIIEVIQKVNSLQEELRNIKETSKEKLKNKETLFRDISLINGTIRKNI NFKIKEMVLDIVRMDEIRHINIHIYYKGENYTRSNIIKFKYAIDGENKKY YLKQHEINDINLELKDKFVTLICNMDKHPNKNKQTINLESNYIQNVKFII P |
| SEQ ID NO: 168 | Cas13c | MENKGNNKKIDFDENYNILVAQIKEYFTKEIENYNNRIDNIIDKKELLKY SEKKEESEKNKKLEELNKLKSQKLKILTDEEIKADVIKIIKIFSDLRHSLM HYEYKYFENLFENKKNEELAELLNLNLFKNLTLLRQMKIENKTNYLEG REEFNIIGKNIKAKEVLGHYNLLAEQKNGFNNFINSFFVQDGTENLEFKK LIDEHFVNAKKRLERNIKKSKKLEKELEKMEQHYQRLNCAYVWDIHTS TTYKKLYNKRKSLIEEYNKQINEIKDKEVITAINVELLRIKKEMEEITKSN SLFRLKYKMQIAYAFLEIEFGGNIAKFKDEFDCSKMEEVQKYLKKGVK YLKYYKDKEAQKNYEFPFEEIFENKDTHNEEWLENTSENNLFKFYILTY LLLPMEFKGDFLGVVKKHYYDIKNVDFTDESEKELSQVQLDKMIGDSF FHKIRLFEKNTKRYEIIKYSILTSDEIKRYFRLLELDVPYFEYEKGTDEIGI FNKNIILTIFKYYQIIFRLYNDLEIHGLFNISSDLDKILRDLKSYGNKNINF REFLYVIKQNNNSSTEEEYRKIWENLEAKYLRLHLLTPEKEEIKTKTKEE LEKLNEISNLRNGICHLNYKEIIEEILKTEISEKNKEATLNEKIRKVINFIKE NELDKVELGFNFINDFFMKKEQFMFGQIKQVKEGNSDSITTERERKEKN NKKLKETYELNCDNLSEFYETSNNLRERANSSSLLEDSAFLKKIGLYKV KNNKVNSKVKDEEKRIENIKRKLLKDSSDIMGMYKAEVVKKLKEKLILI FKHDEEKRIYVTVYDTSKAVPENISKEILVKRNNSKEEYFFEDNNKKYV TEYYTLEITETNELKVIPAKKLEGKEFKTEKNKENKLMLNNHYCFNVKI IY |
| SEQ ID NO: 169 | Cas13c | MEEIKHKKNKSSIIRVIVSNYDMTGIKEIKVLYQKQGGVDTFNLKTIINL ESGNLEIISCKPKEREKYRYEFNCKTEINTISITKKDKVLKKEIRKYSLEL YFKNEKKDTVVAKVTDLLKAPDKIEGERNHLRKLSSSTERKLLSKTLCK NYSEISKTPIEEIDSIKIYKIKRFLNYRSNFLIYFALINDFLCAGVKEDDINE VWLIQDKEHTAFLENRIEKITDYIFDKLSKDIENKKNQFEKRIKKYKTSL EELKTETLEKNKTFYIDSIKTKITNLENKITELSLYNSKESLKEDLIKIISIF TNLRHSLMHYDYKSFENLFENIENEELKNLLDLNLFKSIRMSDEFKTKN RTNYLDGTESFTIVKKHQNLKKLYTYYNNLCDKKNGFNTFINSFFVTDG IENTDFKNLIILHFEKEMEEYKKSIEYYKIKISNEKNKSKKEKLKEKIDLL QSELINMREHKNLLKQIYFFDIHNSIKYKELYSERKNLIEQYNLQINGVK DVTAINHINTKLLSLKNKMDKITKQNSLYRLKYKLKIAYSFLMIEFDGD VSKFKNNFDPTNLEKRVEYLDKKEEYLNYTAPKNKFNFAKLEEELQKI QSTSEMGADYLNVSPENNLFKFYILTYIMLPVEFKGDFLGFVKNHYYNI KNVDFMDESLLDENEVDSNKLNEKIENLKDSSFFNKIRLFEKNIKKYEIV KYSVSTQENMKEYFKQLNLDIPYLDYKSTDEIGIFNKNMILPIFKYYQN VFKLCNDIEIHALLALANKKQQNLEYAIYCCSKKNSLNYNELLKTFNRK TYQNLSFIRNKIAHLNYKELFSDLFNNELDLNTKVRCLIEFSQNNKFDQI DLGMNFINDYYMKKTRFIFNQRRLRDLNVPSKEKIIDGKRKQQNDSNN ELLKKYGLSRTNIKDIFNKAWY |
| SEQ ID NO: 170 | Cas13c | MKVRYRKQAQLDTFIIKTEIVNNDIFIKSIIEKAREKYRYSFLFDGEEKYH FKNKSSVEIVKNDIFSQTPDNMIRNYKITLKISEKNPRVVEAEIEDLMNST ILKDGRRSARREKSMTERKLIEEKVAENYSLLANCPIEEVDSIKIYKIKRF LTYRSNMLLYFASINSFLCEGIKGKDNETEEIWHLKDNDVRKEKVKENF KNKLIQSTENYNSSLKNQIEEKEKLSSKEFKKGAFYRTIIKKLQQERIKEL SEKSLTEDCEKIIKLYSELRHPLMHYDYQYFENLFENKENSELTKNLNL DIFKSLPLVRKMKLNNKVNYLEDNDTLFVLQKTKKAKTLYQIYDALCE QKNGFNKFINDFFVSDGEENTVFKQIINEKFQSEMEFLEKRISESEKKNE KLKKKLDSMKAHFRNINSEDTKEAYFWDIHSSRNYKTKYNERKNLVNE YTKLLGSSKEKKLLREEITKINRQLLKLKQEMEEITKKNSLFRLEYKMKI AFGFLFCEFDGNISKFKDEFDASNQEKIIQYHKNGEKYLTSFLKEEEKEK FNLEKMQKIIQKTEEEDWLLPETKNNLFKFYLLTYLLLPYELKGDFLGF VKKHYYDIKNVDFMDENQNNIQVSQTVEKQEDYFYHKIRLFEKNTKK YEIVKYSIVPNEKLKQYFEDLGIDIKYLTGSVESGEKWLGENLGIDIKYL TVEQKSEVSEEKNKKVSLKNNGMFNKTILLFVFKYYQIAFKLFNDIELY SLFFLREKSEKPFEVFLEELKDKMIGKQLNFGQLLYVVYEVLVKNKDLD KILSKKIDYRKDKSFSPEIAYLRNFLSHLNYSKFLDNFMKINTNKSDENK EVLIPSIKIQKMIQFIEKCNLQNQIDFDNFVNDFYMRKEKMFFIQLKQIF PDINSTEKQKKSEKEEILRKRYHLINKKNEQIKDEHEAQSQLYEKILSLQ KIFSCDKNNFYRRLKEEKLLFLEKQGKKKISMKEIKDKIASDISDLLGILK KEITRDIKDKLTEKFRYCEEKLLNISFYNHQDKKKEEGIRVFLIRDKNSD NFKFESILDDGSNKIFISKNGKEITIQCCDKVLETLMIEKNTLKISSNGKIIS LIPHYSYSIDVKY |

In some cases, a suitable programmable nuclease for use in the compositions and methods herein comprises an amino acid sequence having at least 60% amino acid sequence identity to any one of SEQ ID NO: 396 - SEQ ID NO: 423. In some cases, a suitable programmable nuclease for use in the compositions and methods herein comprises an amino acid sequence having at least 80% amino acid sequence identity to any one of SEQ ID NO: 396 - SEQ ID NO: 423. In some cases, a subject programmable nuclease for use in the compositions and methods herein may include an amino acid sequence having 80% or more (e.g., 85% or more, 90% or more, 95% or more, 98% or more, 99% or more, 99.5% or more, or 100%) amino acid sequence identity with the amino acid seauence set forth in any one of SEQ ID NO: 396 - SEQ ID NO: 423. In some cases, a suitable programmable nuclease polypeptide comprises an amino acid sequence having at least 80%, at least 85%, at least 90%, at least 95%, at least 98%, at least 99%, or 100%, amino acid sequence identity to the amino acid sequence set forth in SEQ ID NO: 396. In some cases, a suitable programmable nuclease polypeptide comprises an amino acid sequence having at least 80%, at least 85%, at least 90%, at least 95%, at least 98%, at least 99%, or 100%, amino acid sequence identity to the amino acid sequence set forth in SEQ ID NO: 397. In some cases, a suitable programmable nuclease polypeptide comprises an amino acid sequence having at least 80%, at least 85%, at least 90%, at least 95%, at least 98%, at least 99%, or 100%, amino acid sequence identity to the amino acid sequence set forth in SEQ ID NO: 398. In some cases, a suitable programmable nuclease polypeptide comprises an amino acid sequence having at least 80%, at least 85%, at least 90%, at least 95%, at least 98%, at least 99%, or 100%, amino acid sequence identity to the amino acid sequence set forth in SEQ ID NO: 399. In some cases, a suitable programmable nuclease polypeptide comprises an amino acid sequence having at least 80%, at least 85%, at least 90%, at least 95%, at least 98%, at least 99%, or 100%, amino acid sequence identity to the amino acid sequence set forth in SEQ ID NO: 400. In some cases, a suitable programmable nuclease polypeptide comprises an amino acid sequence having at least 80%, at least 85%, at least 90%, at least 95%, at least 98%, at least 99%, or 100%, amino acid sequence identity to the amino acid sequence set forth in SEQ ID NO: 401. In some cases, a suitable programmable nuclease polypeptide comprises an amino acid sequence having at least 80%, at least 85%, at least 90%, at least 95%, at least 98%, at least 99%, or 100%, amino acid sequence identity to the amino acid sequence set forth in SEQ ID NO: 402. In some cases, a suitable programmable nuclease polypeptide comprises an amino acid sequence having at least 80%, at least 85%, at least 90%, at least 95%, at least 98%, at least 99%, or 100%, amino acid sequence identity to the amino acid sequence set forth in SEQ ID NO: 403. In some cases, a suitable programmable nuclease polypeptide comprises an amino acid sequence having at least 80%, at least 85%, at least 90%, at least 95%, at least 98%, at least 99%, or 100%, amino acid sequence identity to the amino acid sequence set forth in SEQ ID NO: 404. In some cases, a suitable programmable nuclease polypeptide comprises an amino acid sequence having at least 80%, at least 85%, at least 90%, at least 95%, at least 98%, at least 99%, or 100%, amino acid sequence identity to the amino acid sequence set forth in SEQ ID NO: 405. In some cases, a suitable programmable nuclease polypeptide comprises an amino acid sequence having at least 80%, at least 85%, at least 90%, at least 95%, at least 98%, at least 99%, or 100%, amino acid sequence identity to the amino acid sequence set forth in SEQ ID NO: 406. In some cases, a suitable programmable nuclease polypeptide comprises an amino acid sequence having at least 80%, at least 85%, at least 90%, at least 95%, at least 98%, at least 99%, or 100%, amino acid sequence identity to the amino acid sequence set forth in SEQ ID NO: 407. In some cases, a suitable programmable nuclease polypeptide comprises an amino acid sequence having at least 80%, at least 85%, at least 90%, at least 95%, at least 98%, at least 99%, or 100%, amino acid sequence identity to the amino acid sequence set forth in SEQ ID NO: 408. In some cases, a suitable programmable nuclease polypeptide comprises an amino acid sequence having at least 80%, at least 85%, at least 90%, at least 95%, at least 98%, at least 99%, or 100%, amino acid sequence identity to the amino acid sequence set forth in SEQ ID NO: 409. In some cases, a suitable programmable nuclease polypeptide comprises an amino acid sequence having at least 80%, at least 85%, at least 90%, at least 95%, at least 98%, at least 99%, or 100%, amino acid sequence identity to the amino acid sequence set forth in SEQ ID NO: 410. In some cases, a suitable programmable nuclease polypeptide comprises an amino acid sequence having at least 80%, at least 85%, at least 90%, at least 95%, at least 98%, at least 99%, or 100%, amino acid sequence identity to the amino acid sequence set forth in SEQ ID NO: 411. In some cases, a suitable programmable nuclease polypeptide comprises an amino acid sequence having at least 80%, at least 85%, at least 90%, at least 95%, at least 98%, at least 99%, or 100%, amino acid sequence identity to the amino acid sequence set forth in SEQ ID NO: 412. In some cases, a suitable programmable nuclease polypeptide comprises an amino acid sequence having at least 80%, at least 85%, at least 90%, at least 95%, at least 98%, at least 99%, or 100%, amino acid sequence identity to the amino acid sequence set forth in SEQ ID NO: 413. In some cases, a suitable programmable nuclease polypeptide comprises an amino acid sequence having at least 80%, at least 85%, at least 90%, at least 95%, at least 98%, at least 99%, or 100%, amino acid sequence identity to the amino acid sequence set forth in SEQ ID NO: 414. In some cases, a suitable programmable nuclease polypeptide comprises an amino acid sequence having at least 80%, at least 85%, at least 90%, at least 95%, at least 98%, at least 99%, or 100%, amino acid sequence identity to the amino acid sequence set forth in SEQ ID NO: 415. In some cases, a suitable programmable nuclease polypeptide comprises an amino acid sequence having at least 80%, at least 85%, at least 90%, at least 95%, at least 98%, at least 99%, or 100%, amino acid sequence identity to the amino acid sequence set forth in SEQ ID NO: 416. In some cases, a suitable programmable nuclease polypeptide comprises an amino acid sequence having at least 80%, at least 85%, at least 90%, at least 95%, at least 98%, at least 99%, or 100%, amino acid sequence identity to the amino acid sequence set forth in SEQ ID NO: 417. In some cases, a suitable programmable nuclease polypeptide comprises an amino acid sequence having at least 80%, at least 85%, at least 90%, at least 95%, at least 98%, at least 99%, or 100%, amino acid sequence identity to the amino acid sequence set forth in SEQ ID NO: 418. In some cases, a suitable programmable nuclease polypeptide comprises an amino acid sequence having at least 80%, at least 85%, at least 90%, at least 95%, at least 98%, at least 99%, or 100%, amino acid sequence identity to the amino acid sequence set forth in SEQ ID NO: 419. In some cases, a suitable programmable nuclease polypeptide comprises an amino acid sequence having at least 80%, at least 85%, at least 90%, at least 95%, at least 98%, at least 99%, or 100%, amino acid sequence identity to the amino acid sequence set forth in SEQ ID NO: 420. In some cases, a suitable programmable nuclease polypeptide comprises an amino acid sequence having at least 80%, at least 85%, at least 90%, at least 95%, at least 98%, at least 99%, or 100%, amino acid sequence identity to the amino acid sequence set forth in SEQ ID NO: 421. In some cases, a suitable programmable nuclease polypeptide comprises an amino acid sequence having at least 80%, at least 85%, at least 90%, at least 95%, at least 98%, at least 99%, or 100%, amino acid sequence identity to the amino acid sequence set forth in SEQ ID NO: 422. In some cases, a suitable programmable nuclease polypeptide comprises an amino acid sequence having at least 80%, at least 85%, at least 90%, at least 95%, at least 98%, at least 99%, or 100%, amino acid sequence identity to the amino acid sequence set forth in SEQ ID NO: 423.

**Table 20: Amino Acid Sequences of Exemplary Programmable Nucleases**

| **SEQ ID NO:** | **Programmable Nuclease Amino Acid Sequence** |
|---|---|
| **SEQ ID NO: 396** | MAGKKKDKDVINKTLSVRIIRPRYSDDIEKEISDEKAKRKQDGKTGELDR AFFSELKSRNPDIITNDELFPLFTEIQKNLTEIYNKSISLLYMKLIVEEEGGST ASALSAGPYKECKARFNSYISLGLRQKIQSNFRRKELKGFQVSLPTAKSDR FPIPFCHQVENGKGGFKVYETGDDFIFEVPLIKYTATNKKSTSGKNYTKVQ LNNPPVPMNVPLLLSTMRRRQTKKGMQWNKDEGTNAELRRVMSGEYK VSYAEIIRRTRFGKHDDWFVNFSIKFKNKTDELNQNVRGGIDIGVSNPLVC AVTNGLDRYIVANNDIMAFNERAMARRRTLLRKNRFKRSGHGAKNKLEP ITVLTEKNERFRKSILQRWAREVAEFFKRTSASVVNMEDLSGITEREDFFST KLRTTWNYRLMQTTIENKLKEYGIAVNYISPKYTSQTCHSCGKRNDYFTF SYRSENNYPPFECKECNKVKCNADFNAAKNIALKVVL |
| **SEQ ID NO: 397** | MCMKITKIDGISHKKYKEKGKLIKNNDTAKDIIEERFNDIEKKTKELFQKT LDFYVKNYEKCKEQNKERREKAKNYFSKVKILVDNKKITICNENTEKMEI EDFNEYDVRSGKYFNVLNKILNGENYTEEDLEVFENDLQKRTGRIKSIKN SLEENKAHFKKESINNNIIYDRVKGNNKKSLFYEYYRISSKHQEYVNNIFE AFDKLYSNSHEAMNNLFSEITKDSKDRNIRKIREAYHEILNKNKTEFGEEL YKKIQDNRNNFDKLLEIEPEIKELTKSQIFYKYYIDKVNLDETSIKHCFCHL VEIEVNQLLKNYVYSKRNINKEKLENIFEYCKLKNLIKNKLVNKLNNYIR NCGKYNAYISNNDVVVNSEKISEIRTKEAFLRSIIGVSSSAYFSLRNILNTD NTQDITNKVDKEVDKLYQENKKIELEERLKLFFGNYFDINNQQEIKVFLM NIDKIISSIRHEIIHFKMETNAQNIFDFNNVNLGNTAKNIFSNEINEEKIKFKI FKQLNSANVFDYLSNKDITEYMDKVVFSFTNRNVSFVPSFTKIYNRVQDL ANSLEIKKWKIPDKSEGKDAQIYLLKNIYYGKFLDEFLNEENGIFISIKDKII ELNRNQNKRTGFYKLEKFEKIEETNPKKYLEIIQSLYMINIEEIDSEGKNIFL DFIQKIFLKGFFEFIKNNYNYLLELKKIQDKKNIFDSEMSEYIAGEKTLEDI GEINEIIQDIKITEIDKILNQTDKINCFYLLLKLLNYKEITELKGNLEKYQILS KTNVYEKELMLLNIVNLDNNKVKIENFKILAEEIGKFIEKINIEEINKNKKI KTFEELRNFEKGENTGEYYNIYSDDKNIKNIRNLYNIKKYGMLDLLEKISE KTNYCIKKKDLEEYSELRKQLEDEKTNFYKIQEYLHSKYQQKPKKILLKN NKNDYEKYKKSIENIEKYVHLKNKIEFNELNLLQSLLLKILHRLVGFTSIW ERDLRFRLIGEFPDELDVEDIFDHRKRYKGTGKGICKKYDRFINTHTEYKN NNKMENVKFADNNPVRNYIAHFNYLPNPKYSILKMMEKLRKLLDYDRK LKNAVMKSIKDILEEYGFKAEFIINSDKEIILNLVKSVEIIHLGKEDLKSRRN SEDLCKLVKAMLEYSK* |
| **SEQ ID NO: 398** | MEKIKKPSNRNSIPSIIISDYDANKIKEIKVKYLKLARLDKITIQDMEIVDNI VEFKKILLNGVEHTIIDNQKIEFDNYEITGCIKPSNKRRDGRISQAKYVVTI TDKYLRENEKEKRFKSTERELPNNTLLSRYKQISGFDTLTSKDIYKIKRYID FKNEMLFYFQFIEEFFNPLLPKGKNFYDLNIEQNKDKVAKFIVYRLNDDFK NKSLNSYITDTCMIINDFKKIQKILSDFRHALAHFDFDFIQKFFDDQLDKN KFDINTISLIETLLDQKEEKNYQEKNNYIDDNDILTIFDEKGSKFSKLHNFY TKISQKKPAFNKLINSFLSQDGVPNEEFKSYLVTKKLDFFEDIHSNKEYKKI YIQHKNLVIKKQKEESQEKPDGQKLKNYNDELQKLKDEMNTITKQNSLN RLEVKLRLAFGFIANEYNYNFKNFNDEFTNDVKNEQKIKAFKNSSNEKLK EYFESTFIEKRFFHFSVNFFNKKTKKEETKQKNIFNSIENETLEELVKESPLL QIITLLYLFIPRELQGEFVGFILKIYHHTKNITSDTKEDEISIEDAQNSFSLKF KILAKNLRGLQLFHYSLSHNTLYNNKQCFFYEKGNRWQSVYKSFQISHNQ DEFDIHLVIPVIKYYINLNKLMGDFEIYALLKYADKNSITVKLSDITSRDDL KYNGHYNFATLLFKTFGIDTNYKQNKVSIQNIKKTRNNLAHQNIENMLKA FENSEIFAQREEIVNYLQTEHRMQEVLHYNPINDFTMKTVQYLKSLSVHS QKEGKIADIHKKESLVPNDYYLIYKLKAIELLKQKVIEVIGESEDEKKIKN AIAKEEQIKKGNN |
| **SEQ ID NO: 399** | MKDYIRKTLSLRILRPYYGEEIEKEIAAAKKKSQAEGGDGALDNKFWDRL KAEHPEIISSREFYDLLDAIQRETTLYYNRAISKLYHSLIVEREQVSTAKALS AGPYHEFREKFNAYISLGLREKIQSNFRRKELARYQVALPTAKSDTFPIPIY KGFDKNGKGGFKVREIENGDFVIDLPLMAYHRVGGKAGREYIELDRPPAV LNVPVILSTSRRRANKTWFRDEGTDAEIRRVMAGEYKVSWVEILQRKRF GKPYGGWYVNFTIKYQPRDYGLDPKVKGGIDIGLSSPLVCAVTNSLARLTI RDNDLVAFNRKAMARRRTLLRQNRYKRSGHGSANKLKPIEALTEKNELY RKAIMRRWAREAADFFRQHRAATVNMEDLTGIKDREDYFSQMLRCYWN YSQLQTMLENKLKEYGIAVKYIEPKDTSKTCHSCGHVNEYFDFNYRSAH KFPMFKCEKCGVECGADYNAARNIAQA |
| **SEQ ID NO: 400** | MKITKIDGILHKKYIKEGKLVKSTSEENKTDERLSELLTIRLDTYIKNPDNA SEEENRIRRETLKEFFSNKVLYLKDSILYLKDRREKNQLQNKNYSEEDISE YDLKNKNSFLVLKKILLNEDINSEELEIFRNDFEKKLDKINSLKYSLEENK ANYQKINENNIKKVEGKSKRNIFYNYYKDSAKRNDYINNIQEAFDKLYKK EDIENLFFLIENSKKHEKYKIRECYHKIIGRKNDKENFATIIYEEIQNVNNM KELIEKVPNVSELKKSQVFYKYYLNKEKLNDENIKYVFCHFVEIEMSKLL KNYVYKKPSNISNDKVKRIFEYQSLKKLIENKLLNKLDTYVRNCGKYSFY LQDGEIATSDFIVGNRQNEAFLRNIIGVSSTAYFSLRNILETENENDITGRIK GKTVKNKKGEEKYISGEIDKLYDNNKQNEVKKNLKMFYSYDFNMNRKK EIEDFFSNIDEAISSIRHGIVHFNLELEGKDIFTFKNIVPSQISKKMFQNEINE KKLKLKIFRQLNSANVFRYLEKYKILNYLNRTRFEFVNKNIPFVPSFTKLY SRIDDLKNSLCIYWKIPKANDNNKTKEITDAQIYLLKNIYYGEFLNYFMSN NGNFFEIIKEIIELNKNDKRNLKTGFYKLQKFENLQEKTPKEYLANIQSFY MIDAGNKDEEEKDAYIDFIQKIFLKGFMTYLANNGRLSLMYIGNDEQINT SLAGKKQEFDKFLKKYEQNNNIEIPHEINEFVREIKLGKILKYTESLNMFY LILKLLNHKELTNLKGSLEKYQSANKEEAFSDQLELINLLNLDNNRVTEDF ELEADEIGKFLDFNGNKVKDNKELKKFDTNKIYFDGENIIKHRAFYNIKK YGILNLLEKISDEAKYKISIEELKNYSNKKIEIEKNHTTQENLHRKYARPRK DEKFNDEDYKKYEKTIRNIQQYTHLKNKVEFNELNLLQSLLLRILHRLVG YTSIWERDLRFRLKGEFPENQYIEEIFNFDNSKNVKYKNGQIVEKYISFYK ELYKDDMEKISIYSDKKVKELKKEKKDLYIRNYIAHFNYIPNAEVSLLEVL ENLRKLLSYDRKLKNAIMKSIVDILKEYGFVVTFKIEKDKKIRIESLKSEEV VHLKKLKLKDNDKKKEPIKTYRNSKELCKLVKVMFEYKMKEKKSEN* |
| **SEQ ID NO: 401** | MKITKIDGISHKKYIKEGKLVKSTSEENKTDERLSELLTIRLDTYIKNPDNA SEEENRIRRENLKEFFSNKVLYLKDGILYLKDRREKNQLQNKNYSEEDISE YDLKNKNSFLVLKKILLNEDINSEELEIFRKDVEAKLNKINSLKYSFEENK ANYQKINENNVEKVGGKSKRNIIYDYYRESAKRNDYINNVQEAFDKLYK KEDIEKLFFLIENSKKHEKYKIRECYHKIIGRKNDKENFAKIIYEEIQNVNNI KELIEKVPDMSELKKSQVFYKYYLDKEELNDKNIKYAFCHFVEIEMSQLL KNYVYKRLSNISNDKIKRIFEYQNLKKLIENKLLNKLDTYVRNCGKYNYY LQDGEIATSDFIAGNRQNEAFLRNIIGVSSVAYFSLRNILETENKDDITGKM RGKTRIDSKTGEEKYIPGEVDQIYYENKQNEVKNKLKMFYGYDFDMDN KKEIEDFFANIDEAISSIRHGIVHFNLDLDGKDIFAFKNIVPSEISKKMFQNE INEKKLKLKIFRQLNSANVFRYLEKYKILNYLKRTRFEFVNKNIPFVPSFTK LYSRIDDLKNSLGIYWKTPKTNDDNKTKEIIDAQIYLLKNIYYGEFLNYFM SNNGNFFEISREIIELNKNDKRNLKTGFYKLQKFEDIQEKTPKKYLANIQSL YMINAGNQDEEEKDTYIDFIQKIFLKGFMTYLANNGRLSLMYIGNDEQIN TSLAGKKQEFDKFLKKYEQNNNIEIPHEINEFLREIKLGKILKYTESLNMF YLILKLLNHKELTNLKGSLEKYQSANKEETFSDELELINLLNLDNNRVTED FELEANEIGKFLDFNGNKIKDRKELKKFDTKKIYFDGENIIKHRAFYNIKK YGMLNLLEKIADKAKYKISLKELKEYSNKKNEIEKNYTMQQNLHRKYAR PKKDEKFNDEDYKEYEKAIGNIQKYTHLKNKVEFNELNLLQGLLLKILHR LVGYTSIWERDLRFRLKGEFPENQYIEEIFNFDNSKNVKYKSGQIVEKYIN FYKELYKDNVEKRSIYSDKKVKKLKQEKKDLYIRNYIAHFNYIPHAEISLL EVLENLRKLLSYDRKLKNAIMKSVVDILKEYGFVATFKIGADKKIGIQTLE SEKIVHLKNLKKKKLMTDRNSEELCKLVKVMFEYKMEEKNLKTKKCKVI * |
| **SEQ ID NO: 402** | MKNQNTLPSNPTDILKDKPFWAAFFNLARHNVYLTVNHINKLLDLEKLY NKDKHKEIFEHEDIFNISDDVMNDVNSNGKKRKLDIKKIWANLDTDLTRK YQLRELILKHFPFIQPAIIGAQTKERTTIDKDKRSTSTSNDSLKPTGEGDIND PLSLSNVKSIFFRLLQMLEQLRNYYSHVKHSKSATMPNFDEGLLKSMYNI FIDSVNKVKEDYSSNSVIDPNTSFSHLISKDEQGEIKPCRYSFTSKDGSINAS GLLFFVSLFLEKQDSIWMQKKIPGFKKTSENYMKMTNEVFCRNHILLPKM RLETVYDKDWMLLDMLNEVVRCPLSLYKRLAPADQNKFKVPEKSSDNA NRQEDDNPFSRILVRHQNRFPYFALRFFDLNEVFTTLRFQINLGCYHFAIC KKQIGDKKEVHHLTRTLYGFSRLQNFTQNTRPEEWNTLVKTTEPSSGNDG KTVQGVPLPYISYTIPHYQIENEKIGIKIFDGDTAVDTDIWPSVSTEKQLNK PDKYTLTPGFKADVFLSVHELLPMMFYYQLLLCEGMLKTDAGNAVEKVL IDTRNAIFNLYDAFVQEKINTITDLENYLQDKPILIGHLPKQMIDLLKGHQR DMLKAVEQKKAMLIKDTERRLERLNKQPEQKPNVAAKNTGTLLRNGQIA DWLVKDMMRFQPVKRDKEGNPINCSKANSTEYQMLQRAFAFYTTDSYR LPRYFEQLHLINCDNSHLFLSRFEYDKQPNLIAFYAAYLEAKLEFLNELQP QNWASDNYFLLLRAPKNDRQKLAEGWKNGFNLPRGLFTEKIKTWFNEH KTIVDISDCDIFKNRVGQVARLIPVFFDKKFKDHSQPFYTYNFNVGNVSKI TEANYLSKEKRENLFKSYQNKFKNNIPAEKTKEYREYKNFSSWKKFEREL RLIKNQDILTWLMCKNLFDEKIKPKKDILEPRIAVSYIKLDSLQTNTSTAGS LNALAKVVPMTLAIHIDSPKPKGKAGNNEKENKEFTVYIKEEGTKLLKW GNFKTLLADRRIKGLFSYIEHDDINLEKYPLTKYQVDSELDLYQKYRIDIF KQTLDLEAQLLDKYSDLNTDNFNQMLSGWSEKEGIPRNIKQDVAFLIGVR NGFSHNQYPDSKRIAFSRIKKFNPKTSSLQESEGLNIAKQMYEEAQQVVN KIKNIESFD* |
| **SEQ ID NO: 403** | MKVTKIDGISHKKFEDEGKLVKFTGHFNIKNEMKERLEKLKELKLSNYIK NPENVKNKDKNKEKETKSRRENLKKYFSEIILRKKEEKYLLKKTRKFKNI TEEINYDDIKKRENQQKIFDVLKELLEQRINENDKEEILNFDSVKLKEAFE EDFIKKELKIKAIEESLEKNRADYRKDYVELENEKYEDVKGQNKRSLVFE YYKNPENREKFKENIKYAFENLYTEENIKNLYSEIKEIFEKVHLKSKVRYF YQNEIIGESEFSEKDEEGISILYKQIINSVEKKEKFIEFLQKVKIKDLTRSQIF YKYFLENEELNDENIKYVFSYFVEIEVNKLLKENVYKTKKFNEGNKYRV KNIFNYDKLKNLVVYKLENKLNNYVRNCGKYNYHMENGDIATSDINMK NRQTEAFLRSILGVSSFGYFSLRNILGVNDDDFYKIEKDERKNENFILKKA KEDFTSKNIFEKVVDKSFEKKGIYQIKENLKMFYGNSFDKVDKDELKKFF VNMLEAITSVRHRIVHYNINTNSENIFDFSNIEVSKLLKNIFEKEIDTRELKL KIFRQLNSAGVFDYWESWVIKKYLENVKFEFVNKNVPFVPSFKKLYDRID NLKGWNALKLGNNINIPKRKEAKDSQIYLLKNIYYGEFVEKFVNDNKNF EKIVKEIIEINRGAGTNKKTGFYKLEKFETLKANTPTKYLEKLQSLHKISY DKEKIEEDKDVYYVDFVQKIFLKGFVNYLKKLDSLKSLNLLNLRKDETITD KKSVHDEKLKLWENSGSNLSKMPEEIYEYVKKIKISNINYNDRMSIFYLLL KLIDYRELTNLRGNLEKYESMNKNKIYSEELTIINLVNLDNNKVRTNFSLE AEDIGKFLKSSITIKNIAQLNNFSKIFADGENVIKHRSFYNIKKYGILDLLEK IVAKADLKITKEEIKKYENLQNELKRNDFYKIQEQIHRNYNQKPFSIKKIEN KKDFEKYKKVIEKIQDYTQLKNKIEFNDLNLLQSLIFRILHRLAGYTSLWE RDLQFKLKGEFPEDKYIDEIFNSDGNNNQKYKHGGIADKYANFLIEKKEE KSGEILNKKQRKKKIKEDLEIRNYIAHFNYLPNAEKSILEILEELRELLKHD RKLKNAVMKSIKDIFREYGFIVEFTISHTKNGKKIKVCSVKSEKIKHLKNN ELITTRNSEDLCELVKIMLEHKELQK* |
| **SEQ ID NO: 404** | MRISKTLSLRIVRPFYTPEVEAGIKAEKDKREAQGQTRSLDAKFFNELKK KHSEIILSSEFYSLLSEVQRQLTSIYNHAMSNLYHKIIVEGEKTSTSKALSNI GYDECKAIFPSYMALGLRQKIQSNFRRRDLKNFRMAVPTAKSDKFPIPIYR QVDGSKGGFKISENDGKDFIVELPLVDYVAEEVKTAKGRFTKINISKPPKI KNIPVILSTLRRRQSGQWFSDDGTNAEIRRVISGEYKVSWIEIVRRTRFGK HDDWFVNMVIKYDKPEEGLDSKVVGGIDVGVSSPLVCALNNSLDRYFVK SSDIIAFNKRAMARRRTLLRQNKYKRSGHGSKNKLEPITVLTEKNERFKK SIMQRWAKEVAEFFRGKGASVVRMEELSGLKEKDNFFSSYLRMYWNYG QLQQIIENKLKEYGIKVNYVSPKDTSKKCHSCTHINEFFTFEYRQKNNFPL FKCEKCGVECSADYNAAKNMAIA |
| **SEQ ID NO: 405** | VKISKTLSLRIIRPYYTPEVESAIKAEKDKREAQGQTRNLDAKFFNELKKK HPQIILSGEFYSLLFEMQRQLTSIYNRAMSSLYHKIIVEGEKTSTSKALSDIG YDECKSVFPSYIALGLRQKIQSNFRRKELKGFRMAVPTAKSDKFPIPIYKQ VDDGKGGFKISENKEGDFIVELPLVEYTAEDVKTAKGKFTKINISKPPKIK NIPVILSTLRRKQSGQWFSDEGTNAEIRRVISGEYKVSWIEVVRRTRFGKH DDWFLNIVIKYDKTEDGLDPEVVGGIDVGVSTPLVCAVNNSLDRYFVKSS DIIAFKKRAMARRRTLLRQNRFKRSGHGSKSKLEPITILTEKNERFKKSIM QRWAKEVAEFFKGERASVVQMEELSGLKEKDNFFGSYLRMYWNYGQLQ QIIENKLKEYGIKVNYVSPKDTSKKCHSCGYINEFFTFEFRQKNNFPLFKC KKCGVECNADYNAAKNIAIA |
| **SEQ ID NO: 406** | VKLPILKPLHKQILSEEYSTSFKIKAFENDNEVLKAIDTFWNEHIEKSIHPV TGNKFNILSKIENLCDQLQKYKDKDLEKLFIERKNLSTVSHQVYGQWNII RDALRMHLEMNNKNIKEKDIDKYLDNDAFSWKEIKDSIKIYKEHVEDAK ELNENGIIKYFSAMSINEEDDEKEYSISLIKNINEKYNNVKSILQEDRTGKS DLHQDKEKVGIIKEFLDSLKQLQWFLRLLYVTVPLDEKDYEFYNELEVYY EALLPLNSLYNKVRNYMTRKPYSVEKFKLNFNSPTLLDGWDKNKETANL SIILRKNGKYYLGIMNKENNTIFEYYPGTKSNDYYEKMIYKLLPGPNKML PKVFFSKKGLEYYNPPKEILNIYEKGEFKKDKSGNFKKESLHTLIDFYKEA IAKNEDWEVFNFKFKNTKEYEDISQFYRDVEEQGYLITFEKVDANYVDK LVKEGKLYLFQIYNKDFSENKKSKGNPNLHTIYWKGLYDSENLKNVVYK LNGEAEVFYRKKSIDYPEEIYNHGHHKEELLGKFNYPIIKDRRYTQDKFLF HVPITMNFISKEEKRVNQLACEYLSATKEDVHIIGIDRGERHLLYLSLIDKE GNIKKQLSLNTIKNENYDKEIDYRVKLDEKEKKRDEARKNWDVIENIKEL KEGYMSQVIHIIAKMMVEEKAILIMEDLNIGFKRGRFKVEKQVYQKFEK MLIDKLNYLVFKNKNPLEPGGSLNAYQLTSKFDSFKKLGKQSGFIFYVPSA YTSKIDPTTGFYNFIQVDVPNLEKGKEFFSKFEKIIYNTKEDYFEFHCKYG KFVSEPKNKDNDRKTKESLTYYNAIKDTVWVVCSTNHERYKIVRNKAGY YESHPVDVTKNLKDIFSQANINYNEGKDIKPIIIESNNAKLLKSIAEQLKLIL AMRYNNGKHGDDEKDYILSPVKNKQGKFFCTLDGNQTLPINADANGAY NIALKGLLLIEKIKKQQGKIKDLYISNLEWFMFMMSR |
| **SEQ ID NO: 407** | MIKNPSNRHSLPKVIISEVDHEKILEFKIKYEKLARLDRFEVKAMHYEGKE IVFDEVLVNGGLIEVEYQDDNKTLFVKVGEKSYSIRGKKVGGKQRLLEDR VSKTKVQLELSDGVVDNKGNLRKSRTERELIVADNIKLYSQIVGREVTTT KEIYLVKRFLAYRSDLLFYYSFVDNFFKVAGNEKELWKINFDDATSAQFM GYIPFMVNDNLKNDNAYLKDYVRNDVQIKDDLKKVQTIFSALRHTLLHF NYEFFEKLFNGEDVGFDFDIGFLNLLIENIDKLNIDAKKEFIDNEKIRLFGE NLSLAKVYRLYSDICVNRVGFNKFINSMLIKDGVENQVLKAEFNRKFGGN AYTIDIHSNQEYKRIYNEHKKLVIKVSTLKDGQAIRRGNKKISELKEQMKS MTKKNSLARLECKMRLAFGFLYGEYNNYKAFKNNFDTNIKNSQFDVND VEKSKAYFLSTYERRKPRTREKLEKVAKDIESLELKTVIANDTLLKFILLM FVFMPQELKGDFLGFVKKYYHDVHSIDDDTKEQEEDVVEAMSTSLKLKI LGRNIRSLTLFKYALSSQVNYNSTDNIFYVEGNRYGKIYKKLGISHNQEEF DKTLVVPLLRYYSSLFKLMNDFEIYSLAKANPTAVSLQELVDDETSPYKQ GNYFNFNKMLRDIYGLTSDEIKSGQVVFMRNKIAHFDTEVLLSKPLLGQT KMNLQRKDIVSFIEARGDIKELLGYDAINDFRMKVIHLRTKMRVYSDKLQ TMMDLLRNAKTPNDFYNVYKVKGVESINKHLLEVLAQTAEERTVEKQIR DGNEKYDL |
| **SEQ ID NO: 408** | LNSIEKIKKPSNRNSIPSIIISDYDENKIKEIKVKYLKLARLDKITIQDMEIRD NIVEFKKILLNGIEHTIKDNQKIEFDNYEITAYVRASKQRRDGKITQAKYV VTITDKYLRDNEKEKRFKSTERELPNDTLLMRYKQISGFDTLTSKDIYKIK RYIDFKNEMLFYFQFIEEFFSPLLPKGTNFYSLNIEQNKDKVVKYIVYRLN DDFKNQSLNQFIKKTDTIKYDFLKIQKILSDFRHALAHFDFDFIQKFFDDEL DKNRFDISTISLIKTMLQEKEEKYYQEKNNYIEDSDTLTLFDEKESNFSKIH NFYIKISQKKPAFNKLINSFLSKDGVPNEELKSYLATKKIDFFEDIHSNKEY KKIYIKHKNLVVEKQKEESQEKPNGQKLKNYNDELQKLKDEMNKITKQN SLNRLEVKLRLAFGFIANEYNYNFKNFNDKFTLDVKKEQKIKVFKNSSNE KLKEYFESTFIEKRFFHFCVKFFNKKTKKEETKQKNIFNLIENETLEELVKE SPLLQIITLLYLFIPKELQGEFVGFILKIYHHTKNITNDTKEDEKSIEDTQNSF SLKLKILAKNLRGLQLFNYSLSHNTLYNTKEHFFYEKGNRWQSVYKSLEI SHNQDEFDIHLVIPVIKYYINLNKLIGDFEIYALLTYADKNSITEKLSDITKR DDLKFRGYYNFSTLLFKTFMINTNYEQNQKSTQYIKQTRNDIAHQNIENM LKAFENNEIFAQREEIVNYLQKEHKMQEILHYNPINDFTMKTVQYLKSLNI HSQKESKIADIHKKESLVPNDYYLIYKLKVIELLKQKVIEAIGETKDEEKIK NAIAKEEQIKKGYNK |
| **SEQ ID NO: 409** | MLKHKRKNKNSLARVVLSNYDSNNIYEIKIKYEKLAKLDKINIIEMDYDA DNNVMFKKVLFNNKEIDLSHKDKTKINIELDNKKYNISAKKQIGKTHLVV RNKQTSKISRIKKIQDTYYRGKDVFILDNNIEILDKKQTKDKFIVTLNDITN NKTTSTEAELIDDTKDIFKKISAKKDLKSSDIYKIKRFISIRSNFSFYYTFVD NYFKIFHAKKDKNKEELYKIKFKDEINIKPYLENILDNMKNKNGILYNYAN DRKKVLNDLRNIQYVFKEFRHKLAHFDYNFLDNFFSNSVEEKYKQKVNE IKLLDILLDNIDSLNVVPKQNYIEDETISVFDAKDIKLKRLYTYYIKLTINYP GFKKLINSFFIQDGIENQELKEYINNKEKDTQVLKELDNKAYYMDISQYR KYKNIYNKHKELVSEKELSSDGKKINSLNQKINKLKIDMKNITKPNALNR LIYRLRVAFGFIYKEYATINNFNKSFLQDTKTKRFENISQQDIKSYLDISYQD KGKFFVKSKKTFKNKTTVKYTFEDLDLTLNEIITQDDIFVKVIFLFSIFMPK ELNGDFFGFINMYYHKMKNISYDTKDIDMLDTISQNMKLKILEQNIKKTY VFKYYLDLDSSIYSKLVQNIKITEDIDSKKYLYAKIFKYYQHLYKLISDVEI YLLYKYNSKENLSITIDKDELKHRGYYNFQSLLIKNNINKDDAYWSIVNM RNNLSHQNIDELVGHFCKGCLRKSTTDIAELWLRKDILTITNEIINKIESFK DIKITLGYDCVNDFTQKVKQYKQKLKASNERLAKKIEEKQNQVVDEKNK EELEKNILNMKNIQKINRYILDIL |
| **SEQ ID NO: 410** | MLKHKRKNKNSLARVVLSNYDSNNIYEIKIKYEKLAKLDKINIIEMDYDA DNNVMFKKVLFNNKEIDLSHKDKTKINIELDNKKYNISAKKQIGKTHLVV RDKQTSKISRIKKIQDTYYRGKDVFILDNNIEILDKKQTKDKFIVTLNDITN DKTTSTEAELIDDTKDIFKKISAKKDLKSSDIYKIKRFISIRSNFSFYYTFVD NYFKIFHAKKDKNKEELYKIKFKDEINIKPYLENILDNMKNKNGILYDYAD DREKVLNDLKNIQYVFTEFRHKLAHFDYNFLDNFFSNSVTDQYKQKVNE IKLLDILLDNIDSLNVVPKQNYIEDETISVFDAKDIKLKRLYTYYIKLTINYP GFKKLINSFFIQDGIENQELKEYINNKEKDTQVLKELDNKAYYMDISQYR KYKNIYNKHKELVSEKELSSDGQKINSLNQKINKLKIEMKNITKPNALNR LIYRLRVAFGFIYKEYATINNFNKSFLQDTKIKRFENISQQDIKNYLDISYQD KGKFFVKSKKTFKNKTTIKYTFEDLDLTLNEIITQDDIFVKVIFLFSIFMPKE LNGDFFGFINMYYHKMKNISYDTKDIDMLDTISQNMKLKILEQNIKKTYV FKYYLDLDSSIYSKLVQNIKITEDIDSKKYLYAKIFKYYQHLYKLISDVEIY LLYKYNSKENLSITIDKDELKHRGYYNFQSLLIKNNINKDDAYWSIVNMR NNLSHQNIDELVGHFCKGCLRKSTTDIAELWLRKDILTITNEIINKIESFKDI KITLGYDCVNDFTQKVKQYKQKLKASNERLAKKIEEKQNQVVDEKNKE ELEKKILNMKNIQKINRYILDIL |
| **SEQ ID NO: 411** | MSQLKNPSNKNSLPRIIISDFNEIKINEIKIKYHKLDRLDKIIVKEMEIINNKI FFKKILFNNQIKDINSENIELENYILAGEVKPSNTKIILNRDGKEKSFIVYDG FTFKYKPNDKRISETKTNAKYILTIKDKTRHRESSTQRDILKSSIIETYKQIS GFENITSKDIYTIKRYIDFKNEMMFYYTFIDDFFFPITGKNKQDKKNNFYN YKIKENAKKFISLINYRINDDFKNKNGILYDYLSNKEEIIINDFIHIQTILKDV RHAIAHFNFDFIQKLFDNEQAFNSKFDGIEILNILFNQKQEKYFEAQTNYIE EETIKILDEKELSFKKLHSFYSQICQKKPAFNKLINSFIIQDGIENKELKDYIS QKYNSKFDYYLDIHTCKIYKDIYNQHKKFVADKQFLENQKTDGQKIKKL NDQINQLKTKMNNLTKKNSLKRLEIKFRLAFGFIFTEYQTFKNFNERFIEDI KANKYSTKIELLDYGKIKEYISITHEEKRFFNYKTFNKKTNKNINKTIFQSL EKETFENLVKNDNLIKMMFLFQLLLPRELKGEFLGFILKIYHDLKNIDNDT KPDEKSLSELNISTALKLKILVKNIRQINLFNYTISNNTKYEEKEKRFYEEG NQWKDIYKKLYISHDFDIFDIHLIIPIIKYNINLYKLIGDFEVYLLLKYLERN TNYKTLDKLIEAEELKYKGYYNFTTLLSKAINIALNDKEYHNITHLRNNTS HQDIQNIISSFKNNKLLEQRENIIELISKESLKKKLHFDPINDFTMKTLQLLK SLEVHSDKSEKIENLLKKEPLLPNDVYLLYKLKGIEFIKKELISNIGITKYEE KIQEKIAKGVEK |
| **SEQ ID NO: 412** | MVKNPANRHALPKVIISEVDNNNILEFKIKYEKLARLDKVEVKSMHFDNN KQVVFDEVVINGGLIEPTYEDKHKKLVVTAGEKSYSIVGQKVGGKPRLLE DRVSKTKVQLELTNYVEDKEGKKRVSKTERELIVADNIELYSQIVGREVKT TKEIYLIKRFLEYRSDLLFYYGFVDNFFKVAGNGKELWKIDFTNSDSLHLI EYFKFSINDNLKNDENYLKNYVSDNTKIENDLVKCQNNFNSLRHALMHF DYDFFEKLFNGEDVGFDFDIEFLNIMIDKVDKLNIDTKKEFIDDEEVTLFG EALSLKKLYGLFSHIAINRVAFNKLINSFIIEDGIENKELKDFFNNKKESQAY EIDIHSNAEYKALYVQHKKLVMATSAMTDGDEIAKKNQEISDLKEKMKVI TKENSLARLEHKLRLAFGFIYTEYKDYKTFKKHFDQDIKGAKYKGLNVE KLKEYYETTLKNSKPKTDEKLEDVAKKIDKLSLKELIDDDTLLKFVLLLFI FMPQELKGDFLGFIKKYYHDKKHIDQDTKDKDTEIEELSTGLKLKVLDKN IRSLSILKHSFSFQVKYNRKDKNFYEDGNLHGKFYKKLSISHNQEEFNKSV YAPLFRYYSALYKLINDFEIYALAQHVENHETLADQVNKSQFIQKSYFNFR KLLDNTDSISQSSSYNTLIVMRNDISHLSYEPLFNYPLDERKSYKKKTQKG VKTFHVELLYISRAKIIELISLQTDMKKLLGYDAVNDFNMKVVHLRKRLS VYANKEESIRKMQADAKTPNDFYNIYKVKGVESINQHLLKVIGVTEAEKS IEKQINEGNKKHNT |
| **SEQ ID NO: 413** | MTKKPSNRNSLPKVIINKVDESSILEFKIKYEKLARLDRFEVRSMRYDGDG RIIFDEVVANAGLLDVDYEDDNRTIVVKIENKAYNIYGKKVGGEKRLNGK ISKAKVQLILTDSIRKNANDTHRHSLTERELINKNEVDLYSKIAEREISTTK DIYLVKRFLAYRSDLLLYYAFINHYVRVNGNKKEFWKTEIDDKIIDYFIYTI NDTLKNKEGYLEKYIVDRDQIKKDLEKIKQIFSHLRHKLMHYDFRFFTDL FDGKDVDIKVDNSIQKISELLDIEFLNIVIDKLEKLNIDAKKEFIDDEKITLF GQEIELKKLYSLYAHTSINRVAFNKLINSFLIKDGVENKELKEYFNAHNQG KESYYIDIHQNQEYKKLYIEHKNLVAKLSATTDGKEIAKINRELADKKEQ MKQITKANSLKRLEYKLRLAFGFIYTEYKDYERFKNSFDTDTKKKKFDAI DNAKIIEYFEATNKAKKIEKLEEILKGIDKLSLKTLIQDDILLKFLLLFFTFL PQEIKGEFLGFIKKYYHDITSLDEDTKDKDDEITELPRSLKLKIFSKNIRKLS ILKHSLSYQIKYNKKESSYYEAGNVFNKMFKKQAISHNLEEFGKSIYLPM LKYYSALYKLINDFEIYALYKDMDTSETLSQQVDKQEYKRNEYFNFETLL RKKFGNDIEKVLVTYRNKIAHLDFNFLYDKPINKFISLYKSREKIVNYIKNH DIQAVLKYDAVNDFVMKVIQLRTKLKVYADKEQTIESMIQNTQNPNGFYN IYKVKAVENINRHLLKVIGYTESEKAVEEKIRAGNTSKS |
| **SEQ ID NO: 414** | MIKNPSNRYALPKVIISKIDNQNILEFKIKYKKLSKLDIVKVKSMHYDDRAI IFDEVIVNDGLIDVEYRDNHKTIFVKVGNKSYSISGQKVGGKERLLENRVS KTKVQLELKDKATNRVSKTERELIVDDNIKIYSQIVGRDVKTTKDIYLIKR FLAYRSDLLFYYGFVNNFFHVANNRSEFWKIDFNDSNNSKLIEYFKFTIND HLKNDENYLKDYISDNEKLKNDLIKVKNSFEKIRHALMHFDYDFFVKLF NGEDVGLELDIEFLDIMIDKLDKLNIDTKKEFIDDEKITIFGEELSLAKLYRF YAHTAINRVAFNKLINSFIIENGVENQSLKEYFNQQAGGIAYEIDIHQNREY KNLYNEHKKLVSRVLSISDGQEIAILNQKIAKLKDQMKQITKANSIKRLEY KLRLALGFIYTEYENYEEFKNNFDTDIKNGRFTPKDNDGNKRAFDSRELE QLKGYYEATIQTQKPKTDEKIEEVSKKIDRLSLKSLIADDILLKFILLMFTF MPQELKGEFLGFIKKYYHDTKHIDQDTISDSDDTIETLSIGLKLKILDKNIR SLSILKHSLSFQTKYNKKDRNYYEDGNIHGKFFKKLGISHNQEEFNKSVY APLFRYYSALYKLINDFEIYTLSLHIVGSETLTDQVNKSQFLSGRYFNFRKL LTQSYHINNNSTHSTIFNAVINMRNDISHLSYEPLFDCPLNGKKSYKRKIRN QFKTINIKPLVESRKIIIDFITLQTDMQKVLGYDAVNDFTMKIVQLRTRLKA YANKEQTIQKMITEAKTPNDFYNIYKVQGVEEINKYLLEVIGETQAEKEIR EKIERGNIANF |
| **SEQ ID NO: 415** | MKKSIFDQFVNQYALSKTLRFELKPVGETGRMLEEAKVFAKDETIKKKYE ATKPFFNKLHREFVEEALNEVELAGLPEYFEIFKYWKRYKKKFEKDLQKK EKELRKSVVGFFNAQAKEWAKKYETLGVKKKDVGLLFEENVFAILKERY GNEEGSQIVDESTGKDVSIFDSWKGFTGYFIKFQETRKNFYKDDGTATAL ATRIIDQNLKRFCDNLLIFESIRDKIDFSEVEQTMGNSIDKVFSVIFYSSCLL QEGIDFYNCVLGGETLPNGEKRQGINELINLYRQKTSEKVPFLKLLDKQIL SEKEKFMDEIENDEALLDTLKIFRKSAEEKTTLLKNIFGDFVMNQGKYDL AQIYISRESLNTISRKWTSETDIFEDSLYEVLKKSKIVSASVKKKDGGYAFP EFIALIYVKSALEQIPTEKFWKERYYKNIGDVLNKGFLNGKEGVWLQFLLI FDFEFNSLFEREIIDENGDKKVAGYNLFAKGFDDLLNNFKYDQKAKVVIK DFADEVLHIYQMGKYFAIEKKRSWLADYDIDSFYTDPEKGYLKFYENAY EEIIQVYNKLRNYLTKKPYSEDKWKLNFENPTLADGWDKNKEADNSTVI LKKDGRYYLGLMARGRNKLFDDRNLPKILEGVENGKYEKVVYKYFPDQ AKMFPKVCFSTKGLEFFQPSEEVITIYKNSEFKKGYTFNVRSMQRLIDFYK DCLVRYEGWQCYDFRNLRKTEDYRKNIEEFFSDVAMDGYKISFQDVSES YIKEKNQNGDLYLFEIKNKDWNEGANGKKNLHTIYFESLFSADNIAMNFP VKLNGQAEIFYRPRTEGLEKERIITKKGNVLEKGDKAFHKRRYTENKVFF HVPITLNRTKKNPFQFNAKINDFLAKNSDINVIGVDRGEKQLAYFSVISQR GKILDRGSLNVINGVNYAEKLEEKARGREQARKDWQQIEGIKDLKKGYIS QVVRKLADLAIQYNAIIVFEDLNMRFKQIRGGIEKSVYQQLEKALIDKLTF LVEKEEKDVEKAGHLLKAYQLAAPFETFQKMGKQTGIVFYTQAAYTSRI DPVTGWRPHLYLKYSSAEKAKADLLKFKKIKFVDGRFEFTYDIKSFREQK EHPKATVWTVCSCVERFRWNRYLNSNKGGYDHYSDVTKFLVELFQEYGI DFERGDIVGQIEVLETKGNEKFFKNFVFFFNLICQIRNTNASELAKKDGKD DFILSPVEPFFDSRNSEKFGEDLPKNGDDNGAFNIARKGLVIMDKITKFAD ENGGCEKMKWGDLYVSNVEWDNFVANK |
| **SEQ ID NO: 416** | MFNNFIKKYSLQKTLRFELKPVGETADYIEDFKSEYLKDTVLKDEQRAKD YQEIKTLIDDYHREYIEECLREPVDKKTGEILDFTQDLEDAFSYYQKLKEN PTENRVGWEKEQESLRKKLVTSFVGNDGLFKKEFITRDLPEWLQKKGLW GEYKDTVENFKKFTTYFSGFHENRKNMYTAEAQSTAIANRLMNDNLPKF FNNYLAYQTIKEKHPDLVFRLDDALLQAAGVEHLDEAFQPRYFSRLFAQS GITAFNELIGGRTTENGEKIQGLNEQINLYRQQNPEKAKGFPRFMPLFKQIL SDRETHSFLPDAFENDKELLQALRDYVDAATSEEGMISQLNKAMNQFVT ADLKRVYIKSAALTSLSQELFHFFGVISDAIAWYAEKRLSPKKAQESFLKQ EVYAIEELNQAVVGYIDQLEDQSELQQLLVDLPDPQKPVSSFILTHWQKSQ EPLQAVIAKVEPLFELEELSKNKRAPKHDKDQGGEGFQQVDAIKNMLDA FMEVSHAIKPLYLVKGRKAIDMPDVDTGFYADFAEAYSAYEQVTVSLYNK TRNHLSKKPFSKDKIKINFDAPTLLNGWDLNKESDNKSIILRKDGNFYLAI MHPKHTKVFDCYSASEAAGKCYEKMNYKLLSGANKMLPKVFFSKKGIE TFSPPQEILDLYKNNEHKKGATFKLESCHKLIDFFKRNIPKYKVHPTDNFG WDVFGFHFSPTSSYGDLSGFYREVEAQGYKLWFSDVSEAYINKCVEEGK LFLFQIYNKDFSPNSTGKPNLHTLYWKGLFEPENLKDVVLKLNGEAEIFY RKHSIKHEDKTIHRAKDPIANKNADNPKKQSVFDYDIIKDKRYTQDKFFF HVPISLNFKSQGVVRFNDKINGLLAAQDDVHVIGIDRGERHLLYYTVVNG KGEVVEQGSLNQVATDQGYVVDYQQKLHAKEKERDQARKNWSTIENIK ELKAGYLSQVVHKLAQLIVKHNAIVCLEDLNFGFKRGRFKVEKQVYQKF EKALIDKLNYLVFKERGATQAGGYLNAYQLAAPFESFEKLGKQTGILYYV RSDYTSKIDPATGFVDFLKPKYESMAKSKVFFESFERIQWNQAKGYFEFEF DYKKMCPSRKFGDYRTRWVVCTFGDTRYQNRRNKSSGQWETETIDVTA QLKALFAAYGITYNQEDNIKDAIAAVKYTKFYKQLYWLLRLTLSLRHSVT GTDEDFILSPVADENGVFFDSRKATDKQPKDADANGAYHIALKGLWNLQ QIRQHDWNVEKPKKLNLAMKNEEWFGFAQKKKFRA |
| **SEQ ID NO: 417** | MIKNPSNRYALPKVIISKIDNQNILEFKIKYKKLSKLDIVKVKSMHYDDRAI IFDEVIVNDGLIDVEYRDNHKTIFVKVGNKSYSISGQKVGGKERLLENRVS KTKVQLELKDKATNRVSKTERELIVDDNIKIYSQIVGRDVKTTKDIYLIKR FLAYRSDLLFYYGFVNNFFHVANNRSEFWKIDFNDSNNSKLIEYFKFTIND HLKNDENYLKDYISDNEKLKNDLIKVKNSFEKIRHALMHFDYDFFVKLF NGEDVGLELDIEFLDIMIDKLDKLNIDTKKEFIDDEKITIFGEELSLAKLYRF YAHTAINRVAFNKLINSFIIENGVENQSLKEYFNQQAGGIAYEIDIHQNREY KNLYNEHKKLVSRVLSISDGQEIAILNQKIAKLKDQMKQITKANSIKRLEY KLRLALGFIYTEYENYEEFKNNFDTDIKNGRFTPKDNDGNKRAFDSRELE QLKGYYEATIQTQKPKTDEKIEEVSKKIDRLSLKSLIADDILLKFILLMFTF MPQELKGEFLGFIKKYYHDTKHIDQDTISDSDDTIETLSIGLKLKILDKNIR SLSILKHSLSFQTKYNKKDRNYYEDGNIHGKFFKKLGISHNQEEFNKSVY APLFRYYSALYKLINDFEIYTLSLHIVGSETLTDQVNKSQFLSGRYFNFRKL LTQSYHINNNSTHSTIFNAVINMRNDISHLSYEPLFDCPLNGKKSYKRKIRN QFKTINIKPLVESRKIIIDFITLQTDMQKVLGYDAVNDFTMKIVQLRTRLKA YANKEQTIQKMITEAKTPNDFYNIYKVQGVEEINKYLLEVIGETQAEKEIR EKIERGNIANF |
| **SEQ ID NO: 418** | MIKNPSNRHSLPKVIISEVDHEKILEFKIKYEKLARLDRFEVKAMHYEGKE IVFDEVLVNGGLIEVEYQDDNKTLFVKVGEKSYSIRGKKVGGKQRLLEDR VSKTKVQLELSDGVVDNKGNLRKSRTERELIVADNIKLYSQIVGREVTTT KEIYLVKRFLAYRSDLLFYYSFVDNFFKVAGNEKELWKINFDDATSAQFM GYIPFMVNDNLKNDNAYLKDYVRNDVQIKDDLKKVQTIFSALRHTLLHF NYEFFEKLFNGEDVGFDFDIGFLNLLIENIDKLNIDAKKEFIDNEKIRLFGE NLSLAKVYRLYSDICVNRVGFNKFINSMLIKDGVENQVLKAEFNRKFGGN AYTIDIHSNQEYKRIYNEHKKLVIKVSTLKDGQAIRRGNKKISELKEQMKS MTKKNSLARLECKMRLAFGFLYGEYNNYKAFKNNFDTNIKNSQFDVND VEKSKAYFLSTYERRKPRTREKLEKVAKDIESLELKTVIANDTLLKFILLM FVFMPQELKGDFLGFVKKYYHDVHSIDDDTKEQEEDVVEAMSTSLKLKI LGRNIRSLTLFKYALSSQVNYNSTDNIFYVEGNRYGKIYKKLGISHNQEEF DKTLVVPLLRYYSSLFKLMNDFEIYSLAKANPTAVSLQELVDDETSPYKQ GNYFNFNKMLRDIYGLTSDEIKSGQVVFMRNKIAHFDTEVLLSKPLLGQT KMNLQRKDIVSFIEARGDIKELLGYDAINDFRMKVIHLRTKMRVYSDKLQ TMMDLLRNAKTPNDFYNVYKVKGVESINKHLLEVLAQTAEERTVEKQIR DGNEKYDL |
| **SEQ ID NO: 419** | MEEKMLKSYDYFTKLYSLQKTLRFELKPIGKTLEHIKNSGIIESDETLEEQ YAIVKNIIDKLHRKHIDEALSLVDFTKHLDTLKTFQELYLKRGKTDKEKEE LEKLSADLRKLIVSYLKGNVKEKTQHNLNPIKERFEILFGKELFTNEEFFLL AENEKEKKAIQAFKGFTTYFKGFQENRKNMYSEEGNSTSIAYRIINENLPL FIENIARFQKVMSTIEKTTIKKLEQNLKTELKKHNLPGIFTIEYFNNVLTQE GISRYNTIIGGKTTHEGVKIQGLNEIINLYNQQSKDVKLPILKPLHKQILSEE YSTSFKIKAFENDNEVLKAIDTFWNEHIEKSIHPVTGNKFNILSKIENLCDQ LQKYKDKDLEKLFIERKNLSTVSHQVYGQWNIIRDALRMHLEMNNKNIK EKDIDKYLDNDAFSWKEIKDSIKIYKEHVEDAKELNENGIIKYFSAMSINE EDDEKEYSISLIKNINEKYNNVKSILQEDRTGKSDLHQDKEKVGIIKEFLDS LKQLQWFLRLLYVTVPLDEKDYEFYNELEVYYEALLPLNSLYNKVRNYM TRKPYSVEKFKLNFNSPTLLDGWDKNKETANLSIILRKNGKYYLGIMNKE NNTIFEYYPGTKSNDYYEKMIYKLLPGPNKMLPKVFFSKKGLEYYNPPKE ILNIYEKGEFKKDKSGNFKKESLHTLIDFYKEAIAKNEDWEVFNFKFKNT KEYEDISQFYRDVEEQGYLITFEKVDANYVDKLVKEGKLYLFQIYNKDFS ENKKSKGNPNLHTIYWKGLYDSENLKNVVYKLNGEAEVFYRKKSIDYPE EIYNHGHHKEELLGKFNYPIIKDRRYTQDKFLFHVPITMNFISKEEKRVNQ LACEYLSATKEDVHIIGIDRGERHLLYLSLIDKEGNIKKQLSLNTIKNENYD KEIDYRVKLDEKEKKRDEARKNWDVIENIKELKEGYMSQVIHIIAKMMV EEKAILIMEDLNIGFKRGRFKVEKQVYQKFEKMLIDKLNYLVFKNKNPLE PGGSLNAYQLTSKFDSFKKLGKQSGFIFYVPSAYTSKIDPTTGFYNFIQVDV PNLEKGKEFFSKFEKIIYNTKEDYFEFHCKYGKFVSEPKNKDNDRKTKES LTYYNAIKDTVWVVCSTNHERYKIVRNKAGYYESHPVDVTKNLKDIFSQ ANINYNEGKDIKPIIIESNNAKLLKSIAEQLKLILAMRYNNGKHGDDEKDY ILSPVKNKQGKFFCTLDGNQTLPINADANGAYNIALKGLLLIEKIKKQQGK IKDLYISNLEWFMFMMSR |
| **SEQ ID NO: 420** | MNTQKKEFNPKSFKDFTNLYSLNKTLRFSLTPNKKTAEILEFNKQKEVKCF SNDRKIAGAYQEIKKYLNKLHQEFIQEAMKFFAFSEEELKGFEKEYLNLL NFTDKDNFKKKNKIRNEYEQERKILTIKIATYFSKFKSEKYQSFNLANITG KKVFSILEQKYKEDKKTLKIIHIFKYKPTKDEKKEGEAVNFSTYLTGFNEN RKNFYKSEDKAGQFATRTIDNLAQFIKNKKLFEDKYQKNYSKIGILDEQIK IFNLDYFNNLFLQEGLDEYNGILGNNKGEENKSNEGINQKINIFKQKEKAR LKKEKENFNKSDFPLFKELYKQIGSIRKENDVYVEIKTDKELVEELNNFPK NVENYLKDIQSFYKTFFEKLQNEEYELDKIYLPKSVGTYFSYIAFSDWNK LAFIYNKRYKNEKIKIVEGGDVNVQYRSLEVLKNRIDELKDEDNLNFNKF FIDKLKFNEAKKENNWQNFWFCIEYYINSQFIGGEKNILNKEKNEYEILPF GSLKELKEKYFEAVKKYKEKMVDTESGLTDDEEKEIKETLKNYLDRIKEI ERIAKYFDLKKSFEEIKQEDLDSNFYGEYQKVVDKTNELKIYQYYSEFRN YLTQNNSVEEKIKLNFNSGLLLDGWDLNKEKVKFSIIFQENGKYYLGIINK EKDKTILDKDKHPEIFTKNSDFRKMEYKLFPSPSKMLPKISFSETAKKGDE DVGWSEEIQKIKDEFAEFQEYKKKSKDNWKDEFNRGKLNKLIDYYKQVL EKHSEGYMNTYNFELKDSSKYKNLGEFNDDIARQNYKVKFVGIDKNYID EKVANGELFLFQIYNKDFSEDKKEGSTNNLETIYFKELFSKENLENPVFKL SGGAEMFFRNKIEKKKEKKKLDKDGKPMISKKGEKVVDKRRFSENKILF HLPIEINYGKGKMPNFNKKINEYISKNPENIKIIGIDRGEKHLLYYSIIDQNG NNIESMSLNAVDEFGNFVNPEKLEEYEIDNNGKKERRWKYIVNDKEIKVT NYQRKLDELEKERQKSRQSWQNINKIKNLKKGYISFVVKKIVDLAIENNA IIILEDLNFGFKSFRQKIEKNVYQQFEKALIDKLGFVVDKQKNQRFAPQL SAPFESFQKIGKQTGIVYYVLANNTSKVCPSCQWIKNFYLKYEKKNTIFNL QKNQKLKVFFEQEKNRFRFEYQMSKEYISVYSDVDRQRYDKTKNQNKG GYLEYKNSNQKEIIDKDGVIQKQSITLQLKELFKENHIDLEKEILKQLDNK KEKNSGYTGVYNKFIYLFNLILQIRNAISFREKDYIQCPSCHFDTRKENYL KINDGDGNGAYNIALRGLYLLKGKNGIINNLEKIKLIFSNNDYFQWAKKL KNKK |
| **SEQ ID NO: 421** | MQNKQSFADFTNLYSLSKTLRFELKPIGQTQAMLDENKIFEVDENRKKAY DKTKPYFDRLHREFINESLSNAQLKGISEYFETFKQFRSNQNNKDLKELIN KQQKFLRHQIVTLFDENGKHWATTKYAHLKIKKKNLDILFDEQVFYILKE RYGSEKETQLVDKETGAVTSIFDNWKGFTGYFTKFFETRKNFYKSDGTST ALATRIIDQNLNRFFDNLETFHKIKDKIDVKEVEIFFKLKADNVFSIDFYNQ CLLQNGIDKYNDFLGGQTLENGEKQKGINEIINKYRQDNKDQKLPFLKKL DKQILSEKDRFINEIESKEEFFQVLTEFYQSATVKVTIIKTLLNDFVHNTDK YKLEKIYLTKEAFNTIANKWTDETQIFEDNLDLVLKNKKITAKQDFIPLAYI KEALEVIEKDRKFFKDRYYNDPQIGFFPDQSYWEQFLAILNFEFMTHFQR VAKDKITGKKIELGYFVFEKRIKELLDSDPSLNSQSKIIIKEFADEVLHIFQM AKYFALEKKREWKGDYYQLDDQFYNHIDYGFKDQFYENAYEKIVQPYN KIRNYLTKKPYSDVKWKLNFGNPTLANGWDKNKEADNTAVILKKDGNY YLGVMKKGKNKIFSDQNKEKYKAYNSAYYEKLVYKLFPDPSKMFPKVCF SKKGLNFFQPSEEILRIYKNNEFKKGNTFSISSMQKLIAFYIDCLGLYEGWK HYEFKNIKDVRQYKENIGEFYADVAESGYKLWFEKISEEYITQKNQLGELF LFQIYNKDFAKKTTGRKNLHTIYFEELFSQTNIDNNFPFKLNGQAELFYRP KSLEKIEEKRNFKRSIVNKKRYTQNKIFFHVPITLNRTSENIGRFNVRVNNF LANNSNVNIVGVDRGEKNLAYYSIIKQNGEVLKSGSLNIINGVDYHALLT DRAQRREQERRNWQDVESIKDLKRGYISQVVHELVSLAIKYNAIIVMEDL NMRFKQIRGGIEKSTYQQLEKALIEKLNFLVNKEETDSNQAGNLLNAYQL TAPFKTFKDMGKQTGIIFYTQASYTSKIDPLTGWRPNIYLRYSNAKQAKA DILMFTNIYFSEKKDRFEFTYDLEKIDDKRKDLPIKTEWTVCSNVERFSWE KSLNNNKGGYVHYPIQDSNGEESITSKLKKLFMDFGIDLTDIKTQIESLDT NKKDNANFFRKFIFYFQLICQIRNTQVNKSDDGNDFIFSPVEPFFDSRFAD KFRKNLPKNGDENGAYNIARKGLIILHKISDYFVKEGSTDKISWKDLSISQ TEWDNFTTDK |
| **SEQ ID NO: 422** | MKKEKEFKSFGDFTNLYEISKTLRFELKPVENTQTMLDEADVFGKDKVIK DKYTKTKPFIDKLHREFVDESLKDVSLSGLKKYSEVLENWKKNKKDKDI VKELKKEEERLRKEVVEFFDNTAKKWANEKYKELGLKKKDIGILFEESVF DLLKEKYGEEQDSFLKEEKGDFLKNEKGEKVSIFDEWKGFVGYFTKFQE TRKNFYKNDGTETALATRIIDQNLKRFCDNIDDFKKIKNKIDFSEVEKNFN KTADVFSLDFYNQCLLQKGIDSYNEFIGGKTLENGKKLKGVNELVNEYR QKNKNEKVSFLKLLDKQILSEKEKLSFGIENDEQLLVVLNSFYETAEEKTK ILRTLFGDFVEHNENYDLDKTYISKVAFNTISHKWTNETHKFEELLYGAM KEDKPIGLNYDKKEDSYKFPDFIALGYLKKCLNNLDCDTKFWKEKYYEN NADKKDKDKGFLTGGQNAWDQFLQIFIFEFNQLFNSEAFDNKGKEIKIGY DNFRKDFEEIINQKDFKNDENLKIAIKNFADSVLWIYQMAKYFAIEKKRG WDDDFELSEFYTNPSNGYSLFYDRAYEEIVQKYNDLRNYLTKKPYKEDK WKLNFENPTLANGFDKNKESDNSTVILRKKRKYYLGLMKKGNNKIFEDR NKAEFIRNIESGAYEKMAYKYLPDVAKMIPKCSTQLNEAKNHFRNSADDL EIKKSFSNPLKITKRIFDLNNIQYDKTNVSKKISGDNKGIKIFQKEYYKISG DFDVYKSALNDWIDFCKDFLSKYDSTKDFDFSILRKTKDYKSLDEFYVDV AKITYKISFTPVSESYIDQKNKNGELYLFEIYNQDFAKGKMGAKNLHTLYF ENVFSPENISKNFPIKLNGNAELFFRPKSIESKKEKRNFVREIVNKKRYSED KIFFHCPITLNRETGSIYRFNNYVNNFLSENNINIIGVDRGEKHLAYYSVID KNGVKIGGGSFNEINKVDYAKKLEERAGEREQSRKDWQVVEGIKDLKKG YISQVVRELADLAIKHNAIIVLEDLNMRFKQIRGGIEKSIYQQLEKALIDKL SFLVEKGEKDPNQAGHILKAYQLAAPFTSFKDMGKQTGIVFYTQASYTSK TCPNCGFRKNNNKFYFENNIGKAQDALKKLKTFEYDSENKCFGLSYCLS DFANKEEVEKNKNKKRNNAPYSDIEKKDCFELSTKDAVRYRWHDKNTER GKTFFEGESVYEEKEEKEIGQTKRGLVKEYDISKCLIGLFEKTGLDYKQNL LDKINSGKFDGTFYKNLFNYLNLLFEIRNSISGTEIDYISCPECQFHTDKSK TIKNGDDNGSYNIARKGMIILDKIKQFKKENGSLDKMGWGELFIDLEEWD KFAQKKNNNIIDK |
| **SEQ ID NO: 423** | MKYTDFTGIYSVSKTLRFELIPQGSTVENMKREGILNNDMHRADSYKEM KKLIDEYHKAFIERCLSDFSLKYDDTGKHDSLEEYFFYYEQKRNDKTKKI FEDIQVALRKQISKRFTGDTAFKRLFKKELIKEDLPSFVKNDPVKTELIKEF SDFTTYFQEFHKNRKNMYTSDAKSTAIAYRIINENLPKFIDNINAFDIVAKV PEMQEHFKTIADELRSHLQVGNDIDKMFNLQFFNKVLTQSQLDVYNAVIG GKSEGNKKIQGINEYINLYNQQHKKARLPMLKLLYKQILSDRVAISWLQD EFDNDQDMLDTIEAFYNKLNSNETGVLGEGKLKQILMGLDGYNLDGVF MRNDLQLSEVSQRLCGGWNIIKDAMTSDLKRSVQKKKKETDADFEERVS KLFSAQNSFSIAYINQCLGQAGIRCKIQDYFACLGAKEGENEAETTPDIFD QIAEAYHGAAPILNARPSSHNLAQDIEKVKAIKALLDALKRLQRFVKPLL GRGDEGDKDNFFYGDFMPIWEVLDQLTPLYNKVRNRMTRKPYSQEKIKL NFENSTLLNGWDLNKEHDNTSVILRREGLYYLGIMNKNYNKIFDANNVE TIGDCYEKMIYKLLPGPNKMLPKVFFSKSRVQEFSPSKKILEIWESKSFKK GDNFNLDDCHALIDFYKDSIAKHPDWNKFNFKFSDTQSYTNISDFYRDVN QQGYSLSFTKVSVDYVNRMVDEGKLYLFQIYNKDFSPQSKGTPNMHTLY WRMLFDERNLHNVIYKLNGEAEVFYRKASLRCDRPTHPAHQPITCKNEN DSKRVCVFDYDIIKNRRYTVDKFMFHVPITINYKCTGSDNINQQVCDYLR SAGDDTHIIGIDRGERNLLYLVIIDQHGTIKEQFSLNEIVNEYKGNTYCTNY HSLLEEKEAGNKKARQDWQTIESIKELKEGYLSQVIHKISMLMQRYHAIV VLEDLNGSFMRSRQKVEKQVYQKFEHMLINKLNYLVNKQYDATEPGGL LHALQLTSRMDSFKKLGKQSGFLFYIPAWNTSKIDPVTGFVNLFDTRYCN EAKAKEFFEKFDDISYNDERDWFEFSFDYRHFTNKPTGTRTQWTLCTQGT RVRTFRNPEKSNHWDNEEFDLTQAFKDLFNKYGIDIASGLKARIVNGQLT KETSAVKDFYESLLKLLKLTLQMRNSVTGTDIDYLVSPVADKDGIFFDSRT CGSLLPANADANGAFNIARKGLMLLRQIQQSSIDAEKIQLAPIKNEDWLEF AQEKPYL |

The programmable nuclease can be Cas13. Sometimes the Cas13 can be Cas13a, Cas13b, Cas13c, Cas13d, or Cas13e. In some cases, the programmable nuclease can be Mad7 or Mad2. In some cases, the programmable nuclease can be Cas12. Sometimes the Cas12 can be Cas12a, Cas12b, Cas12c, Cas12d, or Cas12e. In some cases, the programmable nuclease can be Csm1, Cas9, C2c4, C2c8, C2c5, C2c10, C2c9, or CasZ. Sometimes, the Csm1 can also be also called smCms1, miCms1, obCms1, or suCms1. Sometimes Cas13a can also be also called C2c2. Sometimes CasZ can also be called Cas14a, Cas14b, Cas14c, Cas14d, Cas14e, Cas14f, Cas14g, Cas14h, Cas14i, Cas14j, or Cas14k. Sometimes, the programmable nuclease can be a type V CRISPR-Cas system. In some cases, the programmable nuclease can be a type VI CRISPR-Cas system. Sometimes the programmable nuclease can be a type III CRISPR-Cas system. In some cases, the programmable nuclease can be from at least one of *Leptotrichia shahii (Lsh), Listeria seeligeri (Lse), Leptotrichia buccalis (Lbu), Leptotrichia wadeu (Lwa), Rhodobacter capsulatus (Rca), Herbinix hemicellulosilytica (Hhe), Paludibacter propionicigenes (Ppr), Lachnospiraceae bacterium (Lba), [Eubacterium] rectale (Ere), Listeria newyorkensis (Lny), Clostridium aminophilum (Cam), Prevotella sp. (Psm), Capnocytophaga canimorsus (Cca, Lachnospiraceae bacterium (Lba), Bergeyella zoohelcum (Bzo), Prevotella intermedia (Pin), Prevotella buccae (Pbu), Alistipes sp. (Asp), Riemerella anatipestifer (Ran), Prevotella aurantiaca (Pau), Prevotella saccharolytica (Psa), Prevotella intermedia (Pin2), Capnocytophaga canimorsus (Cca), Porphyromonas gulae (Pgu), Prevotella sp. (Psp), Porphyromonas gingivalis (Pig), Prevotella intermedia (Pin3), Enterococcus italicus (Ei), Lactobacillus salivarius (Ls),* or *Thermus thermophilus (Tt).* Sometimes the Cas13 is at least one of *LbuCas13a, LwaCas13a, LbaCas13a, HheCas13a, PprCas13a,* EreCas13a, CamCas13a, or LshCas13a. The trans cleavage activity of the CRISPR enzyme can be activated when the crRNA is complexed with the target nucleic acid. The trans cleavage activity of the CRISPR enzyme can be activated when the guide nucleic acid comprising a tracrRNA and crRNA are complexed with the target nucleic acid. The target nucleic acid can be RNA or DNA.

In some embodiments, a programmable nuclease as disclosed herein is an RNA-activated programmable RNA nuclease. In some embodiments, a programmable nuclease as disclosed herein is a DNA-activated programmable RNA nuclease. In some embodiments, a programmable nuclease is capable of being activated by a target RNA to initiate trans cleavage of an RNA reporter and is capable of being activated by a target DNA to initiate trans cleavage of an RNA reporter, such as a Type VI CRISPR/Cas enzyme (e.g., Cas13). For example, Cas13a of the present disclosure can be activated by a target RNA to initiate trans cleavage activity of the Cas13a for the cleavage of an RNA reporter and can be activated by a target DNA to initiate trans cleavage activity of the Cas13a for trans cleavage of an RNA reporter. An RNA reporter can be an RNA-based reporter molecule. In some embodiments, the Cas13a recognizes and detects ssDNA to initiate transcleavage of RNA reporters. Multiple Cas13a isolates can recognize, be activated by, and detect target DNA, including ssDNA, upon hybridization of a guide nucleic acid with the target DNA. For example, Lbu-Cas13a and Lwa-Cas13a can both be activated to transcollaterally cleave RNA reporters by target DNA. Thus, Type VI CRISPR/Cas enzyme (e.g., Cas13, such as Cas13a) can be DNA-activated programmable RNA nucleases, and therefore, can be used to detect a target DNA using the methods as described herein . DNA-activated programmable RNA nuclease detection of ssDNA can be robust at multiple pH values. For example, target ssDNA detection by Cas13 can exhibit consistent cleavage across a wide range of pH conditions, such as from a pH of 6.8 to a pH of 8.2. In contrast, target RNA detection by Cas13 may exhibit high cleavage activity of pH values from 7.9 to 8.2. In some embodiments, a DNA-activated programmable RNA nuclease that also is capable of being an RNA-activated programmable RNA nuclease, can have DNA targeting preferences that are distinct from its RNA targeting preferences. For example, the optimal ssDNA targets for Cas13a have different properties than optimal RNA targets for Cas13a. As one example, gRNA performance on ssDNA may not necessarily correlate with the performance of the same gRNAs on RNA. As another example, gRNAs can perform at a high level regardless of target nucleotide identity at a 3' position on a target RNA sequence. In some embodiments, gRNAs can perform at a high level in the absence of a G at a 3' position on a target ssDNA sequence. Furthermore, target DNA detected by Cas13 disclosed herein can be directly from organisms, or can be indirectly generated by nucleic acid amplification methods, such as PCR and LAMP or any amplification method described herein. Key steps for the sensitive detection of a target DNA, such as a target ssDNA, by a DNA-activated programmable RNA nuclease, such as Cas13a, can include: (1) production or isolation of DNA to concentrations above about 0.1 nM per reaction for in vitro diagnostics, (2) selection of a target sequence with the appropriate sequence features to enable DNA detection as these features are distinct from those required for RNA detection, and (3) buffer composition that enhances DNA detection. The detection of a target DNA by a DNA-activated programmable RNA nuclease can be connected to a variety of readouts including fluorescence, lateral flow, electrochemistry, or any other readouts described herein. Multiplexing of programmable DNA nuclease, such as a Type V CRISPR-Cas protein, with a DNA-activated programmable RNA nuclease, such as a Type VI protein, with a DNA reporter and an RNA reporter, can enable multiplexed detection of target ssDNAs or a combination of a target dsDNA and a target ssDNA, respectively. Multiplexing of different RNA-activated programmable RNA nucleases that have distinct RNA reporter cleavage preferences can enable additional multiplexing. Methods for the generation of ssDNA for DNA-activated programmable RNA nuclease-based diagnostics can include (1) asymmetric PCR, (2) asymmetric isothermal amplification, such as RPA, LAMP, SDA, etc. (3) NEAR for the production of short ssDNA molecules, and (4) conversion of RNA targets into ssDNA by a reverse transcriptase followed by RNase H digestion. Thus, DNA-activated programmable RNA nuclease detection of target DNA is compatible with the various systems, kits, compositions, reagents, and methods disclosed herein.

### Engineered programmable nucleases

Disclosed herein are non-naturally occurring compositions and systems comprising at least one of an engineered Cas protein and an engineered guide nucleic acid, which may simply be referred to herein as a Cas protein and a guide nucleic acid, respectively. In general, an engineered Cas protein and an engineered guide nucleic acid refer to a Cas protein and a guide nucleic acid, respectively, that are not found in nature. In some instances, systems and compositions comprise at least one non-naturally occurring component. For example, compositions and systems may comprise a guide nucleic acid, wherein the sequence of the guide nucleic acid is different or modified from that of a naturally-occurring guide nucleic acid. In some instances, compositions and systems comprise at least two components that do not naturally occur together. For example, compositions and systems may comprise a guide nucleic acid comprising a repeat region and a spacer region which do not naturally occur together. Also, by way of example, composition and systems may comprise a guide nucleic acid and a Cas protein that do not naturally occur together. Conversely, and for clarity, a Cas protein or guide nucleic acid that is "natural," "naturally-occurring," or "found in nature" includes Cas proteins and guide nucleic acids from cells or organisms that have not been genetically modified by a human or machine.

In some instances, the guide nucleic acid may comprise a non-natural nucleobase sequence. In some instances, the non-natural sequence is a nucleobase sequence that is not found in nature. The non-natural sequence may comprise a portion of a naturally occurring sequence, wherein the portion of the naturally occurring sequence is not present in nature absent the remainder of the naturally-occurring sequence. In some instances, the guide nucleic acid may comprise two naturally occurring sequences arranged in an order or proximity that is not observed in nature. In some instances, compositions and systems comprise a ribonucleotide complex comprising a CRISPR/Cas effector protein and a guide nucleic acid that do not occur together in nature. Engineered guide nucleic acids may comprise a first sequence and a second sequence that do not occur naturally together. For example, an engineered guide nucleic acid may comprise a sequence of a naturally occurring repeat region and a spacer region that is complementary to a naturally occurring eukaryotic sequence. The engineered guide nucleic acid may comprise a sequence of a repeat region that occurs naturally in an organism and a spacer region that does not occur naturally in that organism. An engineered guide nucleic acid may comprise a first sequence that occurs in a first organism and a second sequence that occurs in a second organism, wherein the first organism and the second organism are different. The guide nucleic acid may comprise a third sequence disposed at a 3' or 5' end of the guide nucleic acid, or between the first and second sequences of the guide nucleic acid. For example, an engineered guide nucleic acid may comprise a naturally occurring crRNA and tracrRNA coupled by a linker sequence.

In some instances, compositions and systems described herein comprise an engineered Cas protein that is similar to a naturally occurring Cas protein. The engineered Cas protein may lack a portion of the naturally occurring Cas protein. The Cas protein may comprise a mutation relative to the naturally-occurring Cas protein, wherein the mutation is not found in nature. The Cas protein may also comprise at least one additional amino acid relative to the naturally-occurring Cas protein. For example, the Cas protein may comprise an addition of a nuclear localization signal relative to the natural occurring Cas protein. In certain embodiments, the nucleotide sequence encoding the Cas protein is codon optimized (e.g., for expression in a eukaryotic cell) relative to the naturally occurring sequence.

In some instances, compositions and systems provided herein comprise a multi-vector system encoding a Cas protein and a guide nucleic acid described herein, wherein the guide nucleic acid and the Cas protein are encoded by the same or different vectors. In some embodiments, the engineered guide and the engineered Cas protein are encoded by different vectors of the system.

### Thermostable programmable nuclease

Described herein are various embodiments of thermostable programmable nucleases. In some embodiments, a programmable nuclease is referred to as an effector protein. An effector protein may be thermostable. In some instances, known effector proteins (e.g., Cas12 nucleases) are relatively thermo-sensitive and only exhibit activity (e.g., cis and/or trans cleavage) sufficient to produce a detectable signal in a diagnostic assay at temperatures less than 40° C, and optimally at about 37° C. A thermostable protein may have enzymatic activity, stability, or folding comparable to those at 37 °C. In some instances, the trans cleavage activity (e.g., the maximum trans cleavage rate as measured by fluorescent signal generation) of an effector protein in a trans cleavage assay at 40 °C may be at least 50% of that at 37 °C. In some instances, the trans cleavage activity of an effector protein in a trans cleavage assay at 40 °C may be at least 55% of that at 37 °C. In some instances, the trans cleavage activity of an effector protein in a trans cleavage assay at 40 °C may be at least 60 % of that at 37 °C. In some instances, the trans cleavage activity of an effector protein in a trans cleavage assay at 40 °C may be at least 65% of that at 37 °C. In some instances, the trans cleavage activity of an effector protein in a trans cleavage assay at 40 °C may be at least 70 % of that at 37 °C. In some instances, the trans cleavage activity of an effector protein in a trans cleavage assay at 40 °C may be at least 75% of that at 37 °C. In some instances, the trans cleavage activity of an effector protein in a trans cleavage assay at 40 °C may be at least 80% of that at 37 °C. In some instances, the trans cleavage activity of an effector protein in a trans cleavage assay at 40 °C may be at least 85% of that at 37 °C. In some instances, the trans cleavage activity of an effector protein in a trans cleavage assay at 40 °C may be at least 90% of that at 37 °C. In some instances, the trans cleavage activity of an effector protein in a trans cleavage assay at 40 °C may be at least 95% of that at 37 °C. In some instances, the trans cleavage activity of an effector protein in a trans cleavage assay at 40°C may be at least 100% of that at 37 °C. In some instances, the trans cleavage activity of an effector protein in a trans cleavage assay at 40 °C may be at least 1-fold of that at 37 °C. In some instances, the trans cleavage activity of an effector protein in a trans cleavage assay at 40 °C may be at least 2-fold of that at 37 °C. In some instances, the trans cleavage activity of an effector protein in a trans cleavage assay at 40 °C may be at least 3-fold of that at 37 °C. In some instances, the trans cleavage activity of an effector protein in a trans cleavage assay at 40 °C may be at least 4-fold of that at 37 °C. In some instances, the trans cleavage activity of an effector protein in a trans cleavage assay at 40 °C may be at least 5-fold of that at 37 °C. In some instances, the trans cleavage activity of an effector protein in a trans cleavage assay at 40 °C may be at least 6-fold of that at 37 °C. In some instances, the trans cleavage activity of an effector protein in a trans cleavage assay at 40 °C may be at least 7-fold of that at 37 °C. In some instances, the trans cleavage activity of an effector protein in a trans cleavage assay at 40 °C may be at least 8-fold of that at 37 °C. In some instances, the trans cleavage activity of an effector protein in a trans cleavage assay at 40 °C may be at least 9-fold of that at 37 °C. In some instances, the trans cleavage activity of an effector protein in a trans cleavage assay at 40 °C may be at least 10-fold of that at 37 °C. In some instances, the trans cleavage activity of an effector protein in a trans cleavage assay at 40 °C may be at least 11-fold, at least 12-fold, at least 13-fold, at least 14-fold, at least 15-fold, at least 20-fold, at least 25-fold, at least 30-fold, at least 35-fold, at least 40-fold, at least 45-fold, at least 50-fold or more of that at 37 °C.

In some instances, the trans cleavage activity of an effector protein in a trans cleavage assay at 45 °C may be at least 50 % of that at 37 °C. In some instances, the trans cleavage activity of an effector protein in a trans cleavage assay at 45 °C may be at least 55 % of that at 37 °C. In some instances, the trans cleavage activity of an effector protein in a trans cleavage assay at 45 °C may be at least 60 % of that at 37 °C. In some instances, the trans cleavage activity of an effector protein in a trans cleavage assay at 45 °C may be at least 65 % of that at 37 °C. In some instances, the trans cleavage activity of an effector protein in a trans cleavage assay at 45 °C may be at least 70 % of that at 37 °C. In some instances, the trans cleavage activity of an effector protein in a trans cleavage assay at 45 °C may be at least 75 % of that at 37 °C. In some instances, the trans cleavage activity of an effector protein in a trans cleavage assay at 45 °C may be at least 80 % of that at 37 °C. In some instances, the trans cleavage activity of an effector protein in a trans cleavage assay at 45 °C may be at least 85 % of that at 37 °C. In some instances, the trans cleavage activity of an effector protein in a trans cleavage assay at 45 °C may be at least 90 % of that at 37 °C. In some instances, the trans cleavage activity of an effector protein in a trans cleavage assay at 45 °C may be at least 95 % of that at 37 °C. In some instances, the trans cleavage activity of an effector protein in a trans cleavage assay at 45 °C may be at least 100 % of that at 37 °C. In some instances, the trans cleavage activity of an effector protein in a trans cleavage assay at 45 °C may be at least 1-fold of that at 37 °C. In some instances, the trans cleavage activity of an effector protein in a trans cleavage assay at 45 °C may be at least 2-fold of that at 37 °C. In some instances, the trans cleavage activity of an effector protein in a trans cleavage assay at 45 °C may be at least 3-fold of that at 37 °C. In some instances, the trans cleavage activity of an effector protein in a trans cleavage assay at 45 °C may be at least 4-fold of that at 37 °C. In some instances, the trans cleavage activity of an effector protein in a trans cleavage assay at 45 °C may be at least 5-fold of that at 37 °C. In some instances, the trans cleavage activity of an effector protein in a trans cleavage assay at 45 °C may be at least 6-fold of that at 37 °C. In some instances, the trans cleavage activity of an effector protein in a trans cleavage assay at 45 °C may be at least 7-fold of that at 37 °C. In some instances, the trans cleavage activity of an effector protein in a trans cleavage assay at 45 °C may be at least 8-fold of that at 37 °C. In some instances, the trans cleavage activity of an effector protein in a trans cleavage assay at 45°C may be at least 9-fold of that at 37 °C. In some instances, the trans cleavage activity of an effector protein in a trans cleavage assay at 45 °C may be at least 10-fold of that at 37 °C. In some instances, the trans cleavage activity of an effector protein in a trans cleavage assay at 45°C may be at least 11-fold, at least 12-fold, at least 13-fold, at least 14-fold, at least 15-fold, at least 20-fold, at least 25-fold, at least 30-fold, at least 35-fold, at least 40-fold, at least 45-fold, at least 50-fold or more of that at 37 °C.

In some instances, the trans cleavage activity of an effector protein in a trans cleavage assay at 50 °C may be at least 50 % of that at 37 °C. In some instances, the trans cleavage activity of an effector protein in a trans cleavage assay at 50 °C may be at least 55 % of that at 37 °C. In some instances, the trans cleavage activity of an effector protein in a trans cleavage assay at 50 °C may be at least 60 % of that at 37 °C. In some instances, the trans cleavage activity of an effector protein in a trans cleavage assay at 50 °C may be at least 65 % of that at 37 °C. In some instances, the trans cleavage activity of an effector protein in a trans cleavage assay at 50 °C may be at least 70 % of that at 37 °C. In some instances, the trans cleavage activity of an effector protein in a trans cleavage assay at 50 °C may be at least 75 % of that at 37 °C. In some instances, the trans cleavage activity of an effector protein in a trans cleavage assay at 50 °C may be at least 80 % of that at 37 °C. In some instances, the trans cleavage activity of an effector protein in a trans cleavage assay at 50 °C may be at least 85 % of that at 37 °C. In some instances, the trans cleavage activity of an effector protein in a trans cleavage assay at 50 °C may be at least 90 % of that at 37 °C. In some instances, the trans cleavage activity of an effector protein in a trans cleavage assay at 50 °C may be at least 95 % of that at 37 °C. In some instances, the trans cleavage activity of an effector protein in a trans cleavage assay at 50 °C may be at least 100 % of that at 37 °C. In some instances, the trans cleavage activity of an effector protein in a trans cleavage assay at 50 °C may be at least 1-fold of that at 37 °C. In some instances, the trans cleavage activity of an effector protein in a trans cleavage assay at 50 °C may be at least 2-fold of that at 37 °C. In some instances, the trans cleavage activity of an effector protein in a trans cleavage assay at 50 °C may be at least 3-fold of that at 37 °C. In some instances, the trans cleavage activity of an effector protein in a trans cleavage assay at 50 °C may be at least 4-fold of that at 37 °C. In some instances, the trans cleavage activity of an effector protein in a trans cleavage assay at 50 °C may be at least 5-fold of that at 37 °C. In some instances, the trans cleavage activity of an effector protein in a trans cleavage assay at 50 °C may be at least 6-fold of that at 37 °C. In some instances, the trans cleavage activity of an effector protein in a trans cleavage assay at 50 °C may be at least 7-fold of that at 37 °C. In some instances, the trans cleavage activity of an effector protein in a trans cleavage assay at 50 °C may be at least 8-fold of that at 37 °C. In some instances, the trans cleavage activity of an effector protein in a trans cleavage assay at 50 °C may be at least 9-fold of that at 37 °C. In some instances, the trans cleavage activity of an effector protein in a trans cleavage assay at 50 °C may be at least 10-fold of that at 37 °C. In some instances, the trans cleavage activity of an effector protein in a trans cleavage assay at 50 °C may be at least 11-fold, at least 12-fold, at least 13-fold, at least 14-fold, at least 15-fold, at least 20-fold, at least 25-fold, at least 30-fold, at least 35-fold, at least 40-fold, at least 45-fold, at least 50-fold or more of that at 37 °C.

In some instances, the trans cleavage activity of an effector protein in a trans cleavage assay at 55 °C may be at least 50 % of that at 37 °C. In some instances, the trans cleavage activity of an effector protein in a trans cleavage assay at 55 °C may be at least 55 % of that at 37 °C. In some instances, the trans cleavage activity of an effector protein in a trans cleavage assay at 55 °C may be at least 60 % of that at 37 °C. In some instances, the trans cleavage activity of an effector protein in a trans cleavage assay at 55 °C may be at least 65 % of that at 37 °C. In some instances, the trans cleavage activity of an effector protein in a trans cleavage assay at 55 °C may be at least 70 % of that at 37 °C. In some instances, the trans cleavage activity of an effector protein in a trans cleavage assay at 55 °C may be at least 75 % of that at 37 °C. In some instances, the trans cleavage activity of an effector protein in a trans cleavage assay at 55 °C may be at least 80 % of that at 37 °C. In some instances, the trans cleavage activity of an effector protein in a trans cleavage assay at 55 °C may be at least 85 % of that at 37 °C. In some instances, the trans cleavage activity of an effector protein in a trans cleavage assay at 55 °C may be at least 90 % of that at 37 °C. In some instances, the trans cleavage activity of an effector protein in a trans cleavage assay at 55 °C may be at least 95 % of that at 37 °C. In some instances, the trans cleavage activity of an effector protein in a trans cleavage assay at 55 °C may be at least 100 % of that at 37 °C. In some instances, the trans cleavage activity of an effector protein in a trans cleavage assay at 55 °C may be at least 1-fold of that at 37 °C. In some instances, the trans cleavage activity of an effector protein in a trans cleavage assay at 55 °C may be at least 2-fold of that at 37 °C. In some instances, the trans cleavage activity of an effector protein in a trans cleavage assay at 55 °C may be at least 3-fold of that at 37 °C. In some instances, the trans cleavage activity of an effector protein in a trans cleavage assay at 55 °C may be at least 4-fold of that at 37 °C. In some instances, the trans cleavage activity of an effector protein in a trans cleavage assay at 55 °C may be at least 5-fold of that at 37 °C. In some instances, the trans cleavage activity of an effector protein in a trans cleavage assay at 55 °C may be at least 6-fold of that at 37 °C. In some instances, the trans cleavage activity of an effector protein in a trans cleavage assay at 55 °C may be at least 7-fold of that at 37 °C. In some instances, the trans cleavage activity of an effector protein in a trans cleavage assay at 55 °C may be at least 8-fold of that at 37 °C. In some instances, the trans cleavage activity of an effector protein in a trans cleavage assay at 55 °C may be at least 9-fold of that at 37 °C. In some instances, the trans cleavage activity of an effector protein in a trans cleavage assay at 55 °C may be at least 10-fold of that at 37 °C. In some instances, the trans cleavage activity of an effector protein in a trans cleavage assay at 55 °C may be at least 11-fold, at least 12-fold, at least 13-fold, at least 14-fold, at least 15-fold, at least 20-fold, at least 25-fold, at least 30-fold, at least 35-fold, at least 40-fold, at least 45-fold, at least 50-fold or more of that at 37 °C.

In some instances, the trans cleavage activity of an effector protein in a trans cleavage assay at 60 °C may be at least 50 % of that at 37 °C. In some instances, the trans cleavage activity of an effector protein in a trans cleavage assay at 60 °C may be at least 55 % of that at 37 °C. In some instances, the trans cleavage activity of an effector protein in a trans cleavage assay at 60 °C may be at least 60 % of that at 37 °C. In some instances, the trans cleavage activity of an effector protein in a trans cleavage assay at 60 °C may be at least 65 % of that at 37 °C. In some instances, the trans cleavage activity of an effector protein in a trans cleavage assay at 60 °C may be at least 70 % of that at 37 °C. In some instances, the trans cleavage activity of an effector protein in a trans cleavage assay at 60 °C may be at least 75 % of that at 37 °C. In some instances, the trans cleavage activity of an effector protein in a trans cleavage assay at 60 °C may be at least 80 % of that at 37 °C. In some instances, the trans cleavage activity of an effector protein in a trans cleavage assay at 60 °C may be at least 85 % of that at 37 °C. In some instances, the trans cleavage activity of an effector protein in a trans cleavage assay at 60 °C may be at least 90 % of that at 37 °C. In some instances, the trans cleavage activity of an effector protein in a trans cleavage assay at 60 °C may be at least 95 % of that at 37 °C. In some instances, the trans cleavage activity of an effector protein in a trans cleavage assay at 60 °C may be at least 100 % of that at 37 °C. In some instances, the trans cleavage activity of an effector protein in a trans cleavage assay at 60 °C may be at least 1-fold of that at 37 °C. In some instances, the trans cleavage activity of an effector protein in a trans cleavage assay at 60 °C may be at least 2-fold of that at 37 °C. In some instances, the trans cleavage activity of an effector protein in a trans cleavage assay at 60 °C may be at least 3-fold of that at 37 °C. In some instances, the trans cleavage activity of an effector protein in a trans cleavage assay at 60 °C may be at least 4-fold of that at 37 °C. In some instances, the trans cleavage activity of an effector protein in a trans cleavage assay at 60 °C may be at least 5-fold of that at 37 °C. In some instances, the trans cleavage activity of an effector protein in a trans cleavage assay at 60 °C may be at least 6-fold of that at 37 °C. In some instances, the trans cleavage activity of an effector protein in a trans cleavage assay at 60 °C may be at least 7-fold of that at 37 °C. In some instances, the trans cleavage activity of an effector protein in a trans cleavage assay at 60 °C may be at least 8-fold of that at 37 °C. In some instances, the trans cleavage activity of an effector protein in a trans cleavage assay at 60 °C may be at least 9-fold of that at 37 °C. In some instances, the trans cleavage activity of an effector protein in a trans cleavage assay at 60 °C may be at least 10-fold of that at 37 °C. In some instances, the trans cleavage activity of an effector protein in a trans cleavage assay at 60 °C may be at least 11-fold, at least 12-fold, at least 13-fold, at least 14-fold, at least 15-fold, at least 20-fold, at least 25-fold, at least 30-fold, at least 35-fold, at least 40-fold, at least 45-fold, at least 50-fold or more of that at 37 °C.

In some instances, the trans cleavage activity of an effector protein in a trans cleavage assay at 65 °C may be at least 50 % of that at 37 °C. In some instances, the trans cleavage activity of an effector protein in a trans cleavage assay at 65 °C may be at least 55 % of that at 37 °C. In some instances, the trans cleavage activity of an effector protein in a trans cleavage assay at 65 °C may be at least 60 % of that at 37 °C. In some instances, the trans cleavage activity of an effector protein in a trans cleavage assay at 65 °C may be at least 65 % of that at 37 °C. In some instances, the trans cleavage activity of an effector protein in a trans cleavage assay at 65 °C may be at least 70 % of that at 37 °C. In some instances, the trans cleavage activity of an effector protein in a trans cleavage assay at 65 °C may be at least 75 % of that at 37 °C. In some instances, the trans cleavage activity of an effector protein in a trans cleavage assay at 65 °C may be at least 80 % of that at 37 °C. In some instances, the trans cleavage activity of an effector protein in a trans cleavage assay at 65 °C may be at least 85 % of that at 37 °C. In some instances, the trans cleavage activity of an effector protein in a trans cleavage assay at 65 °C may be at least 90 % of that at 37 °C. In some instances, the trans cleavage activity of an effector protein in a trans cleavage assay at 65 °C may be at least 95 % of that at 37 °C. In some instances, the trans cleavage activity of an effector protein in a trans cleavage assay at 65 °C may be at least 100 % of that at 37 °C. In some instances, the trans cleavage activity of an effector protein in a trans cleavage assay at 65 °C may be at least 1-fold of that at 37 °C. In some instances, the trans cleavage activity of an effector protein in a trans cleavage assay at 65 °C may be at least 2-fold of that at 37 °C. In some instances, the trans cleavage activity of an effector protein in a trans cleavage assay at 65 °C may be at least 3-fold of that at 37 °C. In some instances, the trans cleavage activity of an effector protein in a trans cleavage assay at 65 °C may be at least 4-fold of that at 37 °C. In some instances, the trans cleavage activity of an effector protein in a trans cleavage assay at 65 °C may be at least 5-fold of that at 37 °C. In some instances, the trans cleavage activity of an effector protein in a trans cleavage assay at 65 °C may be at least 6-fold of that at 37 °C. In some instances, the trans cleavage activity of an effector protein in a trans cleavage assay at 65 °C may be at least 7-fold of that at 37 °C. In some instances, the trans cleavage activity of an effector protein in a trans cleavage assay at 65 °C may be at least 8-fold of that at 37 °C. In some instances, the trans cleavage activity of an effector protein in a trans cleavage assay at 65 °C may be at least 9-fold of that at 37 °C. In some instances, the trans cleavage activity of an effector protein in a trans cleavage assay at 65 °C may be at least 10-fold of that at 37 °C. In some instances, the trans cleavage activity of an effector protein in a trans cleavage assay at 65 °C may be at least 11-fold, at least 12-fold, at least 13-fold, at least 14-fold, at least 15-fold, at least 20-fold, at least 25-fold, at least 30-fold, at least 35-fold, at least 40-fold, at least 45-fold, at least 50-fold or more of that at 37 °C. In some instances, the trans cleavage activity of an effector protein in a trans cleavage assay at 70 °C, 75 °C. 80 °C, or more may be at least 50, at least 60 %, at least 65 %, at least 70 %, at least 75 %, at least 80 %, at least 85 %, at least 95 %, at least 100 %, at least 1-fold, at least 2-fold , at least 3-fold , at least 4-fold , at least 5-fold , at least 6-fold , at least 7-fold , at least 8-fold , at least 9-fold , at least 10-fold , at least 11-fold, at least 12-fold, at least 13-fold, at least 14-fold, at least 15-fold, at least 20-fold, at least 25-fold, at least 30-fold, at least 35-fold, at least 40-fold, at least 45-fold, at least 50-fold or more of that at 37 °C.

In some instances, the trans cleavage activity may be measured against a negative control in a trans cleavage assay. In some instances, the trans cleavage activity of an effector protein against a nucleic acid in a trans cleavage assay at 37 °C may be at least 50 %, at least 55 %, at least 60 %, at least 65 %, at least 70 %, at least 75 %, at least 80 %, at least 85 %, at least 90 %, at least 95 %, at least 100 %, at least 1-fold, at least 2-fold, at least 3-fold, at least 4-fold, at least 5-fold, at least 6-fold, at least 7-fold, at least 8-fold, at least 9-fold, or at least 10-fold of that against a negative control nucleic acid. In some instances, the trans cleavage activity of an effector protein against a nucleic acid in a trans cleavage assay at 37 °C may be at least 11-fold, at least 12-fold, at least 13-fold, at least 14-fold, at least 15-fold, at least 20-fold, at least 25-fold, at least 30-fold, at least 35-fold, at least 40-fold, at least 45-fold, at least 50-fold or more of that against a negative control nucleic acid. In some instances, the trans cleavage activity of an effector protein against a nucleic acid in a trans cleavage assay at 40 °C may be at least 50 %, at least 55 %, at least 60 %, at least 65 %, at least 70 %, at least 75 %, at least 80 %, at least 85 %, at least 90 %, at least 95 %, at least 100 %, at least 1-fold, at least 2-fold, at least 3-fold, at least 4-fold, at least 5-fold, at least 6-fold, at least 7-fold, at least 8-fold, at least 9-fold, or at least 10-fold of that against a negative control nucleic acid. In some instances, the trans cleavage activity of an effector protein against a nucleic acid in a trans cleavage assay at 40 °C may be at least 11-fold, at least 12-fold, at least 13-fold, at least 14-fold, at least 15-fold, at least 20-fold, at least 25-fold, at least 30-fold, at least 35-fold, at least 40-fold, at least 45-fold, at least 50-fold or more of that against a negative control nucleic acid. In some instances, the trans cleavage activity of an effector protein against a nucleic acid in a trans cleavage assay at 45 °C may be at least 50 %, at least 55 %, at least 60 %, at least 65 %, at least 70 %, at least 75 %, at least 80 %, at least 85 %, at least 90 %, at least 95 %, at least 100 %, at least 1-fold, at least 2-fold, at least 3-fold, at least 4-fold, at least 5-fold, at least 6-fold, at least 7-fold, at least 8-fold, at least 9-fold, or at least 10-fold of that against a negative control nucleic acid. In some instances, the trans cleavage activity of an effector protein against a nucleic acid in a trans cleavage assay at 45 °C may be at least 11-fold, at least 12-fold, at least 13-fold, at least 14-fold, at least 15-fold, at least 20-fold, at least 25-fold, at least 30-fold, at least 35-fold, at least 40-fold, at least 45-fold, at least 50-fold or more of that against a negative control nucleic acid. In some instances, the trans cleavage activity of an effector protein against a nucleic acid in a trans cleavage assay at 50 °C may be at least 50 %, at least 55 %, at least 60 %, at least 65 %, at least 70 %, at least 75 %, at least 80 %, at least 85 %, at least 90 %, at least 95 %, at least 100 %, at least 1-fold, at least 2-fold, at least 3-fold, at least 4-fold, at least 5-fold, at least 6-fold, at least 7-fold, at least 8-fold, at least 9-fold, or at least 10-fold of that against a negative control nucleic acid. In some instances, the trans cleavage activity of an effector protein against a nucleic acid in a trans cleavage assay at 50 °C may be at least 11-fold, at least 12-fold, at least 13-fold, at least 14-fold, at least 15-fold, at least 20-fold, at least 25-fold, at least 30-fold, at least 35-fold, at least 40-fold, at least 45-fold, at least 50-fold or more of that against a negative control nucleic acid. In some instances, the trans cleavage activity of an effector protein against a nucleic acid in a trans cleavage assay at 55 °C may be at least 50 %, at least 55 %, at least 60 %, at least 65 %, at least 70 %, at least 75 %, at least 80 %, at least 85 %, at least 90 %, at least 95 %, at least 100 %, at least 1-fold, at least 2-fold, at least 3-fold, at least 4-fold, at least 5-fold, at least 6-fold, at least 7-fold, at least 8-fold, at least 9-fold, or at least 10-fold of that against a negative control nucleic acid. In some instances, the trans cleavage activity of an effector protein against a nucleic acid in a trans cleavage assay at 55 °C may be at least 11-fold, at least 12-fold, at least 13-fold, at least 14-fold, at least 15-fold, at least 20-fold, at least 25-fold, at least 30-fold, at least 35-fold, at least 40-fold, at least 45-fold, at least 50-fold or more of that against a negative control nucleic acid. In some instances, the trans cleavage activity of an effector protein against a nucleic acid in a trans cleavage assay at 60 °C may be at least 50 %, at least 55 %, at least 60 %, at least 65 %, at least 70 %, at least 75 %, at least 80 %, at least 85 %, at least 90 %, at least 95 %, at least 100 %, at least 1-fold, at least 2-fold, at least 3-fold, at least 4-fold, at least 5-fold, at least 6-fold, at least 7-fold, at least 8-fold, at least 9-fold, or at least 10-fold of that against a negative control nucleic acid. In some instances, the trans cleavage activity of an effector protein against a nucleic acid in a trans cleavage assay at 60 °C may be at least 11-fold, at least 12-fold, at least 13-fold, at least 14-fold, at least 15-fold, at least 20-fold, at least 25-fold, at least 30-fold, at least 35-fold, at least 40-fold, at least 45-fold, at least 50-fold or more of that against a negative control nucleic acid. In some instances, the trans cleavage activity of an effector protein against a nucleic acid in a trans cleavage assay at 65 °C may be at least 50 %, at least 55 %, at least 60 %, at least 65 %, at least 70 %, at least 75 %, at least 80 %, at least 85 %, at least 90 %, at least 95 %, at least 100 %, at least 1-fold, at least 2-fold, at least 3-fold, at least 4-fold, at least 5-fold, at least 6-fold, at least 7-fold, at least 8-fold, at least 9-fold, or at least 10-fold of that against a negative control nucleic acid. In some instances, the trans cleavage activity of an effector protein against a nucleic acid in a trans cleavage assay at 65 °C may be at least 11-fold, at least 12-fold, at least 13-fold, at least 14-fold, at least 15-fold, at least 20-fold, at least 25-fold, at least 30-fold, at least 35-fold, at least 40-fold, at least 45-fold, at least 50-fold or more of that against a negative control nucleic acid. In some instances, the trans cleavage activity of an effector protein against a nucleic acid in a trans cleavage assay at 70 °C, 75 °C, 80 °C, or more may be at least 50 %, at least 55 %, at least 60 %, at least 65 %, at least 70 %, at least 75 %, at least 80 %, at least 85 %, at least 90 %, at least 95 %, at least 100 %, at least 1-fold, at least 2-fold, at least 3-fold, at least 4-fold, at least 5-fold, at least 6-fold, at least 7-fold, at least 8-fold, at least 9-fold, or at least 10-fold of that against a negative control nucleic acid. In some instances, the trans cleavage activity of an effector protein against a nucleic acid in a trans cleavage assay at 70 °C, 75 °C, 80 °C, or more may be at least 11-fold, at least 12-fold, at least 13-fold, at least 14-fold, at least 15-fold, at least 20-fold, at least 25-fold, at least 30-fold, at least 35-fold, at least 40-fold, at least 45-fold, at least 50-fold or more of that against a negative control nucleic acid.

### Reporters

Described herein are reagents comprising a reporter comprising a detection moiety, wherein the reporter is capable of being cleaved by the activated nuclease, thereby generating a first detectable signal. As used herein, a reporter is used interchangeably with reporter molecule. In some cases, the reporter comprises a single-stranded nucleic acid comprising deoxyribonucleotides. In other cases, the reporter comprises a single-stranded nucleic acid comprising ribonucleotides. The reporter can comprise a single-stranded nucleic acid comprising at least one deoxyribonucleotide and at least one ribonucleotide. In some cases, the reporter comprises a single-stranded nucleic acid comprising at least one ribonucleotide residue at an internal position that functions as a cleavage site. In some cases, the reporter comprises at least 2, 3, 4, 5, 6, 7, 8, 9, or 10 ribonucleotide residues at an internal position. In some cases, the reporter may comprise from 2 to 10, from 3 to 9, from 4 to 8, or from 5 to 7 ribonucleotide residues at an internal position. In some cases, the reporter may comprise from 3 to 10, from 4 to 10, from 5 to 10, from 6 to 10, from 7 to 10, from 8 to 10, from 9 to 10, from 2 to 8, from 3 to 8, from 5 to 8, from 6 to 8, from 7 to 8, from 2 to 5, from 3 to 5, or from 4 to 5 ribonucleotide residues at an internal position. Sometimes the ribonucleotide residues are continuous. Alternatively, the ribonucleotide residues are interspersed in between non-ribonucleotide residues. In some cases, the reporter has only ribonucleotide residues. In some cases, the reporter has only deoxyribonucleotide residues. In some cases, the reporter comprises nucleotides resistant to cleavage by the programmable nuclease described herein. In some cases, the reporter comprises synthetic nucleotides. In some cases, the reporter comprises at least one ribonucleotide residue and at least one non-ribonucleotide residue. In some cases, the reporter is 5-20, 5-15, 5-10, 7-20, 7-15, or 7-10 nucleotides in length. In some cases, the reporter comprises at least one uracil ribonucleotide. In some cases, the reporter comprises at least two uracil ribonucleotides. Sometimes the reporter has only uracil ribonucleotides. In some cases, the reporter comprises at least one adenine ribonucleotide. In some cases, the reporter comprises at least two adenine ribonucleotide. In some cases, the reporter has only adenine ribonucleotides. In some cases, the reporter comprises at least one cytosine ribonucleotide. In some cases, the reporter comprises at least two cytosine ribonucleotide. In some cases, the reporter comprises at least one guanine ribonucleotide. In some cases, the reporter comprises at least two guanine ribonucleotide. A reporter can comprise only unmodified ribonucleotides, only unmodified deoxyribonucleotides, or a combination thereof. In some cases, the reporter is from 5 to12 nucleotides in length. In some cases, the reporter is at least 2, 3, 4, 5, 6, 7, 8, 9, 10, 11, 12, 13, 14, 15, 16, 17, 18, 19, 20, 21, 22, 23, 24, 25, 26, 27, 28, 29, or 30 nucleotides in length. In some cases, the reporter is 2, 3, 4, 5, 6, 7, 8, 9, 10, 11, 12, 13, 14, 15, 16, 17, 18, 19, 20, 21, 22, 23, 24, 25, 26, 27, 28, 29, or 30 nucleotides in length. For cleavage by a programmable nuclease comprising Cas13, a reporter can be 5, 8, or 10 nucleotides in length. For cleavage by a programmable nuclease comprising Cas12, a reporter can be 10 nucleotides in length. A DETECTR reaction can comprise 100 pM to 1 nM of reporters. A DETECTR reaction can comprise 1 nM to 5 nM of reporters. A DETECTR reaction can comprise 5 nM to 20 nM of reporters. A DETECTR reaction can comprise 20 nM to 50 nM of reporters. A DETECTR reaction can comprise 50 nM to 100 nM of reporters. A DETECTR reaction can comprise 100 nM to 250 nM reporters. A DETECTR reaction can comprise 250 nM to 500 nM reporters. A DETECTR reaction can comprise 500 nM to 1000 nM (1 µM) of reporters.

The reporter can comprise a detection moiety (in addition to the nucleic acid) capable of generating a first detectable signal. Sometimes the reporter may comprise a protein capable of generating a signal. A signal can be a colorimetric, potentiometric, amperometric, optical (e.g., fluorescent, colorimetric, etc.), or piezo-electric signal. The generation of the detectable signal from the release of the detection moiety indicates that cleavage by the programmable nuclease has occurred and that the sample contains the target nucleic acid. A detection moiety can be any moiety capable of generating a colorimetric, potentiometric, amperometric, optical (e.g., fluorescent, colorimetric, etc.), or piezo-electric signal. A reporter, sometimes, is protein-nucleic acid that can generate a calorimetric, potentiometric, amperometric, optical (e.g., fluorescent, colorimetric, etc.), or piezo-electric signal upon cleavage of the nucleic acid. Often a calorimetric signal is heat produced after cleavage of the reporters. Sometimes, a calorimetric signal is heat absorbed after cleavage of the reporters. A potentiometric signal, for example, is electrical potential produced after cleavage of the reporters. An amperometric signal can be movement of electrons produced after the cleavage of a reporter. Often, the signal is an optical signal, such as a colorimetric signal or a fluorescence signal. An optical signal is, for example, a light output produced after the cleavage of the reporters. Sometimes, an optical signal is a change in light absorbance between before and after the cleavage of reporters. Often, a piezo-electric signal is a change in mass between before and after the cleavage of the reporter.

Detecting the presence or absence of a target nucleic acid of interest can involve measuring a signal emitted from a detection moiety present in a reporter, after cleavage of the reporter by an activated programmable nuclease. The signal can be measured using one or more sensors integrated with the device or operatively coupled to the device. Thus, the detecting steps disclosed herein can involve measuring the presence of a target nucleic acid, quantifying how much of the target nucleic acid is present, or, measuring a signal indicating that the target nucleic acid is absent in a sample. In some embodiments, a signal is generated upon cleavage of the reporter by the programmable nuclease. In other embodiments, the signal changes upon cleavage of the reporter by the programmable nuclease. In other embodiments, a signal can be present in the absence of reporter cleavage and disappear upon cleavage of the target nucleic acid by the programmable nuclease. For example, a signal can be produced in a microfluidic device or lateral flow device after contacting a sample with a composition comprising a programmable nuclease.

In some cases, the signal can comprise a colorimetric signal or a signal visible by eye. In some instances, the signal is fluorescent, electrical, chemical, electrochemical, or magnetic. A signal can be a calorimetric, potentiometric, amperometric, optical (e.g., fluorescent, colorimetric, etc.), or piezo-electric signal. In some cases, the detectable signal is a colorimetric signal or a signal visible by eye. In some instances, the detectable signal is fluorescent, electrical, chemical, electrochemical, or magnetic. In some cases, the first detection signal is generated by binding of the detection moiety to the capture molecule in the detection region, where the first detection signal indicates that the sample contained the target nucleic acid. Sometimes the system can detect more than one type of target nucleic acid, wherein the system may comprise more than one type of guide nucleic acid and more than one type of reporter. In some cases, the detectable signal is generated directly by the cleavage event. Alternatively, or in combination, the detectable signal is generated indirectly by the signal event. Sometimes the detectable signal is not a fluorescent signal. In some instances, the detectable signal is a colorimetric or color-based signal. In some cases, the detected target nucleic acid is identified based on its spatial location on the detection region of the support medium.

The reporter can comprise a quenching moiety on the other side of the cleavage site. Sometimes the quenching moiety is a fluorescence quenching moiety. In some cases, the quenching moiety is 5' to the cleavage site and the detection moiety is 3' to the cleavage site. In some cases, the detection moiety is 5' to the cleavage site and the quenching moiety is 3' to the cleavage site. Sometimes the quenching moiety is at the 5' terminus of the nucleic acid of the reporter. Sometimes the detection moiety is at the 3' terminus of the nucleic acid of the reporter. In some cases, the detection moiety is at the 5' terminus of the nucleic acid of the reporter. In some cases, the quenching moiety is at the 3' terminus of the nucleic acid of the reporter. In some cases, the reporter comprises at least one population of the single-stranded nucleic acid capable of generating a first detectable signal. In some cases, the reporter comprises a population of the single-stranded nucleic acid capable of generating a first detectable signal. Optionally, there is more than one population of reporter. In some cases, there are 2, 3, 4, 5, 6, 7, 8, 9, 10, 11, 12, 13, 14, 15, 20, 30, 40, 50, or greater than 50, or any number spanned by the range of this list of different populations of reporters capable of generating a detectable signal.

In some cases, the one or more detectable signals generated after cleavage can produce an index of refraction change or one or more electrochemical changes. In some cases, real-time detection of the Cas reaction can be achieved using fluorescence, electrochemical detection, and/or electrochemiluminescence. In some cases, the detectable signals can be detected and analyzed in various ways. For example, the detectable signals can be detected using an imaging device. The imaging device can comprise a digital camera, such as a digital camera on a mobile device. The mobile device can have a software program or a mobile application that can capture fluorescence, ultraviolet (UV), infrared (IR), or visible wavelength signals. Any suitable detection or measurement device can be used to detect and/or analyze the colorimetric, fluorescence, amperometric, potentiometric, or electrochemical signals described herein. In some embodiments, the colorimetric, fluorescence, amperometric, potentiometric, or another electrochemical sign can be detected using a measurement device connected to a detection chamber of the device (e.g., a fluorescence measurement device, a spectrophotometer, and/or an oscilloscope).

In certain aspects of this disclosure, multiplexing refers to parallel sensing of multiple target nucleic acid sequences in one sample by multiple probes. In some cases, there are from 2 to 50, from 3 to 40, from 4 to 30, from 5 to 20, or from 6 to 10 different populations of reporters capable of generating a detectable signal. In some cases there are from 2 to 50, from 5 to 50, from 10 to 50, from 15 to 50, from 20 to 50, from 25 to 50, from 30 to 50, from 35 to 50, from 40 to 50, from 2 to 40, from 5 to 40, from 10 to 40, from 15 to 40, from 20 to 40, from 25 to 40, from 30 to 40, from 35 to 40, from 2 to 30, from 5 to 30, from 10 to 30, from 15 to 30, from 20 to 30, from 25 to 30, from 2 to 20, from 5 to 20, from 10 to 20, from 15 to 20, from 2 to 10, or from 5 to 10 different populations of reporters capable of generating a detectable signal.

**TABLE 5 - Exemplary Single Stranded Reporter**

| **5' DETECTION MOIETY*** | **SEQUENCE (SEQ ID NO:)** | **3' QUENCHER*** |
|---|---|---|
| /56-FAM/ | rUrUrUrUrU (**SEQ ID NO:** 1) | /3IABkFQ/ |
| /5IRD700/ | rUrUrUrUrU (**SEQ ID NO:** 1) | /3IRQC1N/ |
| /5TYE665/ | rUrUrUrUrU (**SEQ ID NO:** 1) | /3IAbRQSp/ |
| /5Alex594N/ | rUrUrUrUrU (**SEQ ID NO:** 1) | /3IAbRQSp/ |
| /5ATTO633N/ | rUrUrUrUrU (**SEQ ID NO:** 1) | /3IAbRQSp/ |
| /56-FAM/ | rUrUrUrUrUrUrUrU(**SEQ ID NO**: 2) | /3IABkFQ/ |
| /5IRD700/ | rUrUrUrUrUrUrUrU(**SEQ ID NO**: 2) | /3IRQC1N/ |
| /5TYE665/ | rUrUrUrUrUrUrUrU(**SEQ ID NO**: 2) | /3IAbRQSp/ |
| /5Alex594N/ | rUrUrUrUrUrUrUrU(**SEQ ID NO**: 2) | /3IAbRQSp/ |
| /5ATTO633N/ | rUrUrUrUrUrUrUrU(**SEQ ID NO**: 2) | /3IAbRQSp/ |
| /56-FAM/ | rUrUrUrUrUrUrUrUrUrU(**SEQ ID NO:** 3) | /3IABkFQ/ |
| /5IRD700/ | rUrUrUrUrUrUrUrUrUrU(**SEQ ID NO:** 3) | /3IRQC1N/ |
| /5TYE665/ | rUrUrUrUrUrUrUrUrUrU(**SEQ ID NO:** 3) | /3IAbRQSp/ |
| /5Alex594N/ | rUrUrUrUrUrUrUrUrUrU(**SEQ ID NO:** 3) | /3IAbRQSp/ |
| /5ATTO633N/ | rUrUrUrUrUrUrUrUrUrU(**SEQ ID NO:** 3) | /3IAbRQSp/ |
| /56-FAM/ | TTTTrUrUTTTT(**SEQ ID NO:** 4) | /3IABkFQ/ |
| /5IRD700/ | TTTTrUrUTTTT(**SEQ ID NO**: 4) | /3IRQC1N/ |
| /5TYE665/ | TTTTrUrUTTTT(**SEQ ID NO**: 4) | /3IAbRQSp/ |
| /5Alex594N/ | TTTTrUrUTTTT(**SEQ ID NO**: 4) | /3IAbRQSp/ |
| /5ATTO633N/ | TTTTrUrUTTTT(**SEQ ID NO**: 4) | /3IAbRQSp/ |
| /56-FAM/ | TTrUrUTT(**SEQ ID NO:** 5) | /3IABkFQ/ |
| /5IRD700/ | TTrUrUTT(**SEQ ID NO:** 5) | /3IRQC1N/ |
| /5TYE665/ | TTrUrUTT(**SEQ ID NO:** 5) | /3IAbRQSp/ |
| /5Alex594N/ | TTrUrUTT(**SEQ ID NO:** 5) | /3IAbRQSp/ |
| /5ATTO633N/ | TTrUrUTT(**SEQ ID NO:** 5) | /3IAbRQSp/ |
| /56-FAM/ | TArArUGC(**SEQ ID NO:** 6) | /3IABkFQ/ |
| /5IRD700/ | TArArUGC(**SEQ ID NO**: 6) | /3IRQC1N/ |
| /5TYE665/ | TArArUGC(**SEQ ID NO:** 6) | /3IAbRQSp/ |
| /5Alex594N/ | TArArUGC(**SEQ ID NO:** 6) | /3IAbRQSp/ |
| /5ATTO633N/ | TArArUGC(**SEQ ID NO:** 6) | /3IAbRQSp/ |
| /56-FAM/ | TArUrGGC(**SEQ ID NO:** 7) | /3IABkFQ/ |
| /5IRD700/ | TArUrGGC(**SEQ ID NO:** 7) | /3IRQC1N/ |
| /5TYE665/ | TArUrGGC(**SEQ ID NO:** 7) | /3IAbRQSp/ |
| /5Alex594N/ | TArUrGGC(**SEQ ID NO:** 7) | /3IAbRQSp/ |
| /5ATTO633N/ | TArUrGGC(**SEQ ID NO:** 7) | /3IAbRQSp/ |
| /56-FAM/ | rUrUrUrUrU(**SEQ ID NO:** 8) | /3IABkFQ/ |
| /5IRD700/ | rUrUrUrUrU(**SEQ ID NO:** 8) | /3IRQC1N/ |
| /5TYE665/ | rUrUrUrUrU(**SEQ ID NO:** 8) | /3IAbRQSp/ |
| /5Alex594N/ | rUrUrUrUrU(**SEQ ID NO:** 8) | /3IAbRQSp/ |
| /5ATTO633N/ | rUrUrUrUrU(**SEQ ID NO:** 8) | /3IAbRQSp/ |
| /56-FAM/ | TTATTATT (**SEQ ID NO:** 9) | /3IABkFQ/ |
| /56-FAM/ | TTATTATT (**SEQ ID NO:** 9) | /3IABkFQ/ |
| /5IRD700/ | TTATTATT (**SEQ ID NO:** 9) | /3IRQC1N/ |
| /5TYE665/ | TTATTATT (**SEQ ID NO:** 9) | /3IAbRQSp/ |
| /5Alex594N/ | TTATTATT (**SEQ ID NO:** 9) | /3IAbRQSp/ |
| /5ATTO633N/ | TTATTATT (**SEQ ID NO:** 9) | /3IAbRQSp/ |
| /56-FAM/ | TTTTTT (**SEQ ID NO:** 10) | /3IABkFQ/ |
| /56-FAM/ | TTTTTTTT (**SEQ ID NO:** 11) | /3IABkFQ/ |
| /56-FAM/ | TTTTTTTTTT (**SEQ ID NO:** 12) | /3IABkFQ/ |
| /56-FAM/ | TTTTTTTTTTTT (**SEQ ID NO:** 13) | /3IABkFQ/ |
| /56-FAM/ | TTTTTTTTTTTTTT (**SEQ ID NO:** 14) | /3IABkFQ/ |
| /56-FAM/ | AAAAAA (**SEQ ID NO:** 15) | /3IABkFQ/ |
| /56-FAM/ | CCCCCC (**SEQ ID NO:** 16) | /3IABkFQ/ |
| /56-FAM/ | GGGGGG (**SEQ ID NO:** 17) | /3IABkFQ/ |
| /56-FAM/ | TTATTATT (**SEQ ID NO:** 9) | /3IABkFQ/ |

| | | |
|---|---|---|
| /56-FAM/: 5' 6-Fluorescein (Integrated DNA Technologies) /3IABkFQ/: 3' Iowa Black FQ (Integrated DNA Technologies) /5IRD700/: 5' IRDye 700 (Integrated DNA Technologies) /5TYE665/: 5' TYE 665 (Integrated DNA Technologies) /5Alex594N/: 5' Alexa Fluor 594 (NHS Ester) (Integrated DNA Technologies) /5TTO633N/: 5' ATTO TM 633 (NHS Ester) (Integrated DNA Technologies) /3IRQC1N/: 3' IRDye QC-1 Quencher (Li-Cor) /3IAbRQSp/: 3' Iowa Black RQ (Integrated DNA Technologies) rU: uracil ribonucleotide rG: guanine ribonucleotide *This Table refers to the detection moiety and quencher moiety as their tradenames and their source is identified. However, alternatives, generics, or non-tradename moieties with similar function from other sources can also be used. | | |

A detection moiety can be an infrared fluorophore. A detection moiety can be a fluorophore that emits fluorescence in the range of from 500 nm and 720 nm. A detection moiety can be a fluorophore that emits fluorescence in the range of from 500 nm and 720 nm. In some cases, the detection moiety emits fluorescence at a wavelength of 700 nm or higher. In other cases, the detection moiety emits fluorescence at about 660 nm or about 670 nm. In some cases, the detection moiety emits fluorescence at in the range of from 500 to 520, 500 to 540, 500 to 590, 590 to 600, 600 to 610, 610 to 620, 620 to 630, 630 to 640, 640 to 650, 650 to 660, 660 to 670, 670 to 680, 6890 to 690, 690 to 700, 700 to 710, 710 to 720, or 720 to 730 nm. A detection moiety can be a fluorophore that emits a fluorescence in the same range as 6-Fluorescein, IRDye 700, TYE 665, Alex Fluor, or ATTO TM 633 (NHS Ester). A detection moiety can be fluorescein amidite, 6-Fluorescein, IRDye 700, TYE 665, Alex Fluor 594, or ATTO TM 633 (NHS Ester). A detection moiety can be a fluorophore that emits a fluorescence in the same range as 6-Fluorescein (Integrated DNA Technologies), IRDye 700 (Integrated DNA Technologies), TYE 665 (Integrated DNA Technologies), Alex Fluor 594 (Integrated DNA Technologies), or ATTO TM 633 (NHS Ester) (Integrated DNA Technologies). A detection moiety can be fluorescein amidite, 6-Fluorescein (Integrated DNA Technologies), IRDye 700 (Integrated DNA Technologies), TYE 665 (Integrated DNA Technologies), Alex Fluor 594 (Integrated DNA Technologies), or ATTO TM 633 (NHS Ester) (Integrated DNA Technologies). Any of the detection moieties described herein can be from any commercially available source, can be an alternative with a similar function, a generic, or a non-tradename of the detection moieties listed.

A detection moiety can be chosen for use based on the type of sample to be tested. For example, a detection moiety that is an infrared fluorophore is used with a urine sample. As another example, SEQ ID NO: 1 with a fluorophore that emits around 520 nm is used for testing in non-urine samples, and SEQ ID NO: 8 with a fluorophore that emits a fluorescence around 700 nm is used for testing in urine samples.

A quenching moiety can be chosen based on its ability to quench the detection moiety. A quenching moiety can be a non-fluorescent fluorescence quencher. A quenching moiety can quench a detection moiety that emits fluorescence in the range of from 500 nm and 720 nm. A quenching moiety can quench a detection moiety that emits fluorescence in the range of from 500 nm and 720 nm. In some cases, the quenching moiety quenches a detection moiety that emits fluorescence at a wavelength of 700 nm or higher. In other cases, the quenching moiety quenches a detection moiety that emits fluorescence at about 660 nm or about 670 nm. In some cases, the quenching moiety quenches a detection moiety emits fluorescence at in the range of from 500 to 520, 500 to 540, 500 to 590, 590 to 600, 600 to 610, 610 to 620, 620 to 630, 630 to 640, 640 to 650, 650 to 660, 660 to 670, 670 to 680, 6890 to 690, 690 to 700, 700 to 710, 710 to 720, or 720 to 730 nm. A quenching moiety can quench fluorescein amidite, 6-Fluorescein, IRDye 700, TYE 665, Alex Fluor 594, or ATTO TM 633 (NHS Ester). A quenching moiety can be Iowa Black RQ, Iowa Black FQ or IRDye QC-1 Quencher. A quenching moiety can quench fluorescein amidite, 6-Fluorescein (Integrated DNA Technologies), IRDye 700 (Integrated DNA Technologies), TYE 665 (Integrated DNA Technologies), Alex Fluor 594 (Integrated DNA Technologies), or ATTO TM 633 (NHS Ester) (Integrated DNA Technologies). A quenching moiety can be Iowa Black RQ (Integrated DNA Technologies), Iowa Black FQ (Integrated DNA Technologies) or IRDye QC-1 Quencher (LiCor). Any of the quenching moieties described herein can be from any commercially available source, can be an alternative with a similar function, a generic, or a non-tradename of the quenching moieties listed.

The generation of the detectable signal from the release of the detection moiety indicates that cleavage by the programmable nuclease has occurred and that the sample contains the target nucleic acid. In some cases, the detection moiety comprises a fluorescent dye. Sometimes the detection moiety comprises a fluorescence resonance energy transfer (FRET) pair. In some cases, the detection moiety comprises an infrared (IR) dye. In some cases, the detection moiety comprises an ultraviolet (UV) dye. Alternatively, or in combination, the detection moiety comprises a polypeptide. Sometimes the detection moiety comprises a biotin. Sometimes the detection moiety comprises at least one of avidin or streptavidin. In some instances, the detection moiety comprises a polysaccharide, a polymer, or a nanoparticle. In some instances, the detection moiety comprises a gold nanoparticle or a latex nanoparticle.

Alternatively, or in combination, in some embodiments, detecting the presence or absence of a target nucleic acid of interest involves measuring a signal emitted from a conjugate bound to a detection moiety present in a reporter, after cleavage of the reporter by an activated programmable nuclease. The conjugates may comprise a nanoparticle, a gold nanoparticle, a latex nanoparticle, a quantum dot, a chemiluminescent nanoparticle, a carbon nanoparticle, a selenium nanoparticle, a fluorescent nanoparticle, a liposome, or a dendrimer. The surface of the conjugate may be coated by a conjugate binding molecule that binds to the detection moiety or another affinity molecule of the cleaved detector molecule as described herein. Thus, the detecting steps disclosed herein involve indirectly (e.g., via a reporter) measuring the presence of a target nucleic acid, quantifying how much of the target nucleic acid is present, or, measuring a signal indicating that the target nucleic acid is absent in a sample. In some embodiments, a signal is generated upon cleavage of the reporter by the programmable nuclease. In other embodiments, the signal changes upon cleavage of the reporter by the programmable nuclease. In other embodiments, a signal may be present in the absence of reporter cleavage and disappear upon cleavage of the target nucleic acid by the programmable nuclease. For example, a signal may be produced in a microfluidic device or lateral flow device after contacting a sample with a composition comprising a programmable nuclease.

A detection moiety can be any moiety capable of generating a calorimetric, potentiometric, amperometric, optical (e.g., fluorescent, colorimetric, etc.), or piezo-electric signal. A reporter, sometimes, is protein-nucleic acid that is capable of generating a calorimetric, potentiometric, amperometric, optical (e.g., fluorescent, colorimetric, etc.), or piezo-electric signal upon cleavage of the nucleic acid. Often a calorimetric signal is heat produced after cleavage of the reporters. Sometimes, a calorimetric signal is heat absorbed after cleavage of the reporters. A potentiometric signal, for example, is electrical potential produced after cleavage of the reporters. An amperometric signal can be movement of electrons produced after the cleavage of a reporter. Often, the signal is an optical signal, such as a colorimetric signal or a fluorescence signal. An optical signal is, for example, a light output produced after the cleavage of the reporters. Sometimes, an optical signal is a change in light absorbance between before and after the cleavage of reporters. Often, a piezo-electric signal is a change in mass between before and after the cleavage of the reporter.

Often, the protein-nucleic acid is an enzyme-nucleic acid. The enzyme may be sterically hindered when present as in the enzyme-nucleic acid, but then functional upon cleavage from the nucleic acid. Often, the enzyme is an enzyme that produces a reaction with a substrate. An enzyme can be invertase. Often, the substrate of invertase is sucrose and DNS reagent.

Sometimes the protein-nucleic acid is a substrate-nucleic acid. Often the substrate is a substrate that produces a reaction with an enzyme.

A protein-nucleic acid may be attached to a solid support. The solid support, for example, may be a surface. A surface can be an electrode. Sometimes the solid support is a bead. Often the bead is a magnetic bead. Upon cleavage, the protein is liberated from the solid and interacts with other mixtures. For example, the protein is an enzyme, and upon cleavage of the nucleic acid of the enzyme-nucleic acid, the enzyme flows through a chamber into a mixture comprising the substrate. When the enzyme meets the enzyme substrate, a reaction occurs, such as a colorimetric reaction, which is then detected. As another example, the protein is an enzyme substrate, and upon cleavage of the nucleic acid of the enzyme substrate-nucleic acid, the enzyme flows through a chamber into a mixture comprising the enzyme. When the enzyme substrate meets the enzyme, a reaction occurs, such as a calorimetric reaction, which is then detected.

In some embodiments, the reporter comprises a nucleic acid conjugated to an affinity molecule and the affinity molecule conjugated to the fluorophore (e.g., nucleic acid - affinity molecule - fluorophore) or the nucleic acid conjugated to the fluorophore and the fluorophore conjugated to the affinity molecule (e.g., nucleic acid - fluorophore - affinity molecule). In some embodiments, a linker conjugates the nucleic acid to the affinity molecule. In some embodiments, a linker conjugates the affinity molecule to the fluorophore. In some embodiments, a linker conjugates the nucleic acid to the fluorophore. A linker can be any suitable linker known in the art. In some embodiments, the nucleic acid of the reporter can be directly conjugated to the affinity molecule and the affinity molecule can be directly conjugated to the fluorophore or the nucleic acid can be directly conjugated to the fluorophore and the fluorophore can be directly conjugated to the affinity molecule. In this context, "directly conjugated" indicated that no intervening molecules, polypeptides, proteins, or other moieties are present between the two moieties directly conjugated to each other. For example, if a reporter comprises a nucleic acid directly conjugated to an affinity molecule and an affinity molecule directly conjugated to a fluorophore - no intervening moiety is present between the nucleic acid and the affinity molecule and no intervening moiety is present between the affinity molecule and the fluorophore. The affinity molecule can be biotin, avidin, streptavidin, or any similar molecule.

In some cases, the reporter comprises a substrate-nucleic acid. The substrate may be sequestered from its cognate enzyme when present as in the substrate-nucleic acid, but then is released from the nucleic acid upon cleavage, wherein the released substrate can contact the cognate enzyme to produce a detectable signal. Often, the substrate is sucrose and the cognate enzyme is invertase, and a DNS reagent can be used to monitor invertase activity.

A major advantage of the devices and methods disclosed herein is the design of excess reporters to total nucleic acids in an unamplified or an amplified sample, not including the nucleic acid of the reporter. Total nucleic acids can include the target nucleic acids and non-target nucleic acids, not including the nucleic acid of the reporter. The non-target nucleic acids can be from the original sample, either lysed or unlysed. The non-target nucleic acids can also be byproducts of amplification. Thus, the non-target nucleic acids can include both non-target nucleic acids from the original sample, lysed or unlysed, and from an amplified sample. The presence of a large amount of non-target nucleic acids, an activated programmable nuclease may be inhibited in its ability to bind and cleave the reporter sequences. This is because the activated programmable nucleases collaterally cleaves any nucleic acids. If total nucleic acids are present in large amounts, they may outcompete reporters for the programmable nucleases. The devices and methods disclosed herein are designed to have an excess of reporter to total nucleic acids, such that the detectable signals from cleavage reactions (e.g., DETECTR reactions) are particularly superior. In some embodiments, the reporter can be present in at least 1.5 fold, at least 2 fold, at least 3 fold, at least 4 fold, at least 5 fold, at least 6 fold, at least 7 fold, at least 8 fold, at least 9 fold, at least 10 fold, at least 11 fold, at least 12 fold, at least 13 fold, at least 14 fold, at least 15 fold, at least 16 fold, at least 17 fold, at least 18 fold, at least 19 fold, at least 20 fold, at least 30 fold, at least 40 fold, at least 50 fold, at least 60 fold, at least 70 fold, at least 80 fold, at least 90 fold, at least 100 fold, from 1.5 fold to 100 fold, from 2 fold to 10 fold, from 10 fold to 20 fold, from 20 fold to 30 fold, from 30 fold to 40 fold, from 40 fold to 50 fold, from 50 fold to 60 fold, from 60 fold to 70 fold, from 70 fold to 80 fold, from 80 fold to 90 fold, from 90 fold to 100 fold, from 1.5 fold to 10 fold, from 1.5 fold to 20 fold, from 10 fold to 40 fold, from 20 fold to 60 fold, or from 10 fold to 80 fold excess of total nucleic acids.

A second significant advantage of the devices and methods disclosed herein is the design of an excess volume comprising the guide nucleic acid, the programmable nuclease, and the reporter, which contacts a smaller volume comprising the sample with the target nucleic acid of interest. The smaller volume comprising the sample can be unlysed sample, lysed sample, or lysed sample which has undergone any combination of reverse transcription, amplification, and in vitro transcription, as claimed. The presence of various reagents in a crude, non-lysed sample, a lysed sample, or a lysed and amplified sample, such as buffer, magnesium sulfate, salts, the pH, a reducing agent, primers, dNTPs, NTPs, cellular lysates, non-target nucleic acids, primers, or other components, can inhibit the ability of the programmable nuclease to find and cleave the nucleic acid of the reporter. This may be due to nucleic acids that are not the reporter, which outcompete the nucleic acid of the reporter, for the programmable nuclease. Alternatively, various reagents in the sample may simply inhibit the activity of the programmable nuclease. Thus, the devices and methods provided herein for contacting an excess volume comprising the guide nucleic acid, the programmable nuclease, and the reporter to a smaller volume comprising the sample with the target nucleic acid of interest provides for superior detection of the target nucleic acid by ensuring that the programmable nuclease is able to find and cleaves the nucleic acid of the reporter. In some embodiments, the volume comprising the guide nucleic acid, the programmable nuclease, and the reporter (can be referred to as "a second volume") is 4-fold greater than a volume comprising the sample (can be referred to as "a first volume"). In some embodiments, the volume comprising the guide nucleic acid, the programmable nuclease, and the reporter (can be referred to as "a second volume") is at least 1.5 fold, at least 2 fold, at least 3 fold, at least 4 fold, at least 5 fold, at least 6 fold, at least 7 fold, at least 8 fold, at least 9 fold, at least 10 fold, at least 11 fold, at least 12 fold, at least 13 fold, at least 14 fold, at least 15 fold, at least 16 fold, at least 17 fold, at least 18 fold, at least 19 fold, at least 20 fold, at least 30 fold, at least 40 fold, at least 50 fold, at least 60 fold, at least 70 fold, at least 80 fold, at least 90 fold, at least 100 fold, from 1.5 fold to 100 fold, from 2 fold to 10 fold, from 10 fold to 20 fold, from 20 fold to 30 fold, from 30 fold to 40 fold, from 40 fold to 50 fold, from 50 fold to 60 fold, from 60 fold to 70 fold, from 70 fold to 80 fold, from 80 fold to 90 fold, from 90 fold to 100 fold, from 1.5 fold to 10 fold, from 1.5 fold to 20 fold, from 10 fold to 40 fold, from 20 fold to 60 fold, or from 10 fold to 80 fold greater than a volume comprising the sample (can be referred to as "a first volume"). In some embodiments, the volume comprising the sample is at least 0.5 ul, at least 1 ul, at least at least 1 µL, at least 2 µL, at least 3 µL, at least 4 µL, at least 5 µL, at least 6 µL, at least 7 µL, at least 8 µL, at least 9 µL, at least 10 µL, at least 11 µL, at least 12 µL, at least 13 µL, at least 14 µL, at least 15 µL, at least 16 µL, at least 17 µL, at least 18 µL, at least 19 µL, at least 20 µL, at least 25 µL, at least 30 µL, at least 35 µL, at least 40 µL, at least 45 µL, at least 50 µL, at least 55 µL, at least 60 µL, at least 65 µL, at least 70 µL, at least 75 µL, at least 80 µL, at least 85 µL, at least 90 µL, at least 95 µL, at least 100 µL, from 0.5 µL to 5 ul µL, from 5 µL to 10 µL, from 10 µL to 15 µL, from 15 µL to 20 µL, from 20 µL to 25 µL, from 25 µL to 30 µL, from 30 µL to 35 µL, from 35 µL to 40 µL, from 40 µL to 45 µL, from 45 µL to 50 µL, from 10 µL to 20 µL, from 5 µL to 20 µL, from 1 µL to 40 µL, from 2 µL to 10 µL, or from 1 µL to 10 µL. In some embodiments, the volume comprising the programmable nuclease, the guide nucleic acid, and the reporter is at least 10 µL, at least 11 µL, at least 12 µL, at least 13 µL, at least 14 µL, at least 15 µL, at least 16 µL, at least 17 µL, at least 18 µL, at least 19 µL, at least 20 µL, at least 21 µL, at least 22 µL, at least 23 µL, at least 24 µL, at least 25 µL, at least 26 µL, at least 27 µL, at least 28 µL, at least 29 µL, at least 30 µL, at least 40 µL, at least 50 µL, at least 60 µL, at least 70 µL, at least 80 µL, at least 90 µL, at least 100 µL, at least 150 µL, at least 200 µL, at least 250 µL, at least 300 µL, at least 350 µL, at least 400 µL, at least 450 µL, at least 500 µL, from 10 µL to 15 ul µL, from 15 µL to 20 µL, from 20 µL to 25 µL, from 25 µL to 30 µL, from 30 µL to 35 µL, from 35 µL to 40 µL, from 40 µL to 45 µL, from 45 µL to 50 µL, from 50 µL to 55 µL, from 55 µL to 60 µL, from 60 µL to 65 µL, from 65 µL to 70 µL, from 70 µL to 75 µL, from 75 µL to 80 µL, from 80 µL to 85 µL, from 85 µL to 90 µL, from 90 µL to 95 µL, from 95 µL to 100 µL, from 100 µL to 150 µL, from 150 µL to 200 µL, from 200 µL to 250 µL, from 250 µL to 300 µL, from 300 µL to 350 µL, from 350 µL to 400 µL, from 400 µL to 450 µL, from 450 µL to 500 µL, from 10 µL to 20 µL, from 10 µL to 30 µL, from 25 µL to 35 µL, from 10 µL to 40 µL, from 20 µL to 50 µL, from 18 µL to 28 µL, or from 17 µL to 22 µL.

A reporter may be a hybrid nucleic acid reporter. A hybrid nucleic acid reporter comprises a nucleic acid with at least one deoxyribonucleotide and at least one ribonucleotide. In some embodiments, the nucleic acid of the hybrid nucleic acid reporter can be of any length and can have any mixture of DNAs and RNAs. For example, in some cases, longer stretches of DNA can be interrupted by a few ribonucleotides. Alternatively, longer stretches of RNA can be interrupted by a few deoxyribonucleotides. Alternatively, every other base in the nucleic acid may alternate between ribonucleotides and deoxyribonucleotides. A major advantage of the hybrid nucleic acid reporter is increased stability as compared to a pure RNA nucleic acid reporter. For example, a hybrid nucleic acid reporter can be more stable in solution, lyophilized, or vitrified as compared to a pure DNA or pure RNA reporter.

Additionally, target nucleic acid can be amplified before binding to the crRNA of the CRISPR enzyme. This amplification can be PCR amplification or isothermal amplification. This nucleic acid amplification of the sample can improve at least one of sensitivity, specificity, or accuracy of the detection the target RNA. The reagents for nucleic acid amplification can comprise a recombinase, an oligonucleotide primer, a single-stranded DNA binding (SSB) protein, and a polymerase. The nucleic acid amplification can be transcription mediated amplification (TMA). Nucleic acid amplification can be helicase dependent amplification (HDA) or circular helicase dependent amplification (cHDA). In additional cases, nucleic acid amplification is strand displacement amplification (SDA). The nucleic acid amplification can be recombinase polymerase amplification (RPA). The nucleic acid amplification can be at least one of loop mediated amplification (LAMP) or the exponential amplification reaction (EXPAR). Nucleic acid amplification is, in some cases, by rolling circle amplification (RCA), ligase chain reaction (LCR), simple method amplifying RNA targets (SMART), single primer isothermal amplification (SPIA), multiple displacement amplification (MDA), nucleic acid sequence based amplification (NASBA), hinge-initiated primer-dependent amplification of nucleic acids (HIP), nicking enzyme amplification reaction (NEAR), or improved multiple displacement amplification (IMDA). The nucleic acid amplification can be performed for no greater than 1, 2, 3, 4, 5, 6, 7, 8, 9, 10, 11, 12, 13, 14, 15, 16, 17, 18, 19, 20, 25, 30, 40, 50, or 60 minutes. Sometimes, the nucleic acid amplification reaction is performed at a temperature of around 20-45°C. The nucleic acid amplification reaction can be performed at a temperature no greater than 20°C, 25°C, 30°C, 35°C, 37°C, 40°C, 45°C. The nucleic acid amplification reaction can be performed at a temperature of at least 20°C, 25°C, 30°C, 35°C, 37°C, 40°C, or 45°C.

In some cases, an amplification method may comprise a set of multiple distinct primers targeting a single amplicon (e.g., a gene, such as the SARS-Cov-2 E and N genes). The set of primers may all target sequences upstream of a gene of interest, and may comprise overlapping, partially overlapping, or entirely distinct sequences. The use of multiple primers can increase the yield from amplification reactions. In some cases, the use of multiple distinct primers can increase the likelihood that damaged nucleic acids are amplified in a reaction. The use of multiple distinct primers can also facilitate amplification (and therefore detection) of mutant nucleic acids. In some cases, a sample may comprise a mutant strain of SARS-CoV-2 that would not be detectable if a single primer were used for amplification. A primer may be designed for thermal cycling amplification of the target nucleic acid. A primer may be designed for isothermal amplification of the target nucleic acid.

In some embodiments, an amplification method may utilize an amplification activator. A set of amplification reagents may require addition of an amplification activator for activity. In such cases, an amplification activator may enable temporal control over the start of an amplification reaction. In some embodiments, the amplification activator comprises a magnesium salt, deoxyribonucleoside triphosphates (dNTPs), nucleoside triphosphates (NTPs), adenosine triphosphate (ATP), or a combination thereof. In some embodiments, the magnesium salt is magnesium sulfate, magnesium chloride, magnesium acetate, magnesium phosphate, magnesium iodide, magnesium fluoride, magnesium bromide, or a combination thereof.

Disclosed herein are methods of assaying for a target nucleic acid as described herein wherein a signal is detected. For example, a method of assaying for a target nucleic acid in a sample comprises contacting the sample to a complex comprising a guide nucleic acid comprising a segment that is reverse complementary to a segment of the target nucleic acid and a programmable nuclease that exhibits sequence independent cleavage upon forming a complex comprising the segment of the guide nucleic acid binding to the segment of the target nucleic acid; and assaying for a signal indicating cleavage of at least some protein-nucleic acids of a population of protein-nucleic acids, wherein the signal indicates a presence of the target nucleic acid in the sample and wherein absence of the signal indicates an absence of the target nucleic acid in the sample. As another example, a method of assaying for a target nucleic acid in a sample, for example, comprises: a) contacting the sample to a complex comprising a guide nucleic acid comprising a segment that is reverse complementary to a segment of the target nucleic acid (e.g., a nucleic acid from a coronavirus such as SARS-CoV-2) and a programmable nuclease that exhibits sequence independent cleavage upon forming a complex comprising the segment of the guide nucleic acid binding to the segment of the target nucleic acid; b) contacting the complex to a substrate; c) contacting the substrate to a reagent that differentially reacts with a cleaved substrate; and d) assaying for a signal indicating cleavage of the substrate, wherein the signal indicates a presence of the target nucleic acid in the sample and wherein absence of the signal indicates an absence of the target nucleic acid in the sample. Often, the substrate is an enzyme-nucleic acid. Sometimes, the substrate is an enzyme substrate-nucleic acid.

A programmable nuclease can comprise a programmable nuclease capable of being activated when complexed with a guide nucleic acid and target nucleic acid (e.g., a nucleic acid from a coronavirus such as SARS-CoV-2). The programmable nuclease can become activated after binding of a guide nucleic acid with a target nucleic acid, in which the activated programmable nuclease can cleave the target nucleic acid and can have trans cleavage activity. Trans cleavage activity can be non-specific cleavage of nearby nucleic acids by the activated programmable nuclease, such as trans cleavage of reporters with a detection moiety. Once the reporter is cleaved by the activated programmable nuclease, the detection moiety can be released from the reporter and can generate a signal. The detection moiety capable of generating the detectable signal can be immobilized on a support medium for detection. The signal can be visualized to assess whether a target nucleic acid comprises a modification.

Often, the signal is a colorimetric signal or a signal visible by eye. In some instances, the signal is fluorescent, electrical, chemical, electrochemical, or magnetic. A signal can be a calorimetric, potentiometric, amperometric, optical (e.g., fluorescent, colorimetric, etc.), or piezo-electric signal. In some cases, the detectable signal is a colorimetric signal or a signal visible by eye. In some instances, the detectable signal is fluorescent, electrical, chemical, electrochemical, or magnetic. In some cases, the first detection signal is generated by binding of the detection moiety to the capture molecule in the detection region, where the first detection signal indicates that the sample contained the target nucleic acid. Sometimes the system is capable of detecting more than one type of target nucleic acid, wherein the system comprises more than one type of guide nucleic acid and more than one type of reporter. In some cases, the detectable signal is generated directly by the cleavage event. Alternatively, or in combination, the detectable signal is generated indirectly by the signal event. Sometimes the detectable signal is not a fluorescent signal. In some instances, the detectable signal is a colorimetric or color-based signal. In some cases, the detected target nucleic acid is identified based on its spatial location on the detection region of the support medium. In some cases, the second detectable signal is generated in a spatially distinct location than the first generated signal.

In some cases, the threshold of detection, for a subject method of detecting a single-stranded target nucleic acid in a sample, is less than or equal to 10 nM. The term "threshold of detection" is used herein to describe the minimal amount of target nucleic acid that must be present in a sample in order for detection to occur. For example, when a threshold of detection is 10 nM, then a signal can be detected when a target nucleic acid is present in the sample at a concentration of 10 nM or more. In some cases, the threshold of detection is less than or equal to 5 nM, 1 nM, 0.5 nM, 0.1 nM, 0.05 nM, 0.01 nM, 0.005 nM, 0.001 nM, 0.0005 nM, 0.0001 nM, 0.00005 nM, 0.00001 nM, 10 pM, 1 pM, 500 fM, 250 fM, 100 fM, 50 fM, 10 fM, 5 fM, 1 fM, 500 attomole (aM), 100 aM, 50 aM, 10 aM, or 1 aM. In some cases, the threshold of detection is in a range of from 1 aM to 1 nM, 1 aM to 500 pM, 1 aM to 200 pM, 1 aM to 100 pM, 1 aM to 10 pM, 1 aM to 1 pM, 1 aM to 500 fM, 1 aM to 100 fM, 1 aM to 1 fM, 1 aM to 500 aM, 1 aM to 100 aM, 1 aM to 50 aM, 1 aM to 10 aM, 10 aM to 1 nM, 10 aM to 500 pM, 10 aM to 200 pM, 10 aM to 100 pM, 10 aM to 10 pM, 10 aM to 1 pM, 10 aM to 500 fM, 10 aM to 100 fM, 10 aM to 1 fM, 10 aM to 500 aM, 10 aM to 100 aM, 10 aM to 50 aM, 100 aM to 1 nM, 100 aM to 500 pM, 100 aM to 200 pM, 100 aM to 100 pM, 100 aM to 10 pM, 100 aM to 1 pM, 100 aM to 500 fM, 100 aM to 100 fM, 100 aM to 1 fM, 100 aM to 500 aM, 500 aM to 1 nM, 500 aM to 500 pM, 500 aM to 200 pM, 500 aM to 100 pM, 500 aM to 10 pM, 500 aM to 1 pM, 500 aM to 500 fM, 500 aM to 100 fM, 500 aM to 1 fM, 1 fM to 1 nM, 1 fM to 500 pM, 1 fM to 200 pM, 1 fM to 100 pM, 1 fM to 10 pM, 1 fM to 1 pM, 10 fM to 1 nM, 10 fM to 500 pM, 10 fM to 200 pM, 10 fM to 100 pM, 10 fM to 10 pM, 10 fM to 1 pM, 500 fM to 1 nM, 500 fM to 500 pM, 500 fM to 200 pM, 500 fM to 100 pM, 500 fM to 10 pM, 500 fM to 1 pM, 800 fM to 1 nM, 800 fM to 500 pM, 800 fM to 200 pM, 800 fM to 100 pM, 800 fM to 10 pM, 800 fM to 1 pM, from 1 pM to 1 nM, 1 pM to 500 pM, 1 pM to 200 pM, 1 pM to 100 pM, or 1 pM to 10 pM. In some cases, the threshold of detection in a range of from 800 fM to 100 pM, 1 pM to 10 pM, 10 fM to 500 fM, 10 fM to 50 fM, 50 fM to 100 fM, 100 fM to 250 fM, or 250 fM to 500 fM. In some cases, the minimum concentration at which a single-stranded target nucleic acid is detected in a sample is in a range of from 1 aM to 1 nM, 10 aM to 1 nM, 100 aM to 1 nM, 500 aM to 1 nM, 1 fM to 1 nM, 1 fM to 500 pM, 1 fM to 200 pM, 1 fM to 100 pM, 1 fM to 10 pM, 1 fM to 1 pM, 10 fM to 1 nM, 10 fM to 500 pM, 10 fM to 200 pM, 10 fM to 100 pM, 10 fM to 10 pM, 10 fM to 1 pM, 500 fM to 1 nM, 500 fM to 500 pM, 500 fM to 200 pM, 500 fM to 100 pM, 500 fM to 10 pM, 500 fM to 1 pM, 800 fM to 1 nM, 800 fM to 500 pM, 800 fM to 200 pM, 800 fM to 100 pM, 800 fM to 10 pM, 800 fM to 1 pM, 1 pM to 1 nM, 1 pM to 500 pM, from 1 pM to 200 pM, 1 pM to 100 pM, or 1 pM to 10 pM. In some cases, the minimum concentration at which a single-stranded target nucleic acid can be detected in a sample is in a range of from 1 aM to 100 pM. In some cases, the minimum concentration at which a single-stranded target nucleic acid can be detected in a sample is in a range of from 1 fM to 100 pM. In some cases, the minimum concentration at which a single-stranded target nucleic acid can be detected in a sample is in a range of from 10 fM to 100 pM. In some cases, the minimum concentration at which a single-stranded target nucleic acid can be detected in a sample is in a range of from 800 fM to 100 pM. In some cases, the minimum concentration at which a single-stranded target nucleic acid can be detected in a sample is in a range of from 1 pM to 10 pM. In some cases, the devices, systems, fluidic devices, kits, and methods described herein detect a target single-stranded nucleic acid in a sample comprising a plurality of nucleic acids such as a plurality of non-target nucleic acids, where the target single-stranded nucleic acid is present at a concentration as low as 1 aM, 10 aM, 100 aM, 500 aM, 1 fM, 10 fM, 500 fM, 800 fM, 1 pM, 10 pM, 100 pM, or 1 pM.

In some cases, the devices, systems, fluidic devices, kits, and methods described herein detect a target single-stranded nucleic acid (e.g., a nucleic acid from a coronavirus such as SARS-CoV-2) in a sample where the sample is contacted with the reagents for a predetermined length of time sufficient for the trans cleavage to occur or cleavage reaction to reach completion. In some cases, the devices, systems, fluidic devices, kits, and methods described herein detect a target single-stranded nucleic acid in a sample where the sample is contacted with the reagents for no greater than 60 minutes. Sometimes the sample is contacted with the reagents for no greater than 120 minutes, 110 minutes, 100 minutes, 90 minutes, 80 minutes, 70 minutes, 60 minutes, 55 minutes, 50 minutes, 45 minutes, 40 minutes, 35 minutes, 30 minutes, 25 minutes, 20 minutes, 15 minutes, 10 minutes, 5 minutes, 4 minutes, 3 minutes, 2 minutes, or 1 minute. Sometimes the sample is contacted with the reagents for at least 120 minutes, 110 minutes, 100 minutes, 90 minutes, 80 minutes, 70 minutes, 60 minutes, 55 minutes, 50 minutes, 45 minutes, 40 minutes, 35 minutes, 30 minutes, 25 minutes, 20 minutes, 15 minutes, 10 minutes, or 5 minutes. In some cases, the devices, systems, fluidic devices, kits, and methods described herein can detect a target nucleic acid in a sample in less than 10 hours, less than 9 hours, less than 8 hours, less than 7 hours, less than 6 hours, less than 5 hours, less than 4 hours, less than 3 hours, less than 2 hours, less than 1 hour, less than 50 minutes, less than 45 minutes, less than 40 minutes, less than 35 minutes, less than 30 minutes, less than 25 minutes, less than 20 minutes, less than 15 minutes, less than 10 minutes, less than 9 minutes, less than 8 minutes, less than 7 minutes, less than 6 minutes, or less than 5 minutes.

When a guide nucleic acid binds to a target nucleic acid (e.g., a nucleic acid from a coronavirus such as SARS-CoV-2), the programmable nuclease's trans cleavage activity can be initiated, and reporters can be cleaved, resulting in the detection of fluorescence. Some methods as described herein can a method of assaying for a target nucleic acid in a sample comprises contacting the sample to a complex comprising a guide nucleic acid comprising a segment that is reverse complementary to a segment of the target nucleic acid and a programmable nuclease that exhibits sequence independent cleavage upon forming a complex comprising the segment of the guide nucleic acid binding to the segment of the target nucleic acid; and assaying for a signal indicating cleavage of at least some protein-nucleic acids of a population of protein-nucleic acids, wherein the signal indicates a presence of the target nucleic acid in the sample and wherein absence of the signal indicates an absence of the target nucleic acid in the sample. The cleaving of the reporter using the programmable nuclease may cleave with an efficiency of 50% as measured by a change in a signal that is calorimetric, potentiometric, amperometric, optical (e.g., fluorescent, colorimetric, etc.), or piezo-electric, as non-limiting examples. Some methods as described herein can be a method of detecting a target nucleic acid in a sample comprising contacting the sample comprising the target nucleic acid with a guide nucleic acid targeting a target nucleic acid segment, a programmable nuclease capable of being activated when complexed with the guide nucleic acid and the target nucleic acid segment, a single-stranded reporter comprising a detection moiety, wherein the reporter is capable of being cleaved by the activated programmable nuclease, thereby generating a first detectable signal, cleaving the single-stranded reporter using the programmable nuclease that cleaves as measured by a change in color, and measuring the first detectable signal on the support medium. The cleaving of the single-stranded reporter using the programmable nuclease may cleave with an efficiency of 50% as measured by a change in color. In some cases, the cleavage efficiency is at least 40%, 50%, 60%, 70%, 80%, 90%, or 95% as measured by a change in color. The change in color may be a detectable colorimetric signal or a signal visible by eye. The change in color may be measured as a first detectable signal. The first detectable signal can be detectable within 5 minutes of contacting the sample comprising the target nucleic acid with a guide nucleic acid targeting a target nucleic acid segment, a programmable nuclease capable of being activated when complexed with the guide nucleic acid and the target nucleic acid segment, and a single-stranded reporter comprising a detection moiety, wherein the reporter is capable of being cleaved by the activated nuclease. The first detectable signal can be detectable within 1, 2, 3, 4, 5, 6, 7, 8, 9, 10, 11, 12, 13, 14, 15, 20, 25, 30, 35, 40, 45, 50, 55, 60, 70, 80, 90, 100, 110, or 120 minutes of contacting the sample.

In some cases, the methods, reagents, and devices described herein detect a target nucleic acid with a programmable nuclease and a single-stranded reporter in a sample where the sample is contacted with the reagents for a predetermined length of time sufficient for trans cleavage of the single-stranded reporter. For example, a programmable nuclease is LbuCas13a that detects a target nucleic acid and a single-stranded reporter comprises two adjacent uracil nucleotides with a green detectable moiety that is detected upon cleavage. As another example, a programmable nuclease is LbaCas13a that detects a target nucleic acid and a single-stranded reporter comprises two adjacent adenine nucleotides with a red detectable moiety that is detected upon cleavage. The target nucleic acid may be a single-stranded nucleic acid (e.g., a single-stranded DNA (ssDNA) or a single-stranded RNA), or the target nucleic acid may be a double-stranded nucleic acid (e.g., a double-stranded DNA (dsDNA) or a double-stranded RNA).

### Buffers for Detection of Nucleic Acids

The reagents described herein can also include buffers, which are compatible with the devices, systems, fluidic devices, kits, and methods disclosed herein. These buffers are compatible with the other reagents, samples, and support mediums as described herein for detection of an ailment, such as a disease, including those caused by viruses such as influenza. The methods described herein can also include the use of buffers, which are compatible with the methods disclosed herein.

A buffer may be configured to support multiple reactions or processes. A buffer may be configured to enable fast reaction rates for multiple types of reactions. For example, a buffer may enable a reaction to achieve a rate within 25%, 50%, 75%, or 90% of the fastest reported rate for that reaction at a specified temperature. The specified temperature may be between 0 and 10 °C, 10 and 20 °C, 20 and 30 °C, 30 and 40 °C, 40 and 50 °C, 50 and 60 °C, 60 and 70 °C, 70 and 80 °C, 80 and 90 °C, or higher than 90 °C. A buffer may enable amplification and DETECTR reactions to each reach completion (e.g., consume at least 80% of a limiting reagent) in less than two hours, less than 1 hour, less than 45 minutes, less than 30 minutes, less than 20 minutes, or less than 15 minutes. A buffer may enable fast reaction rates for 2, 3, 4, or 5 or more types of reactions, wherein each reaction comprises one or more targets. For example, a buffer may enable fast reaction rates for amplification and DETECTR reactions, and may comprise amplification and DETECTR reagents each targeting 100 nucleic acid sequences.

Time to completion may be measured by the consumption of a reagent. For example, a single-buffer comprising amplification and DETECTR reagents may consume (e.g., subject to transcollateral cleavage) at least 50 nM of reporters within 3 hours, 2 hours, 1.5 hours, 1 hour, 45 minutes, 30 minutes, 20 minutes, or 15 minutes of addition of 10000 copies, 5000 copies, 2000 copies, 1000 copies, 500 copies, 300 copies, 200 copies, 100 copies, 50 copies, or 10 copies of a target nucleic acid. A single-buffer comprising amplification and DETECTR reagents may consume at least 20 nM of reporters within 3 hours, 2 hours, 1.5 hours, 1 hour, 45 minutes, 30 minutes, 20 minutes, or 15 minutes of addition of 10000 copies, 5000 copies, 2000 copies, 1000 copies, 500 copies, 300 copies, 200 copies, 100 copies, 50 copies, or 10 copies of a target nucleic acid. A single-buffer comprising amplification and DETECTR reagents may consume at least 10 nM of reporters within 3 hours, 2 hours, 1.5 hours, 1 hour, 45 minutes, 30 minutes, 20 minutes, or 15 minutes of addition of 10000 copies, 5000 copies, 2000 copies, 1000 copies, 500 copies, 300 copies, 200 copies, 100 copies, 50 copies, or 10 copies of a target nucleic acid. A single-buffer comprising amplification and DETECTR reagents may consume at least 5 nM of reporters within 3 hours, 2 hours, 1.5 hours, 1 hour, 45 minutes, 30 minutes, 20 minutes, or 15 minutes of addition of 10000 copies, 5000 copies, 2000 copies, 1000 copies, 500 copies, 300 copies, 200 copies, 100 copies, 50 copies, or 10 copies of a target nucleic acid. A single-buffer comprising amplification and DETECTR reagents may consume at least 1 nM of reporters within 3 hours, 2 hours, 1.5 hours, 1 hour, 45 minutes, 30 minutes, 20 minutes, or 15 minutes of addition of 10000 copies, 5000 copies, 2000 copies, 1000 copies, 500 copies, 300 copies, 200 copies, 100 copies, 50 copies, or 10 copies of a target nucleic acid. A single-buffer comprising amplification and DETECTR reagents may consume at least 500 pM of reporters within 3 hours, 2 hours, 1.5 hours, 1 hour, 45 minutes, 30 minutes, 20 minutes, or 15 minutes of addition of 10000 copies, 5000 copies, 2000 copies, 1000 copies, 500 copies, 300 copies, 200 copies, 100 copies, 50 copies, or 10 copies of a target nucleic acid.

A buffer may also be configured to maximize the stability of a reagent (e.g., a programmable nuclease or a target nucleic acid) or reaction species (e.g., a DNA molecule produced by reverse transcription of a target RNA molecule). For example, a buffer may stabilize RNA so that its average half-life is 1.5 hours, 1 hour, 30 minutes, 20 minutes, 10 minutes, 5 minutes, 3 minutes, 2 minutes, or 1 minute at room temperature. A buffer may stabilize a reagent or enzyme for long-term storage. A buffer of the present disclosure may enable stable, long-term storage for a set of reagents, such as a composition comprising amplification and DETECTR reagents. A central challenge to the design of such a buffer stems from different proteins having different optimal conditions for stability. Optimal conditions for two proteins may comprise different osmolarities, viscosities, dielectric constants, pH, lipid content, detergent content, proportions of non-aqueous solvents (e.g., methanol, ethanol, acetone, acetonitrile, or a halomethane such as trichloromethane), or densities. The present disclosure provides buffers that are optimized to simultaneously stabilize a plurality of different proteins, such as a polymerase and a programmable nuclease (e.g., CRISPR-Cas enzyme). Enzymes for amplification and CRISPR-Cas reactions may have half-lives of at least three months, two months, one month, two weeks, one week, five days, 3 days, 2 days, 1 day, 12 hours, 8 hours, 6 hours, 4 hours, or 3 hours, 2 hours, or 1 hours at room temperature within a buffer of the present disclosure. Enzymes for amplification and CRISPR-Cas reactions may have half-lives that are at least 100 times, 50 times, 30 times, 20 times, 10 times, 5 times, or 2 times longer in a buffer of the present disclosure than in 50 mM pH 7.0 HEPES buffer, Escherichia coli cell lysate, or pichia pastoris cell lysate. Enzymes for amplification and CRISPR-Cas reactions contained within buffers of the present disclosure may be stable through multiple freeze-thaw cycles (e.g., less than 10%, 5%, 2%, 1% or 0.5% activity through an individual freeze-thaw cycle), therein enabling compositions and kits of the present disclosure to be stored and shipped at temperatures below 0 °C.

A buffer may comprise biological buffer, such as TRIS, DIPSO, HEPES, PIPES, maleic acid, citric acid, malic acid, formic acid, lactic acid, succinic acid, acetic acid, pivalic acid, pyridine, piperazine, picolinic acid, L-histidine, MES, bis-tris, bis-trispropane, MOPS, ADA, ACES, MOPSO, imidazole, MOPS, BES, TES, HEPES, DIPSO, TAPSO, TEA (triethanolamine) ), N-ethylmorpholine, POPSO, EPPS, HEPPS, HEPPSO, Tris, Tricine, glycylglycine, bicine, TAPS, morpholine, N-methyldiethanolamine, AMPD (2-amino-2-methyl-1,3-propanediol) ), Diethanolamine AMPSO, CHES, glycine, CAPSO, ethanolamine, AMP (2-amino-2-methyl-1-propanol), piperazine, CAPS, 1,3-diaminopropane, CABS, or piperidine. A buffer may comprise an inorganic buffer, such as phosphoric acid, carbonate, bicarbonate (e.g., sodium bicarbonate), or ammonia. A buffer may comprise a pH of about 3.5, about 3.6, about 3.7, about 3.8, about 3.9, about 4, about 4.1, about 4.2, about 4.3, about 4.4, about 4.5, about 4.6, about 4.7, about 4.8, about 4.9, about 5, about 5.1, about 5.2, about 5.3, about 5.4, about 5.5, about 6, about 6.5, about 7, about 7.5, about 8, about 8.5, or about 9. In some embodiments, a buffer may comprise a pH of from 3.5 to 4.5, from 4 to 5, from 4.5 to 5.5, from 3.5 to 4, from 4 to 4.5, from 4.5 to 5, from 5 to 5.5, from 5 to 6, from 6 to 7, from 7 to 8, from 8 to 9, or from 9 to 10.

A buffer may comprise a calcium or magnesium concentration of about 0 mM, about 2 mM, about 4 mM, about 5 mM, about 6 mM, about 8 mM, about 10 mM, about 12 mM, about 13 mM, about 14 mM, about 15 mM, about 20 mM, about 25 mM, about 30 mM, about 35 mM, about 40 mM, about 45 mM, about 50 mM, about 55 mM, or about 60 mM of (e.g., MgSO4, MgCl2, Mg(OAc)2, or Ca(NO3)2). A buffer may comprise a mixture of magnesium and/or calcium salts. Magnesium and/or calcium may be added to a sample to activate amplification reagents.

A buffer may comprise a reducing agent (e.g., NAC, DTT, BME, or TCEP) at a concentration of about 1 mM, about 2 mM, about 3 mM, about 4 mM, about 5 mM, about 6 mM, about 7 mM, about 8 mM, about 10 mM, about 12 mM, about 15 mM, about 20 mM, about 25 mM, about 30 mM, about 40 mM, about 50 mM, about 60 mM, about 7 mM, about 80 mM, about 90 mM, about 100 mM, or about 120 mM. A buffer may comprise a reducing agent (e.g., NAC, DTT, BME, or TCEP) at a concentration of from 1 mM to 5 mM, from 5 mM to 10 mM, from 10 mM to 15 mM, from 15 mM to 20 mM, from 20 mM to 25 mM, from 25 mM to 30 mM, from 30 mM to 40 mM, from 40 mM to 50 mM, from 50 mM to 60 mM, from 60 mM to 70 mM, from 70 mM to 80 mM, or from 80 mM to 90 mM, from 90 mM to 100 mM, or from 100 mM to 120 mM. A buffer may comprise a chelator (e.g., EDTA or EGTA) at a concentration of about 0.1 mM, about 0.2 mM, about 0.3 mM, about 0.4 mM, about 0.5 mM, about 0.6 mM, about 0.7 mM, about 0.8 mM, about 0.9 mM, about 1 mM, about 2 mM, about 3 mM, about 4 mM, about 5 mM, about 6 mM, about 7 mM, about 8 mM, about 10 mM, about 12 mM, about 15 mM, about 20 mM, about 25 mM, or about 30 mM. A buffer may comprise a chelator (e.g., EDTA or EGTA) at a concentration of from 0.1 mM to 0.5 mM, from 0.25 mM to 0.5 mM, from 0.4 mM to 0.6 mM, from 0.5 mM to 1 mM, from 1 mM to 5 mM, from 5 mM to 10 mM, from 10 mM to 15 mM, from 15 mM to 20 mM, from 20 mM to 25 mM, or from 25 mM to 30 mM.

A buffer may comprise a salt (e.g., ammonium acetate ((NH4)2OAc), magnesium acetate (Mg(OAc)2), manganese acetate (MnOAc), potassium acetate (K2OAc), sodium acetate (Na2OAc), ammonium chloride (NH4Cl), potassium chloride (KCl), magnesium chloride (MgCl2), manganese chloride (MnCl2), sodium chloride (NaCl), ammonium sulfate ((NH4)2SO4), magnesium sulfate (MgSO4), manganese sulfate (MnSO4), potassium sulfate (K2SO4), or sodium sulfate (Na2SO4)) at a concentration of about 1 mM, about 5 mM, about 10 mM, about 15 mM, about 20 mM, about 25 mM, about 30 mM, about 35 mM, about 40 mM, about 45 mM, about 50 mM, about 55 mM, about 60 mM, about 70 mM, about 80 mM, about 90 mM, or about 100 mM. A buffer may comprise a salt (e.g., (NH4)2OAc, Mg(OAc)2, MnOAc, K2OAc, Na2OAc, NH4Cl, Kl, MgCl2, MnCl2, NaCl, (NH4)2SO4, MgSO4, MnSO4, K2SO4, or Na2SO4) at a concentration of from 1 mM to 5 mM, from 1 mM to 10 mM, from 5 mM to 10 mM, from 10 mM to 15 mM, from 15 mM to 20 mM, from 20 mM to 25 mM, from 25 mM to 30 mM, from 30 mM to 35 mM, from 35 mM to 40 mM, from 40 mM to 45 mM, from 45 mM to 50 mM, from 50 mM to 55 mM, from 55 mM to 60 mM, from 60 mM to 70 mM, from 70 mM to 80 mM, from 80 mM to 90 mM, or from 90 mM to 100 mM.

A buffer may comprise a detergent (e.g., deoxycholate, NP-40 (Ipgal), Triton X-100, or Tween 20) at a concentration of about 0.01%, about 0.05%, about 0.10%, about 0.15%, about 0.20%, about 0.25%, about 0.30%, about 0.35%, about 0.40%, about 0.45%, about 0.50%, about 0.55%, about 0.60%, about 0.65%, about 0.70%, about 0.75%, about 0.80%, about 0.85%, about 0.90%, about 0.95%, about 1.00%, about 1.10%, about 1.20%, about 1.30%, about 1.40%, about 1.50%, about 2.00%, about 2.50%, about 3.00%, about 3.50%, about 4.00%, about 4.50%, or about 5.00%. A buffer may comprise a detergent (e.g., deoxycholate, NP-40 (Ipgal), Triton X-100, or Tween 20) at a concentration of from 0.01% to 0.10%, from 0.05% to 0.15%, from 0.10% to 0.20%, from 0.15% to 0.25%, from 0.20% to 0.30%, from 0.25% to 0.35%, from 0.30% to 0.40%, from 0.35% to 0.45%, from 0.40% to 0.50%, from 0.45% to 0.55%, from 0.50% to 0.60%, from 0.55% to 0.65%, from 0.60% to 0.70%, from 0.65% to 0.75%, from 0.70% to 0.80%, from 0.75% to 0.85%, from 0.80% to 0.90%, from 0.85% to 0.95%, from 0.90% to 1.00%, from 0.95% to 1.10%, from 1.00% to 1.20%, from 1.10% to 1.30%, from 1.20% to 1.40%, from 1.30% to 1.50%, from 1.40% to 1.60%, from 1.50% to 2.00%, from 2.00% to 2.50%, from 2.50% to 3.00%, from 3.00% to 3.50%, from 3.50% to 4.00%, from 4.00% to 4.50%, or from 4.50% to 5.00%.

For example, a buffer comprises 20 mM HEPES pH 7.5, 2.2 mM KOAc, 5.6 mM Mg(OAc)2, 1.1% glycerol, and 0.00018% (volume by volume) Triton-X-100. In some instances the buffer comprises from 0 to 100, 0 to 75, 0 to 50, 0 to 25, 0 to 20, 0 to 10, 0 to 5, 5 to 10,5 to 15, 5 to 20, 5 to 25, to 30, 5 to 40, 5 to 50, 5 to 75, 5 to 100, 10 to 20, 10 to 30, 10 to 40, 10 to 50, 10 to 75, 10 to 100, 15 to 20, 15 to 25, 15 to 30, 15 to 40, 15 to 50, 15 to 75, 15 to 100, 20 to 25, 20 to 30, 20 to 40, 20 to 50, 20 to 75, 20 to 100, 30 to 35, 30 to 40, 30 to 50, 30 to 75, 30 to 100, 40 to 45, 40 to 50, 40 to 60, 40 to 75, 40 to 100, 50 to 55, 50 to 60, 50 to 75 or 50 to 100 mM HEPES. The HEPES can be pH 6, pH 6.1, pH 6.2, pH 6.3, pH 6.4, pH 6.5, pH 6.6, pH 6.7, pH 6.8, pH 6.9, pH 7.0, pH 7.1, pH 7.2, pH 7.3, pH 7.4, pH 7.5, pH 7.6, pH 7.7, pH 7.8, pH 7.9, or pH 8.0. The buffer can comprise to 0 to 20, 0 to 18, 0 to 16, 0 to 15, 0 to 14, 0 to 12, 0 to 10, 0 to 7.5, 0 to 5, 0 to 2.5, 0 to 2, 0 to 1.5, 0 to 1, 0.5 to 2, 0.5 to 3, 0.5 to 4, 0.5 to 5, 0.5 to 6, 0.5 to 8, 0.5 to 10, 0.5 to 15, 0.5 to 20, 1 to 2, 1 to 3, 1 to 4, 1 to 5, 1 to 6, 1 to 8, 1 to 10, 1 to 20, 2 to 2.5, 2 to 3, 2 to 4, 2 to 5, 2 to 10, 2 to 20, 2.5 to 3, 2.5 to 5, or 2.2 mM KOAc. In other instances the buffer comprises 0 to 20, 0 to 18, 0 to 16, 0 to 15, 0 to 14, 0 to 12, 0 to 10, 0 to 7.5, 0 to 5, 0 to 2.5, 0 to 2, 0 to 1.5, 0 to 1, 0.5 to 2, 0.5 to 3, 0.5 to 4, 0.5 to 5, 0.5 to 6, 0.5 to 8, 0.5 to 10, 0.5 to 15, 0.5 to 20, 1 to 2, 1 to 3, 1 to 4, 1 to 5, 1 to 6, 1 to 8, 1 to 10, 1 to 20, 2 to 2.5, 2 to 3, 2 to 4, 2 to 5, 2 to 10, 2 to 20, 2.5 to 3, 2.5 to 5, 2.5 to 6, 2.5 to 8, 2.5 to 10, 2.5 to 20, 3 to 4, 3 to 5, 3 to 6, 3 to 8, 3 to 10, 3 to 15, 3 to 20, 4 to 5, 4 to 6, 4 to 8, 4 to 10, 4 to 15, 4 to 20, 5 to 6, 5 to 8, 5 to 10, 5 to 15, 5 to 20, or 5.6 mM Mg(OAc)2. The buffer can comprise 0 to 25, 0 to 20, 0 to 10, 0 to 5, 0 to 4, 0 to 3, 0 to 2, 0 to 1.5, 0.5 to 1, 0.5 to 1.5, 0.5 to 2, 0.5 to 2.5, 0.5 to 3, 1 to 2, 1 to 3, 5 to 10, 5 to 15, 5 to 20, 5 to 25, 5 to 30%, or 1.1% glycerol. The buffer can comprise 0 to 0.0001%, 0 to 0.0002%, 0 to 0.0003%, 0 to 0.0004%, 0 to 0.0005%, 0 to 0.0006%, 0 to 0.0007%, 0 to 0.0008%, 0 to 0.0009%, 0 to 0.001%, 0.0001% to 0.0002%, 0.0001% to 0.0003%, 0.0001% to 0.0004%, 0.0001% to 0.0005%, 0.0001% to 0.0006%, 0.0001% to 0.0007%, 0.0001% to 0.0008%, 0.0001% to 0.0009%, 0.0001% to 0.001%, 0.0002% to 0.0003%, 0.0002% to 0.0004%, 0.0002% to 0.0005%, 0.0002% to 0.0006%, 0.0002% to 0.0007%, 0.0002% to 0.0008%, 0.0002% to 0.0009%, 0.0002% to 0.001%, or 0.00018% Triton-X-100.\

As another example, a buffer comprises 26.49 mM Tris HCl pH 8.8; 13.25 mM (NH4)2SO4, 66.23 mM KCl, 2.65 mM MgSO4, and 0.13% (volume by volume) Tween 20. In some instances the buffer comprises 0 to 200, 0 to 150, 0 to 100, 0 to 75, 0 to 50, 0 to 40, 0 to 30, 0 to 25, 0 to 20, 0 to 15, 0 to 10, 0 to 5, 0 to 2.5, 2.5 to 200, 2.5 to 150, 2.5 to 100, 2.5 to 75, 2.5 to 50, 2.5 to 40, 2.5 to 30, 2.5 to 25, 2.5 to 20, 2.5 to 15, 2.5 to 10, 2.5 to 5, 5 to 200, 5 to 150, 5 to 100, 5 to 75, 5 to 50, 5 to 40, 5 to 30, 5 to 25, 5 to 20, 5 to 15, 5 to 10, 10 to 200, 10 to 150, 10 to 100, 10 to 75, 10 to 50, 10 to 40, 10 to 30, 10 to 25, 10 to 20, 10 to 15, 15 to 200, 15 to 150, 15 to 100, 15 to 75, 15 to 50, 15 to 40, 15 to 30, 15 to 25, 15 to 20, 20 to 200, 20 to 150, 20 to 100, 20 to 75, 20 to 50, 20 to 40, 20 to 30, 20 to 25, 25 to 200, 25 to 150, 25 to 100, 25 to 75, 25 to 50, 25 to 40, 25 to 30, or 26.49 mM Tris HCl. The Tris HCl can be pH 7.6, pH 7.7, pH 7.8, pH 7.9, pH 8.0, pH 8.1, pH 8.2, pH 8.3, pH 8.4, pH 8.5, pH 8.6, pH 8.7, pH 8.8, pH 8.9, pH 9.0, pH 9.1, pH 9.2, pH 9.3, pH 9.4, pH 9.5, pH 9.6, pH 9.7, or pH 9.8. The buffer can comprise 0 to 200, 0 to 150, 0 to 100, 0 to 75, 0 to 50, 0 to 40, 0 to 30, 0 to 25, 0 to 20, 0 to 15, 0 to 10, 0 to 5, 0 to 2.5, 2.5 to 200, 2.5 to 150, 2.5 to 100, 2.5 to 75, 2.5 to 50, 2.5 to 40, 2.5 to 30, 2.5 to 25, 2.5 to 20, 2.5 to 15, 2.5 to 10, 2.5 to 5, 5 to 200, 5 to 150, 5 to 100, 5 to 75, 5 to 50, 5 to 40, 5 to 30, 5 to 25, 5 to 20, 5 to 15, 5 to 10, 10 to 200, 10 to 150, 10 to 100, 10 to 75, 10 to 50, 10 to 40, 10 to 30, 10 to 25, 10 to 20, 10 to 15, 15 to 200, 15 to 150, 15 to 100, 15 to 75, 15 to 50, 15 to 40, 15 to 30, 15 to 25, 15 to 20, 20 to 200, 20 to 150, 20 to 100, 20 to 75, 20 to 50, 20 to 40, 20 to 30, 20 to 25, 25 to 200, 25 to 150, 25 to 100, 25 to 75, 25 to 50, 25 to 40, 25 to 30, or 13.25 mM (NH4)2SO4. In some instances, the buffer comprises between 0 to 500, 0 to 400, 0 to 300, 0 to 200, 0 to 100, 0 to 75, 0 to 50, 0 to 40, 0 to 30, 0 to 20, 0 to 10, 5 to 500, 5 to 400, 5 to 300, 5 to 200, 5 to 100, 5 to 75, 5 to 50, 5 to 40, 5 to 30, 5 to 20, 5 to 10, 10 to 500, 10 to 400, 10 to 300, 10 to 200, 10 to 100, 10 to 75, 10 to 50, 10 to 40, 10 to 30, 10 to 20, 20 to 500, 20 to 400, 20 to 300, 20 to 200, 20 to 100, 20 to 75, 20 to 50, 20 to 40, 20 to 30, 30 to 500, 30 to 400, 30 to 300, 30 to 200, 30 to 100, 30 to 75, 30 to 50, 30 to 40, 40 to 500, 40 to 400, 40 to 300, 40 to 200, 40 to 100, 40 to 75, 40 to 50, 50 to 500, 50 to 400, 50 to 300, 50 to 200, 50 to 100, 50 to 75, 60 to 500, 60 to 400, 60 to 300, 60 to 200, 60 to 100, 60 to 75, 75 to 500, 75 to 400, 75 to 300, 75 to 200, 75 to 100, or 66.23 mM KCl. In some instances, the buffer comprises between 0 to 20, 0 to 18, 0 to 16, 0 to 15, 0 to 14, 0 to 12, 0 to 10, 0 to 7.5, 0 to 5, 0 to 2.5, 0 to 2, 0 to 1.5, 0 to 1, 0.5 to 2, 0.5 to 3, 0.5 to 4, 0.5 to 5, 0.5 to 6, 0.5 to 8, 0.5 to 10, 0.5 to 15, 0.5 to 20, 1 to 2, 1 to 3, 1 to 4, 1 to 5, 1 to 6, 1 to 8, 1 to 10, 1 to 20, 2 to 2.5, 2 to 3, 2 to 4, 2 to 5, 2 to 10, 2 to 20, 2.5 to 3, 2.5 to 5, or 2.65 mM MgSO4. The buffer may comprise between 0 and 2%, 0 and 1%, 0 and 0.9%, 0 and 0.8%, 0 and 0.7%, 0 and 0.6%, 0 and 0.5%, 0 and 0.4%, 0 and 0.3%, 0 and 0.2%, 0 and 0.1%, 0 and 0.05%, 0 and 0.02%, 0.02% and 2%, 0.02% and 1%, 0.02% and 0.9%, 0.02% and 0.8%, 0.02% and 0.7%, 0.02% and 0.6%, 0.02% and 0.5%, 0.02% and 0.4%, 0.02% and 0.3%, 0.02% and 0.2%, 0.02% and 0.1%, 0.02% and 0.05%, 0.05% and 1%, 0.05% and 0.9%, 0.05% and 0.8%, 0.05% and 0.7%, 0.05% and 0.6%, 0.05% and 0.5%, 0.05% and 0.4%, 0.05% and 0.3%, 0.05% and 0.2%, 0.05% and 0.1%, 0.1% and 1%, 0.1% and 0.9%, 0.1% and 0.8%, 0.1% and 0.7%, 0.1% and 0.6%, 0.1% and 0.5%, 0.1% and 0.4%, 0.1% and 0.3%, 0.1% and 0.2%, 0.2% and 1%, 0.2% and 0.9%, 0.2% and 0.8%, 0.2% and 0.7%, 0.2% and 0.6%, 0.2% and 0.5%, 0.2% and 0.4%, 0.2% and 0.3%, 0.3% and 1%, 0.3% and 0.9%, 0.3% and 0.8%, 0.3% and 0.7%, 0.3% and 0.6%, 0.3% and 0.5%, 0.3% and 0.4%, 0.4% and 1%, 0.4% and 0.9%, 0.4% and 0.8%, 0.4% and 0.7%, 0.4% and 0.6%, 0.4% and 0.5%, 0.5% and 1%, 0.5% and 0.9%, 0.5% and 0.8%, 0.5% and 0.7%, 0.5% and 0.6%, or 0.13% Tween 20.

A buffer may be optimized to support multiple reactions. A challenge associated with nucleic acid detection is that many amplification and nucleic acid detection reactions require specific conditions, such as buffer type, ionic strength, viscosity, pH, osmolarity, and dielectric constant. In many cases, these conditions are not cross compatible between separate reactions (e.g., an amplification reaction and a DETECTR reaction). An aspect of the present disclosure provides buffer compositions that are suitable for supporting amplification and CRISPR-Cas enzyme (e.g., DETECTR) reactions. Such buffers may also support additional types of reactions, such as viral lysis, cellular lysis, reverse transcription, transcription, and demethylation, as claimed. In some instances, a buffer may support fast amplification, CRISPR, and viral lysis reactions. In particular instances, a buffer may support fast RT-LAMP, transcollateral cleavage (e.g., DETECTR), and viral lysis reactions.

In many cases, the buffer composition allows the amplification and CRISPR reactions to approach completion at the same rate or close to its optimal rate (e.g., in a buffer optimized solely for one type of reaction). For example, a buffer may enable an amplification reaction to proceed at least at 90% of its optimal rate and a DETECTR reaction to proceed at least at 85% of its optimal rate. A buffer may enable an amplification reaction to proceed at least at 85% of its optimal rate and a DETECTR reaction to proceed at least at 85% of its optimal rate. A buffer may enable an amplification reaction to proceed at least at 80% of its optimal rate and a DETECTR reaction to proceed at least at 90% of its optimal rate. A buffer may enable an amplification reaction and a DETECTR reaction to each proceed at least at 45% of their optimal rates. A buffer may enable an amplification reaction and a DETECTR reaction to each proceed at least at 50% of their optimal rates. A buffer may enable an amplification reaction and a DETECTR reaction to each proceed at least at 60% of their optimal rates. A buffer may enable an amplification reaction and a DETECTR reaction to each proceed at least at 2/3 of their optimal rates. A buffer may enable an amplification reaction and a DETECTR reaction to each proceed at least at 75% of their optimal rates. A buffer may enable an amplification reaction and a DETECTR reaction to each proceed at least at 80% of their optimal rates. A buffer may enable an amplification reaction and a DETECTR reaction to each proceed at least at 85% of their optimal rates. A buffer may enable an amplification reaction and a DETECTR reaction to each proceed at least at 90% of their optimal rates.

A buffer may enable an amplification reaction and a DETECTR reaction to each reach completion within 2 hours. A buffer may enable an amplification reaction and a DETECTR reaction to each reach completion within 1.5 hours. A buffer may enable an amplification reaction and a DETECTR reaction to each reach completion within 1.2 hours. A buffer may enable an amplification reaction and a DETECTR reaction to each reach completion within 1 hour. A buffer may enable an amplification reaction and a DETECTR reaction to each reach completion within 45 minutes. A buffer may enable an amplification reaction and a DETECTR reaction to each reach completion within 30 minutes. A buffer may enable an amplification reaction and a DETECTR reaction to each reach completion within 20 minutes. A buffer may enable an amplification reaction and a DETECTR reaction to each reach completion within 15 minutes. A buffer may enable an amplification reaction and a DETECTR reaction to each reach completion within 10 minutes. As used herein, the term 'reach completion' can refer to a reaction consuming at least about 80%, at least about 90%, at least about 95%, at least about 99%, or 100% of a limiting reagent (e.g., a reporter, a primer, a dNTP or rNTP, or an amplicon). The term 'reach completion' can also refer to a point in time where the rate of product production has diminished from its maximum rate to less than 5%, 2%, 1%, or 0.5% its maximum rate.

A buffer may comprise an amplification activator. A set of amplification reagents may require an inorganic, molecular, or biological species to initiate an amplification reaction. A buffer many comprise an amplification activator. Conversely, an amplification activator may be provided separately (e.g., in a separate vial in a kit) and added to a reaction mixture at a desired timepoint. An amplification activator may comprise a calcium or magnesium salt (e.g., CaOAc or Mg(OAc)2).

In some cases, a buffer may comprise a catalytic reagent for signal improvement or enhancement. In some cases, the catalytic reagent may enhance signal generation via hydrolysis of inorganic pyrophosphates. In some cases, the catalytic reagent may enhance signal generation via enhancement of DNA replication. In some cases, the catalytic reagent may enhance signal amplification via revival of Mg+2 ions in the buffer solution which may otherwise be taken up by the phosphates produced from usage of dNTPs during the LAMP reaction. In some cases, the catalytic reagent may enhance signal generation by reviving the concentration of Mg+2 ions in the buffer thereby enhancing the function of the Cas nuclease effector enzyme. In some cases, the catalytic reagent for signal improvement may be an enzyme. In some cases, the catalytic reagent for signal improvement may be a Thermostable Inorganic Pyrophosphatase (TIPP).

Any of the systems, methods, or devices described herein may comprise thermostable inorganic pyrophosphatase (TIPP). In some embodiments, a composition for an assay may comprise TIPP. In some embodiments, the composition may comprise about 0.5 enzyme unit (U) per 10 µL of solution. In some embodiments, the composition may comprise at least about 0.1 U per 10 µL of solution. In some embodiments, the composition may comprise at most about 2 U per 10 µL of solution. In some embodiments, the composition may comprise at least about 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1, 2, 3, 4, 5, 6, 7, 8, 9, or 10 U per 10 µL of solution. In some embodiments, the composition may comprise at most about 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1, 2, 3, 4, 5, 6, 7, 8, 9, or 10 U per 10 µL of solution.

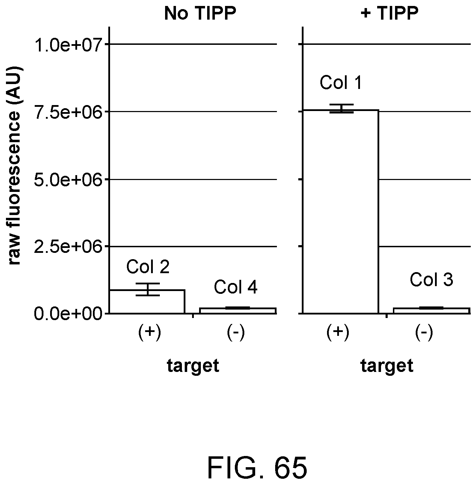

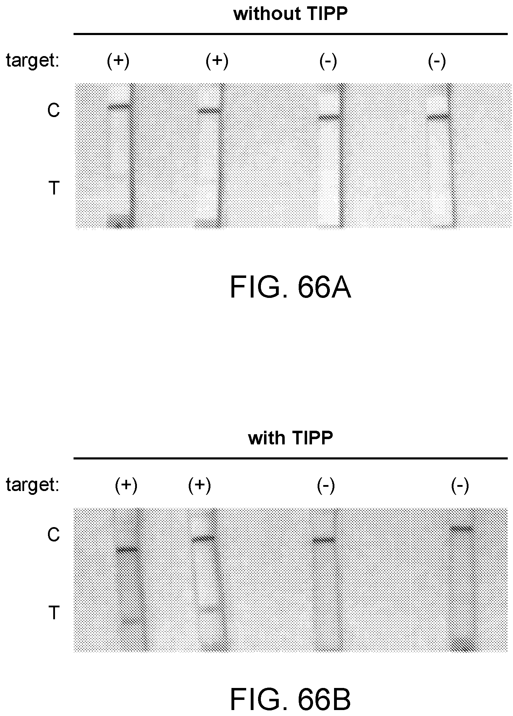

In some embodiments, TIPP may improve the signal to noise ratio of a programmable nuclease-based detection reaction. In some embodiments, TIPP may improve overall signal (e.g., fluorescence of a cleaved reporter as shown in FIG. 65 or intensity of a lateral flow assay strip detection location as shown in FIGS. 66A-66B). TIPP may improve signal by a factor, wherein the signal is indicative of the presence of a target nucleic acid. In some embodiments, the factor may be at least about 1.1. In some embodiments, the factor may be at least about 2. In some embodiments, the factor may be at least about 3. In some embodiments, the factor may be at least about 4. In some embodiments, the factor may be at least about 5. In some embodiments, the factor may be at least about 6. In some embodiments, the factor may be at least about 7. In some embodiments, the factor may be at least about 8. In some embodiments, the factor may be at least about 9. In some embodiments, the factor may be at least about 10.

In some cases, the TIPP enzyme may be present in the HotPot reaction mix at a concentration of 0.125 Units, 0.5 Units, 0.25 Units, 1.0 Units, 2.0 Units, 2.5 Units, or 4 Units per discrete reaction volume. In some cases, the buffer may comprise the TIPP signal enhancement reagent. Conversely, the TIPP signal enhancement reagent may be provided separately (e.g., in a separate vial in a kit) and added to a reaction mixture at a desired timepoint.

A buffer or reaction volume (e.g., a solution comprising reagents, such as a buffer comprising amplification and DETECTR reagents) may comprise varying amounts of total dissolved solids. A buffer may comprise (excluding reagents) at least 50 mM of total dissolved solids. A buffer may comprise (excluding reagents) at least 80 mM of total dissolved solids. A buffer may comprise (excluding reagents) at least 100 mM of total dissolved solids. A buffer may comprise (excluding reagents) at least 120 mM of total dissolved solids. A buffer may comprise (excluding reagents) at least 150 mM of total dissolved solids. A buffer may comprise (excluding reagents) at least 200 mM of total dissolved solids. A buffer may comprise (excluding reagents) at least 250 mM of total dissolved solids. A buffer may comprise (excluding reagents) at least 300 mM of total dissolved solids. A buffer may comprise (excluding reagents) at least 400 mM of total dissolved solids. A buffer may comprise (excluding reagents) at least 500 mM of total dissolved solids. A buffer may comprise (excluding reagents) at least 600 mM of total dissolved solids. A buffer may comprise (excluding reagents) at least 800 mM of total dissolved solids.

A buffer may comprise a viscosity of at least 1.5 centipoise (cP), at least 2 cP, at least 2.5 cP, at least 3 cP, at least 3.5 cP, at least 4 cP, at least 5 cP, at least 6 cP, at least 8 cP, at least 10 cP, at least 12 cP, at least 15 cP, at least 18 cP, at least 24 cP, at least 30 cP, at least 40 cP, or at least 50 cP at room temperature. High viscosities may stabilize enzymes and other reagents for amplification and programmable nuclease enzyme (e.g., CRISPR-Cas enzyme) reactions. High viscosities may also favor faster reaction rates for certain enzymes.

For example, a buffer comprises 20 mM HEPES pH 6.8, 50 mM KCl, 5 mM MgCl2, and 5% glycerol. In some instances the buffer comprises from 0 to 100, 0 to 75, 0 to 50, 0 to 25, 0 to 20, 0 to 10, 0 to 5, 5 to 10,5 to 15, 5 to 20, 5 to 25, to 30, 5 to 40, 5 to 50, 5 to 75, 5 to 100, 10 to 20, 10 to 30, 10 to 40, 10 to 50, 15 to 20, 15 to 25, 15 to 30, 15 to 4, 15 to 50, 20 to 25, 20 to 30, 20 to 40, or 20 to 50 mM HEPES pH 6.8. The buffer can comprise to 0 to 500, 0 to 400, 0 to 300, 0 to 250, 0 to 200, 0 to 150, 0 to 100, 0 to 75, 0 to 50, 0 to 25, 0 to 20, 0 to 10, 0 to 5, 5 to 10, 5 to 15, 5 to 20, 5 to 25, to 30, 5 to 40, 5 to 50, 5 to 75, 5 to 100, 5 to 150, 5 to 200, 5 to 250, 5 to 300, 5 to 400, 5 to 500, 25 to 50, 25 to 75, 25 to 100, 50 to 100, 50 150, 50 to 200, 50 to 250, 50 to 300, 100 to 200, 100 to 250, 100 to 300, or 150 to 250 mM KCl. In other instances the buffer comprises 0 to 100, 0 to 75, 0 to 50, 0 to 25, 0 to 20, 0 to 10, 0 to 5, 5 to 10, 5 to 15, 5 to 20, 5 to 25, to 30, 5 to 40, 5 to 50, 5 to 75, 5 to 100, 10 to 20, 10 to 30, 10 to 40, 10 to 50, 15 to 20, 15 to 25, 15 to 30, 15 to 4, 15 to 50, 20 to 25, 20 to 30, 20 to 40, or 20 to 50 mM MgCl2. The buffer can comprise 0 to 25, 0 to 20, 0 to 10, 0 to 5, 5 to 10, 5 to 15, 5 to 20, 5 to 25, 5 to 30% glycerol.

As another example, a buffer comprises 100 mM Imidazole pH 7.5; 250 mM KCl, 25 mM MgCl2, 50 ug/mL BSA, 0.05% Igepal Ca-630, and 25% Glycerol. In some instances the buffer comprises 0 to 500, 0 to 400, 0 to 300, 0 to 250, 0 to 200, 0 to 150, 0 to 100, 0 to 75, 0 to 50, 0 to 25, 0 to 20, 0 to 10, 0 to 5, 5 to 10, 5 to 15, 5 to 20, 5 to 25, to 30, 5 to 40, 5 to 50, 5 to 75, 5 to 100, 5 to 150, 5 to 200, 5 to 250, 5 to 300, 5 to 400, 5 to 500, 25 to 50, 25 to 75, 25 to 100, 50 to 100, 50 150, 50 to 200, 50 to 250, 50 to 300, 100 to 200, 100 to 250, 100 to 300, or 150 to 250 mM Imidazole pH 7.5. The buffer can comprise to 0 to 500, 0 to 400, 0 to 300, 0 to 250, 0 to 200, 0 to 150, 0 to 100, 0 to 75, 0 to 50, 0 to 25, 0 to 20, 0 to 10, 0 to 5, 5 to 10, 5 to 15, 5 to 20, 5 to 25, to 30, 5 to 40, 5 to 50, 5 to 75, 5 to 100, 5 to 150, 5 to 200, 5 to 250, 5 to 300, 5 to 400, 5 to 500, 25 to 50, 25 to 75, 25 to 100, 50 to 100, 50 150, 50 to 200, 50 to 250, 50 to 300, 100 to 200, 100 to 250, 100 to 300, or 150 to 250 mM KCl. In other instances the buffer comprises 0 to 100, 0 to 75, 0 to 50, 0 to 25, 0 to 20, 0 to 10, 0 to 5, 5 to 10, 5 to 15, 5 to 20, 5 to 25, to 30, 5 to 40, 5 to 50, 5 to 75, 5 to 100, 10 to 20, 10 to 30, 10 to 40, 10 to 50, 15 to 20, 15 to 25, 15 to 30, 15 to 4, 15 to 50, 20 to 25, 20 to 30, 20 to 40, or 20 to 50 mM MgCl2. The buffer, in some instances, comprises 0 to 100, 0 to 75, 0 to 50, 0 to 25, 0 to 20, 0 to 10, 0 to 5, 5 to 50, 5 to 75, 5 to 100, 10 to 20, 10 to 50, 10 to 75, 10 to 100, 25 to 50, 25 to 75 25 to 100, 50 to 75, or 50 to 100 ug/mL BSA. In some instances, the buffer comprises 0 to 1, 0 to 0.5, 0 to 0.25, 0 to 0.01, 0 to 0.05, 0 to 0.025, 0 to 0.01, 0.01 to 0.025, 0.01 to 0.05, 0.01 to 0.1, 0.01 to 0.25, 0.01, to 0.5, 0.01 to 1, 0.025 to 0.05, 0.025 to 0.1, 0.025, to 0.5, 0.025 to 1, 0.05 to 0.1, 0.05 to 0.25, 0.05 to 0.5, 0.05 to 0.75, 0.05 to 1, 0.1 to 0.25, 0.1 to 0.5, or 0.1 to 1 % Igepal Ca-630. The buffer can comprise 0 to 25, 0 to 20, 0 to 10, 0 to 5, 5 to 10, 5 to 15, 5 to 20, 5 to 25, 5 to 30% glycerol.

In some embodiments, a buffer comprises a pH of between 8 and 8.8, 20 mM Tris-HCl, 2 to 10 mM (NH4)2SO4, 2 to 50 mM KCl, 2 to 6.5 mM MgSO4, and 0.1% Tween20. In some embodiments, a buffer comprises a pH of 7.5, 20 mM HEPES, 20 mM KOAc, 5 mM MG(OAc)2, 1% glycerol and 0.00016% Triton X-100. In some embodiments, a buffer comprises a pH of between 7.5 and 8.25 phosphate buffer, between 0 and 2 mM (NH4)2SO4, 2 to 30 mM KCl, 5 mM Mg(OAc)2, 1% glycerol, and either 0.1% Tween 20 or 0.00016% Triton x-100. In some embodiments, a buffer comprises a pH of between 7.5 and 8.25, 0 to 2 mM (NH4)2SO44, 15 to 30 mM KOAc, 2 to 5 mM Mg(OAc)2, 1% glycerol and 0.1% Tween 20. In some embodiments, a buffer comprises a pH of 8, 10 mM phosphate, 0 to 2 mM (NH4)2SO4, 20 mM KOAc, 5 mM Mg(OAc)2, 1% glycerol, and 0.1% Tween 20.

A buffer of the present disclosure may comprise a viral lysis buffer. A viral lysis buffer may lyse a coronavirus capsid in a viral sample (e.g., a sample collected from an individual suspected of having a coronavirus infection), releasing a viral genome. The viral lysis buffer may be compatible with amplification (e.g., RT-LAMP amplification) of a target region of the viral genome. The viral lysis buffer may be compatible with detection (e.g., a DETECTR reaction disclosed herein). A sample may be prepared in a one-step sample preparation method comprising suspending the sample in a viral lysis buffer compatible with amplification, detection (e.g., a DETECTR reaction), or both. A viral lysis buffer compatible with amplification (e.g., RT-LAMP amplification), detection (e.g., DETECTR), or both, may comprise a buffer (e.g., Tris-HCl, phosphate, or HEPES), a reducing agent (e.g., N-Acetyl Cysteine (NAC), Dithiothreitol (DTT), β-mercaptoethanol (BME), or tris(2-carboxyethyl)phosphine (TCEP)), a chelating agent (e.g., EDTA or EGTA), a detergent (e.g., deoxycholate, NP-40 (Ipgal), Triton X-100, or Tween 20), a salt (e.g., ammonium acetate, magnesium acetate, manganese acetate, potassium acetate, sodium acetate, ammonium chloride, potassium chloride, magnesium chloride, manganese chloride, sodium chloride, ammonium sulfate, magnesium sulfate, manganese sulfate, potassium sulfate, or sodium sulfate), or a combination thereof. For example, a viral lysis buffer may comprise a buffer and a reducing agent, or a viral lysis buffer may comprise a buffer and a chelating agent. The viral lysis buffer may be formulated at a low pH. For example, the viral lysis buffer may be formulated at a pH of from about pH 4 to about pH 5. In some embodiments, the viral lysis buffer may be formulated at a pH of from about pH 4 to about pH 9. The viral lysis buffer may further comprise a preservative (e.g., ProClin 150). In some embodiments, the viral lysis buffer may comprise an activator of the amplification reaction. For example, the buffer may comprise primers, dNTPs, or magnesium (e.g., MgSO4, MgCl2 or Mg(OAc)2), or a combination thereof, to activate the amplification reaction. In some embodiments, an activator (e.g., primers, dNTPs, or magnesium) may be added to the buffer following lysis of the coronavirus to initiate the amplification reaction.

A viral lysis buffer may comprise a pH of about 3.5, about 3.6, about 3.7, about 3.8, about 3.9, about 4, about 4.1, about 4.2, about 4.3, about 4.4, about 4.5, about 4.6, about 4.7, about 4.8, about 4.9, about 5, about 5.1, about 5.2, about 5.3, about 5.4, about 5.5, about 6, about 6.5, about 7, about 7.5, about 8, about 8.5, or about 9. In some embodiments, a viral lysis buffer may comprise a pH of from 3.5 to 4.5, from 4 to 5, from 4.5 to 5.5, from 3.5 to 4, from 4 to 4.5, from 4.5 to 5, from 5 to 5.5, from 5 to 6, from 6 to 7, from 7 to 8, or from 8 to 9.

A viral lysis buffer may comprise a magnesium concentration of about 0 mM, about 2 mM, about 4 mM, about 5 mM, about 6 mM, about 8 mM, about 10 mM, about 12 mM, about 13 mM, about 14 mM, about 15 mM, about 20 mM, about 25 mM, about 30 mM, about 35 mM, about 40 mM, about 45 mM, about 50 mM, about 55 mM, or about 60 mM of magnesium (e.g., MgSO4, MgCl2 or Mg(OAc)2. A viral lysis buffer may comprise a magnesium concentration of from 0 mM to 5 mM, from 5 mM to 10 mM, from 10 mM to 15 mM, from 15 mM to 20 mM, from 20 mM to 25 mM, from 25 mM to 30 mM, from 30 mM to 40 mM, from 40 mM to 50 mM, or from 50 mM to 60 mM of magnesium (e.g., MgSO4, MgCl2 or Mg(OAc)2). In some embodiments, the magnesium may be added after viral lysis to activate an amplification reaction.

A viral lysis buffer may comprise a reducing agent (e.g., NAC, DTT, BME, or TCEP) at a concentration of about 1 mM, about 2 mM, about 3 mM, about 4 mM, about 5 mM, about 6 mM, about 7 mM, about 8 mM, about 10 mM, about 12 mM, about 15 mM, about 20 mM, about 25 mM, about 30 mM, about 40 mM, about 50 mM, about 60 mM, about 7 mM, about 80 mM, about 90 mM, about 100 mM, or about 120 mM. A viral lysis buffer may comprise a reducing agent (e.g., NAC, DTT, BME, or TCEP) at a concentration of from 1 mM to 5 mM, from 5 mM to 10 mM, from 10 mM to 15 mM, from 15 mM to 20 mM, from 20 mM to 25 mM, from 25 mM to 30 mM, from 30 mM to 40 mM, from 40 mM to 50 mM, from 50 mM to 60 mM, from 60 mM to 70 mM, from 70 mM to 80 mM, or from 80 mM to 90 mM, from 90 mM to 100 mM, or from 100 mM to 120 mM. A viral lysis buffer may comprise a chelator (e.g., EDTA or EGTA) at a concentration of about 0.1 mM, about 0.2 mM, about 0.3 mM, about 0.4 mM, about 0.5 mM, about 0.6 mM, about 0.7 mM, about 0.8 mM, about 0.9 mM, about 1 mM, about 2 mM, about 3 mM, about 4 mM, about 5 mM, about 6 mM, about 7 mM, about 8 mM, about 10 mM, about 12 mM, about 15 mM, about 20 mM, about 25 mM, or about 30 mM. A viral lysis buffer may comprise a chelator (e.g., EDTA or EGTA) at a concentration of from 0.1 mM to 0.5 mM, from 0.25 mM to 0.5 mM, from 0.4 mM to 0.6 mM, from 0.5 mM to 1 mM, from 1 mM to 5 mM, from 5 mM to 10 mM, from 10 mM to 15 mM, from 15 mM to 20 mM, from 20 mM to 25 mM, or from 25 mM to 30 mM.

A viral lysis buffer may comprise a salt (e.g., ammonium acetate ((NH4)2OAc), magnesium acetate (Mg(OAc)2), manganese acetate (MnOAc), potassium acetate (K2OAc), sodium acetate (Na2OAc), ammonium chloride (NH4Cl), potassium chloride (KCl), magnesium chloride (MgCl2), manganese chloride (MnCl2), sodium chloride (NaCl), ammonium sulfate ((NH4)2SO4), magnesium sulfate (MgSO4), manganese sulfate (MnSO4), potassium sulfate (K2SO4), or sodium sulfate (Na2SO4)) at a concentration of about 1 mM, about 5 mM, about 10 mM, about 15 mM, about 20 mM, about 25 mM, about 30 mM, about 35 mM, about 40 mM, about 45 mM, about 50 mM, about 55 mM, about 60 mM, about 70 mM, about 80 mM, about 90 mM, or about 100 mM. A viral lysis buffer may comprise a salt (e.g., (NH4)2OAc, Mg(OAc)2, MnOAc, K2OAc, Na2OAc, NH4Cl, KCl, MgCl2, MnCl2, NaCl, (NH4)2SO4, MgSO4, MnSO4, K2SO4, or Na2SO4) at a concentration of from 1 mM to 5 mM, from 1 mM to 10 mM, from 5 mM to 10 mM, from 10 mM to 15 mM, from 15 mM to 20 mM, from 20 mM to 25 mM, from 25 mM to 30 mM, from 30 mM to 35 mM, from 35 mM to 40 mM, from 40 mM to 45 mM, from 45 mM to 50 mM, from 50 mM to 55 mM, from 55 mM to 60 mM, from 60 mM to 70 mM, from 70 mM to 80 mM, from 80 mM to 90 mM, or from 90 mM to 100 mM.

A viral lysis buffer may comprise a detergent (e.g., deoxycholate, NP-40 (Ipgal), Triton X-100, or Tween 20) at a concentration of about 0.01%, about 0.05%, about 0.10%, about 0.15%, about 0.20%, about 0.25%, about 0.30%, about 0.35%, about 0.40%, about 0.45%, about 0.50%, about 0.55%, about 0.60%, about 0.65%, about 0.70%, about 0.75%, about 0.80%, about 0.85%, about 0.90%, about 0.95%, about 1.00%, about 1.10%, about 1.20%, about 1.30%, about 1.40%, about 1.50%, about 2.00%, about 2.50%, about 3.00%, about 3.50%, about 4.00%, about 4.50%, or about 5.00%. A viral lysis buffer may comprise a detergent (e.g., deoxycholate, NP-40 (Ipgal), Triton X-100, or Tween 20) at a concentration of from 0.01% to 0.10%, from 0.05% to 0.15%, from 0.10% to 0.20%, from 0.15% to 0.25%, from 0.20% to 0.30%, from 0.25% to 0.35%, from 0.30% to 0.40%, from 0.35% to 0.45%, from 0.40% to 0.50%, from 0.45% to 0.55%, from 0.50% to 0.60%, from 0.55% to 0.65%, from 0.60% to 0.70%, from 0.65% to 0.75%, from 0.70% to 0.80%, from 0.75% to 0.85%, from 0.80% to 0.90%, from 0.85% to 0.95%, from 0.90% to 1.00%, from 0.95% to 1.10%, from 1.00% to 1.20%, from 1.10% to 1.30%, from 1.20% to 1.40%, from 1.30% to 1.50%, from 1.40% to 1.60%, from 1.50% to 2.00%, from 2.00% to 2.50%, from 2.50% to 3.00%, from 3.00% to 3.50%, from 3.50% to 4.00%, from 4.00% to 4.50%, or from 4.50% to 5.00%.

A lysis reaction may be performed at a range of temperatures. In some embodiments, a lysis reaction may be performed at about room temperature. In some embodiments, a lysis reaction may be performed at about 95°C. In some embodiments, a lysis reaction may be performed at from 1 °C to 10 °C, from 4 °C to 8 °C, from 10 °C to 20 °C, from 15 °C to 25 °C, from 15 °C to 20 °C, from 18 °C to 25 °C, from 18 °C to 95 °C, from 20 °C to 37 °C, from 25 °C to 40 °C, from 35 °C to 45 °C, from 40 °C to 60 °C, from 50 °C to 70 °C, from 60 °C to 80 °C, from 70 °C to 90 °C, from 80 °C to 95 °C, or from 90 °C to 99 °C. In some embodiments, a lysis reaction may be performed for about 5 minutes, about 15 minutes, or about 30 minutes. In some embodiments, a lysis reaction may be performed for from 2 minutes to 5 minutes, from 3 minutes to 8 minutes, from 5 minutes to 15 minutes, from 10 minutes to 20 minutes, from 15 minutes to 25 minutes, from 20 minutes to 30 minutes, from 25 minutes to 35 minutes, from 30 minutes to 40 minutes, from 35 minutes to 45 minutes, from 40 minutes to 50 minutes, from 45 minutes to 55 minutes, from 50 minutes to 60 minutes, from 55 minutes to 65 minutes, from 60 minutes to 70 minutes, from 65 minutes to 75 minutes, from 70 minutes to 80 minutes, from 75 minutes to 85 minutes, or from 80 minutes to 90 minutes.

The reagents described herein can also include buffers, which are compatible with the devices, systems, fluidic devices, kits, and methods disclosed herein. The buffers described herein are compatible for use in the devices described herein (e.g., pneumatic valve devices, sliding valve devices, rotating valve devices, and lateral flow devices) and may be used in conjunction with compositions disclosed herein (e.g., programmable nucleases, guide nucleic acids, reagents for in vitro transcription, reagents for amplification, reagents for reverse transcription, reporters, or any combination thereof) to carry out highly efficient, rapid, and accurate reactions for detecting whether the target nucleic acid is in the sample (e.g., DETECTR reactions). These buffers are compatible with the other reagents, samples, and support mediums as described herein for detection of an ailment, such as a disease, cancer, or genetic disorder, or genetic information, such as for phenotyping, genotyping, or determining ancestry. The methods described herein can also include the use of buffers, which are compatible with the methods disclosed herein. For example, a buffer may comprise HEPES, MES, TCEP, EGTA, Tween 20, KC1, MgCl², glycerol, or any combination thereof. In some instances, a buffer may comprise Tris-HCl pH 8.8, VLB, EGTA, CH3COOH, TCEP, IsoAmp, (NH4)2SO4, KCl, MgSO4, Tween20, KOAc, MgOAc, BSA, TCEP, or any combination thereof. In some instances the buffer may comprise from 0 to 100, 0 to 75, 0 to 50, 0 to 25, 0 to 20, 0 to 10, 0 to 5, 5 to 10,5 to 15, 5 to 20, 5 to 25, to 30, 5 to 40, 5 to 50, 5 to 75, 5 to 100, 10 to 20, 10 to 30, 10 to 40, 10 to 50, 15 to 20, 15 to 25, 15 to 30, 15 to 4, 15 to 50, 20 to 25, 20 to 30, 20 to 40, or 20 to 50 mM HEPES pH 6.8. The buffer can comprise to 0 to 500, 0 to 400, 0 to 300, 0 to 250, 0 to 200, 0 to 150, 0 to 100, 0 to 75, 0 to 50, 0 to 25, 0 to 20, 0 to 10, 0 to 5, 5 to 10, 5 to 15, 5 to 20, 5 to 25, to 30, 5 to 40, 5 to 50, 5 to 75, 5 to 100, 5 to 150, 5 to 200, 5 to 250, 5 to 300, 5 to 400, 5 to 500, 25 to 50, 25 to 75, 25 to 100, 50 to 100, 50 150, 50 to 200, 50 to 250, 50 to 300, 100 to 200, 100 to 250, 100 to 300, or 150 to 250 mM KC1. In other instances the buffer may comprise 0 to 100, 0 to 75, 0 to 50, 0 to 25, 0 to 20, 0 to 10, 0 to 5, 5 to 10, 5 to 15, 5 to 20, 5 to 25, to 30, 5 to 40, 5 to 50, 5 to 75, 5 to 100, 10 to 20, 10 to 30, 10 to 40, 10 to 50, 15 to 20, 15 to 25, 15 to 30, 15 to 4, 15 to 50, 20 to 25, 20 to 30, 20 to 40, or 20 to 50 mM MgCl². The buffer can comprise 0 to 25, 0 to 20, 0 to 10, 0 to 5, 5 to 10, 5 to 15, 5 to 20, 5 to 25, 5 to 30% glycerol. The buffer can comprise from 0% to 30%, from 5% to 30%, from 10% to 30%, from 15% to 30%, from 20% to 30%, from 25% to 30%, from 0% to 25%, from 2% to 25%, from 5% to 25%, from 10% to 25%, from 15% to 25%, from 20% to 25%, from 0% to 20%, from 5% to 20%, from 10% to 20%, from 15% to 20%, from 0% to 15%, from 5% to 15%, from 10% to 15%, from 0% to 10%, from 5% to 10%, or from 0% to 5% glycerol. The buffer can comprise to 0 to 500, 0 to 400, 0 to 300, 0 to 250, 0 to 200, 0 to 150, 0 to 100, 0 to 75, 0 to 50, 0 to 25, 0 to 20, 0 to 10, 0 to 5, 5 to 10, 5 to 15, 5 to 20, 5 to 25, to 30, 5 to 40, 5 to 50, 5 to 75, 5 to 100, 5 to 150, 5 to 200, 5 to 250, 5 to 300, 5 to 400, 5 to 500, 25 to 50, 25 to 75, 25 to 100, 50 to 100, 50 150, 50 to 200, 50 to 250, 50 to 300, 100 to 200, 100 to 250, 100 to 300, or 150 to 250 mM Tris-HCl pH 8.8. The buffer can comprise to 0 to 500, 0 to 400, 0 to 300, 0 to 250, 0 to 200, 0 to 150, 0 to 100, 0 to 75, 0 to 50, 0 to 25, 0 to 20, 0 to 10, 0 to 5, 5 to 10, 5 to 15, 5 to 20, 5 to 25, to 30, 5 to 40, 5 to 50, 5 to 75, 5 to 100, 5 to 150, 5 to 200, 5 to 250, 5 to 300, 5 to 400, 5 to 500, 25 to 50, 25 to 75, 25 to 100, 50 to 100, 50 150, 50 to 200, 50 to 250, 50 to 300, 100 to 200, 100 to 250, 100 to 300, or 150 to 250 mM KOAc. The buffer can comprise to 0 to 500, 0 to 400, 0 to 300, 0 to 250, 0 to 200, 0 to 150, 0 to 100, 0 to 75, 0 to 50, 0 to 25, 0 to 20, 0 to 10, 0 to 5, 5 to 10, 5 to 15, 5 to 20, 5 to 25, to 30, 5 to 40, 5 to 50, 5 to 75, 5 to 100, 5 to 150, 5 to 200, 5 to 250, 5 to 300, 5 to 400, 5 to 500, 25 to 50, 25 to 75, 25 to 100, 50 to 100, 50 150, 50 to 200, 50 to 250, 50 to 300, 100 to 200, 100 to 250, 100 to 300, or 150 to 250 mM MgOAc. In some instances the buffer may comprise from 0 to 100, 0 to 75, 0 to 50, 0 to 25, 0 to 20, 0 to 10, 0 to 5, 5 to 10,5 to 15, 5 to 20, 5 to 25, to 30, 5 to 40, 5 to 50, 5 to 75, 5 to 100, 10 to 20, 10 to 30, 10 to 40, 10 to 50, 15 to 20, 15 to 25, 15 to 30, 15 to 4, 15 to 50, 20 to 25, 20 to 30, 20 to 40, or 20 to 50 mM EGTA. The buffer can comprise from 0% to 30%, from 5% to 30%, from 10% to 30%, from 15% to 30%, from 20% to 30%, from 25% to 30%, from 0% to 25%, from 2% to 25%, from 5% to 25%, from 10% to 25%, from 15% to 25%, from 20% to 25%, from 0% to 20%, from 5% to 20%, from 10% to 20%, from 15% to 20%, from 0% to 15%, from 5% to 15%, from 10% to 15%, from 0% to 10%, from 5% to 10%, or from 0% to 5% Tween 20.

### Compositions Comprising One or More Additives for Improving Assay Signals

In some embodiments, the reagents described herein may include a composition for improving detection signal strength, detection reaction time, detection reaction efficiency, stability, solubility, or the like. In some embodiments, the composition may comprise one or more additives. The one or more additives may, for example, comprise amino acids or derivatives thereof, chaotrpes, chelators, cyclodextrins, inhibitors, ionic liquids, linkers, metals, non detergent sulfobetaines, organic acids, osmolytes, peptides, polyamides, polymers, polyols, polyols and salts, salts, or combinations thereof. In some embodiments, the one or more additives may, for example, comprise one or more of trichloroacetic acid, L-Arginine, L-Glutamic acid, glycine, L-Proline, L-Histidine, beta(β)-Alanine, L-Serine, L-Arginine ethyl ester dihydrochloride, L-Argininamide dihydrochloride, 6-Aminohexanoic acid, Gly-gly peptide, Gly-gly-gly peptide, tryptone, betaine monohydrate, D-(+)-Trehalose dihydrate, Xylitol, D-Sorbitol, sucrose, hydroxyectoine, Trimethylamine N-oxide dihydrate, methyl alpha(α)-D-gluocopyranoside, triethylene glycol, spermine tetrahydrochloride, spermidine, 5-aminovaleric acid, glutaric acid, adipic acid, ethylenediamine dihydrochloride, guanidine hydrochloride, urea, N-methylurea, N-ethylurea, N-methylformamide, hypotauring, TCEP hydrochloride, GSH (L-Glutathione reduced), GSSG (L-Glutathione oxidized), benzaminidine hydrochloride, ethylenediaminetetraacetic acid disodium salt dihydrate, magnesium chloride hexahydrate, calcium chloride dihydrate, cadmium chloride hydrate, cobalt(II) chloride hexahydrate, Non Detergent Sulfobetaine 195 (NDSB-195), NDSB-201, NDSB-211, NDSB-221, NDSB-256, taurine, acetamide, oxalic acid dihydrate, sodium malonate pH 7.0, succinic acid pH 7.0, tacsimate pH 7.0, tetraethylammonium bromide, cholin acetate, 1-Ethyl-3-methylimidazolium acetate, 1-Butyl-3-methylimidazolium chloride, ethylammonium nitrate, ammonium sulfate, ammonium chloride, magnesium sulfate hydrate, potassium thiocynate, gadolinium(III) chloride hexahydrate, cesium chloride, 4-aminobutyric acid (GABA), lithium nitrate, DL-malic acid pH 7.0, lithium citrate tribasic tetrahydrate, ammonium acetate, sodium benzenesulfonate, sodium p-toluenesulfonate, sodium chloride, potassium chloride, sodium phosphate monobasic monohydrate, potassium phosphate dibasic, sodium sulfate decahydrate, lithium chloride, sodium bromide, glycerol, ethylene glycol, polyethylene glycol 200 (PEG-200), PEG 3350, PEG 8000, PEG monomethyl ether 550, PEG monomethyl ether 750, PEG monomethyl ether 1900, formamide, polypropylene glycol P 400, pentaerythritol ethoxylate, 1,2-Propanediol, polyvinylpyrrolidone K 15, 6-O-α-Maltosyl-β-cyclodextrin, (2-Hydroxypropyl)-β-cyclodextrin, α-cyclodextrin, β-cyclodextrin, Methyl-β-cyclodextrin, or any combination thereof.

In some embodiments, the reagents described herein may include a composition for increasing the signal strength of any of the assays described herein. In some embodiments, the composition comprises water and an additive. In some embodiments, the additive may comprise trehalose, xylitol, D-sorbitol, sucrose, and trimethylamine N-oxide dihydrate, or any combination thereof. In some embodiments, the composition further comprises one or more targets, one or more enzymes, one or more reporters, one or more substrates, or any combination thereof as described herein.

In some embodiments, the reagents described herein may include a composition for reducing the time it takes for the signal of any of the assays described herein to saturate. In some embodiments, the composition comprises an additive. In some embodiments, the additive may comprise betaine monohydrate, acetamide, GABA, L-proline, beta-alanine, 6-aminohexanonic acid, urea, methylurea, ethylurea, hypotaurine, NDSB-256, ammonium acetate, or any combination thereof. In some embodiments, the composition further comprises one or more targets, one or more enzymes, one or more reporters, one or more substrates, or any combination thereof as described herein.

In some embodiments, the additive is present at a concentration of at least about 1 nM, at least about 2 nM, at least about 3 nM, at least about 4 nM, at least about 5 nM, at least about 6 nM, at least about 7 nM, at least about 8 nM, at least about 9 nM, at least about 10 nM, at least about 20 nM, at least about 30 nM, at least about 40 nM, at least about 50 nM, at least about 60 nM, at least about 70 nM, at least about 80 nM, at least about 90 nM, at least about 100 nM, at least about 200 nM, at least about 300 nM, at least about 400 nM, at least about 500 nM, at least about 600 nM, at least about 700 nM, at least about 800 nM, at least about 900 nM, at least about 1 µM, at least about 2 µM, at least about 3 µM, at least about 4 µM, at least about 5 µM, at least about 6 µM, at least about 7 µM, at least about 8 µM, at least about 9 µM, at least about 10 µM, at least about 20 µM, at least about 30 µM, at least about 40 µM, at least about 50 µM, at least about 60 µM, at least about 70 µM, at least about 80 µM, at least about 90 µM, at least about 100 µM, at least about 200 µM, at least about 300 µM, at least about 400 µM, at least about 500 µM, at least about 600 µM, at least about 700 µM, at least about 800 µM, at least about 900 µM, at least about 1 mM, at least about 2 mM, at least about 3 mM, at least about 4 mM, at least about 5 mM, at least about 6 mM, at least about 7 mM, at least about 8 mM, at least about 9 mM, at least about 10 mM, at least about 20 mM, at least about 30 mM, at least about 40 mM, at least about 50 mM, at least about 60 mM, at least about 70 mM, at least about 80 mM, at least about 90 mM, at least about 100 mM, at least about 200 mM, at least about 300 mM, at least about 400 mM, at least about 500 mM, at least about 600 mM, at least about 700 mM, at least about 800 mM, at least about 900 mM, at least about 1 M, at least about 2 M, at least about 3 M, at least about 4 M, at least about 5 M, at least about 6 M, at least about 7 M, at least about 8 M, at least about 9 M, or at least about 10 M.

In some embodiments, the composition increases the signal strength by at least about a factor of 1.01, at least about a factor of 1.02, at least about a factor of 1.03, at least about a factor of 1.04, at least about a factor of 1.05, at least about a factor of 1.06, at least about a factor of 1.07, at least about a factor of 1.08, at least about a factor of 1.09, at least about a factor of 1.1, at least about a factor of 1.2, at least about a factor of 1.3, at least about a factor of 1.4, at least about a factor of 1.5, at least about a factor of 1.6, at least about a factor of 1.7, at least about a factor of 1.8, at least about a factor of 1.9, at least about a factor of 2, at least about a factor of 3, at least about a factor of 4, at least about a factor of 5, at least about a factor of 6, at least about a factor of 7, at least about a factor of 8, at least about a factor of 9, at least about a factor of 10, at least about a factor of 20, at least about a factor of 30, at least about a factor of 40, at least about a factor of 50, at least about a factor of 60, at least about a factor of 70, at least about a factor of 80, at least about a factor of 90, at least about a factor of 100, at least about a factor of 200, at least about a factor of 300, at least about a factor of 400, at least about a factor of 500, at least about a factor of 600, at least about a factor of 700, at least about a factor of 800, at least about a factor of 900, or at least about a factor of 1000.

In some embodiments, the composition reduces the time it takes for the signal to saturate by at least about a factor of 1.01, at least about a factor of 1.02, at least about a factor of 1.03, at least about a factor of 1.04, at least about a factor of 1.05, at least about a factor of 1.06, at least about a factor of 1.07, at least about a factor of 1.08, at least about a factor of 1.09, at least about a factor of 1.1, at least about a factor of 1.2, at least about a factor of 1.3, at least about a factor of 1.4, at least about a factor of 1.5, at least about a factor of 1.6, at least about a factor of 1.7, at least about a factor of 1.8, at least about a factor of 1.9, at least about a factor of 2, at least about a factor of 3, at least about a factor of 4, at least about a factor of 5, at least about a factor of 6, at least about a factor of 7, at least about a factor of 8, at least about a factor of 9, at least about a factor of 10, at least about a factor of 20, at least about a factor of 30, at least about a factor of 40, at least about a factor of 50, at least about a factor of 60, at least about a factor of 70, at least about a factor of 80, at least about a factor of 90, at least about a factor of 100, at least about a factor of 200, at least about a factor of 300, at least about a factor of 400, at least about a factor of 500, at least about a factor of 600, at least about a factor of 700, at least about a factor of 800, at least about a factor of 900, or at least about a factor of 1000. In some embodiments, the additive is present at a concentration of at least about 1 nM, at least about 2 nM, at least about 3 nM, at least about 4 nM, at least about 5 nM, at least about 6 nM, at least about 7 nM, at least about 8 nM, at least about 9 nM, at least about 10 nM, at least about 20 nM, at least about 30 nM, at least about 40 nM, at least about 50 nM, at least about 60 nM, at least about 70 nM, at least about 80 nM, at least about 90 nM, at least about 100 nM, at least about 200 nM, at least about 300 nM, at least about 400 nM, at least about 500 nM, at least about 600 nM, at least about 700 nM, at least about 800 nM, at least about 900 nM, at least about 1 µM, at least about 2 µM, at least about 3 µM, at least about 4 µM, at least about 5 µM, at least about 6 µM, at least about 7 µM, at least about 8 µM, at least about 9 µM, at least about 10 µM, at least about 20 µM, at least about 30 µM, at least about 40 µM, at least about 50 µM, at least about 60 µM, at least about 70 µM, at least about 80 µM, at least about 90 µM, at least about 100 µM, at least about 200 µM, at least about 300 µM, at least about 400 µM, at least about 500 µM, at least about 600 µM, at least about 700 µM, at least about 800 µM, at least about 900 µM, at least about 1 mM, at least about 2 mM, at least about 3 mM, at least about 4 mM, at least about 5 mM, at least about 6 mM, at least about 7 mM, at least about 8 mM, at least about 9 mM, at least about 10 mM, at least about 20 mM, at least about 30 mM, at least about 40 mM, at least about 50 mM, at least about 60 mM, at least about 70 mM, at least about 80 mM, at least about 90 mM, at least about 100 mM, at least about 200 mM, at least about 300 mM, at least about 400 mM, at least about 500 mM, at least about 600 mM, at least about 700 mM, at least about 800 mM, at least about 900 mM, at least about 1 M, at least about 2 M, at least about 3 M, at least about 4 M, at least about 5 M, at least about 6 M, at least about 7 M, at least about 8 M, at least about 9 M, at least about 10 M.

### Assay Compositions with Thermostable Inorganic Pyrophosphatase

Any of the systems, methods, or devices described herein may comprise using thermostable inorganic pyrophosphatase (TIPP). In some embodiments, a composition for an assay may comprise TIPP. In some embodiments, the composition may comprise about 0.5 enzyme unit (U) per 10 µL of solution. In some embodiments, the composition may comprise at least about 0.1 U per 10 µL of solution. In some embodiments, the composition may comprise at most about 2 U per 10 µL of solution. In some embodiments, the composition may comprise at least about 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1, 2, 3, 4, 5, 6, 7, 8, 9, or 10 U per 10 µL of solution. In some embodiments, the composition may comprise at most about 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1, 2, 3, 4, 5, 6, 7, 8, 9, or 10 U per 10 µL of solution.

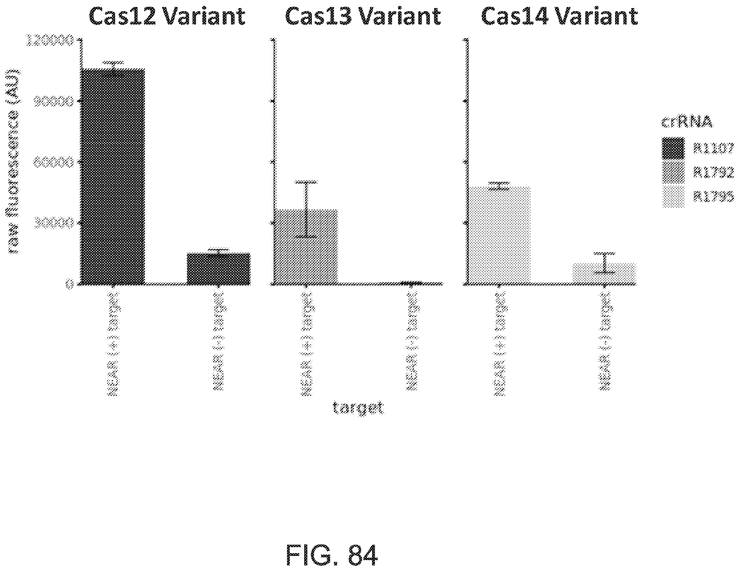

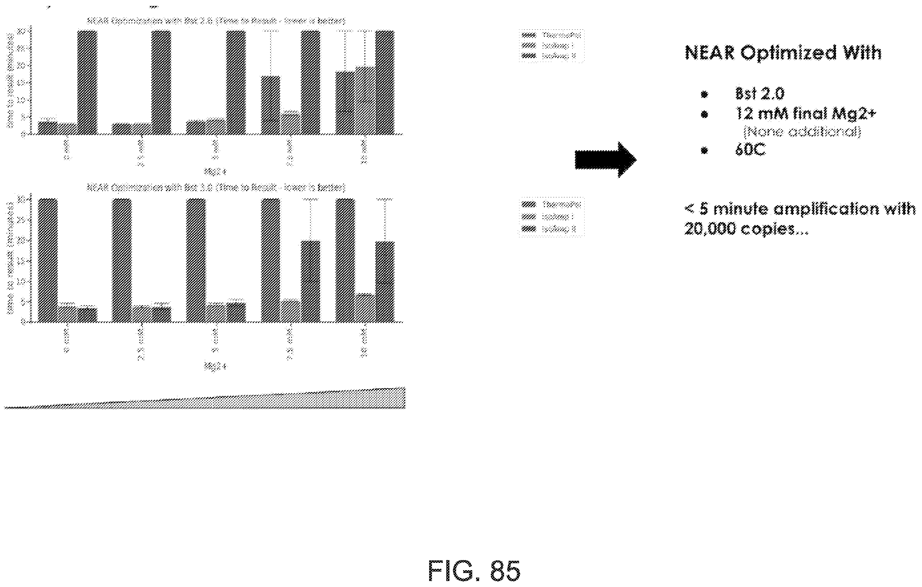

In some embodiments, TIPP may improve the signal to noise ratio of a programmable nuclease-based detection reaction. In some embodiments, TIPP may improve overall signal (e.g., fluorescence of a cleaved reporter as shown in **FIG. 84** or intensity of a lateral flow assay strip detection location as shown in **FIGS. 85A-85B).** TIPP may improve signal by a factor, wherein the signal is indicative of the presence of a target nucleic acid. In some embodiments, the factor may be at least about 1.1. In some embodiments, the factor may be at least about 2. In some embodiments, the factor may be at least about 3. In some embodiments, the factor may be at least about 4. In some embodiments, the factor may be at least about 5. In some embodiments, the factor may be at least about 6. In some embodiments, the factor may be at least about 7. In some embodiments, the factor may be at least about 8. In some embodiments, the factor may be at least about 9. In some embodiments, the factor may be at least about 10.

In some cases, the TIPP enzyme may be present in the HotPot reaction mix at a concentration of 0.125 Units, 0.5 Units, 0.25 Units, 1.0 Units, 2.0 Units, 2.5 Units, or 4 Units per discrete reaction volume. In some cases, the buffer may comprise the TIPP signal enhancement reagent. Conversely, the TIPP signal enhancement reagent may be provided separately (e.g., in a separate vial in a kit) and added to a reaction mixture at a desired timepoint.

In some cases, a buffer may comprise a catalytic reagent for signal improvement or enhancement. In some cases, the catalytic reagent may enhance signal generation via hydrolysis of inorganic pyrophosphates. In some cases, the catalytic reagent may enhance signal generation via enhancement of DNA replication. In some cases, the catalytic reagent may enhance signal amplification via revival of Mg⁺² ions in the buffer solution which may otherwise be taken up by the phosphates produced from usage of dNTPs during the LAMP reaction. In some cases, the catalytic reagent may enhance signal generation by reviving the concentration of Mg+2 ions in the buffer thereby enhancing the function of the Cas nuclease effector enzyme. In some cases, the catalytic reagent for signal improvement may be an enzyme. In some cases, the catalytic reagent for signal improvement may be a Thermostable Inorganic Pyrophosphatase (TIPP).

In some embodiments, the one or more enzymes may comprise a nuclease. In some embodiments, the one or more enzymes may comprise a programmable nuclease. In some embodiments, the one or more enzymes may comprise a Cas12 enzyme. In some embodiments, the one or more enzymes may comprise a Cas14 enzyme. In some embodiments, the one or more enzymes may comprise a CasPhi enzyme. In some embodiments, the one or more enzymes may comprise a Cas13 enzyme. In some embodiments, the one or more enzymes may comprise HRP. In some embodiments, the one or more enzymes may comprise any one or combination of enzymes presented in this disclosure.

In some embodiments, the one or more reporters may comprise a reporter free in solution. In some embodiments, the one or more reporters may comprise a reporter immobilized on a substrate. In some embodiments, the one or more reporters may comprise biotin. In some embodiments, the one or more reporters may comprise a fluorescent moiety. In some embodiments, the one or more reporters may comprise a nucleic acid tether. In some embodiments, the one or more reporters may comprise a linker. In some embodiments, the one or more reporters may comprise any one or combination of reporters presented in this disclosure.

In some embodiments, the one or more substrates may comprise a glassy substance. In some embodiments, the one or more substrates may comprise a polymeric substance. In some embodiments, the one or more substrates may comprise a hydrogel. In some embodiments, the one or more substrates may comprise any one or combination of substrates presented in this disclosure.

### Nicking Enzyme Amplification Reaction (NEAR)

In some embodiments, a target nucleic acid may be amplified using a nicking enzyme amplification reaction (NEAR). NEAR may be used to amplify a region of a nucleic acid comprising a target nucleic acid. NEAR may comprise a forward primer and a reverse primer that at least partially °ls to complementary strands of a target nucleic acid 3' of the region to be amplified. The forward primer and the reverse primer may comprise a stabilizing region that is not complementary to the target sequence. The forward primer and the reverse primer may comprise recognition regions that may be nicked by a nicking enzyme. A polymerase may polymerize a nucleic acid 5' to 3' from the 3' end of the forward primer or the 3' end of the reverse primer, using the strand to which the primer is annealed as a template, resulting in a double stranded nucleic acid product or amplicon. The newly synthesized strand may comprise a restriction site and may then serve as a template. The resulting double stranded nucleic acid amplicon may comprise nicking sites in both strands. A nicking enzyme may nick a single strand of the double stranded product or amplicon. The polymerase may polymerize a nucleic acid 5' to 3' from the 3' end of the nucleic acid amplicon 5' of the nick. The process may be repeated, thereby amplifying the target nucleic acid.

As described herein, a target nucleic acid may be detected using a DNA-activated programmable RNA nuclease (e.g., a Cas13), a DNA-activated programmable DNA nuclease (e.g., a Cas12), or an RNA-activated programmable RNA nuclease (e.g., a Cas13) and other reagents disclosed herein (e.g., RNA components). The target nucleic acid may be detected using DETECTR, as described herein. The target nucleic acid may be an RNA, reverse transcribed RNA, DNA, DNA amplicon, amplified DNA, synthetic nucleic acids, or nucleic acids found in biological or environmental samples. In some embodiments, the target nucleic acid is amplified prior to or concurrent with detection. In some embodiments, the target nucleic acid is reverse transcribed prior to amplification. The target nucleic acid may be amplified via NEAR of a target nucleic acid sequence. In some embodiments, the nucleic acid is amplified using NEAR coupled with reverse transcription (RT-NEAR). The NEAR amplification may be performed independently, or the NEAR amplification may be coupled to DETECTR for detection of the target nucleic acid. The RT-NEAR amplification may be performed independently, or the RT-NEAR amplification may be coupled to DETECTR for detection of the target nucleic acid. The DETECTR reaction may be performed using any method consistent with the methods disclosed herein.

NEAR may be performed as an isothermal reaction, for example NEAR may be performed at from about 37 °C to about 42 °C. In some embodiments, NEAR may be performed at from about 15 °C to about 60 °C, from about 15 °C to about 55 °C, from about 15 °C to about 50 °C, from about 15 °C to about 45 °C, from about 15 °C to about 40 °C, from about 15 °C to about 35 °C, from about 15 °C to about 30 °C, from about 15 °C to about 25 °C, from about 15 °C to about 20 °C, from about 20 °C to about 60 °C, from about 20 °C to about 55 °C, from about 20 °C to about 50 °C, from about 20 °C to about 45 °C, from about 20 °C to about 40 °C, from about 20 °C to about 35 °C, from about 20 °C to about 30 °C, from about 20 °C to about 25 °C, from about 25 °C to about 60 °C, from about 25 °C to about 55 °C, from about 25 °C to about 50 °C, from about 25 °C to about 45 °C, from about 25 °C to about 40 °C, from about 25 °C to about 35 °C, from about 25 °C to about 30 °C, from about 30 °C to about 60 °C, from about 30 °C to about 55 °C, from about 30 °C to about 50 °C, from about 30 °C to about 45 °C, from about 30 °C to about 40 °C, from about 30 °C to about 35 °C, from about 35 °C to about 60 °C, from about 35 °C to about 55 °C, from about 35 °C to about 50 °C, from about 35 °C to about 45 °C, from about 35 °C to about 40 °C, from about 40 °C to about 60 °C, from about 40 °C to about 55 °C, from about 40 °C to about 50 °C, from about 40 °C to about 45 °C, from about 45 °C to about 60 °C, from about 45 °C to about 55 °C, from about 45 °C to about 50 °C, from about 50 °C to about 60 °C, from about 50 °C to about 55 °C, or from about 55 °C to about 60 °C. In some embodiments, NEAR may be performed above about 15 °C, above about 20 °C, above about 25 °C, above about 30 °C, above about 35 °C, above about 40 °C, above about 45 °C, or above about 50 °C. In some embodiments, an SDA reaction may be performed below about 60 °C, below about 55 °C, below about 50 °C, below about 45 °C, below about 40 °C, below about 35 °C, below about 30 °C, below about 25 °C, below about 20 °C, or below about 15 °C. In some embodiments, NEAR may be performed at about room temperature. In some embodiments, a nucleic acid sample may be heated prior to isothermal amplification. In some embodiments, the nucleic acid sample heated prior to isothermal amplification may comprise one or more primers. The nucleic acid sample may be heated to about 95 °C prior to isothermal amplification. The nucleic acid sample may be heated to a temperature sufficient to dissociate two strands of a double stranded nucleic acid sequence.

NEAR may amplify a target nucleic acid to detectable levels within about 30 seconds, 5 minutes, about 10 minutes, about 15 minutes, about 20 minutes, about 25 minutes, about 30 minutes, about 35 minutes, about 40 minutes, about 45 minutes, about 50 minutes, about 55 minutes, or about 60 minutes. NEAR may amplify a target nucleic acid to detectable levels within about 1 hour, about 1.1 hours, about 1.2 hours, about 1.3 hours, about 1.4 hours, about 1.5 hours, about 1.6 hours, about 1.7 hours, about 1.8 hours, about 1.9 hours, about 2 hours, about 2.2 hours, about 2.4 hours, about 2.5 hours, about 2.6 hours, about 2.8 hours, about 3 hours, about 3.5 hours, about 4 hours, about 4.5 hours, or about 5 hours. NEAR may amplify a target nucleic acid to detectable levels within from about 0.1 hours to about 0.5 hours, from about 0.1 hours to about 1 hour, from about 0.1 hours to about 1.5 hours, from about 0.1 hours to about 2 hours, from about 0.1 hours to about 2.5 hours, from about 0.1 hours to about 3 hours, from about 0.1 hours to about 3.5 hours, from about 0.1 hours to about 4 hours, from about 0.1 hours to about 4.5 hours, from about 0.1 hours to about 5 hours, from about 0.5 hours to about 1 hour, from about 0.5 hours to about 1.5 hours, from about 0.5 hours to about 2 hours, from about 0.5 hours to about 2.5 hours, from about 0.5 hours to about 3 hours, from about 0.5 hours to about 3.5 hours, from about 0.5 hours to about 4 hours, from about 0.5 hours to about 4.5 hours, from about 0.5 hours to about 5 hours, from about 1 hour to about 1.5 hours, from about 1 hour to about 2 hours, from about 1 hour to about 2.5 hours, from about 1 hour to about 3 hours, from about 1 hour to about 3.5 hours, from about 1 hour to about 4 hours, from about 1 hour to about 4.5 hours, from about 1 hour to about 5 hours, from about 1.5 hours to about 2 hours, from about 1.5 hours to about 2.5 hours, from about 1.5 hours to about 3 hours, from about 1.5 hours to about 3.5 hours, from about 1.5 hours to about 4 hours, from about 1.5 hours to about 4.5 hours, from about 1.5 hours to about 5 hours, from about 2 hours to about 2.5 hours, from about 2 hours to about 3 hours, from about 2 hours to about 3.5 hours, from about 2 hours to about 4 hours, from about 2 hours to about 4.5 hours, from about 2 hours to about 5 hours, from about 2.5 hours to about 3 hours, from about 2.5 hours to about 3.5 hours, from about 2.5 hours to about 4 hours, from about 2.5 hours to about 4.5 hours, from about 2.5 hours to about 5 hours, from about 3 hours to about 3.5 hours, from about 3 hours to about 4 hours, from about 3 hours to about 4.5 hours, from about 3 hours to about 5 hours, from about 3.5 hours to about 4 hours, from about 3.5 hours to about 4.5 hours, from about 3.5 hours to about 5 hours, from about 4 hours to about 4.5 hours, from about 4 hours to about 5 hours, or from about 4.5 hours to about 5 hours.

### NEAR Amplification and Detection Reaction Mixtures

NEAR reaction components may comprise a polymerase, a nicking enzyme, dNTPs, and one or more nucleic acid primers. In some embodiments, the reaction may further comprise a reverse transcriptase as described herein. In some embodiments, the polymerase may be an exo-Klenow polymerase. The nicking enzyme may be capable of nicking a single strand of a double stranded nucleic acid sequence. In some embodiments, the nicking enzyme may be capable of nicking an unthiolated strand of a double stranded nucleic acid sequence comprising a thiolated strand and an unthiolated strand. In some embodiments, the nicking enzyme may be capable of nicking a single strand comprising an unthiolated region of a double stranded nucleic acid comprising at one or more thiolated regions and one or more unthiolated regions. In some embodiments, the nicking enzyme is a restriction enzyme capable of nicking a single strand of a double stranded nucleic acid sequence. In some embodiments, the nicking enzyme is a modified restriction enzyme. The nicking enzyme may be a strand-limited restriction enzyme. The restriction enzyme may be HincII. In some embodiments, the restriction enzyme may be AluI, BamHI, EcoP15I, EcoRI, EcoRII, EcoRV, HaeIII, HgaI, HindII, HindIII, HinFI, KpnI, NotI, PstI, PvuII, SacI, SalI, Sau3, ScaI, SmaI, SpeI, SphI, StuI, TaqI, or XbaI, or the like. The nicking enzyme may be Nt.BspQI, Nt.CvPII, Nt.BstNBI, Nb.BsrDI, Nb.BtsI, Nt.AlwI, Nb.BbvCI, Nb.BsmI, Nb.BssSI, Nt.BsmAI, Nb.Bpu101, Nt.Bpu110I, Nb.Mva1269I, or I-HmuI, or the like. The one or more nucleic acid primers may comprise two primers. For example, the one or more nucleic acid primers may comprise a first primer (e.g., an S1 primer) and a second primer (e.g., an S2 primer). The target nucleic acid may be single stranded DNA or double stranded DNA. In some embodiments, a target nucleic acid comprising RNA may be reverse transcribed into DNA using a reverse transcriptase prior to NEAR amplification. A reverse transcription reaction may comprise primers, dNTPs, and a reverse transcriptase. In some embodiments, the reverse transcription reaction and the NEAR amplification reaction may be performed in the same reaction. A combined RT-NEAR reaction may comprise NEAR primers, reverse transcription primers, dNTPs, a reverse transcriptase, a polymerase, and dNTPs. In some embodiment, the NEAR primers may comprise the reverse transcription primers.

A DETECTR reaction to detect the target nucleic acid sequence may comprise a guide nucleic acid comprising a segment that is reverse complementary to a segment of the target nucleic acid and a programmable nuclease. The programmable nuclease when activated, as described elsewhere herein, exhibits sequence-independent cleavage of a reporter (e.g., a nucleic acid comprising a moiety that becomes detectable upon cleavage of the nucleic acid by the programmable nuclease). The programmable nuclease is activated upon the guide nucleic acid hybridizing to the target nucleic acid. A combined NEAR DETECTR reaction may comprise a polymerase, a restriction enzyme, dNTPs, one or more nucleic acid primers, a guide nucleic acid, a programmable nuclease, and a substrate nucleic acid. A combined RT-NEAR DETECTR reaction may comprise reverse transcription primers, a reverse transcriptase, a polymerase, a restriction enzyme, dNTPs, one or more nucleic acid primers, a guide nucleic acid, a programmable nuclease, and a substrate nucleic acid. In some embodiment, the primers may comprise the reverse transcription primers. NEAR and DETECTR can be carried out in the same sample volume. NEAR and DETECTR can be carried out concurrently in separate sample volumes or in the same sample volume. RT-NEAR and DETECTR can be carried out in the same sample volume. RT-NEAR and DETECTR can be carried out concurrently in separate sample volumes or in the same sample volume. A NEAR reaction may be multiplexed to amplify a plurality of target nucleic acid sequences in a single reaction.

### NEAR Primers and Guide Nucleic Acids

A number of NEAR primers and NEAR primer design methods are consistent with the methods compositions, reagents, enzymes, and kits disclosed herein. NEAR may comprise a set of primers. In some embodiments, NEAR may be an RT-NEAR reaction, a NEAR DETECTR reaction, or an RT-NEAR DETECTR reaction. The set of NEAR primers may comprise two primers, a first primer and a second primer. In some embodiments, a first primer may comprise a sequence of the first region at the 3' end of the first primer. The sequence of the first region may be from about 16 nucleic acids to about 25 nucleic acids long, or about 20 nucleic acids long. The 3' end of the first primer may hybridize to the first complementary region of the target. The first complementary region may be 3' of the target nucleic acid. The first complementary region may be 3' of a sequence reverse complementary to the target nucleic acid. The first primer may further comprise a cut site 5' of the sequence of the first region that may be recognized and cleaved by a nicking enzyme. The 3' end of the first primer may further comprise a recognition site for a nicking enzyme. In some embodiments, the 3' end of the first primer may further comprise a nicking enzyme stabilization region. The sequence of the second region may be from about 30 nucleic acids to about 38 nucleic acids long. The 3' end of the second primer may hybridize to the second complementary region. The second complementary region may be 3' of a sequence reverse complementary to the target nucleic acid. The second complementary region may be 3' of the target nucleic acid. The second complementary region may be 3' of a sequence reverse complementary to the target nucleic acid. The second primer may further comprise a cut site 5' of the sequence of the second region that may be recognized and cleaved by a nicking enzyme. The 3' end of the second primer may further comprise a recognition site for a nicking enzyme. In some embodiments, the 3' end of the second primer may further comprise a nicking enzyme stabilization region.

The NEAR primers are designed depending on the site of the optimal guide RNA placement, which may or may not be determined by an available PAM sequence. When performing a NEAR-DETECTR reaction, single-stranded DNA is produced by the designed primers. Because the DETECTR reaction will detection single stranded DNA species, the amplification reaction can be biased to produce more of the particular strand than another. This can be done through changing of the ratio of the forward and reverse primer concentrations. In some embodiments, the concentration of forward primer can be 5 times, 4 times, 3 times, 2 times, or equal to the concentration of reverse primer. In some embodiments, the concentration of reverse primer can be 5 times, 4 times, 3 times, 2 times, or equal to the concentration of forward primer.

In some embodiments, the first region, the second region, or both may be about 8 nucleic acids, about 10 nucleic acids, about 12 nucleic acids, about 14 nucleic acids, about 16 nucleic acids, about 18 nucleic acids, about 20 nucleic acids, about 22 nucleic acids, about 24 nucleic acids, about 26 nucleic acids, about 28 nucleic acids, about 30 nucleic acids, about 32 nucleic acids, about 34 nucleic acids, about 36 nucleic acids, about 38 nucleic acids, about 40 nucleic acids, about 42 nucleic acids, about 44 nucleic acids, about 46 nucleic acids, about 48 nucleic acids, or about 50 nucleic acids long.

In some embodiments, the first region, the second region, or both may be from about 8 to about 12, from about 8 to about 16, from about 8 to about 20, from about 8 to about 24, from about 8 to about 28, from about 8 to about 30, from about 8 to about 32, from about 8 to about 34, from about 8 to about 36, from about 8 to about 38, from about 8 to about 40, from about 8 to about 42, from about 8 to about 44, from about 8 to about 48, from about 8 to about 50, from about 12 to about 16, from about 12 to about 20, from about 12 to about 24, from about 12 to about 28, from about 12 to about 30, from about 12 to about 32, from about 12 to about 34, from about 12 to about 36, from about 12 to about 38, from about 12 to about 40, from about 12 to about 42, from about 12 to about 44, from about 12 to about 48, from about 12 to about 50, from about 16 to about 20, from about 16 to about 24, from about 16 to about 28, from about 16 to about 30, from about 16 to about 32, from about 16 to about 34, from about 16 to about 36, from about 16 to about 38, from about 16 to about 40, from about 16 to about 42, from about 16 to about 44, from about 16 to about 48, from about 16 to about 50, from about 20 to about 24, from about 20 to about 28, from about 20 to about 30, from about 20 to about 32, from about 20 to about 34, from about 20 to about 36, from about 20 to about 38, from about 20 to about 40, from about 20 to about 42, from about 20 to about 44, from about 20 to about 48, from about 20 to about 50, from about 24 to about 28, from about 24 to about 30, from about 24 to about 32, from about 24 to about 34, from about 24 to about 36, from about 24 to about 38, from about 24 to about 40, from about 24 to about 42, from about 24 to about 44, from about 24 to about 48, from about 24 to about 50, from about 28 to about 30, from about 28 to about 32, from about 28 to about 34, from about 28 to about 36, from about 28 to about 38, from about 28 to about 40, from about 28 to about 42, from about 28 to about 44, from about 28 to about 48, from about 28 to about 50, from about 30 to about 32, from about 30 to about 34, from about 30 to about 36, from about 30 to about 38, from about 30 to about 40, from about 30 to about 42, from about 30 to about 44, from about 30 to about 48, from about 30 to about 50, from about 32 to about 34, from about 32 to about 36, from about 32 to about 38, from about 32 to about 40, from about 32 to about 42, from about 32 to about 44, from about 32 to about 48, from about 32 to about 50, from about 34 to about 36, from about 34 to about 38, from about 34 to about 40, from about 34 to about 42, from about 34 to about 44, from about 34 to about 48, from about 34 to about 50, from about 36 to about 38, from about 36 to about 40, from about 36 to about 42, from about 36 to about 44, from about 36 to about 48, from about 36 to about 50, from about 38 to about 40, from about 38 to about 42, from about 38 to about 44, from about 38 to about 48, from about 38 to about 50, from about 40 to about 42, from about 40 to about 44, from about 40 to about 48, from about 40 to about 50, from about 42 to about 44, from about 42 to about 48, from about 42 to about 50, from about 44 to about 48, from about 44 to about 50, from about 48 to about 50 nucleic acids long.

In some embodiments, the first region, the second region, or both may comprise a GC content of about 1%, about 5%, about 10%, about 15%, about 20%, about 25%, about 30%, about 40%, about 50%, about 60%, about 70%, about 80%, about 90%, about 95%, or about 99%. In some embodiments, the first region, the second region, or both may comprise a GC content of from about 1% to about 5%, from about 1% to about 10%, from about 1% to about 15%, from about 1% to about 20%, from about 1% to about 25%, from about 1% to about 30%, from about 1% to about 40%, from about 1% to about 50%, from about 1% to about 60%, from about 1% to about 70%, from about 1% to about 80%, from about 1% to about 90%, from about 1% to about 95%, from about 1% to about 99%, from about 5% to about 10%, from about 5% to about 15%, from about 5% to about 20%, from about 5% to about 25%, from about 5% to about 30%, from about 5% to about 40%, from about 5% to about 50%, from about 5% to about 60%, from about 5% to about 70%, from about 5% to about 80%, from about 5% to about 90%, from about 5% to about 95%, from about 5% to about 99%, from about 10% to about 15%, from about 10% to about 20%, from about 10% to about 25%, from about 10% to about 30%, from about 10% to about 40%, from about 10% to about 50%, from about 10% to about 60%, from about 10% to about 70%, from about 10% to about 80%, from about 10% to about 90%, from about 10% to about 95%, from about 10% to about 99%, from about 15% to about 20%, from about 15% to about 25%, from about 15% to about 30%, from about 15% to about 40%, from about 15% to about 50%, from about 15% to about 60%, from about 15% to about 70%, from about 15% to about 80%, from about 15% to about 90%, from about 15% to about 95%, from about 15% to about 99%, from about 20% to about 25%, from about 20% to about 30%, from about 20% to about 40%, from about 20% to about 50%, from about 20% to about 60%, from about 20% to about 70%, from about 20% to about 80%, from about 20% to about 90%, from about 20% to about 95%, from about 20% to about 99%, from about 25% to about 30%, from about 25% to about 40%, from about 25% to about 50%, from about 25% to about 60%, from about 25% to about 70%, from about 25% to about 80%, from about 25% to about 90%, from about 25% to about 95%, from about 25% to about 99%, from about 30% to about 40%, from about 30% to about 50%, from about 30% to about 60%, from about 30% to about 70%, from about 30% to about 80%, from about 30% to about 90%, from about 30% to about 95%, from about 30% to about 99%, from about 40% to about 50%, from about 40% to about 60%, from about 40% to about 70%, from about 40% to about 80%, from about 40% to about 90%, from about 40% to about 95%, from about 40% to about 99%, from about 50% to about 60%, from about 50% to about 70%, from about 50% to about 80%, from about 50% to about 90%, from about 50% to about 95%, from about 50% to about 99%, from about 60% to about 70%, from about 60% to about 80%, from about 60% to about 90%, from about 60% to about 95%, from about 60% to about 99%, from about 70% to about 80%, from about 70% to about 90%, from about 70% to about 95%, from about 70% to about 99%, from about 80% to about 90%, from about 80% to about 95%, from about 80% to about 99%, from about 90% to about 95%, from about 90% to about 99%, or from about 95% to about 99%.

In some embodiments, the first region, the second region, or both may have a melting temperature of about 38 °C, about 40 °C, about 42 °C, about 44 °C, about 46 °C, about 48 °C, about 50 °C, about 52 °C, about 54 °C, about 56 °C, about 58 °C, about 60 °C, about 62 °C, about 64 °C, about 66 °C, about 68 °C, about 70 °C, about 72 °C, about 74 °C, about 76 °C, about 78 °C, about 80 °C, about 82 °C, about 84 °C, about 86 °C, about 88 °C, about 90 °C, or about 92 °C. In some embodiments, the first region, the second region, or both may have a melting temperature of from about 35 °C to about 40 °C, from about 35 °C to about 45 °C, from about 35 °C to about 50 °C, from about 35 °C to about 55 °C, from about 35 °C to about 60 °C, from about 35 °C to about 65 °C, from about 35 °C to about 70 °C, from about 35 °C to about 75 °C, from about 35 °C to about 80 °C, from about 35 °C to about 85 °C, from about 35 °C to about 90 °C, from about 35 °C to about 95 °C, from about 40 °C to about 45 °C, from about 40 °C to about 50 °C, from about 40 °C to about 55 °C, from about 40 °C to about 60 °C, from about 40 °C to about 65 °C, from about 40 °C to about 70 °C, from about 40 °C to about 75 °C, from about 40 °C to about 80 °C, from about 40 °C to about 85 °C, from about 40 °C to about 90 °C, from about 40 °C to about 95 °C, from about 45 °C to about 50 °C, from about 45 °C to about 55 °C, from about 45 °C to about 60 °C, from about 45 °C to about 65 °C, from about 45 °C to about 70 °C, from about 45 °C to about 75 °C, from about 45 °C to about 80 °C, from about 45 °C to about 85 °C, from about 45 °C to about 90 °C, from about 45 °C to about 95 °C, from about 50 °C to about 55 °C, from about 50 °C to about 60 °C, from about 50 °C to about 65 °C, from about 50 °C to about 70 °C, from about 50 °C to about 75 °C, from about 50 °C to about 80 °C, from about 50 °C to about 85 °C, from about 50 °C to about 90 °C, from about 50 °C to about 95 °C, from about 55 °C to about 60 °C, from about 55 °C to about 65 °C, from about 55 °C to about 70 °C, from about 55 °C to about 75 °C, from about 55 °C to about 80 °C, from about 55 °C to about 85 °C, from about 55 °C to about 90 °C, from about 55 °C to about 95 °C, from about 60 °C to about 65 °C, from about 60 °C to about 70 °C, from about 60 °C to about 75 °C, from about 60 °C to about 80 °C, from about 60 °C to about 85 °C, from about 60 °C to about 90 °C, from about 60 °C to about 95 °C, from about 65 °C to about 70 °C, from about 65 °C to about 75 °C, from about 65 °C to about 80 °C, from about 65 °C to about 85 °C, from about 65 °C to about 90 °C, from about 65 °C to about 95 °C, from about 70 °C to about 75 °C, from about 70 °C to about 80 °C, from about 70 °C to about 85 °C, from about 70 °C to about 90 °C, from about 70 °C to about 95 °C, from about 75 °C to about 80 °C, from about 75 °C to about 85 °C, from about 75 °C to about 90 °C, from about 75 °C to about 95 °C, from about 80 °C to about 85 °C, from about 80 °C to about 90 °C, from about 80 °C to about 95 °C, from about 85 °C to about 90 °C, from about 85 °C to about 95 °C, or from about 90 °C to about 95 °C.

A set of NEAR primers may be designed for use in combination with a DETECTR reaction. Exemplary NEAR primer sequences are provided in Table 19 below. The amplified nucleic acid sequence may comprise a sequence that hybridizes to a guide RNA. The amplified nucleic acid sequence may comprise a target nucleic acid. The guide RNA may hybridize to the target nucleic acid. The amplified nucleic acid sequence may comprise corresponding to a guide RNA. The amplified nucleic acid sequence may comprise a sequence reverse complementary to the target nucleic acid. All or part of the guide RNA may be reverse complementary to all or part of the target nucleic acid. The amplified nucleic acid sequence may comprise a protospacer adjacent motif (PAM) positioned next to or near the target sequence. The PAM may be 3' of the target nucleic acid. In some embodiments, a portion of a sequence that hybridizes the guide RNA may be located between the first region and the second complementary region. The portion of a sequence that hybridizes the guide RNA located between the first region and the second complementary region may comprise at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 99%, or more of the sequence that hybridizes guide RNA. In some embodiments, the 5' end of the sequence that hybridizes the guide RNA is 3' of the 3' end of the first region and 3' end of the sequence that hybridizes the guide RNA is 5' of the 5' end of the second complementary region. In some embodiments, a portion of a sequence that hybridizes the guide RNA may be located between the second region and the first complementary region. The portion of a sequence that hybridizes the guide RNA located between the second region and the first complementary region may comprise at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 99%, or more of the sequence that hybridizes the guide RNA. In some embodiments, the 5' end of the sequence that hybridizes the guide RNA is 3' of the 3' end of the second region and 3' end of the sequence that hybridizes the guide RNA is 5' of the 5' end of the first complementary region.

**In** some embodiments, a sequence that hybridizes the guide RNA may overlap the first region, the first complementary region, the second region, or the second complementary region by no more than no more than about 5%, no more than about 10%, no more than about 15%, no more than about 20%, no more than about 25%, no more than about 30%, no more than about 35%, no more than about 40%, no more than about 45%, no more than about 50%, no more than about 55%, no more than about 60%, no more than about 65%, no more than about 70%, no more than about 75%, no more than about 80%, no more than about 85%, no more than about 90%, or no more than about 95%. In some embodiments, the sequence that hybridizes the guide RNA does not overlap the first region, the first complementary region, the second region, or the second complementary region. In some embodiments, the guide RNA does not hybridize to the first primer or the second primer.

In some embodiments, a NEAR primer set may be designed using a commercially available primer design software. A NEAR primer set may be designed for use in combination with a DETECR reaction, a reverse transcription reaction, or both. One or more methods of designing a set of NEAR primers may be readily apparent to one skilled in the art and may be employed in any of the compositions, kits and methods described herein.

**TABLE 19 - Exemplary NEAR Primers**

| **SEQ ID NO:** | PRIMER NAME | ALTERNATIVE NAME | SEQUENCE |
|---|---|---|---|
| **SEQ ID NO: 377** | M2805 | COV2-M1 | TTGCTTTCGTGGTA TT |
| **SEQ ID NO: 378** | M2811 | COV2-M2 | GGATGGCTAGTGT AA |
| **SEQ ID NO: 379** | R1763 F8 | COV2-F8 | TTTGCCCCCAGCG CTT |
| **SEQ ID NO: 380** | R1763 R8 | COV2-R8 | ATGCGCGACATTC CGA |
| **SEQ ID NO: 381** | R1763 F7 | COV2-F7 | ATTTGCCCCCAGC GCT |
| **SEQ ID NO: 382** | R1763 R7 | COV2-R7 | TGCGCGACATTCC GAA |
| **SEQ ID NO: 383** | R1763 F6 | COV2-F6 | AATTTGCCCCCAG CGC |
| **SEQ ID NO: 384** | R1763 R6 | COV2-R6 | GCGCGACATTCCG AAG |
| **SEQ ID NO: 385** | R1763 F5 | COV2-F5 | CAATTTGCCCCCA GCG |
| **SEQ ID NO: 386** | R1763 R5 | COV2-R5 | CGCGACATTCCGA AGA |
| **SEQ ID NO: 387** | R1763 F4 | COV2-F4 | ACAATTTGCCCCC AGC |
| **SEQ ID NO: 388** | R1763 R4 | COV2-R4 | GCGACATTCCGAA GAA |
| **SEQ ID NO: 389** | R1763 F3 | COV2-F3 | CACAATTTGCCCC CAG |
| **SEQ ID NO: 390** | R1763 R3 | COV2-R3 | CGACATTCCGAAG AAC |
| **SEQ ID NO: 391** | R1763 F2 | COV2-F2 | TCACAATTTGCCC CCA |
| **SEQ ID NO: 392** | R1763 R2 | COV2-R2 | GACATTCCGAAGA ACG |
| **SEQ ID NO: 393** | R1763 F1 | COV2-F1 | TTCACAATTTGCC CCC |
| **SEQ ID NO: 394** | R1763 R1 | COV2-R1 | ACATTCCGAAGAA CGC |
| **SEQ ID NO: 395** | EXAMPLE PRIMIER 1F | EXAMPLE PRIMER 1F | TAGCCAAACAGGA ACA |
| **SEQ ID NO: 396** | EXAMPLE PRIMER 2R | EXAMPLE PRIMER 2R | GCCAGCAATCTGA GGA |

### Amplification and Detection of a Gene of Interest

A DETECTR reaction may be used to detect the presence of a specific target gene in the same. The DETECTR reaction may produce a detectable signal, as described elsewhere herein, in the presence of a target nucleic acid sequence comprising a target gene. The DETECTR reaction may not produce a signal in the absence of the target nucleic acid or in the presence of a nucleic acid sequence that does not comprise the specific SNP allele or comprises a different SNP allele. In some embodiments, a DETECTR reaction may comprise a guide RNA reverse complementary to a portion of a target nucleic acid sequence comprising a specific SNP allele. The guide RNA and the target nucleic acid comprising the specific SNP allele may bind to and activate a programmable nuclease, thereby producing a detectable signal as described elsewhere herein. The guide RNA and a nucleic acid sequence that does not comprise the specific SNP allele may not bind to or activate the programmable nuclease and may not produce a detectable signal. In some embodiments, a target nucleic acid sequence that may or may not comprise a specific SNP allele may be amplified using, for example, a LAMP amplification reaction, an RPA amplification reaction, an SDA amplification reaction, a NEAR amplification reaction, or any other amplification method. In some embodiments, the amplification reaction may be combined with a reverse transcription reaction, a DETECTR reaction, or both. For example, the amplification reaction may be an RT-NEAR reaction, a NEAR DETECTR reaction, or an RT-NEAR DETECTR reaction. In some embodiments, the target nucleic acid sequence can comprise a SNP. In some embodiments, the target nucleic acid sequence can comprise a sequence indicative of a human disease.

A DETECTR reaction, as described elsewhere herein, may produce a detectable signal specifically in the presence of a target nucleic acid sequence comprising a target gene. In addition to the DETECTR reaction, the target nucleic acid having the target gene may be concurrently, sequentially, concurrently together in a sample, or sequentially together in a sample be carried out alongside NEAR or RT- NEAR. For example, the reactions can comprise NEAR and DETECTR reactions, or RT- NEAR and DETECTR reactions. Performing a DETECTR reaction in combination with a NEAR reaction may result in an increased detectable signal as compared to the DETECTR reaction in the absence of the NEAR reaction. In some embodiments, the target nucleic acid sequence can comprise a SNP. In some embodiments, the target nucleic acid sequence can comprise a sequence indicative of a human disease.

In some embodiments, the detectable signal produced in the DETECTR reaction may be higher in the presence of a target nucleic acid comprising target nucleic acid than in the presence of a nucleic acid that does not comprise the target nucleic acid. In some embodiments, the DETECTR reaction may produce a detectable signal that is at least 1-fold, at least 2-fold, at least 3-fold, at least 4-fold, at least 5-fold, at least 10-fold, at least 20-fold, at least 30-fold, at least 40-fold, at least 50-fold, at least 100-fold, at least 200-fold, at least 300-fold, at last 400-fold, at least 500-fold, at least 1000-fold, at least 2000-fold, at least 3000-fold, at least 4000-fold, at least 5000-fold, at least 6000-fold, at least 7000-fold, at least 8000-fold, at least 9000-fold, at least 10000-fold, at least 50000-fold, at least 100000-fold, at least 500000-fold, or at least 1000000-fold greater in the presence of a target nucleic acid comprising a target nucleic acid than in the presence of a nucleic acid that does not comprise the target nucleic acid. In some embodiments, the DETECTR reaction may produce a detectable signal that is from 1-fold to 2-fold, from 2-fold to 3-fold, from 3-fold to 4-fold, from 4-fold to 5-fold, from 5-fold to 10-fold, from 10-fold to 20-fold, from 20-fold to 30-fold, from 30-fold to 40-fold, from 40-fold to 50-fold, from 50-fold to 100-fold, from 100-fold to 500-fold, from 500-fold to 1000-fold, from 1000-fold to 10,000-fold, from 10,000-fold to 100,000-fold, or from 100,000-fold to 1,000,000-fold greater in the presence of a target nucleic acid comprising a specific SNP allele than in the presence of a nucleic acid that does not comprise the specific SNP allele. In some embodiments, the target nucleic acid sequence can comprise a SNP. In some embodiments, the target nucleic acid sequence can comprise a sequence indicative of a human disease.

A DETECTR reaction may be used to detect the presence of a target nucleic acid associated with a disease or a condition in a nucleic acid sample. The DETECTR reaction may reach signal saturation within about 30 seconds, about 5 minutes, about 10 minutes, about 15 minutes, about 20 minutes, about 25 minutes, about 30 minutes, about 35 minutes, about 40 minutes, about 45 minutes, about 50 minutes, about 55 minutes, about 60 minutes, about 65 minutes, about 75 minutes, about 80 minutes, or about 85 minutes and be used to detect the presence of a target gene associated with an increased likelihood of developing a disease or a condition in a nucleic acid sample. The DETECTR reaction may be used to detect the presence of a target gene associated with a phenotype in a nucleic acid sample. For example, a DETECTR reaction may be used to detect target gene associated with a disease such as phenylketonuria (PKU), cystic fibrosis, sickle-cell anemia, albinism, Huntington's disease, myotonic dystrophy type 1, hypercholesterolemia, neurofibromatosis, polycystic kidney disease, hemophilia, muscular dystrophy, hypophosphatemic rickets, Rett's syndrome, or spermatogenic failure. A DETECTR reaction may be used to detect a SNP allele associated with an increased risk of cancer, for example bladder cancer, brain cancer, breast cancer, cervical cancer, colon cancer, colorectal cancer, gallbladder cancer, stomach cancer, leukemia, liver cancer, lung cancer, oral cancer, esophageal cancer, ovarian cancer, pancreatic cancer, prostate cancer, skin cancer, testicular cancer, thyroid cancer, neuroblastoma, or lymphoma. A DETECTR reaction may be used to detect a SNP allele associated with an increased risk of a disease, for example Alzheimer's disease, Parkinson's disease, amyloidosis, heterochromatosis, celiac disease, macular degeneration, or hypercholesterolemia. A DETECTR reaction may be used to detect a SNP allele associated with a phenotype, for example, eye color, hair color, height, skin color, race, alcohol flush reaction, caffeine consumption, deep sleep, genetic weight, lactose intolerance, muscle composition, saturated fat and weight, or sleep movement. A DETECTR reaction may also be used to detect the presence of a pathological organism. In some embodiments, the pathological organism is a prokaryote, eukaryote, or a protozoa. In some embodiments, the pathological organism is a virus, an opportunistic pathogen, a parasite, a bacterium, or any combination thereof. In some embodiments, the pathological organism is SARS-CoV-2 or *Streptococcus pyogenes.*

### Detection Devices

In another aspect, the present disclosure provides a detection device or system for target detection. The detection device can be configured for multiplexed target detection. The detection device can be used to collect one or more samples, prepare or process the one or more samples for detection, and optionally divide the one or more samples into a plurality of droplets, aliquots, or subsamples for amplification of one or more target sequences or target nucleic acids. The target sequences may comprise, for example, a biological sequence. The biological sequence can comprise a nucleic acid sequence or an amino acid sequence. In some embodiments, the target sequences are associated with an organism of interest, a disease of interest, a disease state of interest, a phenotype of interest, a genotype of interest, or a gene of interest.

The detection device can be configured to amplify target nucleic acids contained within the plurality of droplets, aliquots, or subsamples. The detection device can be configured to amplify the target sequences or target nucleic acids contained within the plurality of droplets by individually processing each of the plurality of droplets (e.g., by using a thermocycling process or any other suitable amplification process as described in greater detail below). In some cases, the plurality of droplets can undergo separate thermocycling processes. In some cases, the thermocycling processes can occur simultaneously. In other cases, the thermocycling processes can occur at different times for each droplet.

The detection device can be further configured to remix the droplets, aliquots, or subsamples after the target nucleic acids in each of the droplets undergo amplification. The detection device can be configured to provide the remixed sample comprising the droplets, aliquots, or subsamples to a detection chamber of the device. The detection chamber can be configured to direct the remixed droplets, aliquots, or subsamples to a plurality of programmable nuclease complexes (i.e., programmable nuclease in complex with a guide nucleic acid). The detection chamber can be configured to direct the remixed droplets, aliquots, or subsamples along one or more fluid flow paths such that the remixed droplets, aliquots, or subsamples are positioned adjacent to and/or in contact with the one or more programmable nuclease complexes (i.e., programmable nuclease in complex with a guide nucleic acid). In some cases, the detection chamber can be configured to recirculate or recycle the remixed droplets, aliquots, or subsamples such that the remixed droplets, aliquots, or subsamples are repeatedly placed in contact with one or more programmable nuclease complexes (i.e., programmable nuclease in complex with a guide nucleic acid) over a predetermined period of time.

The detection device can comprise one or more sensors. The one or more sensors of the detection device can be configured to detect one or more signals that are generated after one or more programmable nucleases of the one or more programmable nuclease complexes (i.e., programmable nuclease in complex with a guide nucleic acid) become activated due to a binding of a guide nucleic acid of the programmable nuclease complexes (i.e., programmable nuclease in complex with a guide nucleic acid) with a target nucleic acid present in the sample. As described elsewhere herein, the activated programmable nuclease can cleave the target nucleic acid, which can result in a trans cleavage activity. Trans cleavage activity can be a non-specific cleavage of nearby single-stranded nucleic acids by the activated programmable nuclease, such as trans cleavage of target nucleic acids with a detection moiety. Once the target nucleic acids are cleaved by the activated programmable nucleases, the detection moiety can be released or separated from the reporter, thereby generating one or more detectable signals. The one or more sensors of the detection device can be configured to register and/or process the one or more detectable signals to confirm a presence and/or an absence of a particular target (e.g., a target nucleic acid).

A number of detection devices, systems, and methods are consistent with methods disclosed herein. In some embodiments, a detection device may be used to identify and/or quantify a detectable signal produced by a DETECTR reaction in which amplification and detection are performed in a single buffer. A detection device may procure a signal from a container (e.g., a cuvette , a well, a tube, a centricon, a fluidic channel, a fluidic chamber, or the like) or plurality of containers. For example, a detection device may be capable of detecting signals (e.g., fluorescence signals) from a plurality of wells in a multi well plate. A container may be sealed during detection. For example, a container may comprise a septum or film that the sample can be inserted (e.g., syringed) through, and the container may be placed next to or inside of the detection device after insertion of the sample, without removal of the seal. A device may be configured to simultaneously monitor multiple containers, such as multiple wells within a well plate. A device may perform real time signal monitoring.

A device and/or instrument/system thermally-coupled thereto may be configured to heat a sample (e.g., a sample container). A device may be configured to enact a programmed heating sequence (e.g., holding a sample at a first temperature for a first length of time and then holding the sample at a second temperature for a second length of time). For a sample comprising amplification and programmable nuclease-based detection (e.g., DETECTR) reagents, the programmed heating sequence may first hold the sample temperature or thermocycle the sample at an optimum temperature(s) for amplification and then transition the sample to an optimum temperature for detection (e.g., between 60 and 65 °C for an amplification reaction and between 30 and 40 °C for a DETECTR reaction). In another example, the programming sequence may hold the sample mixture comprising amplification and programmable nuclease-based detection (e.g., DETECTR) reagents at a temperature selected to enable simultaneous amplification and detection (e.g., between 50 to 65°C for a HotPot reaction). A device may perform simultaneous heating and signal detection. For example, a device may comprise a heating block and a fluorescence imaging system.

A device can measure or detect a calorimetric, potentiometric, amperometric, optical (e.g., fluorescent, colorimetric, etc.), or piezo-electric signal. Often a calorimetric signal is heat produced after cleavage of the reporters. Sometimes, a calorimetric signal is heat absorbed after cleavage of the reporters. A potentiometric signal, for example, is electrical potential produced after cleavage of the reporters. An amperometric signal can be movement of electrons produced after the cleavage of a reporter. Often, the signal is an optical signal, such as a colorimetric signal or a fluorescence signal. An optical signal is, for example, a light output produced after the cleavage of the reporters. Sometimes, an optical signal is a change in light absorbance between before and after the cleavage of reporters. Often, a piezo-electric signal is a change in mass between before and after the cleavage of the reporter. Sometimes, the reporter is protein-nucleic acid. Often, the protein-nucleic acid is an enzyme-nucleic acid.

The results from the detection region (e.g., from a container) from a completed assay can be detected and analyzed in various ways, for example, by a glucometer. In some cases, the positive control spot and the detection spot in the detection region is visible by eye, and the results can be read by the user. In some cases, the positive control spot and the detection spot in the detection region is visualized by an imaging device or other device depending on the type of signal. Often, the imaging device is a digital camera, such a digital camera on a mobile device. The mobile device may have a software program or a mobile application that can capture an image of the support medium, identify the assay being performed, detect the detection region and the detection spot, provide image properties of the detection spot, analyze the image properties of the detection spot, and provide a result. Alternatively, or in combination, the imaging device can capture fluorescence, ultraviolet (UV), infrared (IR), or visible wavelength signals. The imaging device may have an excitation source to provide the excitation energy and captures the emitted signals. In some cases, the excitation source can be a camera flash and optionally a filter. In some cases, the imaging device is used together with an imaging box that is placed over the support medium to create a dark room to improve imaging. The imaging box can be a cardboard box that the imaging device can fit into before imaging. In some instances, the imaging box has optical lenses, mirrors, filters, or other optical elements to aid in generating a more focused excitation signal or to capture a more focused emission signal. Often, the imaging box and the imaging device are small, handheld, and portable to facilitate the transport and use of the assay in remote or low resource settings.

The assay described herein can be visualized and analyzed by a mobile application (app) or a software program. Using the graphic user interface (GUI) of the app or program, an individual can take an image of the support medium, including the detection region, barcode, reference color scale, and fiduciary markers on the housing, using a camera on a mobile device. The program or app reads the barcode or identifiable label for the test type, locate the fiduciary marker to orient the sample, and read the detectable signals, compare against the reference color grid, and determine the presence or absence of the target nucleic acid, which indicates the presence of the gene, virus, or the agent responsible for the disease. The mobile application can present the results of the test to the individual. The mobile application can store the test results in the mobile application. The mobile application can communicate with a remote device and transfer the data of the test results. The test results can be viewable remotely from the remote device by another individual, including a healthcare professional. A remote user can access the results and use the information to recommend action for treatment, intervention, cleanup of an environment.

### Methods of Making Polymer Matrices with Immobilized Reporters

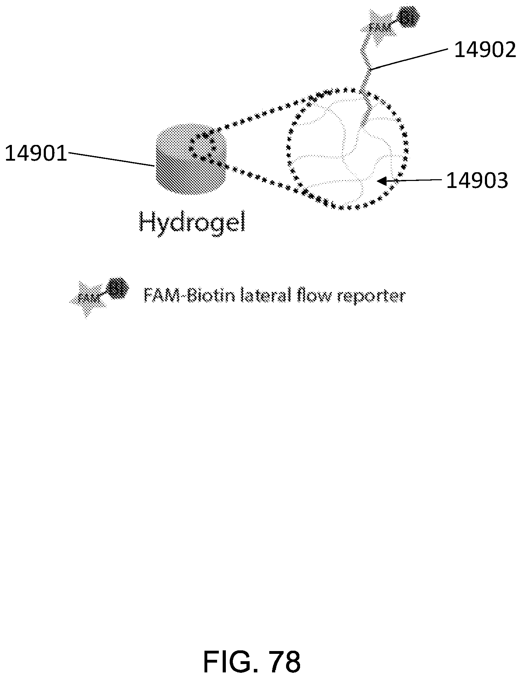

**FIG. 78** shows an exemplary polymer immobilization matrix (14901) comprising a plurality of immobilized DETECTR reaction components. The DETECTR reaction components may comprise one or more reporters, one or more programmable nucleases, and/or one or more guide nucleic acids. In some embodiments, the polymer matrix may comprise a hydrogel. In the exemplary embodiment shown in **FIG. 78****,** a plurality of reporters (14902) may be immobilized within a hydrogel (14901) matrix. In some embodiments, methods of immobilizing a reporter (14902) and/or other DETECTR reaction component may comprise (a) providing a polymerizable composition comprising: (i) a plurality of oligomers, (ii) a plurality of polymerizable (e.g., functionalized) oligomers, (iii) a set of polymerizable (e.g., functionalized) reporters (and/or other DETECTR reaction components), and (iv) a set of polymerization initiators; and (b) initiating the polymerization reaction by providing an initiation stimulus.

Co-polymerization of the reporter into the hydrogel may result in a higher density of reporter/unit volume or reporter/unit area than other immobilization methods utilizing surface immobilization (e.g., onto beads). Co-polymerization of the reporter into the hydrogel may result in less undesired release of the reporter (e.g., during an assay, a measurement, or on the shelf), and thus may cause less background signal, than other immobilization strategies (e.g., conjugation to a pre-formed hydrogel, bead, etc.). In at least some instances this may be due to better incorporation of reporters into the hydrogel as a co-polymer and fewer "free" reporter molecules retained on the hydrogel via non-covalent interactions or non-specific binding interactions.

In some embodiments, the plurality of oligomers and the plurality of polymerizable oligomers may comprise an irregular or non-uniform mixture. The irregularity of the mixture of polymerizable oligomers and unfunctionalized oligomers may allow pores to form within the hydrogel (i.e., the unfunctionalized oligomers may act as a porogen). For example, the irregular mixture of oligomers may result in phase separation during polymerization that allows for the generation of pores of sufficient size for programmable nucleases to diffuse into the hydrogel and access internal reporter molecules. The relative percentages and/or molecular weights of the oligomers may be varied to vary the pore size of the hydrogel. For example, pore size may be tailored to increase the diffusion coefficient of the programmable nucleases.

In some embodiments, the functional groups attached to the reporters may be selected to preferentially incorporate the reporters into the hydrogel matrix via covalent binding at the functional group versus other locations along the nucleic acid of the reporter. In some embodiments, the functional groups attached to the reporters may be selected to favorably transfer free radicals from the functionalized ends of polymerizable oligomers to the functional group on the end of the reporter (e.g., 5' end), thereby forming a covalent bond and immobilizing the reporter rather than destroying other parts of the reporter molecules.

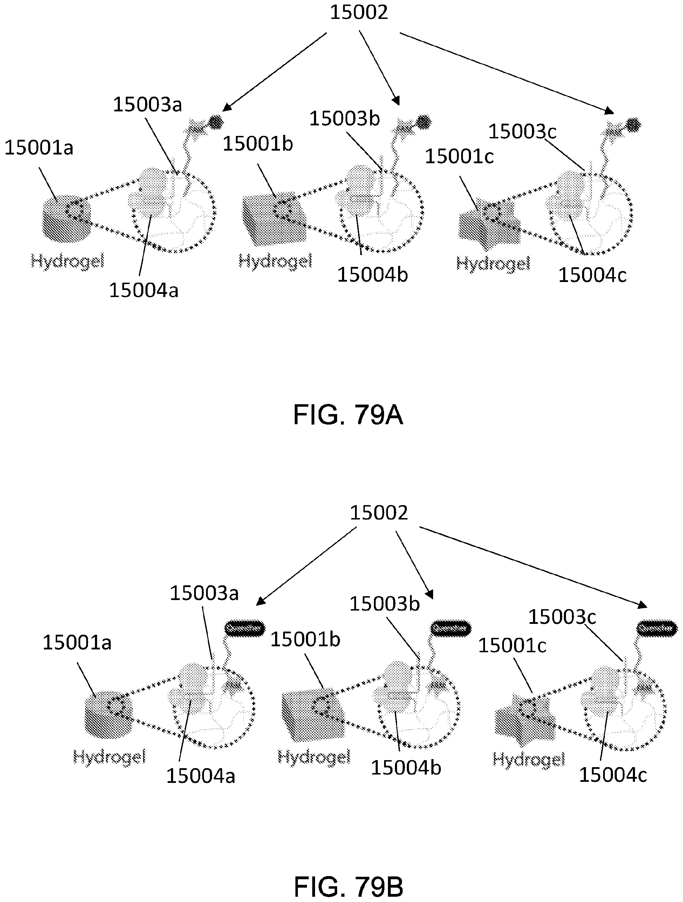

In some embodiments, the polymerizable composition may further comprise one or more polymerizable nucleic acids. In some embodiments, the polymerizable nucleic acids may comprise guide nucleic acids (e.g., guide nucleic acids 15003a, 15003b, or 15003c shown in **FIGS. 79A-79B****).** In some embodiments, the polymerizable nucleic acids may comprise linker or tether nucleic acids. In some embodiments, the polymerizable nucleic acids may be configured to bind to a programmable nuclease (e.g., programmable nuclease 15004a, 15004b, or 15004c shown in **FIGS. 79A-79B****).** In some embodiments, the programmable nuclease may be immobilized in the polymer matrix.

In some embodiments, the oligomers may form a polymer matrix comprising a hydrogel. In some embodiments, the oligomers may comprise poly(ethylene glycol) (PEG), poly(siloxane), poly(hydroxyethyl acrylate, poly(acrylic acid), poly(vinyl alcohol), poly(butyl acrylate), poly(2-ethylhexyl acrylate), poly(methyl acrylate), poly(ethyl acrylate), poly(acrylonitrile), poly(methyl methacrylate), poly(acrylamide), poly(TMPTA methacrylate), chitosan, alginate, or the like, or any combination thereof. One of ordinary skill in the art will recognize that the oligomers may comprise any oligomer or mix of oligomers capable of forming a hydrogel.

In some embodiments, the oligomers may comprise polar monomers, nonpolar monomers, protic monomers, aprotic monomers, solvophobic monomers, or solvophillic monomers, or any combination thereof.

In some embodiments, the oligomers may comprise a linear topology, branched topology, star topology, dendritic topology, hyperbranched topology, bottlebrush topology, ring topology, catenated topology, or any combination thereof. In some embodiments, the oligomers may comprise 3-armed topology, 4-armed topology, 5-armed topology, 6-armed topology, 7-armed topology, 8-armed topology, 9-armed topology, or 10-armed topology.

In some embodiments, the oligomers may comprise at least about 2 monomers, at least about 3 monomers, at least about 4 monomers, at least about 5 monomers, at least about 6 monomers, at least about 7 monomers, at least about 8 monomers, at least about 9 monomers, at least about 10 monomers, at least about 20 monomers, at least about 30 monomers, at least about 40 monomers, at least about 50 monomers, at least about 60 monomers, at least about 70 monomers, at least about 80 monomers, at least about 90 monomers, at least about 100 monomers, at least about 200 monomers, at least about 300 monomers, at least about 400 monomers, at least about 500 monomers, at least about 600 monomers, at least about 700 monomers, at least about 800 monomers, at least about 900 monomers, at least about 1000 monomers, at least about 2000 monomers, at least about 3000 monomers, at least about 4000 monomers, at least about 5000 monomers, at least about 6000 monomers, at least about 7000 monomers, at least about 8000 monomers, at least about 9000 monomers, at least about 10000 monomers, at least about 20000 monomers, at least about 30000 monomers, at least about 40000 monomers, at least about 50000 monomers, at least about 60000 monomers, at least about 70000 monomers, at least about 80000 monomers, at least about 90000 monomers, or at least about 100000 monomers.

In some embodiments, the oligomers may comprise a homopolymer, a copolymer, a random copolymer, a block copolymer, an alternative copolymer, a copolymer with regular repeating units, or any combination thereof.

In some embodiments, the oligomers may comprise 1 type of monomer, 2 types of monomers, 3 types of monomers, 4 types of monomers, 5 types of monomers, 6 types of monomers, 7 types of monomers, 8 types of monomers, 9 types of monomers, or 10 types of monomers.

The polymerizable oligomers may comprise any of the oligomers described herein. In some embodiments, the polymerizable oligomers may comprise one or more functional groups. In some embodiments, the functional group may comprise an acrylate group, N-hydroxysuccinimide ester group, thiol group, carboxyl group, azide group, alkyne group, an alkene group, or any combination thereof. One of ordinary skill in the art will recognize that a variety of functional groups may be used to functionalize oligomers into polymerizable oligomers depending on the desired properties of the polymerizable oligomers.

In some embodiments, the polymerizable oligomers may form a polymer matrix comprising a hydrogel. In some embodiments, the polymerizable oligomers may comprise PEG, poly(siloxane), poly(hydroxyethyl acrylate, poly(acrylic acid), poly(vinyl alcohol), or any combination thereof. One of ordinary skill in the art will recognize that the set of polymerizable oligomers may comprise any polymer capable of forming a hydrogel.

In some embodiments, the set of polymerizable oligomers comprises polar monomers, nonpolar monomers, protic monomers, aprotic monomers, solvophobic monomers, or solvophillic monomers.

In some embodiments, the set of polymerizable oligomers comprises a linear topology, branched topology, star topology, dendritic topology, hyperbranched topology, bottlebrush topology, ring topology, catenated topology, or any combination thereof. In some embodiments, the set of polymerizable oligomers comprises 3-armed topology, 4-armed topology, 5-armed topology, 6-armed topology, 7-armed topology, 8-armed topology, 9-armed topology, or 10-armed topology.

In some embodiments, the set of polymerizable oligomers comprises at least about 2 monomers, at least about 3 monomers, at least about 4 monomers, at least about 5 monomers, at least about 6 monomers, at least about 7 monomers, at least about 8 monomers, at least about 9 monomers, at least about 10 monomers, at least about 20 monomers, at least about 30 monomers, at least about 40 monomers, at least about 50 monomers, at least about 60 monomers, at least about 70 monomers, at least about 80 monomers, at least about 90 monomers, at least about 100 monomers, at least about 200 monomers, at least about 300 monomers, at least about 400 monomers, at least about 500 monomers, at least about 600 monomers, at least about 700 monomers, at least about 800 monomers, at least about 900 monomers, at least about 1000 monomers, at least about 2000 monomers, at least about 3000 monomers, at least about 4000 monomers, at least about 5000 monomers, at least about 6000 monomers, at least about 7000 monomers, at least about 8000 monomers, at least about 9000 monomers, at least about 10000 monomers, at least about 20000 monomers, at least about 30000 monomers, at least about 40000 monomers, at least about 50000 monomers, at least about 60000 monomers, at least about 70000 monomers, at least about 80000 monomers, at least about 90000 monomers, or at least about 100000 monomers. As used herein, "about" may mean plus or minus 1 monomer, plus or minus 10 monomers, plus or minus 100 monomers, plus or minus 1000 monomers, plus or minus 10000 monomers, or plus or minus 100000 monomers.

In some embodiments, the set of polymerizable oligomers comprises a homopolymer, a copolymer, a random copolymer, a block copolymer, an alternative copolymer, a copolymer with regular repeating units, or any combination thereof.

In some embodiments, the set of polymerizable oligomers comprises 1 type of monomer, 2 types of monomers, 3 types of monomers, 4 types of monomers, 5 types of monomers, 6 types of monomers, 7 types of monomers, 8 types of monomers, 9 types of monomers, or 10 types of monomers.

In some embodiments, the polymerizable composition may comprise a mix of unfunctionalized or unmodified oligomers and polymerizable oligomers as described herein. In some embodiments, the unfunctionalized or unmodified oligomers may act as porogens to generate pores within the polymer matrix.

The polymerizable reporters may comprise any of the reporters described herein. In some embodiments, the set of polymerizable reporters may comprise one or more functional groups. In some embodiments, the functional group may comprise a single stranded nucleic acid, a double stranded nucleic acid, an acrydite group, a 5' thiol modifier, a 3' thiol modifier, an amine group, a I-Linker^{™} group, methacryl group, or any combination thereof. One of ordinary skill in the art will recognize that a variety of functional groups may be used with the set of polymerizable reporters depending on the desired properties of the polymerizable reporters.

In some embodiments, the set of initiators may comprise one or more photoinitiators or thermal initiators. In some embodiments, the set of initiators may comprise cationic initiators, anionic initiators, or radical initiators. In some embodiments, the set of initiators may comprise AIBN, AMBN, ADVN, ACVA, dimethyl 2,2'-azo-bis(2methylpropionate), AAPH, 2,2'-azobis[2-(2-imidazolin-2-yl)-propane] dihydrochloride, TBHP, cumene hydroperoxide, di-tert-butyl peroxide, dicumyl peroxide, BPO, dicyandamide, cyclohexyl tosylate, diphenyl(methyl)sulfonium tetrafluoroborate, benzyl(4-hydroxyphenyl)-methylsulfonium hexafluoroantimonate, (4-hydroxyphenyl)methyl-(2-methylbenzyl)sulfonium hexafluoroantimonate, camphorquinone, acetophenone, 3-acetophenol, 4-acetophenol, benzophenone, 2-methylbenzophenone, 3-methylbenzophenone, 3-hydroxybenzophenone, 3,4-dimethylbenzophenone, 4-hydroxybenzophenone, 4-benzoylbenzoic acid, 2-benzoylbenzoic acid, methyl 2-benzoylbenzoate, 4,4'-dihydroxybenzophenone, 4-(dimethylamino)-benzophenone, 4,4'-bis(dimethylamino)-benzophenone, 4,4'-bis(diethylamino)-benzophenone, 4,4'-dichlorobenzophenone, 4-(p-tolylthio)benzophenone, 4-phenylbenzophenone, 1,4-dibenzoylbenzene, benzil, 4,4'-dimethylbenzil, p-anisil, 2-benzoyl-2-propanol, 2-hydroxy-4'-(2-hydroxyethoxy)-2-methylpropiophenone, 1-benzoylchclohexanol, benzoin, anisoin, benzoin methyl ether, benzoin ethyl ether, benzoin isopropyl ether, benzoin isobutyl ether, o-tosylbenzoin, 2,2-diethoxyacetophenone, benzil dimethylketal, 2-methyl-4'-(methylthio)-2-morpholinopropiophenone, 2-benzyl-2-(dimethylamino)-4'-morpholinobutyrophenone, 2-isonitrosopropiophenone, anthraquinone, 2-ethylantraquinone, sodium anthraquinone-2-sulfonate monohydrate, 9,10-phenanthrenequinone, 9,10-phenanthrenequinone, dibenzosuberenone, 2-chlorothioxanthone, 2-isopropylthioxanthone, 2,4-diethylthioxanthen-9-one, 2,2'bis(2-chlorophenyl)-4,4',5,5'-tetraphenyl-1,2'-biimidazole, diphenyl(2,4,6-trimethylbenzoyl)phosphine oxide, phenylbis(2,4,6-trimethyl-benzoyl)phosphine oxide, lithium phenyl(2,4,6-trimethylbenzoyl)phosphinate, diphenyliodonium trifluoromethanesulfonate, diphenyliodonium hexafluorophosphate, diphenyliodonium hexafluoroarsenate, bis(4-tert-butylphenyl)-iodonium triflate, bis(4-tert-butylphenyl)iodonium hexafluorophosphate, 4-isopropyl-4'-methyl-diphenyliodonium tetrakis(pentafluorophenyl)borate, [4-[(2-hydroxytetradecyl)-oxy]phenyl]phenyliodonium hexafluoroantimonate, bis[4-(tert-butyl)phenyl]-iodonium tetra(nonafluoro-tert-butoxy)aluminate, cyclopropyldiphenylsulfonium tetrafluoroborate, triphenylsulfonium bromide, triphenylsulfonium tetrafluoroborate, tri-p-tolylsulfonium triflate, tri-p-tolylsulfonium hexafluorophosphate, 4-nitrobenzenediazonium tetrafluoroborate, 2-(4-methoxyphenyl)-4,6-bis(trichloromethyl)-1,3,5-triazine, 2-(1,3-benzodioxol-5-yl)-4,6-bis(trichloromethyl)-1,3,5-triazine, 2-(4-methoxystyryl)-4,6-bis(trichloromethyl)-1,3,5-triazine, 2-(3,4-dimethoxystyryl)-4,6-bis(trichloromethyl)-1,3,5-triazine, 2-[2-(Furan-2-yl)vinyl]-4,6-bis(trichloromethyl)-1,3,5-triazine, 2-[2-(5-methylfuran-2-yl)vinyl]-4,6-bis(trichloromethyl)-1,3,5-triazine, 2-(9-oxoxanthen-2-yl)proprionic acid 1,5,7-triazabicyclo[4.4.0]dec-5-ene salt, 2-(9-oxoxanthen-2-yl)proprionic acid 1,5-diazabicyclo[4.3.0]non-5-ene salt, 2-(9-oxoxanthen-2-yl)proprionic acid 1,8-diazabicyclo[5.4.0]-undec-7-ene salt, acetophenone O-benzoyloxime, 2-nitrobenzyl cyclohexylcarbamate, 1,2-bis(4-methoxyphenyl)-2-oxoethyl cyclohexylcarbamate, tert-amyl peroxybenzoate, 4,4-azobis(4-cyanovaleric acid), 1,1'-azobis(cyclohexanecarbonitrile), 2,2'-azobisisobutyronitrile, benzoyl peroxide, 2,2-bi(tert-butylperoxy)butane, 1,1-bis(tert-butylperoxy)cyclohexane, 2,5-bis(tert-butylperoxy)-2,5-dimethylhexane, bis(1-(tert-butylperoxy)-1-methylethyl)benzene, 1,1-bis(tert-butylperoxy)-3,3,5-trimethylcyclohexane, tert-butyl hydroperoxide, tert-butyl peracetate, tert-butyl peroxide, tert-butyl peroxybenzoate, tert-butylperoxy isopropyl carbonate, cumene hydroperoxide, cyclohexanone peroxide, dicumyl peroxide, lauroyl peroxide, 2,4-pentanedione peroxide, peracetic acid, potassium persulfate, 2-Hydroxy-2-methylpropiophenone, or any combination thereof. One of ordinary skill in the art will recognize that a variety of initiators may be used depending on the desired reaction conditions and chemistries.

In some embodiments, the initiation stimulus is UV light. In some embodiments, the initiation stimulus is UV light through a photomask. In some embodiments, the initiation stimulus is heat.

In some embodiments, the hydrogel may comprise a circular cross-sectional shape, a rectangular cross-sectional shape, a star cross-sectional shape, a dollop shape, an amorphous shape, or any shape of interest, or any combination thereof (e.g., as shown in **FIGS.** 79A- 79B).

In some embodiments, a mask may be used to shape the initiation stimulus deposition on the polymerizable components (e.g., oligomers, etc.) and thereby shape the resulting polymer matrix. In some embodiments, the mask may comprise a circular shape, a rectangular shape, a star shape, a dollop shape, an amorphous shape, or any shape of interest, or any combination thereof.

### Hydrogel Compositions with Immobilized Reporters

**FIG.** 78 and **FIGS.** 79A-79B show examples of hydrogels comprising immobilized reporters. In some aspects, provided herein are compositions comprising a hydrogel (14901) comprising (a) a network of covalently bound oligomers (14903) and (b) immobilized reporters (14902) covalently bound to said network (14903).

**FIG.** 78 shows an exemplary hydrogel (14901) comprising a plurality of reporters (14902) co-polymerized with a plurality of oligomers (modified and unmodified) to form a network or matrix (14903). **FIGS.** 79A-79B show exemplary multiplexing schemes utilizing hydrogel-immobilized reporters which may be implemented in any of the devices or methods described herein. Multiplexing could be distinguished through spatial multiplexing by knowing the location of hydrogels functionalized with each guide nucleic acid and/or through shape, by using different shapes of hydrogel for each guide nucleic acid.

In some embodiments, the composition may comprise a hydrogel (15001) comprising (a) a polymer network comprising covalently bound oligomers co-polymerized with reporters (15002) to covalently bind and immobilize the reporters to said network, and (b) immobilized programmable nuclease complexes covalently bound to said network (e.g., via co-polymerization or after reporter-immobilized polymer formation), wherein said programmable nuclease complexes may comprise a programmable nuclease (15004) and a guide nucleic acid (15003). In some embodiments, the guide nucleic acid (15003) and/or the programmable nuclease (15004) may be immobilized to or in the hydrogel as described herein (e.g., during or after formation of the hydrogel).

In some embodiments, the network of covalently bound oligomers may comprise a network formed by polymerizing one or more PEG species. In some embodiments, the network of covalently bound oligomers may comprise a network formed by polymerizing PEG comprising acrylate functional groups. In some embodiments, the acrylate functional groups may be PEG end groups. In some embodiments, the network may be formed by polymerizing PEG comprising acrylate functional groups with unmodified PEG. The molecular weight of the acrylate-modified PEG (e.g., PEG-diacrylate) and the unmodified PEG may be the same or different.

In some embodiments, the network of covalently bound oligomers may comprise a network formed from polymerizing one or more PEG species, wherein each PEG species may comprise a linear topology, branched topology, star topology, dendritic topology, hyperbranched topology, bottlebrush topology, ring topology, catenated topology, or any combination thereof. In some embodiments, the network of covalently bound oligomers may comprise a network formed from polymerizing one or more PEG species comprising a 3-armed topology, a 4-armed topology, a 5-armed topology, a 6-armed topology, a 7-armed topology, a 8-armed topology, a 9-armed topology, or a 10-armed topology.

In some embodiments, the immobilized reporter may comprise a reporter molecule covalently bound to a linker molecule, wherein the linker molecule is covalently bound to the hydrogel (e.g., via co-polymerization with the oligomers as described herein). In some embodiments, the linker molecule may comprise a single stranded nucleic acid, a double stranded nucleic acid, an acrydite group, a 5' thiol modifier, a 3' thiol modifier, an amine group, a I-Linker^{™} group, or any combination thereof. One of ordinary skill in the art will recognize that a variety of linker molecules may be used.

In some cases, the immobilized guide nucleic acid may comprise a guide nucleic acid covalently bound to a linker molecule, wherein the linker molecule is covalently bound to the hydrogel. In some embodiments, the linker molecule may comprise a single stranded nucleic acid, a double stranded nucleic acid, an acrydite group, a 5' thiol modifier, a 3' thiol modifier, an amine group, a I-Linker^{™} group, or any combination thereof. One of ordinary skill in the art will recognize that a variety of linker molecules may be used.

In some cases, the immobilized programmable nuclease may comprise a programmable nuclease covalently bound to a linker molecule, wherein the linker molecule is covalently bound to the hydrogel. In some embodiments, the linker molecule may comprise a single stranded nucleic acid, a double stranded nucleic acid, an acrydite group, a 5' thiol modifier, a 3' thiol modifier, an amine group, a I-Linker^{™} group, or any combination thereof. One of ordinary skill in the art will recognize that a variety of linker molecules may be used.

### Methods of Using Hydrogels with Immobilized Reporters

Any of the methods described herein may utilize hydrogels (14901) with immobilized reporters (14902) for target detection assays. In some embodiments, the hydrogel (14901) comprises (a) a network of covalently bound oligomers (14903) and (b) immobilized reporters (14902) covalently bound to said network (14903) as shown in **FIG. 78****.** A solution comprising target nucleic acid molecules and programmable nuclease complexes may be applied to the hydrogel (e.g., by pipetting or flowing over the hydrogel). The immobilized reporters (14902) may comprise a nucleic acid with a sequence cleavable by the programmable complex when the programmable nuclease complex is activated by binding of its associated guide nucleic acid to a target nucleic acid molecule as described herein. When activated, the programmable nuclease complex may trans-cleave the cleavable nucleic acid of the reporter molecule and generates a detectable signal as described herein. For example, the reporter may comprise a detection moiety which may be release upon cleavage of the reporter as described herein. The detection moiety may comprise FAM-biotin which may be captured by one or more capture molecules coupled to a substrate (e.g., a lateral flow assay strip) at a detection location as described herein. Detection of the detectable signal generated at the detection location by the detection moiety may indicate the presence of the target nucleic acid in the sample as described herein.

Any of the multiplexing methods described herein may utilize hydrogels (15001a, 15001b, 15001c, etc.) with immobilized reporters (15002) for multiplexed target detection assays. In some embodiments, each hydrogel (15001a, 150001b, 15001c, etc.) may comprise (a) a polymer network of covalently bound oligomers co-polymerized with reporters (15002) to covalently bind and immobilize the reporters to said network, and (b) one or more immobilized programmable nuclease complexes covalently bound to said network as shown in FIGS. 79A-79B. Each of the programmable nuclease complexes may comprise a programmable nuclease (15004a, 15004b, 15004c, etc.) and a guide nucleic acid (15003a, 15003b, 15003c, etc.). In some embodiments, the guide nucleic acid (15003) and/or the programmable nuclease (15004) may be immobilized to or in the hydrogel as described herein (e.g., during or after formation of the hydrogel). In some embodiments, multiplexing for a plurality of different targets may be facilitated by providing a plurality different and/or spatially separated hydrogels comprising a plurality of different DETECTR reaction components. In some embodiments, each hydrogel may comprise a different programmable nuclease as described herein. Alternatively, or in combination, each hydrogel may comprise a different guide nucleic acid configured to bind to a different target nucleic acid sequence as described herein. Alternatively, or in combination, each hydrogel may comprise a different reporter as described herein. Alternatively, or in combination, each hydrogel may comprise a different shape and be deposited on a substrate at different detection locations. For example, as shown in **FIGS. 79A-79B****,** a first hydrogel (15001a) may comprise a first programmable nuclease (15004a), a first guide nucleic acid (15003a) configured to bind a first target nucleic acid, and a first reporter (15002). A second hydrogel (15001b) may comprise a second programmable nuclease (15004b), a second guide nucleic acid (15003b) configured to bind a second target nucleic acid, and a second reporter (15002). A third hydrogel (15001c) may comprise a third programmable nuclease (15004c), a third guide nucleic acid (15003c) configured to bind a third target nucleic acid, and a third reporter (15002). The programmable nucleases (15004a, 15004b, 15004c) may be the same programmable nuclease or different programmable nuclease. The guide nucleic acids (15003a, 15003b, 15003c) may be different guide nucleic acids configured to recognize different target nucleic acids. The reporters (15002) may be the same reporter or different reporters. A solution comprising one or more target nucleic acid molecules may be applied to the hydrogels (15001a, 15002b, 15003c), e.g., by pipetting or flowing over the hydrogels. The immobilized reporters (15002) may comprise a nucleic acid with a sequence cleavable by the programmable nuclease complexes (15004a, 15004b, 15004c) when the programmable nuclease complexes are activated by binding of their respective guide nucleic acids (15003a, 15003b, 15003c) to their respective target nucleic acid molecules as described herein. When activated, the programmable nuclease complexes may trans-cleave the cleavable nucleic acid of the reporter molecule and generates a detectable signal at the detection location as described herein. For example, the reporter may comprise a detection moiety which may be release upon cleavage of the reporter as described herein. The detection moiety may comprise FAM-biotin as shown in **FIG. 79A** which may be captured by one or more capture molecules coupled to a substrate (e.g., a lateral flow assay strip) at a detection location as described herein. Alternatively, the detection moiety may comprise a quencher moiety which may be released from the hydrogel upon cleavage of the reporter, thereby allowing a fluorescent moiety on the other end of the reporter to fluoresce at the detection location comprising the hydrogel as shown in **FIG. 79B****.** Detection of the detectable signal generated at the detection locations by the detection moiety may indicate the presence of the target nucleic acid in the sample as described herein. Each hydrogel (15001a, 15001b, 15001c) may have a different shape and detection of a target nucleic acid may comprise detecting a particular fluorescent shape corresponding to the hydrogel shape at the detection location.

### Devices Comprising Hydrogels with Immobilized Reporters

Any of the systems or devices described herein may comprise one or more hydrogels with immobilized reporters.

In some embodiments, the systems and devices described herein may comprise a plurality of hydrogels each comprising reporter molecules (e.g., in order to facilitate multiplexing and/or improve signal). In some embodiments, a first hydrogel may comprise a shape different from a shape of a second hydrogel. In some embodiments, the first hydrogel may comprise a plurality of first reporter molecules different from a plurality of second reporter molecules of the second hydrogel. In some embodiments, the reporters are the same in the first and second hydrogels. In some embodiments, the first hydrogel may comprise a circular shape, a square shape, a star shape, or any other shape distinguishable from a shape of the second hydrogel. In some embodiments, the plurality of first reporter molecules may each comprise a sequence cleavable by a programmable nuclease complex comprising a first programmable nuclease and a first guide nucleic acid. In some embodiments, the plurality of second reporter molecules may each comprise a sequence not cleavable by the first programmable nuclease complex.

Any of the systems or devices described herein may comprise a plurality of hydrogels each comprising reporter molecules. For example, a first hydrogel may comprise a plurality of first reporter molecules different from a plurality of second reporter molecules of a second hydrogel. In some embodiments, the plurality of first reporter molecules may each comprise a first fluorescent moiety, wherein the first fluorescent moiety is different than second fluorescent moieties of in each of the plurality of second reporter molecules. In some embodiments, the plurality of first reporter molecules may each comprise a sequence cleavable by a first programmable nuclease complex comprising a first programmable nuclease and a first guide nucleic acid. In some embodiments, the plurality of second reporter molecules may each comprise a sequence cleavable by a second programmable nuclease complex comprising a second programmable nuclease and a second guide nucleic acid.

Any of the systems or devices described herein may comprise at least about 2 hydrogels, at least about 3 hydrogels, at least about 4 hydrogels, at least about 5 hydrogels, at least about 6 hydrogels, at least about 7 hydrogels, at least about 8 hydrogels, at least about 9 hydrogels, at least about 10 hydrogels, at least about 20 hydrogels, at least about 30 hydrogels, at least about 40 hydrogels, at least about 50 hydrogels, at least about 60 hydrogels, at least about 70 hydrogels, at least about 80 hydrogels, at least about 90 hydrogels, at least about 100 hydrogels, at least about 200 hydrogels, at least about 300 hydrogels, at least about 400 hydrogels, at least about 500 hydrogels, at least about 600 hydrogels, at least about 700 hydrogels, at least about 800 hydrogels, at least about 900 hydrogels, at least about 1000 hydrogels,

Any of the systems or devices described herein may comprise one or more compartments, chambers, channels, or locations comprising the one or more hydrogels. In some embodiments, two or more of the compartments may be in fluid communication, optical communication, thermal communication, or any combination thereof with one another. In some embodiments, two or more compartments may be arranged in a sequence. In some embodiments, two or more compartments may be arranged in parallel. In some embodiments, two or more compartments may be arranged in sequence, parallel, or both. In some embodiments, one or more compartments may comprise a well. In some embodiments, one or more compartments may comprise a flow strip. In some embodiments, one or more compartments may comprise a heating element.

In some embodiments, the device may be a handheld device. In some embodiments, the device may be point-of-need device. In some embodiments, the device may comprise any one of the device configurations described herein. In some embodiments, the device may comprise one or more parts of any one of the device configurations described herein.

### Multiplexing

Methods consistent with the present disclosure include a multiplexing method of assaying for a target nucleic acid in a sample. A multiplexing method may comprise contacting the sample to a complex comprising a guide nucleic acid comprising a segment that is reverse complementary to a segment of the target nucleic acid and a programmable nuclease that exhibits sequence independent cleavage upon forming a complex comprising the segment of the guide nucleic acid binding to the segment of the target nucleic acid; and assaying for a signal indicating cleavage of at least some protein-nucleic acids of a population of protein-nucleic acids, wherein the signal indicates a presence of the target nucleic acid in the sample, wherein absence of the signal indicates an absence of the target nucleic acid in the sample. As another example, multiplexing method of assaying for a target nucleic acid in a sample, for example, comprises: a) contacting the sample to a complex comprising a guide nucleic acid comprising a segment that is reverse complementary to a segment of the target nucleic acid and a programmable nuclease that exhibits sequence independent cleavage upon forming a complex comprising the segment of the guide nucleic acid binding to the segment of the target nucleic acid; b) contacting the complex to a substrate; c) contacting the substrate to a reagent that differentially reacts with a cleaved substrate; and d) assaying for a signal indicating cleavage of the substrate, wherein the signal indicates a presence of the target nucleic acid in the sample and wherein absence of the signal indicates an absence of the target nucleic acid in the sample. Often, the substrate is an enzyme-nucleic acid. Sometimes, the substrate is an enzyme substrate-nucleic acid.

Multiplexing may also comprise targeting multiple sequences during amplification. Such amplification multiplexing may comprise targeting distinct sequences (e.g., multiple different genes), and may also comprise targeting different portions of the same target nucleic acid sequence. The buffers of the present disclosure allow for multiplexed amplification and multiplexed programmable nuclease-based detection (e.g., DETECTR) reactions to be performed within the same sample volume.

Multiplexing can be either spatial multiplexing wherein multiple different target nucleic acids are detected at the same time, but the reactions are spatially separated. Often, the multiple target nucleic acids are detected using the same programmable nuclease, but different guide nucleic acids. The multiple target nucleic acids sometimes are detected using the different programmable nucleases. Sometimes, multiplexing can be single reaction multiplexing wherein multiple different target acids are detected in a single reaction volume. Often, a single population of programmable nucleases is used in single reaction multiplexing. Sometimes, at least two different programmable nucleases are used in single reaction multiplexing. For example, multiplexing can be enabled by immobilization of multiple categories of reporters within a fluidic system, to enable detection of multiple target nucleic acids within a single sample.

Sometimes, multiplexing can be single reaction multiplexing wherein multiple different target acids are detected in a single reaction volume. Often, at least two different programmable nucleases are used in single reaction multiplexing. For example, multiplexing can be enabled by immobilization of multiple categories of reporters within a fluidic system, to enable detection of multiple target nucleic acids within a single fluidic system. Multiplexing allows for detection of multiple target nucleic acids in one kit or system. In some cases, the multiple target nucleic acids comprise different target nucleic acids to a virus, a bacterium, or a pathogen responsible for one disease. In some cases, the multiple target nucleic acids comprise different target nucleic acids associated with a cancer or genetic disorder. Multiplexing for one disease, cancer, or genetic disorder increases at least one of sensitivity, specificity, or accuracy of the assay to detect the presence of the disease in the sample. In some cases, the multiple target nucleic acids comprise target nucleic acids directed to different viruses, bacteria, or pathogens responsible for more than one disease. In some cases, multiplexing allows for discrimination between multiple target nucleic acids, such as target nucleic acids that comprise different genotypes of the same bacteria or pathogen responsible for a disease, for example, for a wild-type genotype of a bacteria or pathogen and for genotype of a bacteria or pathogen comprising a mutation, such as a single nucleotide polymorphism (SNP) that can confer resistance to a treatment, such as antibiotic treatment. Multiplexing, thus, allows for multiplexed detection of multiple genomic alleles. For example, multiplexing may comprise method of assaying comprising a single assay for a microorganism species using a first programmable nuclease and an antibiotic resistance pattern in a microorganism using a second programmable nuclease. Sometimes, multiplexing allows for discrimination between multiple target nucleic acids of different HPV strains, for example, HPV16 and HPV18. In some cases, the multiple target nucleic acids comprise target nucleic acids directed to different cancers or genetic disorders. Often, multiplexing allows for discrimination between multiple target nucleic acids, such as target nucleic acids that comprise different genotypes, for example, for a wild-type genotype and for SNP genotype. Multiplexing for multiple diseases, cancers, or genetic disorders provides the capability to test a panel of diseases from a single sample. For example, multiplexing for multiple diseases can be valuable in a broad panel testing of a new patient or in epidemiological surveys. Often multiplexing is used for identifying bacterial pathogens in sepsis or other diseases associated with multiple pathogens.

Furthermore, signals from multiplexing can be quantified. For example, a method of quantification for a disease panel comprises assaying for a plurality of unique target nucleic acids in a plurality of aliquots from a sample, assaying for a control nucleic acid control in a second aliquot of the sample, and quantifying a plurality of signals of the plurality of unique target nucleic acids by measuring signals produced by cleavage of reporters compared to the signal produced in the second aliquot. Often the plurality of unique target nucleic acids are from a plurality of viruses in the sample. Sometimes the quantification of a signal of the plurality correlates with a concentration of a unique target nucleic acid of the plurality for the unique target nucleic acid of the plurality that produced the signal of the plurality.

The methods, reagents, and devices described herein can be multiplexed by various configurations of the reagents and the support medium. In some cases, the kit or system is designed to have multiple support mediums encased in a single housing. Sometimes, the multiple support mediums housed in a single housing share a single sample pad. The single sample pad may be connected to the support mediums in various designs such as a branching or a radial formation. Alternatively, each of the multiple support mediums has its own sample pad. In some cases, the kit or system is designed to have a single support medium encased in a housing, where the support medium comprises multiple detection spots for detecting multiple target nucleic acids. Sometimes, the reagents for multiplexed assays comprise multiple guide nucleic acids, multiple programmable nucleases, and multiple single stranded reporters, where a combination of one of the guide nucleic acids, one of the programmable nucleases, and one of the single stranded reporters detects one target nucleic acid and can provide a detection spot on the detection region. In some cases, the combination of a guide nucleic acid, a programmable nuclease, and a single stranded reporter configured to detect one target nucleic acid is mixed with at least one other combination in a single reagent chamber. In some cases, the combination of a guide nucleic acid, a programmable nuclease, and a single stranded reporter configured to detect one target nucleic acid is mixed with at least one other combination on a single support medium. When these combinations of reagents are contacted with the sample, the reaction for the multiple target nucleic acids occurs simultaneously in the same medium or reagent chamber. Sometimes, this reacted sample is applied to the multiplexed support medium described herein. In some cases, the methods, reagents, and devices described herein can be multiplexed in a configuration lacking a support medium.

In some instances, the multiplexed devices, systems, fluidic devices, kits, and methods detect at least 2 different target nucleic acids in a single reaction. In some instances, the multiplexed devices, systems, fluidic devices, kits, and methods detect at least 3 different target nucleic acids in a single reaction. In some instances, the multiplexed devices, systems, fluidic devices, kits, and methods detect at least 4 different target nucleic acids in a single reaction. In some instances, the multiplexed devices, systems, fluidic devices, kits, and methods detect at least 5 different target nucleic acids in a single reaction. In some cases, the multiplexed devices, systems, fluidic devices, kits, and methods detect at least 6, 7, 8, 9, or 10 different target nucleic acids in a single reaction. In some instances, the multiplexed kits detect at least 2 different target nucleic acids in a single kit. In some instances, the multiplexed kits detect at least 3 different target nucleic acids in a single kit. In some instances, the multiplexed kits detect at least 4 different target nucleic acids in a single kit. In some instances, the multiplexed kits detect at least 5 different target nucleic acids in a single kit. In some instances, the multiplexed kits detect at least 6, 7, 8, 9, or 10 different target nucleic acids in a single kit. In some instances, the multiplexed kits detect from 2 to 10, from 3 to 10, from 4 to 10, from 5 to 10, from 6 to 10, from 7 to 10, from 8 to 10, from 9 to 10, from 2 to 9, from 3 to 9, from 4 to 9, from 5 to 9, from 6 to 9, from 7 to 9, from 8 to 9, from 2 to 8, from 3 to 8, from 4 to 8, from 5 to 8, from 6 to 8, from 7 to 8, from 2 to 7, from 3 to 7, from 4 to 7, from 5 to 7, from 6 to 7, from 2 to 6, from 3 to 6, from 4 to 6, from 5 to 6, from 2 to 5, from 3 to 5, from 4 to 5, from 2 to 4, from 3 to 4, or from 2 to 3 different target nucleic acids in a single kit. Multiplexing can be carried out in a single-pot or "one-pot" reaction, where reverse transcription, amplification, in vitro transcription, or any combination thereof, and detection are carried out in a single volume. Multiplexing can be carried out in a "two-pot reaction", where reverse transcription, amplification, in vitro transcription, or any combination thereof, are carried out in a first volume and detection is carried out in a second volume.

In some cases, multiplexing can comprise detecting multiple targets with a single probe. Alternatively, multiplexing can comprise detecting multiple targets with multiple probes. The multiple probes can be configured to detect a presence of a particular sequence, target nucleic acid, or a plurality of different target sequences or nucleic acids.

In some cases, the combination of a guide nucleic acid, a programmable nuclease, and a single stranded reporter configured to detect one target nucleic acid is provided in its own reagent chamber or its own support medium. In this case, multiple reagent chambers or support mediums are provided in the device, kit, or system, where one reagent chamber is designed to detect one target nucleic acid. In this case, multiple support mediums are used to detect the panel of viral infections, or other diseases of interest.

### Kits

Disclosed herein are kits, reagents, methods, and systems for use to detect a target nucleic acid. A target nucleic acid can be assayed for using the compositions and methods disclosed herein and used in a kit as described herein.

Disclosed herein are kits, reagents, methods, and systems for use to detect a target nucleic acid. In some cases, the target nucleic acid is from a coronavirus. In some cases, the target nucleic acid is from the SARS-CoV-2 coronavirus. Any nucleic acid of the SARS-CoV-2 can be assayed for using the compositions and methods disclosed herein and used in a kit as described herein. In some embodiments, the target nucleic acid comprises the N gene or the E gene of coronavirus and can be assayed for using the compositions and methods disclosed herein and used in a kit as described herein.

In some embodiments, the kit comprises the reagents and a support medium. The reagent may be provided in a reagent chamber or on the support medium. Alternatively, the reagent may be placed into the reagent chamber or the support medium by the individual using the kit. Optionally, the kit further comprises a buffer and a dropper. The reagent chamber be a test well or container. The opening of the reagent chamber may be large enough to accommodate the support medium.

Optionally, the kit further comprises a dropper. The reagent chamber be a test well or container. The opening of the reagent chamber may be large enough to accommodate the support medium. The buffer may be provided in a dropper bottle for ease of dispensing. The dropper can be disposable and transfer a fixed volume. The dropper can be used to place a sample into the reagent chamber or on the support medium. A kit may comprise a plurality of pipettes. The plurality of pipettes may be sufficient for performing one or more than one assays. A plurality of pipettes may comprise a disposable pipette.

A kit may comprise a container (e.g., a PCR tube) comprising reagents for amplification and programmable nuclease-based detection (e.g., DETECTR) reactions. The container may comprise all reagents required for the amplification and programmable nuclease-based detection reactions, or lack a particular reagent that can be provided to initiate a reaction (e.g., a LAMP activator or dNTPs for amplification, reporters for DETECTR reactions, etc.). A kit may comprise separate containers comprising amplification and programmable nuclease-based detection reagents, which can be added at specified times to a reaction chamber or vessel containing the sample. For example, a kit may comprise separate containers for amplification reagents, programmable nuclease-based detection reagents, and a buffer capable of supporting both reactions. In such a case, the sample, amplification reagents, and programmable nuclease-based detection reagents may be added to a container at different times. A kit may comprise reverse transcription reagents, such as a reverse transcriptase, an oligonucleotide primer, and dNTPs. A kit may comprise in vitro transcription reagents, such as transcription reagents comprise an RNA polymerase, a transcription primer, and nucleotide triphosphates (NTPs). A kit may comprise a reagent for stabilizing a target nucleic acid. A kit may comprise a buffer. A kit may comprise a pipette. A kit may comprise a programmable nuclease. A kit may comprise a plurality of containers comprising separate reagents. For example, a kit may comprise an amplification reagent mix within a first vial and the detection reagent mix within a second vial. A kit may comprise a control amplification reagent mix in its own vial. A kit may comprise a control nucleic acid in its own vial. A kit may comprise an amplification activator mix within its own vial. A kit may comprise multiple distinct amplification, detection, control, activator, or any other reagent mixes. For example, a kit may comprise 2, 3, 4, 5, 6, 7, 8, 9, 10, 12, 15, 20, 25, 30, or 40 separate amplification reagent mixes, each in separate containers.

Alternatively, a reagent may be stored in a container, and later placed into a reagent chamber or support medium by the individual using the kit. The container can be any suitable container for stably storing and transporting the reagent. For example, a reagent may be stored in an Eppendorf^{®} tube, a centrifuge tube, a vial, a screw top vial, an ampule, a bottle, a bag, or any other container capable of holding the reagent. The reagent may be provided frozen, in dry form (e.g., as a powder such as a lyophilized powder), or as a solution. A reagent may be divided between multiple containers, or may be provided in a single container.

Conversely, a container may comprise a complete set of reagents for amplification and programmable nuclease-based detection reactions targeting a nucleic acid sequence. The sample may be added to the container to initiate the reactions. A reaction within a container may be monitored in a fluorescence imaging system. A reaction within a container (e.g., a DETECTR reaction) may also be monitored by a smart phone. For example, a smartphone camera may be disposed next to a first translucent portion of a container, and a fluorophore excitation light-source (e.g., a kit-provided lamp) may be directed into a second translucent portion of a container. In some cases, a fluorescence change can be monitored just with a smart phone and ambient lighting. In some cases, an optical signal (e.g., a colorimetric change) can be monitored solely with a smartphone.

A container may comprise a seal. The seal may be irreversibly removable (e.g., a film that can be pealed from an opening into the container), reversibly removable (e.g., a lid that can be removed and refastened over an opening in the container), or puncturable (e.g., a septum or membrane that a needle may be inserted through). A puncturable seal may reseal after puncturing. A container may be provided comprising reagents for a reaction, and a seal to secure the reagents within the container.

A container may comprise multiple compartments. The compartments may be physically separated by puncturable or breakable barriers. A sample may be added to the first chamber, and after a specified period of time the membrane may be broken to release the contents of the first chamber into the second chamber. For example, a container may comprise a first chamber comprising amplification reagents separated from a second chamber comprising programmable nuclease-based detection reagents by a breakable membrane. A sample may first be added to the first chamber for amplification, and then later released into the second chamber (e.g., by breaking or piercing the breakable membrane) to initiate a programmable nuclease-based detection reaction.

In some embodiments, the kit comprises the reagents and a support medium. The reagent may be provided in a reagent chamber or on the support medium. The medium may comprise multiple partitions or chambers that are pre-loaded with reagents. A partition or chamber may comprise a seal, which may be removable or pierceable (e.g., a septum that is pierceable by a syringe). For example, a kit may comprise a well plate that is pre-loaded with reagents and covered with a removable seal.

A support medium may comprise a plurality of containers. A plurality of containers may comprise two containers comprising reagents that target different nucleic acid sequences. A support medium may provide an array of containers comprising reagents targeting different nucleic acid sequences. For example, a kit may comprise a multi-well plate comprising 24, 48, 96, 192, or 384 wells comprising reagents for detecting (e.g., amplification and programmable nuclease-based detection reagents) different nucleic acid sequences. A single sample may be partitioned between each of the wells (e.g., by a multi-channel pipette provided in the kit), the support medium may be placed within or disposed next to a detector (e.g., a fluorescence imaging system) that can generate a real-time fluorescence readout from each well, and the change in signal identified by the detector (e.g., a change in fluorescence intensity) within each well can be correlated with the presence, absence, or concentration of a particular nucleic acid sequence from the sample. The support medium may be disposable (e.g., single use).

Alternatively, a reagent may be stored in a container, and later placed into a reagent chamber or support medium by the individual using the kit. The container can be any suitable container for stably storing and transporting the reagent. For example, a reagent may be stored in an Eppendorf^{®} tube, a centrifuge tube, a vial, a screw top vial, an ampule, a bottle, a bag, or any other container capable of holding the reagent. The reagent may be provided frozen, in dry form (e.g., as a powder such as a lyophilized powder), or as a solution. A reagent may be divided between multiple containers or may be provided in a single container.

In some embodiments, a kit for detecting a target nucleic acid comprising a support medium; a guide nucleic acid targeting a target nucleic acid segment; a programmable nuclease capable of being activated when complexed with the guide nucleic acid and the target nucleic acid segment; and a single stranded reporter comprising a detection moiety, wherein the reporter is capable of being cleaved by the activated nuclease, thereby generating a first detectable signal.

In some embodiments, a kit for detecting a target nucleic acid comprising a PCR plate; a guide nucleic acid targeting a target nucleic acid segment; a programmable nuclease capable of being activated when complexed with the guide nucleic acid and the target nucleic acid segment; and a single stranded reporter comprising a detection moiety, wherein the reporter is capable of being cleaved by the activated nuclease, thereby generating a first detectable signal. The wells of the PCR plate can be pre-aliquoted with the guide nucleic acid targeting a target nucleic acid segment, a programmable nuclease capable of being activated when complexed with the guide nucleic acid and the target sequence, and at least one population of a single stranded reporter comprising a detection moiety. A user can thus add the biological sample of interest to a well of the pre-aliquoted PCR plate and measure for the detectable signal with a fluorescent light reader or a visible light reader.

In some instances, such kits may include a package, carrier, or container that is compartmentalized to receive one or more containers such as vials, tubes, and the like, each of the container(s) comprising one of the separate elements to be used in a method described herein. Suitable containers include, for example, test wells, bottles, vials, and test tubes. In one embodiment, the containers are formed from a variety of materials such as glass, plastic, or polymers.

The kit or systems described herein contain packaging materials. Examples of packaging materials include, but are not limited to, pouches, blister packs, bottles, tubes, bags, containers, bottles, and any packaging material suitable for intended mode of use.

A kit typically includes labels listing contents and/or instructions for use, and package inserts with instructions for use. A set of instructions will also typically be included. In one embodiment, a label is on or associated with the container. In some instances, a label is on a container when letters, numbers or other characters forming the label are attached, molded or etched into the container itself; a label is associated with a container when it is present within a receptacle or carrier that also holds the container, e.g., as a package insert. In one embodiment, a label is used to indicate that the contents are to be used for a specific therapeutic application. The label also indicates directions for use of the contents, such as in the methods described herein.

After packaging the formed product and wrapping or boxing to maintain a sterile barrier, the product may be terminally sterilized by heat sterilization, gas sterilization, gamma irradiation, or by electron beam sterilization. Alternatively, the product may be prepared and packaged by aseptic processing.

A kit may be designed for long term storage. A kit may lose less than 5% accuracy after 3 months of storage. A kit may lose less than 5% accuracy after 6 months of storage. A kit may lose less than 5% accuracy after 9 months of storage. A kit may lose less than 5% accuracy after 12 months of storage. A kit may lose less than 5% accuracy after 18 months of storage. A kit may lose less than 5% accuracy after 24 months of storage. A kit may lose less than 5% accuracy after 36 months of storage. A kit may lose less than 10% accuracy after 3 months of storage. A kit may lose less than 20% accuracy after 3 months of storage. A kit may have a shelf life of at least 3 months after storage at room temperature storage. A kit may have a shelf life of at least 3 months after storage between 1 °C and 30 °C. A kit may have a shelf life of at least 3 months after storage between 10 °C and 30 °C. A kit may have a shelf life of at least 3 months after storage between 15 °C and 35 °C. A kit may have a shelf life of at least 3 months after storage between 20 °C and 40 °C. A kit may have a shelf life of at least 3 months after storage between 1 °C and 15 °C. A kit may have a shelf life of at least 3 months after storage between 1 °C and 10 °C. A kit may be designed for sub 0 °C storage. For example, a kit may comprise storage containers that do not break, seals that do not fail, and reagents that do not lose meaningful activity (e.g., < 5%) after multiple freeze-thaw cycles. The kit may be shipped and stored at room temperature. The kit may be shipped and stored at a temperature between 1 and 10 °C. The kit may be shipped and stored below 0 °C, and then thawed prior to use.

In some embodiments, a kit for detecting a target nucleic acid comprises: an amplification reagent mix comprising target primers designed to hybridize to the target nucleic acid, and a polymerase; and a detection reagent mix comprising a programmable nuclease, a target guide nucleic acid designed to hybridize to the target nucleic acid, and a reporter.

In some embodiments, the kit further comprises: a control amplification mix comprising control primers designed to hybridize to a control nucleic acid, and the polymerase; and a control detection reagent mix comprising a programmable nuclease, a control guide nucleic acid designed to hybridize to the control nucleic acid, and the reporter.

In some embodiments, the target nucleic acid is a viral nucleic acid. In some embodiments, the target nucleic acid is a coronavirus nucleic acid. In some embodiments, the target nucleic acid is from a SARS-CoV-2 N gene or a SARS-CoV-2 E gene. In some embodiments, the target nucleic acid is selected from any one of SEQ ID NO: 179 - SEQ ID NO: 184. In some embodiments, the guide nucleic acid has at least 80%, at least 85%, at least 90%, at least 92%, at least 95%, at least 97%, at least 99%, or 100% sequence identify to any one of SEQ ID NO: 318 - SEQ ID NO: 327. In some embodiments, the guide nucleic acid is selected from any one of SEQ ID NO: 318 - SEQ ID NO: 327.

In some embodiments, the kit further comprises: a second amplification reagent mix comprising second target primers designed to hybridize to a second target nucleic acid, and the polymerase; and a second detection reagent mix comprising the programmable nuclease, a second target guide nucleic acid designed to hybridize to the second target nucleic acid, and the reporter. In some embodiments, the second target nucleic acid is a viral nucleic acid. In some embodiments, the second target nucleic acid is a coronavirus nucleic acid. In some embodiments, the second target nucleic acid is from a SARS-CoV-2 N gene or a SARS-CoV-2 E gene. In some embodiments, the second target nucleic acid is selected from any one of SEQ ID NO: 179 - SEQ ID NO: 184.

In some embodiments, the second target guide nucleic acid comprises a sequence that has at least 80%, at least 85%, at least 90%, at least 92%, at least 95%, at least 97%, at least 99%, or 100% sequence identity to any one of SEQ ID NO: 318 - SEQ ID NO: 327. In some embodiments, the second target guide nucleic acid is selected from any one of SEQ ID NO: 318 - SEQ ID NO: 327. In some embodiments, the control nucleic acid is from an RNase P nucleic acid. In some embodiments, the RNase P nucleic acid is an RNase P POP7 nucleic acid. In some embodiments, the control nucleic acid has at least 80%, at least 85%, at least 90%, at least 92%, at least 95%, at least 97%, at least 99%, or 100% sequence identify to SEQ ID NO: 220. In some embodiments, the control guide nucleic acid has at least 80%, at least 85%, at least 90%, at least 92%, at least 95%, at least 97%, at least 99%, or 100% sequence identify to any one of SEQ ID NO: 320, SEQ ID NO: 326, or SEQ ID NO: 327.

In some embodiments, the kit further comprises in vitro transcription reagents. In some embodiments, the in vitro transcription reagents comprise an RNA polymerase, a transcription primer, and nucleotide triphosphates (NTPs). In some embodiments, the target primers are designed for thermal cycling amplification of the target nucleic acid. In some embodiments, the target primers are designed for isothermal amplification of the target nucleic acid. In some embodiments, the isothermal amplification comprises transcription mediated amplification (TMA), helicase dependent amplification (HDA), circular helicase dependent amplification (cHDA), strand displacement amplification (SDA), loop mediated amplification (LAMP), exponential amplification reaction (EXPAR), rolling circle amplification (RCA), ligase chain reaction (LCR), simple method amplifying RNA targets (SMART), single primer isothermal amplification (SPIA), multiple displacement amplification (MDA), nucleic acid sequence based amplification (NASBA), hinge-initiated primer-dependent amplification of nucleic acids (HIP), nicking enzyme amplification reaction (NEAR), or improved multiple displacement amplification (IMDA). In some embodiments, the target primers comprise a FIP primer, a BIP primer, a LF primer, and a LB primer. In some embodiments, the kit further comprises reverse transcription reagents. In some embodiments, the reverse transcription reagents comprise a reverse transcriptase, an oligonucleotide primer, and dNTPs.

In some embodiments, the amplification reagent mix is contained within a first vial and the detection reagent mix is contained within a second vial. In some embodiments, the control amplification mix is contained within a third vial. In some embodiments, the control nucleic acid is contained within a fourth vial. In some embodiments, the amplification activator is contained within a fifth vial. In some embodiments, the second amplification reagent mix is contained within a sixth vial and the second detection reagent mix is contained within a seventh vial. In some embodiments, the contents of the first vial, the second vial, the third vial, the fourth vial, the fifth vial, the sixths vial, the seventh vial, or combinations thereof are frozen. In some embodiments, the kit further comprising a pipette, a multi-well plate, or both. In some embodiments, the pipette is a disposable pipette.

In some aspects, a method of diagnosing a disease or condition comprises contacting a sample to the kit of any one of previous embodiments. In some embodiments, the method comprises contacting the sample to the amplification reagent mix, the detection reagent mix, or both. In some embodiments, the method comprises contacting the sample to the second amplification reagent mix, the second detection reagent mix, or both. In some embodiments, the method comprises the control nucleic acid to the control amplification mix, the control detection reagent mix, or both. In some embodiments, the method comprises cleavage of the reporter by the programmable nuclease upon contacting the control nucleic acid to the control detection reagent mix. In some embodiments, the method comprises cleavage of the reporter by the programmable nuclease upon contacting the sample to the detection reagent mix, wherein the sample comprises the target nucleic acid. In some embodiments, the method comprises cleavage of the reporter by the programmable nuclease upon contacting the sample to the second detection reagent mix, wherein the sample comprises the target nucleic acid. In some embodiments, the method comprises producing a detectable signal upon cleavage of the reporter by the programmable nuclease.

### EXAMPLES

The following examples are illustrative and non-limiting to the scope of the devices, methods, reagents, systems, compositions, and kits described herein.

### EXAMPLE 1

### Optimization of an RT-LAMP Buffer

This example describes the optimization of a buffer for use in amplification reactions. Various buffer components were interrogated through a design of experimentation (DOE) methodology to maximize the rate of a reverse transcription loop-mediated isothermal amplification (RT-LAMP). In total, 23 phosphate buffers with varying pH, ammonium sulfate concentration, potassium chloride concentration, and magnesium salt-type (summarized in TABLE 6 below) were tested for their abilities to support RT-LAMP reactions.

**TABLE 6**

| FORMULATION NAME | BUFFER SYSTEM | PH | (NH₄)₂SO₄ (MM) | KCL (MM) | MG(OAC)₂ (MM) | MGSO₄ | TWEEN-20 (%) |
|---|---|---|---|---|---|---|---|
| Test 1 | Phosphate | 8.2 | 8 | 38 | 2 | 0 | 0.1 |
| Test 2 | Phosphate | 8.6 | 8 | 2 | 2 | 0 | 0.1 |
| Test 3 | Phosphate | 8.6 | 2 | 38 | 2 | 0 | 0.1 |
| Test 4 | Phosphate | 8.8 | 10 | 50 | 2 | 0 | 0.1 |
| Test 5 | Phosphate | 8.8 | 4 | 14 | 2 | 0 | 0.1 |
| Test 6 | Phosphate | 8.2 | 8 | 50 | 2 | 0 | 0.1 |
| Test 7 | Phosphate | 8.2 | 2 | 14 | 2 | 0 | 0.1 |
| Test 8 | Phosphate | 8.8 | 2 | 2 | 2 | 0 | 0.1 |
| Test 9 | Phosphate | 8.6 | 10 | 50 | 2 | 0 | 0.1 |
| Test 10 | Phosphate | 8.0 | 4 | 14 | 2 | 0 | 0.1 |
| Test 11 | Phosphate | 8.0 | 4 | 2 | 2 | 0 | 0.1 |
| Test 12 | Phosphate | 8.0 | 10 | 38 | 2 | 0 | 0.1 |
| Test 13 | Phosphate | 8.0 | 2 | 50 | 2 | 0 | 0.1 |
| Test 14 | Phosphate | 8.6 | 4 | 38 | 0 | 2 | 0.1 |
| Test 15 | Phosphate | 8.4 | 2 | 2 | 0 | 2 | 0.1 |
| Test 16 | Phosphate | 8.0 | 2 | 50 | 0 | 2 | 0.1 |
| Test 17 | Phosphate | 8.2 | 10 | 2 | 0 | 2 | 0.1 |
| Test 18 | Phosphate | 8.2 | 4 | 50 | 0 | 2 | 0.1 |
| Test 19 | Phosphate | 8.6 | 10 | 14 | 0 | 2 | 0.1 |
| Test 20 | Phosphate | 8.8 | 2 | 38 | 0 | 2 | 0.1 |
| Test 21 | Phosphate | 8.0 | 10 | 26 | 0 | 2 | 0.1 |
| Test 22 | Phosphate | 8.8 | 8 | 14 | 0 | 2 | 0.1 |
| Test 23 | Phosphate | 8.0 | 8 | 2 | 0 | 2 | 0.1 |
| Commercial Control | Tris | 8.8 | 10 | 50 | 0 | 2 | 0.1 |

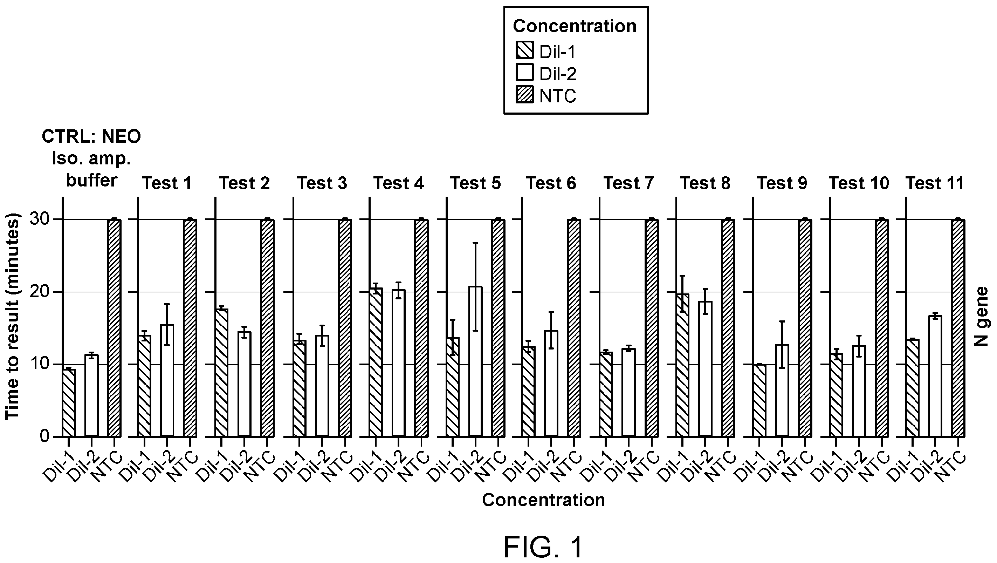

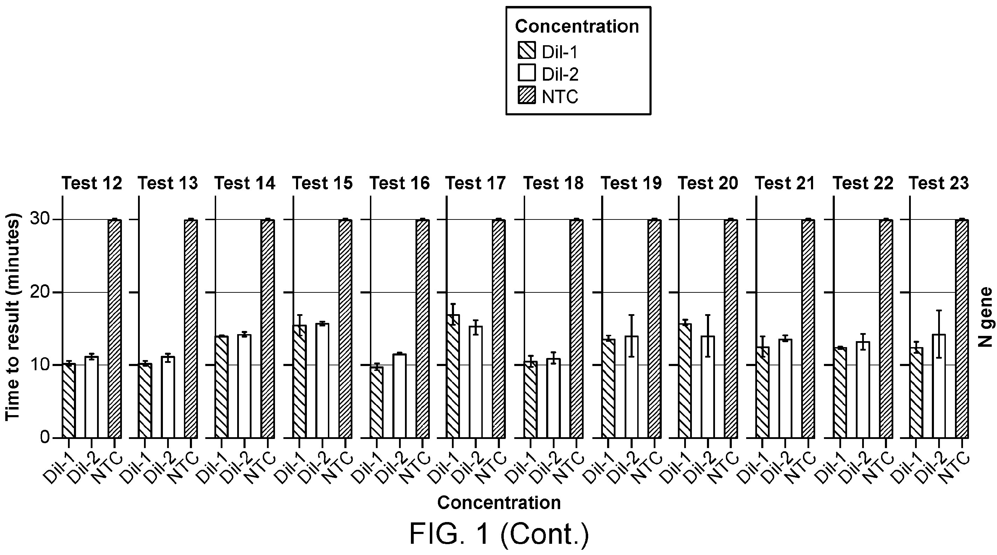

**FIG. 1** shows RT-LAMP results for each buffer, measured as time to final product. The reactions were performed with two separate dilutions (Dil-1 and Dil-2) of SARS-CoV-2 N-gene RNA, as well as in the absence of target nucleic acid (NTC). The reactions were performed at 62 °C with 0.2 µM forward and backward outer primers, 1.6 µM forward and backward inner primers, and 0.8 µM loop forward and loop backward primers. In addition to the buffer constituents listed in TABLE 6, each reaction contained 1.4 mM dNTP, 0.8 unit/µl NEB Bst 2.0 DNA polymerase; 1.4 mM dNTP, 0.56 unit/µL NEB Warmstart^{®} RTx reverse transcriptase, 0.8 unit/µl NEB Murine Rnase inhibitor, 1 µM Invitrogen SYT09 green fluorescent nucleic acid stain, and 4.5 mM magnesium sulfate (in addition to the amount of magnesium sulfate listed in TABLE 6). A number of buffer formulations had comparable times to final product as the commercial control NEB 1 LAMP buffer (e.g., tests 12, 13 & 16).

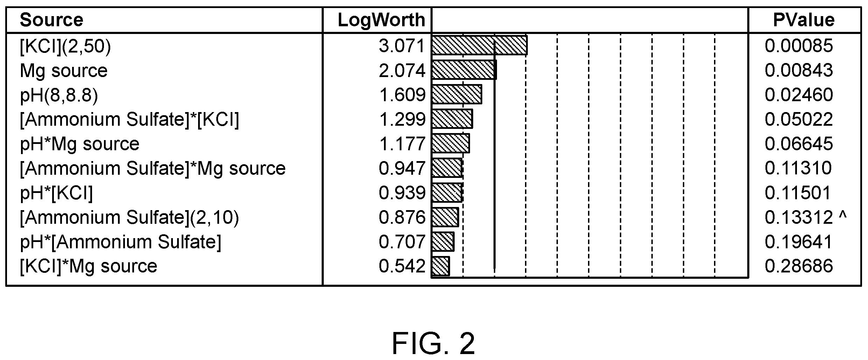

FIG. 2 provides an effect summary for the parameters varied in the assay. The table provides the statistical significance of a variable or set of variables (listed in the 'Source' column) on the time to final product values shown in FIG. 1. Some rows provide a coupled effect for two variables (e.g., 'pH*Mg source'), while others list the significance for a single variable (e.g., [KCl](2,50), which refers to KCl concentration). The variables are listed in order of statistical significance, with 'LogWorth' showing the negative Log10 of the p-value for each variable. The vertical line in the 'LogWorth' column corresponds a p-value of 0.01. As is shown in FIG. 2, KCl concentration and Mg source were found to be significant at a p=0.01 level under the conditions tested.

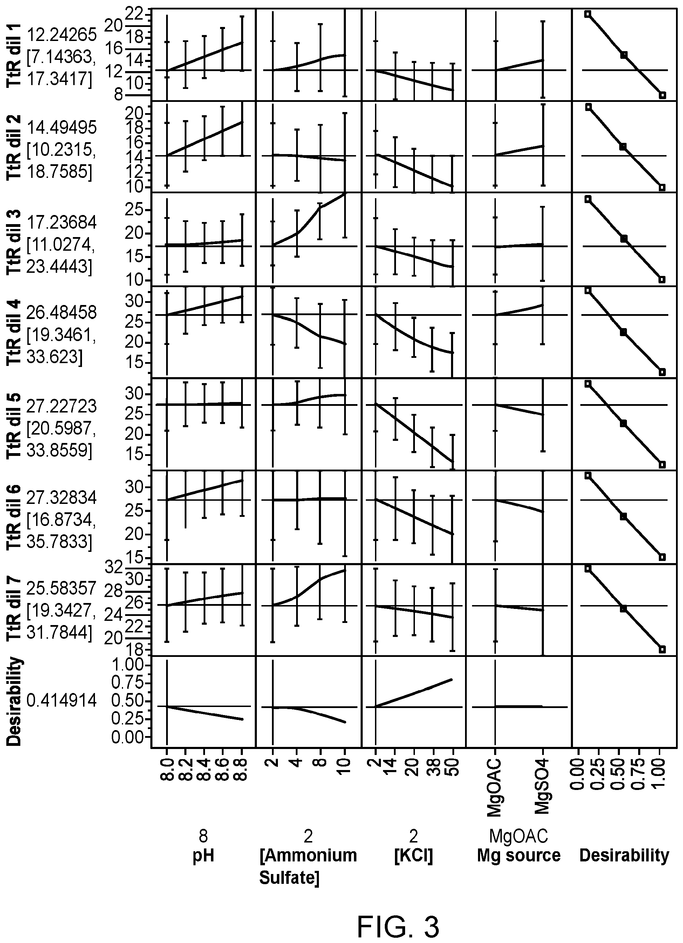

FIG. 3 provides a graphical representation of the anticipated effect of different buffer components on RT-LAMP reaction times. The first three columns show the effects of pH, ammonium sulfate concentration, and potassium chloride (x-axes) on RT-LAMP time to final result (y-axes). The fourth column shows a binary comparison between magnesium acetate and magnesium sulfate as the Mg2+ source on the RT-LAMP time to result. The 'Desirability' column shows the weighting function applied against the measured reaction rates used to generate the bottom ('Desirability) row of FIG. 3, which charts parameter optimizations for each variable. As can be seen from the chart, higher pH, higher ammonium sulfate concentration, and lower KCl concentration may correlate with faster RT-LAMP reactions, while no effect was observed for switching Mg2+ source under the conditions tested.

### EXAMPLE 2

### Optimization of a Buffer For detection of a Target Nucleic Acid

This example describes the optimization of a buffer for use in CRISPR-based detection (e.g., DETECTR) reactions. Test buffers were generated by varying the composition of a buffer capable of supporting DETECTR reactions. Fifteen phosphate buffers with varying pH, ammonium sulfate concentrations, potassium acetate concentrations, detergents, and detergent concentrations (provided in TABLE 7 below) were tested for their abilities to support DETECTR reactions. In addition to the reagents listed, all buffers contained 5 mM magnesium acetate and 1 % glycerol.

**TABLE 7**

| FORMULATION NAME | BUFFER SYSTEM | PH | (NH₄)₂SO₄ (MM) | KOAC (MM) | DETERGENT | DETERGENT CONCENTRATION (% V/V) |
|---|---|---|---|---|---|---|
| Test 1 | Phosphate | 8.25 | 0 | 10 | Triton x-100 | 0.00016 |
| Test 2 | Phosphate | 7.5 | 2 | 30 | Triton x-100 | 0.00016 |
| Test 3 | Phosphate | 7.75 | 0 | 2 | Tween 20 | 0.10000 |
| Test 4 | Phosphate | 8 | 0 | 20 | Tween 20 | 0.10000 |
| Test 5 | Phosphate | 8.25 | 2 | 2 | Tween 20 | 0.10000 |
| Test 6 | Phosphate | 7.5 | 0 | 10 | Tween 20 | 0.10000 |
| Test 7 | Phosphate | 7.5 | 0 | 2 | Triton x-100 | 0.00016 |
| Test 8 | Phosphate | 7.75 | 2 | 30 | Tween 20 | 0.10000 |
| Test 9 | Phosphate | 7.75 | 2 | 10 | Triton x-100 | 0.00016 |
| Test 10 | Phosphate | 7.5 | 2 | 20 | Tween 20 | 0.10000 |
| Test 11 | Phosphate | 8 | 0 | 30 | Triton x-100 | 0.00016 |
| Test 12 | Phosphate | 8.25 | 0 | 30 | Tween 20 | 0.10000 |
| Test 13 | Phosphate | 8 | 2 | 2 | Triton x-100 | 0.00016 |
| Test 14 | Phosphate | 7.75 | 0 | 20 | Triton x-100 | 0.00016 |
| Test 15 | Phosphate | 8.25 | 2 | 20 | Triton x-100 | 0.00016 |
| Commercial Control | HEPES | 7.5 | 0 | 2 | Triton x-100 | 0.00016 |

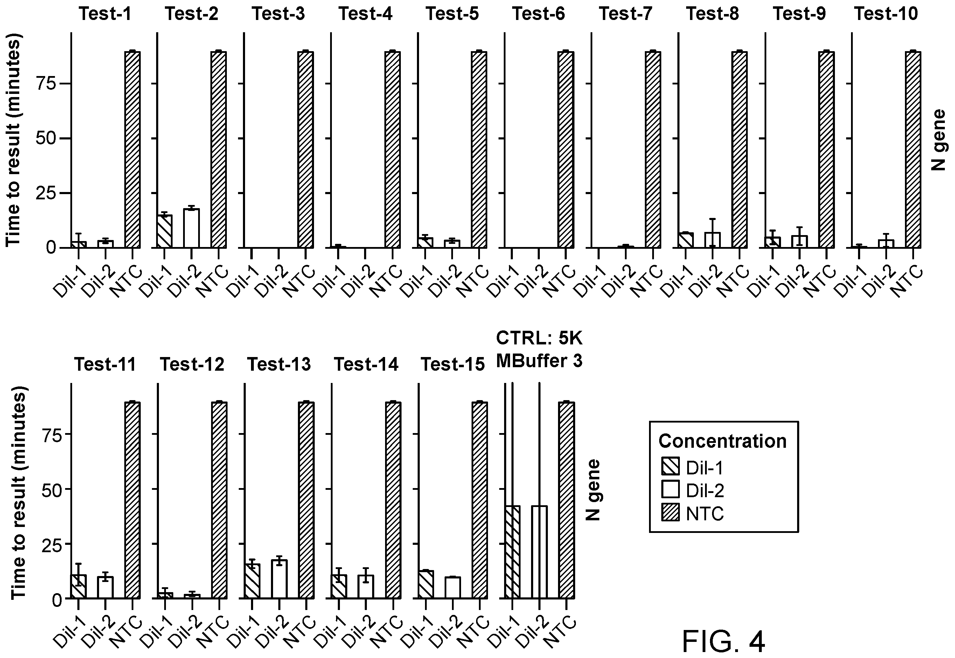

FIG. **4** shows the results of DETECTR reactions performed at 37 °C with the modified DETECTR buffers listed in TABLE 7, **SEQ ID NO:** 28 targeting SARS-CoV-2 N-gene, and two separate dilutions of SARS-CoV-2 N-gene LAMP amplicons, 'Dil 1' & 'Dil 2', corresponding to 1000 and 500 starting copies of the SARS-CoV-2 N gene template, respectively.. Controls were performed with conditions lacking target nucleic acid (NTC). As can be seen in the figure, Tests 1, 2, 4, 5 & 7-15 performed faster DETECTR reactions than the commercially available control buffer. Tests 3 & 6 did not support reactions.

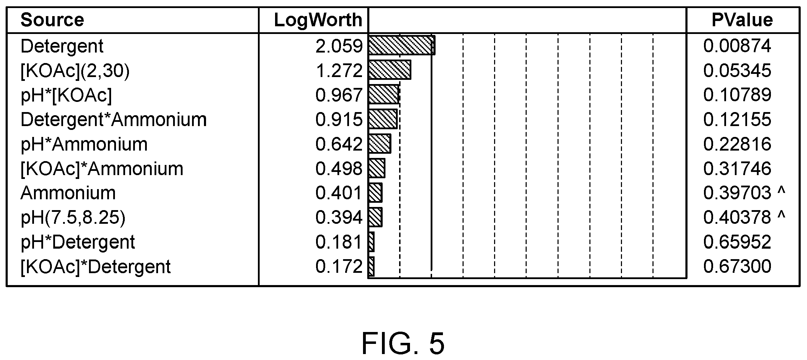

FIG. 5 provides an effect summary for the parameters varied in the assay of FIG. 4. The vertical line in the 'LogWorth' column corresponds to a p-value of 0.01. Among the variables, only detergent was significant to a p=0.01 level, indicating that 0.1% (v/v) Tween 20 supports faster DETECTR reaction rates than 0.00016% (v/v) Triton X-100. The remaining variables had weaker correlations with DETECTR reaction rates under the conditions tested.

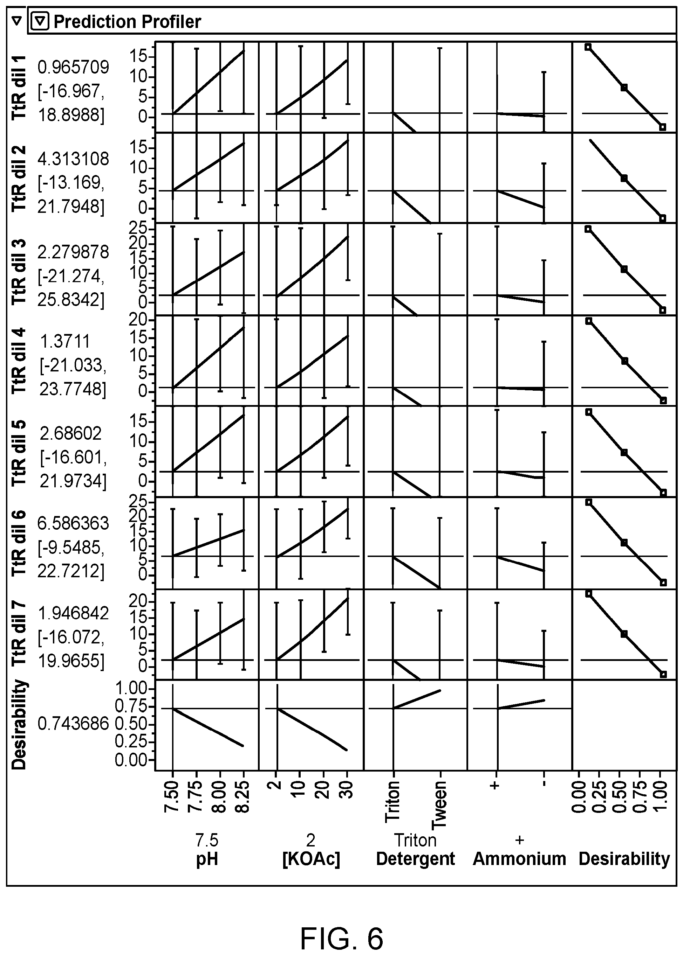

However, as can be seen in FIG. 6, which provides a graphical representation of measured effect sizes for various buffer constituents on DETECTR reaction times, a number of weakly correlating variables had considerable effect sizes. For example, while pH and potassium acetate concentration had p-values of 0.5345 and 0.40378, respectively, both had changes in desirability scores of nearly 0.5 in going from the lowest to highest measured concentrations. Conversely, detergent type had a p-value of 0.00874, but a change in desirability of 0.25, with Tween 20 supporting faster DETECTR reactions than Triton x-100. The absence of ammonium sulfate led to slightly faster DETECTR reactions.

### EXAMPLE 3

### Optimization of a Dual Amplification, CRISPR Reaction Buffer

This example describes the optimization of a buffer that can support both amplification and DETECTR reactions, enabling assays that perform both reactions in a single buffer. For low titer samples (e.g., low viral titer samples), DETECTR reactions often require an amplification step prior to detection. In such cases, the amplification and DETECTR reactions are typically performed in separate buffers, potentially leading to sample loss, contamination, and potentially high user input requirements. This example provides a single buffer capable of supporting both amplification and DETECTR reactions, without hindering the kinetics of either reaction. The single buffer platform is useful for streamlining the assay process, and may reduce the time, complexity, and/or contamination during the assay step.

Buffers for amplification and DETECTR reactions are often optimized separately for dual assays. In such cases, the products of the amplification reaction need to be exchanged into a new buffer capable of supporting the DETECTR reaction prior to the detection step. For example, the inventors previously showed that RT-LAMP could be performed at 60-62 °C in 20 mM, pH 8.8 Tris buffer containing 10 mM (NH4)2SO4, 50 mM KCl, 6.5 mM MgSO4, 0.1% Tween20, and that the RT-LAMP products could be buffer exchanged into 20 mM, pH 7.5 HEPES containing 20 mM KOAc, 5 mM Mg(OAc)2, 1% glycerol, and 0.00016% Triton X-100 for 37 °C DETECTR reactions. However, these two buffer systems were not optimized for cross-compatibility.

In this example, multiple phosphate buffers were formulated based on the results from Examples 1 and 2 indicating the buffer constituents necessary for RT-LAMP and DETECTR. Five buffers were generated with varying concentrations of ammonium sulfate, potassium acetate, potassium chloride, magnesium acetate, magnesium sulfate, detergent and glycerol, and varying pH, summarized in TABLE 8 below. The rates of RT-LAMP and DETECTR were measured in each buffer, and compared against an assay utilizing separate buffers optimized for RT-LAMP and DETECTR reactions (NEB IsoAmp Tris buffer and Mg(OAc)2 containing HEPES buffer, respectively). SARS-CoV-2 N-gene was the target nucleic acid in the amplification and DETECTR reactions. The DETECTR assays utilized SEQ ID NO: 28 as a programmable nuclease. Each buffer was tested with seven separate dilutions of the target nucleic acid (Dilution 1-7) and against a control sample lacking the target nucleic acid (NTC). DETECTR reactions were performed by adding 2 uL RT-LAMP product to 18 uL of DETECTR reagents in the same type of buffer (or different type of buffer for the separate buffer comparator).

**TABLE 8**

| FORMULATION NAME | BUFFER SYSTEM | PH | (NH₄)₂SO₄ (mM) | KOAc (mM) | KCl (mM) | Mg(OAc)₂ (mM) | MgSO₄ (mM) | DETERGENT | DETERGENT CONC (%) | Glycerol (%) |
|---|---|---|---|---|---|---|---|---|---|---|
| Single 8 | PO₄³- | 8 | 2 | 25 | 0 | 2 | 0 | Tween 20 | 0.1 | 1 |
| Single 9 | PO₄³- | 8 | 2 | 20 | 0 | 5 | 0 | Tween 20 | 0.1 | 1 |
| Single 10 | PO₄³- | 8.25 | 2 | 15 | 0 | 5 | 0 | Tween 20 | 0.1 | 1 |
| DET 4 | PO₄³- | 8 | 0 | 20 | 0 | 5 | 0 | Tween 20 | 0.1 | 1 |
| DET 8 | PO₄³- | 7.75 | 2 | 30 | 0 | 5 | 0 | Tween 20 | 0.1 | 1 |
| NEB IsoAmp | Tris | 8.8 | 10 | 0 | 50 | 0 | 2 | Tween 20 | 0.1 | 0 |
| MBuffer3 | HEPES | 7.5 | 0 | 2 | 0 | 5 | 0 | Triton x-100 | 0.00016 | 1 |

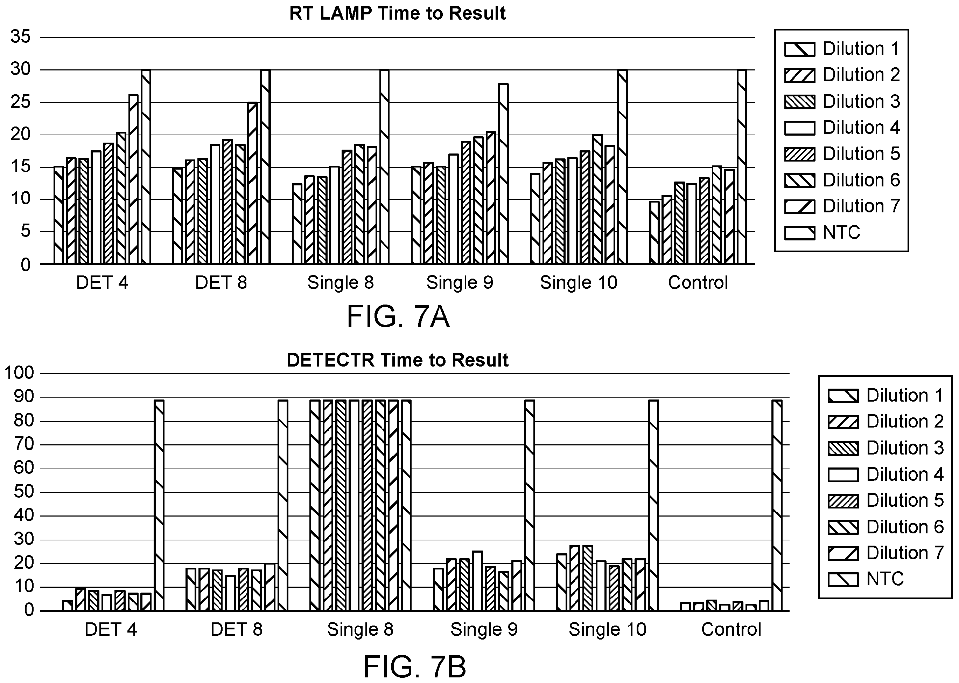

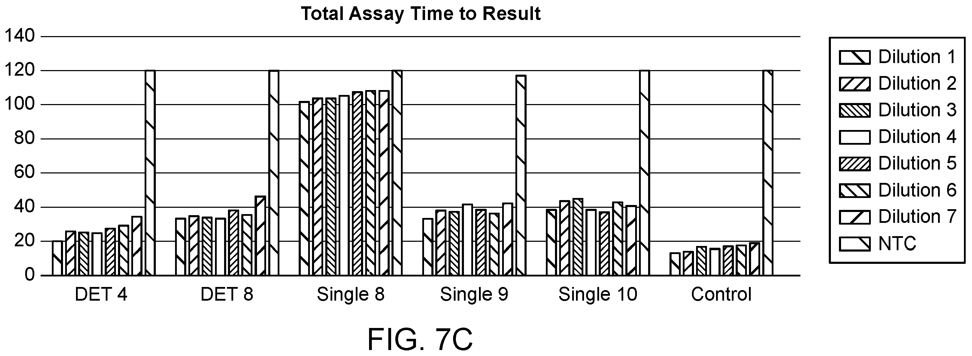

As is shown in **FIG. 7****,** four of the five buffers tested supported RT-LAMP and DETECTR. The total assay times for these four formulations were ~1.5-2.5 greater than for the two buffer system. The formulation 'DET 4' had the shortest average total assay time of around 25 minutes, as compared to 17 minutes for the 2-buffer control system. These results not only show that it is possible to formulate a single buffer system capable of supporting separate amplification and CRISPR reactions, but also that such a buffer can enable comparably fast amplification and CRISPR reaction rates. These results also highlight pH 8.0 phosphate buffer containing 20 mM potassium acetate, 5 mM magnesium acetate, 1 % glycerol and 0.1% Tween-20 as an effective buffer for amplification and CRISPR reactions under the conditions tested.

### EXAMPLE 4

### Formulation of Tris Buffers for Amplification Assays

This example describes the formulation of a buffer RT-LAMP. This assay built on the results of Example 3, which found pH 8.0 buffer with potassium acetate, magnesium acetate, glycerol and Tween-20 to be capable of supporting amplification and CRISPR reactions. While the previous examples cover optimization of phosphate buffers, this example tested the efficacy of various Tris buffer formulations.

The rates of RT-LAMP reactions were measured in four different pH 8.0 Tris-buffer systems. Each buffer contained 20 mM potassium acetate, 5 mM magnesium acetate, 1% glycerol and 0.1% Tween-20. The buffer in test 1 contained 20 mM Tris. The buffer in test 2 contained 20 mM Tris and 2 mM ammonium sulfate. The buffer in test 3 contained 10 mM Tris. The buffer in test 4 contained 10 mM Tris and 2 mM ammonium sulfate. The rates of the four test reactions were compared against the rates of control reactions performed with NEB IsoAMP buffer (TABLE 3).

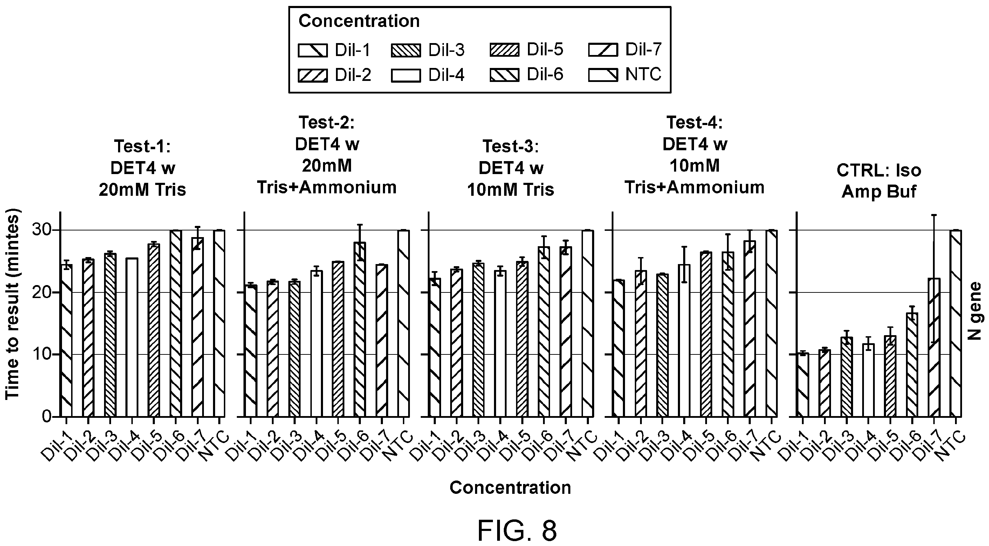

FIG. 8 shows the mean reaction times of the RT-LAMP assays. The rates of RT-LAMP were measured with seven separate dilutions of SARS-CoV-2 N-gene (Dil-1 through Dil-7) and with sample containing no target nucleic acid (NTC). The rates of the four test reactions were approximately half that of the reaction performed in the NEB IsoAmp control buffer. The fastest rates were observed in test 2, which utilized 20 mM Tris buffer with 2 mM ammonium sulfate.

### EXAMPLE 5

### Buffer Formulations for Dual Amplification, CRISPR Assays

This example describes various buffer formulations for dual amplification, CRISPR-based assays. These assays used low input titers comprising 1000 starting copies of the target nucleic acid. Dual RT-LAMP, DETECTR assays were performed in four separate buffer systems, with SARS-CoV-2 RNA as the target nucleic acid and SEQ ID NO: 28 used as the programmable nuclease. The rates of the four assays were compared against an assay performed with separate buffers for the RT-LAMP and DETECTR reactions (NEB IsoAmp and MBuffer, respectively).

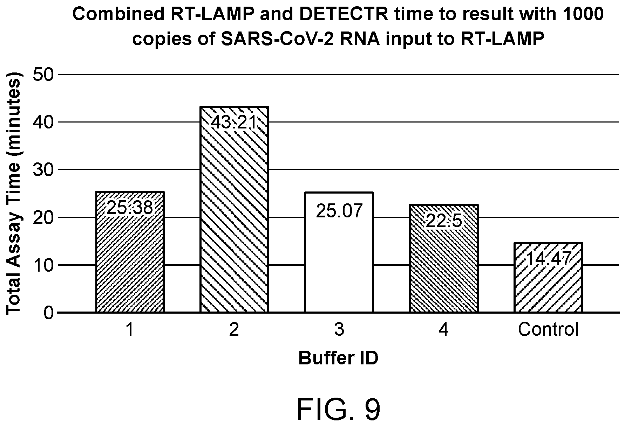

The compositions of the four buffers interrogated are provided in TABLE 9, with each additionally containing 1% glycerol, 0.1% (v/v) Tween 20, and 5 mM magnesium acetate. As is shown in FIG. 9, formulations 1, 3 & 4 supported assays with about half the rate of the two-buffer system. These results show that ammonium salt may not be a necessary component for phosphate buffers used in combined RT-LAMP and DETECTR assays.

**TABLE 9**

| FORMULATION NAME | BUFFER SYSTEM | PH | (NH₄)₂SO₄ (MM) | KOAC (MM) |
|---|---|---|---|---|
| 1 | Phosphate | 8 | 0 | 20 |
| 2 | Phosphate | 7.75 | 2 | 30 |
| 3 | Phosphate | 8.25 | 0 | 30 |
| 4 | Tris | 8 | 2 | 20 |

### EXAMPLE 6

### Buffer-Type Optimization for Amplification Reactions

This example describes the formulation of buffers that can be used for amplification reactions. Amplification reaction rates are heavily dependent on buffer conditions. Thus, buffer optimization is an important component of assay optimization. Previous examples have covered buffer optimization for a single type of buffer system (e.g., phosphate, Tris, etc.). This example covers buffer optimization across multiple types of buffer systems.

The rate of RT-LAMP was measured in ten separate buffer formulations. The RT-LAMP was performed with SARS-CoV-2 N-gene as the target nucleic acid, using input titers with 1000 target nucleic acid copies. Each buffer was set to a pH of 8.0 and contained 20 mM potassium acetate, 5 mM magnesium acetate, 1% glycerol and 0.1% Tween-20. The types and concentrations of ammonium salt and buffer varied between each of the ten formulation, as is shown in TABLE 10, with the buffer designation 'Filtered' (formulation 10) corresponding to 10 mM phosphate buffer passed through a 0.22 micron membrane filter. A control RT-LAMP reaction was performed in NEB IsoAmp Buffer (TABLE 3).

**TABLE 10**

| | FORMULATION NAME | | | | | | | | | |
|---|---|---|---|---|---|---|---|---|---|---|
| | 1 | 2 | 3 | 4 | 5 | 6 | 7 | 8 | 9 | 10 |
| Buffer System | 10 mM Phosphate | 20 mM HEPES | 20 mM HEPES | 20 mM Imidazole | 20 mM Imidazole | 20 mM HEPES | 20 mM Imidazole | 20 mM Tris | 20 mM Phosphate | 10 mM Filtered |
| Ammoni um Salt | 2 mM (NH₄)₂SO 4 | 2 mM (NH₄)₂S O₄ | 0 mM NH₄⁺ | 2 mM (NH₄)₂SO 4 | 0 mM NH₄⁺ | 2 mM NH₄OA c | 2 mM NH₄OAc | 2 mM NH₄OA c | 2 mM NH₄OAc | 2 mM NH₄OA c |

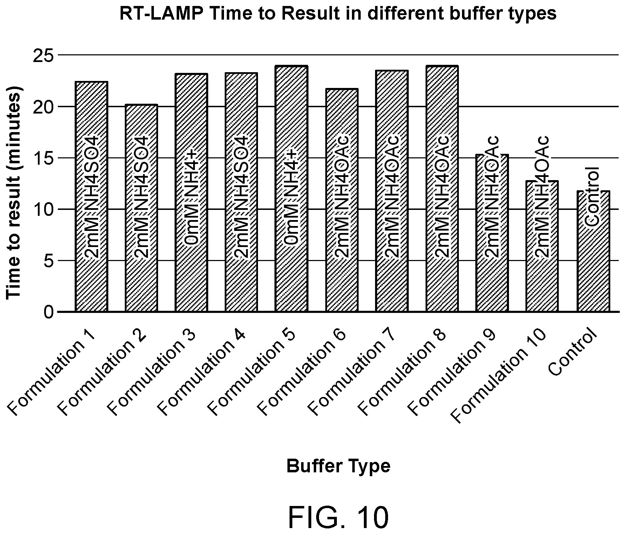

As is shown in **FIG. 10****,** the rates of the RT-LAMP reactions varied from 2 to only 1.1 times the rate of the RT-LAMP reaction performed in the NEB IsoAmp Buffer. Formulation 9, which contained 20 mM phosphate buffer and ammonium acetate, supported an RT-LAMP reaction that was only three minutes slower than the reaction performed in NEB IsoAmp buffer, and twice as fast as the reaction performed with half the phosphate buffer concentration and ammonium sulfate (Formulation 1). The highest performing formulation (Formulation 10 with filtered buffer) facilitated an RT-LAMP reaction that was only one minute slower than the reaction performed in the NEB IsoAmp buffer.

### EXAMPLE 7

### Buffer-System Optimization for Low Titer Amplification Reactions

This example describes the effect of buffer-system on total assay time for assays that involve both amplification and CRISPR reactions. The rates of amplification and CRISPR reactions are strongly dependent on solution composition and reaction conditions. While a number of buffers have been optimized for fast amplification or fast CRISPR reactions, these buffers are often only compatible for use in a single type of reaction. As there is a need for buffers capable of supporting fast rates for amplification and CRISPR reactions, this example explores the buffer-system dependence on amplification and CRISPR reaction rates.

Four separate buffers were formulated for use in dual RT-LAMP, DETECTR reactions. Building on the findings of the previous examples, each buffer was set to pH 8.0 and contained 2 mM Ammonium Acetate, 20 mM Potassium Acetate, 5 mM Magnesium Acetate, 1% Glycerol (v/v), and 0.1% Tween-20 (v/v). The RT-LAMP and DETECTR reactions targeted SARS-CoV-2 N-gene, and the DETECTR reactions utilized SEQ ID NO: 28 as a programmable nuclease. The assays were performed with variable input titers ranging from 250 to 1000 copies of the target nucleic acid. The times required to complete the single buffer assays were compared against the times required to complete corresponding two buffer assays utilizing NEB IsoAmp for RT-LAMP reactions and MBuffer3 for DETECTR reactions (TABLE 3).

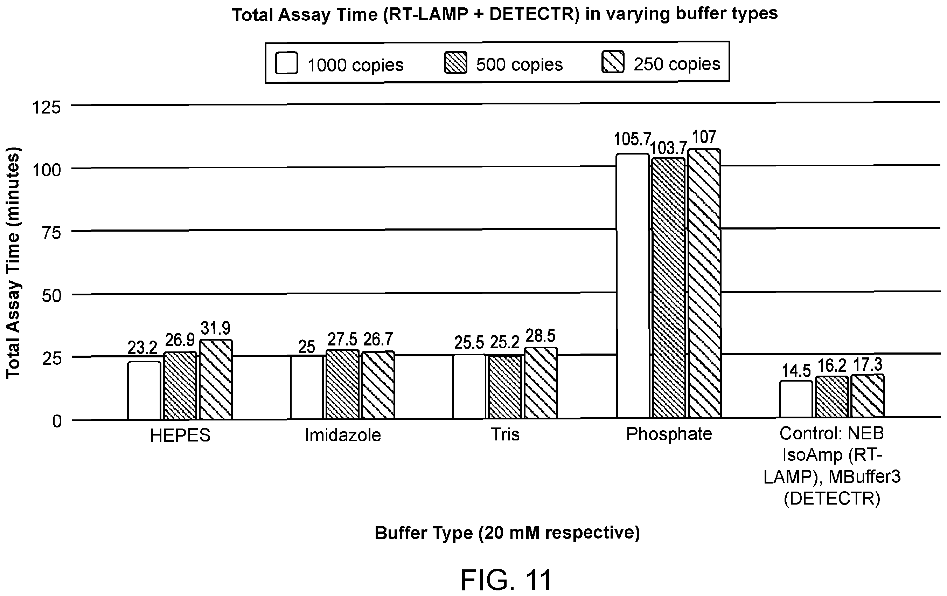

As is shown in FIG. 11, HEPES, Imidazole, and Tris buffer systems were capable of supporting dual amplification, CRISPR reaction assays with about 2/3 the rate of the control assays performed in two separate, single-reaction optimized buffers. The total assay time varied with input titer copy number for HEPES, indicating that amplification was likely rate limiting for the HEPES buffer system. The total assay time was nearly invariant for the imidazole and Tris buffer systems, indicating that the DETECTR reaction was rate limiting for assays performed in those buffer systems. The phosphate buffer systems were able to support amplification, but failed to enable DETECTR reactions under the conditions tested.

### EXAMPLE 8

### Phosphate Buffer Optimization Amplification and CRISPR Reactions

This example describes the optimization of a buffer for use in amplification and CRISPR reactions. The solution and condition requirements for amplification and CRISPR reactions can be divergent, often necessitating the use of separate buffers for the two reactions. Using multiple buffers in a single assay increases the assay time and user input requirements, increases the chances for contamination and sample loss, and complicates the process of automating the assay.

Four separate buffers (outlined below in TABLE 11) were formulated with a range of buffer systems, potassium concentrations, and pH values. In addition to the constituents listed in TABLE 11, each buffer contained 5 mM Mg(OAc)2, 0.1% Tween-20 (v/v), and 1% glycerol (v/v). Each buffer was used in dual RT-LAMP, DETECTR assays with varying target nucleic acid input titers (1000 copies, 500 copies, 250 copies, or 0 copies (NTC)). The rates of the reactions were compared against RT-LAMP reactions performed in NEB IsoAmp Buffer (TABLE 3) and DETECTR reactions performed in MBuffer3 (TABLE 3). The reactions targeted the SARS-CoV-2 N-gene, and the DETECTR reactions utilized SEQ ID NO: 28 as a programmable nuclease.

**TABLE 11**

| FORMULATION NAME | BUFFER SYSTEM | PH | (NH₄)₂SO₄ (MM) | KOAC (MM) |
|---|---|---|---|---|
| DET 4 | Phosphate | 8 | 0 | 20 |
| DET 8 | Phosphate | 7.75 | 2 | 30 |
| DET 12 | Phosphate | 8.25 | 0 | 30 |
| DET 4 with Tris + Ammonium | Tris | 8 | 2 | 20 |

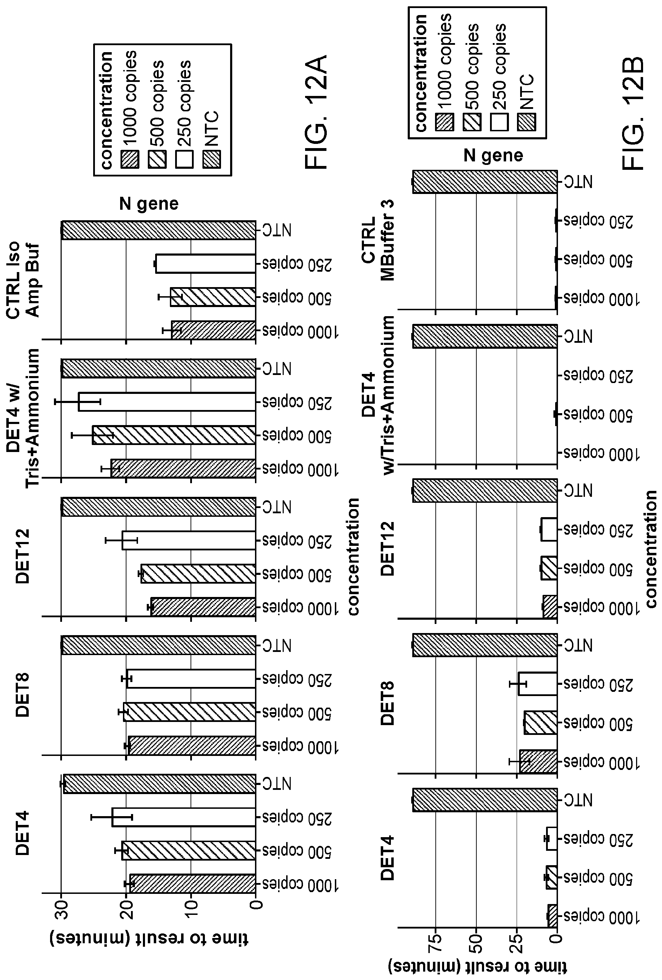

As can be seen in **FIG. 12** panel A, the three phosphate buffers all had comparable RT-LAMP reaction times, all of which were about 5 minutes slower than the RT-LAMP reactions performed in NEB IsoAmp Buffer. The DET 4 formulation with Tris buffer had the slowest RT-LAMP rates, requiring about twice as much time to complete RT-LAMP. However, as is shown in **FIG. 12** panel B, the DET 4 formulation with Tris buffer had the fastest DETECTR reaction rates. While the RT-LAMP times varied minimally between the three phosphate buffers tested, the DETECTR reaction rates supported by the three buffers varied considerably, with DET 8 supporting the slowest reactions and DET 4 supporting the fastest reactions. These results highlight the potential importance of high pH and low ammonium sulfate content for phosphate buffers used in DETECTR reactions.

### EXAMPLE 9

### Design of Experimentation Approach to Buffer Optimization

This example describes a multivariate optimization of a buffer for dual use in amplification and CRISPR reactions. Amplification and CRISPR reaction rates depend on solution composition and constituents. In many cases, a buffer optimized for use in an amplification or CRISPR reaction is unable to support the other type of reaction. Optimization of a buffer suitable for amplification and CRISPR reactions is a complex process, as the buffers for each type of reaction tend to be complex, and the effects imparted by individual constituents (e.g., a particular magnesium salt) can be affected by other components present in the buffer.

Thus, a design of experimentation approach was taken to separate and deconvolute the features necessary for supporting amplification and CRISPR reactions. RT-LAMP was performed at 62 °C in the buffers summarized in TABLE 6 with 0.2 µM forward and backward outer primers, 1.6 µM forward and backward inner primers, and 0.8 µM loop forward and loop backward primers. In addition to the buffer constituents listed in TABLE 6, each reaction contained 1.4 mM dNTP, 0.8 unit/µl NEB Bst 2.0 DNA polymerase; 1.4 mM dNTP, 0.56 unit/µL NEB Warmstart^{®} RTx reverse transcriptase, 0.8 unit/µl NEB Murine Rnase inhibitor, 1 µM Invitrogen SYT09 green fluorescent nucleic acid stain, and 4.5 mM magnesium sulfate (in addition to the amount of magnesium sulfate listed in TABLE 6). DETECTR was performed at 37 °C with SEQ ID NO: 28 in the buffers summarized in TABLE 7. Both reactions targeted SARS-Cov-2 N gene.

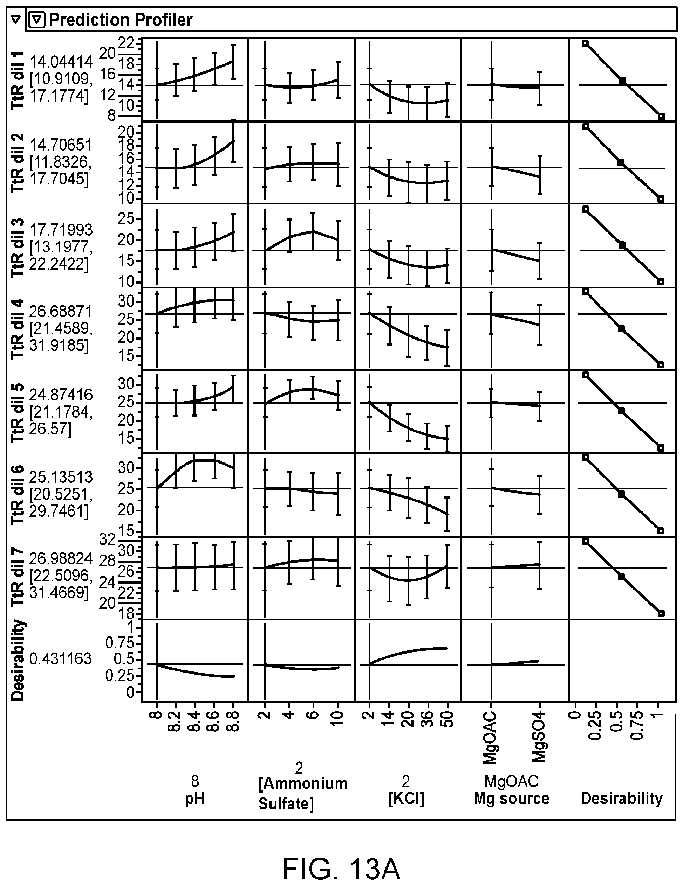

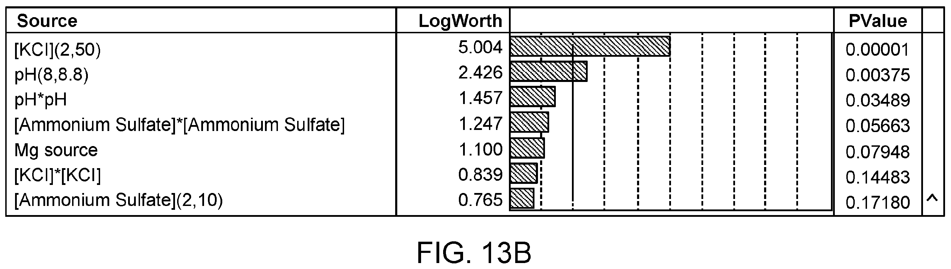

FIG. 13 shows the effect size and statistical significance of different buffer parameters on the rates of RT-LAMP reactions. Panel A provides a graphical view of the anticipated effect of each buffer component on RT-LAMP times. The effects were calibrated to desirability curves that assigned higher values to faster RT-LAMP reaction times. As can be seen in the bottom row (titled 'desirability), high pH, ammonium sulfate concentrations approaching 5 mM, high potassium chloride concentrations, and the use of magnesium sulfate as the magnesium source correlated with faster RT-LAMP reaction rates. As can be seen in panel B, potassium chloride concentration and pH were found to be statistically significant beyond a p-value threshold of 0.01.

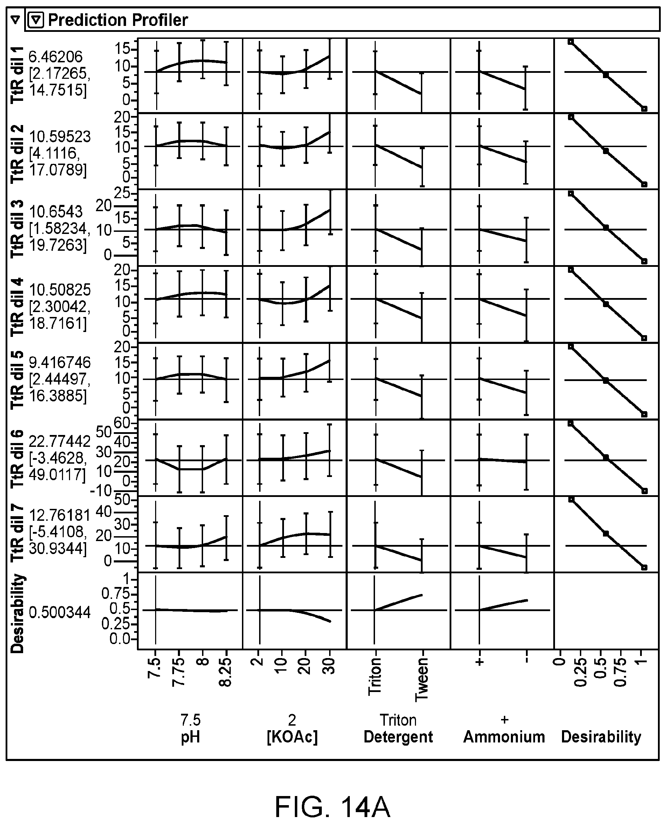

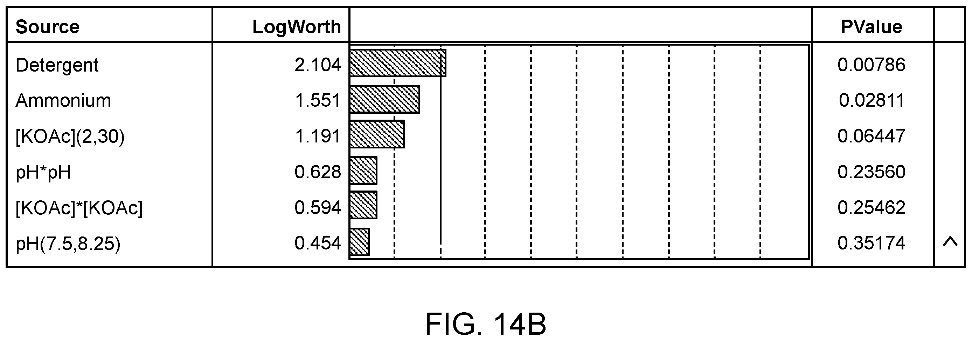

FIG. 14 shows the effect size and statistical significance of buffer parameters on the rates of DETECTR reactions. As can be seen in panel A, increased potassium acetate concentration, the presence of ammonium, and the use of Tween-20 (rather than Triton x-100) as detergent correlated with faster DETECTR reaction rates, while pH (over the 7.5-8.25 range tested) did not correlate with DETECTR reaction rate. As can be seen in panel B, only detergent type was found to have a statistically significant effect beyond a p-value threshold of 0.01.

### EXAMPLE 10

### Screening Buffers and Alternative Polymerases that Enable RT-LAMP to Work at Lower Temperatures (LowLAMP)

This example describes the effect of varying buffer parameters, such as higher amounts of Mg2+ and lower amounts of salt effect an RT-LAMP assay (compared to NEB IsoAmp). The following variations to the buffer compositions were tested:
- Isothermal Buffer "IB" 1 - potassium acetate instead of potassium chloride, magnesium acetate instead of magnesium sulfate
- IB2 - higher Mg2+
- IB3 - lower potassium acetate
- IB4 - with 0.25% PEG-8000

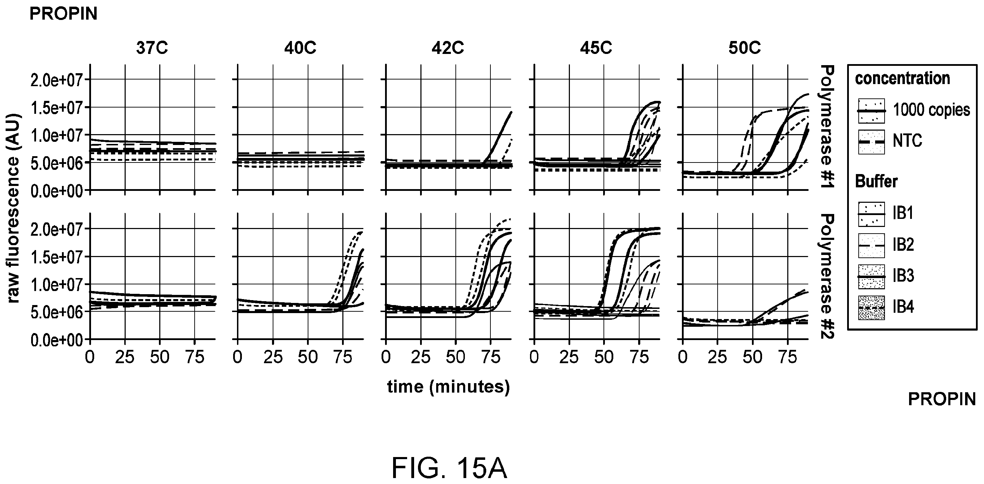

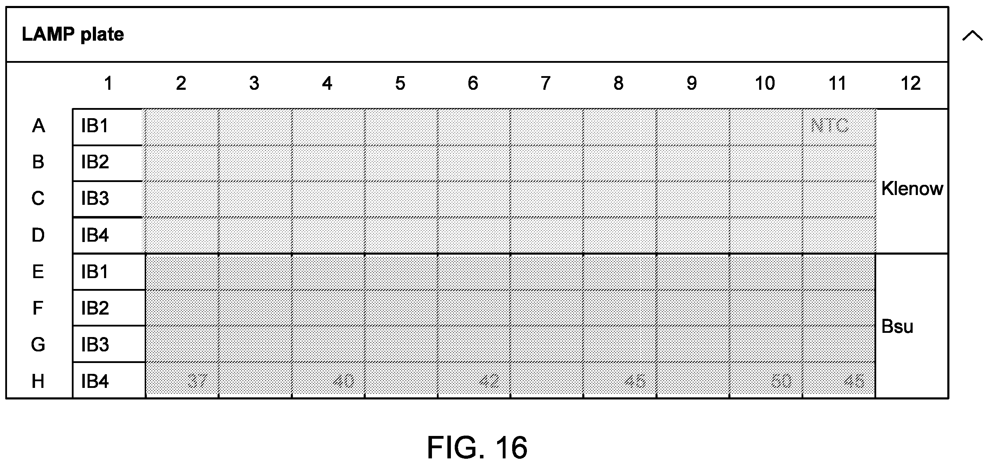

RT-LAMP was performed on the buffers listed above, with both Klenow and Bsu polymerases, the results of which are shown in FIG. 15A. LAMP mixtures were then prepared. 7 uL of the LAMP mixtures were aliquoted into wells of a 96 well plate as shown in FIG. 16 and 1 uL of isothermal buffer was pipetted into the wells of the 06 well plate, as shown in FIG. 16. 2 µL of target nucleic acid was added to each well at 500 copies/ µ L and 1000 copies/rxn. The reaction was run on a qPCR machine for 90 minutes at the following temperatures - 37, 40, 42, 45, 50, and 45 degrees Celsius.

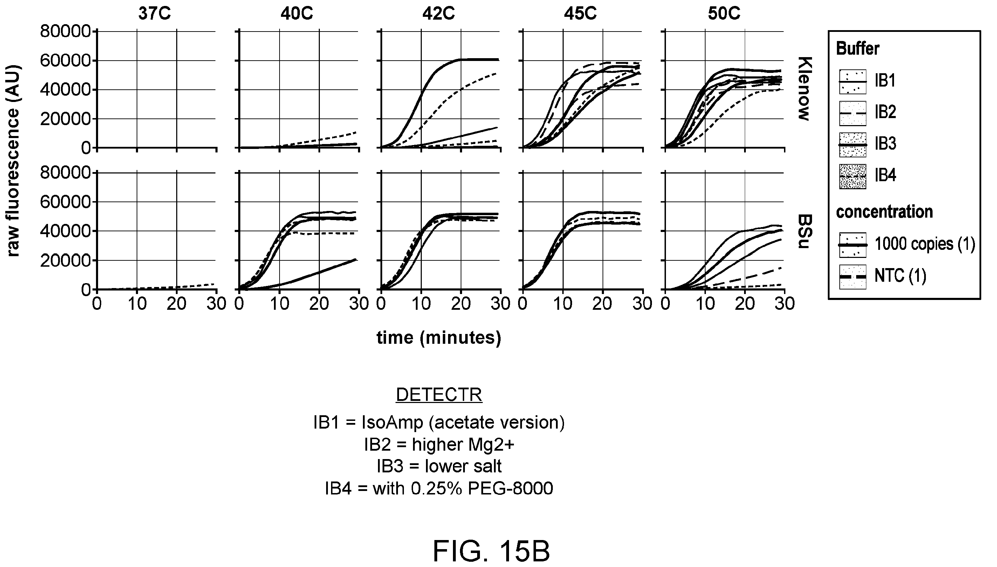

Next a DETECTR assay was performed, the results of which are shown in FIG. 15B. A complexing reaction was performed using the complexing mix comprising 40nM each of a Cas12 variant (SEQ ID NO:28) and a guide RNA. The reaction was incubated at 37C for 30 minutes, after which a reporter substrate was added. The RT-LAMP reactions were then added as follows: 14 µL of 1X MBuffer 3 was added in wells on a 384-well plate, after which 5 µL of complexing reaction was added to the wells. Finally, 1 µL of the LowLAMP product was added to the wells on a 384-well plate.

The plate was read on a plate reader with extended gain setting for 30 minutes at 37C. **FIGS. 15A** and **15B** show the RT-LAMP data and DETECTR data, respectively, for all four buffers with the target nucleic acid at 1000 copies and a control. For some embodiments, RT-LAMP can be performed at lower temperatures by using Klenow(exo-) or Bsu polymerases: LowLAMP. RT-LAMP is normally performed at temperatures between 55C-70C. Results can be seen in **FIG. 15A****.** This temperature range is influenced by the polymerase used. In standard RT-LAMP, a Bst or Bsm polymerase is typically used, which shows peak activity above 55C. In this embodiment, the performance of RT-LAMP at temperatures from 37C to 50C was evaluated with different reagents and conditions. Klenow(exo-) and Bsu polymerases were evaluated and functional RT-LAMP was demonstrated on an RNA target of SARS-CoV-2 N-gene at temperatures as low as 40C under the conditions tested. For this embodiment, the performance of these enzymes in four different buffers was tested and peak activity for Klenow(exo-) was observed at 50C, and peak activity for Bsu was at 45C under the conditions tested. For some embodiments, this method can be referred to as LowLAMP, as it functions at lower temperatures than standard RT-LAMP.

The RT-LowLAMP data further showed that the addition of 0.25% PEG-8000 to the buffer helps speed up amplification with the Bsu polymerase especially at the lower temperatures, under the conditions tested. Increasing Mg2+ appeared to reduce the overall reaction performance under the conditions tested while lowering the total salt content did not have much effect on overall reaction performance under the conditions tested.

### EXAMPLE 11

### Evaluating the Performance of Cas Variants on Putative Target Sites in SARS-CoV-2

This example demonstrates that Cas14a.1 (SEQ ID NO: 63) and Cas14a.3 (SEQ ID NO: 61) programmable nucleases may target and cleave distinct sequences from separate organisms. Six different DETECTR assays were performed with either Cas14a.1 (SEQ ID NO: 63) or Cas14a.3, and with one of three following three targets: (1) the N-gene of SARS-CoV-2; (2) a sequence from the Mammuthus primigenius mitochondrial DNA; and (3) POP7, a target site in the human gene POP7 (RNase P). Guide nucleic acids were provided in the form of separate crRNA and tracrRNA molecules.

Ribonucleoproteins (RNPs, e.g. programmable nuclease complexes) were prepared by complexing 40 nM of a programmable nuclease with equimolar equivalents of crRNA targeting one of the three target sequences and one of two different tracrRNAs. The programmable nucleases, crRNA, and tracrRNA were incubated at 37 °C for 30 minutes in nuclease-free water comprising pH 7.2 phosphate and EDTA buffered saline and bovine serum albumin. The resulting ribonucleoproteins were then mixed with 100 µM of a reporter nucleic acid and transferred to ice for storage.

Assays were performed by mixing 5 µL of the ribonucleoproteins with 2 µL of 10 nM target nucleic acid and 13 µL pH 7.2 phosphate EDTA buffer in wells of a 96-well plate incubated over ice. Control reactions were prepared by instead mixing 5 µL of the ribonucleoproteins with 15 µL pH 7.2 phosphate EDTA buffer and no target nucleic acid. The plates were sealed and spun at 2000 rcf for 30 seconds, and then incubated at 50 °C over 90 minutes fluorescence readouts of the DETECTR reactions.

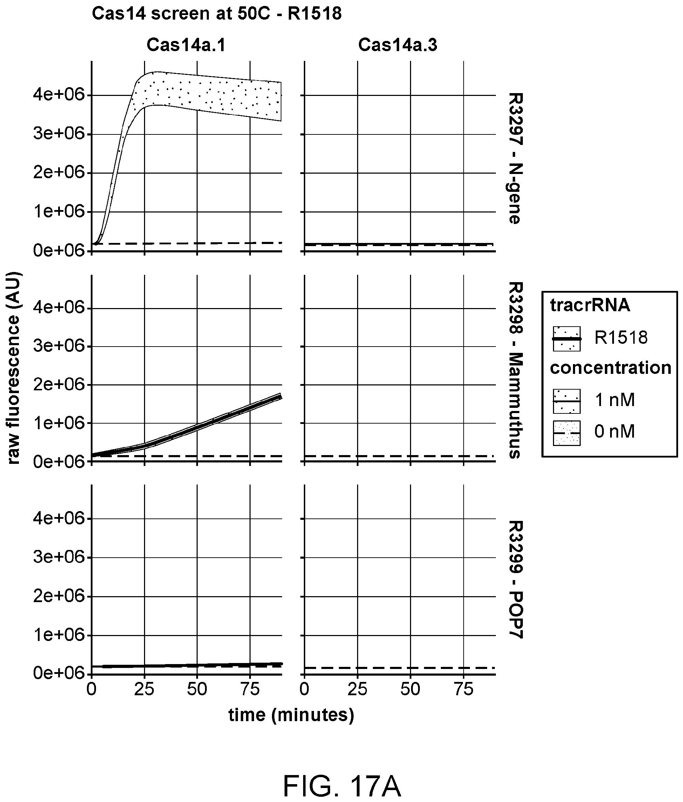

FIG. 17A provides results for the DETECTR reactions with a first of the tracrRNAs. As can be seen from the figure, Cas14a.3 (SEQ ID NO: 61)
was inactive in the presence of all three target sequences. Cas14a.1 (SEQ ID NO: 63) had the highest activity in the presence of the SARS CoV-2 N-gene, with complete reporter cleavage in around 25 minutes. Cas14a.1 exhibited moderate activity in the presence of the Mammuthus gene, and no activity in the presence of Human RNAse PoP P 7 under the conditions tested. The controls performed in the absence of target nucleic acid confirmed negligible resting transcollateral cleavage activity for Cas14a.1 and Cas14a.3 (SEQ ID NO: 61).

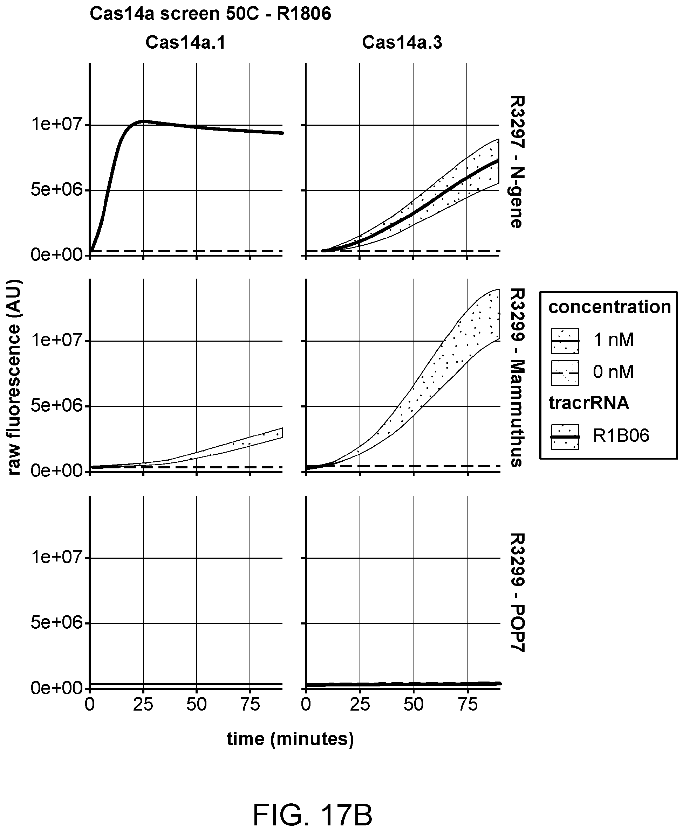

FIG. 17B provides results for the DETECTR reactions with the second of the tracrRNAs. The second tracrRNA imparted activity for Cas14a.1 (SEQ ID NO: 63) and Cas14a.3 (SEQ ID NO: 61). Cas14a.1 and Cas14a.3 exhibited activity in the presence of the target nucleic acid SARS CoV-2 N-gene and the Mammuthus gene, with Cas14a.1 (SEQ ID NO: 63) exhibiting faster kinetics in the presence of the SARS CoV-2 N-gene and Cas14a.3 exhibiting faster kinetics in the presence of the Mammuthus gene. Neither programmable nuclease exhibited activity in the presence of Human RNAse PoP P 7.

### EXAMPLE 12

### HotPot: Integrating LAMP amplification with Cas14a.1 DETECTR in a single reaction (one-pot) volume

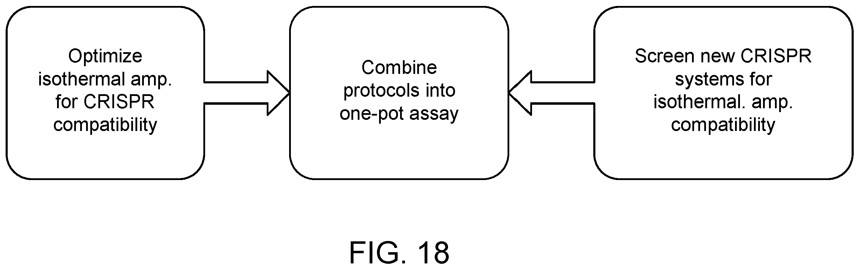

This example describes one approach to combining isothermal amplification protocols described herein with new CRISPR systems to create one-pot DETECTR and other assays, as outlined by the strategy in **FIG. 18****.** In particular, this example demonstrates that Cas14a.1 **(SEQ ID NO:** 63) programmable nucleases are compatible with LowLAMP conditions such that one may amplify a and detect target sequences in a one-pot reaction using these techniques. To develop a one-pot Cas14a.1 LowLAMP protocol (HotPot), various DETECTR assays were performed to determine feasible one-pot conditions, as described herein. As used herein, "HotPot" refers to a one-pot reaction in which both amplification (e.g., LowLAMP) and detection (e.g., DETECTR) reactions occur simultaneously. In many embodiments, a HotPot reaction may utilize a thermostable programmable nuclease which exhibits trans cleavage activity at elevated temperatures (e.g., greater than 37C).

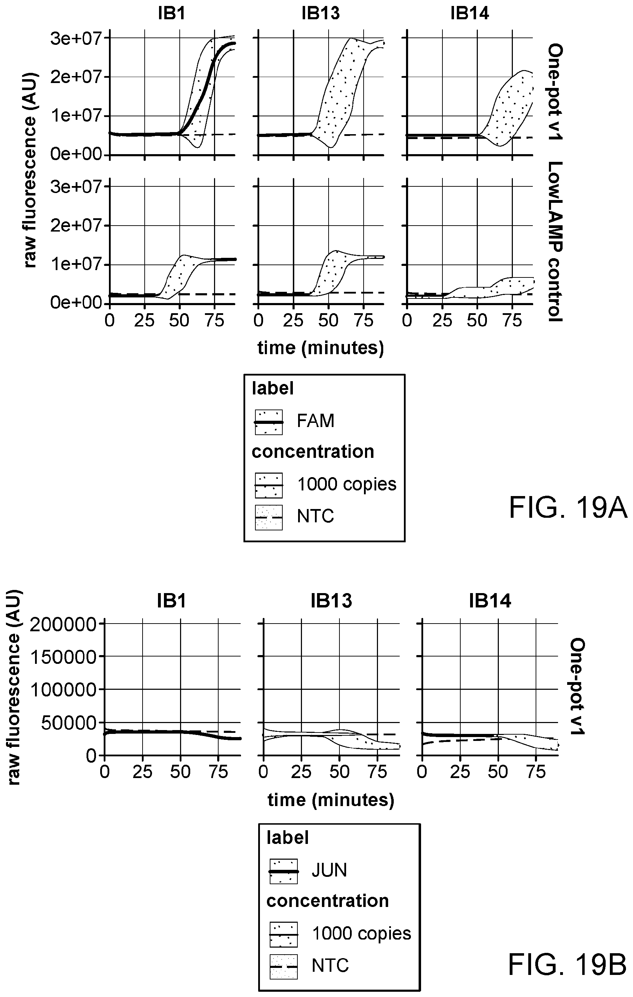

FIG. 19A-19B presents results for an embodiment of HotPot involving LAMP amplification with Cas14a.1 (SEQ ID NO: 63) DETECTR in single reaction volume (one-pot). As shown in the figure, Cas14a.1 failed to function in one-pot LAMP conditions. Specifically, Cas14a.1 was tested to see if it can function in a one-pot reaction using LowLAMP at 50C using Klenow(exo-) as the DNA polymerase. FIG. 19A shows the signal from SYTO9, a DNA binding dye, that indicates the production of DNA by LAMP. It was confirmed that LowLAMP was able to generate DNA amplicon in the presence of Cas14 in three different buffers. FIG. 19B shows the signal from the fluorophore-quencher (FQ) reporter in the one-pot reaction condition shown in FIG. 19A. No signal was detected in the one-pot reaction, even though DNA was generated. The results of this method suggest that Cas14 was inhibited in the LAMP reaction under the conditions tested.

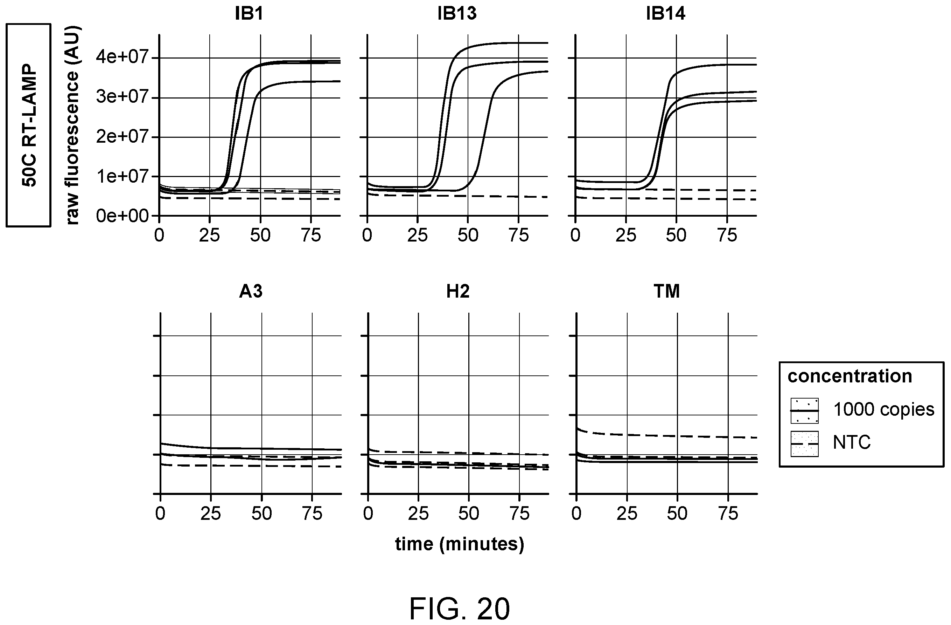

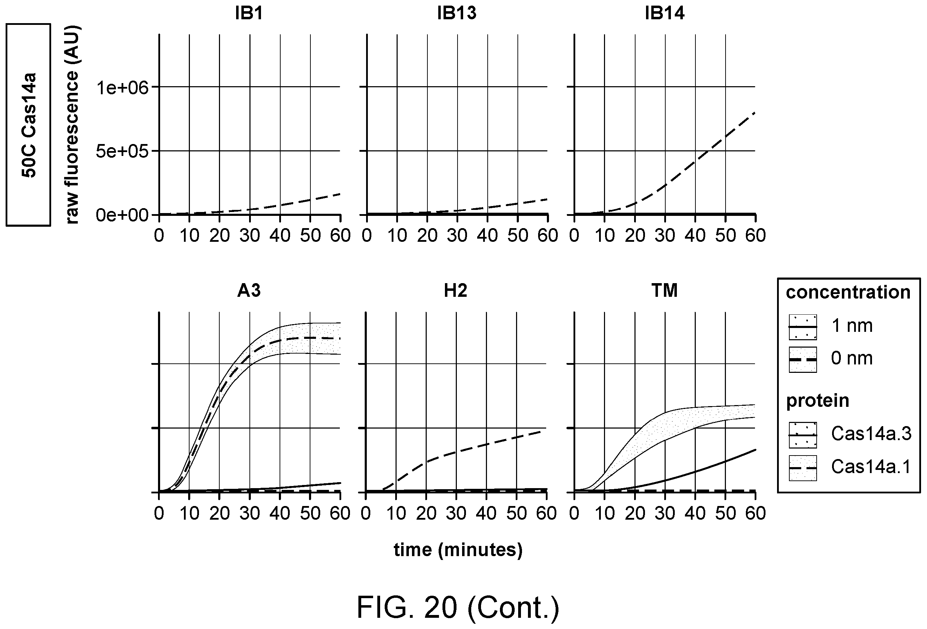

FIG. 20 presents the results from screening various buffers for HotPot, Cas14a.1 (SEQ ID NO: 63) and LowLAMP, compatibility. LowLAMP and Cas14a.1 were found to be optimally functional in different buffers. The performance of LowLAMP at 50C in buffers that are optimal for LowLAMP (IB1, IB13, IB14) and buffers that are optimal for Cas14a.1 (A3, H2, TM) was evaluated as well as the performance of Cas14a.1 in the same buffers at 50C. Results in FIG. 20 show that both LowLAMP and Cas14a.1 were functional in the LowLAMP optimal buffers, but Cas14a.1 was most compatible with buffer IB14. This work identified a buffer condition that was compatible with both isothermal amplification and Cas14a.1 DETECTR, a key step in moving towards a one-pot reaction.

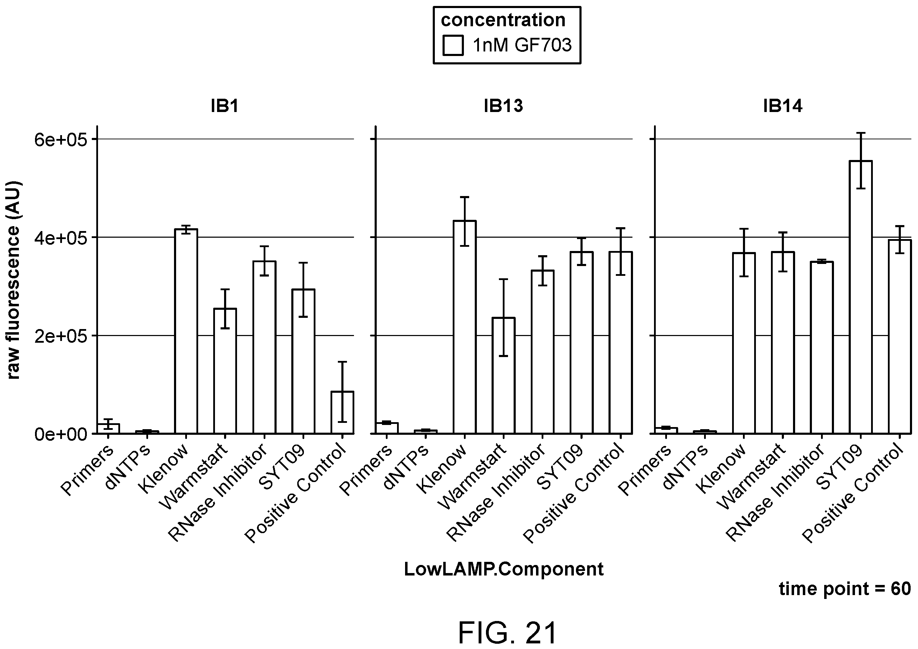

FIG. 21 shows that primers and dNTPs were found to have the greatest inhibitory effect on Cas14 performance in one-pot reactions. The impact of individual components of LowLAMP on the performance of Cas14 were tested to determine which components might be responsible for the inhibition seen when attempting one-pot reactions. Each component was added to the Cas14a.1 (SEQ ID NO: 63) reaction with 1 nM of target dsDNA and the reaction was run for 60 minutes at 50C. The results indicate that primers and dNTPs contribute to the inhibition of LAMP. Therefore, it was necessary to investigate whether lower concentrations of these components could be tolerated in LowLAMP.

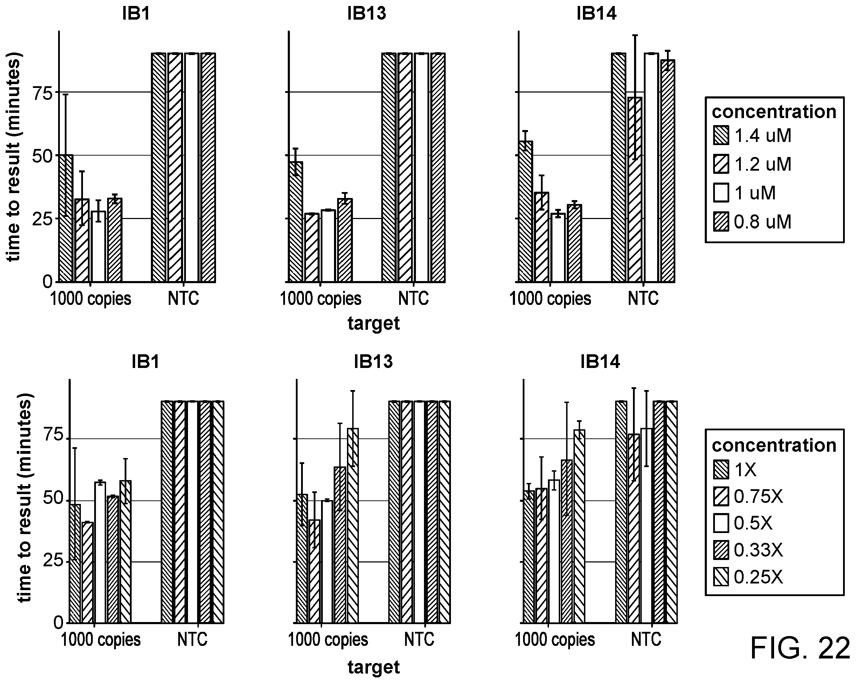

In some embodiments, LAMP functions with lower concentrations of dNTPs and primers. FIG. 22 shows that LowLAMP functions with lower concentrations of dNTPs and primers as compared to other methods described herein (e.g., Examples 1-11, etc.). The performance of lower concentrations of dNTPs and primers was tested to see the impact on the performance of LowLAMP using Klenow(exo-) at 50C. For this embodiment, results show a time-to-result where a lower value indicates faster amplification. (a) Titration of dNTPs shows that LowLAMP functions down to 0.8 mM dNTPs. The performance of the assay was improved at 1.2 mM and less versus the standard 1.4 mM. (b) Titration of primers in LowLAMP shows that going below 0.5X of primers can have an inhibitory effect. Together these results suggest that the dNTPs and primers concentrations can be reduced without negatively impacting LowLAMP performance, which was investigated to help relieve the observed inhibition of Cas14.

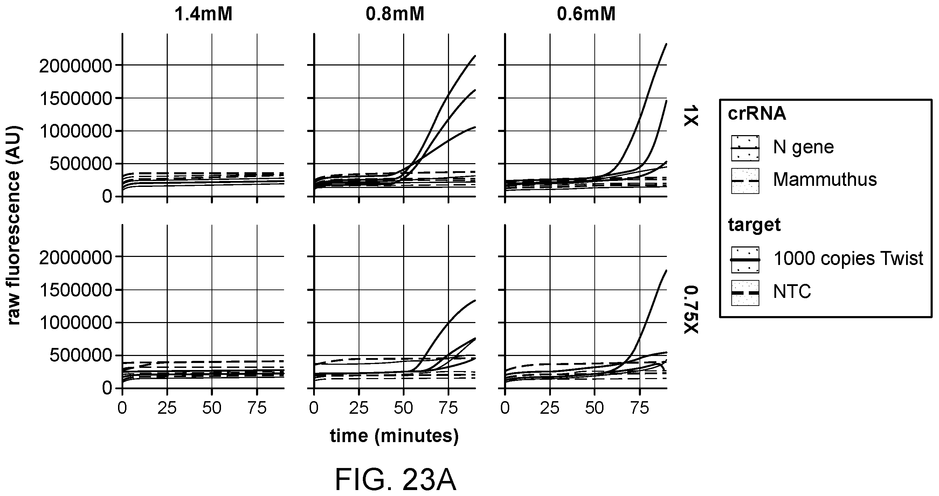

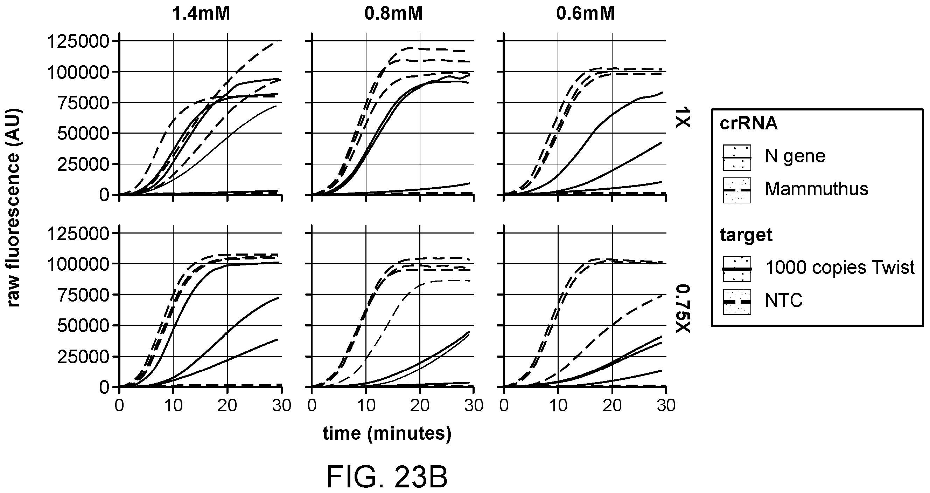

The results of the primer and dNTPs titrations were applied to the one-pot Cas14a.1 (SEQ ID NO: 63) DETECTR and LowLAMP reaction. In some embodiments, one-pot Cas14a.1 DETECTR with LowLAMP was tested at 50C. For such embodiments, one-pot DETECTR using Cas14 and LowLAMP at 50C using a Klenow(exo-) DNA polymerase were tested at lower primer and dNTP concentrations. As demonstrated in FIGS. 23A-23B, one-pot DETECTR using Cas14a.1 (SEQ ID NO: 63) and LowLAMP at 50C using a Klenow(exo-) DNA polymerase was shown to be functional under lower primer and dNTPs conditions. Other examples have suggested that Cas14a.1 may be inhibited at the standard 1.4 mM and 1X primer concentration. FIG. 23A shows signal from Cas14a.1 (SEQ ID NO: 63) one-pot. One-pot DETECTR reactions using a Cas14 were either complexed with a SARS-CoV-2 N-gene crRNA that targeted the amplicon generated by LowLAMP, or complexed with a non-target crRNA that targeted a gene in the Mammuthus primigenius mtDNA. Results from this embodiment, showed a signal only in the reaction that were run with target RNA and the crRNA targeting the amplicon. No signal was seen when there was no target present, or the Cas14a.1 crRNA was targeting the Mammuthus gene FIG. 23B shows the generation of the target of interest by LAMP, amplicons generated in the one-pot reaction were run in a Cas12 DETECTR reaction using a SARS-CoV-2 N-gene crRNA to confirm the results in FIG. 23A. These results demonstrated that the amplicon was generated when the target RNA was present. Furthermore, the results indicate that the one-pot reaction is functional at 0.8 and 0.6 mM dNTPS, but not at 1.4 mM dNTPs at least in part due to Cas14a.1 inhibition at this concentration of dNTPs under the conditions tested.

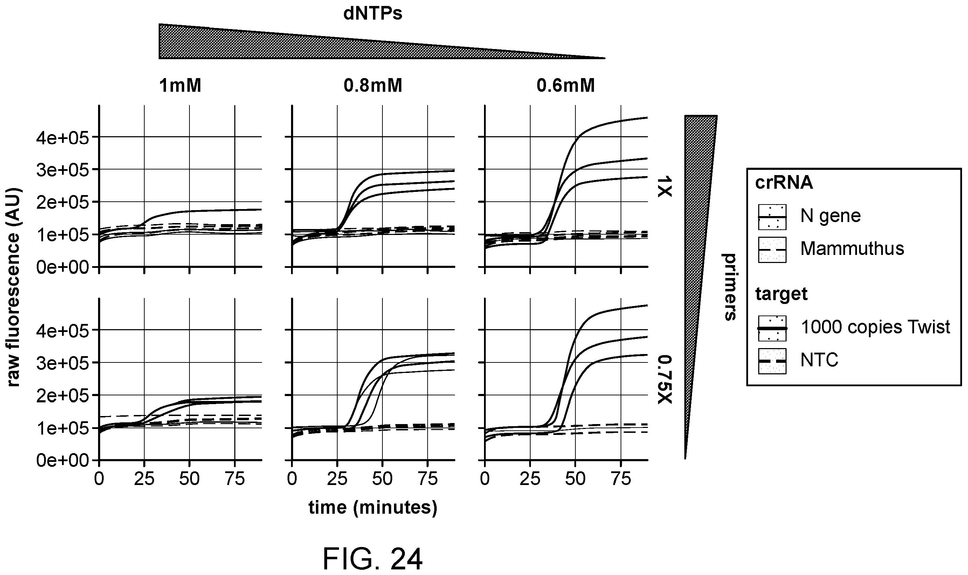

In some embodiments, Cas14a.1, or another thermostable Cas effector protein, may be used with a polymerase (e.g., a Bsm DNA polymerase, a Bst DNA polymerase, a Klenow(exo-) DNA polymerase, or a Bsu DNA polymerase) (55°C) for the HotPot assay. The one-pot reaction may be faster if the reaction temperature is increased from 50°C to 55°C. However, the DNA polymerase used in LowLAMP (Klenow(exo-)) was not functional at 55°C, so here Bsm DNA polymerase was used, which worked more robustly at 55°C than other LAMP polymerases tested such as Bst. Several different concentrations of dNTPs and primers were tested and performance of the assay was assessed. Results shown in FIG. 24 indicate that a signal was generated by Cas14a.1 (SEQ ID NO: 63) when Cas14a.1 had the crRNA that matched the target amplicon (SARS-Cov-2 N-gene) and when the target RNA was present in the reaction. The speed of the reaction was fastest with 1 mM dNTPs, but the overall signal was lower than when 0.8 and 0.6mM dNTPs were used.

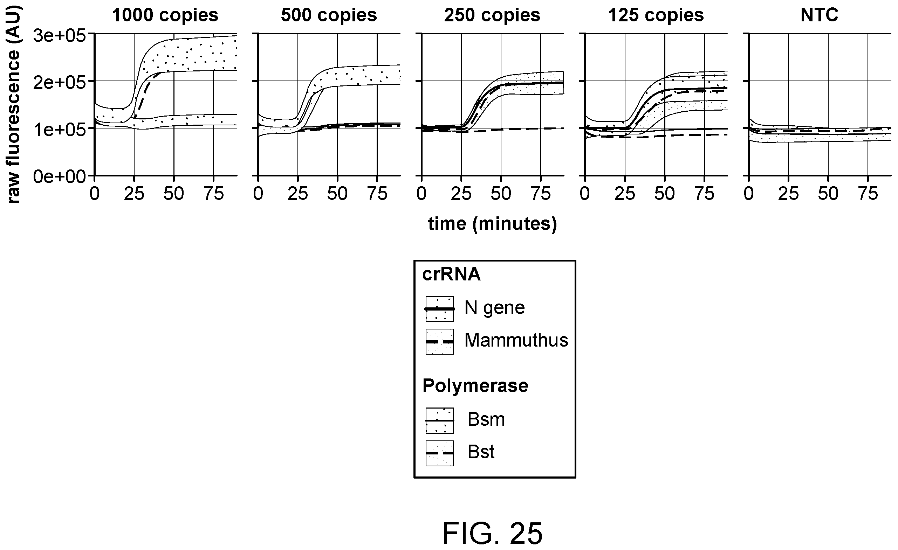

The initial performance of the one-pot DETECTR reaction, called HotPot, was then evaluated, as demonstrated in FIG. 25. In this experiment, a limit of detection experiment for two different DNA polymerases at 55C was performed. Cas14a.1 crRNAs (SEQ ID NO: 63) targeting either the SARS-CoV-2 N-gene or an off-target gene were included in the experiment. The assay at 1000, 500, 250, or 125 input copies/rxn of SARS-CoV-2 RNA genome was tested. Robust performance of the assay at the lowest tested concentration of 125 copies/rxn for 3/3 replicates is shown in FIG. 25.

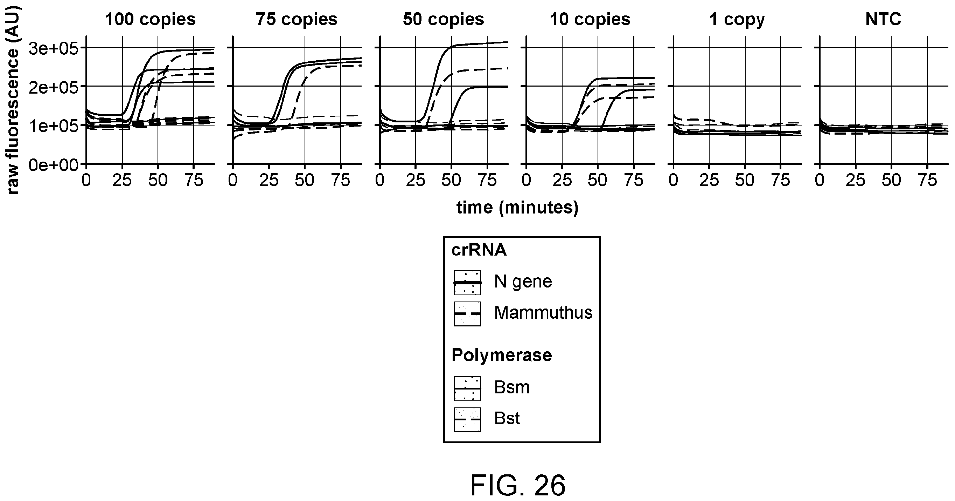

Limit of detection experiments were performed using two different DNA polymerases at 55°C as shown in FIG. 26. Cas14a.1 crRNAs (SEQ ID NO: 63) targeting either the SARS-CoV-2 N-gene or an off-target gene were included in the experiments and the assay at 100, 75, 50, 10, or 1 input copies/rxn of SARS-CoV-2 RNA genomes were tested. Robust performance of the assay at 100 copies/rxn for 3/3 replicates was observed. At 75 copies and below several replicates did not show up, but detection down to 10 copies/rxn for 2/3 replicates was observed

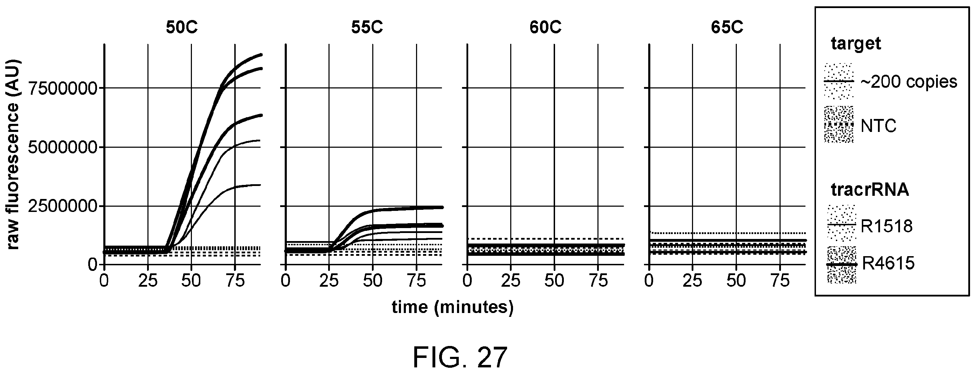

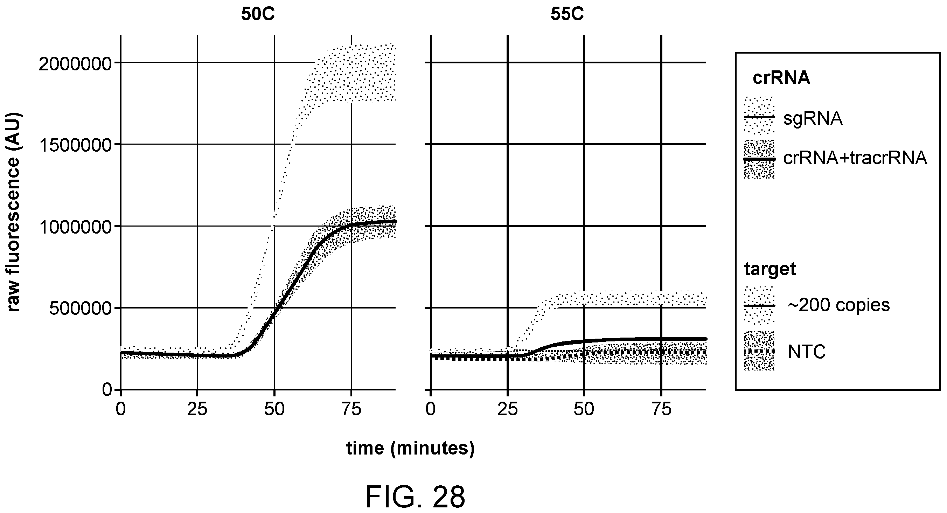

The effect of different tracrRNA and sgRNA on one-pot DETECR performance were investigated. As shown in FIG. 27, a new tracrRNA R4614 was designed to be more thermostable than the native tracrRNA (R1518) for Cas14a.1 (SEQ ID NO: 63), for one-pot DETECTR uses. R4614 was used in the improved conditions previously described herein while modifying temperature to investigate whether a more thermostable tracrRNA would allow for higher reaction temperature conditions. As shown in FIG. 27, R4614 performed better than the native tracrRNA at 50C and 55C. Neither tracrRNA enabled a signal from either the 60C or 65C conditions. Similarly, a sgRNA was tested against the use of native tracrRNA and crRNA two RNA component system for Cas14a.1 (SEQ ID NO: 63) in a one-pot DETECTR reaction, as shown in FIG. 28. From this analysis, we observed that the sgRNA outperformed the two RNA component system at both 50C and 55C. These results suggest that sgRNA and thermostable non-native tracrRNAs can improve Cas14a.1 (SEQ ID NO: 63) performance in DETECR assays under the conditions tested.

### EXAMPLE 13

### One-pot NECTR: NEAR amplification + Cas14a.1 DETECTR in single reaction volume

This example describes one approach to combining isothermal amplification protocols described herein with new CRISPR systems to create one-pot DETECTR and other assays, as outlined by the strategy in FIG. 18. In particular, this example demonstrates that Cas14a.1 programmable nucleases are compatible with NEAR conditions such that one may amplify a and detect target sequences in a one-pot reaction using these techniques. To develop a one-pot Cas14a.1 NEAR protocol (NECTR), various DETECTR assays were performed to determine feasible one-pot conditions, as described herein.

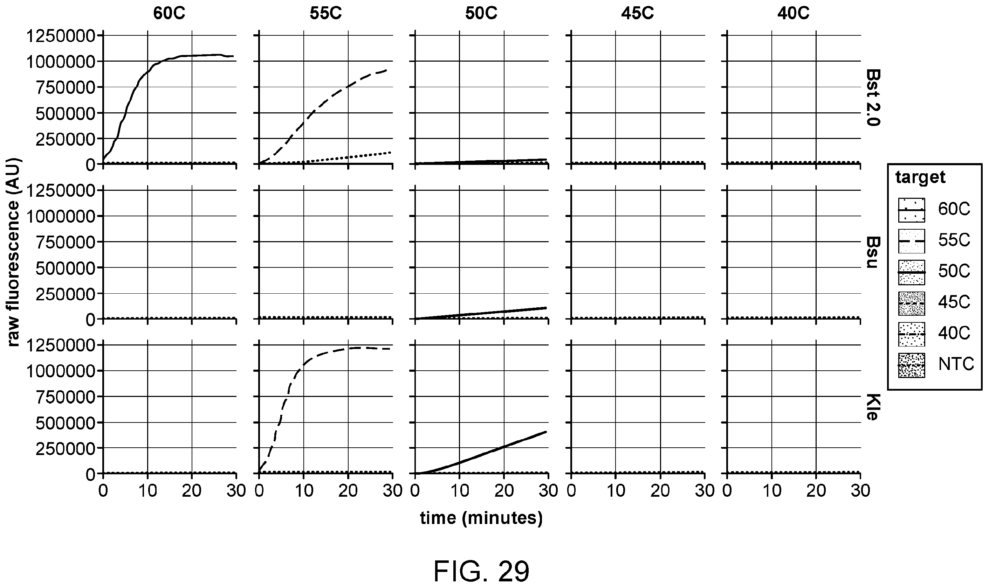

For some embodiments, replacing Bst polymerase in NEAR can enable SARS-CoV-2 detection at lower temperatures as shown in FIG. 29. Many NEAR protocol use Bst 2.0 to generate an amplicon from SARS-CoV-2 at 60C. This polymerase also functions at 55C, but at reduced capacity. Accordingly, the performance of Bsu and Klenow(-exo) polymerases at lower temperatures was evaluated for these embodiments. It was found that Klenow(-exo) polymerase enabled robust amplification at 55C and 50C. FIG. 29 shows a Cas14a.1 DETECTR reaction following a NEAR amplification reaction. Amplification reactions were performed at the indicated temperature for 10 minutes in a buffer that was composed of a mixture of 1X IsoAmp buffer + 0.5X CutSmart buffer. It was observed in this embodiment that Cas14a.1 shows peak activity at 55C and 50C, but not at 60C.

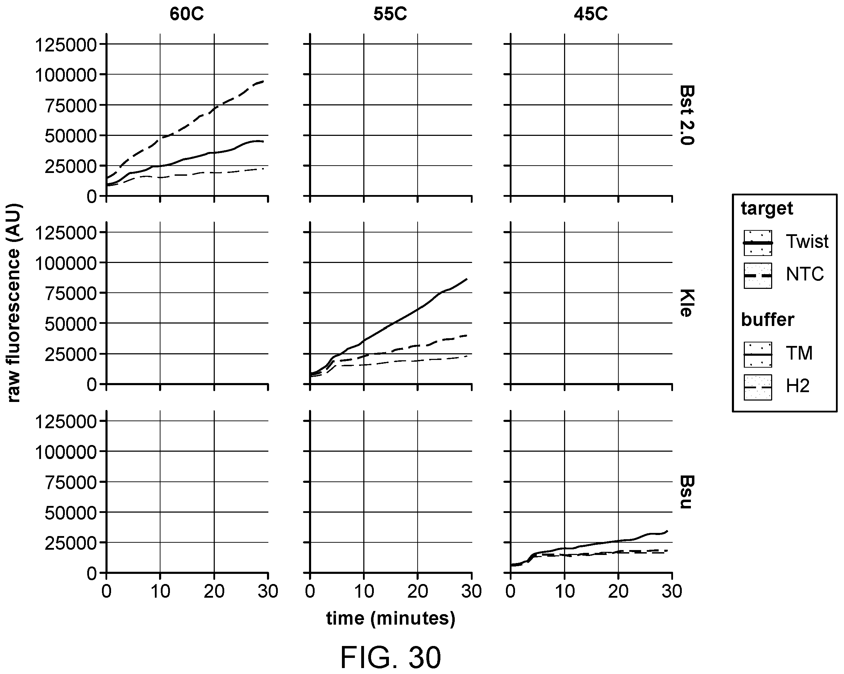

For some embodiments, NEAR amplification functions in Cas14a.1 optimal buffers as shown in FIG. 30. The optimal buffer for Cas14a.1 has less salt and higher Mg2+ than the optimal buffer for NEAR amplification. Therefore, the performance of NEAR amplification in two buffers for Cas14a: 1X TM Buffer and 1X H2 Buffer was investigated. Amplification reactions were performed with the indicated polymerase, buffer, and reaction temperature with Nt.BstNBI, Omniscript RT, M2805 FWD, M2811 REV. FIG. 30 shows the result of a Cas14a.1 DETECTR reaction to evaluate the amount of amplicon produced. For this embodiment, results indicate that TM Buffer and H2 Buffer may function for NEAR amplification. However, results suggest that the reactions produced ~10 times less amplicon than an optimal NEAR reaction. This suggests room for further buffer optimization. 1X TM Buffer used in the experiments contained: 20 mM Tricine, pH 8.5; 2 mM KOAc; 100 µg/mL BSA; 15 mM MgOAc. 1X H2 Buffer used in the experiments contained: 20 mM Tris-HCl, pH 8.8; 2 mM KOAc; 0.1% Tween-20; 17.5 mM MgOAc.

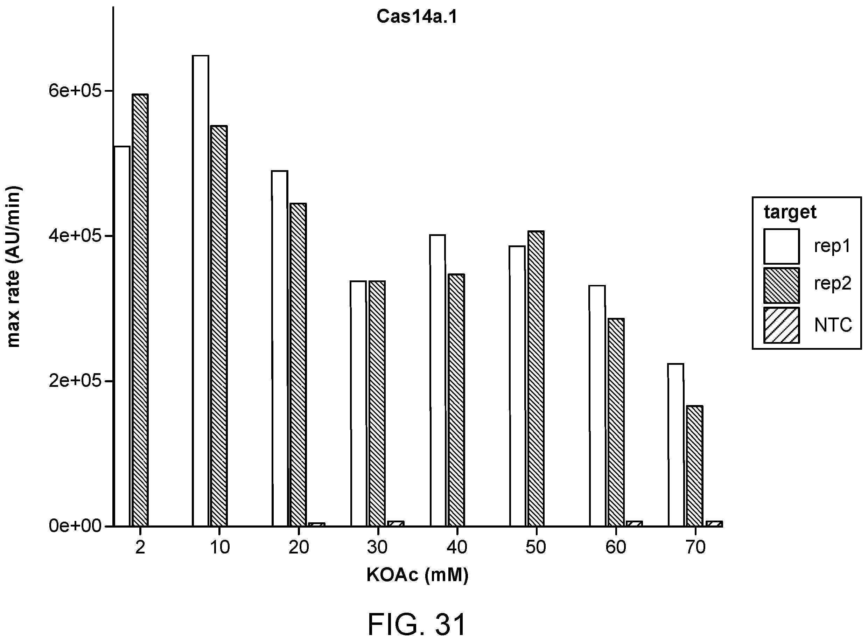

Cas14a.1 functioned in a range of KOAc salt concentrations as shown in FIG. 31. In some embodiments, Cas14a.1 reaction buffer (H2 Buffer) contains 2 mM KOAc. In some embodiments, amplification reactions often require higher concentrations for optimal efficiency. In some embodiments, the performance of a Cas14a.1 DETECTR reaction in increasing concentrations of KOAc was investigated. In some embodiments, Cas14a.1 DETECTR reaction was evaluated with tracrRNA R1518, crRNA R2424, at a target concentration of 1 nM, and a reaction temperature of 50C. Results shown in FIG. 31 indicate that Cas14a.1 is maximally active at 2-10 mM KOAc, but remains robustly active up to 60 mM. At 70 mM KOAc performance of Cas14a.1 begins to drop off. In the experiments 1X H2 Buffer was composed of: 20 mM Tris-HCl; pH 8.8; 2 mM KOAc; 0.1% Tween-20; and 17.5 mM MgOAc.

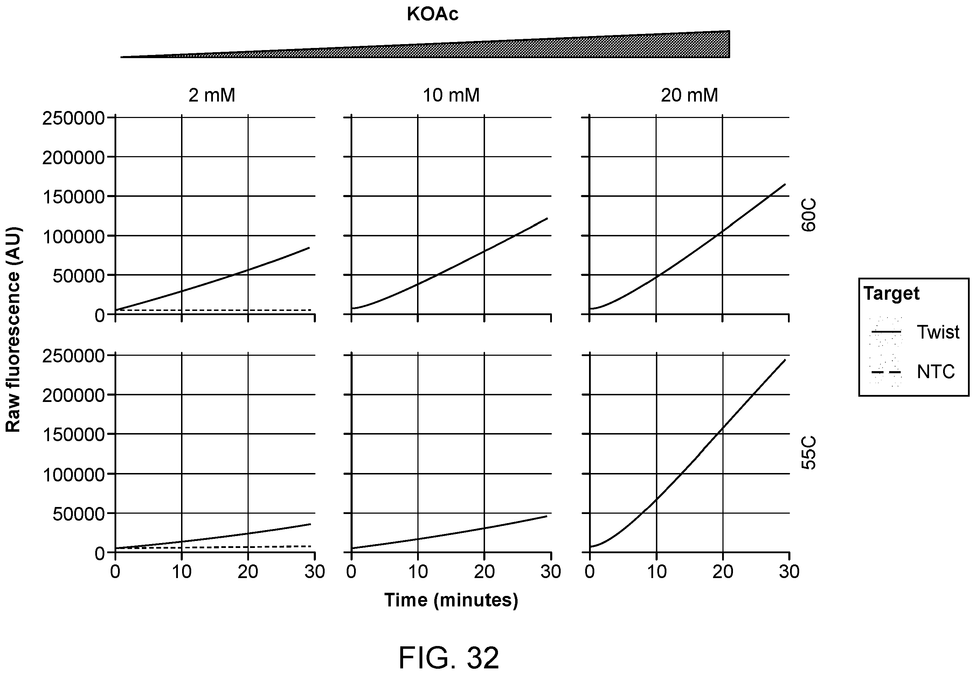

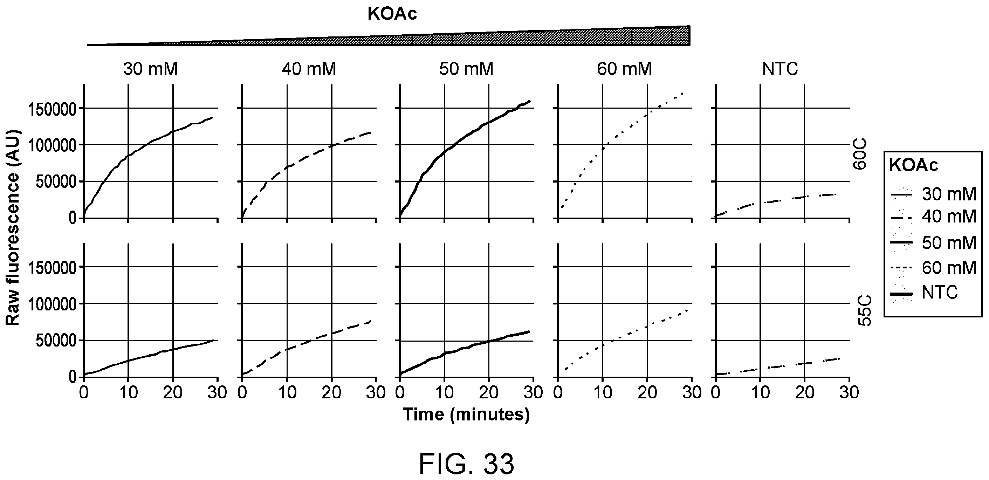

In some embodiments, increasing concentrations of KOAc may improve NEAR performance in Cas14a.1 optimal buffers, as seen in FIG. 32. NEAR amplification in Cas14a.1 optimal reaction buffers was initially seen to be less efficient than when amplification is performed in the optimal NEAR buffer (1X IsoAmp + 0.5X CutSmart). The impact of increasing amounts of KOAc on the performance of NEAR amplification in the background of a Cas14a.1 optimal amplification buffer (H2 Buffer) was investigated. As seen in FIG. 32 results show how Cas14a.1 DETECTR assay performed after NEAR amplification to measure amplification efficiency. Results indicate that increasing KOAc to up to 60 mM improved the amount of NEAR amplicon generated under the conditions tested. From 2 mM to 60 mM KOAc were functional ranges for Cas14a.1 under the conditions tested. In these experiments 1X H2 Buffer contained: 20 mM Tris-HCl, pH 8.8; 2 mM KOAc; 0.1% Tween-20; 17.5 mM MgOAc. In some embodiments, it was also shown that increasing concentrations of KOAc improved NEAR performance in Cas14a.1 optimal buffers as seen in FIG. 33.

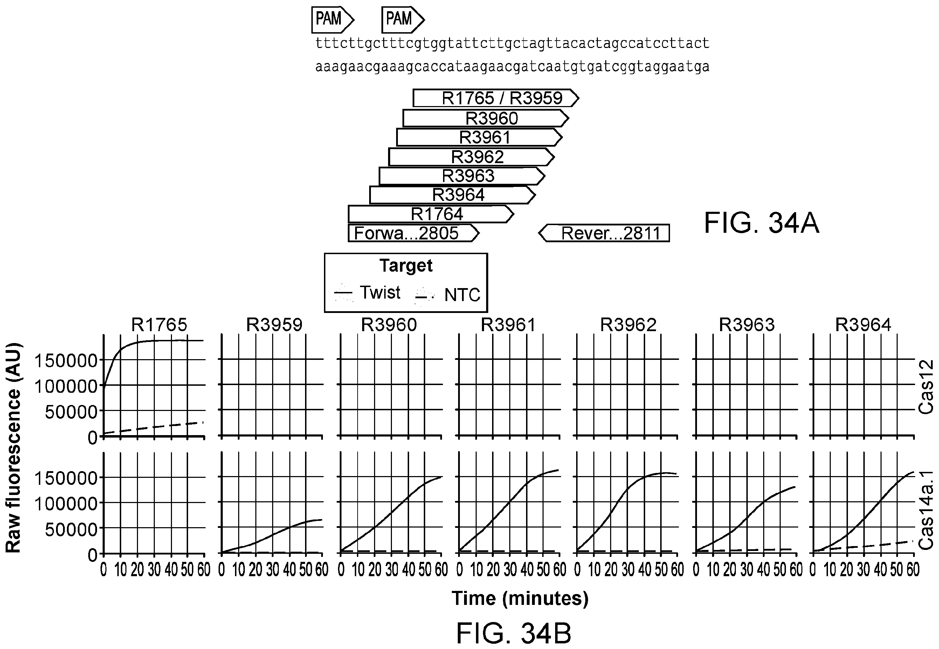

Performance of Cas14a.1 crRNAs on SARS-CoV-2 E-gene amplicon are shown in FIGS. 34A-34B. In some embodiments, Cas14a.1 crRNAs, were designed to target the SARS-CoV-2 E-gene NEAR amplicon generated by primers M2805/M2811. Results are shown in FIG. 34. FIG. 34A shows the position of the crRNAs and primers. R1765 and R1764 are Cas14a.1 crRNAs that target the amplicon. PAM sequences for Cas14a.1 are indicated, but there are no TTTR PAMs for Cas14a.1 to use within this amplicon. FIG. 34B shows the performance of Cas14a.1 crRNAs on NEAR amplicon (generated at 60C for 15 minutes). All tested crRNAs worked robustly with the least amount of background from R3960, R3961, and R3962.

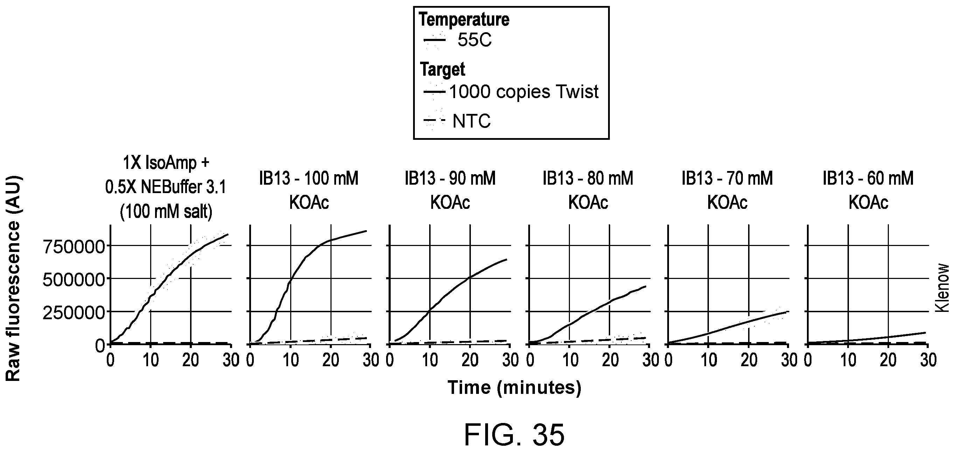

In some embodiments, the performance of Klenow(exo-) NEAR assay in IB13 buffer at decreasing salt concentrations was evaluated as shown in FIG. 35. In these embodiments, Standard NEAR reaction buffer was composed of a mixture of 1X IsoAmp buffer and 0.5X NEBuffer 3.1, which gave a final salt concentration of 100 mM. Variations of the IB13 buffer, with various concentrations of KOAc as the salt, were tested. In these buffer variations the NEAR assay using Klenow(exo-) as the polymerase was run at 55C for 20 minutes. To readout the amount of amplicon produced, a Cas14a.1 DETECTR assay on 2 µL of the NEAR amplicon was performed. The results indicate that the best NEAR performance was with 100 mM KOAc, similar to the standard NEAR reaction buffer, and that the amount of amplicon produced was reduced as the salt is reduced. The assay has acceptable performance at 80 - 70 mM KOAc. In these experiments, the IB13 buffer had a composition of: 20 mM Tris-HCl, pH 8.8; 10 mM (NH4)2SO4; 50 mM KOAc; 5 mM MgOAc; 1% Tween-20; 1 mg/mL BSA.

### EXAMPLE 14

### One-pot sRCA: Rolling circle amplification with Cas14a.1, a Cas12 Variant, or a Cas13 DETECTR reaction in single reaction volume

This example describes one approach to combining isothermal amplification protocols described herein with new CRISPR systems to create one-pot DETECTR and other assays, as outlined by the strategy in FIG. 18. In particular, this example demonstrates that Cas14a.1 programmable nucleases are compatible with RCA conditions such that one may amplify a and detect target sequences in a one-pot reaction using these techniques. To develop a one-pot Cas14a.1 RCA protocol (One-pot sRCA), various DETECTR assays were performed to determine feasible one-pot conditions, as described herein.

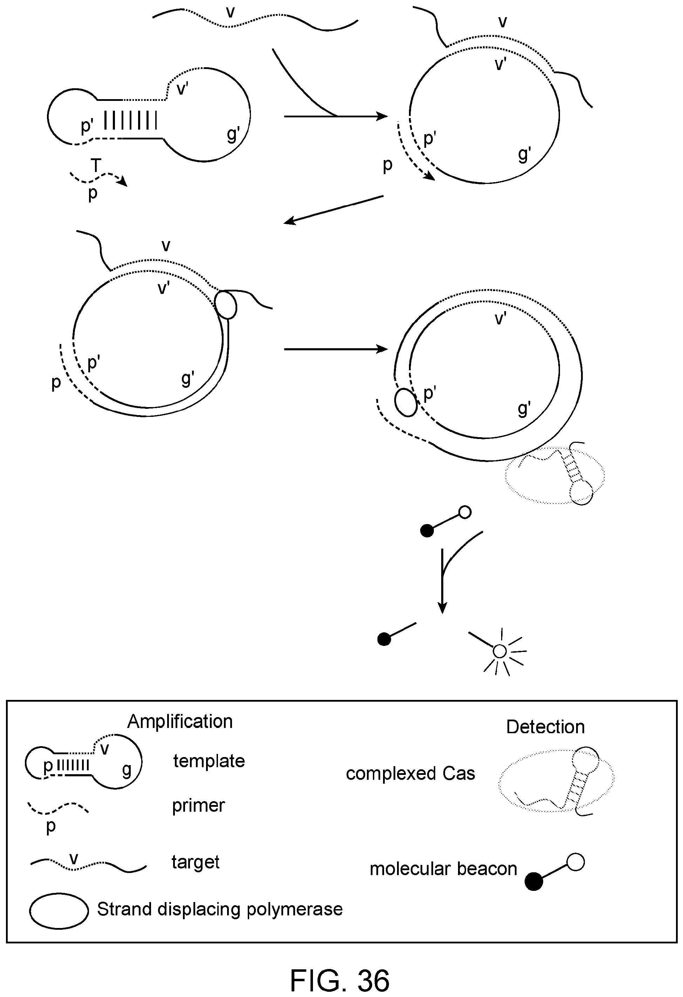

FIG. 36 presents an overview of sRCA. In this system, a target nucleic acid is added to a system that contains components for a one-pot RCA + Cas protein reaction. The RCA portion of the system is composed of a dumbbell-shaped DNA template, a primer, and a DNA polymerase. For some embodiments, the DETECTR portion of the reaction is composed of a Cas protein, such as a Cas12 or Cas14a.1, a crRNA that targets the amplicon generated by RCA, but not the dumbbell-shaped DNA template, and a FQ ssDNA reporter. A target nucleic acid (e.g. viral RNA), which is capable of binding to the dumbbell DNA template, is added to the system. The target nucleic acid base pairs with the DNA template. The more extensive base pairing between the target and the DNA template causes the internal base pairing of the dumbbell to be disrupted, which opens up a binding site for the primer. The DNA polymerase can then use this primer to begin RCA. In some embodiments, As RCA proceeds, the amplicon is generated which contains the target site for the Cas protein. The Cas protein recognizes this site through base pairing with the crRNA, and initiates trans-cleavage of the FQ ssDNA reporter. In some embodiments, the system contains fewer components than other one-pot approaches and does not require a RT enzyme.

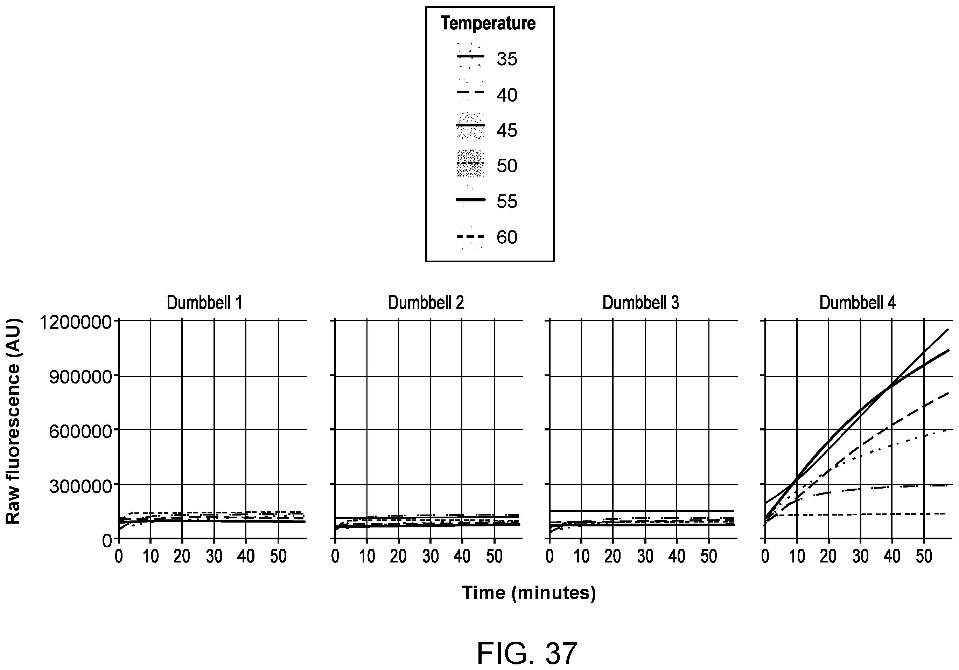

In some embodiments, dumbbell DNA templates were screened for sRCA performance, as shown in FIG. 37. In some embodiments, four dumbbell DNA templates for RCA contained a Cas12 or Cas14a.1 target sites. For these embodiments, A DNA binding dye, SYTO9, was used to monitor whether these dumbbells were functional in RCA at a variety of temperatures for 1 hour. Results as seen in FIG. 37 indicate that only dumbbell 4 was functional in generating DNA by RCA. The peak performance of the system was seen at 35-45C.

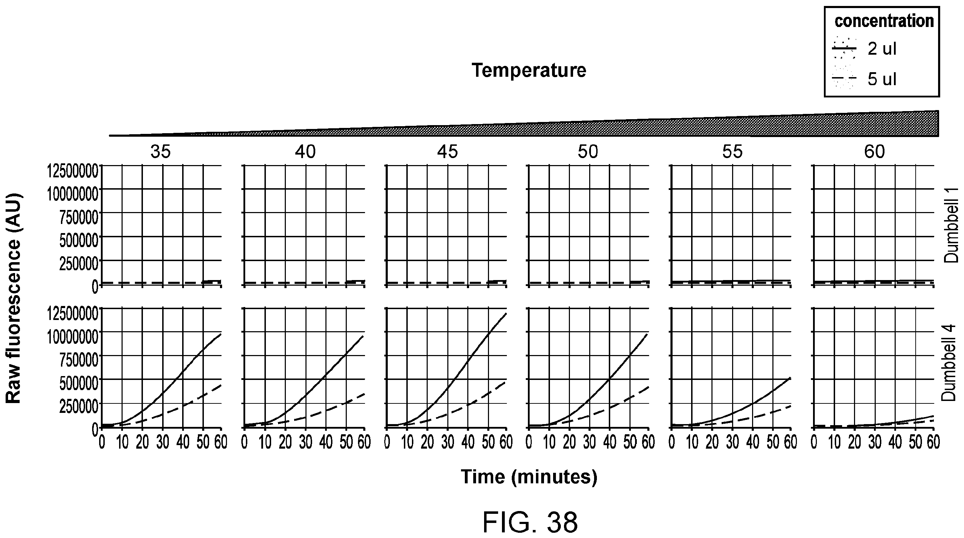

The performance of Cas14a.1 to detect product of RCA reaction was monitored, as seen in FIG. 38. It was shown that Dumbbell 4 was functional in RCA and that Dumbbell 1 was not functional under the conditions tested. Either 2 µL or 5 µL of the completed RCA reactions were added to a 20 µL Cas14a.1 DETECTR reaction that contained a crRNA that was capable of detecting the amplicon generated by RCA, but not the Dumbbell DNA template. The results indicate that Cas14a.1 is capable of detecting the amplicon as expected, and that increased performance is seen with lower amounts of RCA added to the reaction.

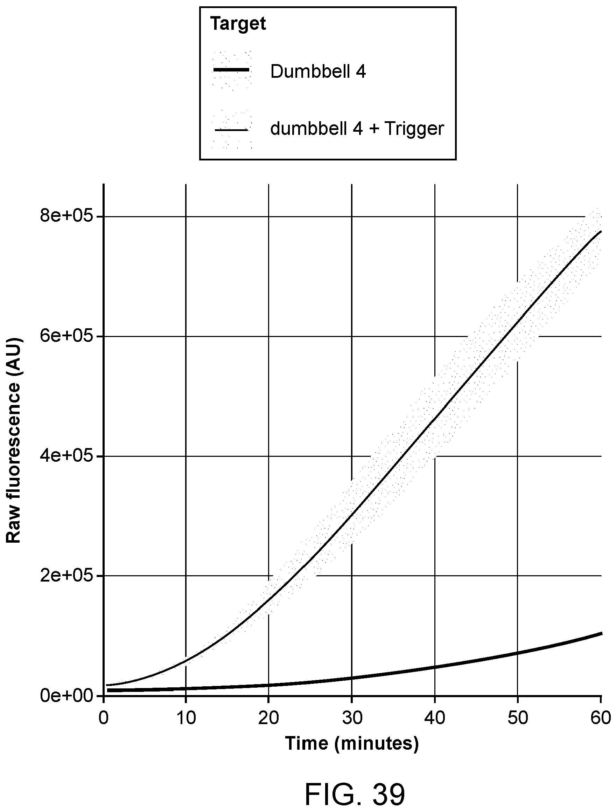

In some embodiments of the One-Pot assay sRCA Cas14a.1 was used, as shown in FIG 39. The reaction was performed at 45C in two conditions as two embodiments. In a first embodiment, the DNA template, Dumbbell 4, did not contain a trigger oligo and in the second embodiment the DNA template did have a trigger oligo. In the first embodiment with the trigger oligo condition, which initiates RCA, higher signal was observed as compared to the second embodiment condition without the trigger oligo. This demonstrates that Cas14a.1 is capable of detecting the RCA amplicon in a one-pot reaction, and that the sRCA reaction can be controlled by the presence of the trigger oligo.

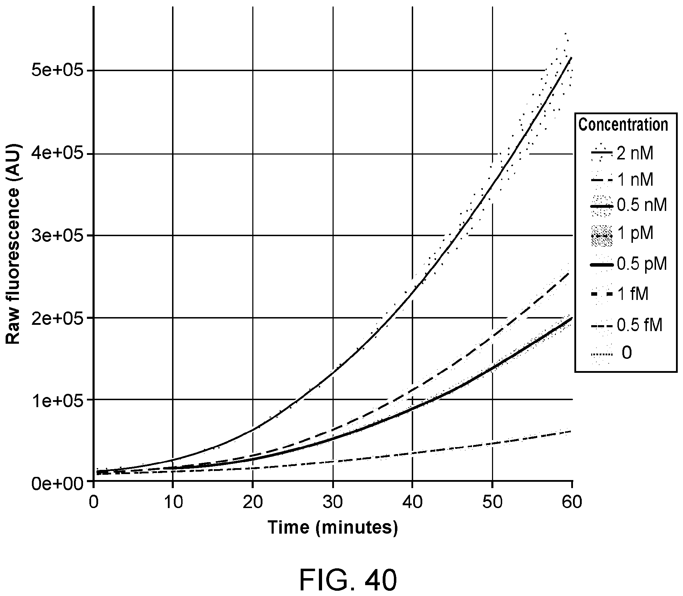

In some embodiments, a trigger oligo was titrated for the Cas14a.1 One-Pot sRCA assay. In this embodiment, the minimal concentration of trigger oligo that was required to initiate the one-pot Cas14a.1 sRCA reaction was determined. Results shown in FIG. 40 indicate that at least 0.5 nM of trigger oligo was required to initiate the sRCA reaction under the conditions tested.

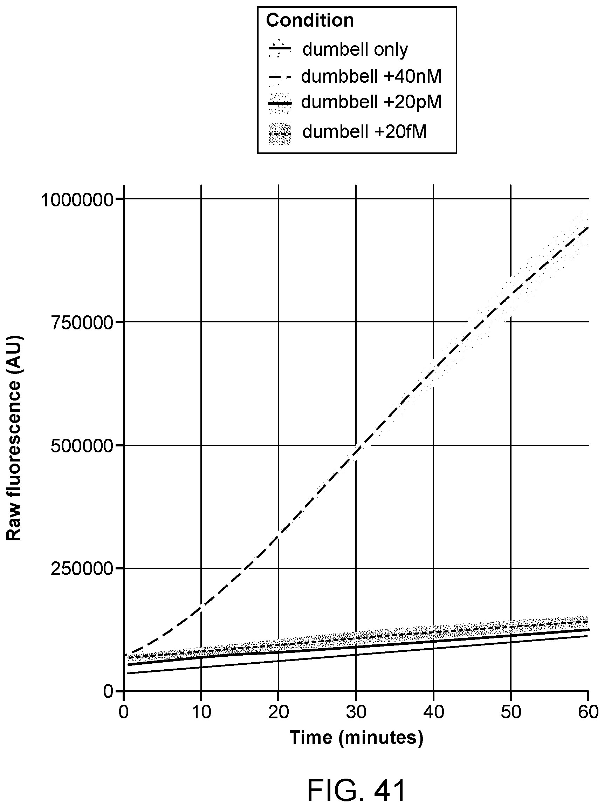

In another example, a Cas12 variant enzyme (SEQ ID NO: 28) was used in the one-pot sRCA assay. In other embodiments, it has been shown that Cas14a.1 was capable of functioning in a one-pot sRCA reaction. In this embodiment, it was shown that the Cas12 variant was also capable of functioning in this assay at 45C. The results from the cleavage of ssDNA FQ reporter included in the sRCA reaction are shown in FIG. 41. The results for such an embodiment indicate that Cas12 variant was capable of functioning in the one-pot sRCA format. For such an embodiments, the concentration of stock trigger oligo was: (1) 40 nM stock = 2 nM final conc; (2) 20 pM stock = 1 pM final conc.; and (3) 20 fm stock = 1 fM final conc.

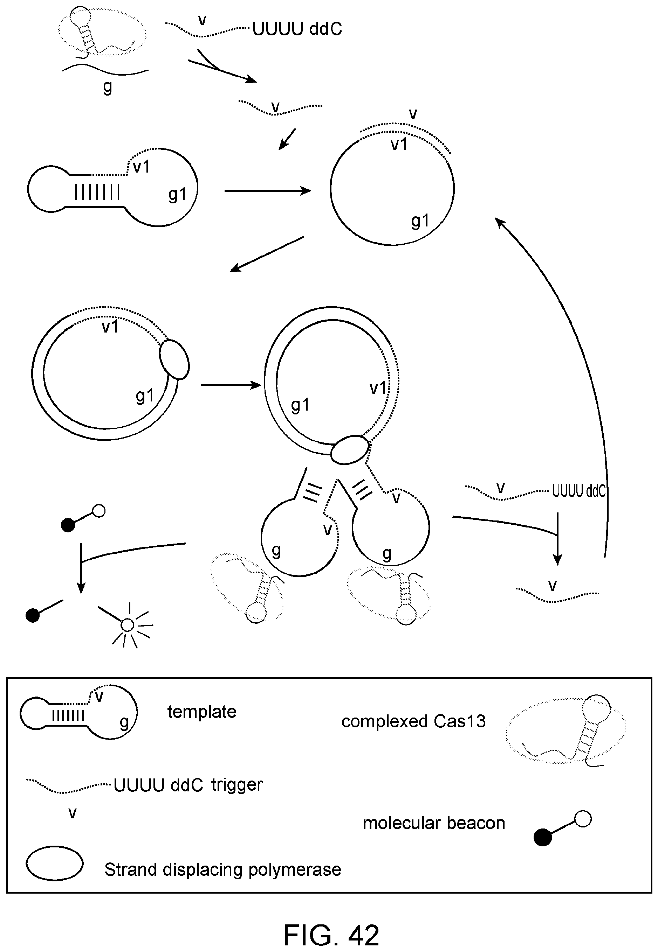

FIG. 42 presents an overview of an RCA-based positive feedback system for a Cas13 effector protein. In some embodiments, Cas13 may be programmed to recognize an RNA target. When the viral RNA target is present, a blocking motif on the 3' end of the primer is removed. After removal of this blocking motif, the primer can then serve to open up the circular template and allow for amplification by RCA using a DNA polymerase. As the amplicon is generated the same target sequence as the original RNA is generated. This ssDNA target sequence is then capable of being recognized by Cas13 which can either remove additional blocking groups from the primer or cleave a FQ reporter that generates a fluorescent signal. This system forms a positive feedback loop for Cas13. In general, a "blocking motif" refers to a moiety at the 3' end of a polynucleotide that impedes formation of a covalent bond between the 3' hydroxyl moiety of the nucleotide and the 5' phosphate of another nucleotide. A variety of suitable blocking motifs are available, non-limiting examples of which are described in US20210198730.

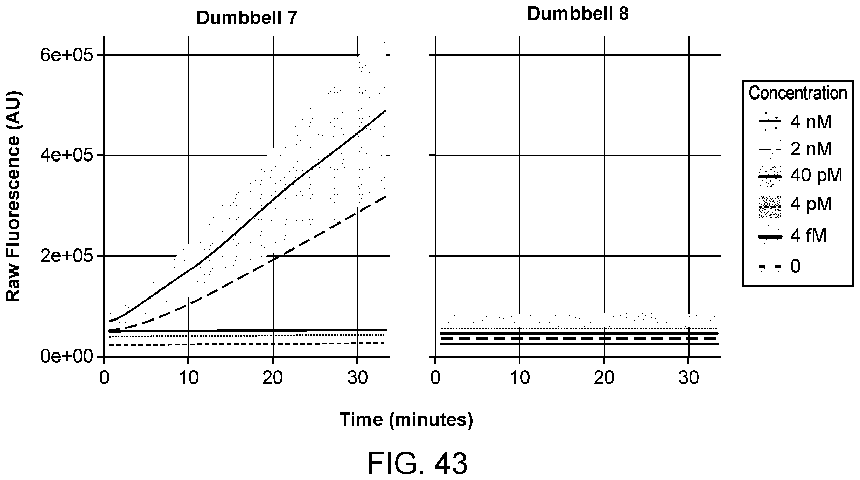

FIG. 43 presents results for the evaluation of Cas13-compatible DNA templates for RCA. In some embodiments, two dumbbell DNA templates for RCA contained a Cas13 ssDNA targeting site. In some embodiments, a DNA binding dye, SYTO9, was used to monitor whether these two dumbbells were functional in RCA at 30C. The performance of the two templates was monitored with a titration of the primers that were used to trigger the amplification. The results indicate that Dumbbell 7, but not Dumbbell 8 is compatible with RCA as shown in FIG. 43 under the conditions tested.

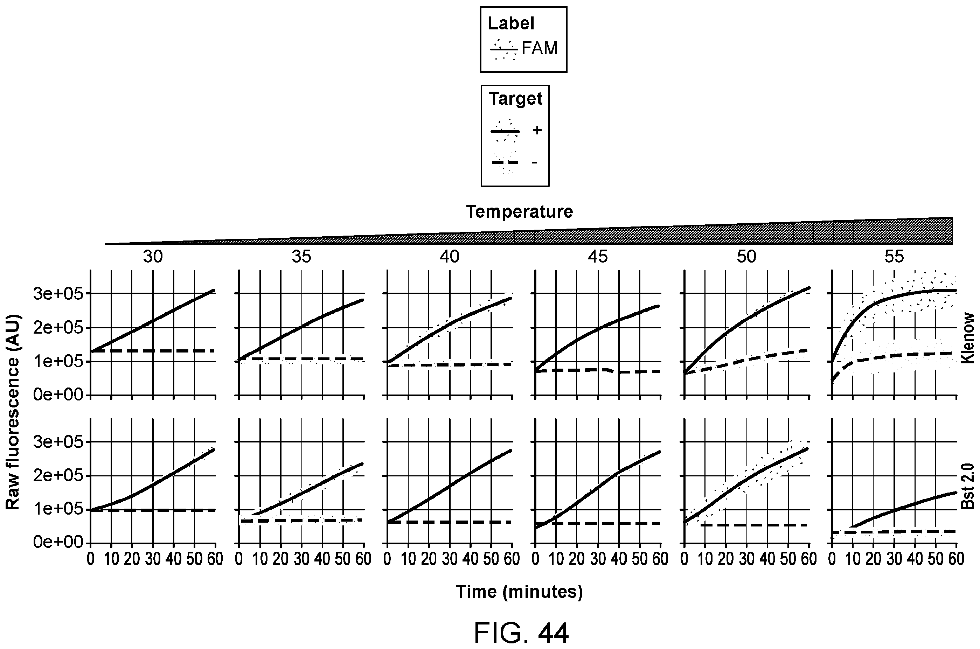

In some embodiments, Cas13-compatible DNA template was used for RCA. FIG. 44 presents results for such embodiments, to determine whether Cas13-compatible DNA template was functional in RCA. In some embodiments, a circular DNA dumbbell for sRCA had a Cas13 ssDNA target site, known as dumbbell 7. A DNA binding dye, SYTO9, was used to monitor whether the DNA template was functional in RCA at various temperatures for two different polymerases using 2 nM of trigger oligo (the primer for sRCA). The results shown in FIG. 44 indicate that dumbbell 7 was capable of generating amplicon at temperatures from 30 to 55C. These conditions, consisting of the utilization of dumbbell 7 for generating amplicons from 30 to 55 C, enabled for the use of the Cas13 enzyme in the OnePot reaction.

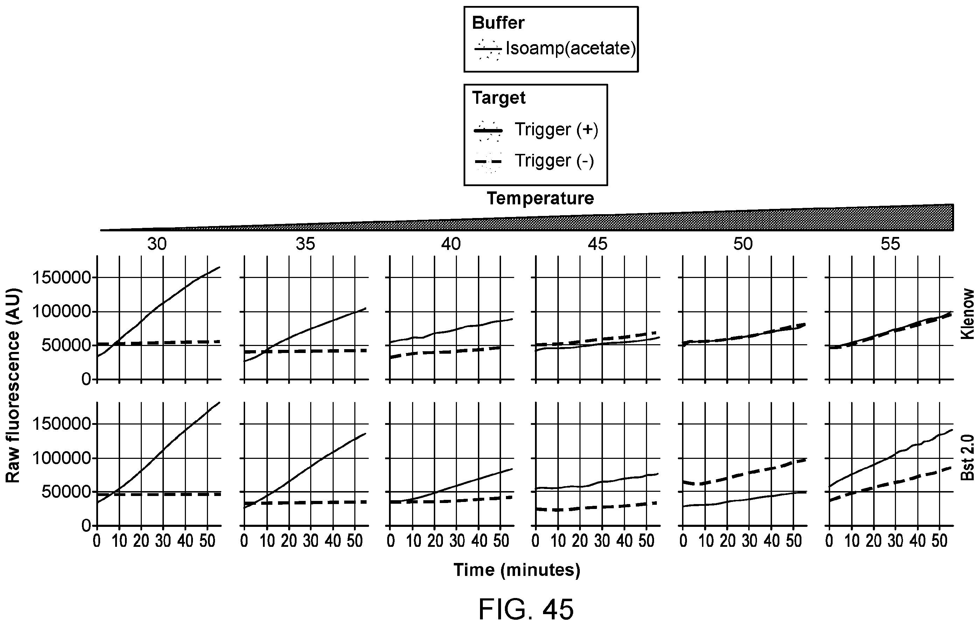

In some embodiments, a Cas13 effector protein (SEQ ID NO: 154) was used in the one-pot sRCA reaction. FIG. 45 presents results for such an embodiment, where the amplicon generated by RCA contained a ssDNA region that was capable of being recognized by a Cas13 gRNA. As the reaction proceeded, additional ssDNA target was produced, while the activated the Cas13 cleaved a FQ RNA reporter to generate a signal. The performance of this reaction at a variety of temperatures was evaluated and it was shown that the Cas 13 was capable of detecting this ssDNA region of the RCA amplicon from 30-40C, which aligns with the previously established active temperatures for the Cas 13. The performance of two different polymerases at the temperatures of interest was also compared. The results shown in FIG. 79 suggest that Cas 13 can be used in a one-pot reaction where RCA is the amplification method, and that the Cas13's ability to detect ssDNA was preserved in this embodiment under the conditions tested.

### EXAMPLE 15

### CasPin: Cas13 positive-feedback loop leveraging Cas13 ssDNA targeting

This example describes one approach to combining isothermal amplification protocols described herein with new CRISPR systems to create one-pot DETECTR and other assays, as outlined by the strategy in FIG. 18. In particular, this example demonstrates that Cas13 programmable nucleases can be used to create a feedback loop amplification system. To develop such a system (CasPin), various DETECTR assays were performed to determine feasible conditions, as described herein.

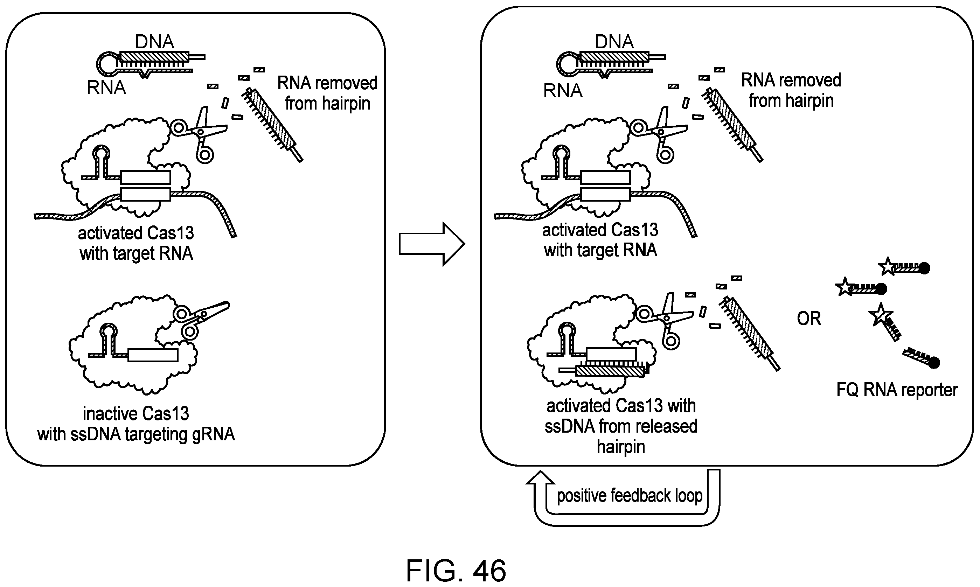

FIG. 46 presents an overview of CasPin. In some embodiments, the CasPin system uses two populations of Cas13. One is programmed with a crRNA that targets an RNA of interest (primary target), such as a viral genome. The other population is programmed with a crRNA that is optimal for ssDNA detection. The systems also contain a hairpin-shaped oligo that is composed of both DNA and RNA. Finally, in some embodiments, there is a FQ RNA reporter that is used to readout the result of the assay. When Cas13 detects the RNA of interest, it can either cleave the FQ RNA reporter or the RNA on the hairpin oligo. When the RNA on the hairpin oligo is cleaved, it dissociates from the DNA revealing a ssDNA target site (secondary target) that can be recognized by the other population of Cas13 RNPs. This initiates a positive feedback loop where Cas13 recognizes the ssDNA target and cleaves more hairpin molecules, which increases the overall amount of target in the system (either primary target or secondary target) and leads to further activation of the system. As this process continues more and more FQ RNA reporter is cleaved, which is the ultimate readout of the assay.

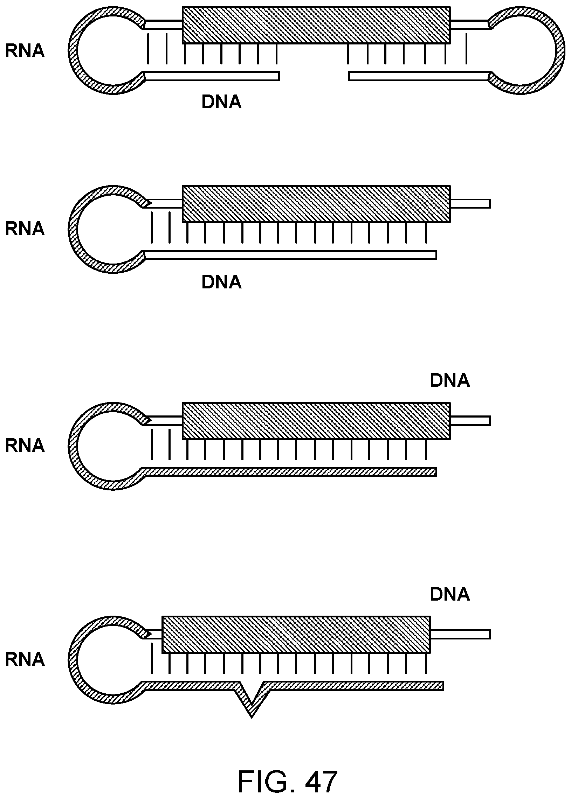

FIG. 47 presents some exemplary structures of hairpins for CasPin. In some embodiments, the target ssDNA sequence is indicated by the rectangle. RNA loop structures may occur on either side of the target strand (either 5', 3' or both). The strand that is complementary to the target site may be DNA or RNA. The strand that is complementary to the target site may also be a perfect match to the target site, be shorter than the target site, or contain mismatches to help destabilize or promote trans-cleavage by Cas13.

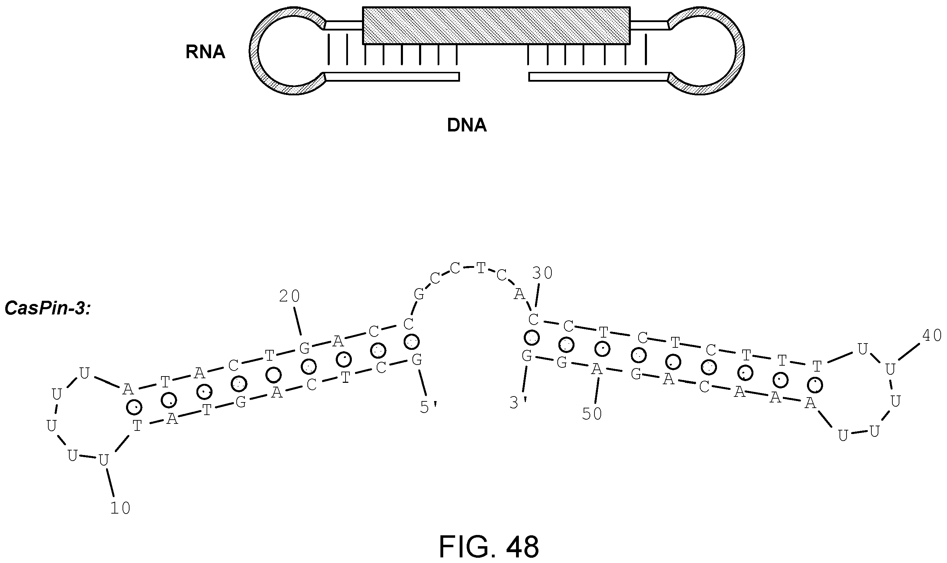

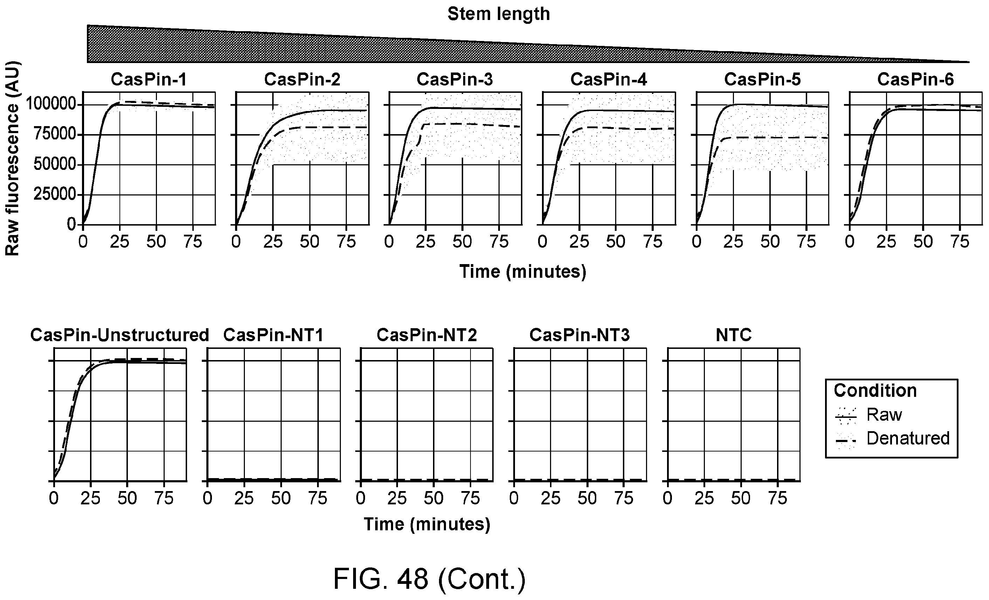

In some embodiments, two hairpins were used on either end of the target site. FIG. 48 presents results indicating capability for blocking Cas13 from recognizing the ssDNA target site. CasPin oligos had varying lengths of hairpin stems. In some embodiments, CasPin olgio did not have stem structures. In other embodiments, CasPin oligos contained another DNA sequence. These were evaluated and found to not be recognized by the crRNA. Both raw oligos from the manufacture and those that had been denatured and refolded at 25C in a Cas13 DETECTR reaction were tested. The results of this experiment, shown in FIG. 48, demonstrate that Cas13 was able to recognize the target site regardless of the stem length. In some embodiments, longer stem length oligos block Cas13 recognition without RNA cleavage to release the structure, suggesting that it may be desirable to have longer hairpins.

### EXAMPLE 16

### DETECTR Immobilization

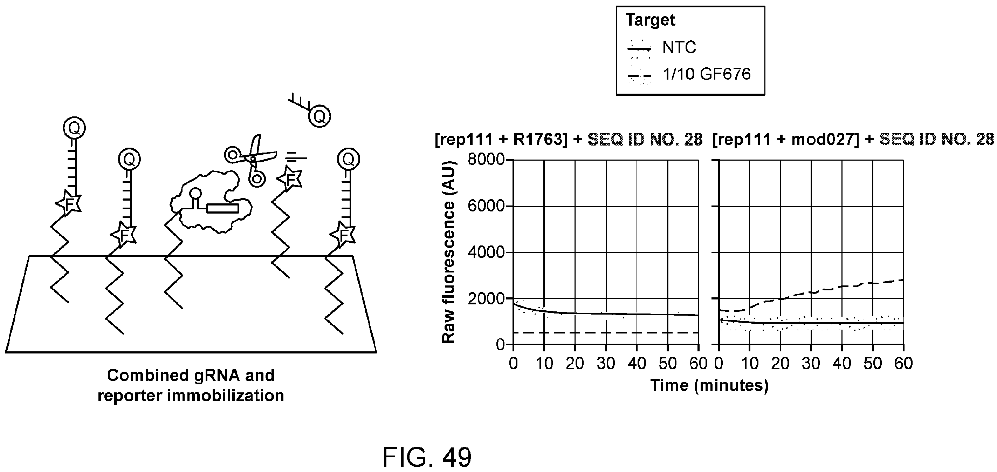

In some embodiments, NHS-Amine chemistry may be used for immobilization of DETECTR components. FIG. 49 presents a schematic of combined gRNA and reporter immobilization and results. In this embodiment, a functional DETECTR reaction was immobilized to solid substrate (NHS plate) using primary amine modified reporters and gRNAs. In the example shown, an amine-modified reporter molecule (rep111) was bound to the surface in combination with either an unmodified crRNA (R1763) or a modified crRNA (mod027). After incubating these nucleic acids on the surface, the surface was washed 3 times, and then a Cas12 variant (SEQ ID NO: 28) was added with the target dsDNA. The immobilized DETECTR reaction was then incubated in a plate reader at 37C for 60 minutes with continuous monitoring of the fluorescence.

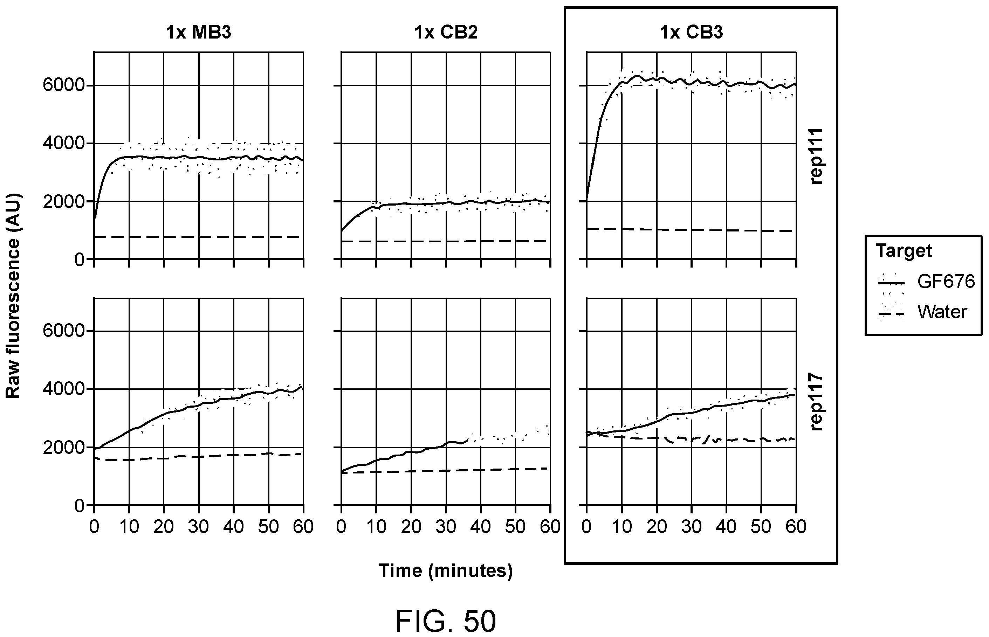

FIG. 50 presents results for various embodiments involving the optimizing conjugation buffer to reduce non-specific binding of DETECTR reagents. For some embodiments, 1x Conjugation Buffer 3 (CB3) was selected as the buffer to perform binding studies. It was found that CB3 improved the binding of the amine-modified reporter (rep111) and reduced the binding of a biotinylated reporter (rep117) which should not bind to NHS covalently. The wash buffer used was 1x MB3. In some embodiments, 1x MB3: 20mM HEPES, pH 7.2, 2mM KOAc, 5mM MgAc, 1% Glycerol, 0.0016% Triton X-100 was used. In other embodiments, 1x CB2: 20mM HEPES, pH 8.0, 2mM KOAc, 5mM MgAc, 1% Glycerol, 0.0016% Triton X-100 was used. In this embodiment, 1x CB3: 100mM HEPES, pH 8.0, 10mM KOAc, 25mM MgAc was used.

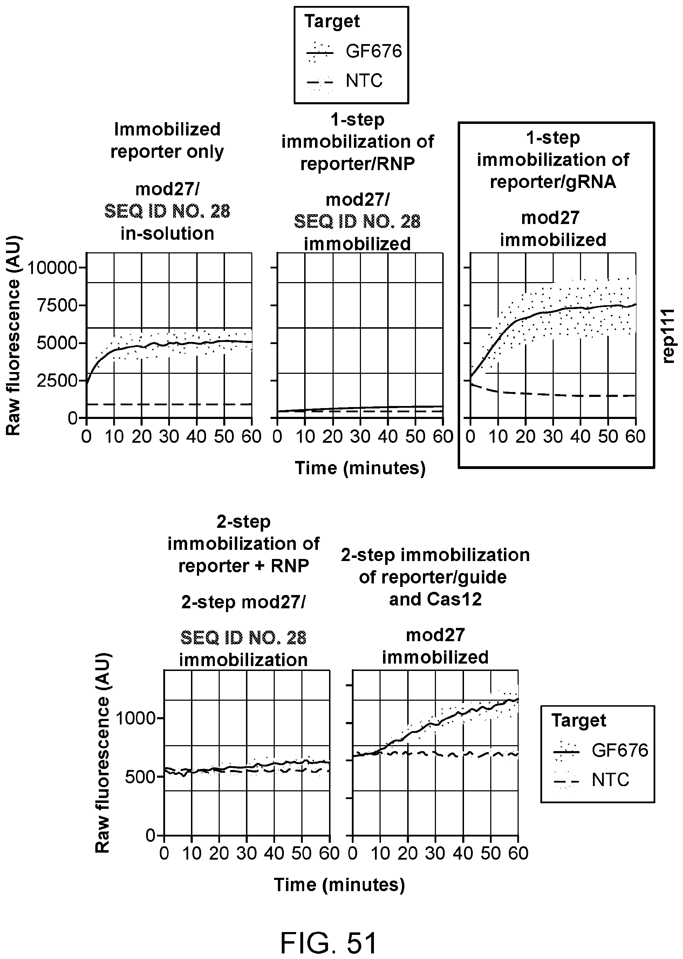

In this embodiment, different combinations of reporter + guide + the Cas12 variant may be immobilized. FIG. 51 presents results of such embodiments, involving the optimization of the assay. For such embodiments, it was found that immobilizing guide and reporter first followed by the addition of the Cas12 variant and target at the same time gave sufficient signal.

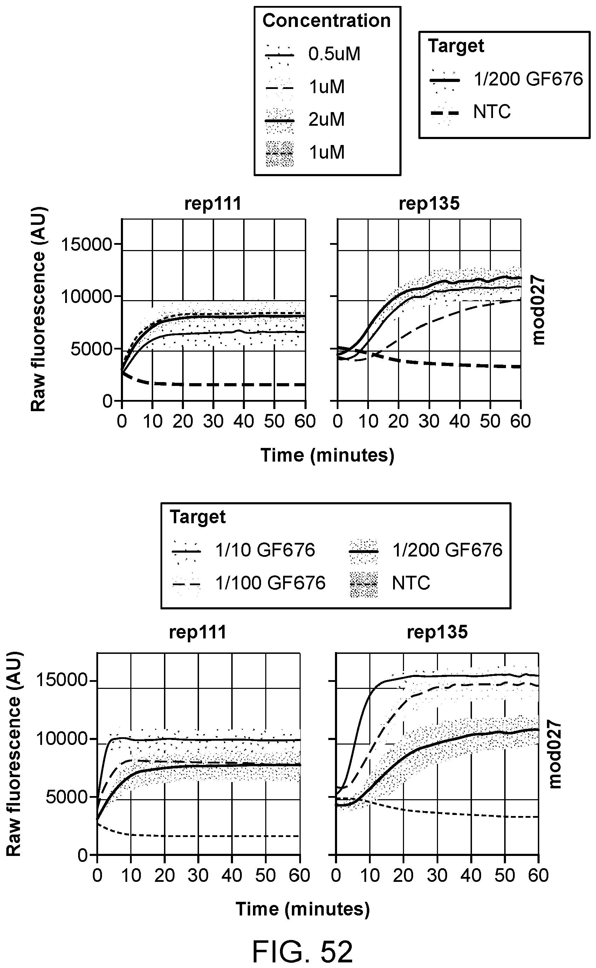

The results for optimizing gRNA and target concentrations to improve single-to-noise ratio for immobilized DETECTR assay are shown in FIG. 52. In this embodiment, guide RNA concentration is increased while keeping reporter concentration constant at 0.5***µ***M, as seen on the left of FIG. 52, the signal is not changed very much. In some embodiments, as seen on the right of FIG. 52, increasing target concentration 2-fold helped improve the overall signal with rep135. Additionally, for such embodiments, rep135 gave a better signal strength compared to rep111. The sequences for the two reporters were: rep111: 5AmMC6T//i6-FAMK/TTTTTTTTTTTT/3IABkFQ/ and rep135: 5AmMC12//i6-FAMK/TTTTTTTTTTTTTTTTTTT/3IABkFQ/.

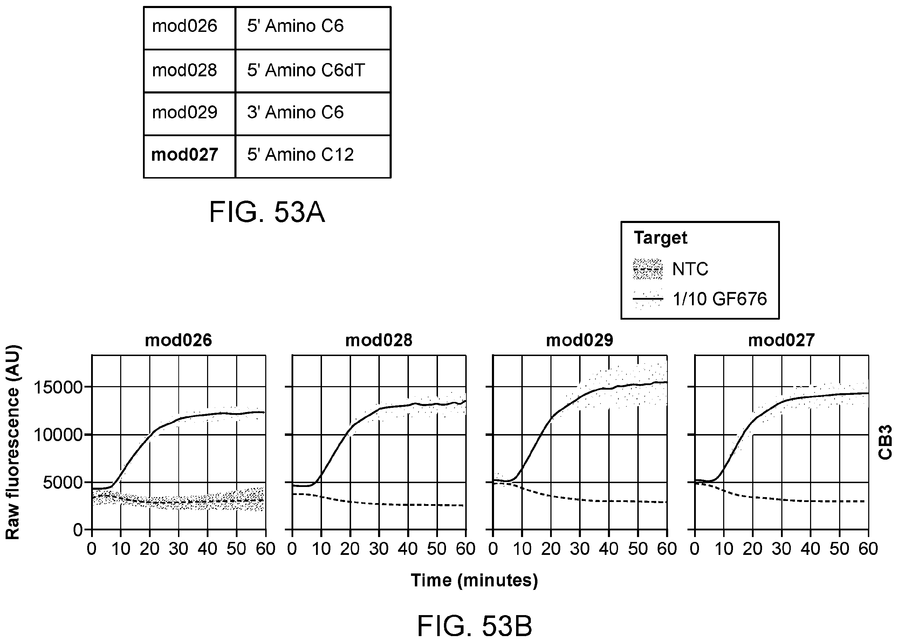

In some embodiments, amino modifications were used for DETECTR immobilization, as presented in FIG. 53. For each subplot raw fluorescence was plotted against time in minutes. Each of the four subplots represent different modifications. The dashed trace represents the no target control (NTC) and the solid trace represents a 1:10 dilution of target - GF676.

### EXAMPLE 17

### Rapid PCR (FASTR) Thermocycling

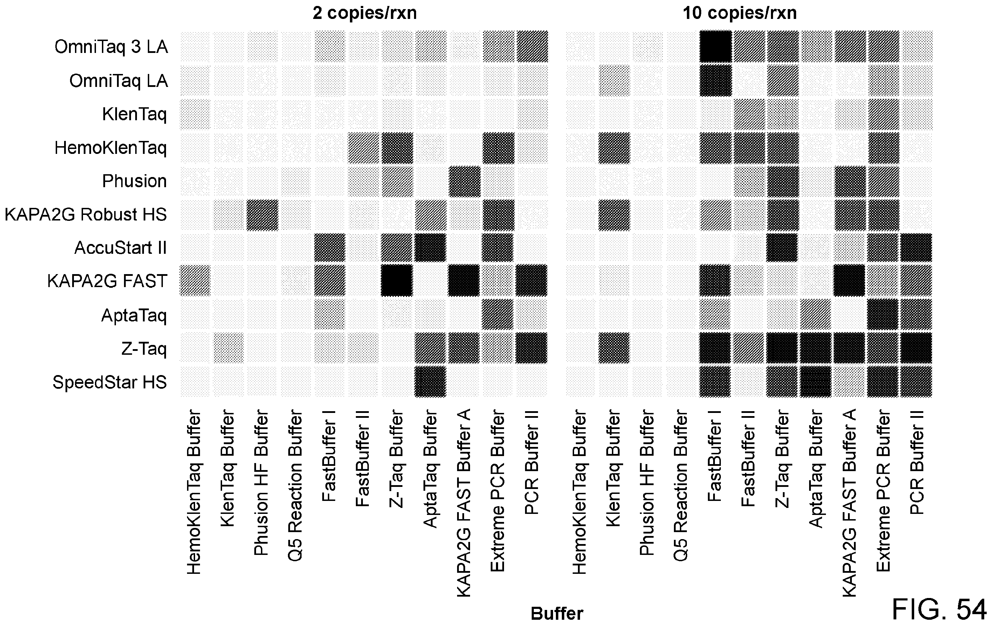

In some embodiments, rapid thermocycling and CRISPR diagnostics were used to detect SARS-CoV-2. Results are shown in FIG. 54. For some embodiments, polymerase and buffer combinations were identified that enabled the rapid amplification of SARS-CoV-2 using the N2 primers from the CDC assay. Assays were performed at two target concentrations: 2 input copies/rxn and 10 input copies/rxn. Reaction conditions were as follows: initial denaturation at 98C for 30 seconds, followed by 45 cycles consisting of 1 second at 98C and 3 seconds at 65C. Following thermocycling, amplicon was transferred to a Cas12 variant (SEQ ID NO: 28) detection reaction for 30 minutes at 37C. Best performing enzyme/buffer pairs shown in FIG. 54 were those that gave strong signal in both tested concentrations.

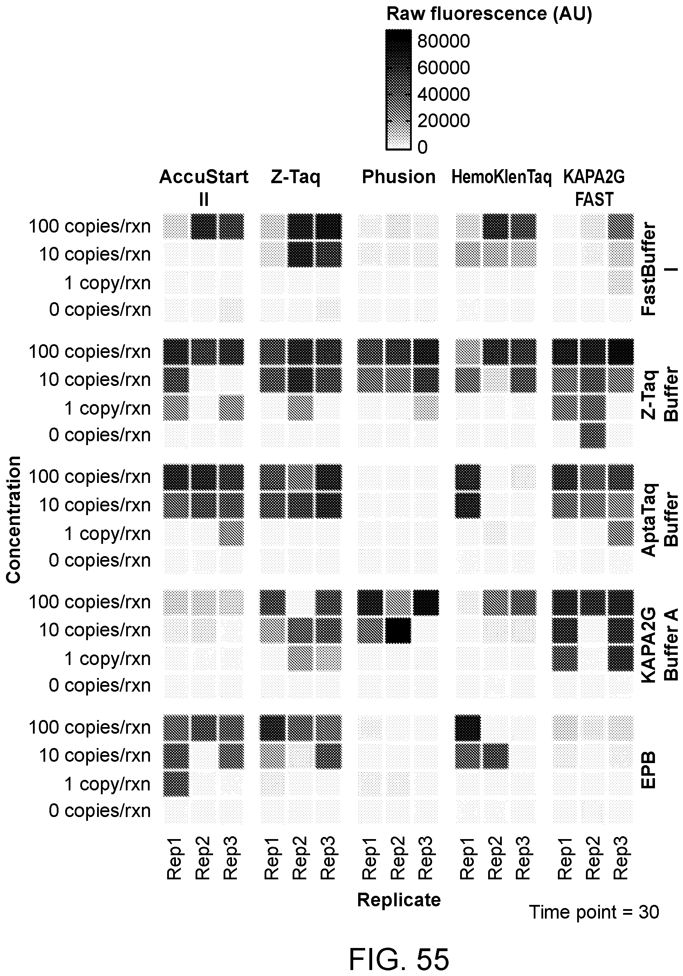

The top enzymes and buffers identified previously at various concentrations and with multiple replicates were tested for the FASTR assay. In some embodiments, the best performing enzymes and buffers as identified in the previously disclosed screening studies were used. Results of such embodiments are shown in FIG. 55. Reaction conditions of such embodiments were as follows: initial denaturation at 98C for 30 seconds, followed by 45 cycles consisting of 1 second at 98C and 3 seconds at 65C. Primers used were from the CDC N2 assay for SARS-CoV-2. Following thermocycling, amplicon was transferred to a Cas12 variant (SEQ ID NO: 28) detection reaction for 30 minutes at 37C. The data present is the signal from the CRISPR reaction. Best performing enzyme/buffer pairs were those that gave strong signal at the lowest tested concentrations and with consistent detection across replicates.

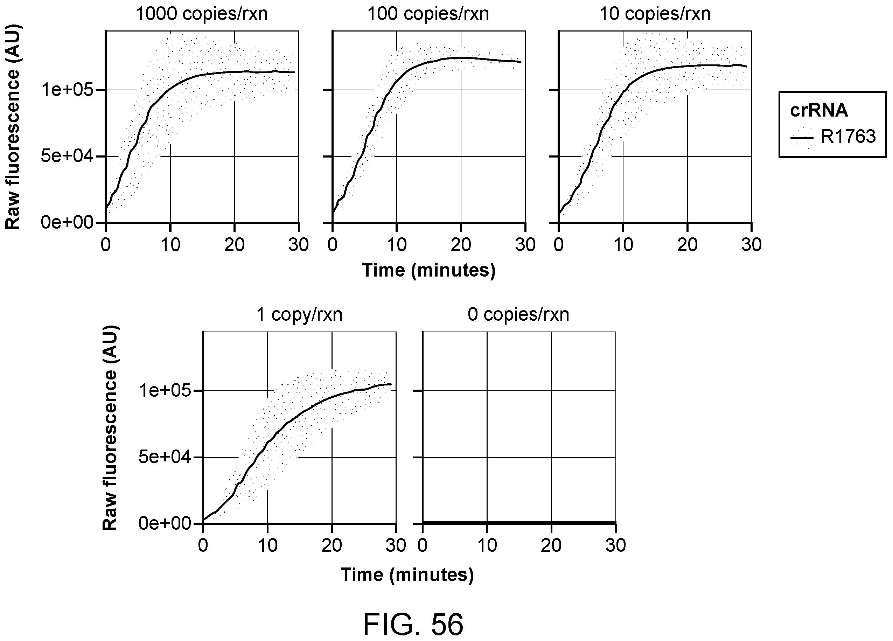

For some embodiments, single copy detection of SARS-CoV-2 with FASTR assay was demonstrated as shown in FIG. 56. The limit of detection of the FASTR assay was evaluated using solutions composed of 1000 copies of SARS-CoV-2 per reaction to 1 copy per reaction. Reaction conditions were as follows: reverse transcription at 55C for 60 seconds, initial denaturation at 98C for 30 seconds, followed by 45 cycles consisting of 1 second at 98C and 3 seconds at 65C. Primers used were from the CDC N2 assay for SARS-CoV-2. Following thermocycling, amplicon was transferred to a Cas12 variant (SEQ ID NO: 28) detection reaction for 30 minutes at 37C. The data presented in FIG. 56 is the signal from the CRISPR reaction. It was found that the limit of detection of the CRISPR assay was 1 copy of SARS-CoV-2 per reaction.

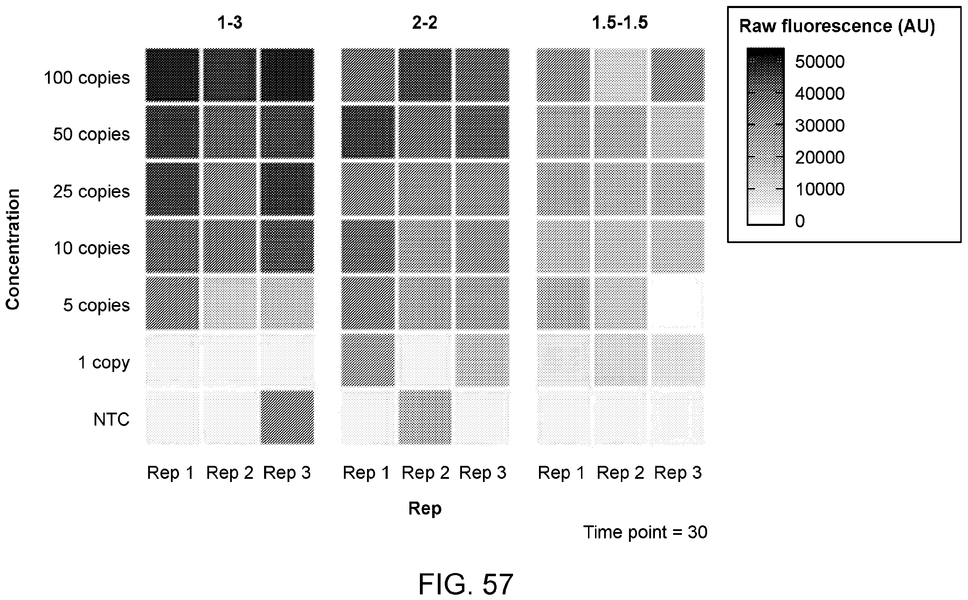

Rapid cycling times were varied to evaluate denaturation and annealing/extension for the FASTR assay, as shown in FIG. 57. Reverse transcription was run at 55C for 60 seconds and initial denaturation at 98C for 30 seconds. In some embodiments, the tested cycling conditions were: 98C for 1 second, 65C for 3 seconds; 98C for 2 seconds, 65C for 2 seconds; or 98C for 1.5 seconds, 65C for 1.5 seconds. Primers used were from the CDC N2 assay for SARS-CoV-2. Following thermocycling, amplicon was transferred to a Cas12 variant (SEQ ID NO: 28) detection reaction for 30 minutes at 37C. The results shown in FIG. 57 indicate that >2 seconds of annealing/extension time at 65C was able to generate robust sensitivity.

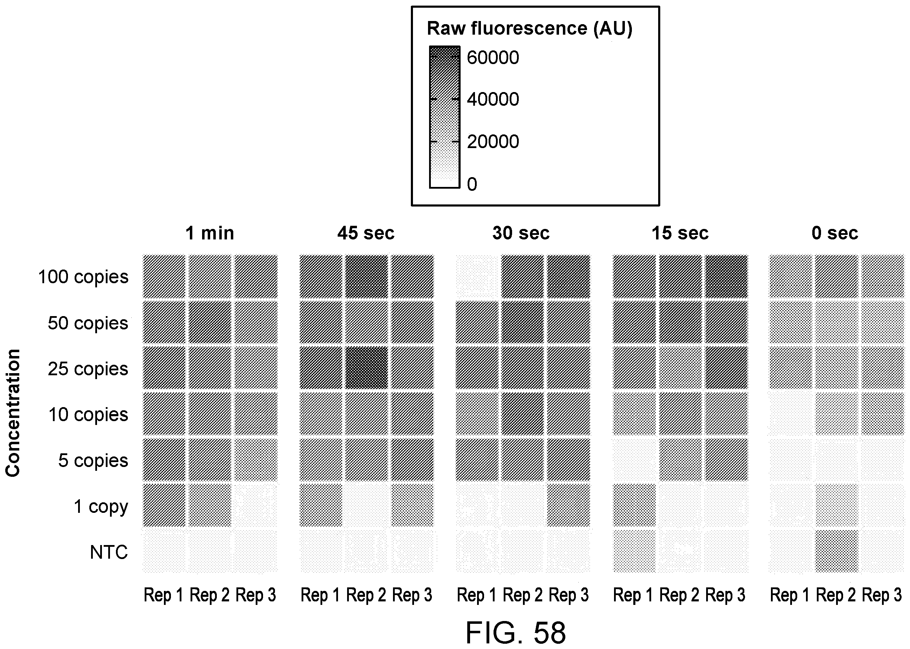

FIG. 58 presents results for Minimizing RT time for FASTR. The performance of the FASTR assay was evaluated, for various embodiments, where the reverse transcription incubation times were varied holding temperature at 55C. The results shown in FIG. 58 indicate the assay is most robust above 30 seconds of reverse transcription.

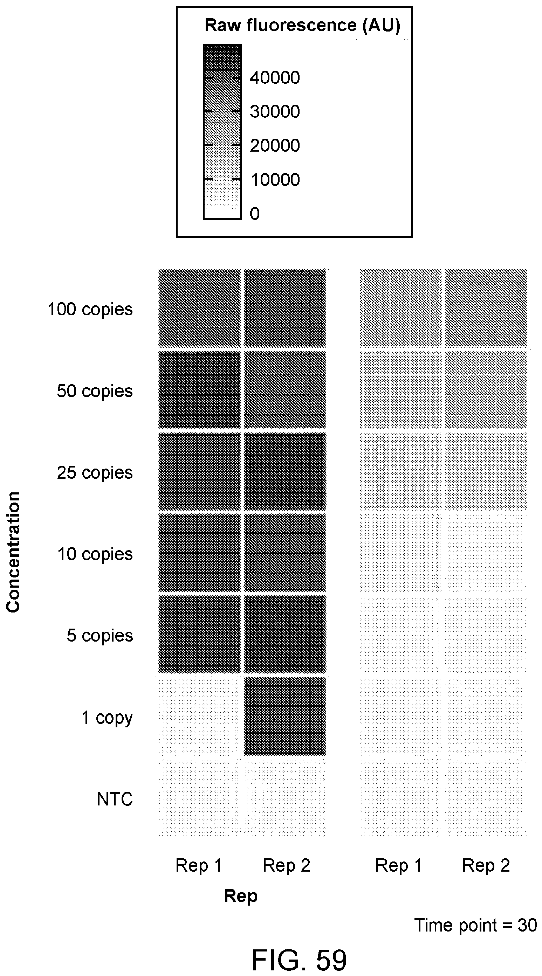

FIG. 59 presents results for Higher pH buffers improve FASTR performance. The FASTR assay utilized buffers with pH of either 9.2 or pH 7.8. The FASTR assay was evaluated using these buffer pH values. The results as shown in FIG. 59 indicate that the higher pH buffer produced superior results in terms of amplicon yield and sensitivity.

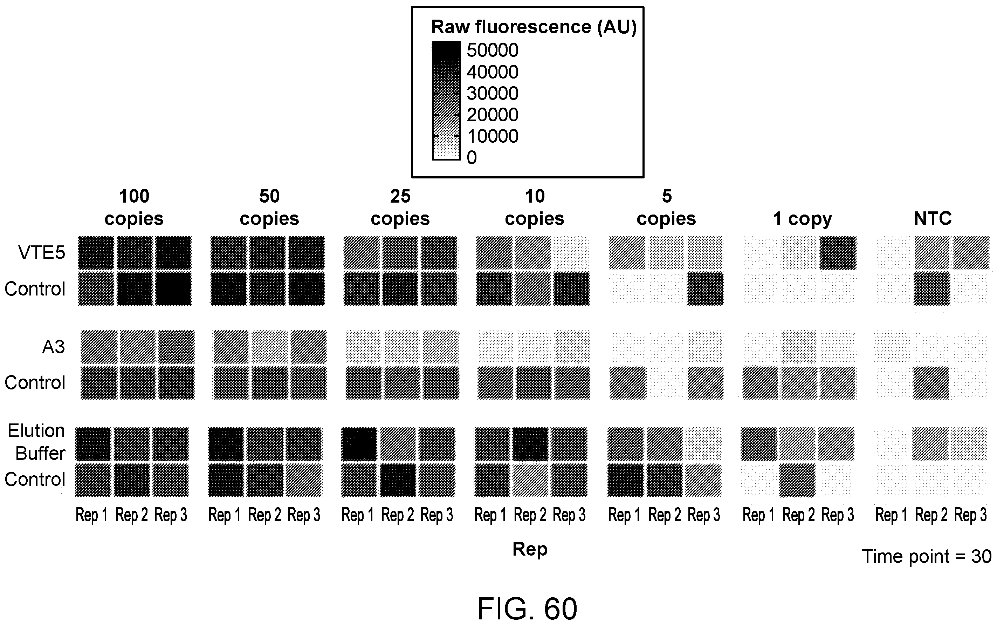

The FASTR assay compatibility with crude lysis buffers was investigated. Results are shown in FIG. 60 where there are three row groups, each consisting of two sub rows representing a buffer and control from top to bottom respectively. The buffers, VTES, A3 and Elution buffer are plotted against a control, from top to bottom, respectively. In FIG. 60 there are also seven subgroups showing the number of copies decreasing from left to right. The performance of the FASTR assay, when combined with various crude lysis buffers, was evaluated, where crude lysis buffers VTE5, A3, and the Elution Buffer from the ChargeSwitch kit (Thermo) were tested. The FASTR assay performed best for VTE5, but was performed slightly less robustly in the A3 buffer and the Elution Buffer from the ChargeSwitch kit performed similarly to the control reactions (water).

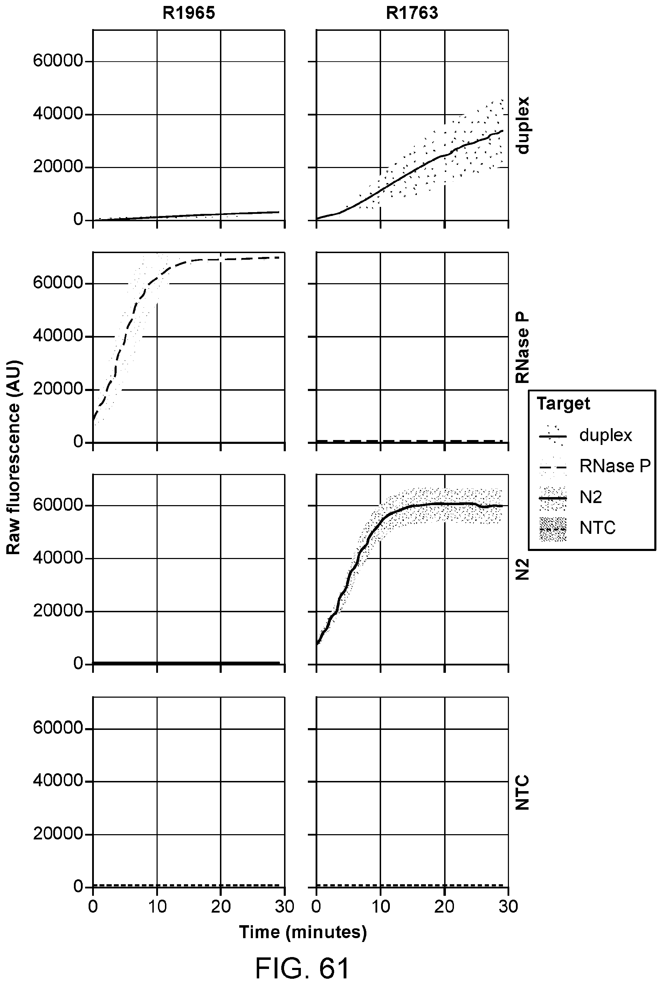

Non-optimized multiplexing of FASTR was demonstrated as shown in FIG. 61. In FIG. 61, raw fluorescence is plotted in the y-axis and time is plotted in the x-axis for each subplot. Each column illustrates a particular guide RNA sequence: R1965 and R1763, respectively Each row represents duplex, RNase P, N2 and the no target control, from top to bottom respectively. Initial testing of multiplexed FASTR for SARS-CoV-2 and RNase P POP7 (endogenous control) showed that while the single-plex assays generated a robust signal in DETECTR, the duplex assay tended to generate a weak signal for SARS-CoV-2 (R1763) and almost no signal for RNase P (R1965). Reaction conditions were as follows: reverse transcription at 55C for 60 seconds, initial denaturation at 98C for 30 seconds, followed by 45 cycles consisting of 1 second at 98C and 3 seconds at 65C. Primers from the CDC N2 assay for SARS-CoV-2, and M3637/M3638 were used.

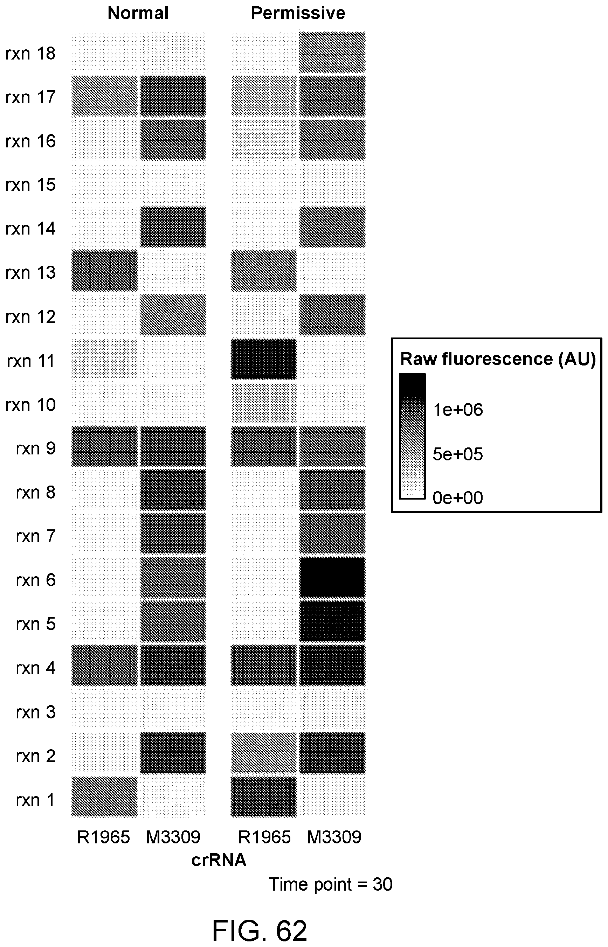

FASTR can be used for multiplexed detection, as shown in FIG. 62. The components of a FASTR reaction, such as primer concentration, dNTP concentration, presence/absence of DMSO, and other factors, can impact the performance of a multiplex FASTR reaction. FIG. 62 shows 18 different experimental conditions for a multiplex FASTR reaction targeting human RNase P POP7 and SARS-CoV-2. In FIG. 62 each row of the y-axis represents experimental runs 1-18 and each column represents the detection signal from a particular crRNA at a time point of 30 minutes in the reaction. The shading represents the value of the raw fluorescence. In some embodiments, the multiplexed FASTR assay for SARS-CoV-2 and RNase P, comprise a set of SARS-CoV-2 primers (M3257/M3258). A series of experiments of such embodiments was performed with varied reaction conditions containing different combinations of buffers, primer concentrations, dNTPs, and DMSO. Results identified two reaction conditions that performed robustly for the multiplex reaction. In one of these embodiments, reaction 4, conditions consisted of: 1X FastBuffer 2; 1 uM RNase P primers; 0.5 uM CoV primers; 0.2 mM dNTPs; and 2% DMSO. In another embodiment, reaction 9, conditions consisted of: 1X Klentaq1 buffer; 1 uM RNase P primers; 0.5 uM CoV primers; 0.4 mM dNTPs; and 0% DMSO. In some embodiments, normal reaction conditions consisted of reverse transcription at 55C for 60 seconds, initial denaturation at 98C for 30 seconds, followed by 45 cycles consisting of 1 second at 98C and 3 seconds at 65C. In various aspects, permissive reaction conditions consisted of reverse transcription at 55C for 60 seconds, initial denaturation at 98C for 30 seconds, followed by 45 cycles consisting of 3 seconds at 98C and 5 seconds at 65C.

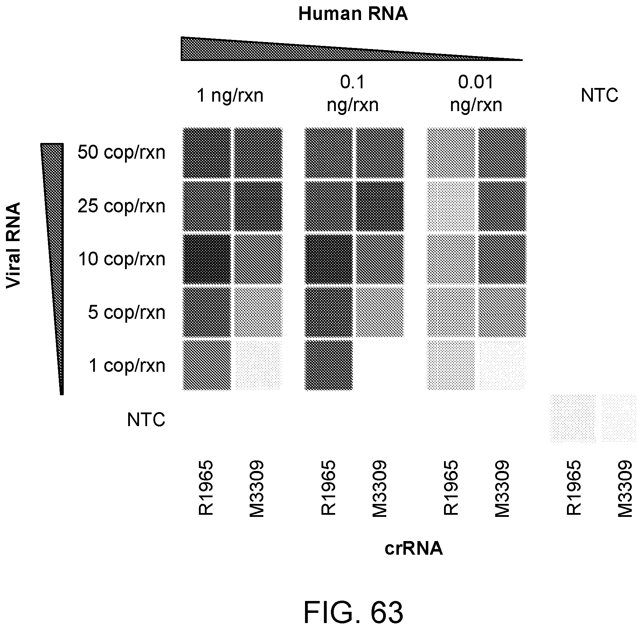

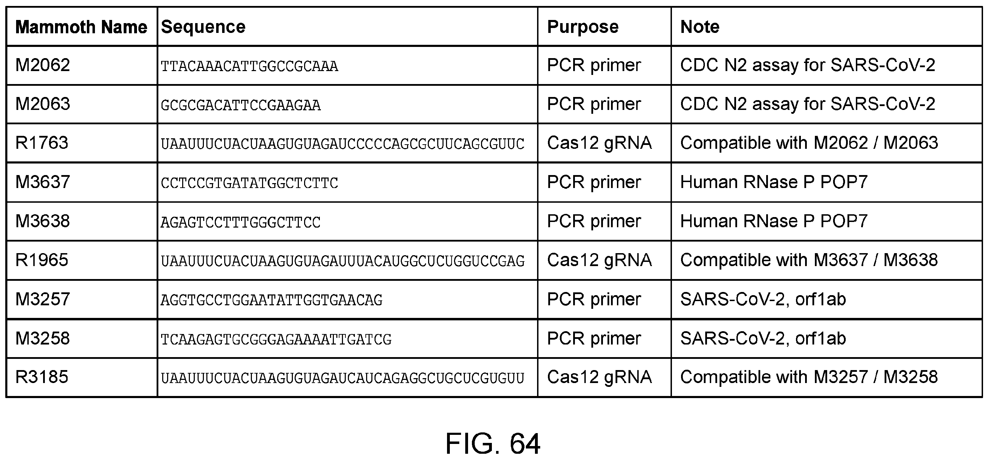

The FASTR assay enables multiplexed detection. Results of a limit of detection (LOD) study of such embodiments are shown in FIG. 63. In FIG. 63, the x-axis shows the number of copies per reaction for viral RNA and the y-axis of each subplot identifies the particular crRNA. Each subplot shows nanograms of human RNA per reaction, decreasing in concentration from left to right. The 4th subplot contains no human RNA, labeled as the no target control (NTC). An optimized multiplexed FASTR assay was ran at various concentrations of human RNA and viral RNA. Results indicated that the assay performed at a range of human RNA concentrations, while maintaining a sensitivity of ~5 copies per reaction. Results shown are from DETECTR reactions using either R1965 to detect the human RNase P, or R3185 (labeled M3309) to detect SARS-CoV-2. Reaction conditions were as follows: reverse transcription at 55C for 60 seconds, initial denaturation at 98C for 30 seconds, followed by 45 cycles consisting of 1 second at 98C and 3 seconds at 65C. Primers used were M3257/M3258 (SARS-CoV-2) and M3637/M3638 (RNase P). Key primers and gRNAs have the sequences as listed in FIG. 64.

### Example 18

### DETECTR-based HotPot reactions using Thermostable Inorganic Pyrophosphatase

This example demonstrates compositions and methods for HotPot reactions using thermostable inorganic pyrophosphatase (TIPP) to improve signal generation. TIPP is an enzyme that can catalyze the hydrolysis of inorganic pyrophosphates. This example demonstrates compositions and methods for HotPot reactions using thermostable inorganic pyrophosphatase (TIPP) to amplify output signals.

HotPot reactions were carried out under with various compositions as shown in Table 12. In the Experimental Condition, the HotPot reaction was carried out in a solution comprising both TIPP and target nucleic acid. In Control A, the HotPot reaction was carried out with target nucleic acid but without TIPP. In Control B, HotPot reaction was carried out without target RNA. In Control C, HotPot reaction was carried out without TIPP and without target RNA.

**Table 12 shows example amounts of components used in the HotPot reactions.**

| **Component** | **Amount in (µL)** | | | |
|---|---|---|---|---|
| | **Experimental Condition** | **Control A** | **Control B** | **Control C** |
| Nuclease-free water | 1.975 | 1.975 | 1.975 | 1.975 |
| 5X IB15 | 2 | 2 | 2 | 2 |
| 10 mM dNTP (NEB) | 1 | 1 | 1 | 1 |
| 100 µM rep033 | 0.1 | 0.1 | 0.1 | 0.1 |
| RNase Inhibitor, Murine (NEB) | 0.05 | 0.05 | 0.05 | 0.05 |
| Bsm (Thermo) | 0.5 | 0.5 | 0.5 | 0.5 |
| Warmstart RTx (NEB) | 0.125 | 0.125 | 0.125 | 0.125 |
| Cas14a complexing reaction | 1 | 1 | 1 | 1 |
| 10X Primer Mix | 1 | 1 | 1 | 1 |
| TIPP (2U/µL) | 0.25 | - | 0.25 | - |
| Target RNA | 2 | 2 | - | - |
| Total | 10 | 9.75 | 8 | 7.75 |

Briefly, in a first experimental example, Cas14a.1 (SEQ ID NO. 63) effector proteins were complexed with sgRNA for 30 minutes at 37°C. The 1x concentration of proteins was 40 nM and the final concentration of sgRNA was 40 nM. 1uL of the Cas14a complexing reaction was combined with the HotPot components listed in Table 9 for each experimental or control condition listed. Reactions were carried out at 55°C for 60 minutes. Trans cleavage activity was detected by fluorescence signal upon cleavage of a fluorophore-quencher reporter in the HotPot DETECTR reaction. FIG. 65 shows HotPot reaction results under the various conditions. The experimental condition, compared to control A without TIPP, had greater than 5-fold increase in the output fluorescence signal, while control A still had significantly higher signal than control D (no TIPP or target nucleic acid) and both conditions were able to detect the target nucleic acid. Control B without target RNA had about the same output fluorescence signal as control C without TIPP and without target RNA.

In a second experimental example, a 2:1 ratio mixture of unfunctionalized PEG (MW=600 monomers) and PEG-diacrylate (MW=700 monomers) were mixed together with a photoinitiator (2-Hydroxy-2-methylpropiophenone (Darocur 1173)) and 100 µM of Acrydite-modified "Rep172" reporter (/5Acryd/TTT TTT TTT TTT TTT TTT TT/i6-FAMK//3Bio/). The mixture was exposed to UV light (365 nm, 200 ms) under a photomask to generate circular cross-section rods of hydrogel containing immobilized reporters. Cas14a.1 effector proteins were complexed with sgRNA for 30 minutes at 37°C. The 1x concentration of proteins was 40 nM and the final concentration of sgRNA was 40 nM. 5 uL of these RNPs was combined with the following components for a final volume of 50 uL (listed at final concentration): 10 uL of target RNaseP RNA (45 pg/uL) or no target control ("(-)"), reporter-immobilized hydrogels (10 hydrogels/uL), IB15 one pot LAMP trans-cleavage buffer, dNTPs (1mM), RNAse inhibitor, Bsm DNA polymerase, Warmstart RTx reverse transcriptase, RNase P LAMP primer mix, and TIPP (0.5 U/uL) or water ("no TIPP"). Reactions were carried out at 55°C for 35 minutes. Trans cleavage activity was detected with lateral flow assay strips. The supernatant for each reaction was applied to the sample pad of a lateral flow assay strip containing anti-FITC conjugate particles (colloidal gold). If trans cleavage occurred, the supernatant contained cleaved FAM-biotin-labeled reporter molecules which bound to an anti-biotin (e.g., streptavidin) target line on the lateral flow strip. The anti-FITC conjugate particles bound the FAM moiety on the reporter molecules and a target band appeared on lateral flow strips at the anti-biotin target line. If trans cleavage did not occur (as in NTC or no guide RNA reactions), the supernatant did not contain any FAM-biotin-labeled molecules, and nothing bound to the anti-biotin target line. The lateral flow assay strip also contained an anti-IgG flow control line, downstream of the anti-biotin target line, which bound to the anti-FITC moiety of the conjugate particles to confirm that the lateral flow assay functioned properly. Lateral flow strips without TIPP are shown in FIG. 66A and lateral flow strips with TIPP are shown in FIG. 66B. The presence of target RNA is indicated with (+) and the absence of target RNA is indicated with (-). As in the case with a fluorescent signal output, lateral flow assay signal was improved by the addition of TIPP.

Without being bound to any particular theory, it is believed that TIPP may be responsible for processing phosphates which are produced when the LAMP reaction uses up dNTPs. Those phosphates can take Mg2+ ions out of solution, which may be cause a Cas enzyme to exhaust the remaining Mg2+ quickly and cause the reaction to plateau more rapidly than would occur with additional Mg2+ in the system. With the presence of TIPP, those phosphates may be processed and the Mg2+ may be released for use by a Cas enzyme, which can improve DETECTR signal strength as the Cas enzyme is able to react unhindered by a lack of Mg2+.

The results show that the addition of TIPP improved signal compared to without TIPP and made detection on lateral flow strips more robust. The amount of TIPP added can be varied to allow flexibility in assay designs.

### Example 19

### SARS-CoV-2 Detection Kits

In some embodiments, the compositions herein can comprise a kit for use in detecting SARS-CoV-2 from samples. In some embodiments, the methods and compositions herein may be formulated to overcome the challenges in SARS detection. For example, a challenge in detecting SARS viruses is that the virus may be present in low concentrations. For example, a buccal swab from a SARS infected patient may only comprise a couple hundred copies of the SARS genome. If such a sample is stored prior to testing, the genome copy number may be less than 100. Further augmenting the challenge of detecting SARS is the inherent instability of SARS RNA, with half-lives on the order of minutes. Thus, a number of major technical challenges have to be overcome to formulate a kit for generalized, celeritous, and facile SARS detection.

In some embodiments, the kit provides reagents for RNA amplification and detection. Given that SARS is often present in low titers, the kit provides reagents for isothermal SARS RNA amplification, split among four vials. In some embodiments, the first three vials contain reverse transcription loop-mediated isothermal amplification (RT-LAMP) reagents targeting the SARS-CoV-2 N-gene, the SARS-CoV-2 E-gene, and human RNase P POP 7. In some embodiments, the SARS-CoV-2 E- and N-genes may comprise a sequence with at least 80% sequence similarity to any one of SEQ ID NOs: 179-184, presented in TABLE 16. The SARS-CoV-2 E- and N-genes serve as two targets for the assay, while the human RNase P POP 7 gene (GGAGTATTGAATAGTTGGGAATTGGAACCCCTCCAGGGGGAACCAAACATTGTCGT TCAGAAGAAGACAAAGAGAGATTGAAATGAAGCTGTTGATTTCAACACACAAATTC TGGTGGTAGATGAAAGCAAAGCAAGTAAGTTTCTCCGAATCCCTAGTCAACTGGAG GTAGAGACGGACTGCGCAGGTTAACTACAGCTCCCAGCATGCCTGAGGGGCGGGCT CAGCGGCTGCGCAGACTGGCGCGCGCGGACGGTCATGGGACTTCAGCATGGCGGTG TTTGCAGATTTGGACCTGCGAGCGGGTTCTGACCTGAAGGCTCTGCGCGGACTTGTG GAGACAGCCGCTCACCTTGGCTATTCAGTTGTTGCTATCAATCATATCGTTGACTTTA AGGAAAAGAAACAGGAAATTGAAAAACCAGTAGCTGTTTCTGAACTCTTCACAACT TTGCCAATTGTACAGGGAAAATCAAGACCAATTAAAATTTTAACTAGATTAACAATT ATTGTCTCGGATCCATCTCACTGCAATGTTTTGAGAGCAACTTCTTCAAGGGCCCGG CTCTATGATGTTGTTGCAGTTTTTCCAAAGACAGAAAAGCTTTTTCATATTGCTTGCA CACATTTAGATGTGGATTTAGTCTGCATAACTGTAACAGAGAAACTACCATTTTACT TCAAAAGACCTCCTATTAATGTGGCGATTGACCGAGGCCTGGCTTTTGAACTTGTCT ATAGCCCTGCTATCAAAGACTCCACAATGAGAAGGTATACAATTTCCAGTGCCCTCA ATTTGATGCAAATCTGCAAAGGAAAGAATGTAATTATATCTAGTGCTGCAGAAAGG CCTTTAGAAATAAGAGGGCCATATGACGTGGCAAATCTAGGCTTGCTGTTTGGGCTC TCTGAAAGTGACGCCAAGGCTGCGGTGTCCACCAACTGCCGAGCAGCGCTTCTCCAT GGAGAAACTAGAAAAACTGCTTTTGGAATTATCTCTACAGTGAAGAAACCTCGGCC ATCAGAAGGAGATGAAGATTGTCTTCCAGCTTCCAAGAAAGCCAAGTGTGAGGGCT GAAAAGAATGCCCCAGTCTCTGTCAGCACTCCCTTCTTCCCTTTTATAGTTCATCAGC CACAACAAAAATAAAACCTTTGTGTGATTTACTGTTTTCATTTGGAGCTAGAAATCA ATAGTCTATAAAAACAGTTTTACTTGCAATCCATTAAAACAACAAACGAAACCTAGT GAAGCATCTTTTTAAAAGGCTGCCAGCTTAATGAATTTAGATGTACTTTAAGAGAGA AAGACTGGTTATTTCTCCTTTGTGTAAGTGATAAACAACAGCAAATATACTTGAATA AAATGTTTCAGGTATTTTTGTTTCATTTTGTTTTTGAGATAGGGTCTTTGTTGCTCAGG CTGGAGTACAGTGGCATAATCACAGCTCACTGCAACCTCAATCCTGGGCTCAAGTGA TCCTCCCGCTTCAGCCTCTCAAGCAGCGGGAACTACAGGTGTGCACTACCACACCTG GCTATTTTTTTTTTTTTTTTTTTTTTCCCTTGTAGAGACATGGTCTCACTATGTTGCTGA GGCTGGTCTCAAACTCCTAGGATCAAGCCATCCTCCCGCTTTGGCCTCCTAAAGTGC TGGGATTACATGAGCCACCACATGCAGCCAGATGTTTGAATATTTTAAGAGCTTCTT TCGAAAGTTTCTTGTTCATACTCAAATAGTAGTTATTTTGAAGATATTCAAACTTATA TTGAAGAAGTGACTTTAGTTCCTCTTGTTTTAAGCTTCTTTCATGTATTCAAATCAGC ATTTTTTTCTAAGAAATTGCTATAGAATTTGTGGAAGGAGAGAGGATACACATGTAA AATTACATCTGGTCTCTTCCTTCACTGCTTCATGCCTACGTAAGGTCTTTGAAATAGG ATTCCTTACTTTTAGTTAGAAACCCCTAAAACGCTAATATTGATTTTCCTGATAGCTG TATTAAAAATAGCAAAGCATCGGACTGA, SEQ ID NO: 220) serves as an internal control. In some embodiments, the buffer in the three amplification reagent vials is pH 8.8 Tris HCl containing (NH4)2SO4, KCl, MgSO4 and Tween-20 detergent. In some embodiments, the buffers are produced with nuclease-free water mixed with RNase inhibitor to prevent target nucleic acid degradation. In some cases, the three RT-LAMP reagent vials contain all of the reagents required for RT-LAMP, including dNTPs, a reverse transcriptase, and a polymerase. In some embodiments, the RT-LAMP reagent vials lack a reverse-transcriptase activator, and may not perform RT-LAMP until mixed with contents from the fourth vial, which contains an RT-LAMP activator mix consisting of 100 mM MgSO4. The contents of the four amplification reagent vials are summarized in TABLE 13.

**TABLE 13 - RT-LAMP Vial Constituents**

| **Vial** | **Constituent** | **SEQ ID NO** | **Concentration in Vial** |
|---|---|---|---|
| | PRIMER: | 300 | 0.26 µM |
| | M1957 F3 2019-nCoV-N-set1 - | | |
| | AACACAAGCTTTCGGCAG | | |
| | PRIMER | 301 | 0.26 µM |
| | M1958 B3 2019-nCoV-N-set1 - | | |
| | GAAATTTGGATCTTTGTCATCC | | |
| | PRIMER | 302 | 2.12 µM |
| Vial 1, SARS-CoV-2 RT-LAMP Master Mix 1 - N Gene | M1959 FIP 2019-nCoV-N-set1 - | | |
| | TGCGGCCAATGTTTGTAATCAGCCA AGGAAATTTTGGGGAC | | |
| | PRIMER | 303 | 2.12 µM |
| | M1960 BIP 2019-nCoV-N-set1 - | | |
| | CGCATTGGCATGGAAGTCACTTTGA TGGCACCTGTGTAG | | |
| | PRIMER | 304 | 1.06 µM |
| | M1961 LF 2019-nCoV-N-set1 - TTCCTTGTCTGATTAGTTC | | |
| | PRIMER | 305 | 1.06 µM |
| | M1962 LB 2019-nCoV-N-set1 - | | |
| | ACCTTCGGGAACGTGGTT | | |
| | dNTPs | - | 1.85 mM |
| | Bst 2.0 Polymerase | - | 1.06 units/µl |
| | Warmstart RTx | - | 0.75 units/µl |
| | RNase Inhibitor | - | 1.06 units/µl |
| | Tris HCl pH 8.8 | - | 26.49 mM |
| | (NH₄)₂SO₄ | - | 13.25 mM |
| | KCl | - | 66.23 mM |
| | MgSO₄ | - | 2.65 mM |
| | Tween 20 | - | 0.13% (v/v) |
| Vial 2, SARS-CoV-2 RT-LAMP Master Mix 2 - E Gene | PRIMER - | 306 | 0.26 µM |
| | M2106 F3 2019-nCoV-E-set13 | | |
| | CCGACGACGACTACTAGC | | |
| | PRIMER - | 307 | 0.26 µM |
| | M2107 B3 2019-nCoV-E-set13 | | |
| | AGAGTAAACGTAAAAAGAAGGTT | | |
| | PRIMER - | 308 | 2.12 µM |
| | M2108 FIP 2019-nCoV-E-set13 | | |
| | ACCTGTCTCTTCCGAAACGAATTTGT AAGCACAAGCTGATG | | |
| | PRIMER - | 309 | 2.12 µM |
| | M2109 BIP 2019-nCoV-E-set13 | | |
| | CTAGCCATCCTTACTGCGCTACTCAC GTTAACAATATTGCA | | |
| | PRIMER - | 310 | 1.06 µM |
| | M2110 LF 2019-nCoV-E-set13 | | |
| | TCGATTGTGTGCGTACTGC | | |
| | PRIMER | 311 | 1.06 µM |
| | M2111 LB 2019-nCoV-E-set13 | | |
| | TGAGTACATAAGTTCGTAC | | |
| | dNTPs | - | 1.85 mM |
| | Bst 2.0 Polymerase | - | 1.06 units/µl |
| | Warmstart RTx | | 0.75 units/µl |
| | RNase Inhibitor | | 1.06 units/µl |
| | Tris HCl pH 8.8 | - | 26.49 mM |
| | (NH₄)₂SO₄ | - | 13.25 mM |
| | KCl | - | 66.23 mM |
| | MgSO₄ | - | 2.65 mM |
| | Tween 20 | - | 0.13% (v/v) |
| Vial, SARS-CoV-2 Control RT-LAMP Master Mix 3 - RNase POP P 7 | PRIMER - | 312 | 0.26 µM |
| | JPB RNase P POP7 F3 | | |
| | TTGATGAGCTGGAGCCA | | |
| | PRIMER - | 313 | 0.26 µM |
| | JPB RNase P POP7 B3 | | |
| | CACCCTCAATGCAGAGTC | | |
| | PRIMER - | 314 | 2.12 µM |
| | JPB RNase P POP7 FIP | | |
| | GTGTGACCCTGAAGACTCGGTTTTA GCCACTGACTCGGATC | | |
| | PRIMER - | 315 | 2.12 µM |
| | JPB RNase P POP7 BIP | | |
| | CCTCCGTGATATGGCTCTTCGTTTTT TTCTTACATGGCTCTGGTC | | |
| | PRIMER - | 316 | 1.06 µM |
| | JPB RNase P POP7 LF | | |
| | ATGTGGATGGCTGAGTTGTT | | |
| | PRIMER - | 317 | 1.06 µM |
| | JPB RNase P POP7 LB | | |
| | CATGCTGAGTACTGGACCTC | | |
| | dNTPs | - | 1.85 mM |
| | Bst 2.0 Polymerase | - | 1.06 units/µl |
| | Warmstart RTx | - | 0.75 units/µl |
| | RNase Inhibitor | - | 1.06 units/µl |
| | Tris HCl pH 8.8 | - | 26.49 mM |
| | (NH₄)₂SO₄ | - | 13.25 mM |
| | KCl | - | 66.23 mM |
| | MgSO₄ | - | 2.65 mM |
| | Tween 20 | - | 0.13% (v/v) |
| SARS-CoV-2 Amp Activator | Bulk 100 mM MgSO₄ | - | 100 mM |

In some embodiments, a kit for detecting a target nucleic acid (e.g., a SARS-CoV-2 nucleic acid) comprises a support medium (e.g., a 384-well block), optionally a reverse transcriptase (e.g., in cases where the target nucleic acid is an RNA molecule), a polymerase (e.g., Bst 2.0 polymerase), a primer (e.g., a primer that targets or is upstream from the target nucleic acid sequence), a guide nucleic acid targeting a sequence from the target nucleic acid, a programmable nuclease (e.g., a programmable nuclease with SEQ ID NO: 18), and a reporter (e.g., a reporter nucleic acid comprising a fluorophore-quencher pair and SEQ ID NO: 9). In some embodiments, the primer may comprise a sequence selected from among the group consisting of SEQ ID NO: 300-317. The guide nucleic acid may comprise a sequence selected from among the group consisting of SEQ ID NO: 318 - SEQ ID NO: 327 presented in TABLE 15. The guide nucleic acid may comprise a sequence comprising at least 80% identity to any one of SEQ ID NO: 318 - SEQ ID NO: 327. In some embodiments, the target gene for detection of coronaviruses may comprise a sequence of any one of SEQ ID NOs: 179-184 listed in TABLE 16.

For detection, the kit contains DETECTR reagents split between three vials. Each vial contains SEQ ID NO: 18 and a reporter containing the dye-quencher pair Alexa Fluor 594-3' Iowa Black^{®} RQ. The three vials contain separate guide nucleic acids targeting the SARS-CoV-2 N-gene, the SARS-CoV-2 E-gene, or the human RNase gene POP P 7. The constituents in each vial are dissolved in pH 7.5 HEPES buffer containing potassium acetate, magnesium acetate, glycerol and the detergent Triton-X. The compositions of the DETECTR vials are summarized in TABLE 14.

**TABLE 14 - DETECTR Vial Constituents**

| **Vial** | **Constituent** | **SEQ ID NO** | **Concentration in Vial** |
|---|---|---|---|
| Vial 5, SARS-CoV-2 DETECTR Master Mix 1 - N Gene | R1763 CDC-N2-Wuhan Guide RNA | 318 | 44.4 nM |
| | UAAUUUCUACUAAGUGUAGAUCCCCC AGCGCUUCAGCGUUC | | |
| | Bulk, **SEQ ID NO:** 18 | | 44.4 nM |
| | HEPES pH 7.5 | | 0.5 M |
| | Potassium Acetate | | 2.2 mM |
| | Magnesium Acetate | | 5.6 mM |
| | Glycerol | | 1.1% |
| | Triton-X-100 | | 0.00018% |
| | Reporter Nucleic Acid - rep33 /AF594/**SEQ ID NO:** 9/3IAbRQSp/ | | 111.11 nM |
| Vial 6, SARS-CoV-2 DETECTR Master Mix 2 - E-Gene | Vial, SARS-CoV-2 DETECTR Master Mix 2 - E Gene | 319 | 44.4 nM |
| | UAAUUUCUACUAAGUGUAGAUGUGG UAUUCUUGCUAGUUAC | | |
| | Bulk, **SEQ ID NO:** 18 | | 44.4 nM |
| | HEPES pH 7.5 | | 0.5 M |
| | Potassium Acetate | | 2.2 mM |
| | Magnesium Acetate | | 5.6 mM |
| | Glycerol | | 1.1% |
| | Triton-X-100 | | 0.00018% |
| | Reporter - rep33 /AF594/TTATTATT/3IAbRQSp/ | | 111.11 nM |
| Vial 7, SARS-CoV-2 DETECTR Master Mix - RNase POP P7 | Vial, SARS-CoV-2 DETECTR Master Mix 2 - RNAse P | 320 | 44.4 nM |
| | UAAUUUCUACUAAGUGUAGAUAAUU ACUUGGGUGUGACCCU | | |
| | Bulk, **SEQ ID NO**: 18 | | 44.4 nM |
| | HEPES pH 7.5 | | 0.5 M |
| | Potassium Acetate | | 2.2 mM |
| | Magnesium Acetate | | 5.6 mM |
| | Glycerol | | 1.1% |
| | Triton-X-100 | | 0.00018% |
| | Reporter - rep33 | | 111.11 nM |
| | /AF594/TTATTATT/3IAbRQSp/ | | |

In some embodiments, the kit also contains the equipment needed to perform the SARS-CoV-2 detection assay. In some embodiments, the kit provides a 96 well plate for the RT-LAMP reactions and a 384 well plate for the DETECTR reactions, as well as films for sealing the well plates (ultra-Clear Polyester Heat-Resistant Films). In some embodiments, the kit further provides a set of pipettes and pipette tips for reagent transfer during the assay.

In some embodiments, as an internal control, the kit provides a vial containing genomic DNA comprising the human RNAse POP P 7 gene. In some embodiments, a control can be run in parallel using this DNA sample the kit is used to assay for SARS-CoV-2. In some embodiments, the kit thus allows a single user to perform a large number of parallel assays for SARS-CoV-2 genomic markers.

### EXAMPLE 20

### Assay for Detecting SARS-CoV-2

In some embodiments, the methods and compositions herein outline an assay for using the kit from example 18 to detect SARS-CoV-2 from a sample. In some embodiments, the assay involves an initial RT-LAMP reaction that produces DNA amplicons from SARS genomic RNA, followed by a DETECTR reaction to determine whether SARS-Cov-2 was present in the assayed sample, to overcome the challenge of a low concentration of the SARS virus. In some embodiments, the assay also involves RT-LAMP and DETECTR on a control sample to enable easier visualization of the SARS DETECTR reaction results.

In some embodiments, the kit can be stably stored by -25 °C and -15 °C, and may need to be thawed at room temperature for 30 minutes prior to use. In some embodiments, once the reagents have thawed, the amplification master mixes are prepared by mixing 13 µl aliquots of Amp-A (vial 4) into 13 µl aliquots of Amp-N (vial 1), Amp-E (vial 2), and Amp-RP (vial 3). In some embodiments, the amplification master mixes are vortexed and spun, and then dispensed in 8 µl portions into individual wells in the 96- or 384- well plates. In some embodiments, RNAs from the biological samples are extracted using spin columns, beads or just crude extractions and 2 µl of the extracted sample is added to each well containing amplification reagents, and the well plates are sealed with film, spun down, and then heated to 62 °C for 30 minutes to enable the RT-LAMP reactions.

In some embodiments, the products of the RT-LAMP reactions are then transferred to a separate well plate pre-loaded with DETECTR mix. Each well is loaded with 18 µl DETECTR reagents from one of vials 5, 6 or 7 (targeting the SARS-CoV-2 N-gene, the SARS-Cov-2 E-gene, and RNase POP P 7, respectively) and 2 µl amplification products. In some embodiments, the plate is then sealed with film, spun down for 30 s at 2000 rcf, and heated to 37 °C to enable the DETECTR reaction. In some embodiments, the progress of the DETECTR reactions can be monitored with a commercial fluorimeter (e.g., with a ThermoFisher QS5).

**TABLE 15: Exemplary gRNA Sequences for Detection of Coronaviruses**

| **SEQ ID NO:** | gRNA | Target | Sequence |
|---|---|---|---|
| **SEQ ID NO: 318** | R1763 | CDC-N2-Wuhan | UAAUUUCUACUAAGUGUAGAUCCCC CAGCGCUUCAGCGUUC |
| **SEQ ID NO: 321** | R1764 | E-Sarbeco-1 | UAAUUUCUACUAAGUGUAGAUUUGC UUUCGUGGUAUUCUUG |
| **SEQ ID NO: 319** | R1765 | E-Sarbeco-2 | UAAUUUCUACUAAGUGUAGAUGUGG UAUUCUUGCUAGUUAC |
| **SEQ ID NO: 322** | R1766 | CDC-N2-SARS | UAAUUUCUACUAAGUGUAGAUCUCC AAGUGCCUCUGCAUUC |
| **SEQ ID NO: 323** | R1767 | N-Sarbeco-1 | UAAUUUCUACUAAGUGUAGAUGCAA UGUUGUUCCUUGAGGA |
| **SEQ ID NO: 324** | R1768 | ORF1ab-Wuhan | UAAUUUCUACUAAGUGUAGAUCACA UACCGCAGACGGUACA |
| **SEQ ID NO: 325** | R1769 | CDC-RNaseP | UAAUUUCUACUAAGUGUAGAUGACC UGCGAGCGGGUUCUGA |
| **SEQ ID NO: 320** | R779 | RNaseP POP7 | UAAUUUCUACUAAGUGUAGAUAAUU ACUUGGGUGUGACCCU |
| **SEQ ID NO: 326** | R1965 | RNaseP POP7 v2 | UAAUUUCUACUAAGUGUAGAUUUAC AUGGCUCUGGUCCGAG |
| **SEQ ID NO: 327** | R780 | RNaseP POP7 v3 | UAAUUUCUACUAAGUGUAGAUGGCT TCCAGGGAACAGGCCT |

**TABLE 16: Exemplary Coronavirus N-Gene and E-Gene Gene Fragments**

| **SEQ ID NO:** | Target | Sequence |
|---|---|---|
| **SEQ ID NO: 179** | 2019-nCoV N-gene | CCAAATTGGCTACTACCGAAGAGCTACCAGACGAATTCGTGGTGG TGACGGTAAAATGAAAGATCTCAGTCCAAGATGGTATTTCTACTA CCTAGGAACTGGGCCAGAAGCTGGACTTCCCTATGGTGCTAACAA AGACGGCATCATATGGGTTGCAACTGAGGGAGCCTTGAATACAC CAAAAGATCACATTGGCACCCGCAATCCTGCTAACAATGCTGCAA TCGTGCTACAACTTCCTCAAGGAACAACATTGCCAAAAGGCTTCT ACGCAGAAGGGAGCAGAGGCGGCAGTCAAGCCTCTTCTCGTTCCT CATCACGTAGTCGCAACAGTTCAAGAAATTCAACTCCAGGCAGCA GTAGGGGAACTTCTCCTGCTAGAATGGCTGGCAATGGCGGTGATG CTGCTCTTGCTTTGCTGCTGCTTGACAGATTGAACCAGCTTGAGA GCAAAATGTCTGGTAAAGGCCAACAACAACAAGGCCAAACTGTC ACTAAGAAATCTGCTGCTGAGGCTTCTAAGAAGCCTCGGCAAAA ACGTACTGCCACTAAAGCATACAATGTAACACAAGCTTTCGGCAG ACGTGGTCCAGAACAAACCCAAGGAAATTTTGGGGACCAGGAAC TAATCAGACAAGGAACTGATTACAAACATTGGCCGCAAATTGCA CAATTTGCCCCCAGCGCTTCAGCGTTCTTCGGAATGTCGCGCATT GGCATGGAAGTCACACCTTCGGGAACGTGGTTGACCTACACAGGT GCCATCAAATTGGATGACAAAGATCCAAATTTCAAAGATCAAGTC ATTTTGCTGAATAAGCATATTGACGCATACAAAACATTCCCACCA ACAGAGCCTAAAAAGGACAAAAAGAAGAAGGCTGATGAAACTC AAGCCTTACCGCAGAGACAGAAGAAACAGCAAACTGTG |
| **SEQ ID NO: 180** | SARS-CoV N-gene | CCAAATTGGCTACTACCGAAGAGCTACCCGACGAGTTCGTGGTGG TGACGGCAAAATGAAAGAGCTCAGCCCCAGATGGTACTTCTATTA CCTAGGAACTGGCCCAGAAGCTTCACTTCCCTACGGCGCTAACAA AGAAGGCATCGTATGGGTTGCAACTGAGGGAGCCTTGAATACAC CCAAAGACCACATTGGCACCCGCAATCCTAATAACAATGCTGCCA CCGTGCTACAACTTCCTCAAGGAACAACATTGCCAAAAGGCTTCT ACGCAGAGGGAAGCAGAGGCGGCAGTCAAGCCTCTTCTCGCTCC TCATCACGTAGTCGCGGTAATTCAAGAAATTCAACTCCTGGCAGC AGTAGGGGAAATTCTCCTGCTCGAATGGCTAGCGGAGGTGGTGA AACTGCCCTCGCGCTATTGCTGCTAGACAGATTGAACCAGCTTGA GAGCAAAGTTTCTGGTAAAGGCCAACAACAACAAGGCCAAACTG TCACTAAGAAATCTGCTGCTGAGGCATCTAAAAAGCCTCGCCAAA AACGTACTGCCACAAAACAGTACAACGTCACTCAAGCATTTGGG AGACGTGGTCCAGAACAAACCCAAGGAAATTTCGGGGACCAAGA CCTAATCAGACAAGGAACTGATTACAAACATTGGCCGCAAATTGC ACAATTTGCTCCAAGTGCCTCTGCATTCTTTGGAATGTCACGCATT GGCATGGAAGTCACACCTTCGGGAACATGGCTGACTTATCATGGA GCCATTAAATTGGATGACAAAGATCCACAATTCAAAGACAACGT CATACTGCTGAACAAGCACATTGACGCATACAAAACATTCCCACC AACAGAGCCTAAAAAGGACAAAAAGAAAAAGACTGATGAAGCT CAGCCTTTGCCGCAGAGACAAAAGAAGCAGCCCACTGTG |
| **SEQ ID NO: 181** | bat-SL-CoVZC 45 N-gene | CCAAATTGGCTACTACCGTAGAGCTACCAGACGAATTCGTGGTGG TGACGGTAAAATGAAAGAGCTCAGCCCCAGATGGTATTTTACTA TCTAGGAACTGGACCAGAAGCTGGACTTCCCTATGGTGCTAACAA AGAAGGCATCATATGGGTTGCAACTGAGGGAGCCTTAAACACAC CGAAAGACCACATTGGCACCCGCAATCCTGCTAACAATGCTGCAA TCGTGCTACAACTTCCTCAAGGAACAACATTGCCAAAAGGCTTCT ACGCAGAAGGGAGCAGAGGCGGCAGTCAAGCTTCTTCACGCTCC TCATCACGTAGTCGCAACAGTTCAAGAAACTCAACTCCAGGCAGC AGTAGGGGAACTTCTCCTGCTAGAATGGCTGGCAATGGCGGTGAC ACTGCTCTTGCTTTGCTGCTGCTAGATAGGTTGAACCAGCTTGAG AACAAAGTATCTGGCAAAGGCCAACAACAACAGGGCCAAACTGT CACTAAGAAATCTGCTGCTGAGGCATCTAAAAAGCCTCGCCAAA AACGTACTGCTACAAAACAGTACAACGTCACTCAAGCATTTGGGA GACGTGGTCCAGAACAAACCCAAGGAAATTTTGGGGACCAAGAA TTAATCAGACAAGGAACTGATTACAAACATTGGCCGCAAATTGCA CAATTTGCTCCAAGTGCCTCTGCATTCTTTGGAATGTCACGCATTG GCATGGAAGTCACACCTTCGGGAACATGGCTGACTTATCATGGAG CCATTAAATTGGATGACAAAGATCCACAATTCAAAGATAACGTCA TACTGCTGAATAAGCACATTGACGCATACAAAACATTCCCACCAA CAGAGCCTAAAAAGGACAAAAAGAAAAAGGCTGATGAACTTCAG GCTTTACCGCAGAGACAGAAGAAACAACAAACTGTG |
| **SEQ ID NO: 182** | 2019-nCoV E-gene | ACTATTACCAGCTGTACTCAACTCAATTGAGTACAGACACTGGTG TTGAACATGTTACCTTCTTCATCTACAATAAAATTGTTGATGAGCC TGAAGAACATGTCCAAATTCACACAATCGACGGTTCATCCGGAGT TGTTAATCCAGTAATGGAACCAATTTATGATGAACCGACGACGAC TACTAGCGTGCCTTTGTAAGCACAAGCTGATGAGTACGAACTTAT GTACTCATTCGTTTCGGAAGAGACAGGTACGTTAATAGTTAATAG CGTACTTCTTTTTCTTGCTTTCGTGGTATTCTTGCTAGTTACACTAG CCATCCTTACTGCGCTTCGATTGTGTGCGTACTGCTGCAATATTGT TAACGTGAGTCTTGTAAAACCTTCTTTTACGTTTACTCTCGTGTT AAAAATCTGAATTCTTCTAGAGTTCCTGATCTTCTGGTCTAAACG AACTAAATATTATATTAGTTTTTCTGTTTGGAACTTTAATTTTAGC CATGGCAGATTCCAACGGTACTATTACCGTTGAAGAGCTTAAAAA GCTCCTTGAACAATGGAACCTAGTAATAGGTTTCCTATTCCTTAC ATGGATT |
| **SEQ ID NO: 183** | SARS-CoV E-gene | TTTACTACCAGCTTGAGTCTACACAAATTACTACAGACACTGGTA TTGAAAATGCTACATTCTTCATCTTTAACAAGCTTGTTAAAGACCC ACCGAATGTGCAAATACACACAATCGACGGCTCTTCAGGAGTTGC TAATCCAGCAATGGATCCAATTTATGATGAGCCGACGACGACTAC TAGCGTGCCTTTGTAAGCACAAGAAAGTGAGTACGAACTTATGTA CTCATTCGTTTCGGAAGAAACAGGTACGTTAATAGTTAATAGCGT ACTTCTTTTTCTTGCTTTCGTGGTATTCTTGCTAGTCACACTAGCC ATCCTTACTGCGCTTCGATTGTGTGCGTACTGCTGCAATATTGTTA ACGTGAGTTTAGTAAAACCAACGGTTTACGTCTACTCGCGTGTTA AAAATCTGAACTCTTCTGAAGGAGTTCCTGATCTTCTGGTCTAAA CGAACTAACTATTATTATTATTCTGTTTGGAACTTTAACATTGCTT ATCATGGCAGACAACGGTACTATTACCGTTGAGGAGCTTAAACAA CTCCTGGAACAATGGAACCTAGTAATAGGTTTCCTATTCCTAGCC TGGATT |
| **SEQ ID NO: 184** | bat-SL-CoVZC 45 E-gene | ATTACTACCAGCTGTACTCAACACAAGTGAGTACAGACACTGGTG TTGAACATGTTACTTTCTTCATCTACAATAAAATTGTTGATGAGCC TGAAGAACATGTTCAAATTCACACAATCGACGGTACATCTGGAGT TGTTAATCCAGCAATGGAACCAATTTATGATGAACCGACGACGAC TACTAGCGTGCCTTTGTAAGCACAAGCTGATGAGTACGAACTTAT GTACTCATTCGTTTCGGAAGAGACAGGTACGTTAATAGTTAATAG CGTACTTCTTTTTCTTGCTTTTGTGGTATTCTTGCTAGTCACACTAG CCATCCTTACTGCGCTTCGATTGTGTGCGTACTGCTGCAATATTGT TAACGTGAGTCTTGTAAAACCTTCTTTTACGTTTACTCTCGTGTT AAAAATCTGAATTCTTCTAGAGTTCCTGATCTTTTGGTCTAAACGA ACTAAATATTATATTAGTCTTTCTGTTTGGAACTTTAATTTTAGCC ATGTCAGGTGACAACGGTACCATTACCGTTGAAGAGCTTAAAAA GCTCTTAGAACAATGGAACCTAGTAATAGGATTCTTGTTTCTTAC ATGGATT |

### EXAMPLE 21

### Assay Testing using Bead Immobilized Reporters following HotPot Protocol

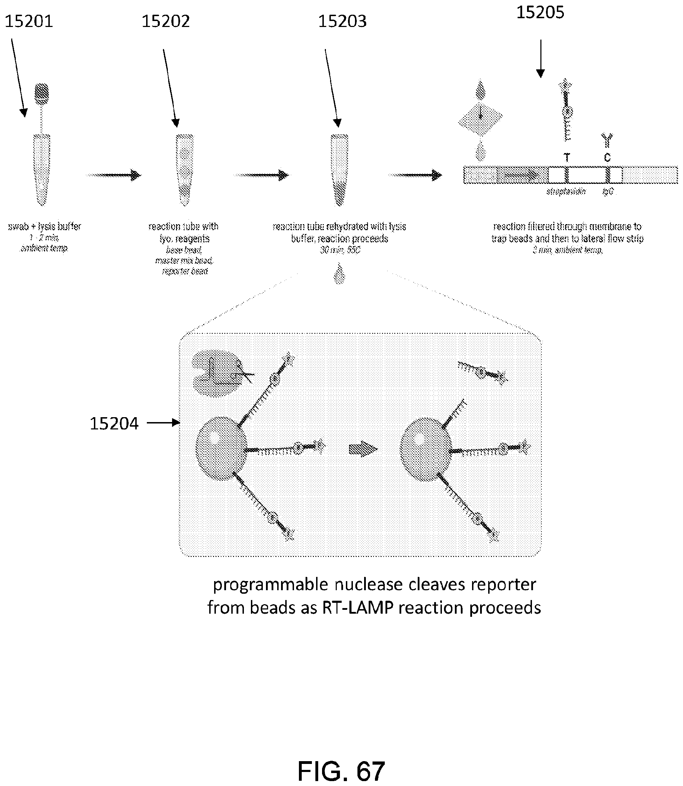

**FIG. 67** shows a drawing illustrating the manual HotPot experimental protocol used to test bead-immobilized reporter cleavage. A sample containing target nucleic acids was added to a tube containing lysis buffer (15201). After lysing 1-2 minutes at ambient temperature, the solution was transferred to a reaction tube (15202) containing lyophilized reagents (i.e., the base bead, the master mix bead, and the reporter bead). Contents of the reaction tube were rehydrated and reconstituted with lysis buffer, and the HotPot reaction was started and maintained at 55 °C for 30 minutes (15203). During the reaction, programmable nucleases in the solution cleaved reporter molecules from the beads at the same time as an RT-LAMP reaction proceeded to amplify the target nucleic acids (15204). The reaction medium was then filtered through a membrane to trap the beads and a first portion of the filtered product was used to measure fluorescence thereof on a fluorescence reader. A second portion of the filtered product was applied to a sample pad of a lateral flow strip. The lateral flow strip included a target capture area (T) comprising streptavidin and a control area (C) comprising IgG. The lateral flow strip assay was allowed to run for 3 minutes at ambient temperature (15205) before pictures were taken of the resulting bands at the target capture area T and the control area C.

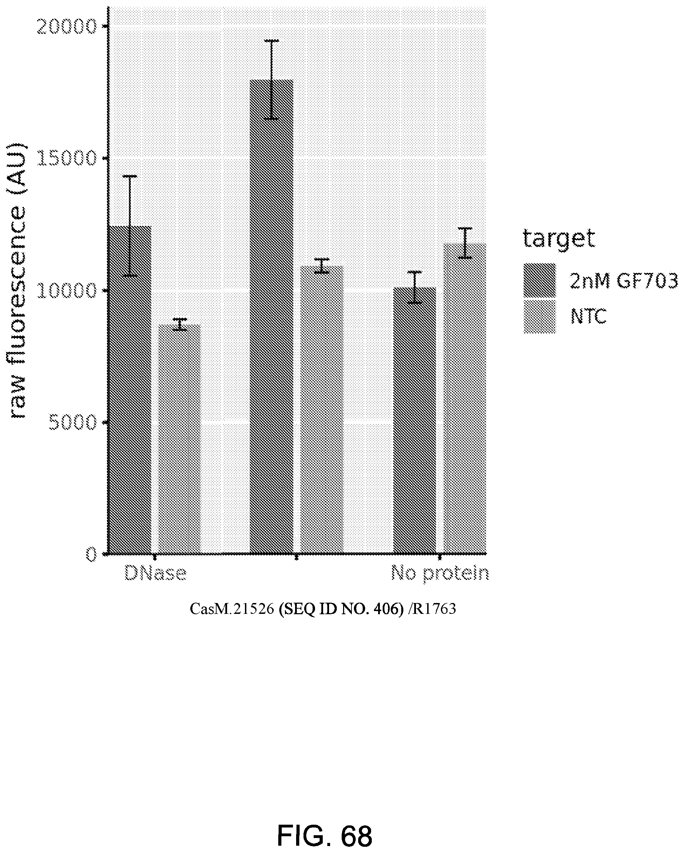

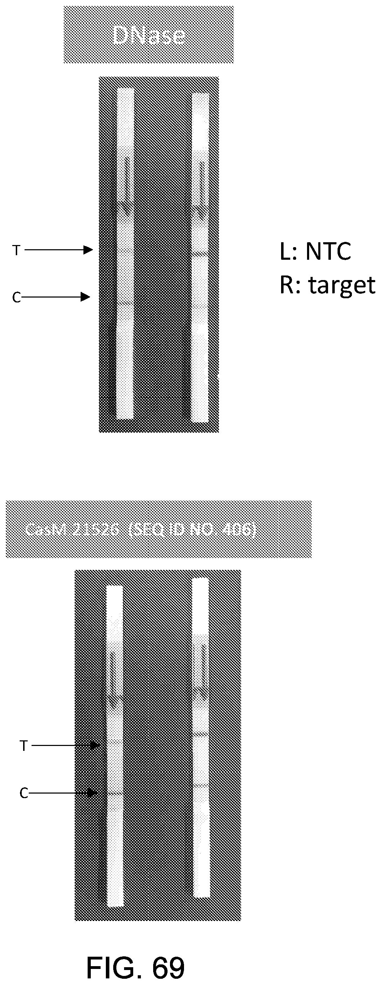

**FIG. 68** shows fluorescence DETECTR results with reporters immobilized onto glass beads. Experiments with both DNase and CasM.21526 **(SEQ ID NO. 406)**/R1763 showed larger fluorescence signal in the presence of target nucleic acids (2 nM, GF703) compared to the no target control experiments (NTC), thus the HotPot DETECTR reaction successfully cleaved the immobilized reporters from the glass beads. Experiments with CasM.21526 **(SEQ ID NO. 406)** were carried out at 55 °C with H2.B buffer. Experiments with DNase were carried out at 37 °C with 1x Turbo DNase buffer. **FIG. 69** shows photographs of the lateral flow strips to which the DNase and CasM.21526 **(SEQ ID NO. 406)** samples from **FIG. 68** were applied.

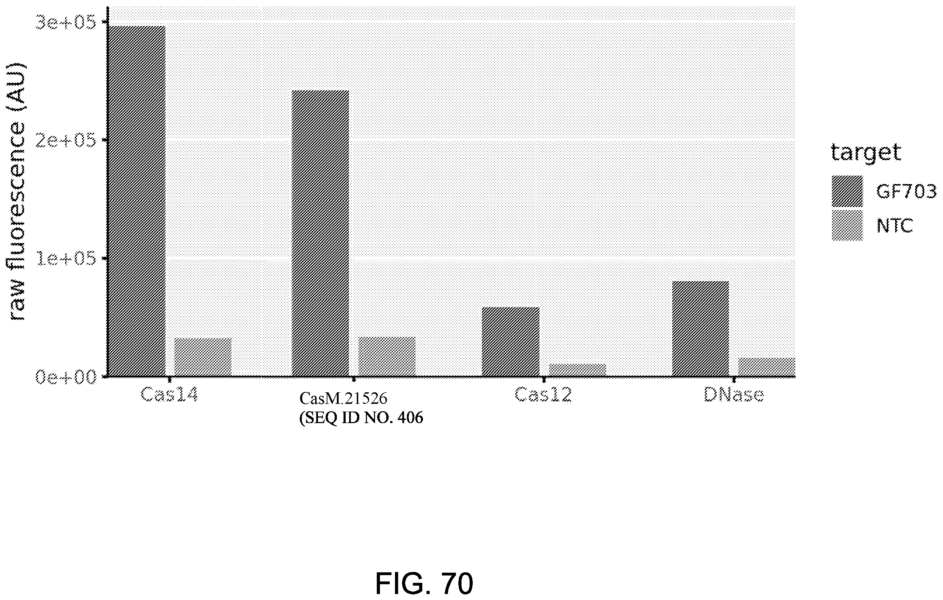

**FIG. 70** shows results with maleimide-coated magnetic beads immobilized with thiol-FAM reporter. Experiments with each protein (a Cas14 variant **(SEQ ID NO:** 63), CasM.21526 **(SEQ ID NO. 406),** a Cas12 variant **(SEQ ID NO:** 28)) resulted in larger signals with target nucleic acids (GF703) compared to the no target control experiments (NTC), thus the HotPot DETECTR reaction successfully cleaved the immobilized reporters from the maleimide-coated magnetic beads.

### Example 22

### HotPot DETECTR-based assay for respiratory virus targets

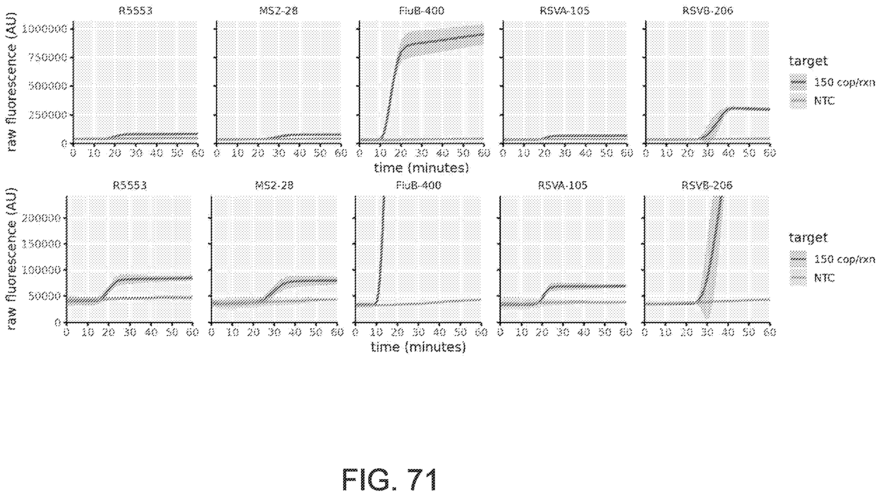

**FIG. 71** shows the results HotPot DETECTR-based assays using the protocol shown in **FIG. 67** with Cas14a.1 **(SEQ ID NO:** 63) for multiple different respiratory virus target nucleic acids including SARS-CoV-2, MS2, FluB, RSV-A, and RSV-B and fluorescence-based readout. Plots in the top row and the plots in the bottom row show the same data, with different y-axis scale. Each plot shows the raw fluorescence measured during the assay of unique nucleic acid sequences as a function of time. Depending on the target, the saturation signal strength varied from about 70000 AUs to 1000000 AUs, and the saturation time was reached in between about 20 to about 40 minutes (SARS-CoV-2 = 25 min, MS2 = 35 min, FluB = 20 min, RSV-A = 25 min, RSV-B = 40 min). Depending on the target, the signal strength from the HotPot DETECTR assay ranged from about 1x to about 25x the signal strength of the SARS-CoV-2 reaction (SARS-CoV-2 = 1x, MS2 = 1x, FluB = 25x, RSV-A = 0.9X, RSV-B = 7x).

### Example 23

### HotPot Limit of Detection (LOD) assay for SARS-CoV-2 target

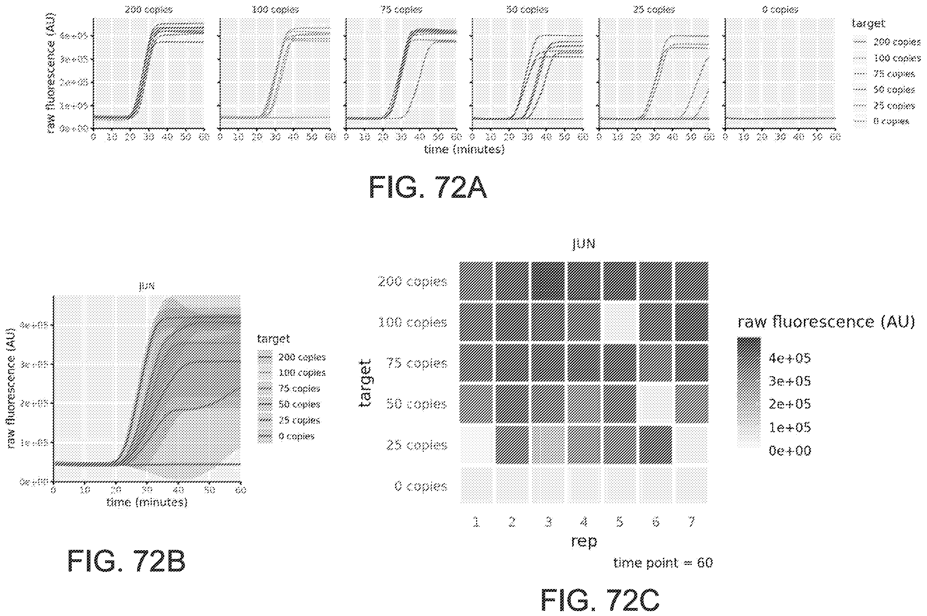

The following describes experiments carried out to determine limit of detection for HotPot reactions using CasM.21526 **(SEQ ID NO. 406).** The HotPot DETECTR-based assays were run using the protocol shown in **FIG. 67** and a fluorescence-based readout. **FIG. 72A** shows results of experiments carried out using IB 15 buffer with different target concentrations ranging from 200 copies to 0 copies. Multiple replicates were conducted at each concentration, and the results of each replicate are shown as individual lines in the plots. **FIG. 72B** shows an aggregated plot of the data in **FIG. 72A** where the average and the error from the multiple replicates are plotted in a single plot. **FIG. 72C** shows the saturation fluorescence heatmap of the multiple replicates (from 1 to 7) of **FIG. 72A** at different target concentrations. Strong signal was produced at all target concentrations, with about 5 copies being the LOD for the experimental conditions tested.

### Example 24

### Additive screening for SARS-CoV-2 target HotPot

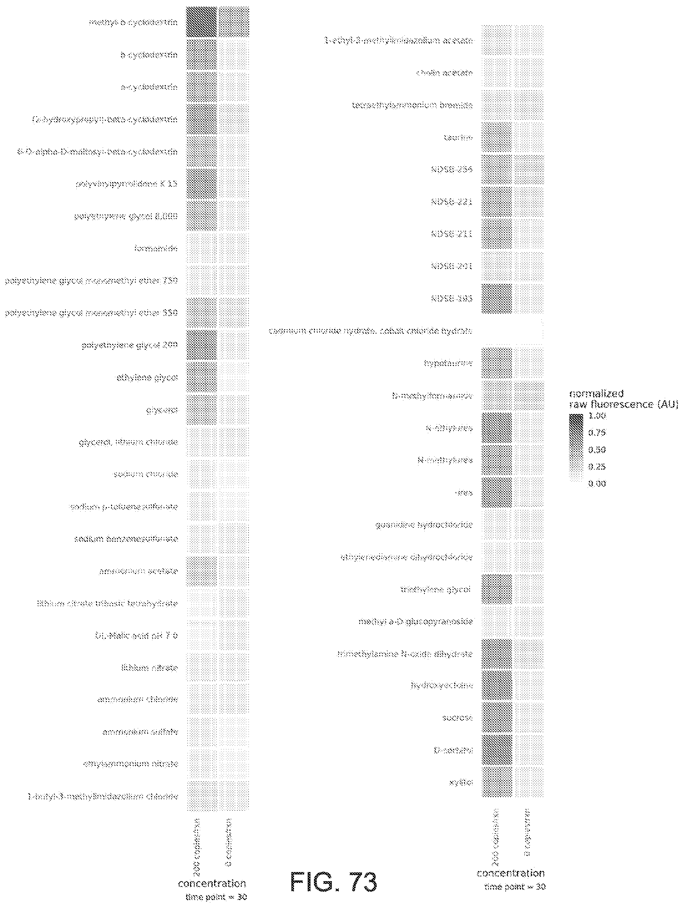

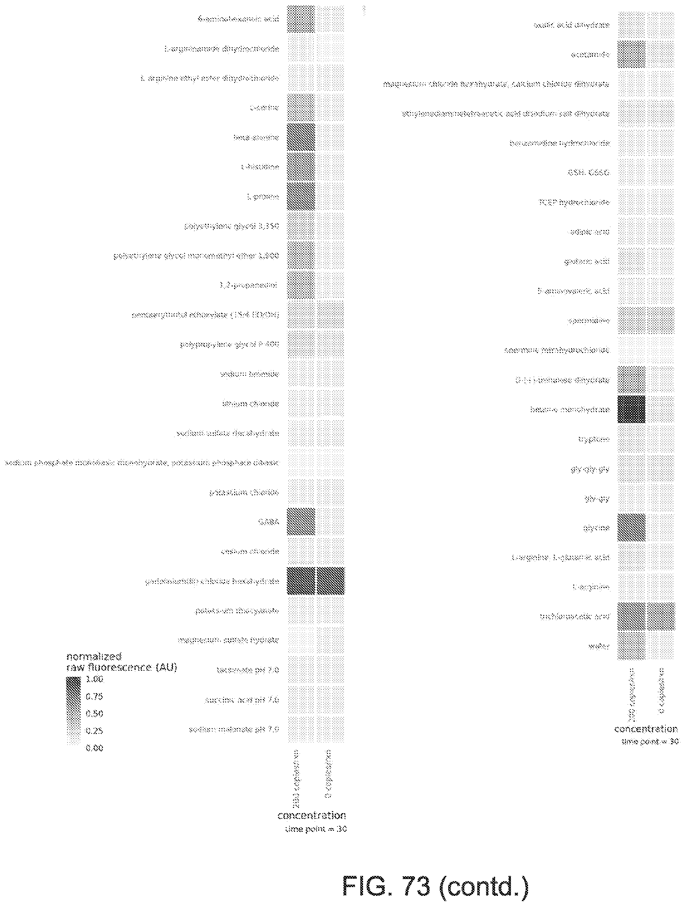

These experiments were conducted to identify potential additives for HotPot assays using Cas14a.1 **(SEQ ID NO:** 63). 96 potential additives were identified and individually screened for their influence on the output fluorescence. **FIG. 73** shows the fluorescence detected from the assays when using a target concentration of 200 input copies/reaction (left column) and when using a target concentration of 0 copies/reaction (i.e., no target, right column). The color intensity of the squares in the heatmap indicate the strength of the fluorescence detected.

Additives that were identified to increase the speed of the reaction under the conditions tested included: betaine monohydrate, acetamide, GABA, L-proline, beta-alanine, 6-aminohexanoic acid, urea, methylurea, ethylurea, hypotaurine, NDSB-256, and ammonium acetate.

Additives that were identified to increase the signal strength from the reaction under the conditions tested included: trehalose, xylitol, D-sorbitol, sucrose, and trimethylamine N-oxide dihydrate.

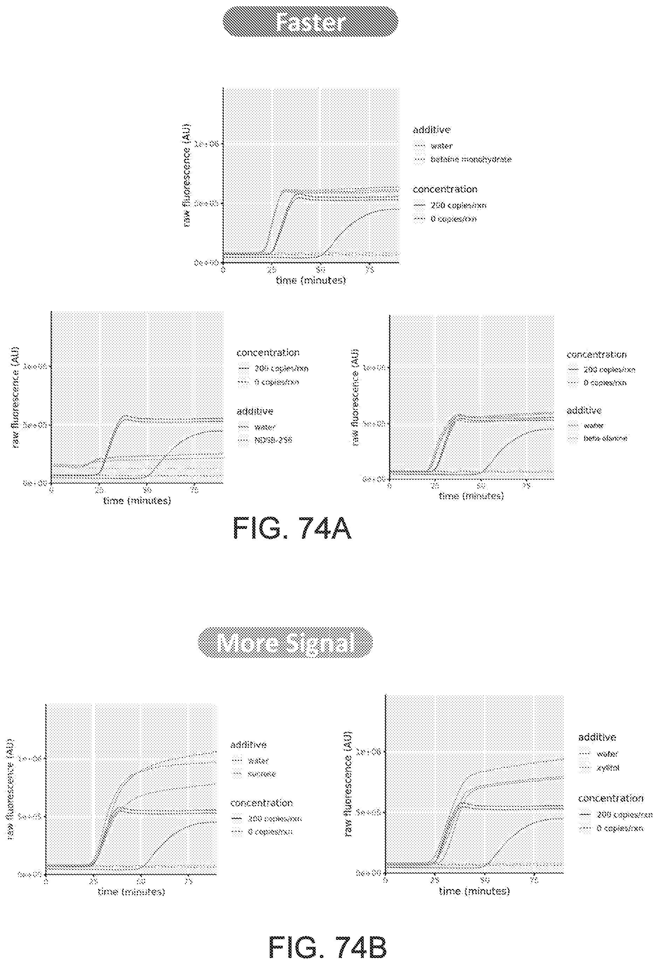

**FIG. 74A** shows the influence of some additives that increased the speed of the reaction under the conditions tested. The influence of each additive (e.g., betaine monohydrate, NDSB-256, and beta-alanine) is shown against control experiments carried out with added water (i.e., without additive). Experiments carried out with no target (i.e., 0 copies/reaction) showed negligible fluorescence signals. In each scenario, the presence of the additives reduced the amount of time it took for fluorescence signals to reach saturation point (i.e., a plateau in the plot).

**FIG. 74B** shows the influence of some additives that increased the signal strength of the reaction under the conditions tested. The influence of each additive (e.g., sucrose and xylitol) is shown against control experiments carried out with added water (i.e., without additive). Experiments carried out with no target (i.e., 0 copies/reaction) showed negligible fluorescence signal. In each scenario, the presence of the additives increased the overall fluorescence signal strength generated in the presence of targets.

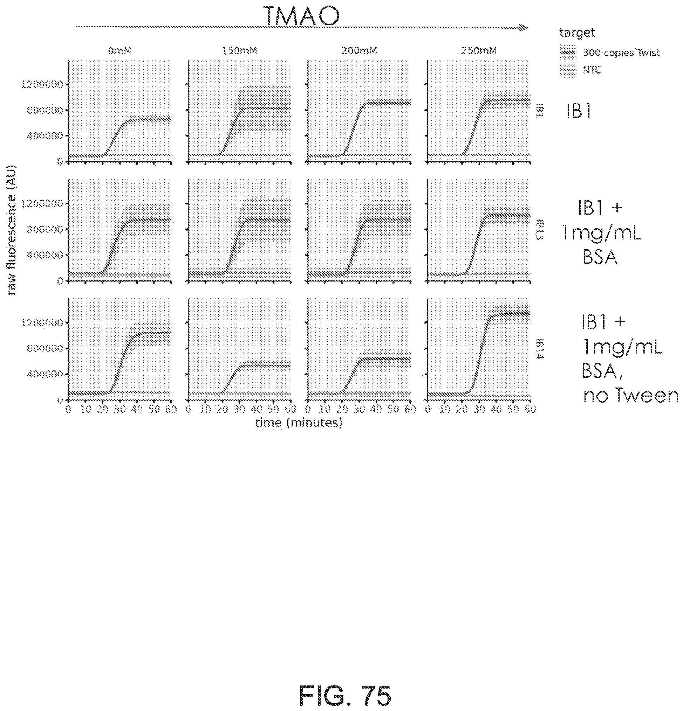

**FIG. 75** shows HotPot DETECTR results for various combinations of additives with 300 copies/reaction of Twist SC2 RNA or with no target controls (NTC). Experiments were conducted in the presence of different amounts of trimethylamine N-oxide dihydrate (TMAO, concentrations shown on top of columns), in combination with IB1 buffer (top row), IB13 (compring IB1 buffer plus 1 mg/ml bovine serum albumin, middle row), or IB14 (compring IB1 buffer plus 1 mg/ml bovine serum albumin and no Tween, bottom row). All conditions tested exhibited strong DETECTR signals, with 250mM TMAO appearing to provide the strongest signal in all buffer conditions.

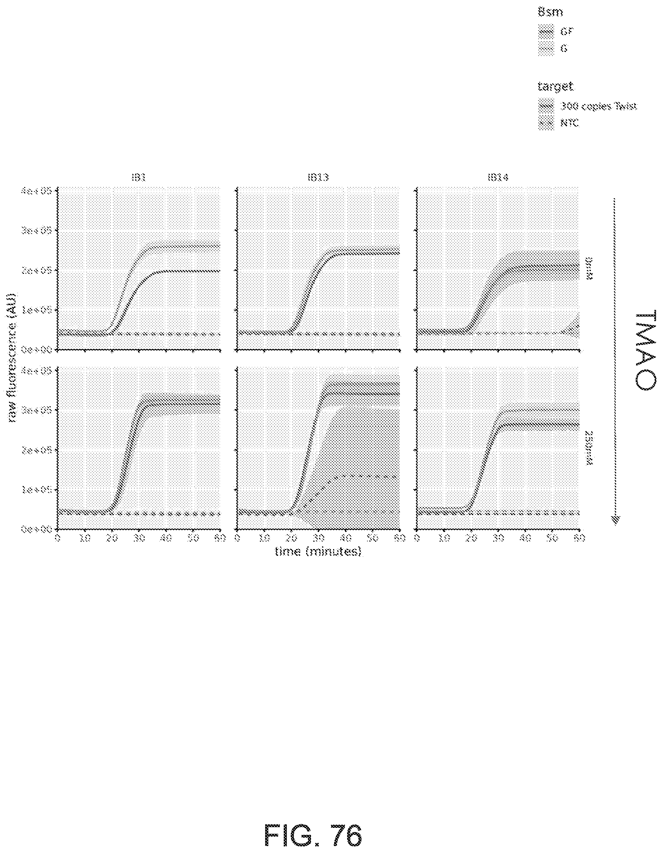

Further HotPot experiments were carried out to determine the effects of using glycerol free (GF) Bsm DNA polymerase and glycerol (G) containing Bsm DNA polymerase with or without 250mM TMAO added. **FIG. 76** shows that both G and GF Bsm DNA polymerases were able to output satisfactory fluorescence signals with each formulation.

### Example 25

### DETECTR-based OnePot and HotPot reactions using reporter immobilization within hydrogels

These experiments were carried out to synthesize hydrogels containing immobilized reporters co-polymerized with a mixture of oligomers as described in **FIG. 78** and **FIGS. 79A-****79B** and determine their applicability for OnePot and HotPot DETECTR assays. **FIG. 78** illustrates the hydrogel structure with a covalently incorporated reporter that was generated via co-polymerization with the reporter.

Reporter was covalently incorporated into PEG hydrogels during polymerization. A 2:1 ratio mixture of unfunctionalized PEG (MW=600 monomers) and PEG-diacrylate (MW=700 monomers) were mixed together with a photoinitiator (2-Hydroxy-2-methylpropiophenone (Darocur 1173)) and 100 µM of Acrydite-modified Reporter 172 (/5Acryd/TTT TTT TTT TTT TTT TTT TT/i6-FAMK//3Bio/). The mixture was exposed to UV light (365 nm, 200 ms) under a photomask. The mask was configured to polymerize the mix into circular cross-sectional rods of hydrogel 400 µm in diameter. Excess material was washed off hydrogels after polymerization. The acrydite group on the 5' end of the reporter was covalently reacted with the acrylate groups of PEG-diacrylate oligomers during co-polymerization in order to incorporate the reporter into the hydrogel.

OnePot (using a Cas12 enzyme, **SEQ ID NO:** 28) and HotPot (using Cas14a.1, **SEQ ID NO:** 63) DETECTR reactions were run as described herein by applying the programmable nuclease complexes and target nucleic acids to a tube containing the hydrogels. 6 hydrogels/reaction were added for OnePot DETECTR and 10 hydrogels/reaction for Cas14a.1 HotPot DETECTR assays. DETECTR reactions were run for 60 min at 37 °C with mixing for Cas 12 OnePot or 60 min at 55 °C with mixing for Cas14a.1 HotPot. Duplicate reactions were run for each of a target RNA and the NTC for both Cas 12 OnePot and Cas14a.1 HotPot.

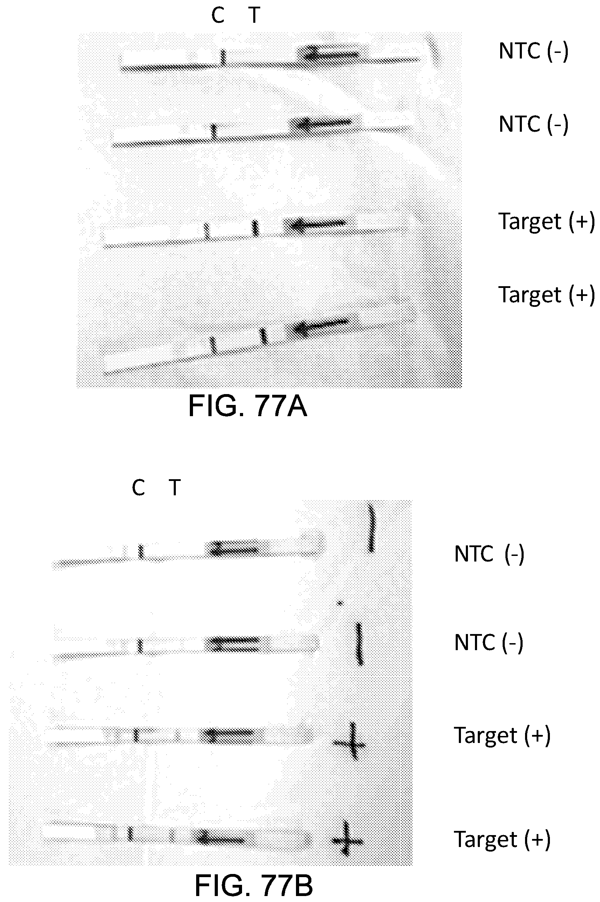

The tubes were then spun down and the supernatant was applied to lateral flow strips. The sample pad of lateral flow strip contained anti-FITC conjugate particles (colloidal gold). If target was present, the supernatant contained cleaved FAM-biotin-labeled reporter molecules which bound to an anti-biotin (e.g., streptavidin) target line on the lateral flow strip. The anti-FITC conjugate particles bound the FAM moiety on the reporter molecules and a target band appeared on lateral flow strips at the anti-biotin target line. If target was not present (as in NTC DETECTR reactions), the supernatant did not contain any FAM-biotin-labeled molecules and nothing bound to the anti-biotin target line. The lateral flow assay strip also contained an anti-IgG flow control line, downstream of the anti-biotin target line, which bound to the anti-FITC moiety of the conjugate particles to confirm that the lateral flow assay functioned properly. **FIG. 77A** shows the results of the Cas12 OnePot DETECTR assays. **FIG. 77B** shows the results of the Cas14a.1 HotPot DETECTR assays. Strong signals were seen in both positive sample replicates while minimal background appeared in NTC replicate strips at the target line.

### Example 26

### Isothermal Nicking Enzyme Amplification for DETECTR Reactions

This example describes isothermal nicking enzyme pre-amplification for DETECTR reactions. CRISPR-based diagnostic DETECTR reactions using loop-mediated isothermal amplification (LAMP) can be completed in as little as 30 minutes. In some cases, longer LAMP amplification may be required to achieve single copy sensitivity. A DETECTR reaction utilizing nicking enzyme amplification (NEAR) was developed to reduce amplification time relative to methods utilizing LAMP amplification. The DETECTR reaction with NEAR also showed improved sensitivity over NEAR reactions performed with a beacon. In the DETECTR reaction with NEAR, a 30 to 40 nucleotide region of a target nucleic acid was amplified using NEAR and detected using a Cas12 variant **(SEQ ID NO: 28).**

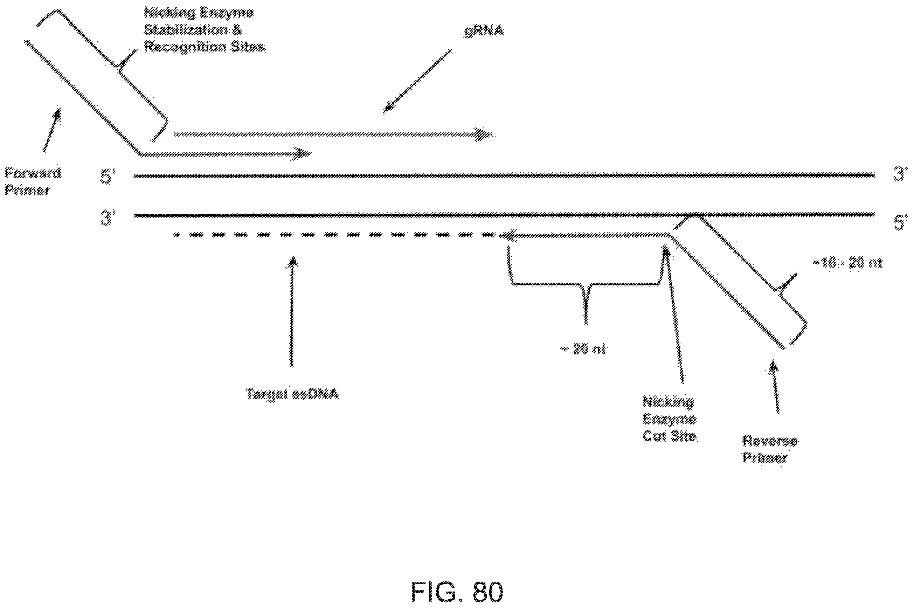

NEAR amplification was performed by contacting the target nucleic acid with a forward primer, a reverse primer, dNTPs, a DNA polymerase, and a nicking endonuclease. The reaction was performed at 60 °C for 10 minutes then cooled to 4 °C. The forward primer contained a 16 to 20 nucleotide region reverse complementary to a first strand of the target nucleic acid and a 16 to 20 nucleotide nicking enzyme stabilization, binding, and recognition site 5' of the region reverse complementary to the target nucleic acid. The reverse primer contained a 16 to 20 nucleotide region reverse complementary to a second strand of the target nucleic acid and a 16 to 20 nucleotide nicking enzyme stabilization, binding, and recognition site 5' of the region reverse complementary to the target nucleic acid. A NEAR duplex incorporating a nicking enzyme stabilization, binding, and recognition site into an amplicon also containing the region of the target nucleic acid was then generated, as illustrated in **FIG. 80****.** The duplex was then nicked by the nicking endonuclease and amplified using the DNA polymerase. The amplicons were detected using a DETECTR reaction with the Cas12 variant as described in at least Examples 30-33.

### Example 27:

### Detection of a Strep-A Target Nucleic Acid Using DETECTR with NEAR

This example describes detection of Streptococcus pyogenes using NECTR (NEAR combined with DETECTR) via amplification of the Strep-A target. The Strep-A target was amplified using the process described in Example 26. Specific conditions were optimized for Strep-A detection.

A 10 µL preamplification reaction was prepared at a final concentration of 1X Isothermal Amplification Buffer (NEB), 1X NEBuffer 3.1 (NEB), 300 nM dNTPs (NEB), 4U of Bst 2.0, 3U of Nt BstNBI, 500 nM of the forward primer (SEQ ID NO: 359), and 100 nM of the reverse primer (SEQ ID NO: 360). In order to prevent non-specific amplification, the primers were added last.

8 µL of the prepared pre-amplification mixture was dispensed into a 96-well or 384-well plate. 2 µL of the target nucleic acid was added to each well in order to prevent early initiation of the reaction. Sample mixtures are mixed thoroughly and centrifuged to ensure that mixture is at the bottom of the wells. The pre-amplification reactions were incubated at 60 °C for 10 minutes and immediately placed at 4 °C or on ice to stop the reaction.Detection of the Strep-A was achieved by our DETECTR technology via Cas12a. A Cas12a complexing reaction was prepared at a final concentration of 1X MB3, 160 nM of Cas12 variant (SEQ ID NO: 28) and 160 nM of R1107 crRNA (SEQ ID NO: 358) and incubated at 37 °C for 30 minutes. After incubation, the Beacon-AlexaFluor 594 (SEQ ID NO: 357) was added to the complexing reaction at a final concentration of 400 nM.

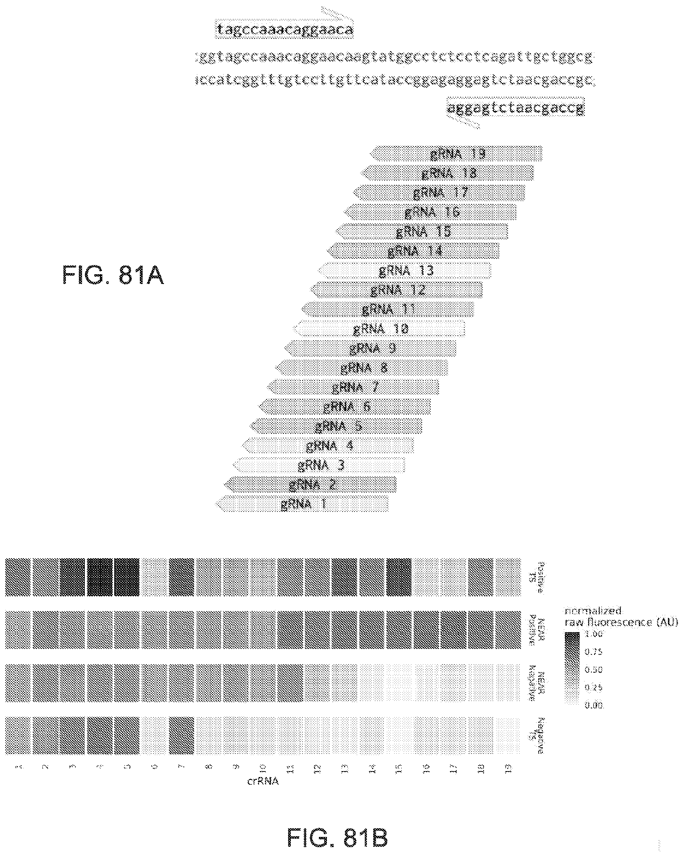

In a 384-well black assay plate on ice, 13 µL of the 1X MB3 and 5 µL of the Cas12a complexing mixture was added. Using aseptic technique to prevent any nucleic acid contamination, 2 µL of the pre-amplification reaction was added to the assay plate. The plate was sealed with an optically clear adhesive and spun at 2000 rcf. The plate was read at AF594 setting with extended gain. FIGS. 81A-81B, 84-85, and 91 show results obtained using variations of the Strep-A method described here.

**Table 17: Nucleic acid sequences for NEAR reaction for Strep-A and SARS-CoV-2 Target Nucleic Acid.**

| SEQ ID NO | Name | Sequence |
|---|---|---|
| **SEQ ID NO: 357** | Beacon | ACAAGTATGTGAGGAGAGGCCATACTTGT |
| **SEQ ID NO: 358** | | UAAUUUCUACUAAGUGUAGAUUAGGUCAACAUGCAGCU ACA |
| **SEQ ID NO:** 359 | | GCCACCCCAAAAAUGAAGGGACUAAAACAGGUCAACA UGCAGCUACAGG |
| **SEQ ID NO: 360** | | GACGAAUGAAGGAAUGCAACUAGGUCAACAUGCAGCUA CA |
| **SEQ ID NO: 361** | | GACGAAUGAAGGAAUGCAACGUAGGUCAACAUGCAGCU AC |
| **SEQ ID NO: 362** | | TTGCTTTCGTGGTATT |
| **SEQ ID NO: 363** | | GGATGGCTAGTGTAA |

### Example 28:

### Detection of a SARS-CoV-2 Target Nucleic Acid Using DETECTR with NEAR

This example describes detection of SARS-CoV-2 using NECTR (NEAR combined with DETECTR) via amplification of the E-gene target. The Strep-A target was amplified using the process described in Example 26. Specific conditions were optimized for E-gene detection.

A 10 µL preamplification reaction was prepared at a final concentration of 1X Isothermal Amplification Buffer (NEB), 1X NEBuffer 3.1 (NEB), 300 nM dNTPs (NEB), 4U of Bst 2.0, 3U of Nt BstNBI, 500 nM of the Forward primer (SEQ ID NO: 362), and 100 nM of the reverse primer (SEQ ID NO: 363). In order to prevent non-specific amplification, the primers were added last.

8 µL of the prepared pre-amplification mixture was dispensed into a 96-well or 384-well plate. 2 µL of the target nucleic acid was added to each well in order to prevent early initiation of the reaction. Sample mixtures are mixed thoroughly and centrifuged to ensure that mixture is at the bottom of the wells. The pre-amplification reactions were incubated at 60 °C for 10 minutes and immediately placed at 4 °C or on ice to stop the reaction.

Detection of the Strep-A was achieved by our DETECTR technology via Cas12a. A Cas12a complexing reaction was prepared at a final concentration of 1X MB3, 160 nM of a Cas12 variant (SEQ ID NO: 28), and 160 nM of R1107 crRNA (SEQ ID NO: 358) and incubated at 37 °C for 30 minutes. After incubation, the Beacon-AlexaFluor 594 (SEQ ID NO: 361) was added to the complexing reaction at a final concentration of 400 nM.

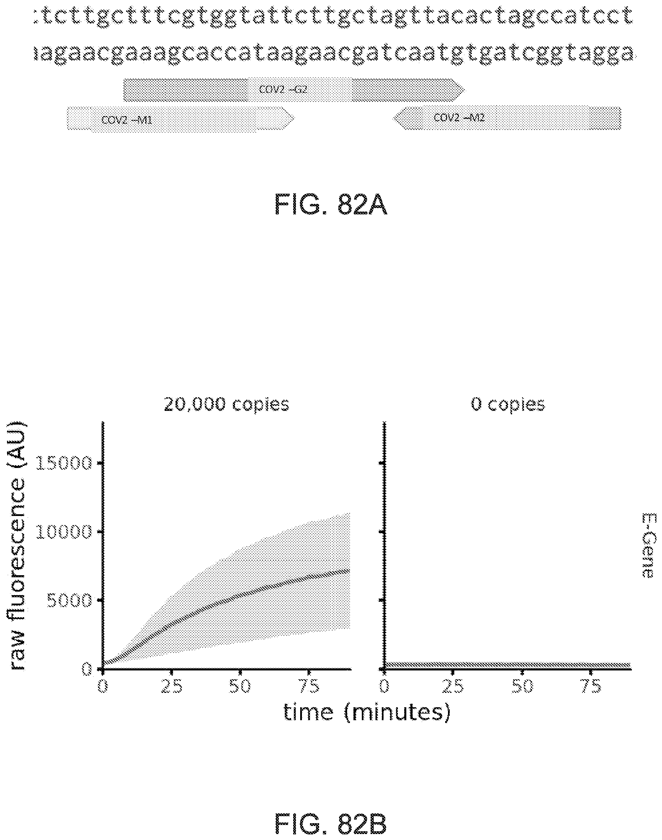

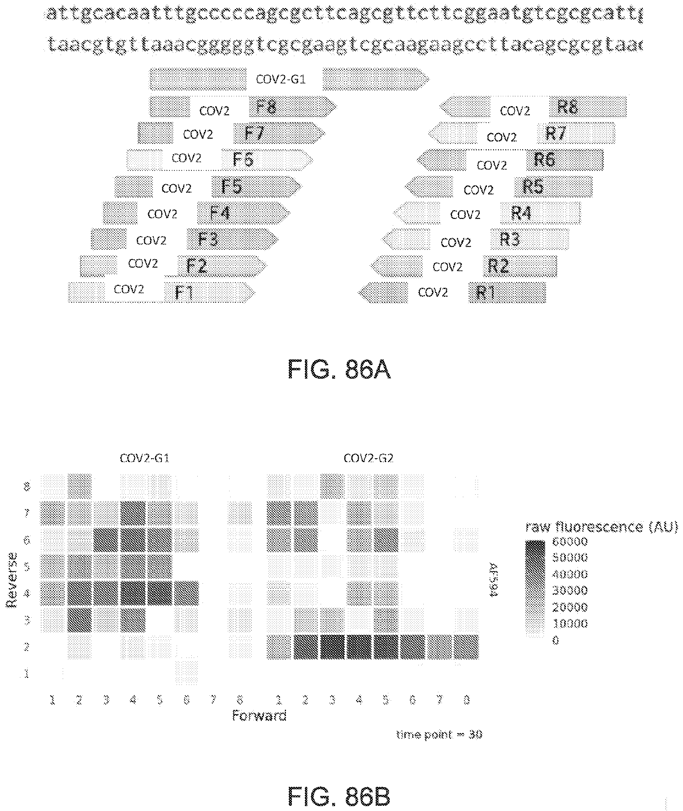

In a 384-well black assay plate on ice, 13 µL of the 1X MBuffer 3 and 5 µL of the Cas12a complexing mixture was added. Using aseptic technique to prevent any nucleic acid contamination, 2 µL of the pre-amplification reaction was added to the assay plate. The plate was sealed with an optically clear adhesive and spun at 2000 x g rcf. The plate was read at AF594 setting with extended gain. FIGS. 82A-82B and 86A-90 show results obtained using variations of the SARS-CoV-2 E-gene method described here.

### Example 29:

### NEAR + DETECTR Reaction Optimization

### Determining the Mg2+ concentration for a NEAR reaction

This example describes optimization of Mg2+ concentration for the NEAR reaction. In addition, the performance of Bst 2.0 and Bst 3.0 were assessed. In this example, the protocol used was identical to that used in **Example 26.**
After amplification of Strep-A, the samples were tested in different buffers, Thrmopol, IsoAmp I, and IsoAmp II at different added Mg2+ concentrations - 0 mM, 2.5 mM, 5 mM, 7.5 mM, and 10 mM. The elapsed time at which it took to achieve a fluorescent readout from the Beacon plate reader was measured, with a lower value indicating that there were more copies of the amplicon present in the solution.

It was found that no additional magnesium was required and that Bst 2.0 was preferred for the NEAR reactions under the conditions tested. As shown in **FIG. 85****,** Bst 2.0 was able to generate an observable fluorescent signal (~20,000 copies) in less than 5 minutes. In **FIG. 85****,** Bst 3.0 performed slightly slower in IsoAmp I and IsoAmp II buffers. Therefore, Bst 2.0 and a concentration of 12 mM Mg2+ were selected for future NEAR reaction conditions.

### Determining the preferred guide sequence

This example describes how to select a guide RNA for detection of Strep-A, but it will be understood that the teachings described here may be used to select guides for other targets of interest as desired. The same primers and probe used in **Example 27** were used. The following experiment determined if ssDNA could be detected using DETECTR from a NEAR reaction. Due to the biased reaction, gRNAs were generated in the reverse direction to detect any ssDNA produced from the nicking occurring on the forward primer nicking site.

A panel of 19 gRNAs as shown in **TABLE 18** was synthesized in order to determine which gRNA worked best in the DETECTR reaction under the conditions tested. As shown in **FIGS. 81A-81B****,** all gRNAs successfully detected the positive control which comprised an oligonucleotide that was a complement of the gRNA. Guide RNAs 14-19 successfully detected the NEAR reaction products containing the target and positive control, but did not detect the NEAR negative control and negative (no input) controls. It was observed that the maximum overlap with the guide RNA allowed was 3 nucleotides, otherwise signal detection in the negative controls were detected under the conditions tested.

### Characterizing NEAR RNA targets Using DETECTR

This example describes quantification of NEAR RNA targets derived from SARS COV-2 using the NECTR system described in **Example 28.** Two NEAR primers were used to amplify the SARS-CoV-2 target sequence and a guide design as shown in **FIG. 82A****.** As shown in **FIGS. 82A-82B****,** the results of the SARS-CoV-2 E-gene DETECTR reaction are shown for samples including 20,000 NEAR copies or 0 NEAR copies. The DETECTR reaction successfully detected SARS-CoV-2 E-gene in the 20,000 target copy system and did not produce a signal in the 0 copy negative control conditions.

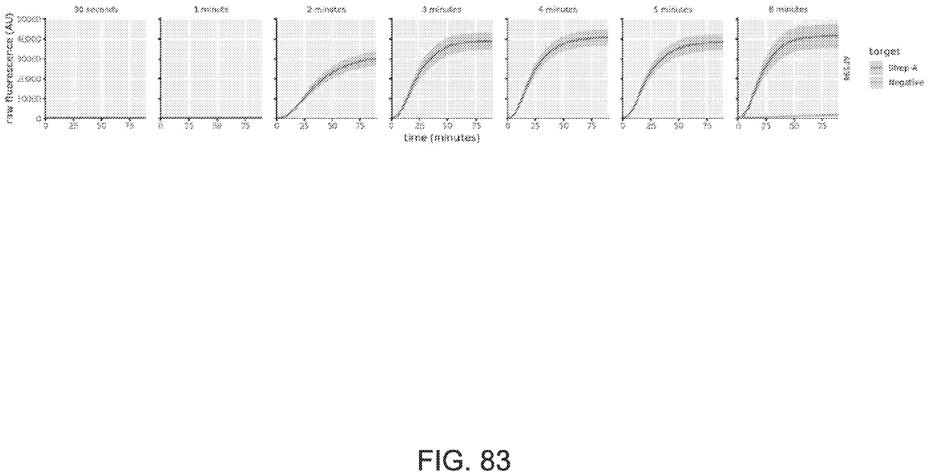

Next, the minimal incubation time of a NEAR reaction of Strep-A targets prior to detection via DETECTR was determined. The NEAR protocol was substantially similar to that described in **Example 27.** Incubation of the target with NEAR reaction components proceeded for 30 seconds, 1 minute, 2 minutes, 3 minutes, 4 minutes, 5 minutes, or 6 minutes prior to DETECTR. Under the conditions tested, a minimum of 2 minutes was observed to be required to achieve a detectable DETECTR signal, however, 6 minutes was found to be more optimal as it achieved a signal much higher than the limit of detection, as shown in **FIG. 83****.**

### Detecting NEAR RNA targets Using Cas Variants

This example describes a comparison of NEAR amplicon detection using orthogonal Cas system to detect the target. The protocol of **Example 27** was used for this example. Here, a Cas12 variant **(SEQ ID NO: 28),** a Cas13 variant **(SEQ ID NO: 154),** and a Cas14 variant **(SEQ ID NO: 63)** were utilized. They were chosen because they have different cleavage preferences for the reporter molecule and can be multiplexed together. In **FIG. 84****,** the Cas12 variant showed superior signal after NEAR DETECTR reaction under the conditions tested. These results showed that it is possible to multiplex different NEAR-DETECTR reactions with orthogonal Cas systems.

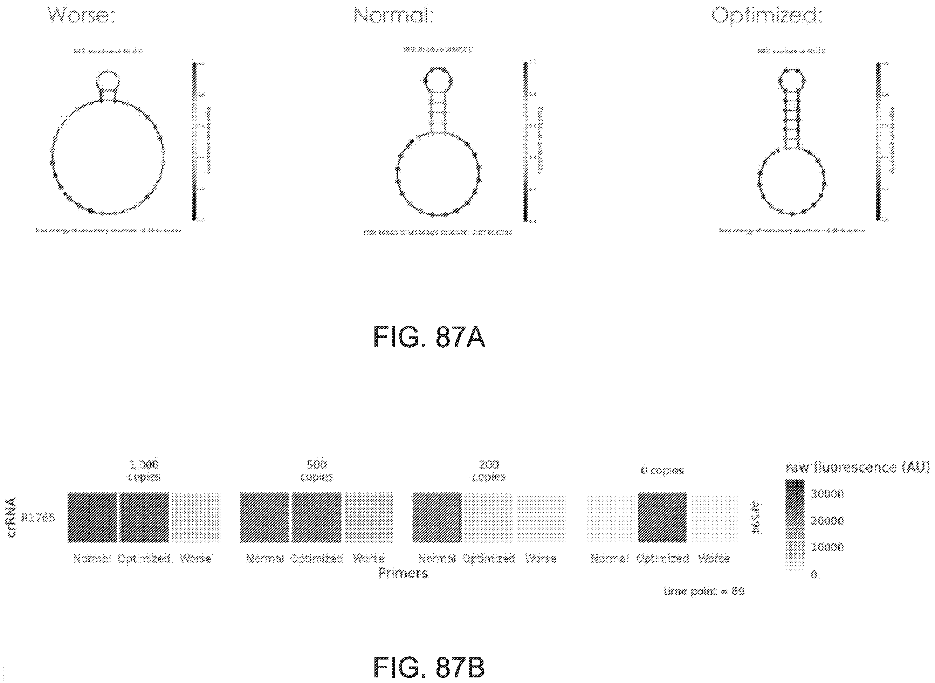

We also optimized the stability of the hairpin loop in the nickase stabilization region present in the NEAR primers. Without being bound to any particular theory, it is thought that the hairpin loop may impact the nickase activity of the enzyme. To test this, alternative primer pairs to destroy, maintain, or enhance the hairpin loop formation **(****FIG. 87A****)** were used. The performance of these new hairpin loops were tested on detection of SARS-COV-2 E-gene as described in **Example 28.** In **FIG. 87B****,** the destroyed hairpin loops showed reduced signal compared to both the normal and optimized hairpins, however, the enhanced hairpin showed strong signal even in the negative control under the conditions tested. It was determined that based on the results of this study that the normal primers would be used for future NEAR reactions.

### Determining the optimal Reverse Transcriptase

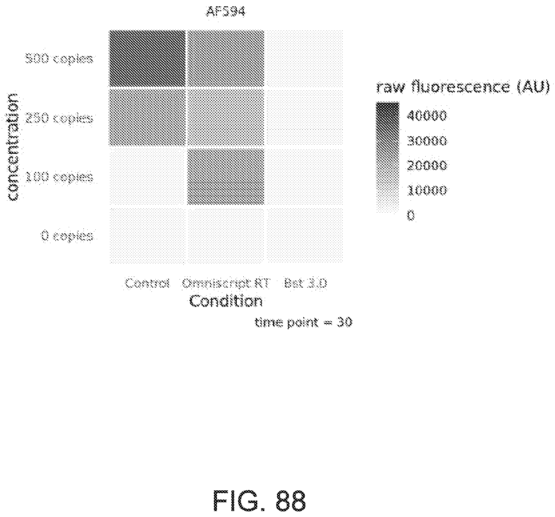

This example describes comparison of different reverse transcriptases for the RT-NECTR reaction described in **Example 28.** In this study, Wartmstart RTx (NEB), Bst 3.0, and Omniscript RT (Qiagen) were tested and they were compared by using the limit of detection as a metric. **FIG. 88** shows that Omniscript achieved a better limit of detection compared to the other two reverse transcriptases under the conditions tested.

### Optimizing primer concentrations

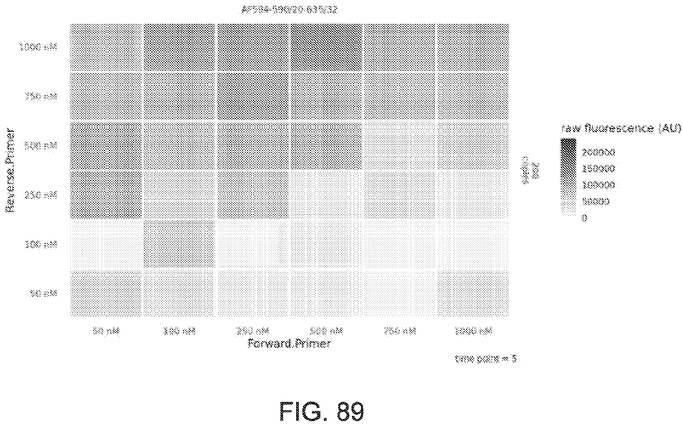

Primer concentrations were optimized in order to determine which concentrations would result in a better signal using the RT-NEAR reaction described in **Example 28.** In **FIG. 89****,** primer concentrations of the forward and reverse NEAR primers were prepared at a concentration of 1000 nM, 750 nM, 500 nM, 250 nM nM, 100 nM, and 50 nM and mixed in a pairwise fashion in order to determine which concentration of forward and reverse primer will result in the best detectable signal. The forward primer used was M2805 (GAC TCC ACA CGG AGT Ctt gct ttc gtg gta tt) and the reverse primer used was M2811 (GAC TCC ACA CGG AGT Cgg atg gct agt gta ac). For this study, NEAR reactions were incubated and amplified targets for 5 minutes at 60 °C. A combination of 1000 nM of the reverse primer and 500 nM of the forward primer performed best and resulted in a raw fluorescence greater than all other primer combinations tested. It was determined that by increasing the concentration of either the reverse or the forward primer resulted in increased efficiency of the reaction.

### Optimizing amplification times

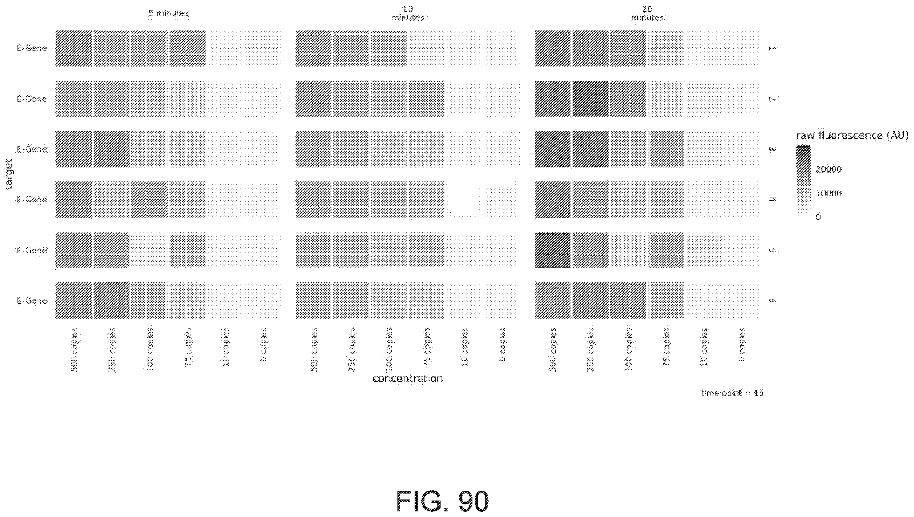

Due to the need to reverse transcribe the SARS-CoV2 prior to detection, different amplification times need to be tested in order to determine the optimal resulting signal from RT-NECTR protocol of **Example 28.** In **FIG. 90****,** different amplification times of 5 minutes, 10 minutes, and 20 minutes were tested on different cDNA (SARS-CoV2 E-gene) inputs (500, 250, 100, 75, 10, and 0 cDNA copies). In this experiment, higher amplification times resulted in increased DETECTR signal, however, even after 20 minutes of incubation, DETECTR only resulted in a slight signal in 10 copies. At 75 copies, 10 minutes of amplification was sufficient to detect 75 copies of SAR-CoV2 via DETECTR within 5 minutes.

**TABLE 18 - Exemplary Guides**

| **SEQ ID NO:** | GUIDE NAME | ALTERNATIVE NAME | Sequence |
|---|---|---|---|
| **SEQ ID NO: 328** | R1763 | COV2-G1 | TTTGCCCCCAGCGCTTCAGC GTTC |
| **SEQ ID NO: 329** | R1765 | COV2-G2 | TTTCGTGGTATTGCTAGTTAC |
| **SEQ ID NO: 330** | EXAMPLE GUIDE 19 | EXAMPLE GUIDE 19 | CAATCTGAGGAGAGGCCATA |
| **SEQ ID NO: 331** | EXAMPLE GUIDE 18 | EXAMPLE GUIDE 18 | AATCTGAGGAGAGGCCATAC |
| **SEQ ID NO: 332** | EXAMPLE GUIDE 17 | EXAMPLE GUIDE 17 | ATCTGAGGAGAGGCCATACT |
| **SEQ ID NO: 333** | EXAMPLE GUIDE16 | EXAMPLE GUIDE16 | TCTGAGGAGAGGCCATACTT |
| **SEQ ID NO: 334** | EXAMPLE GUIDE 15 | EXAMPLE GUIDE 15 | CTGAGGAGAGGCCATACTTG |
| **SEQ ID NO: 335** | EXAMPLE GUIDE 14 | EXAMPLE GUIDE 14 | TGAGGAGAGGCCATACTTGT |
| **SEQ ID NO: 336** | EXAMPLE GUIDE 13 | EXAMPLE GUIDE 13 | GAGGAGAGGCCATACTTGTT |
| **SEQ ID NO: 337** | EXAMPLE GUIDE 12 | EXAMPLE GUIDE 12 | AGGAGAGGCCATACTTGTTC |
| **SEQ ID NO: 338** | EXAMPLE GUIDE 11 | EXAMPLE GUIDE 11 | GGAGAGGCCATACTTGTTCC |
| **SEQ ID NO: 339** | EXAMPLE GUIDE 10 | EXAMPLE GUIDE 10 | GAGAGGCCATACTTGTTCCT |
| **SEQ ID NO: 340** | EXAMPLE GUIDE 9 | EXAMPLE GUIDE 9 | AGAGGCCATACTTGTTCCTG |
| **SEQ ID NO: 341** | EXAMPLE GUIDE 8 | EXAMPLE GUIDE 8 | GAGGCCATACTTGTTCCTGT |
| **SEQ ID NO: 342** | EXAMPLE GUIDE 7 | EXAMPLE GUIDE 7 | AGGCCATACTTGTTCCTGTT |
| **SEQ ID NO: 343** | EXAMPLE GUIDE 6 | EXAMPLE GUIDE 6 | GGCCATACTTGTTCCTGTTT |
| **SEQ ID NO: 344** | EXAMPLE GUIDE 5 | EXAMPLE GUIDE 5 | GCCATACTTGTTCCTGTTTG |
| **SEQ ID NO: 345** | EXAMPLE GUIDE 4 | EXAMPLE GUIDE 4 | CCATACTTGTTCCTGTTTGG |
| **SEQ ID NO: 346** | EXAMPLE GUIDE 3 | EXAMPLE GUIDE 3 | CATACTTGTTCCTGTTTGGC |
| **SEQ ID NO: 347** | EXAMPLE GUIDE 2 | EXAMPLE GUIDE 2 | ATACTTGTTCCTGTTTGGCT |
| **SEQ ID NO: 348** | EXAMPLE GUIDE 1 | EXAMPLE GUIDE 1 | TACTTGTTCCTGTTTGGCTA |

### Example 30:

### Testing Cas System Orthogonality

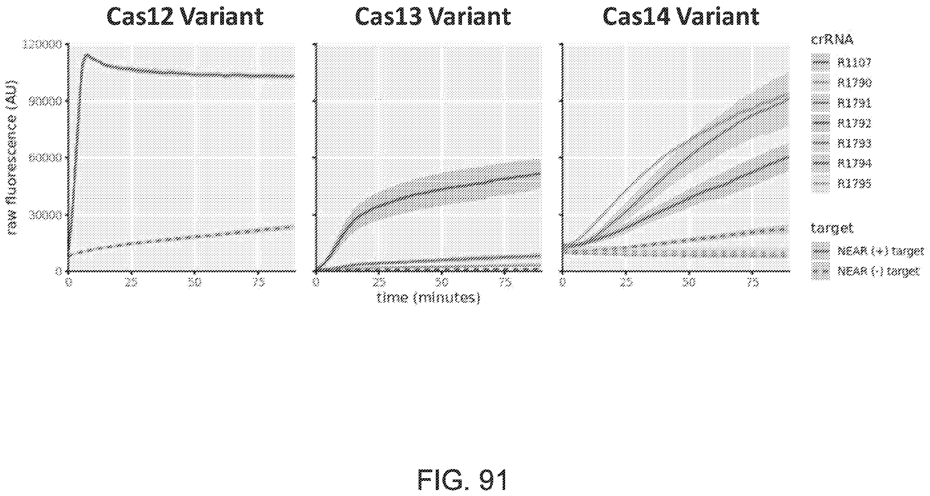

This example describes testing of different Cas systems for orthogonality of the NEAR-DETECTR reaction described in **Example 27**. Cas14 variant **(SEQ ID NO: 63)** has been previously reported to detect ssDNA, whereas Cas13 variant **(SEQ ID NO: 154)** has been determined to be capable of detecting ssDNA. It was determined whether it was possible for the Cas14 variant or Cas 13 variant to be used in NEAR-DETECTR to detect ssDNA. As shown in **FIG. 91****,** Strep-A was detected using the primers M2048 and M2049, using the Cas12 variant **(SEQ ID NO: 28),** Cas13 variant **(SEQ ID NO: 154),** and Cas14 variant **(SEQ ID NO: 63)** systems. Detection was shown to be possible with Cas13 and Cas14 systems, suggesting the possibility of multiplexing NEAR amplicons using Cas12/13 or Cas 14/13 reaction combinations.

## Claims

1. A system for detecting a target nucleic acid, comprising a buffer comprising:
i. amplification reagents for an amplification reaction targeting the target nucleic acid; and
ii. detection reagents for a detection reaction targeting the target nucleic acid;
wherein the amplification reagents comprise one or more oligonucleotide primers, and a DNA polymerase;
wherein the detection reagents comprise a programmable nuclease, a non-naturally occurring guide nucleic acid, and reporters;
wherein the non-naturally occurring guide nucleic acid comprises a sequence that hybridizes to a segment of the target nucleic acid or DNA amplicons thereof;
wherein the amplification reagents are present in amounts effective to amplify the target nucleic acid in a test sample to produce DNA amplicons of the target nucleic acid;
wherein the programmable nuclease and non-naturally occurring guide nucleic acid form a complex in the buffer that is activated upon binding one of the DNA amplicons to induce detectable transcollateral cleavage of the reporters; and
wherein the buffer is a lysis buffer.

2. The system of claim 1, wherein (a) at least 1 nM of the reporters undergo transcollateral cleavage within one hour of addition of at least 5000 copies of the target nucleic acid to the system; (b) at least 5 nM of the reporters undergo transcollateral cleavage within one hour of addition of at least 5000 copies of the target nucleic acid to the system; (c) at least 10 nM of the reporters undergo transcollateral cleavage within one hour of addition of at least 5000 copies of the target nucleic acid to the system; (d) at least 1 nM of the reporters undergo transcollateral cleavage within one hour of addition of at least 1000 copies of the target nucleic acid to the system; or (e) at least 1 nM of the reporters undergo transcollateral cleavage within one hour of addition of at least 1000 copies of the target nucleic acid to the system.

3. The system of claim 1 or claim 2, wherein the amplification reagents comprise reagents for isothermal amplification; optionally wherein the amplification reagents comprise reagents for transcription mediated amplification (TMA), helicase dependent amplification (HDA), circular helicase dependent amplification (cHDA), strand displacement amplification (SDA), loop mediated amplification (LAMP), exponential amplification reaction (EXPAR), rolling circle amplification (RCA), ligase chain reaction (LCR), simple method amplifying RNA targets (SMART), single primer isothermal amplification (SPIA), multiple displacement amplification (MDA), nucleic acid sequence based amplification (NASBA), hinge-initiated primer-dependent amplification of nucleic acids (HIP), nicking enzyme amplification reaction (NEAR), or improved multiple displacement amplification (IMDA).

4. The system of claim 1, further comprising an activator for the amplification reaction; optionally wherein the activator for the amplification reaction comprises a magnesium or calcium salt.

5. The system of any one of claims 1-4, wherein the programmable nuclease comprises at least 60% sequence identity to SEQ ID NO: 18-170, 221-268 or 397-423.

6. The system of any one of claims 1-5, wherein the programmable nuclease is a CRISPR-Cas enzyme, optionally a Type V CRISPR/Cas effector protein.

7. The system of any one of claims 1-6, further comprising a reverse transcriptase, an oligonucleotide primer, and dNTPs for reverse transcribing the target nucleic acid.

8. The system of any one of claims 1-7, wherein the buffer comprises a pH of 7.5 to 8.5, at least 10 mM of a buffering agent, at least 1 mM ammonium acetate, at least 10 mM potassium acetate, at least 2.5 mM magnesium acetate, and at least 0.5% glycerol; optionally wherein: (a) the buffer comprises a pH of 7.7 to 8.3, or a pH of 7.85 to 8.15; (b) the buffering agent comprises HEPES, imidazole, TRIS-HCl, or phosphate; and/or (c) the buffer further comprises at least 0.05% by volume of a detergent, optionally wherein the detergent comprises Tween 20.

9. The system of any one of claims 1-7, wherein the buffer comprises a pH of 7.5 to 8.5, at least 5 mM of a buffering agent, at least 20 mM potassium acetate, at least 2.5 mM magnesium acetate, and at least 0.5% glycerol; optionally wherein: (a) the buffer comprises a pH of 7.7 to 8.3, or a pH of 7.85 to 8.15; (b) the buffering agent comprises phosphate or TRIS-HCl; (c) the buffer comprises at least 1 mM ammonium sulfate; and/or (d) the buffer comprises at least 0.05% by volume of a detergent, optionally wherein the detergent comprises Tween 20.

10. The system of any one of claims 1-7, wherein the buffer comprises a pH of 7.25 to 8.75, at least 5 mM of a buffering agent, at least 7.5 mM potassium acetate, at least 1 mM magnesium acetate, and at least 0.5% glycerol; optionally wherein: (a) the buffering agent comprises phosphate; (b) the buffer comprises a pH of 7.5 to 8.5, or a pH of 7.75 to 8.25; (c) the buffer further comprises at least 1 mM ammonium sulfate; and/or (d) the buffer further comprises at least 0.05% by volume of a detergent, optionally wherein the detergent comprises Tween 20.

11. The system of claim 1 or 2, further comprising a circular template with internal complementarity formed from a single polynucleotide strand, wherein:
(a) the circular template comprises a first portion with complementarity to one of the one or more oligonucleotide primers and a second portion with complementarity to a portion of the target nucleic acid;
(b) the internal complementarity comprises part of the first portion and part of the second portion;
(c) the second portion has a total length that is longer than a combined length of the first portion and second portion that are within the internal complementarity; and
(d) the circular template undergoes a conformational change upon hybridization to the target nucleic acid to expose the first portion to hybridization to the oligonucleotide primer.

12. The system of claim 1 or 2, further comprising a circular template, wherein:
(a) the circular template comprises a first portion with complementarity to one of the one or more oligonucleotide primers and a second portion with complementarity to the target nucleic acid;
(b) the oligonucleotide primer complementary to the first portion comprises a blocking motif at its 3' end; and
(c) the oligonucleotide primer complementary to the first portion undergoes cleavage to remove the blocking motif by the programmable nuclease in the presence of the target nucleic acid.

13. The system of any one of claims 1-12, further comprising a polymer matrix, wherein the polymer matrix is complexed with the reporters; optionally wherein: (a) the polymer matrix is formed from copolymerization of at least a first plurality of monomers with the reporters, and/or (b) the polymer matrix comprises a hydrogel.

14. A method of assaying for a target nucleic acid in a sample, the method comprising:
a. amplifying a portion of the target nucleic acid with a DNA polymerase to produce DNA amplicons of the target nucleic acid;
b. forming a complex comprising one of the DNA amplicons, a programmable nuclease, and a non-naturally occurring guide nucleic acid that hybridizes to a segment of the DNA amplicon, thereby activating the programmable nuclease;
c. cleaving reporters with the activated programmable nuclease; and
d. detecting a change in a signal, wherein the change in the signal is produced by cleavage of the reporters;
wherein the target nucleic acid and reagents for the amplifying and cleaving are present in the same reaction volume;
wherein the method further comprises lysing a cell or virus comprising the target nucleic acid and wherein the lysing is performed in the same reaction volume as the amplifying and the cleaving.

15. A system for detecting a target nucleic acid, comprising reagents in a buffer, wherein:
(a) the reagents comprise hairpin polynucleotides, programmable nucleases, non-naturally occurring guide nucleic acids, and reporters;
(b) each hairpin polynucleotide comprises one or more RNA loops, a first portion comprising DNA, and a second portion joined to the first portion by one of the one or more RNA loops;
(c) each non-naturally occurring guide nucleic acid comprises a sequence that hybridizes to a segment of the target nucleic acid;
(d) in each hairpin polynucleotide, the second portion of the hairpin polynucleotide hybridizes to a segment of the first portion;
(e) the programmable nucleases and non-naturally occurring guide nucleic acids form complexes in the buffer that are activated upon binding the target nucleic acid;
(f) an activated programmable nuclease is effective to induce (i) transcollateral cleavage of the one or more RNA loops, and (ii) detectable transcollateral cleavage of the reporters; and
(g) cleavage of the one or more RNA loops of one of the hairpin polynucleotides is effective to release the first portion of the hairpin polynucleotide to hybridize with one of the non-naturally occurring guide nucleic acids and form a further activated programmable nuclease.

16. A method of assaying for a target nucleic acid in a sample, the method comprising the following steps in a single reaction volume:
(a) forming a complex comprising the target nucleic acid, a first programmable nuclease, and a first non-naturally occurring guide nucleic acid that hybridizes to a segment of the target nucleic acid, thereby activating the programmable nuclease;
(b) cleaving a hairpin polynucleotide of a plurality of hairpin polynucleotides with the activated programmable nuclease, wherein each hairpin polynucleotide comprises (i) one or more RNA loops that are cleaved, (i) a first portion comprising DNA, and (iii) a second portion joined to the first portion by one of the one or more RNA loops, wherein the second portion is hybridized to a segment of the first portion;
(c) forming a second complex comprising the first portion of the cleaved hairpin polynucleotide, a second programmable nuclease, and a second non-naturally occurring guide nucleic acid that hybridizes to the first portion of the cleaved hairpin, thereby activating the second programmable nuclease;
(d) cleaving reporters with the activated first or second programmable nuclease; and
(e) detecting a change in a signal, wherein the change in the signal is produced by cleavage of the reporters.

## Patentansprüche

1. System zum Nachweisen einer Zielnukleinsäure, umfassend einen Puffer, umfassend:
i. Amplifikationsreagenzien für eine Amplifikationsreaktion, die auf die Zielnukleinsäure abzielt; und
ii. Nachweisreagenzien für eine Nachweisreaktion, die auf die Zielnukleinsäure abzielt;
wobei die Amplifikationsreagenzien einen oder mehrere Oligonukleotidprimer und eine DNA-Polymerase umfassen; wobei die Nachweisreagenzien eine programmierbare Nuklease, eine nicht natürlich vorkommende Guide-Nukleinsäure und Reporter umfassen;
wobei die nicht natürlich vorkommende Guide-Nukleinsäure eine Sequenz umfasst, die an ein Segment der Zielnukleinsäure oder DNA-Amplicons davon hybridisiert; wobei die Amplifikationsreagenzien in Mengen vorhanden sind, die zum Amplifizieren der Zielnukleinsäure in einer Testprobe wirksam sind, um DNA-Amplicons der Zielnukleinsäure herzustellen;
wobei die programmierbare Nuklease und die nicht natürlich vorkommende Guide-Nukleinsäure einen Komplex in dem Puffer bilden, der bei Bindung eines der DNA-Amplicons aktiviert wird, um eine nachweisbare transkollaterale Spaltung der Reporter zu induzieren; und wobei der Puffer ein Lysepuffer ist.

2. System nach Anspruch 1, wobei (a) mindestens 1 nM der Reporter innerhalb einer Stunde ab Zugabe von mindestens 5000 Kopien der Zielnukleinsäure zu dem System einer transkollateralen Spaltung unterzogen werden; (b) mindestens 5 nM der Reporter innerhalb einer Stunde ab Zugabe von mindestens 5000 Kopien der Zielnukleinsäure zu dem System einer transkollateralen Spaltung unterzogen werden; (c) mindestens 10 nM der Reporter innerhalb einer Stunde ab Zugabe von mindestens 5000 Kopien der Zielnukleinsäure zu dem System einer transkollateralen Spaltung unterzogen werden; (d) mindestens 1 nM der Reporter innerhalb einer Stunde ab Zugabe von mindestens 1000 Kopien der Zielnukleinsäure zu dem System einer transkollateralen Spaltung unterzogen werden; oder (e) mindestens 1 nM der Reporter innerhalb einer Stunde ab Zugabe von mindestens 1000 Kopien der Zielnukleinsäure zu dem System einer transkollateralen Spaltung unterzogen werden.

3. System nach Anspruch 1 oder Anspruch 2, wobei die Amplifikationsreagenzien Reagenzien für isotherme Amplifikation umfassen; optional wobei die Amplifikationsreagenzien Reagenzien für transkriptionsvermittelte Amplifikation (TMA), Helicaseabhängige Amplifikation (HDA), zirkuläre Helicaseabhängige Amplifikation (CHDA), Strangverdrängungs-Amplifikation (SDA), Loop-vermittelte Amplifikation (LAMP), exponentielle Amplifikationsreaktion (EXPAR), Rolling-Circle-Amplifikation (RCA), Ligasekettenreaktion (LCR), Simple-Method-Amplifizierung-von-RNA-Zielen (SMART), Single-Primer-isothermale-Amplifikation (SPIA), Multiple-Displacement-Amplifikation (MDA), Nukleinsäuresequenz-basierte Amplifikation (NASBA), Hinge-initiierte Primer-dependente-Amplifikation von Nukleinsäuren(HIP), Nicking-Enzym-Amplifikationsreaktion (NEAR) oder improved-Multiple-Displacement-Amplifikation (IMDA) umfassen.

4. System nach Anspruch 1, ferner umfassend einen Aktivator für die Amplifikationsreaktion; optional wobei der Aktivator für die Amplifikationsreaktion ein Magnesium- oder Calciumsalz umfasst.

5. System nach einem der Ansprüche 1-4, wobei die programmierbare Nuklease mindestens 60 % Sequenzidentität zu SEQ ID NO: 18-170, 221-268 oder 397-423 umfasst.

6. System nach einem der Ansprüche 1-5, wobei die programmierbare Nuklease ein CRISPR-Cas-Enzym, optional ein Typ-V-CRISPR/Cas-Effektorprotein, ist.

7. System nach einem der Ansprüche 1-6, ferner umfassend eine reverse Transkriptase, einen Oligonukleotidprimer und dNTPs zum reversen Transkribieren der Zielnukleinsäure.

8. System nach einem der Ansprüche 1-7, wobei der Puffer einen pH-Wert von 7,5 bis 8,5, mindestens 10 mM eines Puffermittels, mindestens 1 mM Ammoniumacetat, mindestens 10 mM Kaliumacetat, mindestens 2,5 mM Magnesiumacetat und mindestens 0,5 % Glycerin umfasst; optional wobei: (a) der Puffer einen pH-Wert von 7,7 bis 8,3 oder einen pH-Wert von 7,85 bis 8,15 umfasst; (b) das Puffermittel HEPES, Imidazol, TRIS-HCl oder Phosphat umfasst; und/oder (c) der Puffer ferner mindestens 0,05 Vol.-% eines Detergens umfasst, wobei das Detergens optional Tween 20 umfasst.

9. System nach einem der Ansprüche 1-7, wobei der Puffer einen pH-Wert von 7,5 bis 8,5, mindestens 5 mM eines Puffermittels, mindestens 20 mM Kaliumacetat, mindestens 2,5 mM Magnesiumacetat und mindestens 0,5 % Glycerin umfasst; optional wobei: (a) der Puffer einen pH-Wert von 7,7 bis 8,3 oder einen pH-Wert von 7,85 bis 8,15 umfasst; (b) das Puffermittel Phosphat oder TRIS-HCl umfasst; (c) der Puffer mindestens 1 mM Ammoniumsulfat umfasst; und/oder (d) der Puffer mindestens 0,05 Vol.-% eines Detergens umfasst, wobei das Detergens optional Tween 20 umfasst.

10. System nach einem der Ansprüche 1-7, wobei der Puffer einen pH-Wert von 7,25 bis 8,75, mindestens 5 mM eines Puffermittels, mindestens 7,5 mM Kaliumacetat, mindestens 1 mM Magnesiumacetat und mindestens 0,5 % Glycerin umfasst; optional wobei: (a) das Puffermittel Phosphat umfasst; (b) der Puffer einen pH-Wert von 7,5 bis 8,5 oder einen pH-Wert von 7,75 bis 8,25 umfasst; (c) der Puffer ferner mindestens 1 mM Ammoniumsulfat umfasst; und/oder (d) der Puffer ferner mindestens 0,05 Vol.-% eines Detergens umfasst, wobei das Detergens optional Tween 20 umfasst.

11. System nach Anspruch 1 oder 2, ferner umfassend eine zirkuläre Matrize mit interner Komplementarität, die aus einem einzelnen Polynukleotidstrang gebildet ist, wobei:
(a) die zirkuläre Matrize einen ersten Abschnitt mit Komplementarität zu einem des einen oder der mehreren Oligonukleotidprimer und einen zweiten Abschnitt mit Komplementarität zu einem Abschnitt der Zielnukleinsäure umfasst;
(b) die interne Komplementarität einen Teil des ersten Abschnitts und einen Teil des zweiten Abschnitts umfasst;
(c) der zweite Abschnitt eine Gesamtlänge aufweist, die länger ist als eine kombinierte Länge des ersten Abschnitts und des zweiten Abschnitts, die innerhalb der internen Komplementarität liegen; und
(d) die zirkuläre Matrize bei Hybridisierung an die Zielnukleinsäure eine Konformationsänderung erfährt, so dass der erste Abschnitt zur Hybridisierung an den Oligonukleotidprimer freigelegt wird.

12. System nach Anspruch 1 oder 2, das ferner eine zirkuläre Matrize umfasst, wobei:
(a) die zirkuläre Matrize einen ersten Abschnitt mit Komplementarität zu einem des einen oder der mehreren Oligonukleotidprimer und einen zweiten Abschnitt mit Komplementarität zu der Zielnukleinsäure umfasst;
(b) der Oligonukleotidprimer, der zu dem ersten Abschnitt komplementär ist, ein Blockierungsmotiv an seinem 3'-Ende umfasst; und
(c) der zum ersten Abschnitt komplementäre Oligonukleotidprimer eine Spaltung zum Entfernen des Blockierungsmotivs durch die programmierbare Nuklease in Gegenwart der Zielnukleinsäure erfährt.

13. System nach einem der Ansprüche 1-12, ferner umfassend eine Polymermatrix, wobei die Polymermatrix mit den Reportern komplexiert ist; optional wobei: (a) die Polymermatrix durch Copolymerisation von mindestens einer ersten Vielzahl von Monomeren mit den Reportern gebildet wird, und/oder (b) die Polymermatrix ein Hydrogel umfasst.

14. Verfahren zum Testen auf eine Zielnukleinsäure in einer Probe, wobei das Verfahren Folgendes umfasst:
a. Amplifizieren eines Abschnitts der Zielnukleinsäure mit einer DNA-Polymerase, um DNA-Amplicons der Zielnukleinsäure herzustellen;
b. Bilden eines Komplexes, umfassend eines der DNA-Amplikons, eine programmierbare Nuklease und eine nicht natürlich vorkommende Guide-Nukleinsäure, die an ein Segment des DNA-Amplikons hybridisiert, wodurch die programmierbare Nuklease aktiviert wird;
c. Spalten von Reportern mit der aktivierten programmierbaren Nuklease; und
d. Detektieren einer Änderung eines Signals, wobei die Änderung des Signals durch die Spaltung der Reporter erzeugt wird;
wobei die Zielnukleinsäure und die Reagenzien für das Amplifizieren und Spalten in demselben Reaktionsvolumen vorliegen;
wobei das Verfahren ferner das Lysieren einer Zelle oder eines Virus umfasst, die/das die Zielnukleinsäure umfasst, und wobei das Lysieren in demselben Reaktionsvolumen wie das Amplifizieren und das Spalten durchgeführt wird.

15. System zum Nachweis einer Zielnukleinsäure, umfassend Reagenzien in einem Puffer, wobei:
(a) die Reagenzien Haarnadelpolynukleotide, programmierbare Nukleasen, nicht natürlich vorkommende Guide-Nukleinsäuren und Reporter umfassen;
(b) jedes Haarnadelpolynukleotid eine oder mehrere RNA-Schleifen, einen ersten Abschnitt, der DNA umfasst, und einen zweiten Abschnitt, der durch eine der einen oder der mehreren RNA-Schleifen mit dem ersten Abschnitt verbunden ist, umfasst;
(c) jede nicht natürlich vorkommende Guide-Nukleinsäure eine Sequenz umfasst, die an ein Segment der Zielnukleinsäure hybridisiert;
(d) in jedem Haarnadelpolynukleotid der zweite Abschnitt des Haarnadelpolynukleotids an ein Segment des ersten Abschnitts hybridisiert;
(e) die programmierbaren Nukleasen und nicht natürlich vorkommenden Guide-Nukleinsäuren Komplexe im Puffer bilden, die bei Bindung der Zielnukleinsäure aktiviert werden;
(f) eine aktivierte programmierbare Nuklease wirksam ist, um (i) eine transkollaterale Spaltung der einen oder mehreren RNA-Schleifen und (ii) eine nachweisbare transkollaterale Spaltung der Reporter zu induzieren; und
(g) die Spaltung der einen oder der mehreren RNA-Schleifen eines der Haarnadelpolynukleotide wirksam ist, um den ersten Abschnitt des Haarnadelpolynukleotids freizugeben, um mit einer der nicht natürlich vorkommenden Guide-Nukleinsäuren zu hybridisieren und eine weitere aktivierte programmierbare Nuklease zu bilden.

16. Verfahren zum Testen auf eine Zielnukleinsäure in einer Probe, wobei das Verfahren die folgenden Schritte in einem einzigen Reaktionsvolumen umfasst:
(a) Bilden eines Komplexes, umfassend die Zielnukleinsäure, eine erste programmierbare Nuklease und eine erste nicht natürlich vorkommende Guide-Nukleinsäure, die an ein Segment der Zielnukleinsäure hybridisiert, wodurch die programmierbare Nuklease aktiviert wird;
(b) Spalten eines Haarnadelpolynukleotids einer Vielzahl von Haarnadelpolynukleotiden mit der aktivierten programmierbaren Nuklease, wobei jedes Haarnadelpolynukleotid (i) eine oder mehrere RNA-Schleifen, die gespalten werden, (ii) einen ersten Abschnitt, der DNA umfasst, und (iii) einen zweiten Abschnitt, der durch eine der einen oder der mehreren RNA-Schleifen mit dem ersten Abschnitt verbunden ist, umfasst, wobei der zweite Abschnitt an ein Segment des ersten Abschnitts hybridisiert ist;
(c) Bilden eines zweiten Komplexes, umfassend den ersten Abschnitt des gespaltenen Haarnadelpolynukleotids, eine zweite programmierbare Nuklease und eine zweite nicht natürlich vorkommende Guide-Nukleinsäure, die an den ersten Abschnitt der gespaltenen Haarnadel hybridisiert, wodurch die zweite programmierbare Nuklease aktiviert wird;
(d) Spalten von Reportern mit der aktivierten ersten oder zweiten programmierbaren Nuklease; und
(e) Nachweisen einer Änderung eines Signals, wobei die Änderung des Signals durch Spaltung der Reporter erzeugt wird.

## Revendications

1. Système de détection d'un acide nucléique cible comprenant un tampon comprenant :
i. des réactifs d'amplification pour une réaction d'amplification ciblant l'acide nucléique cible ; et
ii. des réactifs de détection pour une réaction de détection ciblant l'acide nucléique cible ;
dans lequel les réactifs d'amplification comprennent une ou plusieurs amorces oligonucléotidiques, et une ADN polymérase ;
dans lequel les réactifs de détection comprennent une nucléase programmable, un acide nucléique guide d'origine non naturelle, et des rapporteurs ;
dans lequel l'acide nucléique guide d'origine non naturelle comprend une séquence qui s'hybride avec un segment de l'acide nucléique cible ou des amplicons d'ADN correspondants ;
dans lequel les réactifs d'amplification sont présents en quantités efficaces pour amplifier l'acide nucléique cible dans un échantillon d'essai afin de produire des amplicons d'ADN de l'acide nucléique cible ;
dans lequel la nucléase programmable et l'acide nucléique guide d'origine non naturelle forment un complexe dans le tampon qui est activé lors de la liaison d'un des amplicons d'ADN pour induire un clivage transcollatéral détectable des rapporteurs ; et
dans lequel le tampon est un tampon de lyse.

2. Système selon la revendication 1, dans lequel (a) au moins 1 nM des rapporteurs subissent un clivage transcollatéral dans l'heure suivant l'ajout d'au moins 5 000 copies de l'acide nucléique cible au système ; (b) au moins 5 nM des rapporteurs subissent un clivage transcollatéral dans l'heure suivant l'ajout d'au moins 5 000 copies de l'acide nucléique cible au système ; (c) au moins 10 nM des rapporteurs subissent un clivage transcollatéral dans l'heure suivant l'ajout d'au moins 5 000 copies de l'acide nucléique cible au système ; (d) au moins 1 nM des rapporteurs subissent un clivage transcollatéral dans l'heure suivant l'ajout d'au moins 1 000 copies de l'acide nucléique cible au système ; ou (e) au moins 1 nM des rapporteurs subissent un clivage transcollatéral dans l'heure suivant l'ajout d'au moins 1 000 copies de l'acide nucléique cible au système.

3. Système selon la revendication 1 ou la revendication 2, dans lequel les réactifs d'amplification comprennent des réactifs pour amplification isotherme ; facultativement, les réactifs d'amplification comprenant des réactifs pour amplification médiée par transcription (TMA), amplification dépendante de l'hélicase (HDA), amplification dépendante de l'hélicase circulaire (cHDA), amplification par déplacement de brin (SDA), amplification médiée par boucle (LAMP), réaction d'amplification exponentielle (EXPAR), amplification en cercle roulant (RCA), réaction en chaîne de ligase (LCR), méthode simple d'amplification des cibles d'ARN (SMART), amplification isotherme à amorce unique (SPIA), amplification par déplacement multiple (MDA), amplification basée sur une séquence d'acides nucléiques (NASBA), amplification des acides nucléiques dépendante d'une amorce initiée par une charnière (HIP), réaction d'amplification par enzyme de coupure (NEAR), ou amplification par déplacement multiple améliorée (IMDA).

4. Système selon la revendication 1, comprenant en outre un activateur pour la réaction d'amplification ; facultativement, dans lequel l'activateur pour la réaction d'amplification comprend un sel de magnésium ou de calcium.

5. Système selon l'une quelconque des revendications 1 à 4, dans lequel la nucléase programmable comprend au moins 60 % d'identité de séquence avec SEQ ID NO: 18-170, 221-268 ou 397-423.

6. Système selon l'une quelconque des revendications 1 à 5, dans lequel la nucléase programmable est une enzyme CRISPR-Cas, facultativement, une protéine effectrice CRISPR/Cas de type V.

7. Système selon l'une quelconque des revendications 1 à 6, comprenant en outre une transcriptase inverse, une amorce oligonucléotidique, et des dNTP pour la transcription inverse de l'acide nucléique cible.

8. Système selon l'une quelconque des revendications 1 à 7, le tampon comprenant un pH de 7,5 à 8,5, au moins 10 mM d'un agent tampon, au moins 1 mM d'acétate d'ammonium, au moins 10 mM d'acétate de potassium, au moins 2,5 mM d'acétate de magnésium, et au moins 0,5 % de glycérol ; facultativement dans lequel : (a) le tampon comprend un pH de 7,7 à 8,3, ou un pH de 7,85 à 8,15 ; (b) l'agent tampon comprend de l'HEPES, de l'imidazole, du TRIS-HCl, ou du phosphate ; et/ou (c) le tampon comprend en outre au moins 0,05 % en volume d'un détergent, facultativement le détergent comprenant du Tween 20.

9. Système selon l'une quelconque des revendications 1 à 7, le tampon comprenant un pH de 7,5 à 8,5, au moins 5 mM d'un agent tampon, au moins 20 mM d'acétate de potassium, au moins 2,5 mM d'acétate de magnésium, et au moins 0,5 % de glycérol ; facultativement dans lequel : (a) le tampon comprend un pH de 7,7 à 8,3, ou un pH de 7,85 à 8,15 ; (b) l'agent tampon comprend du phosphate ou du TRIS-HCl ; (c) le tampon comprend au moins 1 mM de sulfate d'ammonium ; et/ou (d) le tampon comprend au moins 0,05 % en volume d'un détergent, facultativement le détergent comprend du Tween 20.

10. Système selon l'une quelconque des revendications 1 à 7, le tampon comprenant un pH de 7,25 à 8,75, au moins 5 mM d'un agent tampon, au moins 7,5 mM d'acétate de potassium, au moins 1 mM d'acétate de magnésium, et au moins 0,5 % de glycérol ; facultativement dans lequel : (a) l'agent tampon comprend du phosphate ; (b) le tampon comprend un pH de 7,5 à 8,5, ou un pH de 7,75 à 8,25 ; (c) le tampon comprend en outre au moins 1 mM de sulfate d'ammonium ; et/ou (d) le tampon comprend en outre au moins 0,05 % en volume d'un détergent, facultativement le détergent comprenant du Tween 20.

11. Système selon la revendication 1 ou 2, comprenant en outre une matrice circulaire ayant une complémentarité interne formée à partir d'un seul brin polynucléotidique, dans lequel :
(a) la matrice circulaire comprend une première portion présentant une complémentarité avec l'une de la ou des amorces oligonucléotidiques et une deuxième portion présentant une complémentarité avec une portion de l'acide nucléique cible ;
(b) la complémentarité interne comprend une partie de la première portion et une partie de la deuxième portion ;
(c) la deuxième portion présente une longueur totale plus grande qu'une longueur combinée des première et deuxième portions qui sont comprises dans la complémentarité interne ; et
(d) la matrice circulaire subit un changement conformationnel lors de l'hybridation avec l'acide nucléique cible pour exposer la première portion à l'hybridation avec l'amorce oligonucléotidique.

12. Système selon la revendication 1 ou 2, comprenant en outre une matrice circulaire, dans lequel :
(a) la matrice circulaire comprend une première portion ayant une complémentarité avec une de la ou des amorces oligonucléotidiques et une deuxième portion ayant une complémentarité avec l'acide nucléique cible ;
(b) l'amorce oligonucléotidique complémentaire à la première portion comprend un motif de blocage au niveau de son extrémité 3' ; et
(c) l'amorce oligonucléotidique complémentaire à la première partie subit un clivage pour éliminer le motif de blocage par la nucléase programmable en présence de l'acide nucléique cible.

13. Système selon l'une quelconque des revendications 1 à 12, comprenant en outre une matrice de polymère, dans lequel la matrice de polymère est complexée avec les rapporteurs ; facultativement dans lequel : (a) la matrice de polymère est formée par copolymérisation d'au moins une première pluralité de monomères avec les rapporteurs, et/ou (b) la matrice de polymère comprend un hydrogel.

14. Procédé d'essai d'un acide nucléique cible dans un échantillon, le procédé comprenant :
a. l'amplification d'une portion de l'acide nucléique cible avec une ADN polymérase afin de produire des amplicons d'ADN de l'acide nucléique cible ;
b. la formation d'un complexe comprenant un des amplicons d'ADN, une nucléase programmable, et un acide nucléique guide d'origine non naturelle qui s'hybride avec un segment de l'amplicon d'ADN, activant ainsi la nucléase programmable ;
c. le clivage des rapporteurs avec la nucléase programmable activée ; et
d. la détection d'un changement d'un signal, le changement du signal étant produit par clivage des rapporteurs ;
dans lequel l'acide nucléique cible et les réactifs pour l'amplification et le clivage sont présents dans le même volume réactionnel ;
dans lequel le procédé comprend en outre la lyse d'une cellule ou d'un virus comprenant l'acide nucléique cible et dans lequel la lyse est effectuée dans le même volume de réaction que l'amplification et le clivage.

15. Système pour détecter un acide nucléique cible, comprenant des réactifs dans un tampon, dans lequel :
(a) les réactifs comprennent des polynucléotides en épingle à cheveux, des nucléases programmables, des acides nucléiques guides d'origine non naturelle et des rapporteurs ;
(b) chaque polynucléotide en épingle à cheveux comprend une ou plusieurs boucles d'ARN, une première portion comprenant de l'ADN, et une deuxième portion reliée à la première portion par une de la ou des boucles d'ARN ;
(c) chaque acide nucléique guide d'origine non naturelle comprend une séquence qui s'hybride avec un segment de l'acide nucléique cible ;
(d) dans chaque polynucléotide en épingle à cheveux, la deuxième portion du polynucléotide en épingle à cheveux s'hybride avec un segment de la première portion ;
(e) les nucléases programmables et les acides nucléiques guides d'origine non naturelle forment des complexes dans le tampon qui sont activés lors de la liaison de l'acide nucléique cible ;
(f) une nucléase programmable activée est efficace pour induire (i) le clivage transcollatéral de la ou des boucles d'ARN, et (ii) le clivage transcollatéral détectable des rapporteurs ; et
(g) le clivage d'une ou plusieurs boucles d'ARN d'un des polynucléotides en épingle à cheveux est efficace pour libérer la première portion du polynucléotide en épingle à cheveux afin de s'hybrider avec l'un des acides nucléiques guides d'origine non naturelle et de former une nucléase programmable activée supplémentaire.

16. Procédé de dosage d'un acide nucléique cible dans un échantillon, le procédé comprenant les étapes suivantes dans un seul volume de réaction :
(a) la formation d'un complexe comprenant l'acide nucléique cible, une première nucléase programmable, et un premier acide nucléique guide d'origine non naturelle qui s'hybride avec un segment de l'acide nucléique cible, activant ainsi la nucléase programmable ;
(b) le clivage d'un polynucléotide en épingle à cheveux d'une pluralité de polynucléotides en épingle à cheveux avec la nucléase programmable activée, dans lequel chaque polynucléotide en épingle à cheveux comprend (i) une ou plusieurs boucles d'ARN qui sont clivées, (i) une première portion comprenant de l'ADN, et (iii) une deuxième portion jointe à la première portion par une des une ou plusieurs boucles d'ARN, dans lequel la deuxième portion est hybridée avec un segment de la première portion ;
(c) la formation d'un second complexe comprenant la première portion du polynucléotide en épingle à cheveux clivé, une seconde nucléase programmable et un second acide nucléique guide d'origine non naturelle qui s'hybride à la première portion de l'épingle à cheveux clivée, activant ainsi la seconde nucléase programmable ;
(d) le clivage des rapporteurs avec la première ou la deuxième nucléase programmable activée ; et
(e) la détection d'un changement dans un signal, le changement dans le signal étant produit par clivage des rapporteurs.
